# EUROPEAN PATENT APPLICATION

(11) **EP 1 016 656 A1**
(43) Date of publication of application: **05.07.2000**
(21) Application number: 98941671.4
(22) Date of filing: 02.09.1998
(51) Int. Cl.: C07C 317/22, A01N 25/22, C08K 5/41, C09K 3/00

(54) **MOLECULAR COMPOUNDS CONTAINING PHENOL DERIVATIVES AS CONSTITUENT**

(30) Priority: 02.09.1997 JP 25293097; 22.10.1997 JP 30805897
(71) Applicant: NIPPON SODA CO., LTD., Chiyoda-ku, Tokyo 100-8165 (JP)
(72) Inventor: AOKI, Izuo, R&D Lab. for Specialty Chemicals, Ichihara-shi, Chiba 290-0045 45 (JP); SATO, Takehiro, R&D Lab. for Specialty Chemicals, Ichihara-shi, Chiba 290-0045 (JP); AMAIKE, Masato, R&D Lab. for Specialty Chemicals, Ichihara-shi, Chiba 290-0045 (JP); SUZUKI, Hiroshi, R&D Lab. for Specialty Chemicals, Ichihara-shi, Chiba 290-0045 (JP)
(74) Representative: Wibbelmann, Jobst, Dr., Dipl.-Chem.
(86) International application number: JP9803917
(87) International publication number: WO9911609

(57) **Abstract**

Novel molecular compounds which have an excellent performance in the technical fields where a useful substance is selectively separated, chemically stabilized, rendered nonvolatile or gradually releasable, powdered, or otherwise treated. The molecular compounds are produced from a phenol derivative represented by general formula (I), e.g., 3,3'-bis(phenylsulfonyl)-4,4'-dihydroxydiphenyl sulfone or 2,4-bis(phenylsulfonyl)phenol, as a constituent compound.

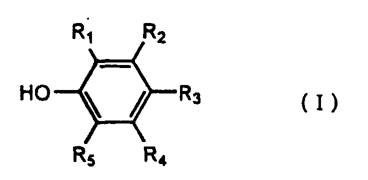

## Description

### Technical Fields:

This invention relates to novel molecular compounds, and, in more detail, to molecular compounds containing phenol derivatives with a specific structure as a constituent and to processes for producing them.

### Background Art:

Molecular compounds are compounds that two or more compounds are bonded by relatively weak interactions, other than covalent bonds, which are represented by hydrogen bonds and van der Waals forces. They can be dissociated into each original compound by means of simple operations. Because of this they are expected in recent years to be applicable in technical fields where a useful substance is selectively separated, chemically stabilized, rendered nonvolatile, gradually releasable, powdered or otherwise treated.

A concrete example of the molecular compounds is clathrate compounds. For example, clathrate compounds of 5-chloro-2-methyl-4-isothiazolin-3-one with 1,1,6,6-tetraphenyl-2,4-hexadiyne-1,6-diol or 1,1-(2,4-dimethylphenyl)-2-propyn-1-ol are described in Japanese Patent Laid-Opened No. Sho 61-53201, and with 1,1'-bis-2-naphthol in Japanese Patent Laid-Opened No. Sho 62-22701. In Japanese Patent Laid-Opened No. Hei 3-279373 clathrate compounds composed of bisphenol compounds and isothiazolone compounds are reported. Furthermore clathrate compounds of tetrakisphenols with various organic compounds are disclosed in Japanese Patent Laid-Opened No. Hei 6-166646.

Conventional technologies have not, however, yet produced molecular compounds with fully satisfactory performances in selective separation, chemical stabilization, rendering nonvolatile, gradual release, powdering and other treatments.

### Disclosure of the Invention:

It is an object for the present invention to provide novel molecular compounds that contain phenol derivatives with a specific structure as a constituent and that have excellent performances in technological fields where a useful substance is selectively separated, chemically stabilized, rendered nonvolatile, gradually releasable, powdered or otherwise treated.

The inventors of this invention have made intensive investigation to achieve the object mentioned above, and found that phenol derivatives having a sulfonyl group at the ortho position of a hydrogen group and a carbonyl group produce molecular compounds effectively and that the molecular compounds have excellent performances in technological fields where a useful substance is selectively separated, chemically stabilized, rendered nonvolatile, gradually releasable, powdered or otherwise treated. Thus the present invention has been accomplished.

This invention is directed to molecular compounds containing, as a constituent, phenol derivatives of Formula (I) 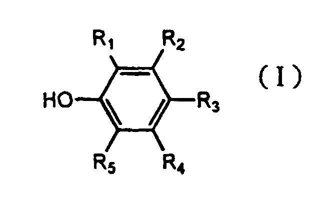 [wherein R₁ and R₅ are, same or different, groups selected from hydrogen, halogen, alkyl having 1 to 4 carbons, alkenyl having 2 to 4 carbons, alkoxy having 1 to 4 carbons, hydroxyl, or 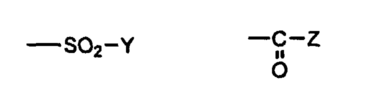 (wherein Y and Z are alkyl having 1 to 8 carbons, alkenyl having 2 to 8 carbons, alkoxy having 1 to 6 carbons, hydroxyl, optionally substituted amino, optionally substituted cycloalkyl, optionally substituted phenyl or optionally substituted aralkyl);

R₂ and R₄ are, same or different, groups selected from hydrogen, halogen, alkyl having 1 to 4 carbons, alkenyl having 2 to 4 carbons, alkoxy having 1 to 4 carbons or hydroxyl; but, in case R₁, R₃ or R₅ is alkoxy of 1 to 4 carbons or hydroxyl, they are hydrogen, halogen, alkyl having 1 to 4 carbons, alkenyl having 2 to 4 carbons, alkoxy having 1 to 4 carbons, hydroxyl or 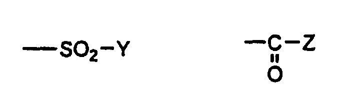 (wherein Y and Z are as defined above);

R₃ is a group selected from hydrogen, halogen, alkyl having 1 to 4 carbons, alkenyl having 2 to 4 carbons, alkoxy having 1 to 4 carbons, hydroxyl, Formula (II) or Formula (III) 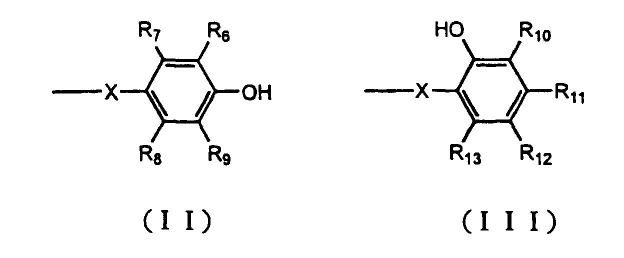 {wherein X is 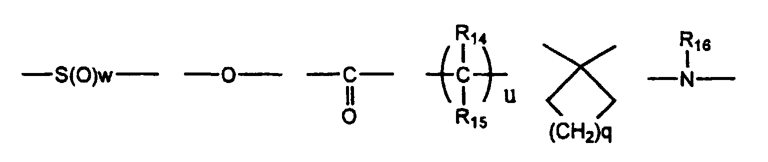 (wherein w is 0, 1 or 2; u is 0 or 1; q is 0 to 4; R₁₄ and R₁₅ are, same or different, hydrogen, halogen, alkyl having 1 to 4 carbons, alkenyl having 2 to 4 carbons, alkoxy having 1 to 4 carbons, hydroxyl, optionally substituted phenyl or optionally substituted aralkyl; R₁₆ is hydrogen, alkyl having 1 to 4 carbons, alkenyl having 2 to 4 carbons, alkoxy having 1 to 4 carbons, hydroxyl, optionally substituted phenyl or optionally substituted aralkyl);
R₆, R₉ and R₁₀ are, same or different each other, groups selected from hydrogen, halogen, alkyl having 1 to 4 carbons, alkenyl having 2 to 4 carbons, alkoxy having 1 to 4 carbons, hydroxyl or 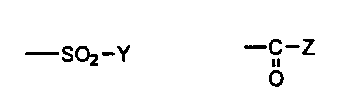 (wherein Y and Z are as defined above);
R₇, R₈, R₁₁ and R₁₃ are, same or different each other, hydrogen, halogen, alkyl having 1 to 4 carbons, alkenyl having 2 to 4 carbons, alkoxy having 1 to 4 carbons or hydroxyl; but, in case R₁₂ is alkoxy having 1 to 4 carbons or hydroxyl, R₁₁ is hydrogen, halogen, alkyl having 1 to 4 carbons, alkenyl having 2 to 4 carbons, alkoxy having 1 to 4 carbons, hydroxyl, or 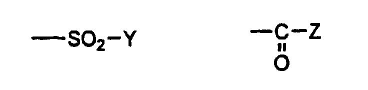 (wherein Y and Z are as defined above);
R₁₂ is a group selected from hydrogen, halogen, alkyl having 1 to 4 carbons, alkenyl having 2 to 4 carbons, alkoxy having 1 to 4 carbons, hydroxyl, or 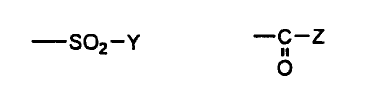 (wherein Y and Z are as defined above)}, or 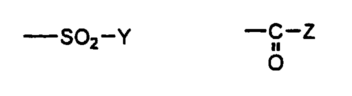 (wherein Y and Z are as defined above), or
in case R₃ is represented by Formula (II), one of R₁, R₅, R₆ and R₉ is 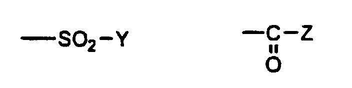 (wherein Y and Z are as defined above),
in case R₃ is represented by Formula (III), at least one of R₁, R₅ and R₁₀ is 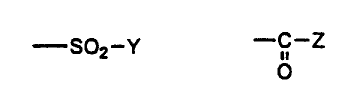 (wherein Y and Z are as defined above), and
in case R₃ is a group other than Formula (II) or (III), either R₁ or R₅ is 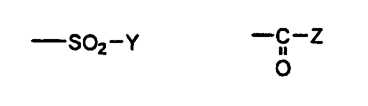 (wherein Y and Z are as defined above)].

The present invention also relate to molecular compounds that contain a phenol derivative of Formula (I) as a constituent and that are characterized to be clathrate compounds, and to molecular compounds containing, as constituents, a phenol derivative of Formula (I) and antibacterial agents, antifungal agents, insecticides, noxious insect repellants, perfumes, deodorants, antifouling agents, curing agents and accelerators for coating materials, resins and adhesives, natural essential oils, antioxidants, vulcanization accelerators or organic solvents that react with the said phenol derivative to form a molecular compound. The present invention further relates to processes for producing any of the molecular compounds mentioned above by reacting a phenol derivative of Formula (I) with constituent compounds that react with the said phenol derivative to form a molecular compound.

The molecular compounds of the present invention are defined as compounds that two or more constituent compounds able to exist stably on their own are bonded by relatively weak interactions, other than covalent bonds, which are represented by hydrogen bonds and van der Waals forces. Compounds such as hydrates, solvates, adducts and clathrate compounds are included in them.

In Formula (I), R₁ and R₅ are groups selected from hydrogen, halogen, alkyl having 1 to 4 carbons, alkenyl having 2 to 4 carbons, alkoxy having 1 to 4 carbons, hydroxyl or 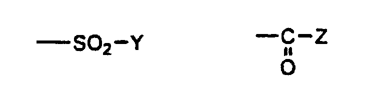 (wherein Y and Z are alkyl having 1 to 8 carbons, alkenyl having 2 to 8 carbons, alkoxy having 1 to 6 carbons, hydroxyl, optionally substituted amino, optionally substituted cycloalkyl, optionally substituted phenyl or optionally substituted aralkyl).

Their examples include fluorine, chlorine, bromine, iodine, methyl, ethyl, n-propyl, isopropyl, n-butyl, isobutyl, sec-butyl, tert-butyl, vinyl, allyl, isopropenyl, 1-propenyl, 2-butenyl, 3-butenyl, 1,3-butanedienyl, methoxy, ethoxy, n-propoxy, isopropoxy, n-butoxy, isobutoxy, sec-butoxy or tert-butoxy.

Examples of Y and Z include methyl, ethyl, n-propyl, isopropyl, n-butyl, isobutyl, sec-butyl, tert-butyl, n-pentyl, isopentyl, sec-pentyl, neo-pentyl, tert-pentyl, n-hexyl, isohexyl, sec-hexyl, n-heptyl, isoheptyl, sec-heptyl, n-octyl, isooctyl, sec-octyl, vinyl, allyl, 1-propenyl, isopropenyl, 1-butenyl, 2-butenyl, 3-butenyl, 1,3-butanedienyl, 1-pentenyl, 2-pentenyl, 3-pentenyl, 4-pentenyl, hexynyl, hexydinyl, heptynyl, heptydinyl, octynyl, octydinyl, cyclopentyl, methylcyclopentyl, dimethylcyclopentyl, cyclohexyl, methylcyclohexyl, dimethylcyclohexyl, trimethylcyclohexyl, tetramethylcyclohexyl, pentamethylcyclohexyl, hexamethylcyclohexyl, cycloheptyl, methylcycloheptyl, phenyl, o-tolyl, m-tolyl, p-tolyl, 2,3-xylyl, 2,4-xylyl, 2,5-xylyl, 2,6-xylyl, 3,4-xylyl, 3,5-xylyl, o-cumenyl, m-cumenyl, p-cumenyl, mesityl, benzyl, o-tolylmethyl, m-tolylmethyl, p-tolylmethyl, 2,3-xylylmethyl, 2,4-xylylmethyl, 2,5-xylylmethyl, 2,6-xylylmethyl, 3,4-xylylmethyl, 3,5-xylylmethyl, mesitylmethyl, o-cumenylmethyl, m-cumenylmethyl, p-cumenylmethyl, phenethyl, α-methylbenzyl, 1-naphthyl, 2-naphthyl, methoxy, ethoxy or dimethylamino. The examples of Y and Z in R₂, R₃ and R₄ are as described above in this text unless otherwise described.

R₂ and R₄ are hydrogen, halogen, alkyl having 1 to 4 carbons, alkenyl having 2 to 4 carbons, alkoxy having 1 to 4 carbons, or hydroxyl. But, in case R₃ is alkoxy having 1 to 4 carbons or hydroxyl, they are groups selected from hydrogen, halogen, alkyl having 1 to 4 carbons, alkenyl having 2 to 4 carbons, alkoxy having 1 to 4 carbons, hydroxyl, or 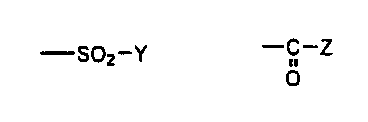 (wherein Y and Z are as defined above). Their examples include fluorine, chlorine, bromine, iodine, methyl, ethyl, n-propyl, isopropyl, n-butyl, isobutyl, sec-butyl, vinyl, allyl, isopropenyl, 1-propenyl, 2-butenyl, 3-butenyl, 1,3-butanedienyl, methoxy, ethoxy, n-propoxy, isopropoxy, n-butoxy, isobutoxy, sec-butoxy, tert-butoxy, phenylsulfonyl or benzoyl.

R₃ is a group selected from hydrogen, halogen, alkyl having 1 to 4 carbons, alkenyl having 2 to 4 carbons, alkoxy having 1 to 4 carbons, hydroxyl, Formula (II), Formula (III), or 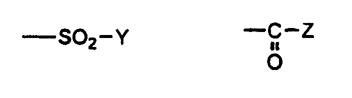 (wherein Y and Z are as defined above). Its examples include fluorine, chlorine, bromine, iodine, methyl, ethyl, n-propyl, isopropyl, n-butyl, isobutyl, sec-butyl, tert-butyl, vinyl, allyl, isopropenyl, 1-propenyl, 2-butenyl, 3-butenyl, 1,3-butanedienyl, methoxy, ethoxy, n-propoxy, isopropoxy, n-butoxy, isobutoxy, sec-butoxy, tert-butoxy, 2-hydroxy-3-phenylsulfonyl-phenylsulfonyl or 4-hydroxy-3-phenylsulfonyl-phenylsulfonyl.

In Formulae (II) and (III), X is 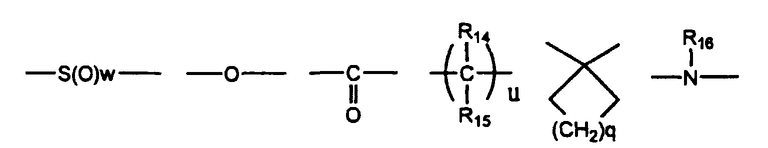 (wherein w is 0, 1 or 2; u is 0 or 1; q is 0 to 4; R₁₄ and R₁₅ are hydrogen, halogen, alkyl having 1 to 4 carbons, alkenyl having 2 to 4 carbons, alkoxy having 1 to 4 carbons, hydroxyl, optionally substituted phenyl or optionally substituted aralkyl; R₁₆ is hydrogen, alkyl having 1 to 4 carbons, alkenyl having 2 to 4 carbons, alkoxy having 1 to 4 carbons, hydroxyl, optionally substituted phenyl or optionally substituted aralkyl).

Its examples include 1,1-dimethylmethylene, 1-methyl-t-butyl-methylene, 1-methyl-1-phenyl-methylene, 1-methyl-1-hydroxymethylene, N-methylimino, N-methoxyimino, N-allylimino, 1,1-cyclohexylene or 1,1-cyclopentylene.

In Formulae (II) and (III), R₆, R₉ and R₁₀ are groups selected from hydrogen, halogen, alkyl having 1 to 4 carbons, alkenyl having 2 to 4 carbons, alkoxy having 1 to 4 carbons, hydroxyl or 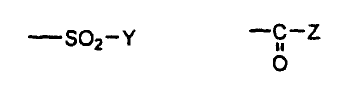 (wherein Y and Z are as defined above).

Their examples include fluorine, chlorine, bromine, iodine, methyl, ethyl, n-propyl, isopropyl, n-butyl, isobutyl, sec-butyl, tert-butyl, vinyl, allyl, isopropenyl, 1-propenyl, 2-butenyl, 3-butenyl, 1,3-butanedienyl, methoxy, ethoxy, n-propoxy, isopropoxy, n-butoxy, isobutoxy, sec-butoxy, tert-butoxy, phenylsulfonyl or benzoyl.

R₇, R₈, R₁₁ and R₁₃ are hydrogen, halogen, alkyl having 1 to 4 carbons, alkenyl having 2 to 4 carbons, alkoxy having 1 to 4 carbons or hydroxyl, but, in case R₁₂ is alkoxy having 1 to 4 carbons or hydroxyl, R₁₁ is a group selected from hydrogen, halogen, alkyl having 1 to 4 carbons, alkenyl having 2 to 4 carbons, alkoxy having 1 to 4 carbons, hydroxyl or 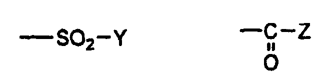 (wherein Y and Z are as defined above).

Their examples include fluorine, chlorine, bromine, iodine, methyl, ethyl, n-propyl, isopropyl, n-butyl, isobutyl, sec-butyl, tert-butyl, vinyl, allyl, isopropenyl, 1-propenyl, 2-butenyl, 3-butenyl, 1,3-butanedienyl, methoxy, ethoxy, n-propoxy, isopropoxy, n-butoxy, isobutoxy, sec-butoxy, tert-butoxy, phenylsulfonyl or benzoyl.

R₁₂ is a group selected from hydrogen, halogen, alkyl having 1 to 4 carbons, alkenyl having 1 to 4 carbons, hydroxyl or 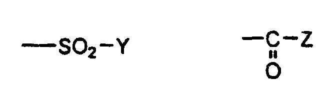 (wherein Y and Z are as defined above).

Its examples include fluorine, chlorine, bromine, iodine, methyl, ethyl, n-propyl, isopropyl, n-butyl, isobutyl, sec-butyl, tert-butyl, vinyl, allyl, isopropenyl, 1-propenyl, 2-butenyl, 3-butenyl, 1,3-butanedienyl, methoxy, ethoxy, n-propoxy, isopropoxy, n-butoxy, isobutoxy, sec-butoxy, tert-butoxy, phenylsulfonyl or benzoyl.

Phenol derivatives used in the present invention are not particularly restricted if they are compounds represented by Formula (I). Examples of the compounds of Formula (I) are listed in Tables 1, 2 and 3.

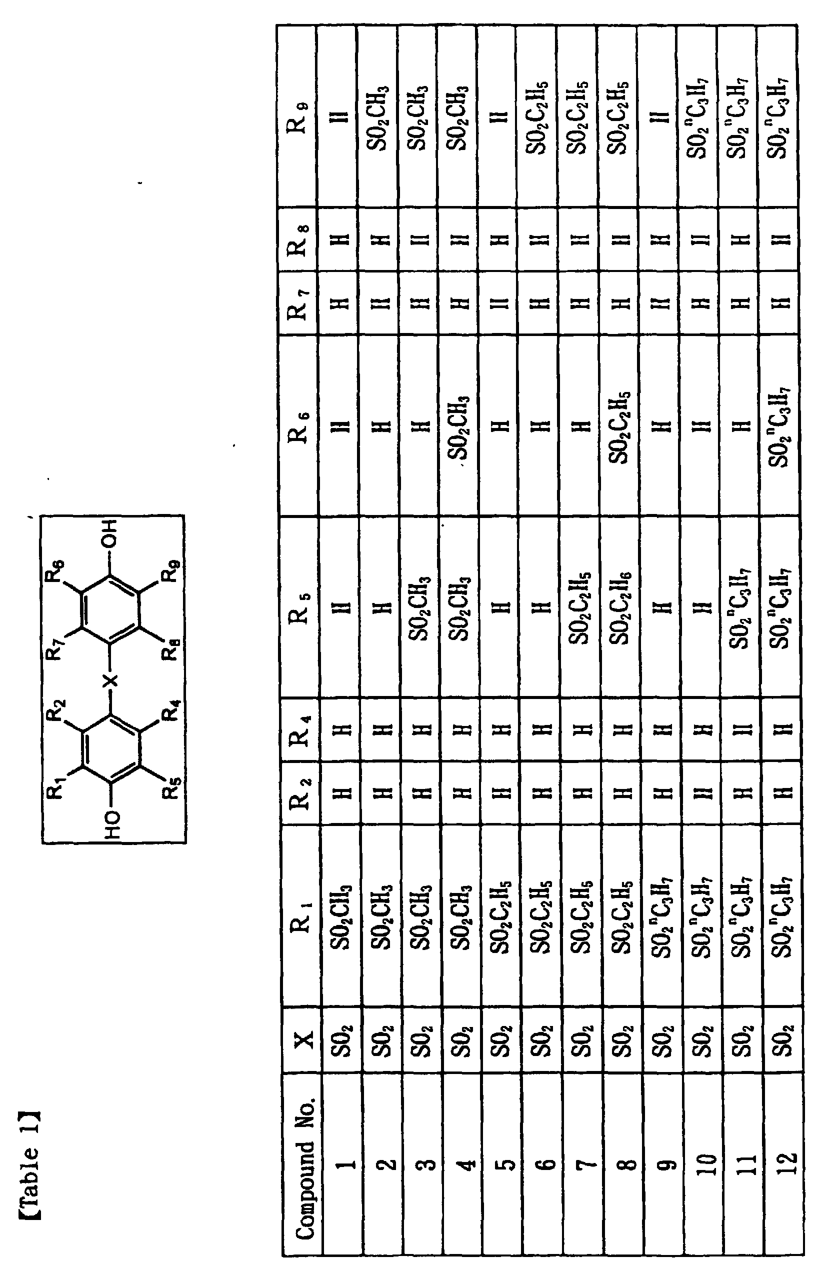

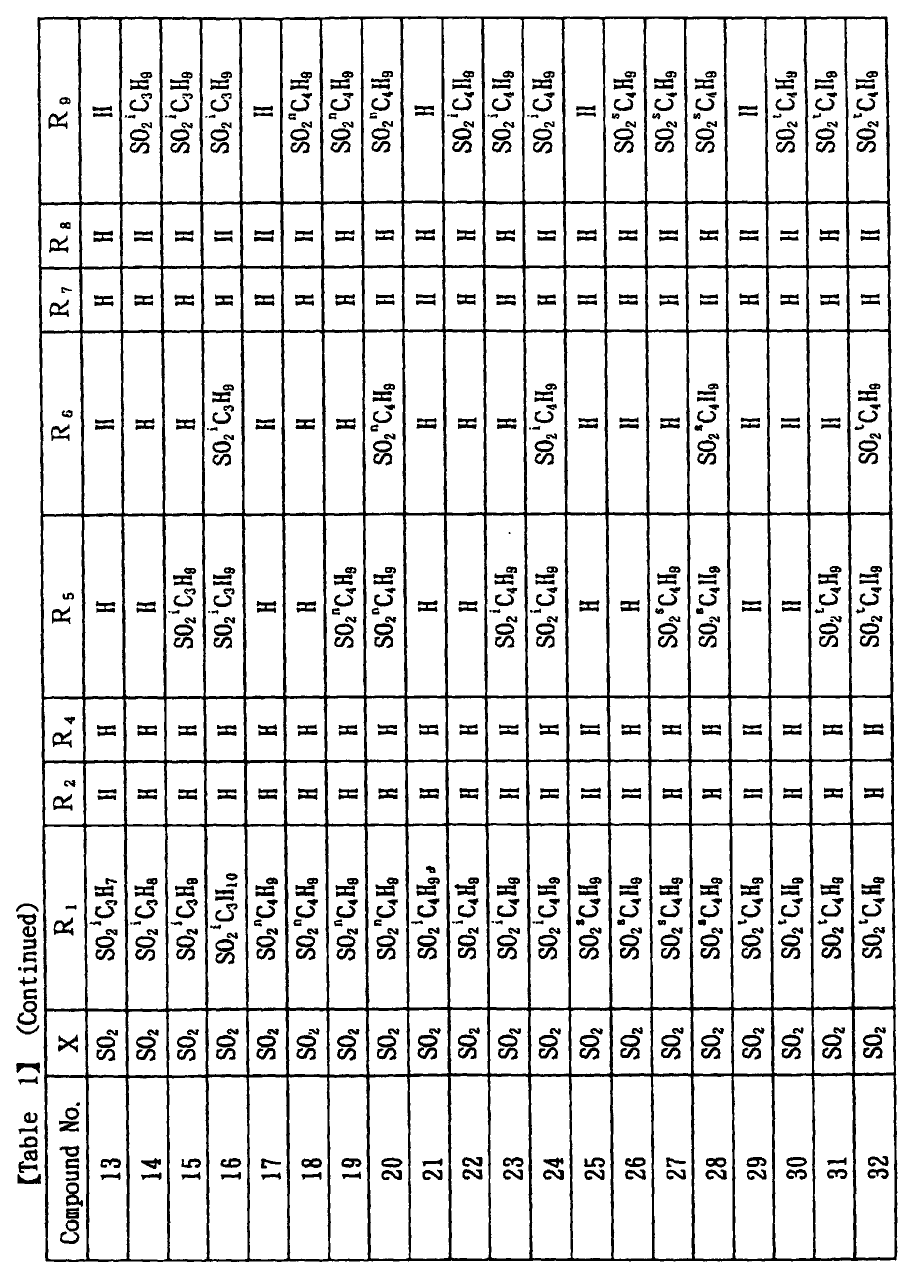

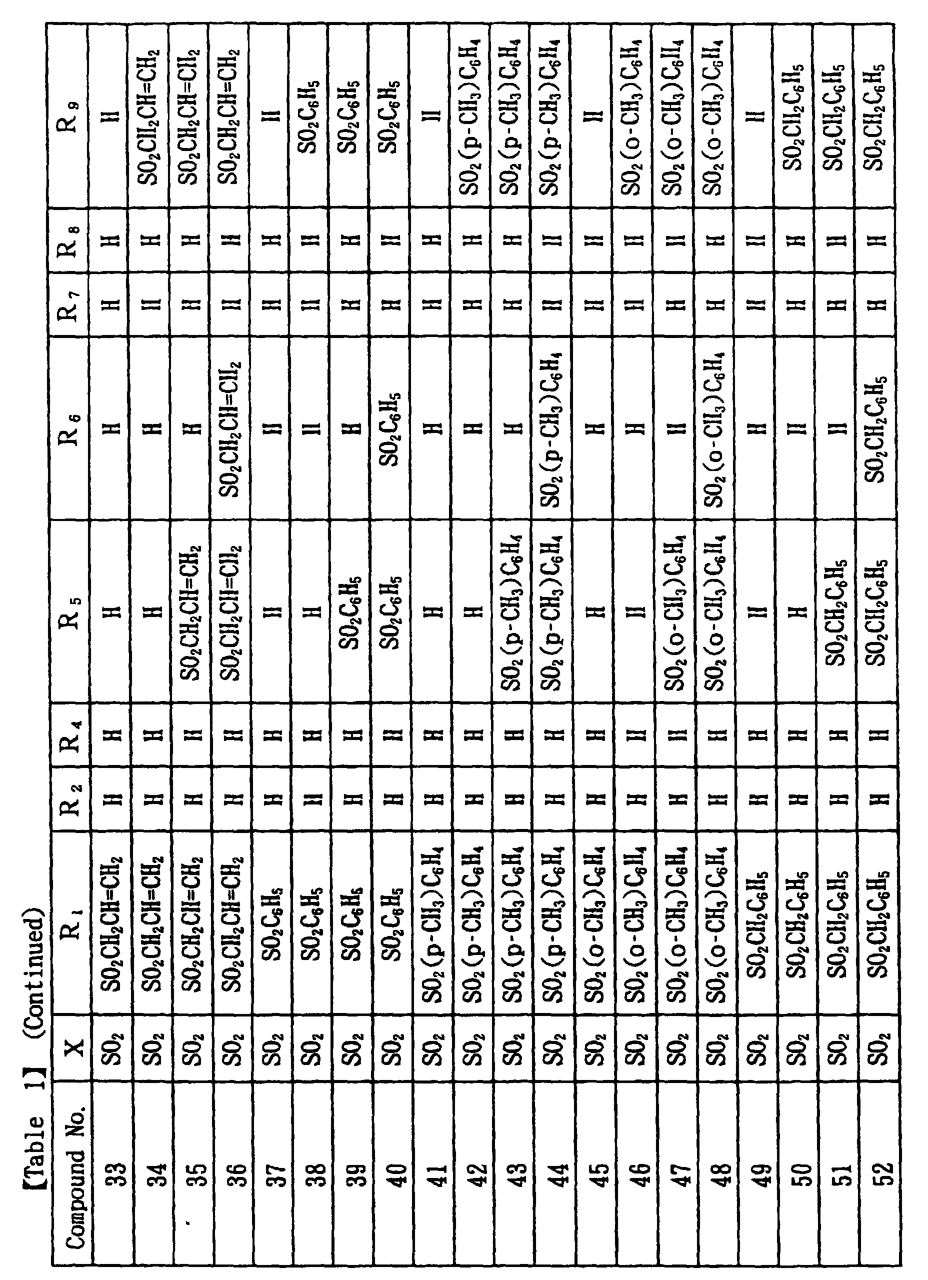

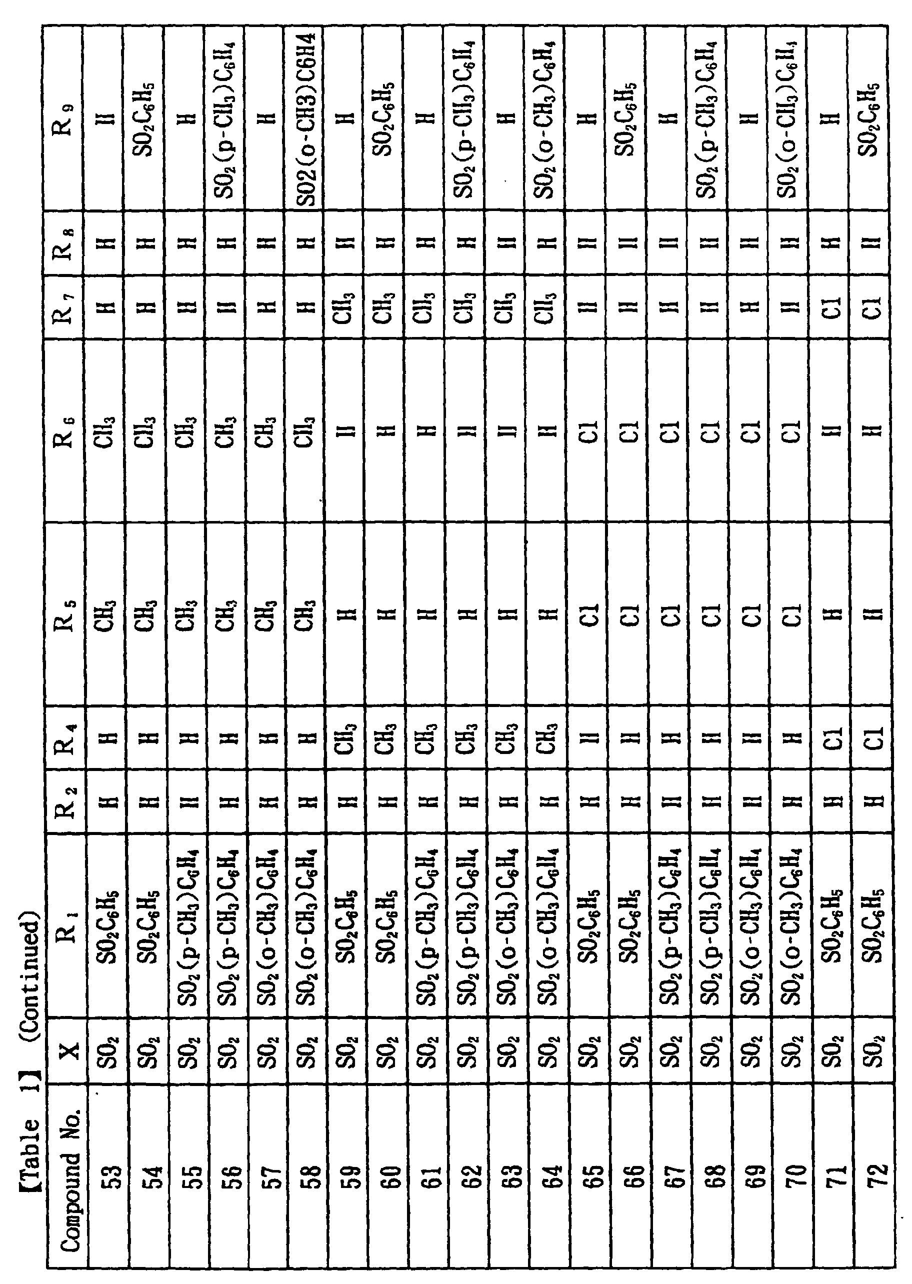

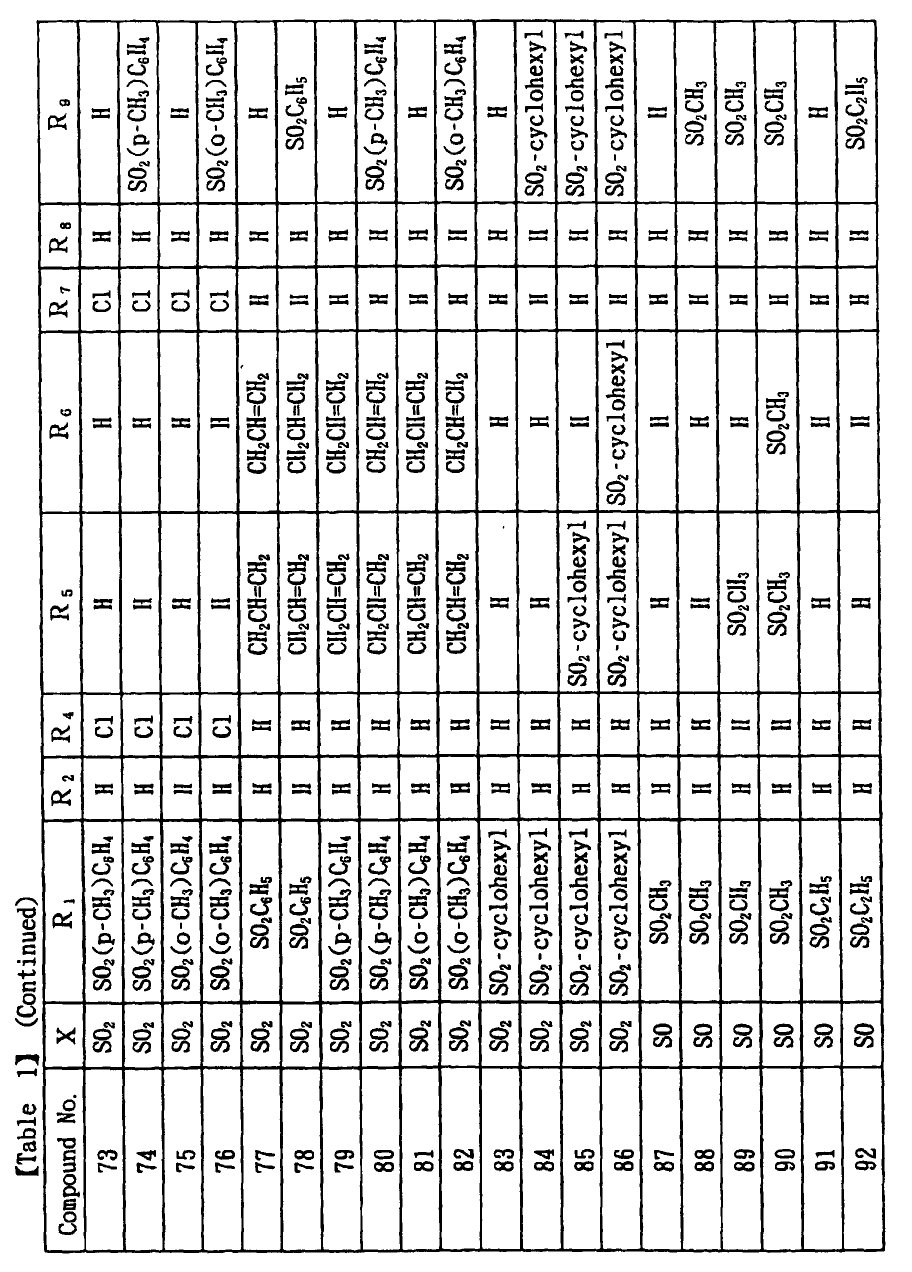

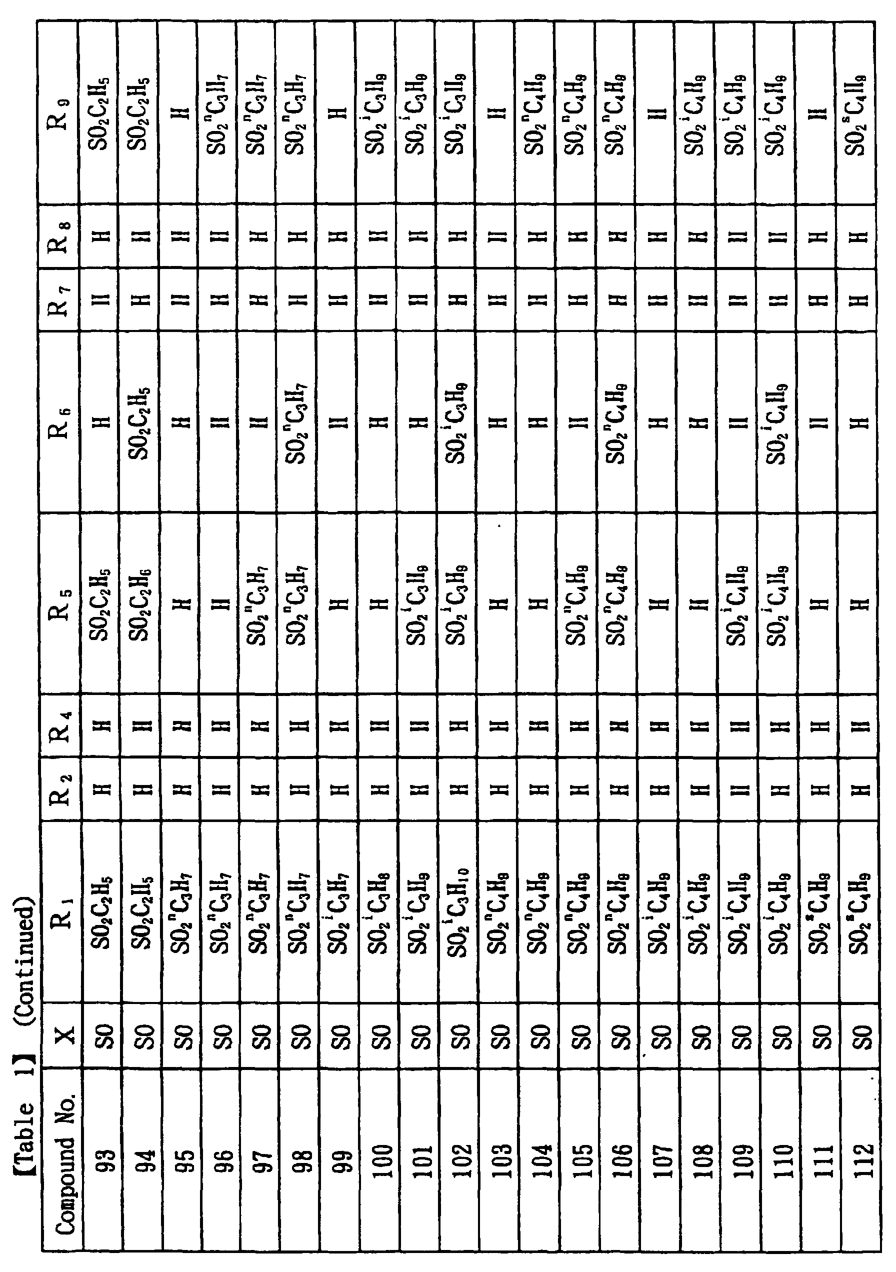

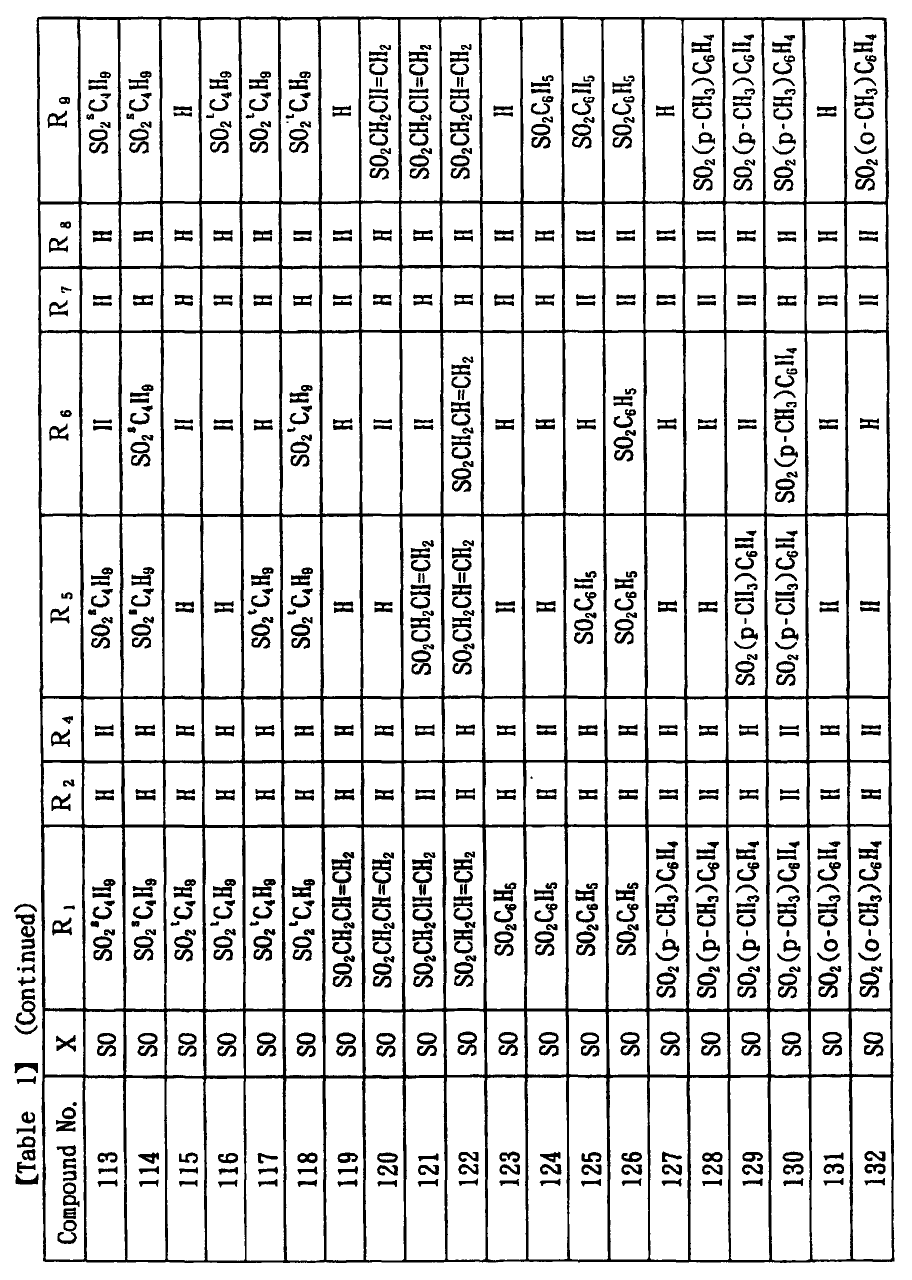

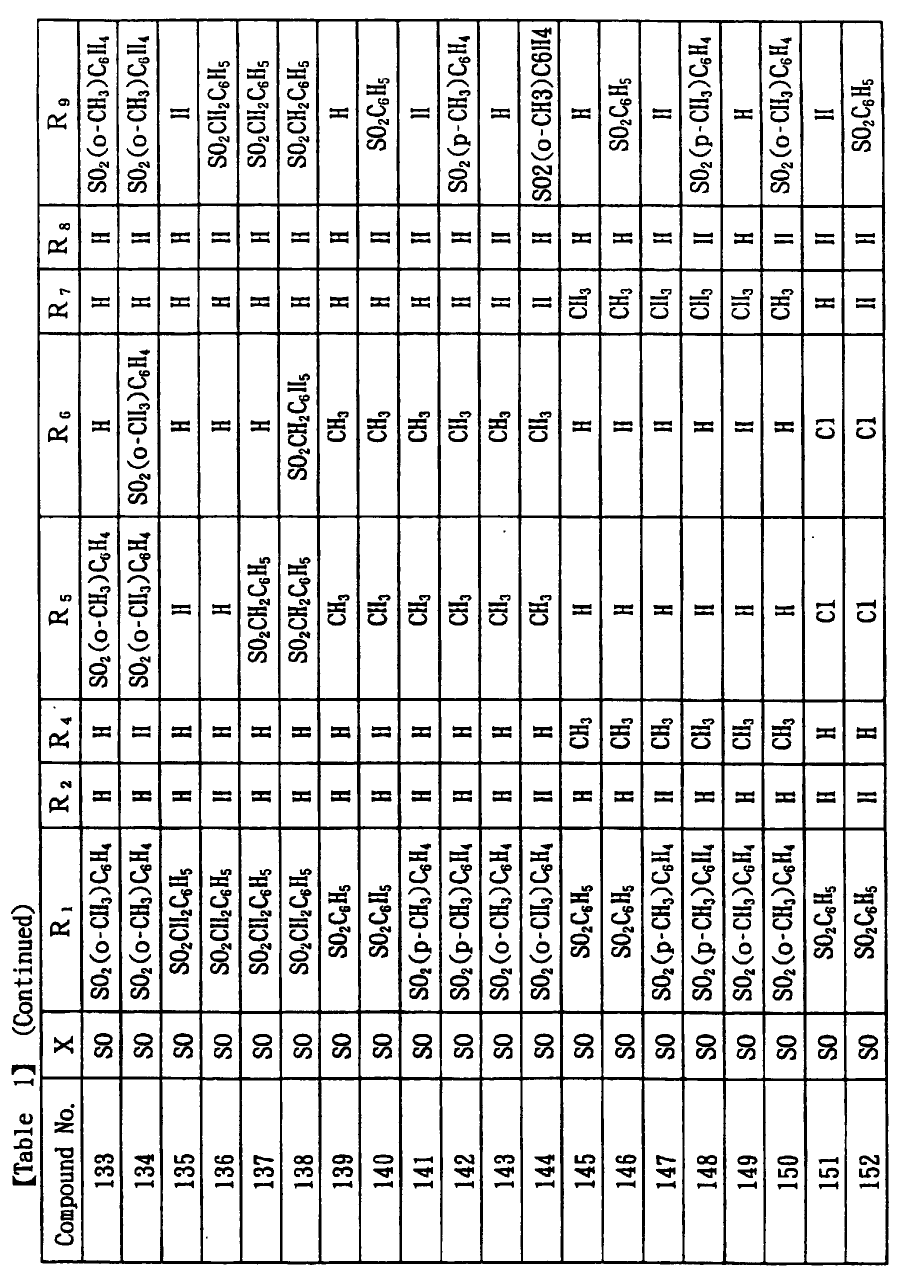

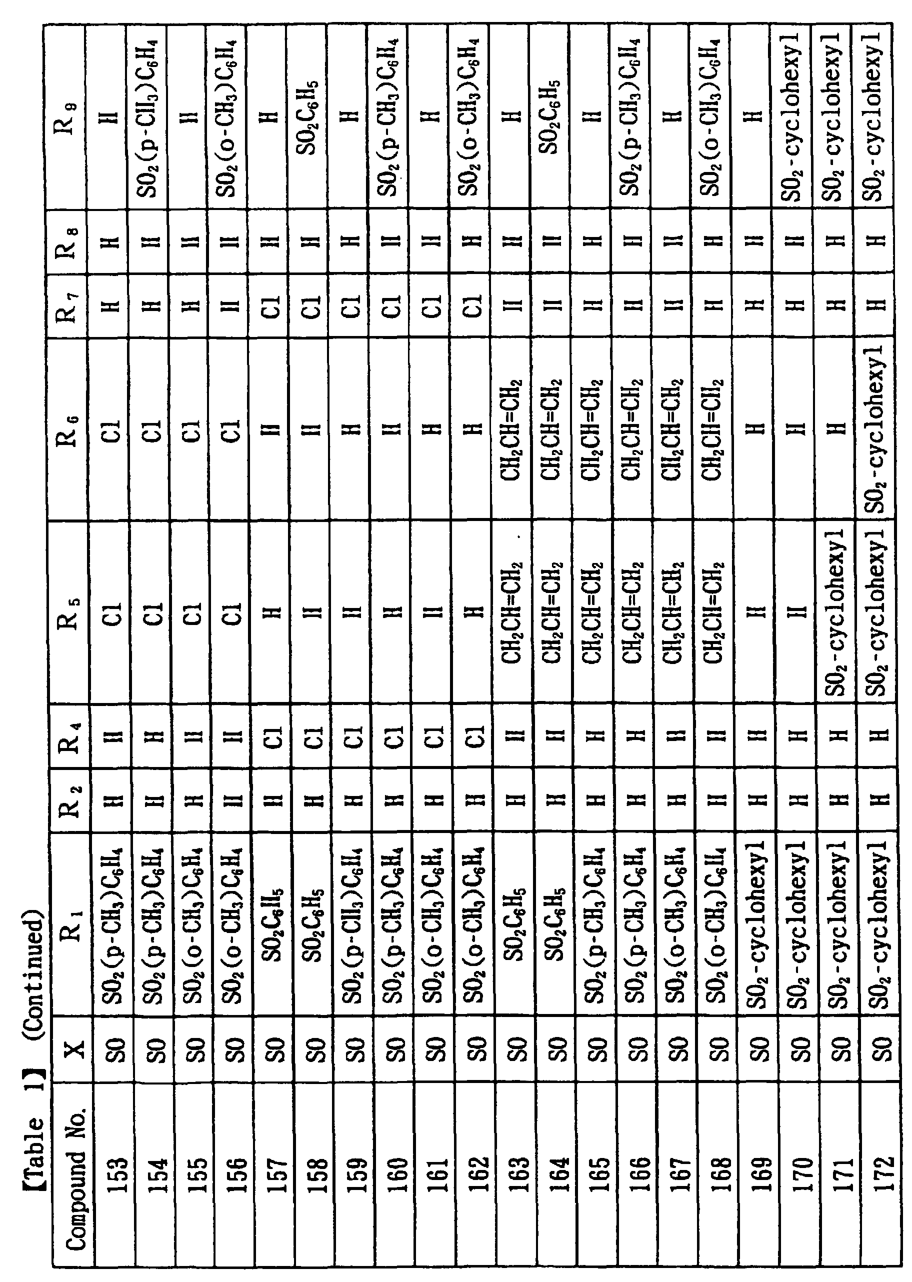

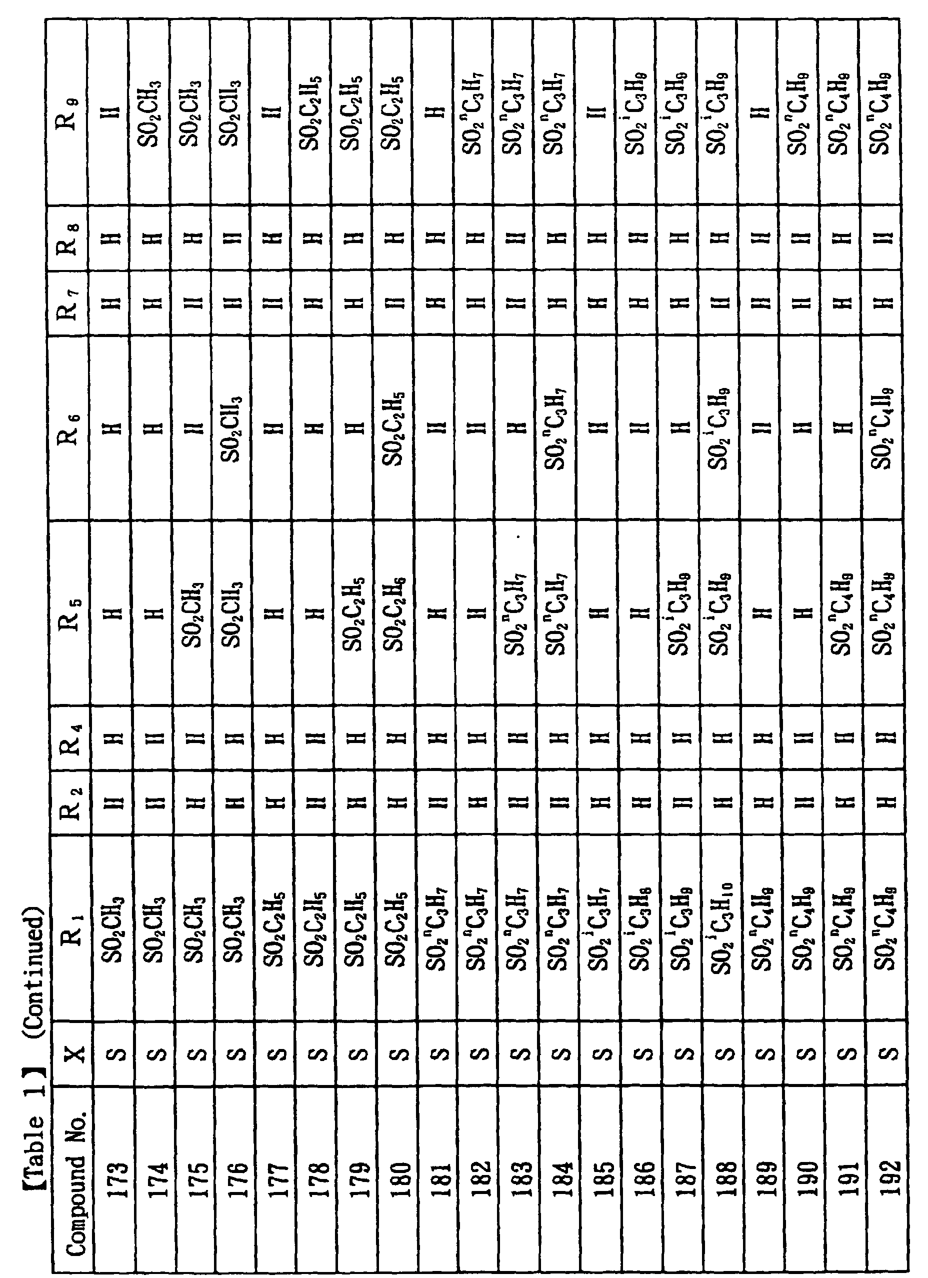

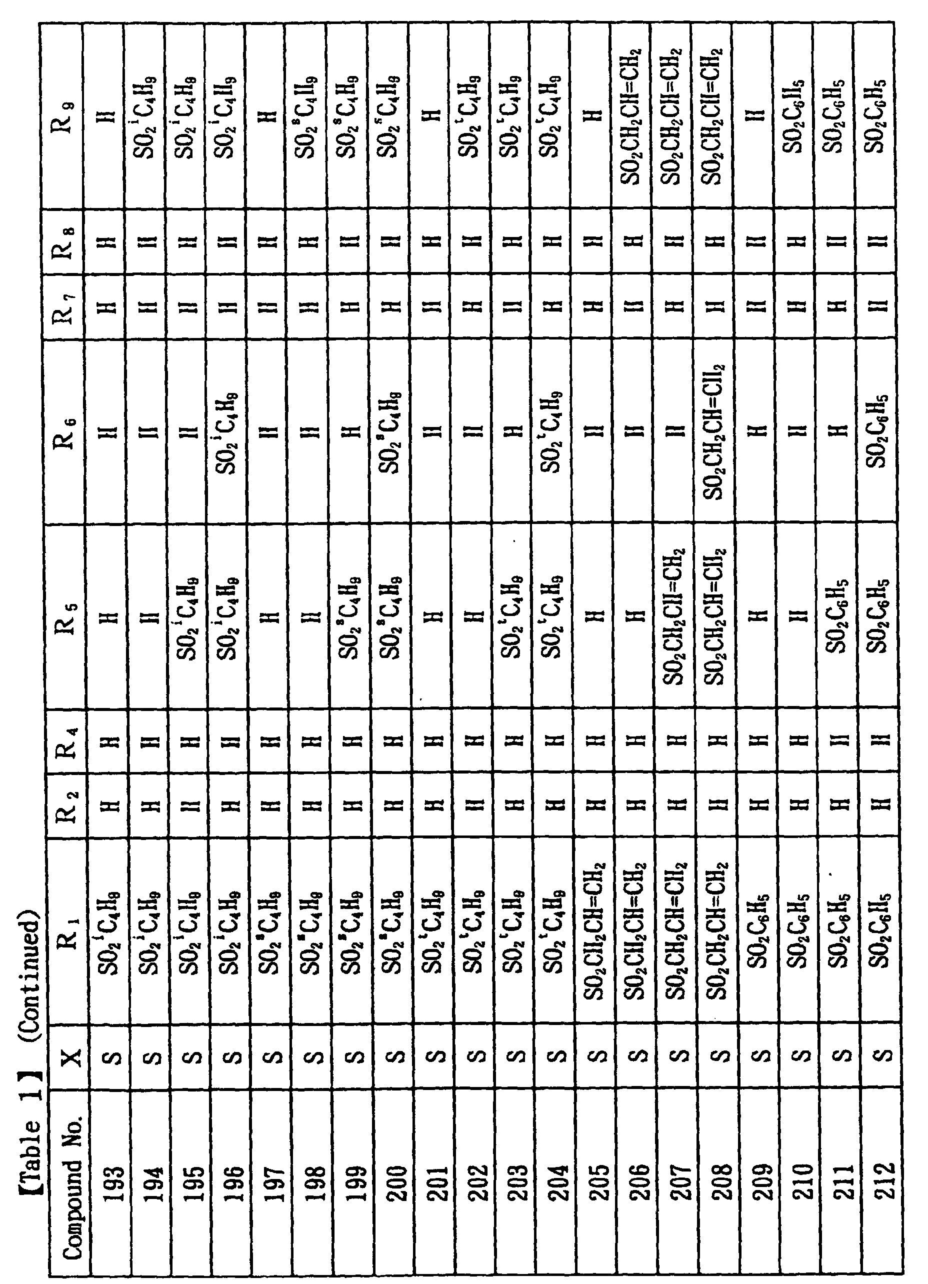

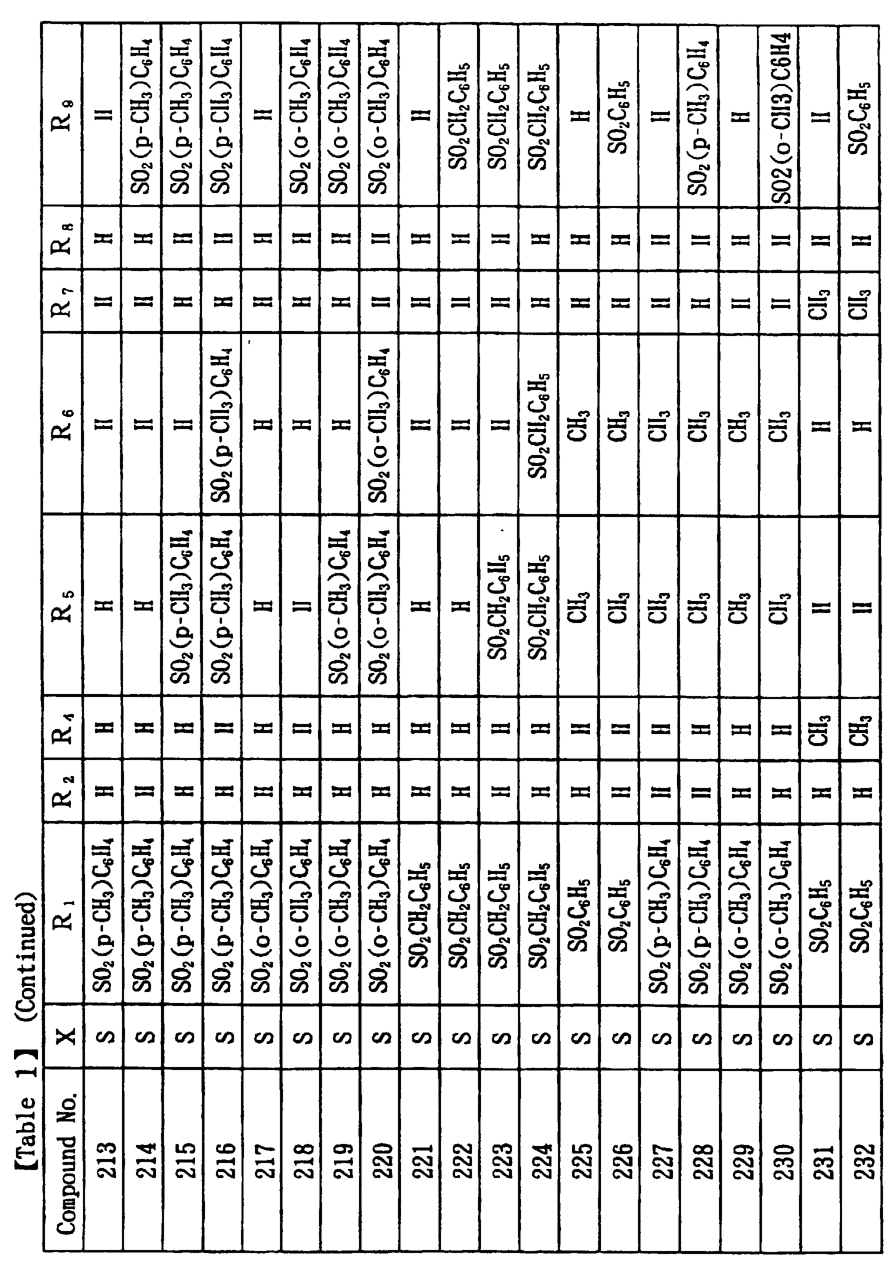

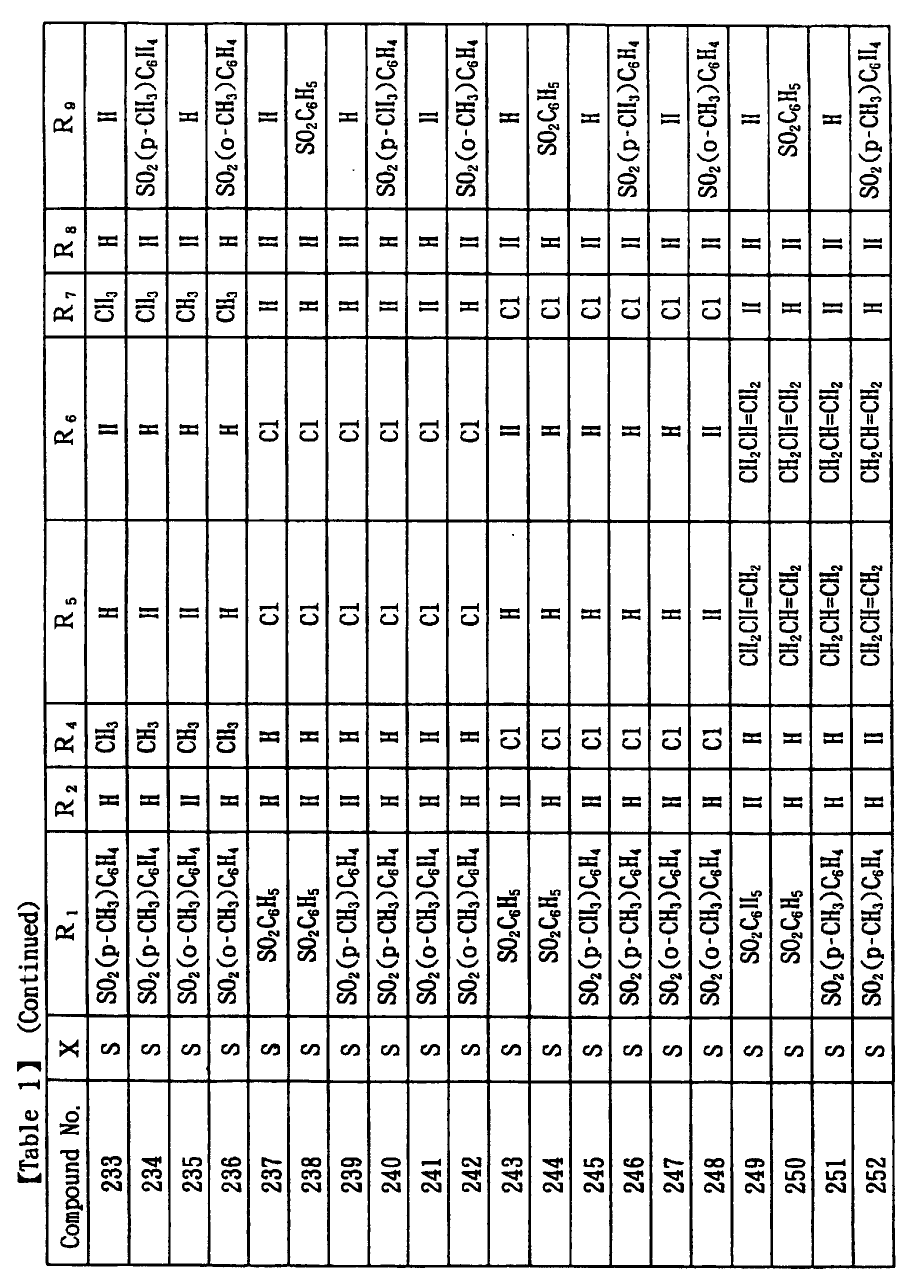

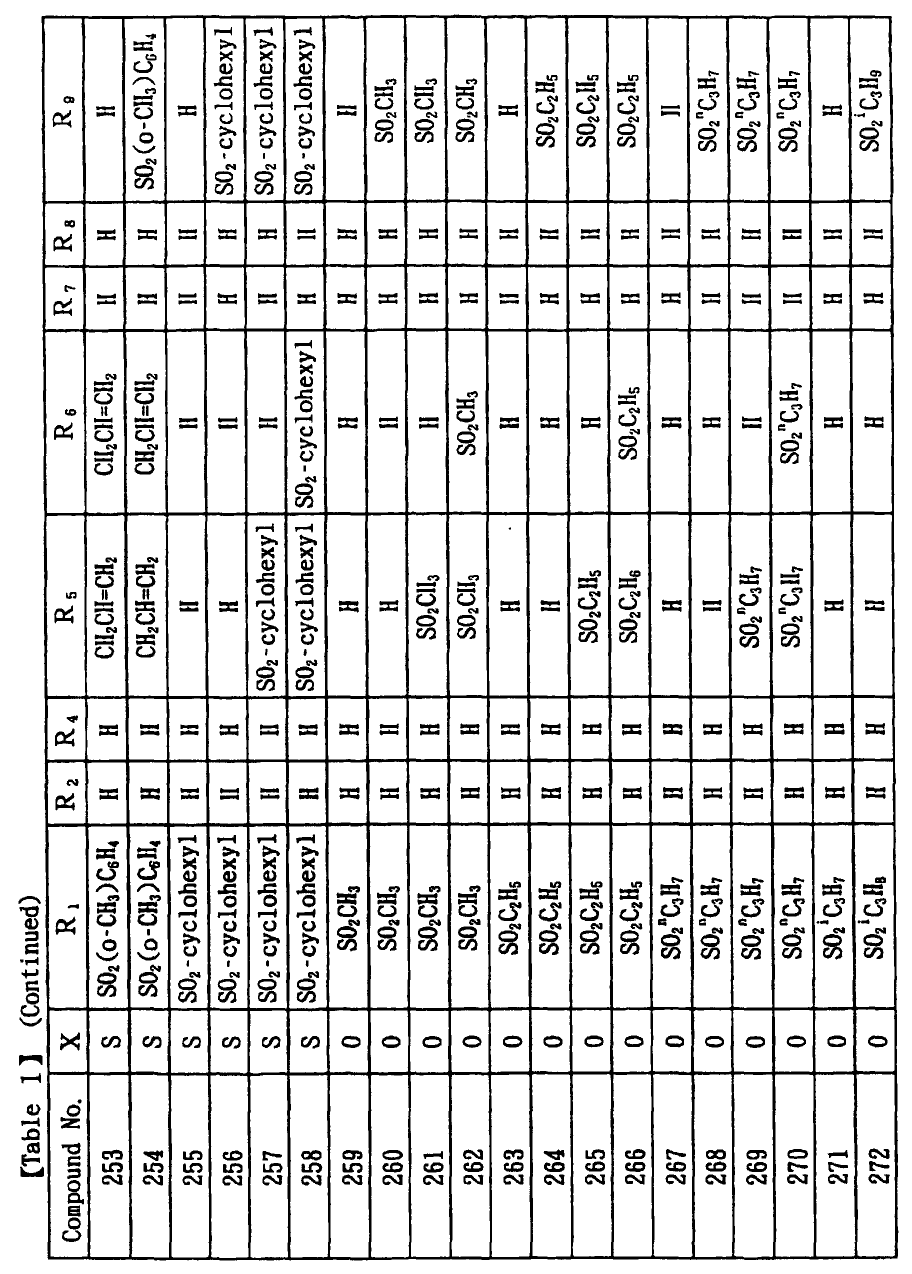

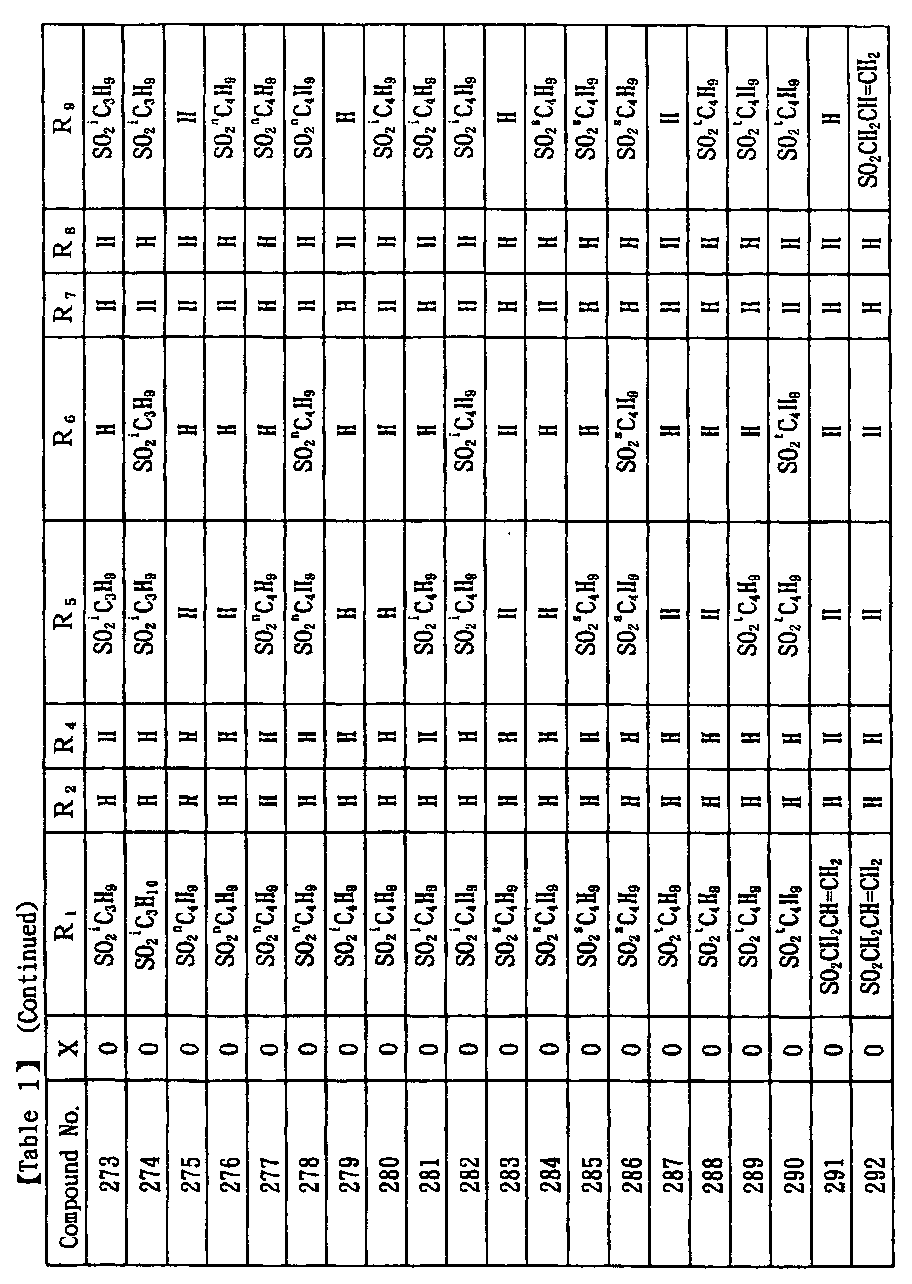

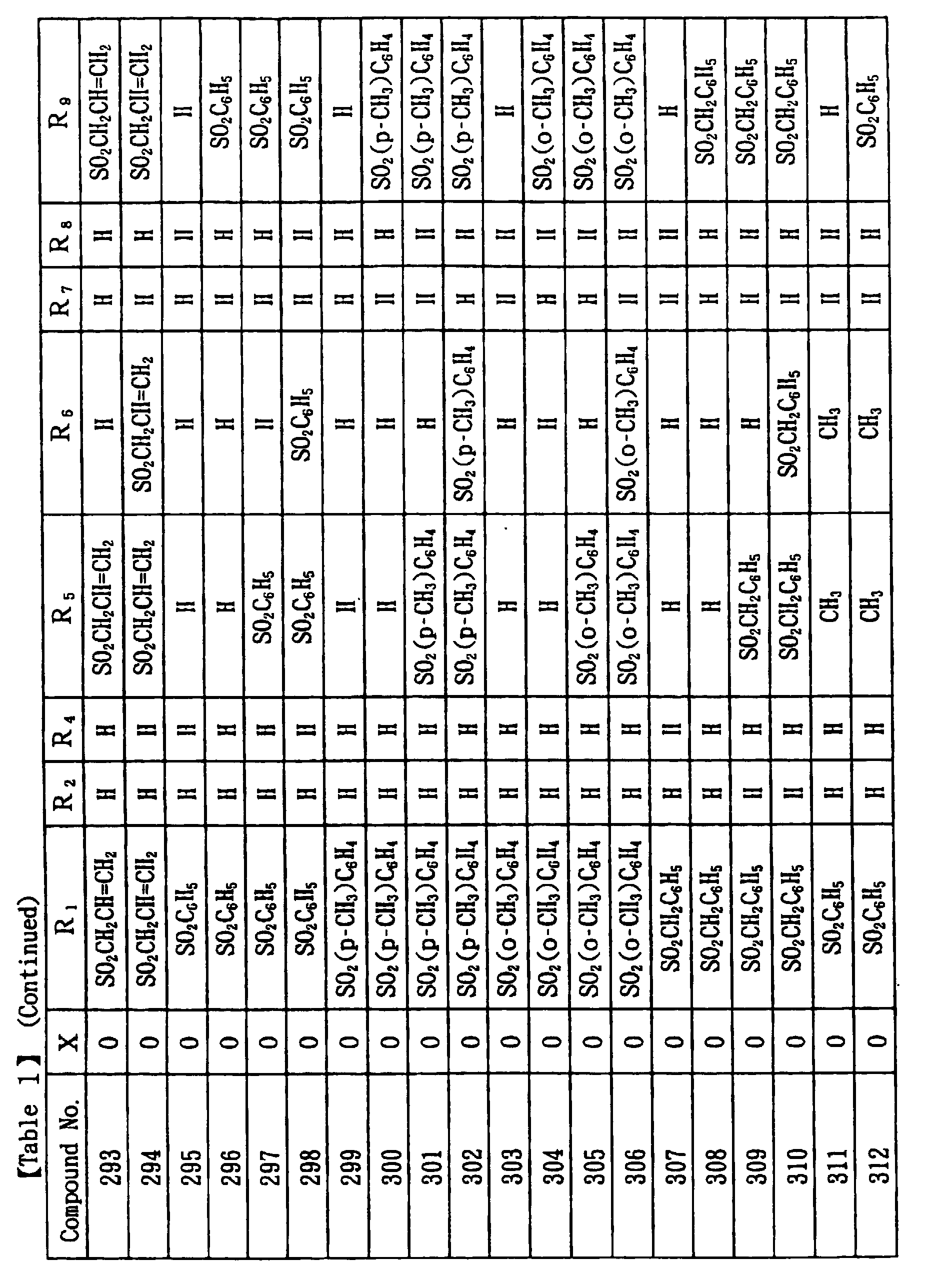

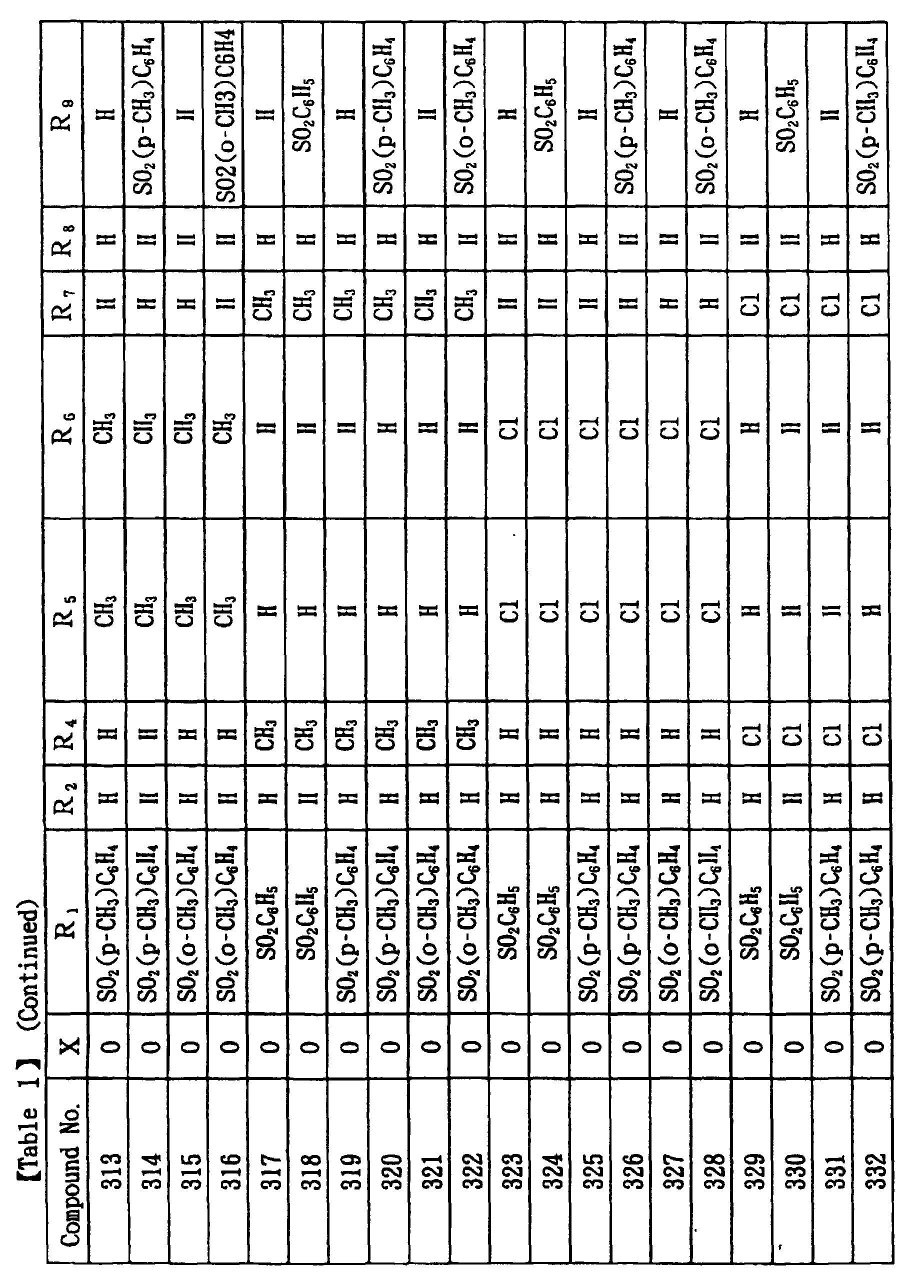

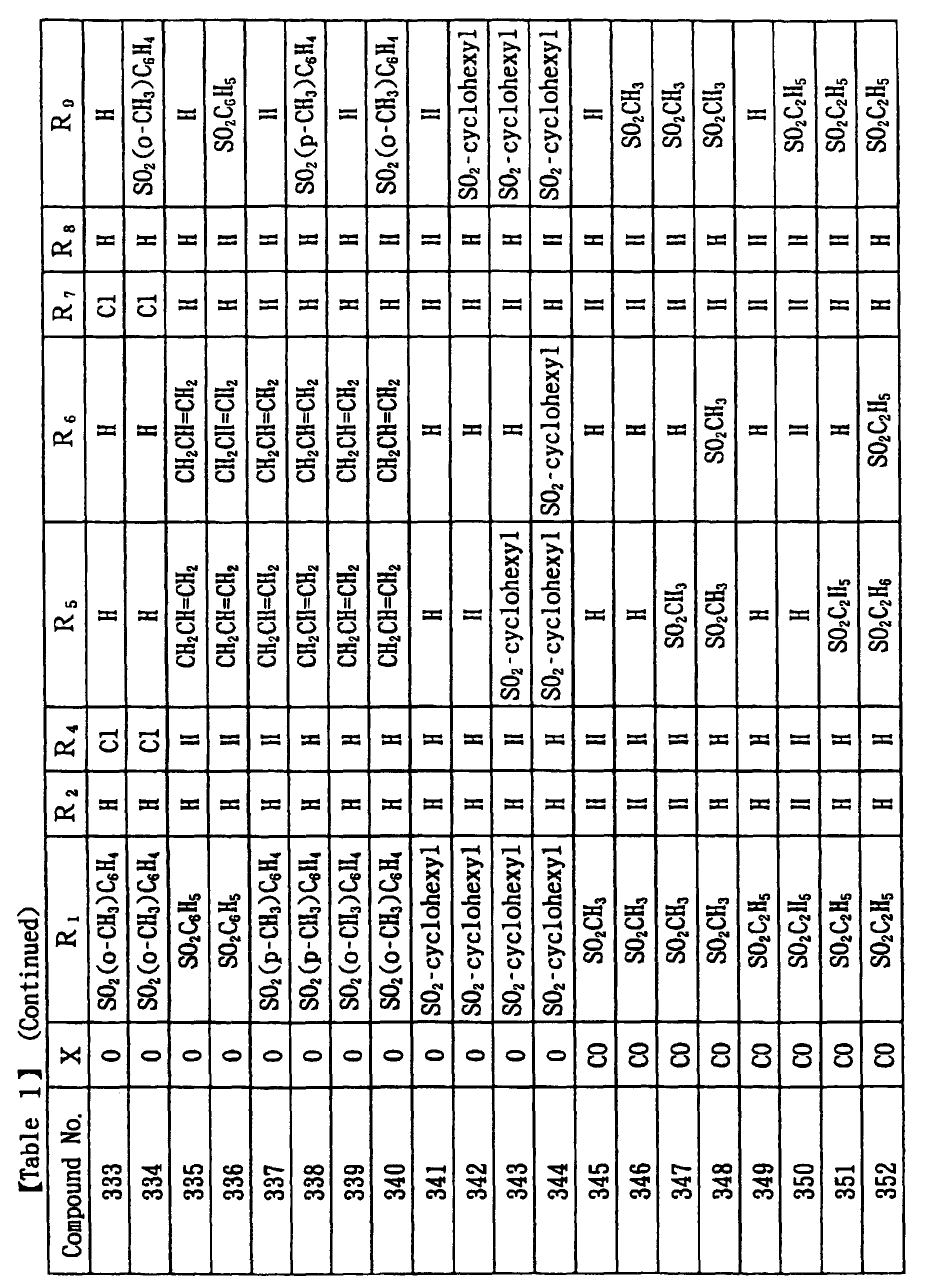

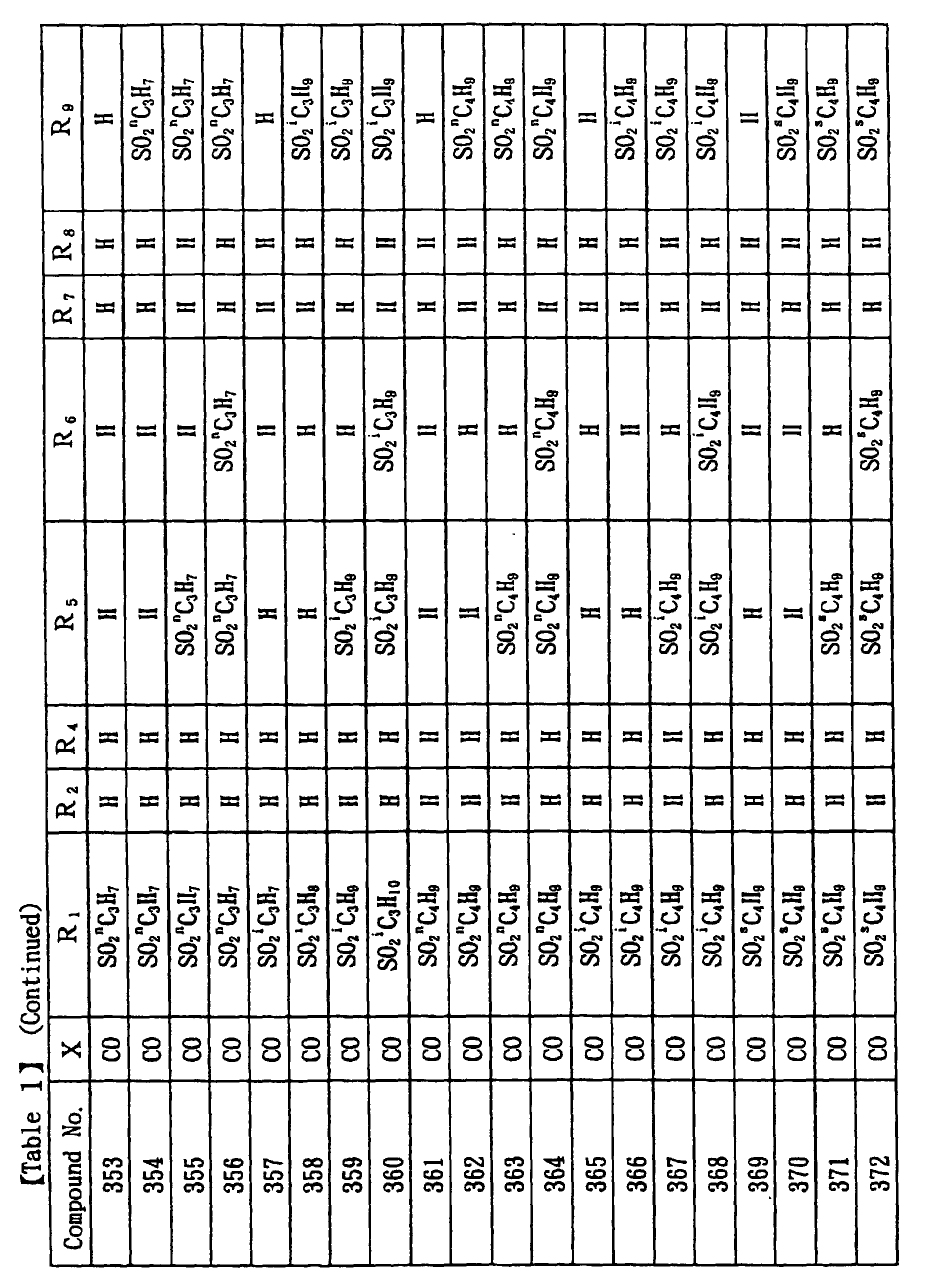

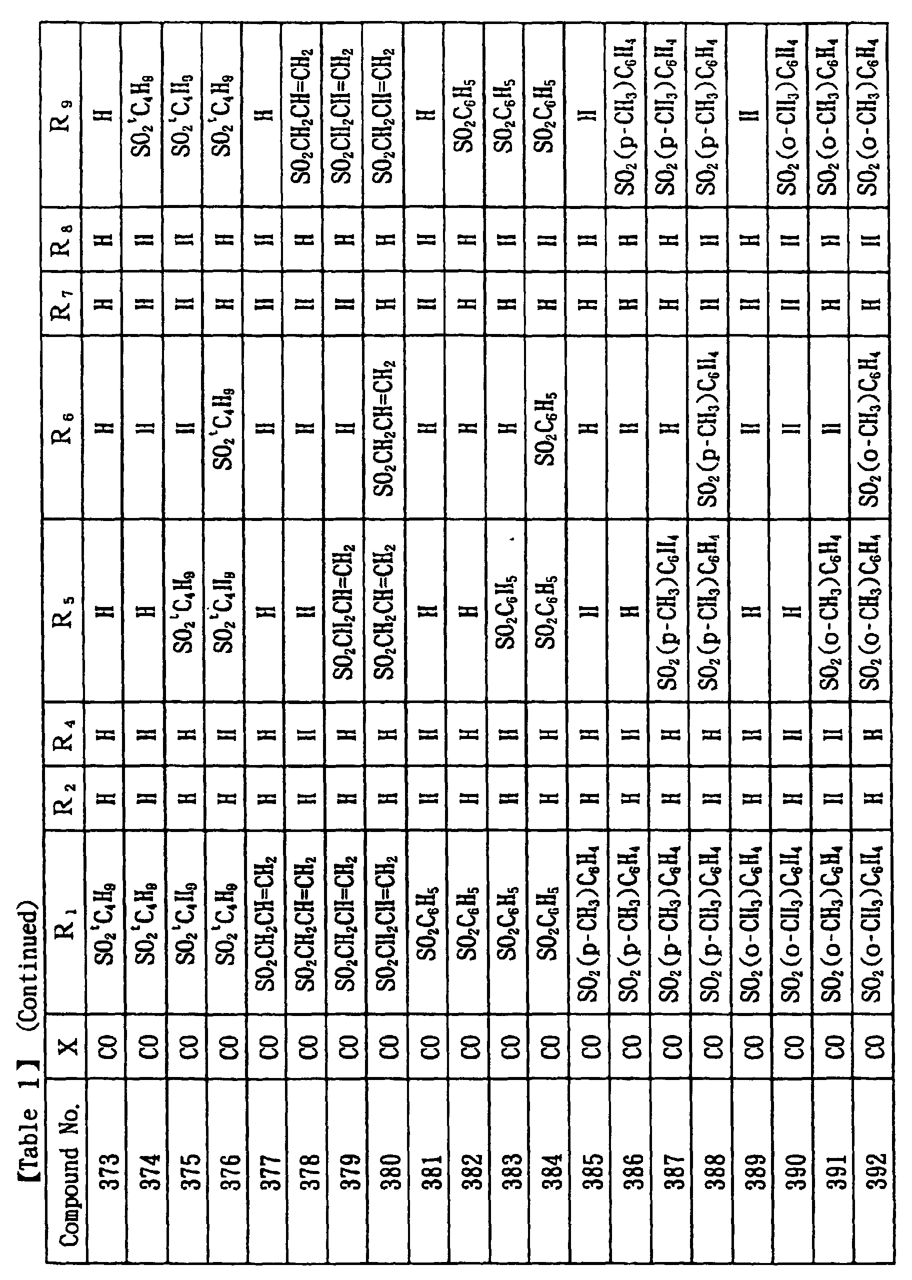

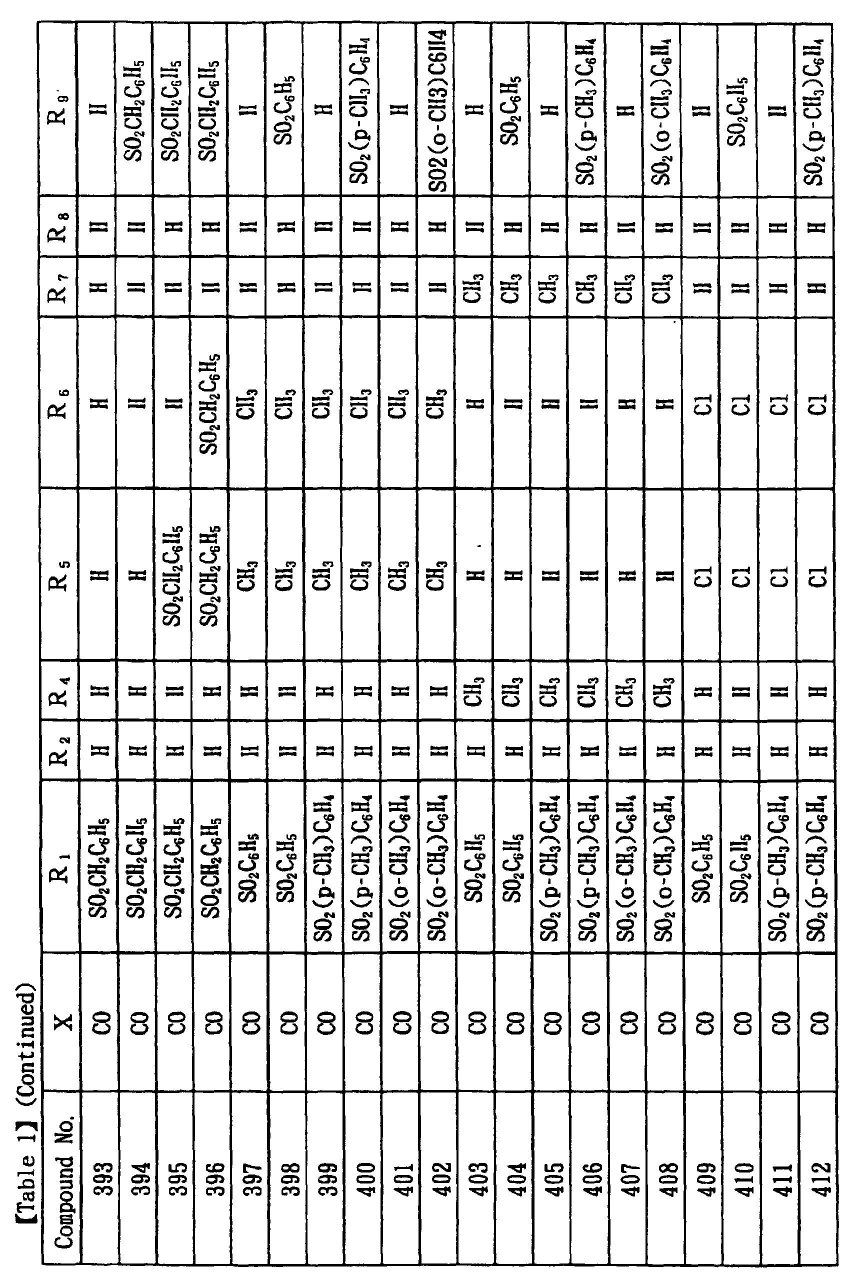

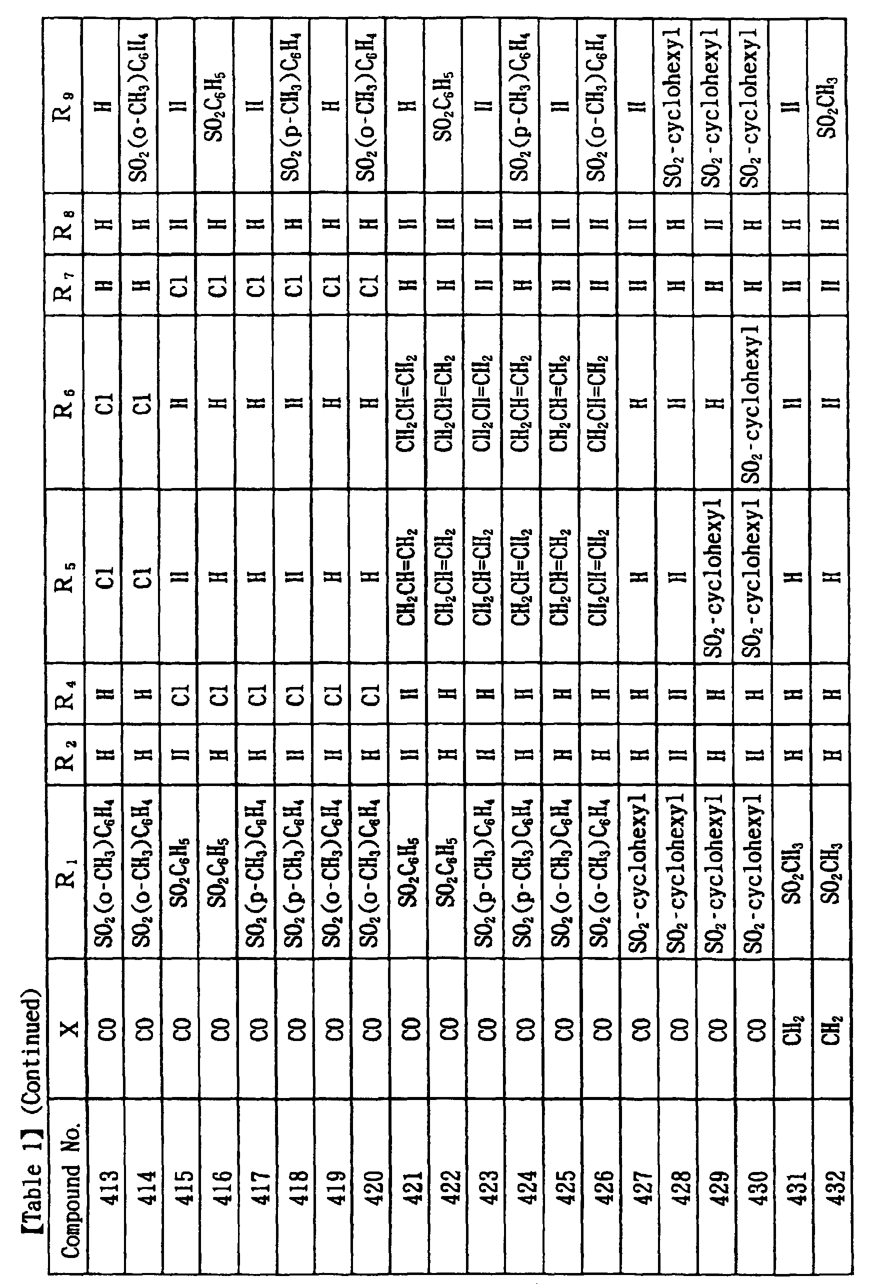

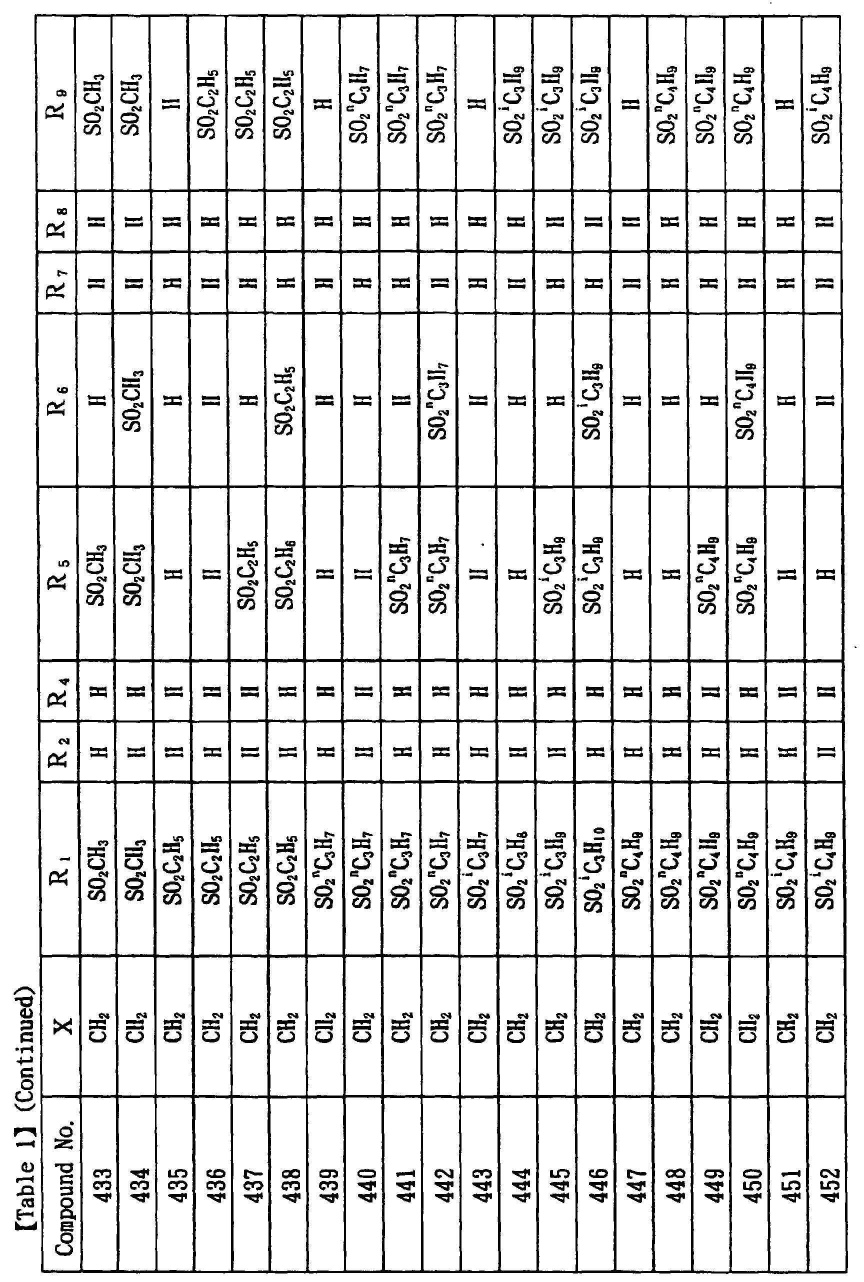

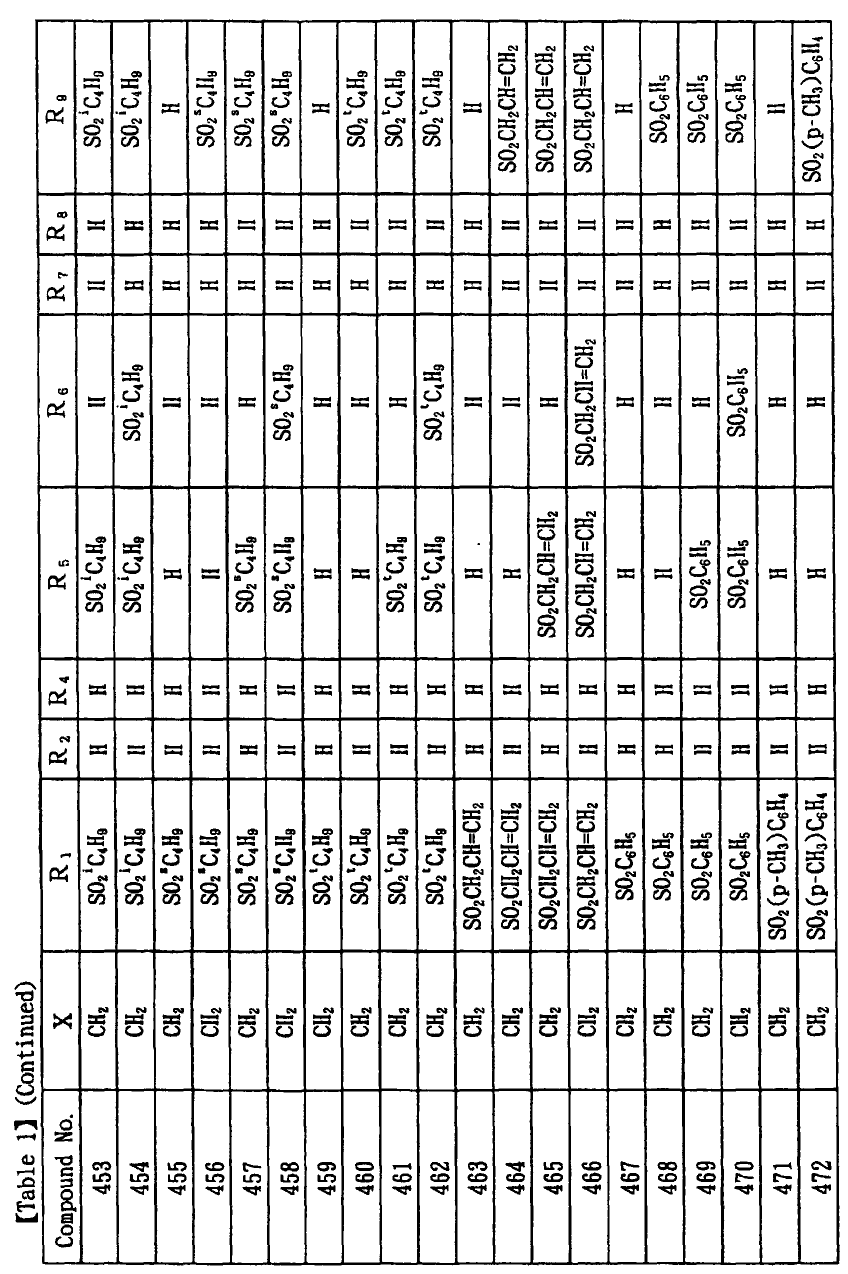

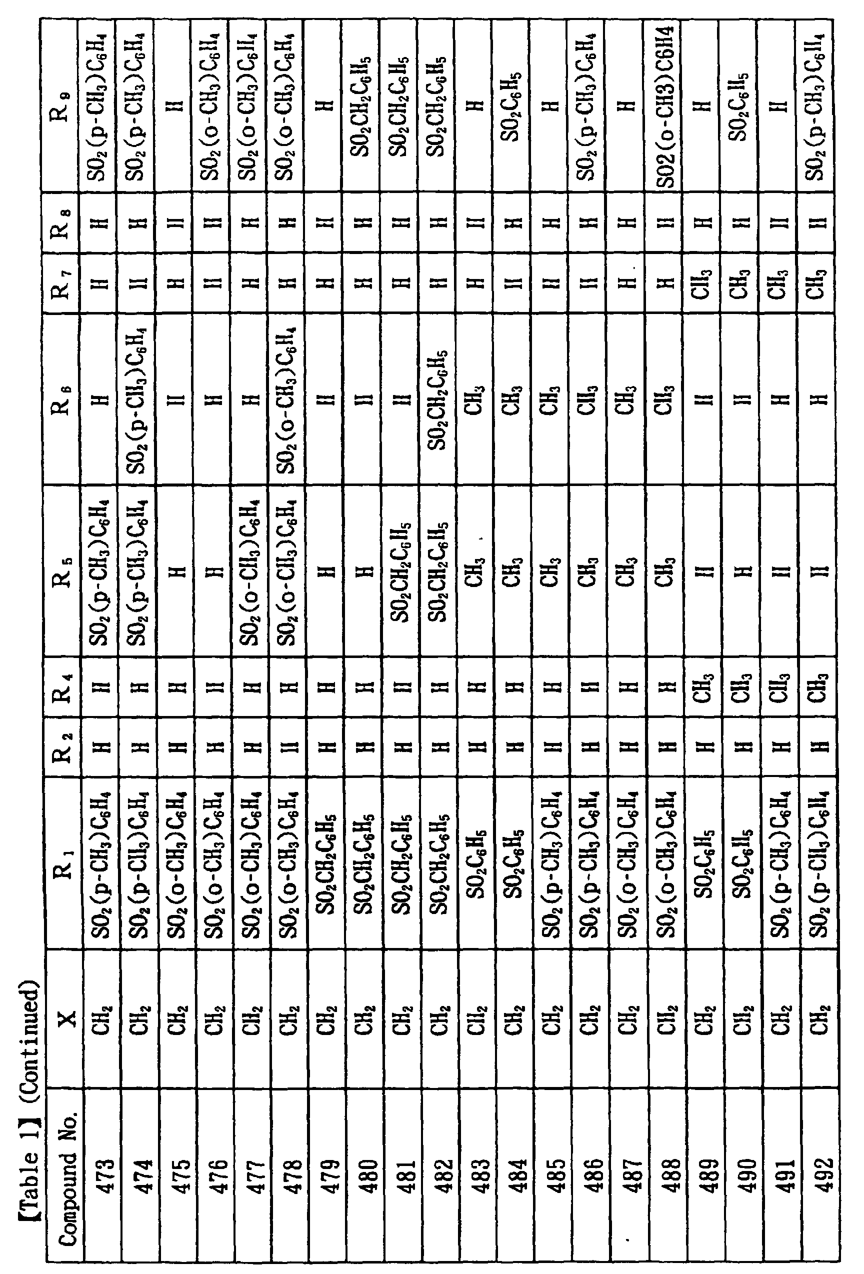

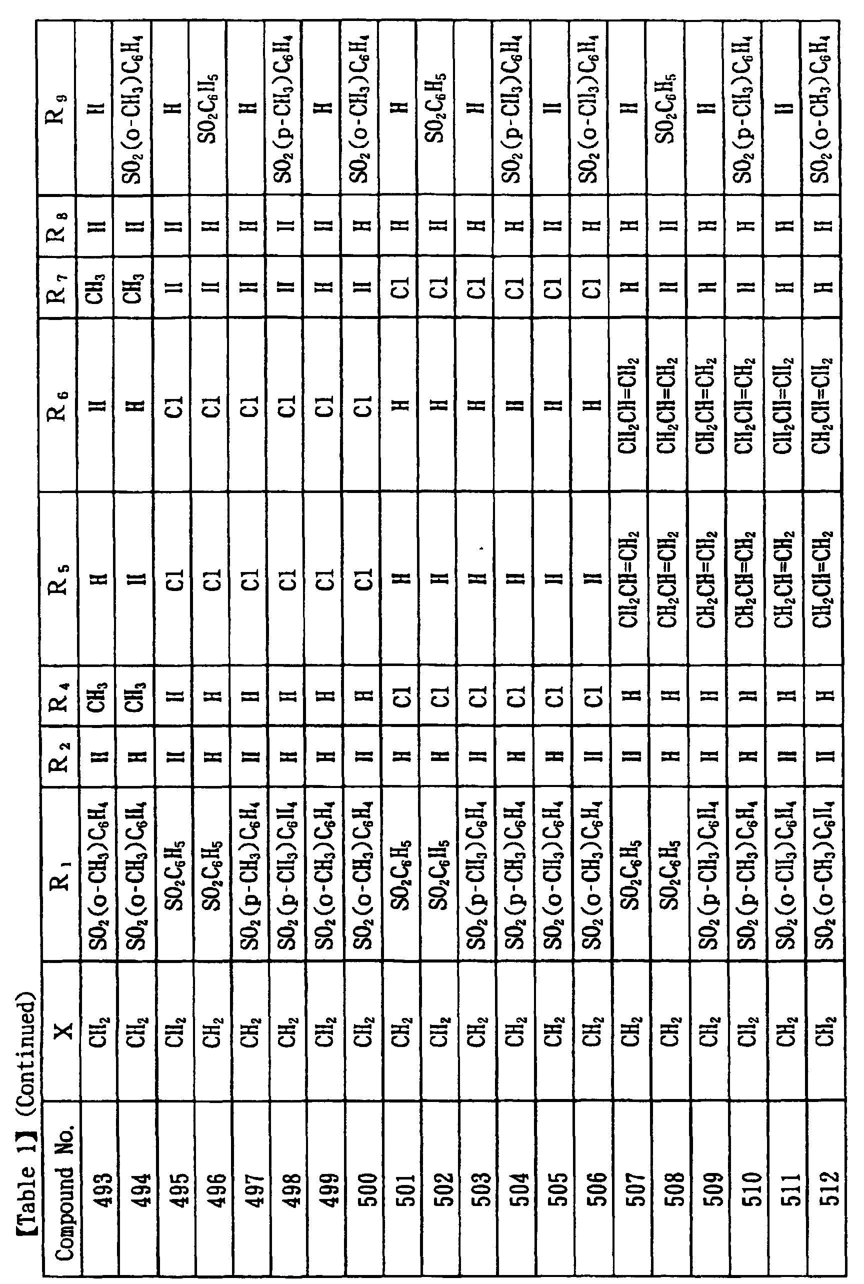

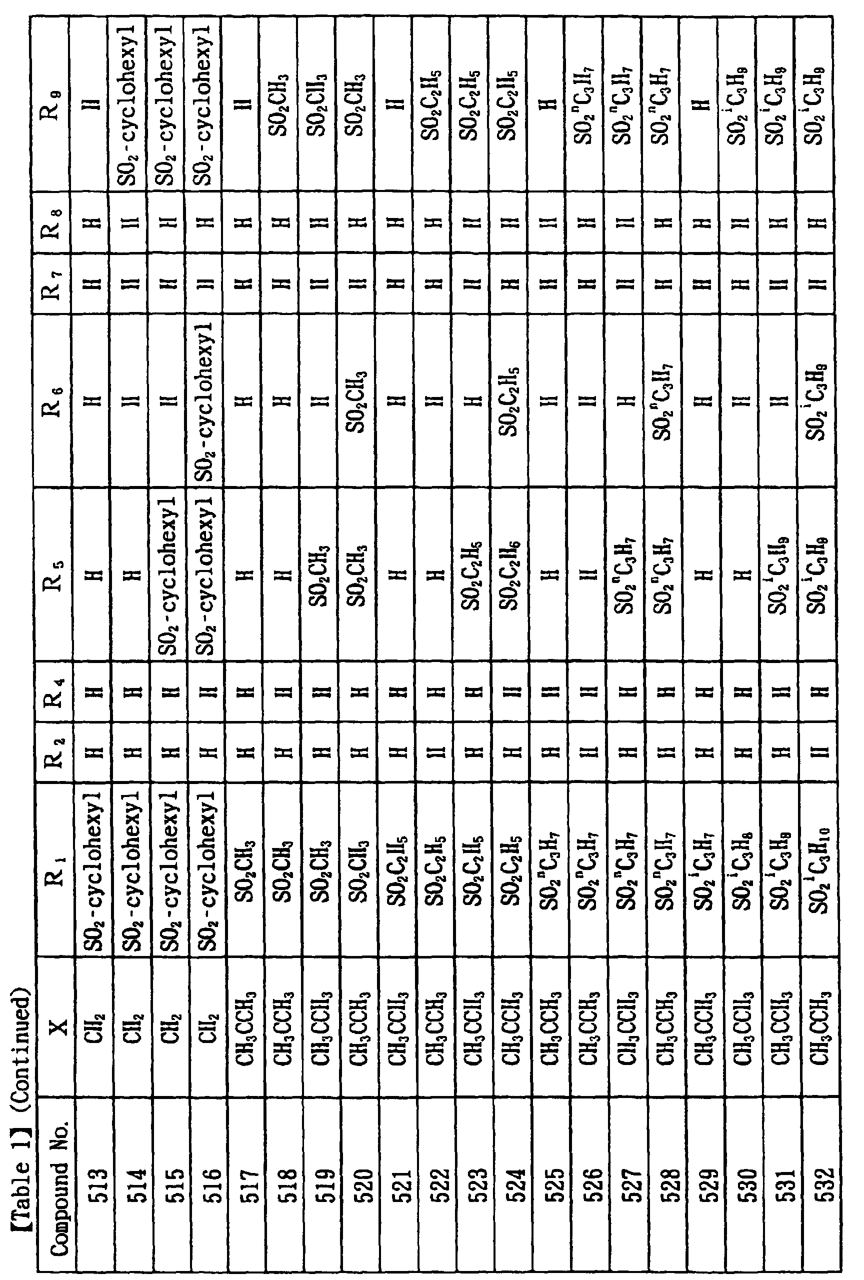

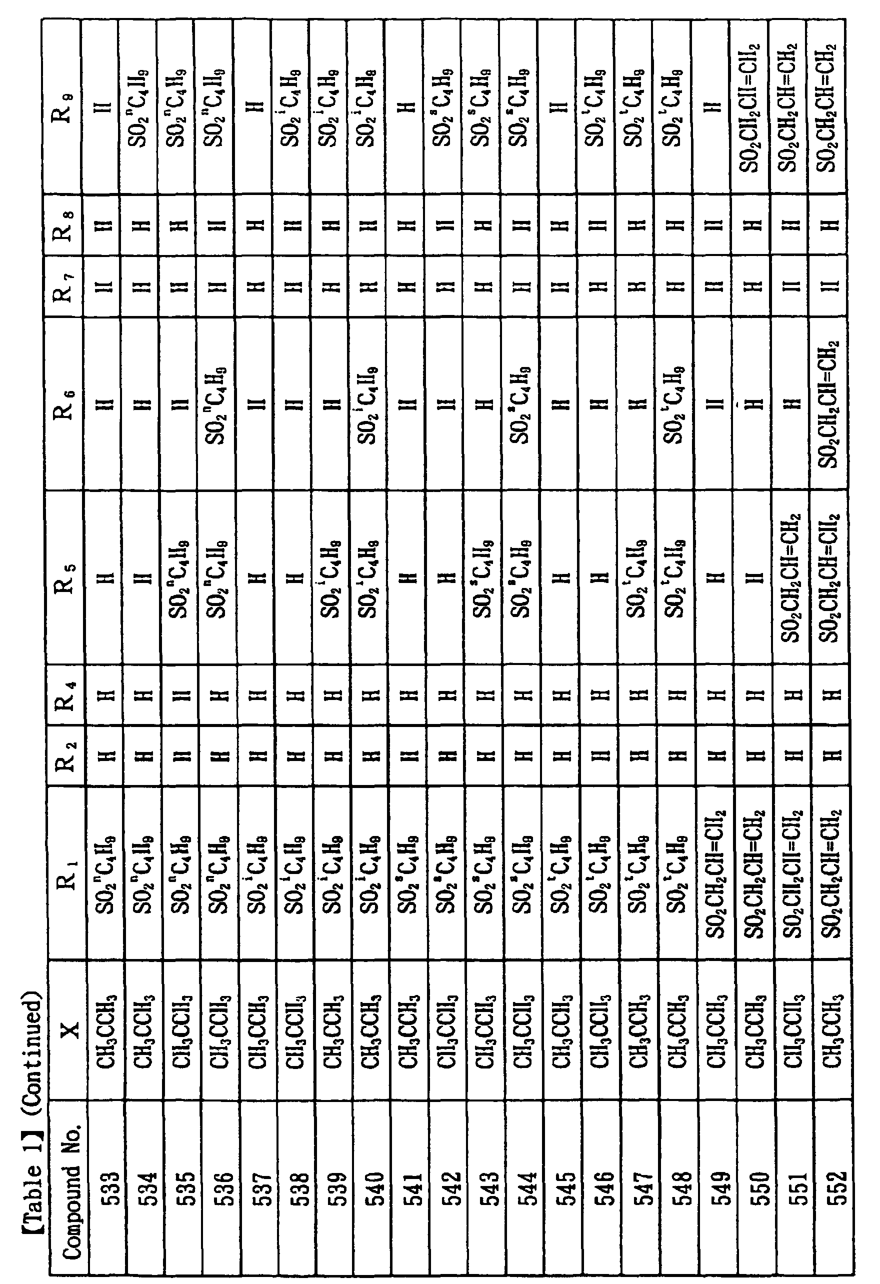

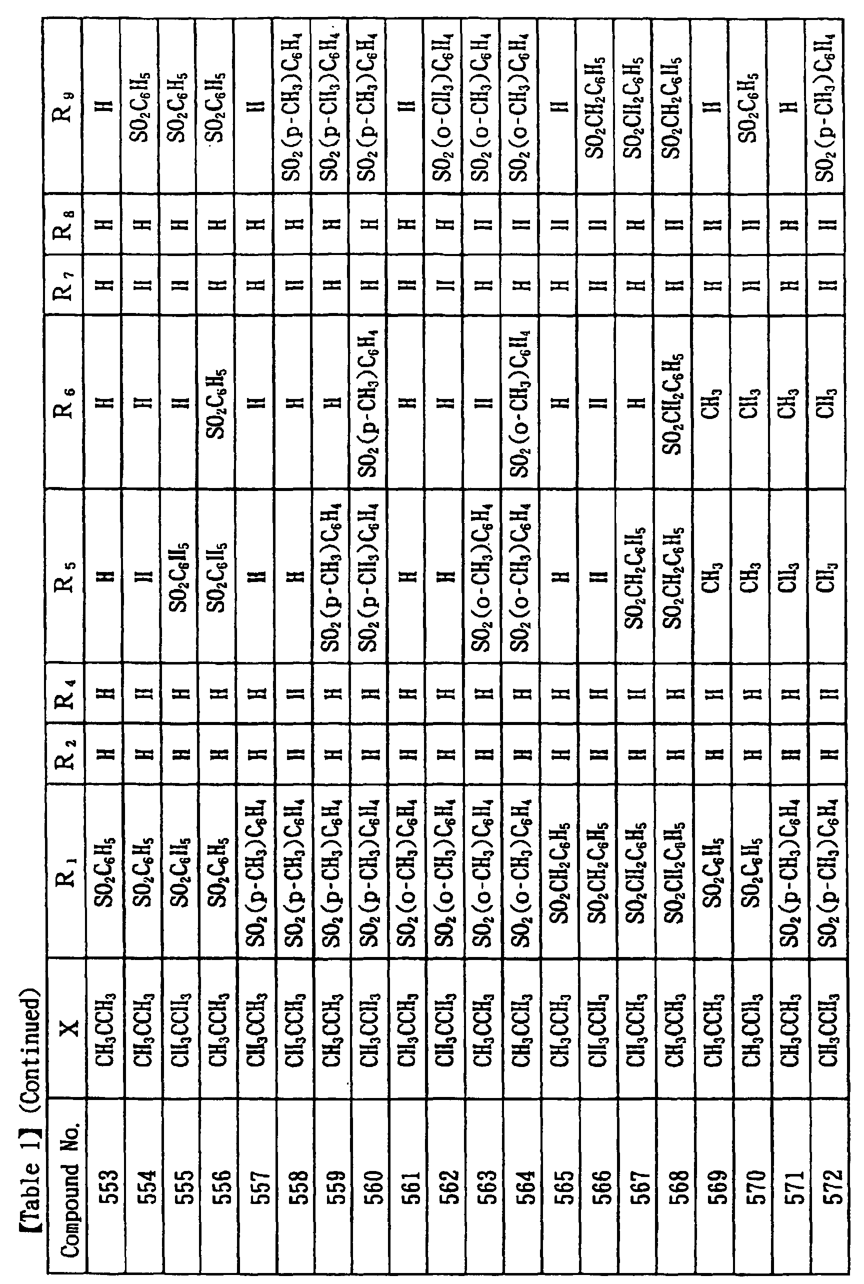

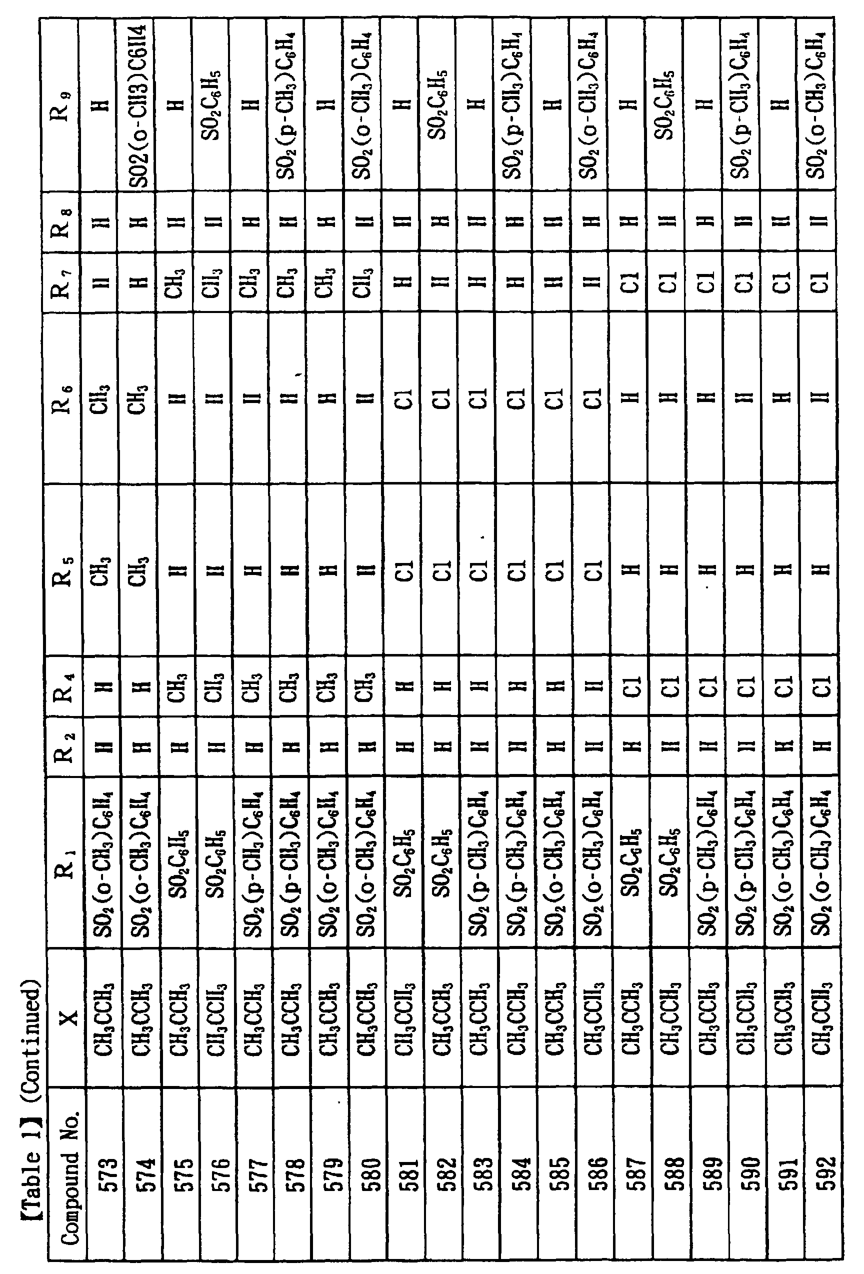

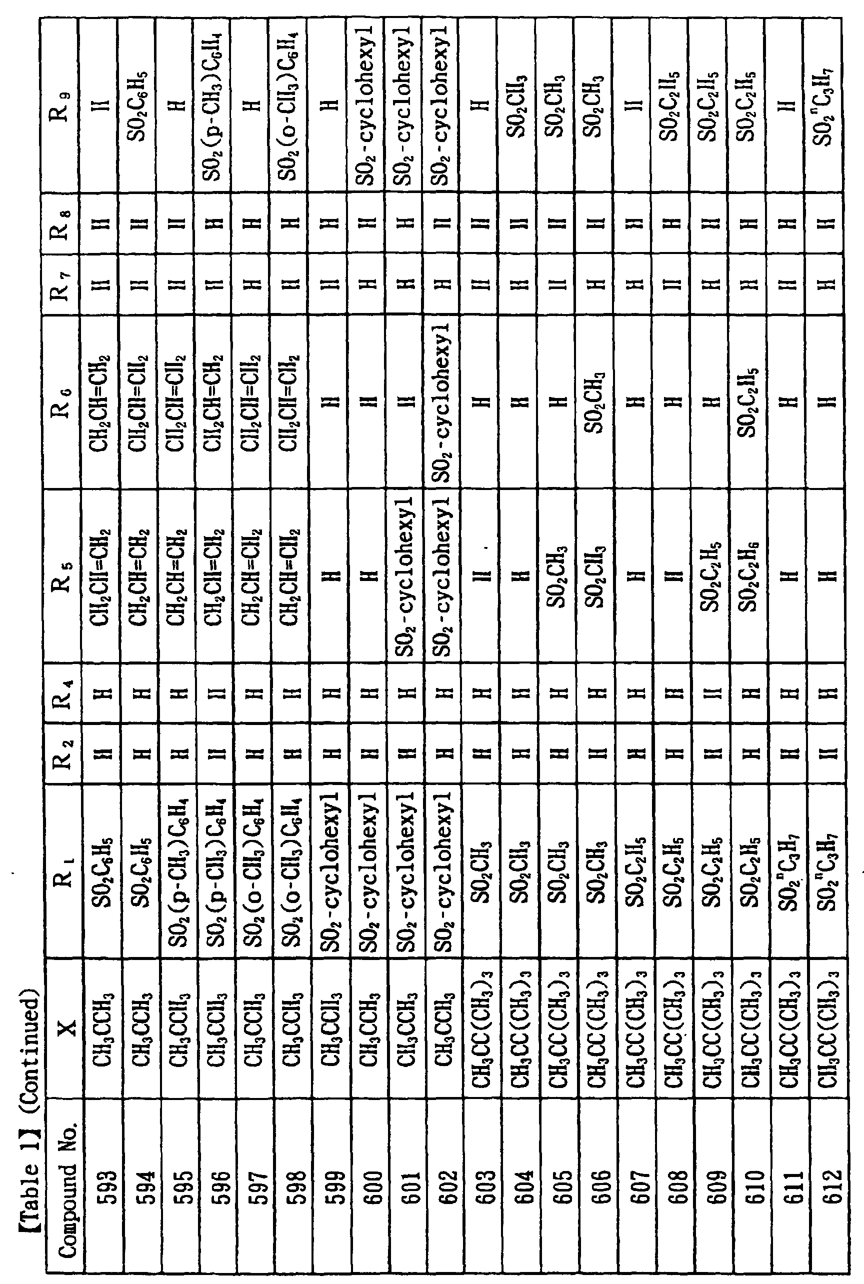

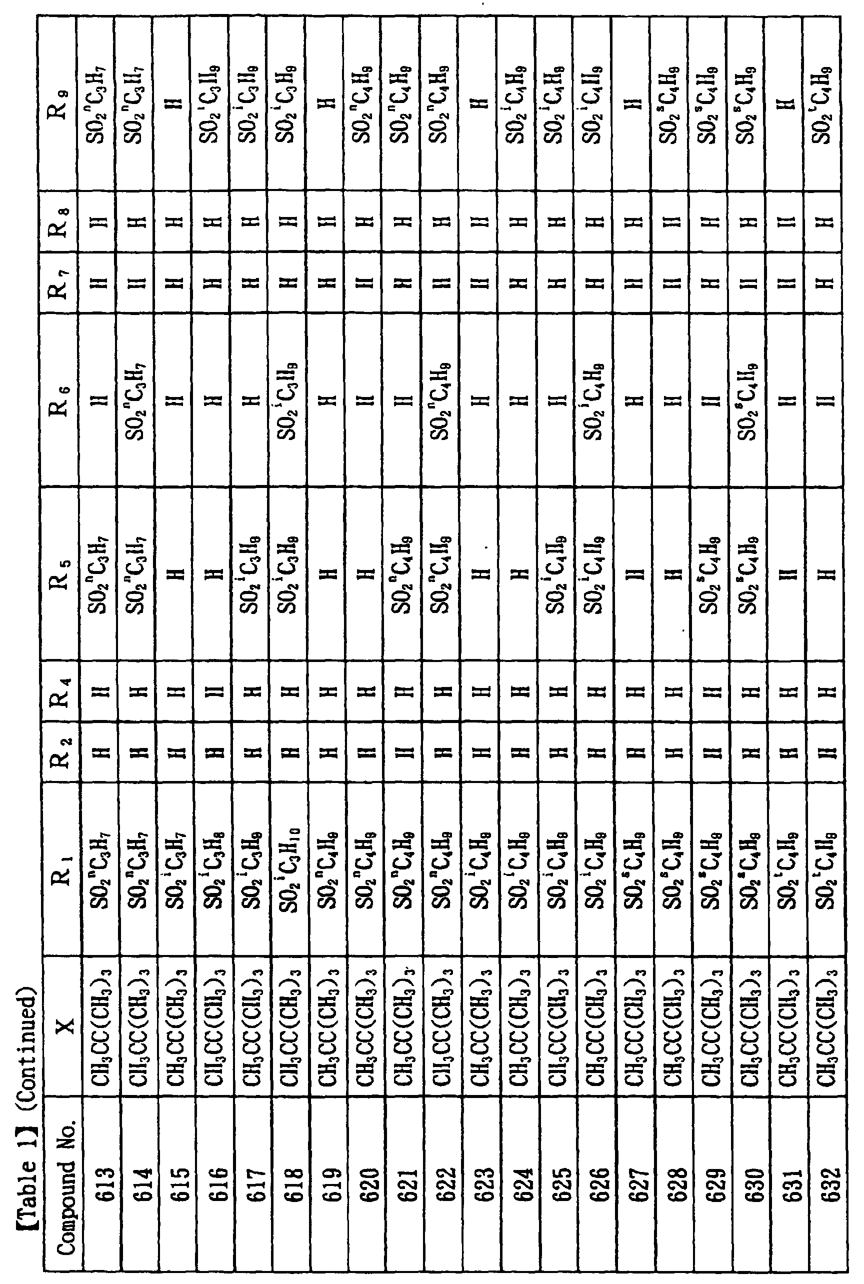

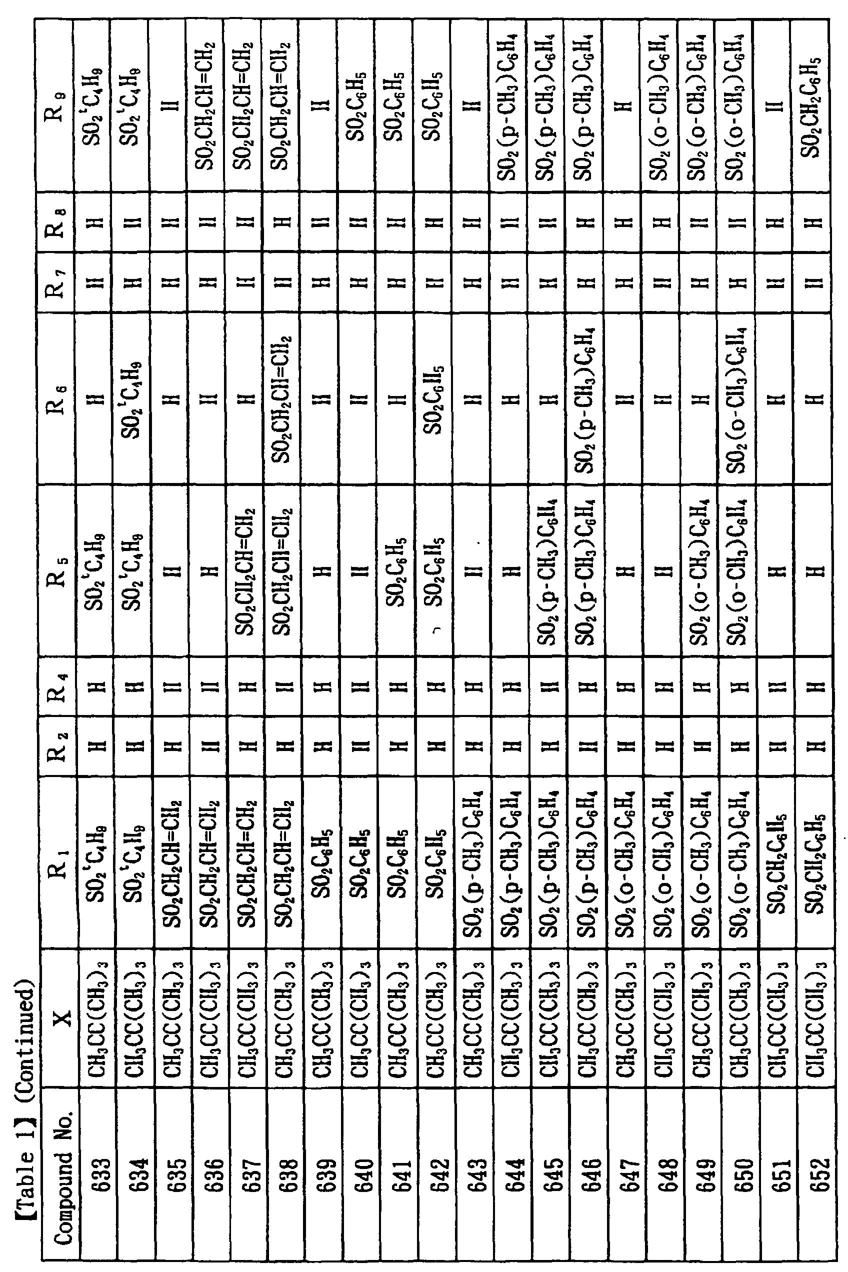

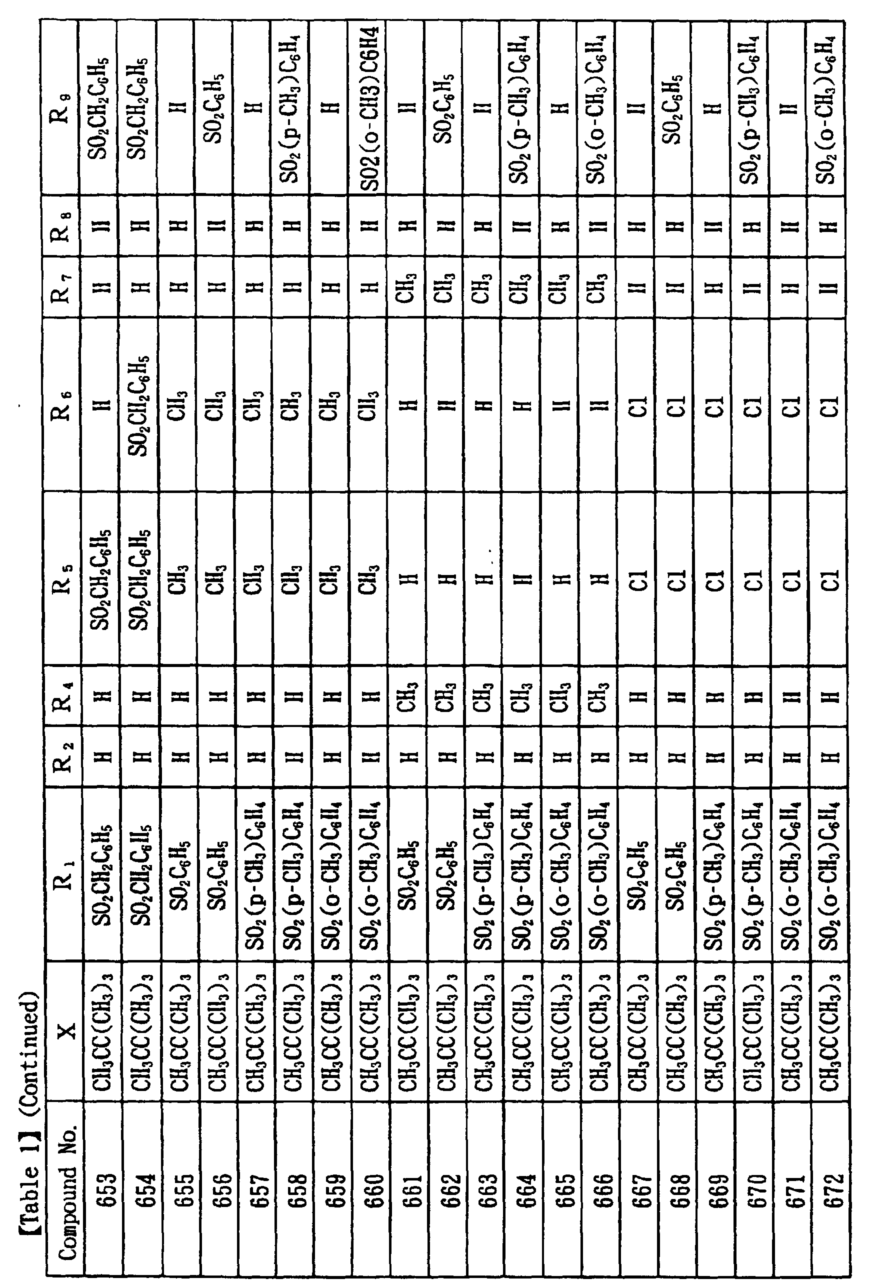

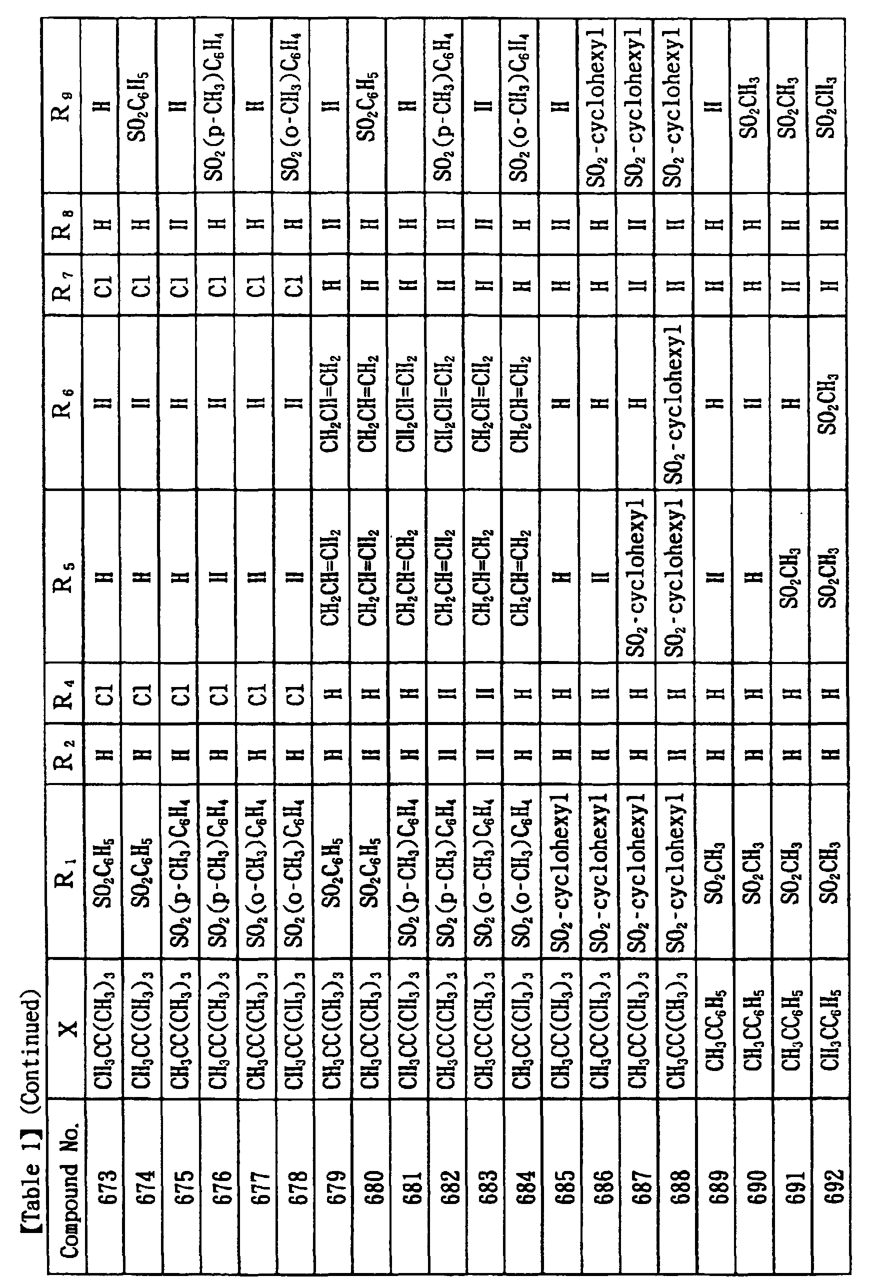

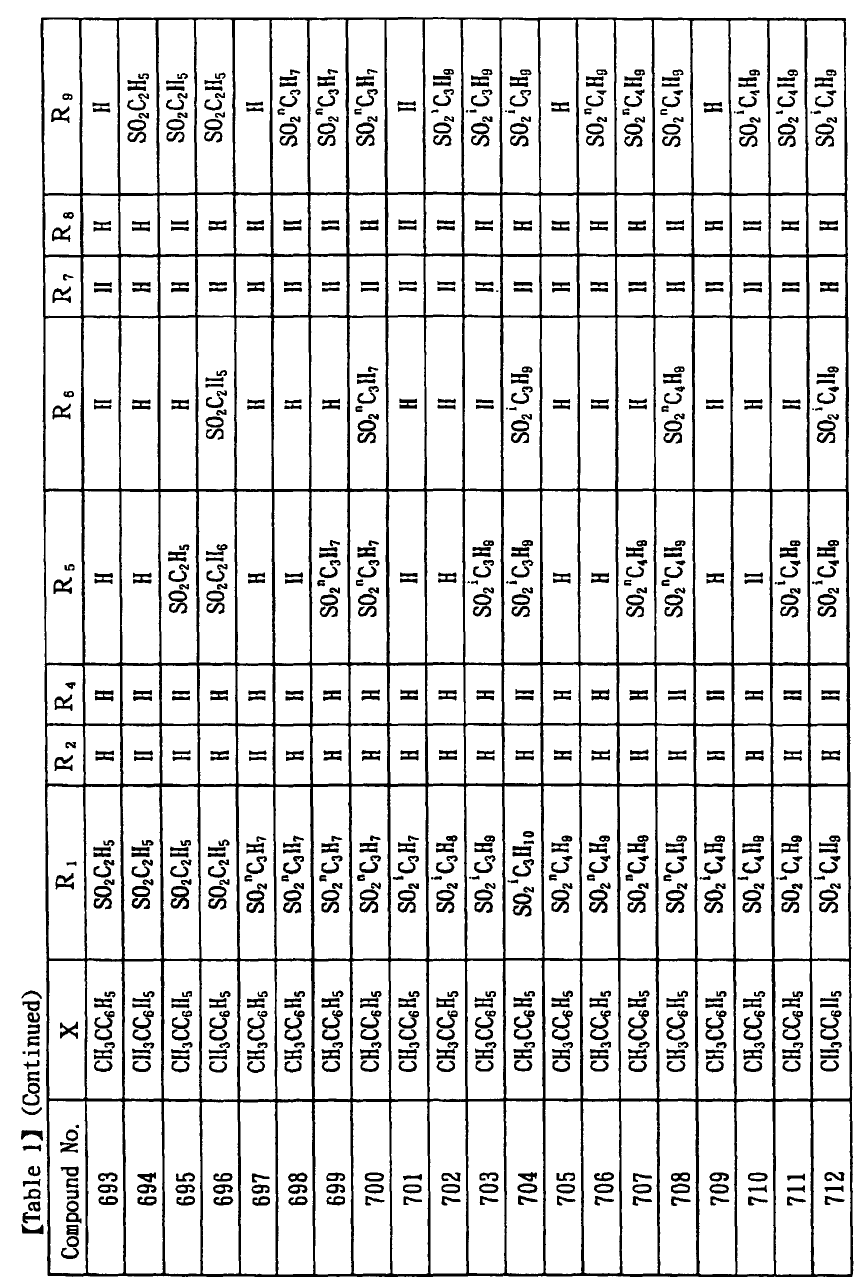

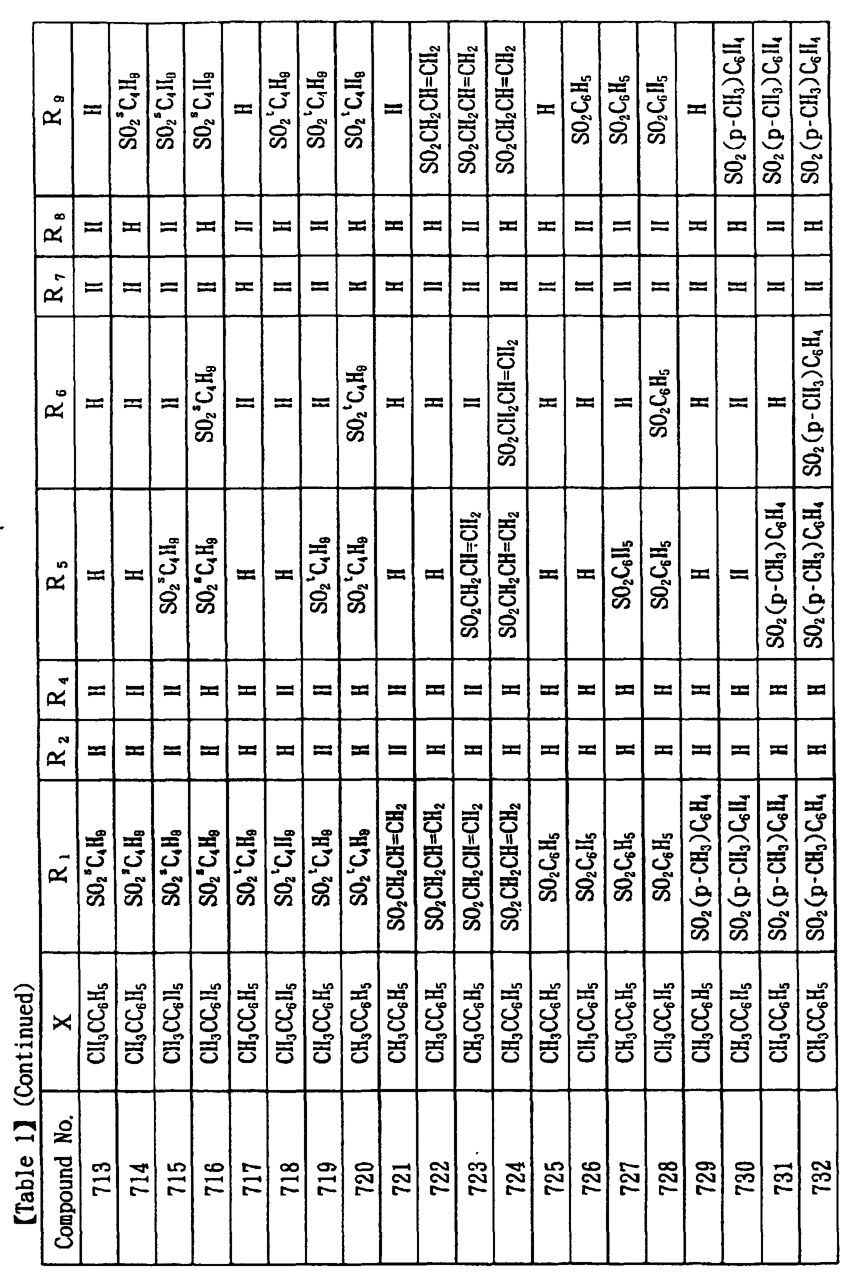

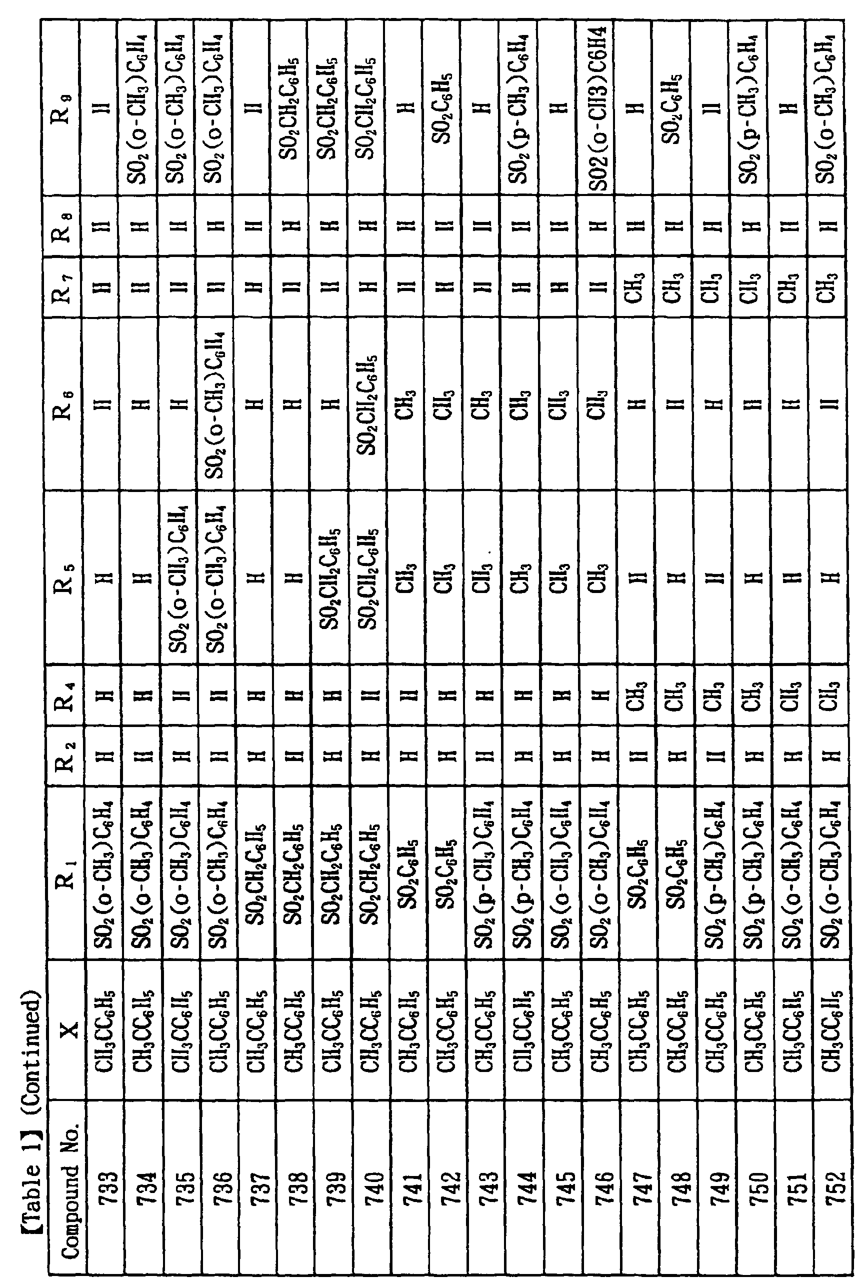

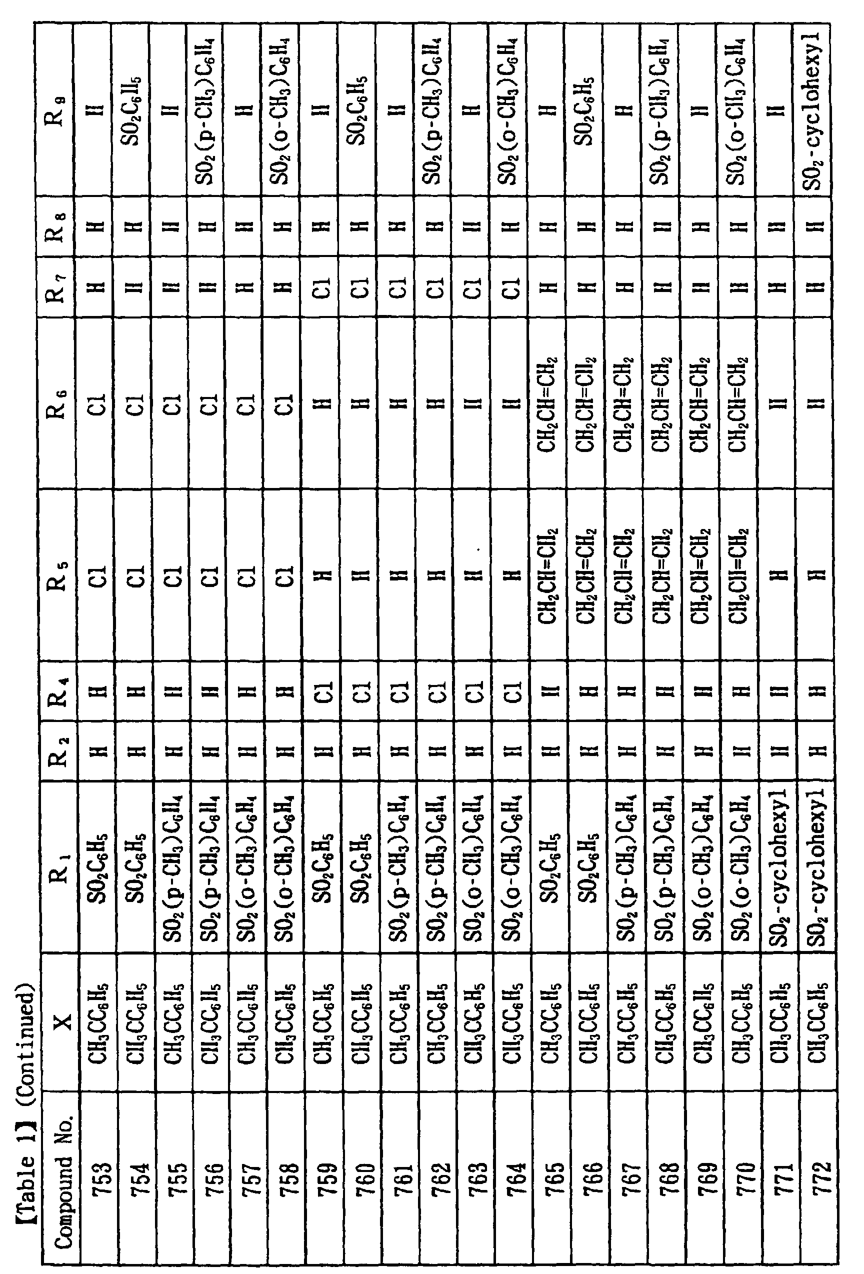

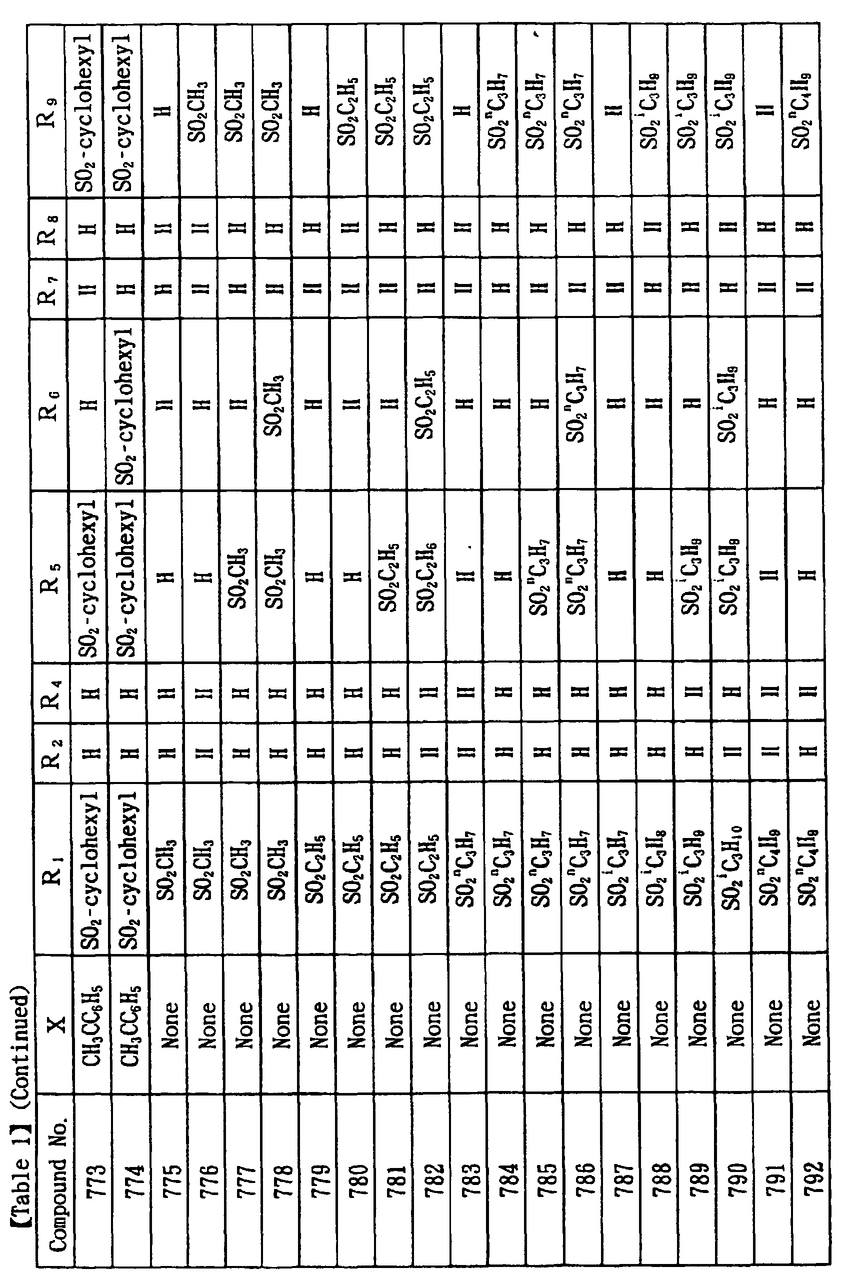

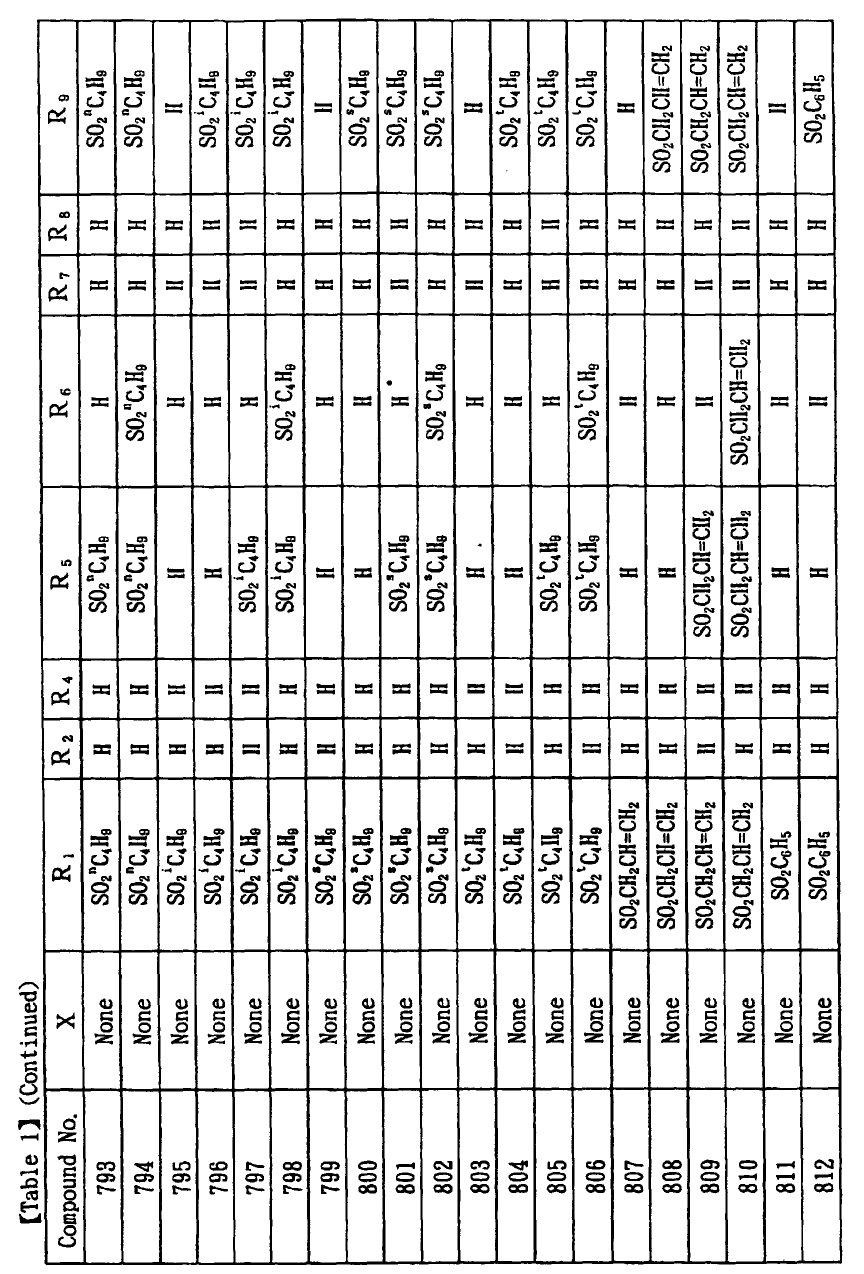

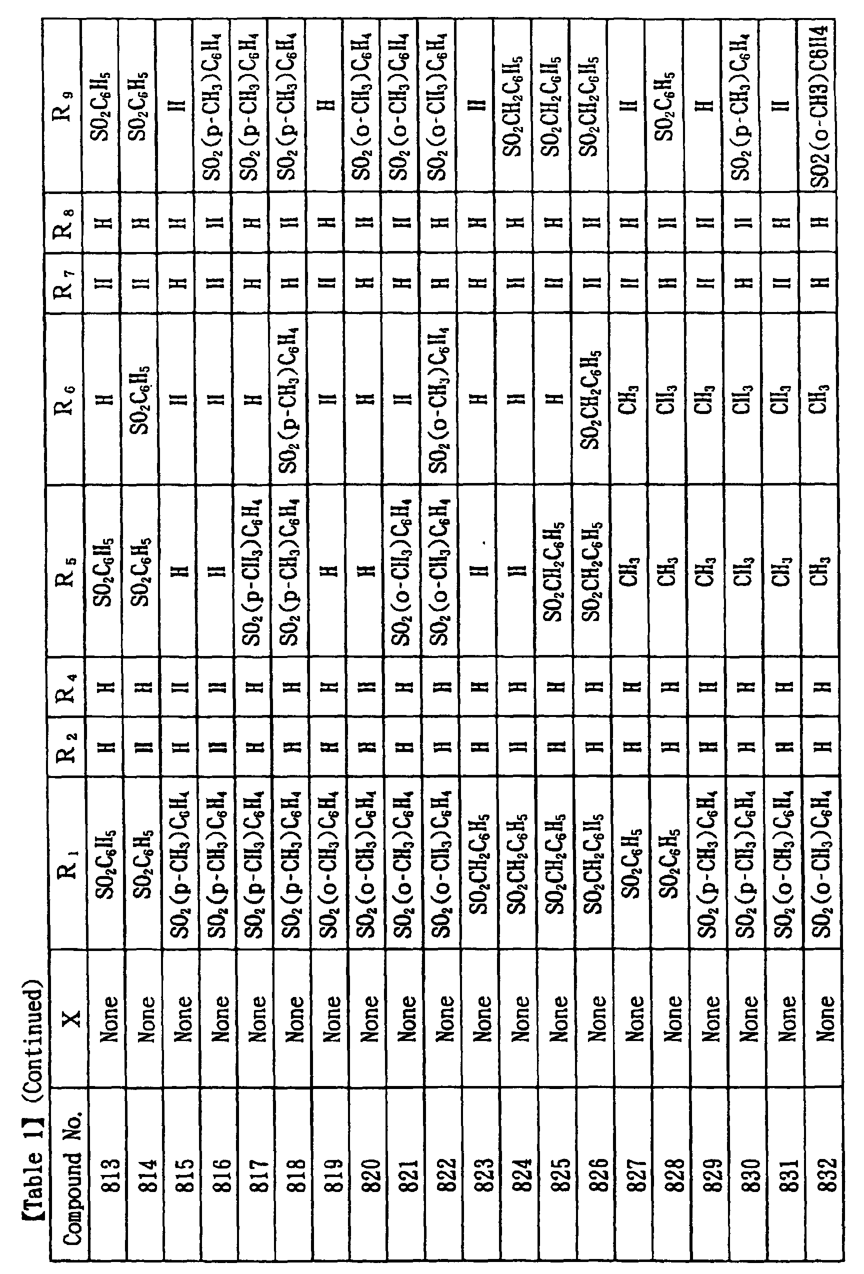

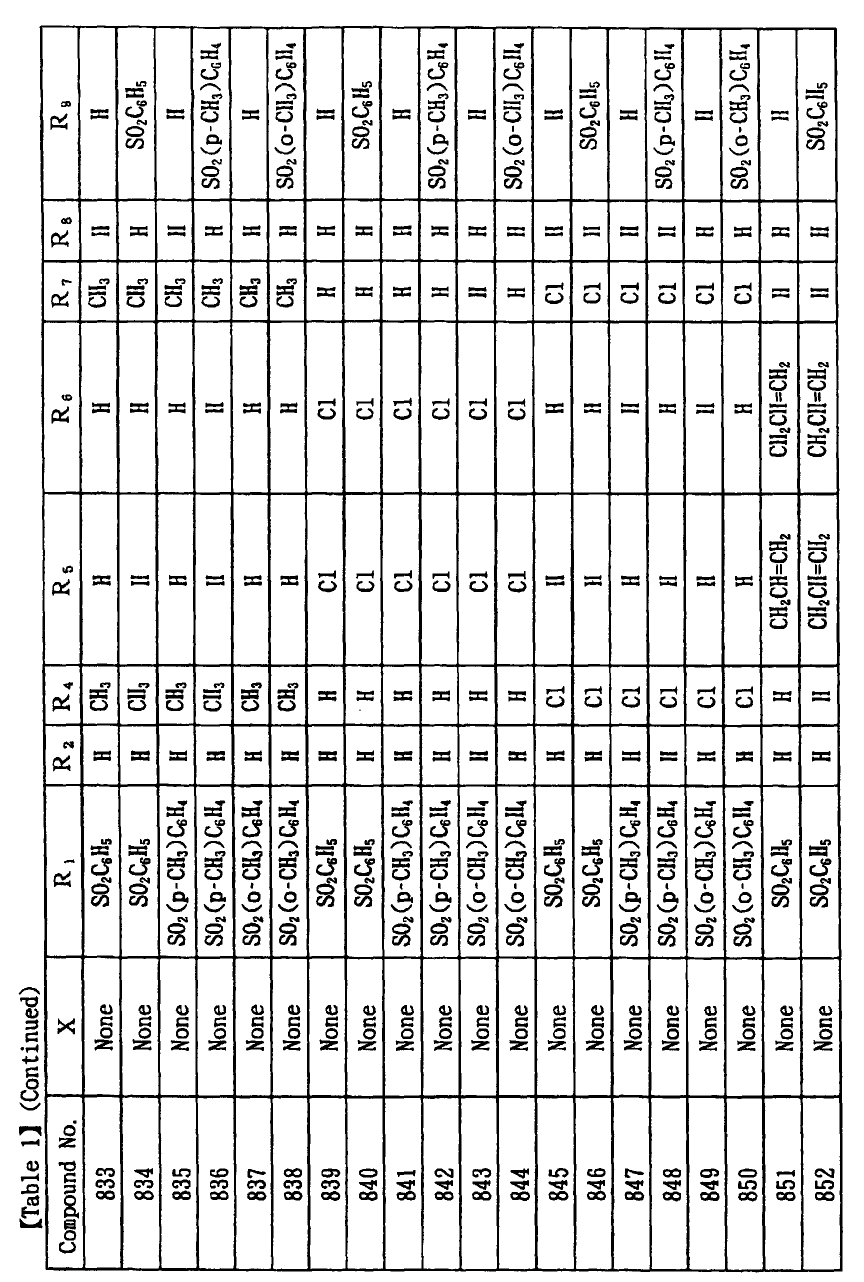

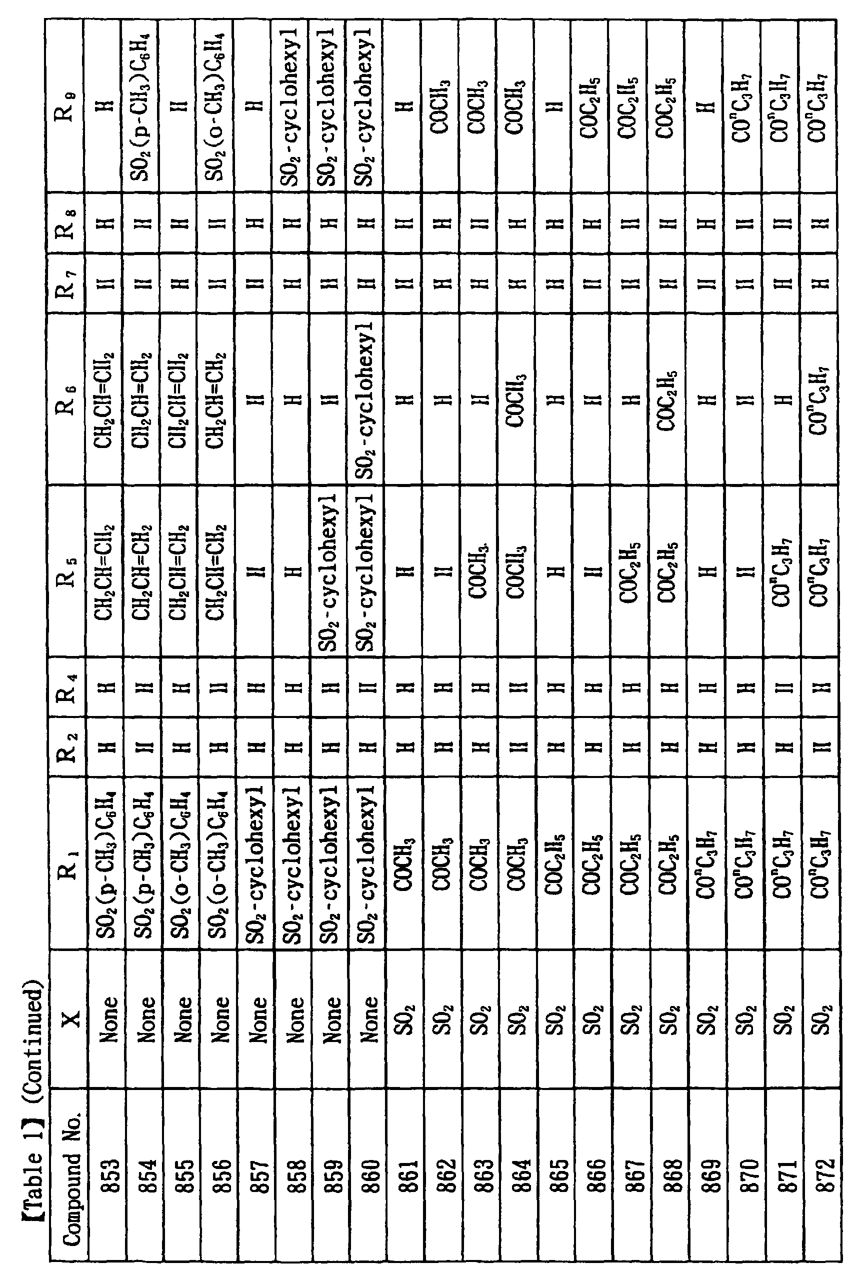

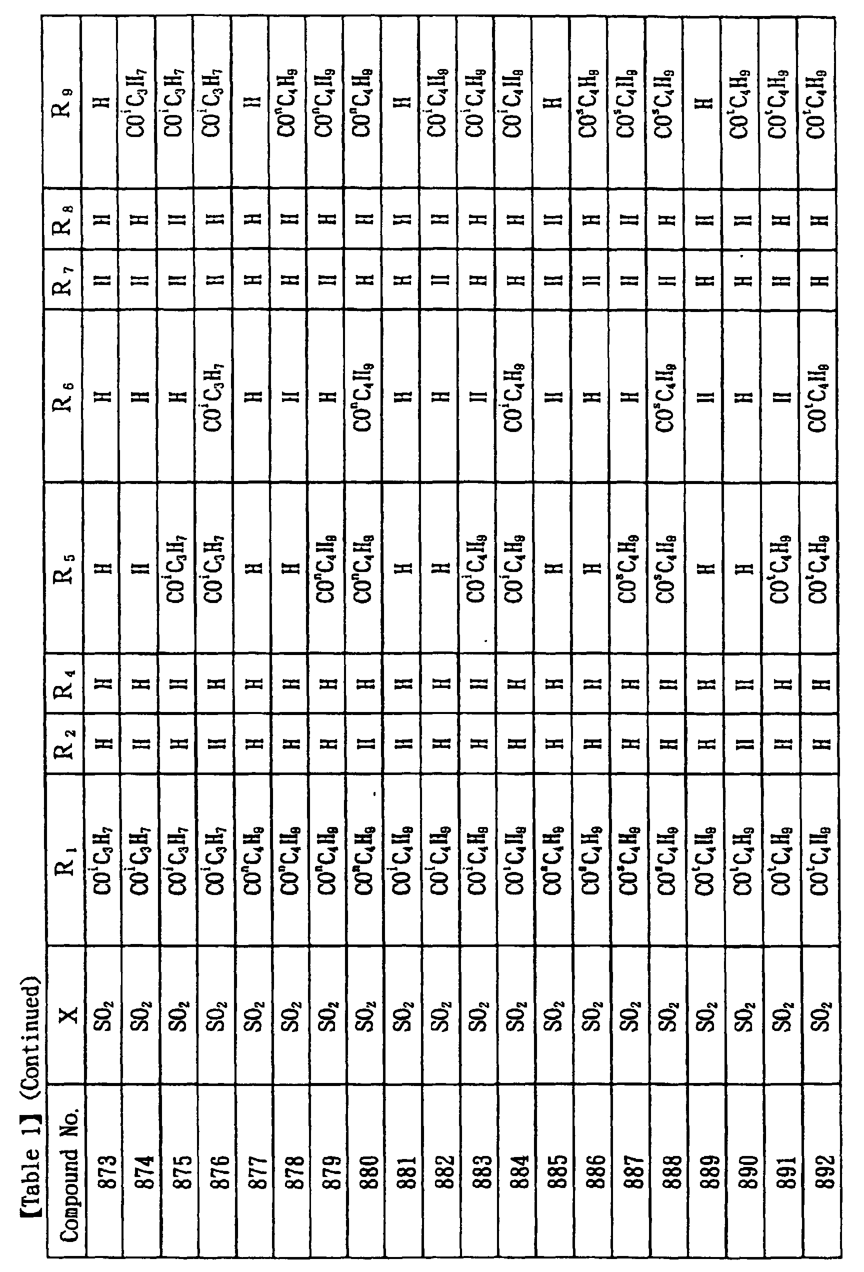

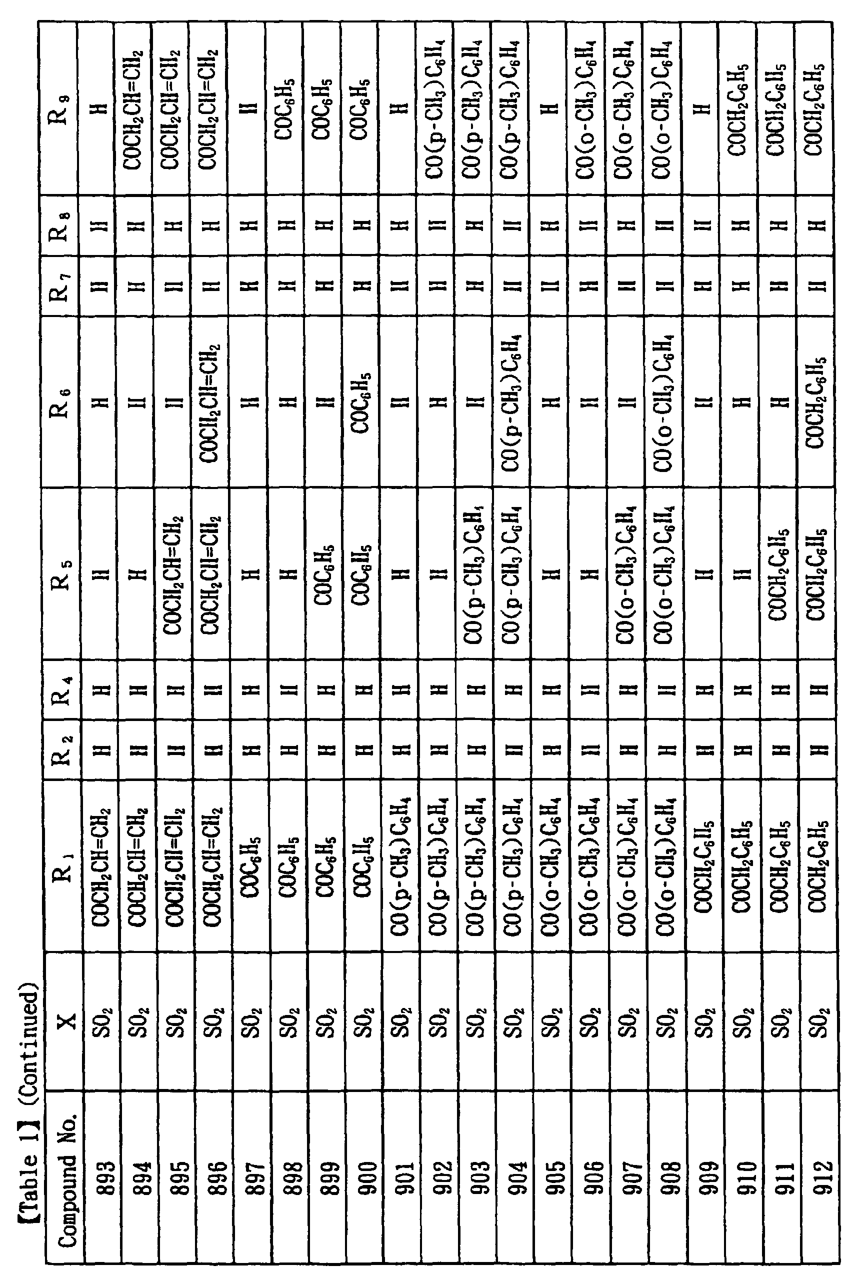

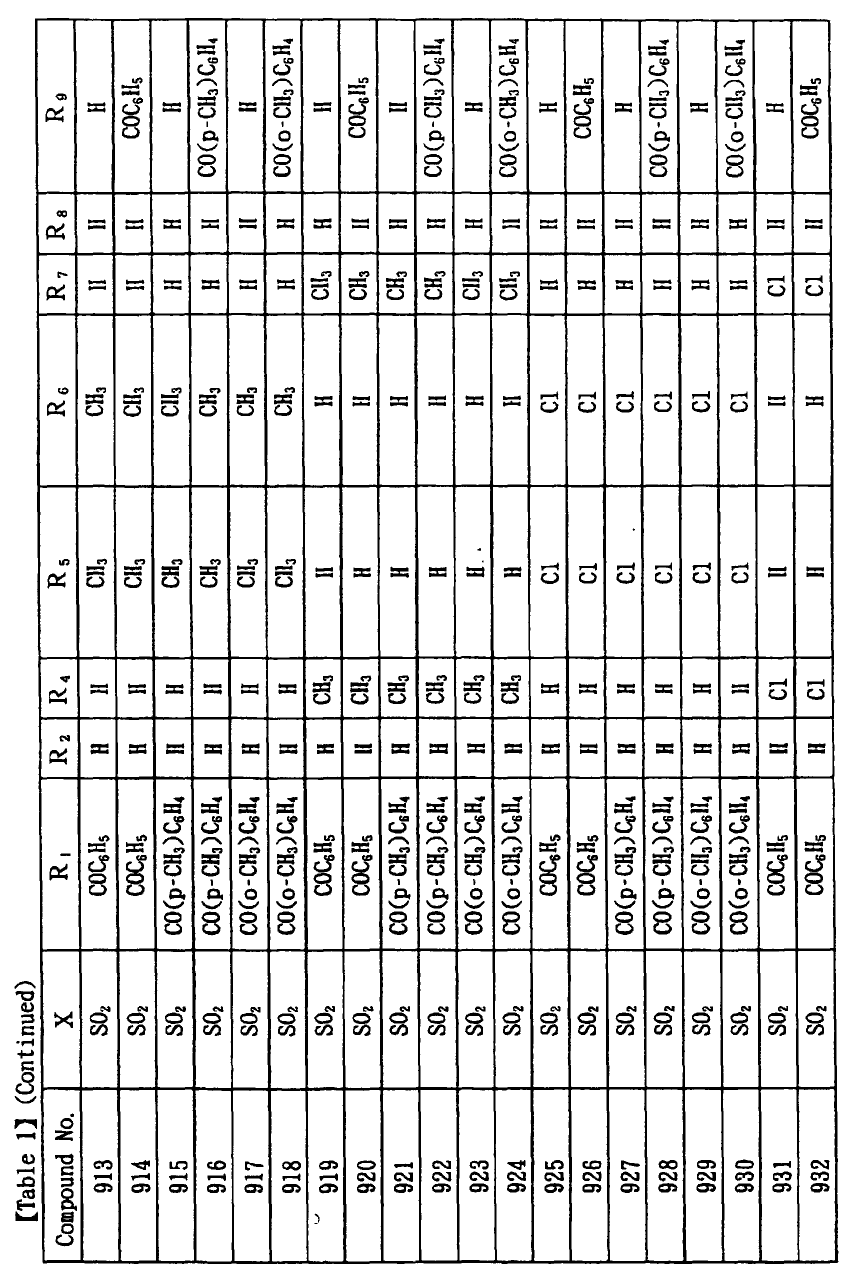

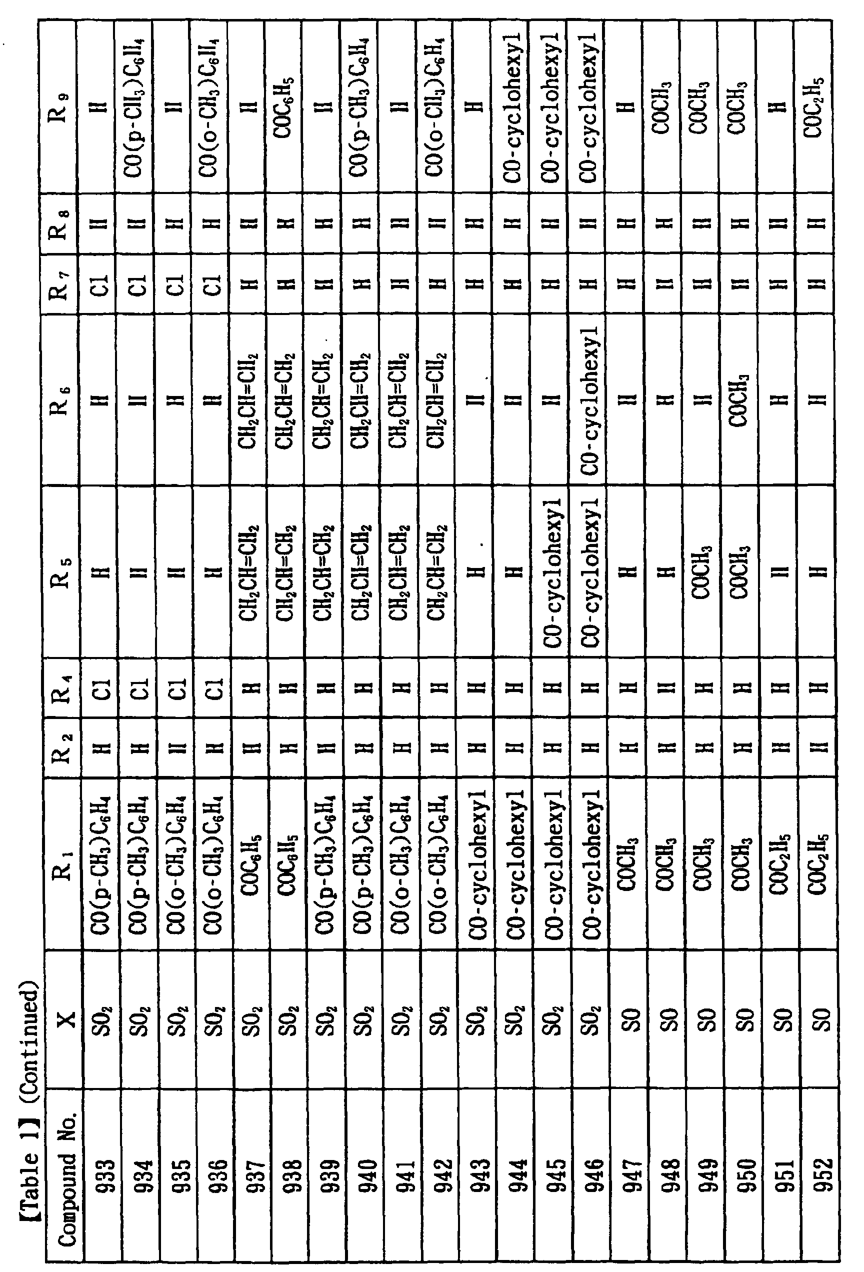

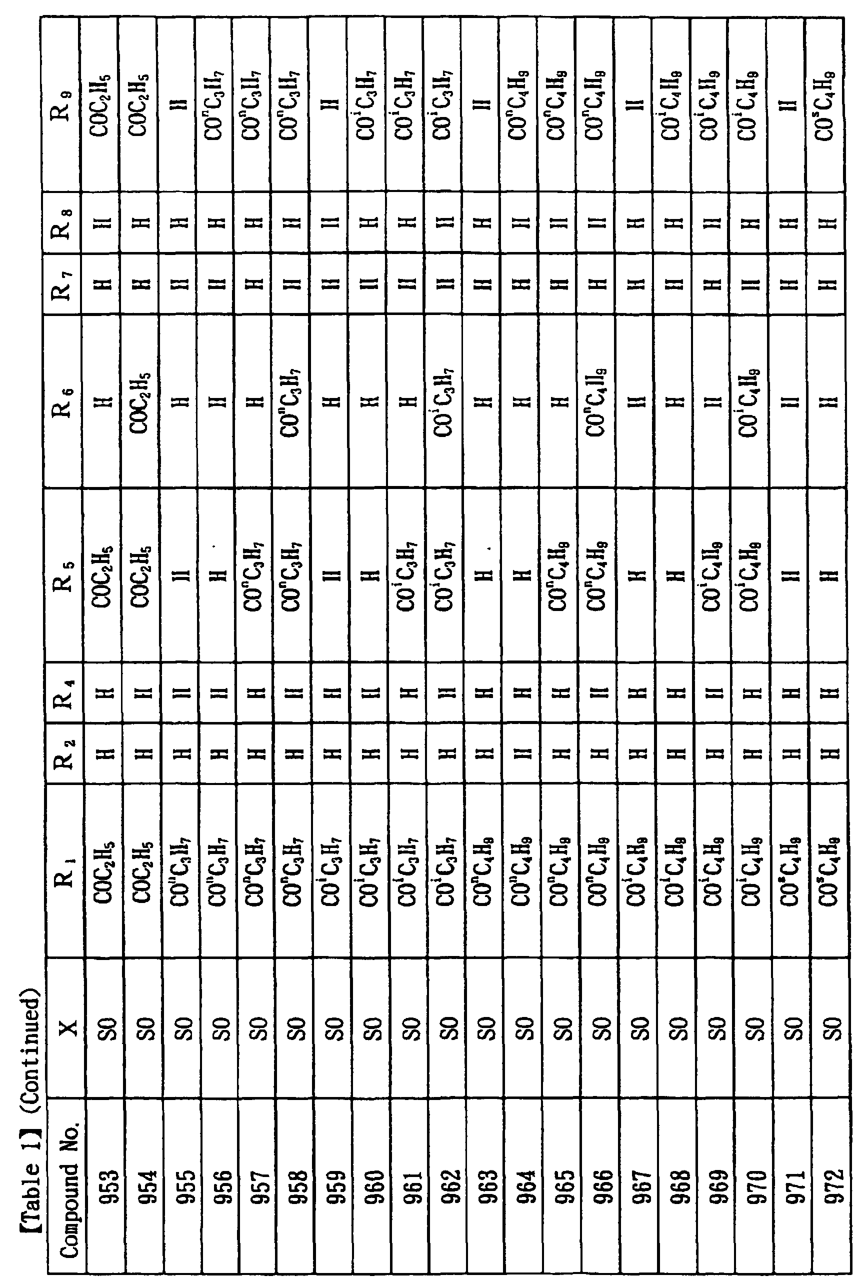

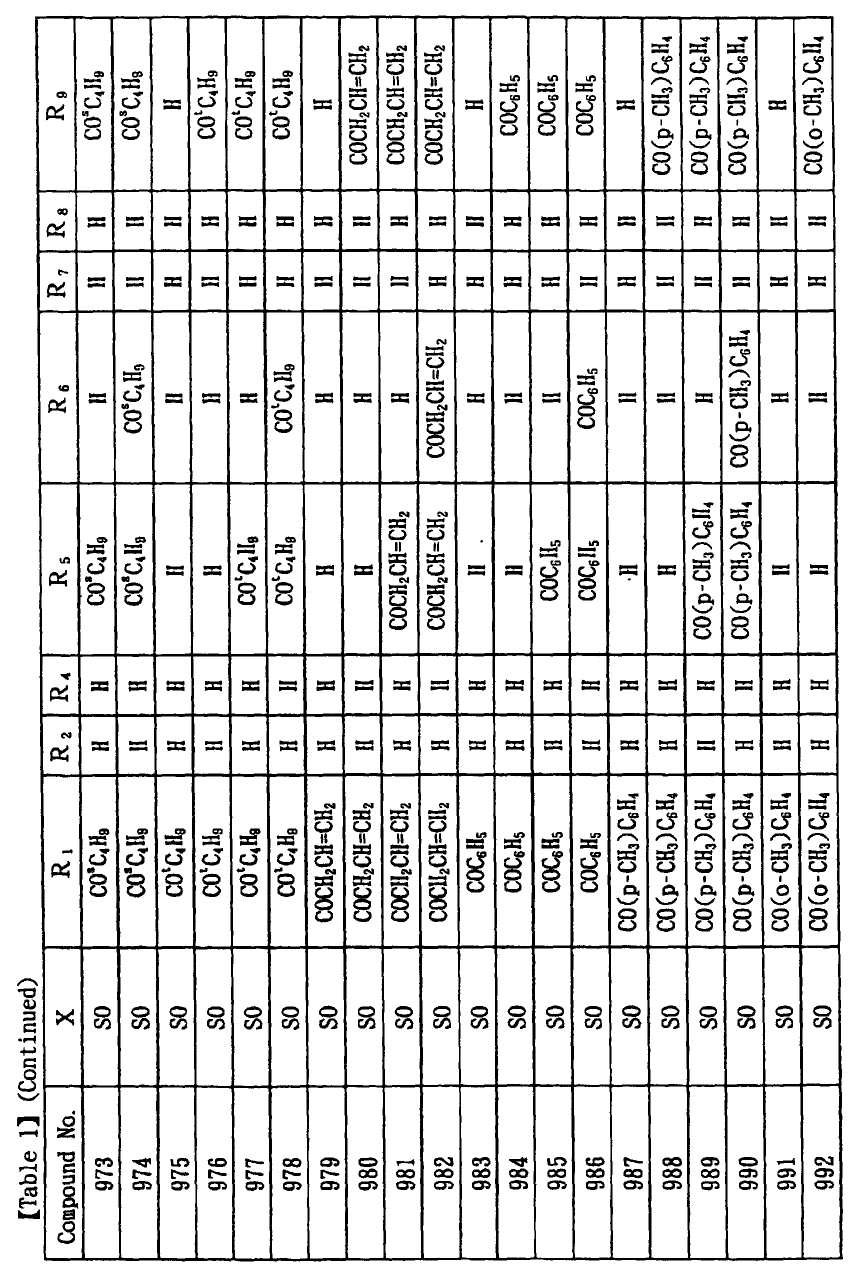

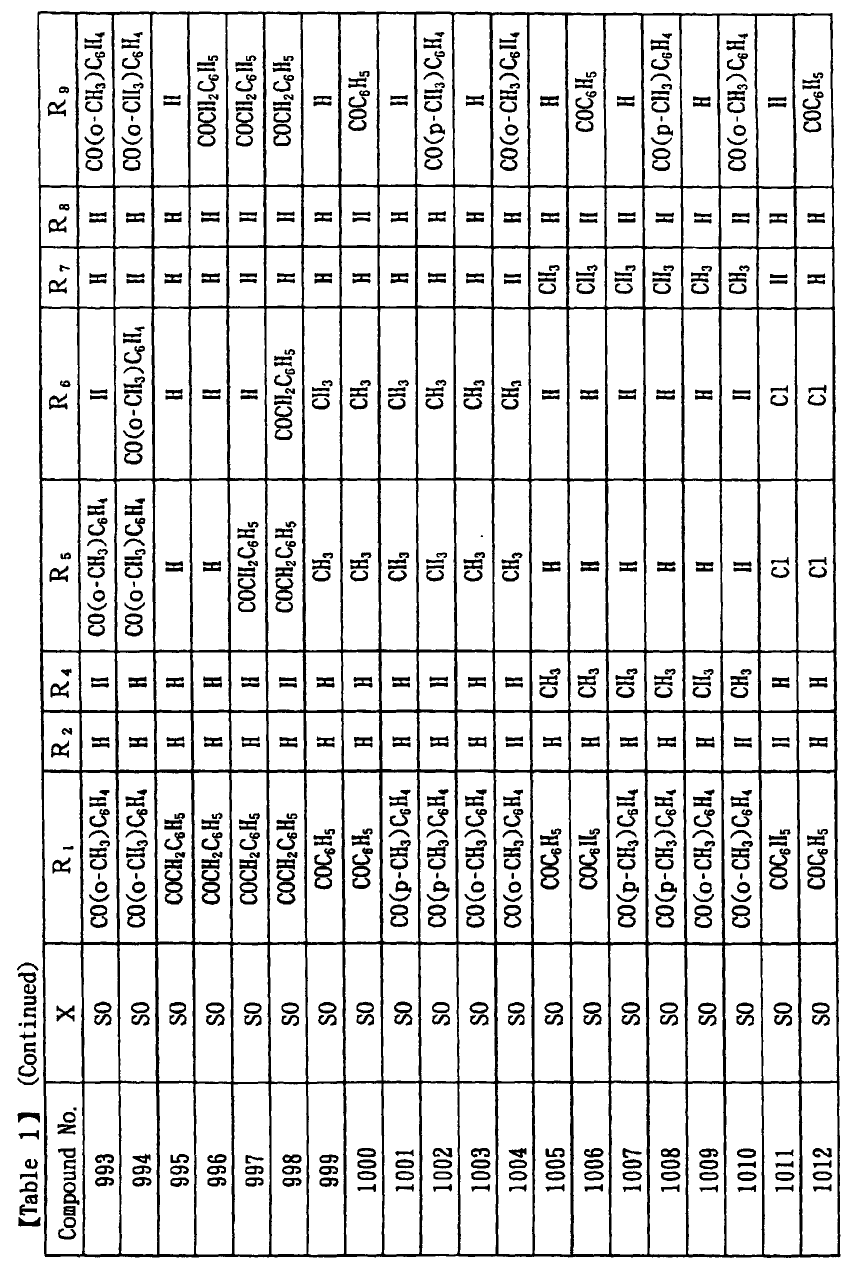

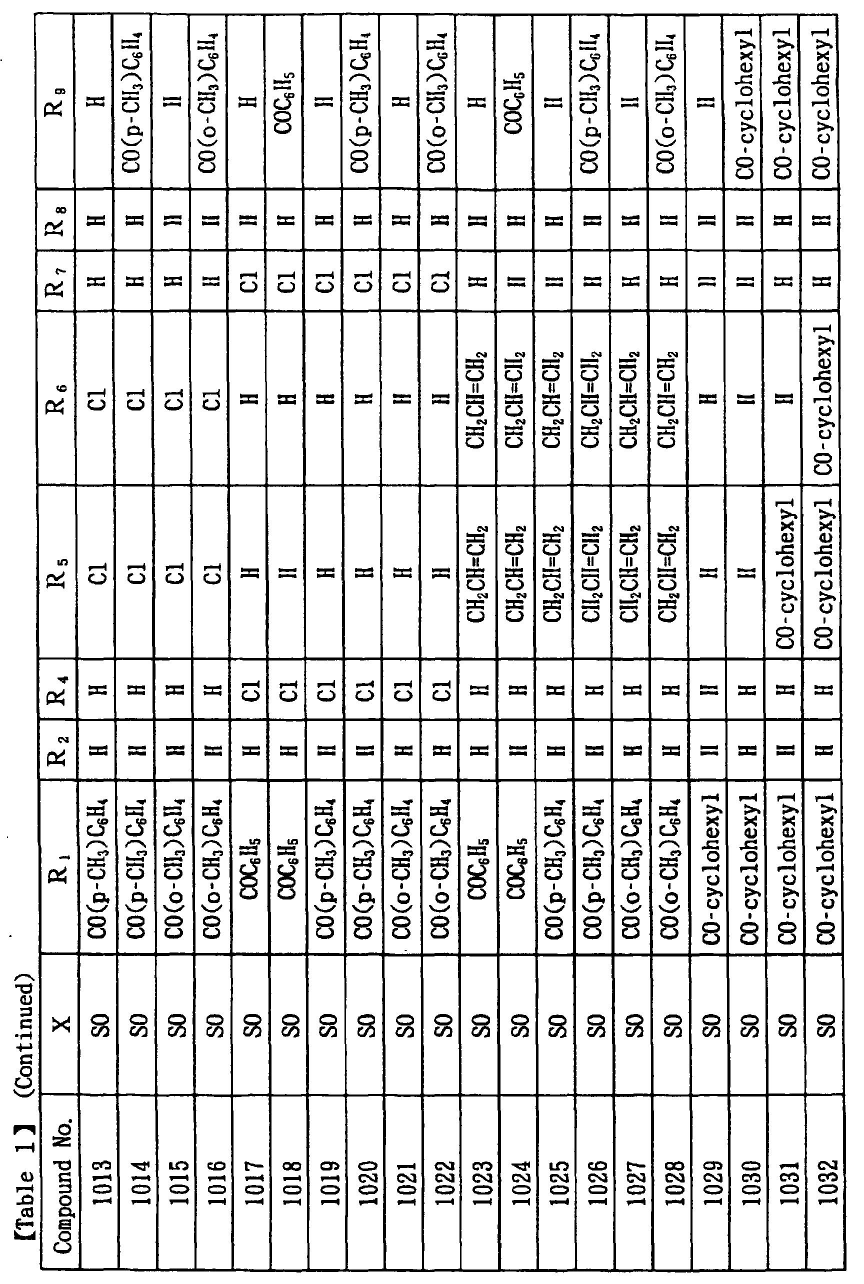

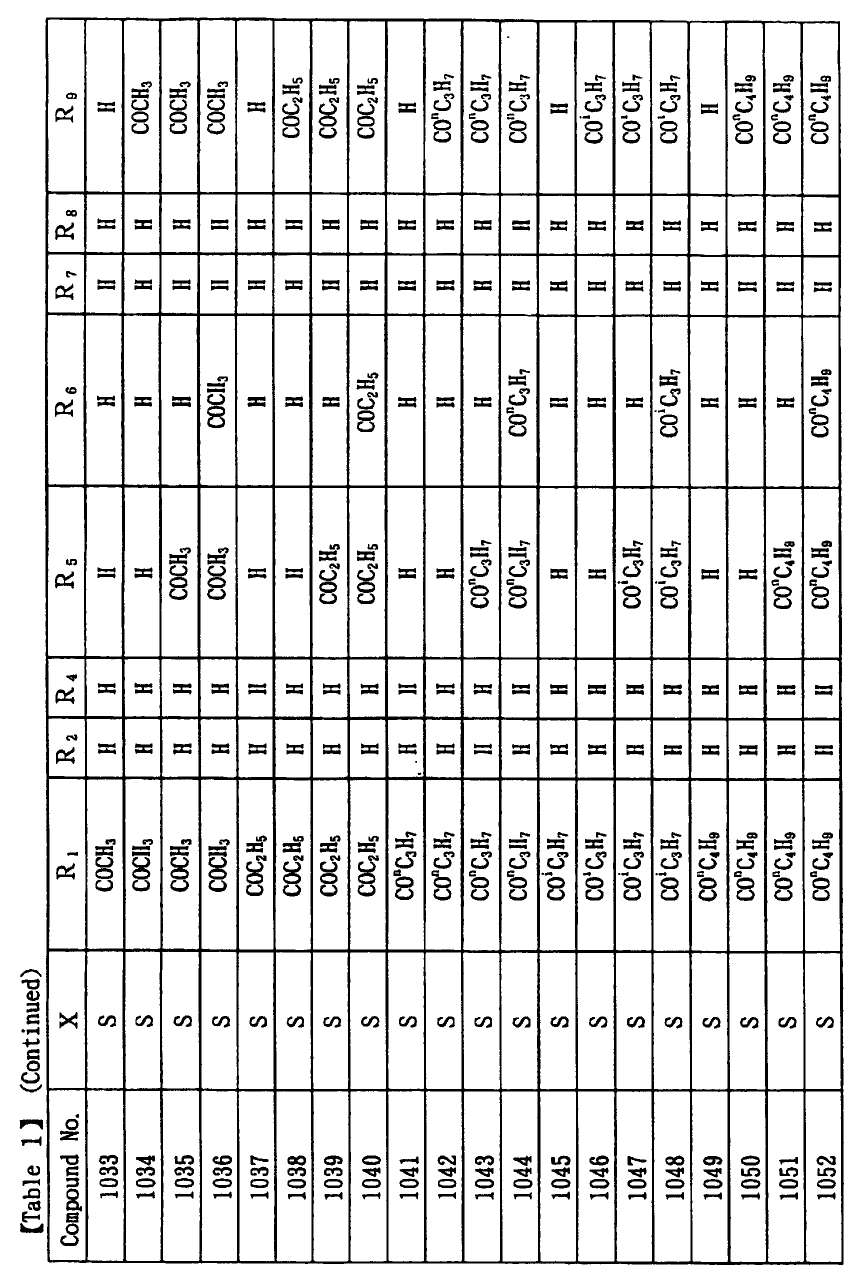

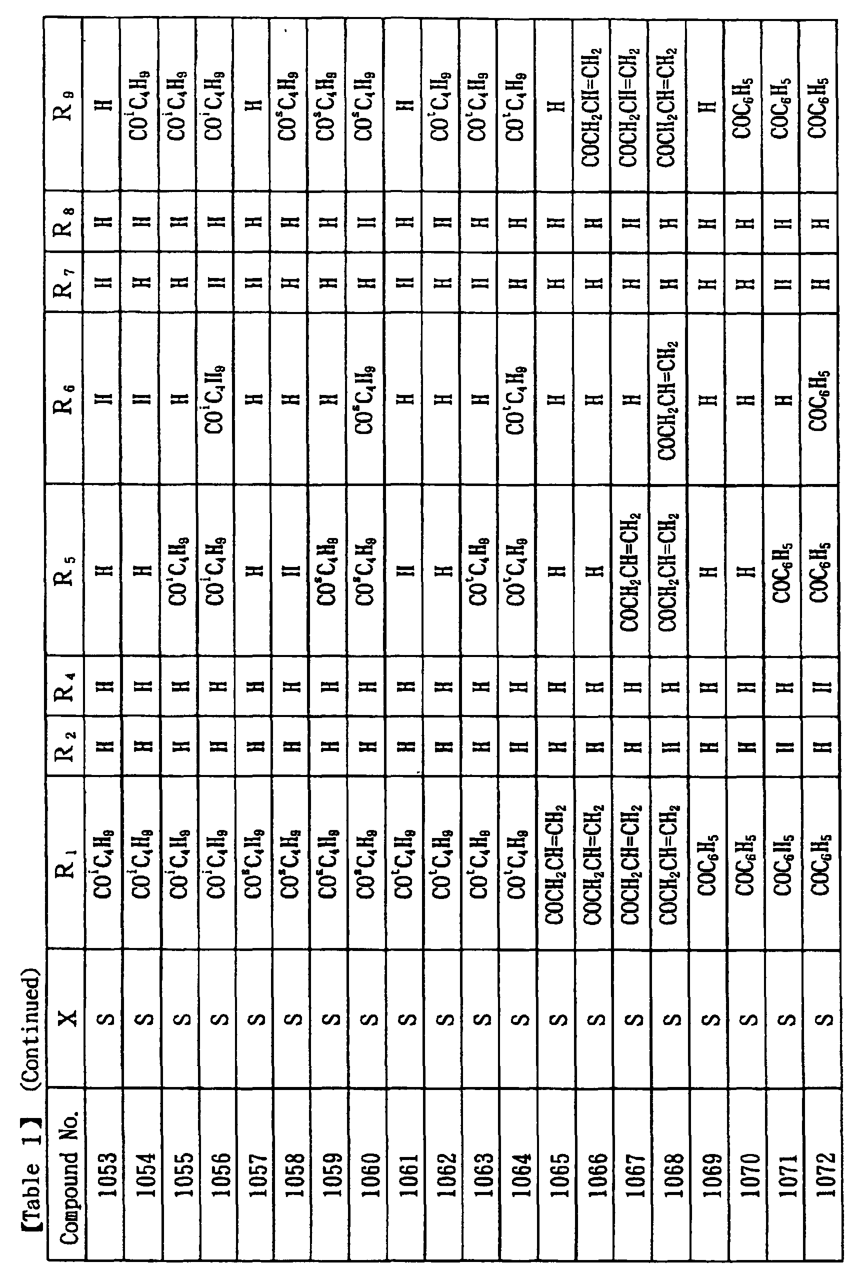

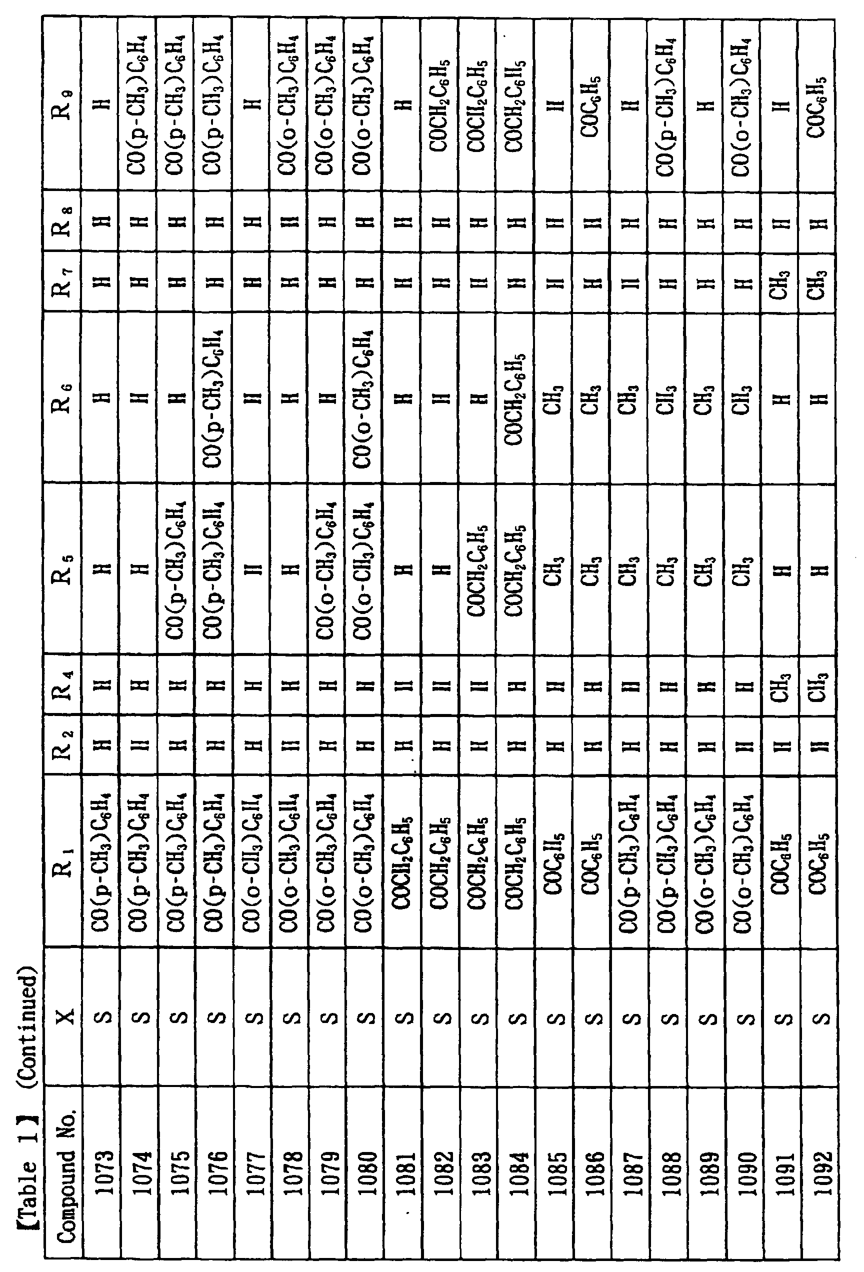

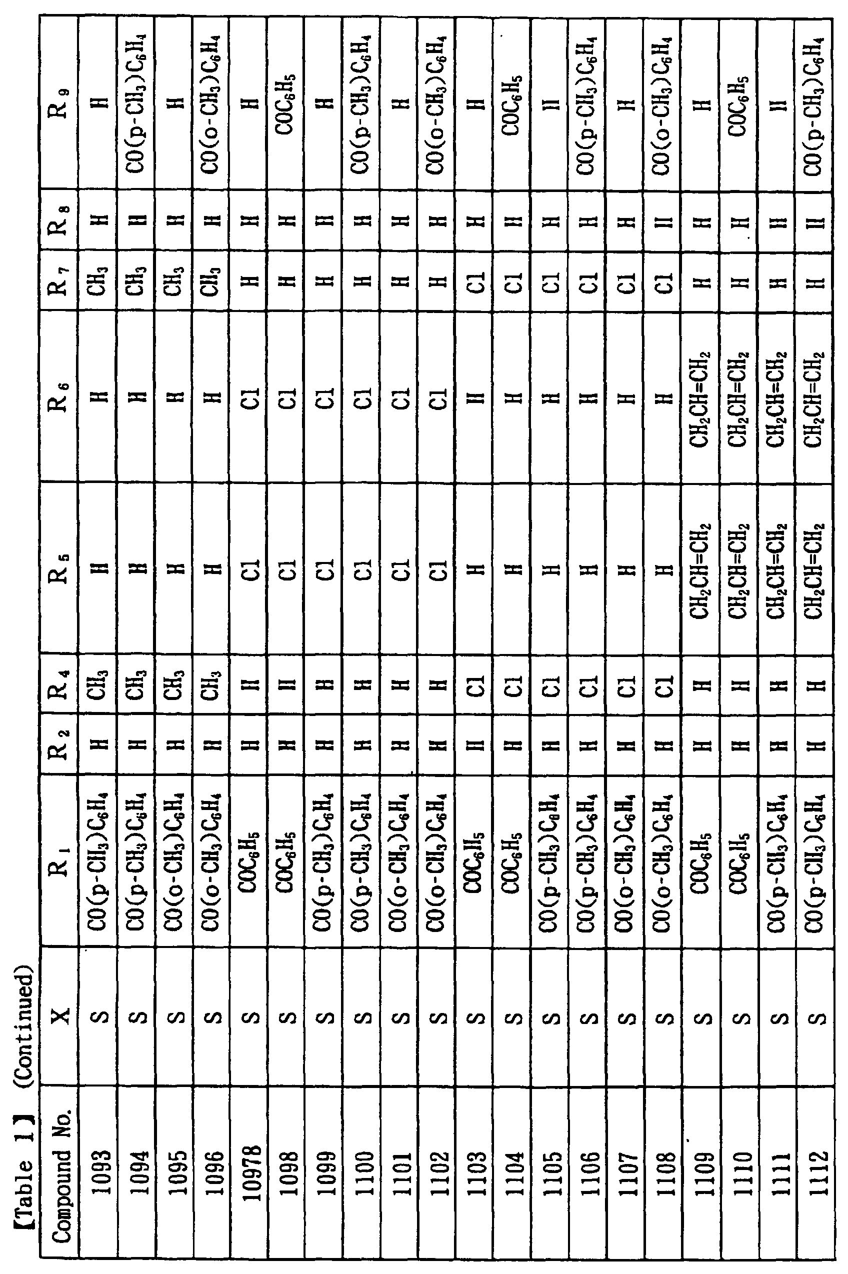

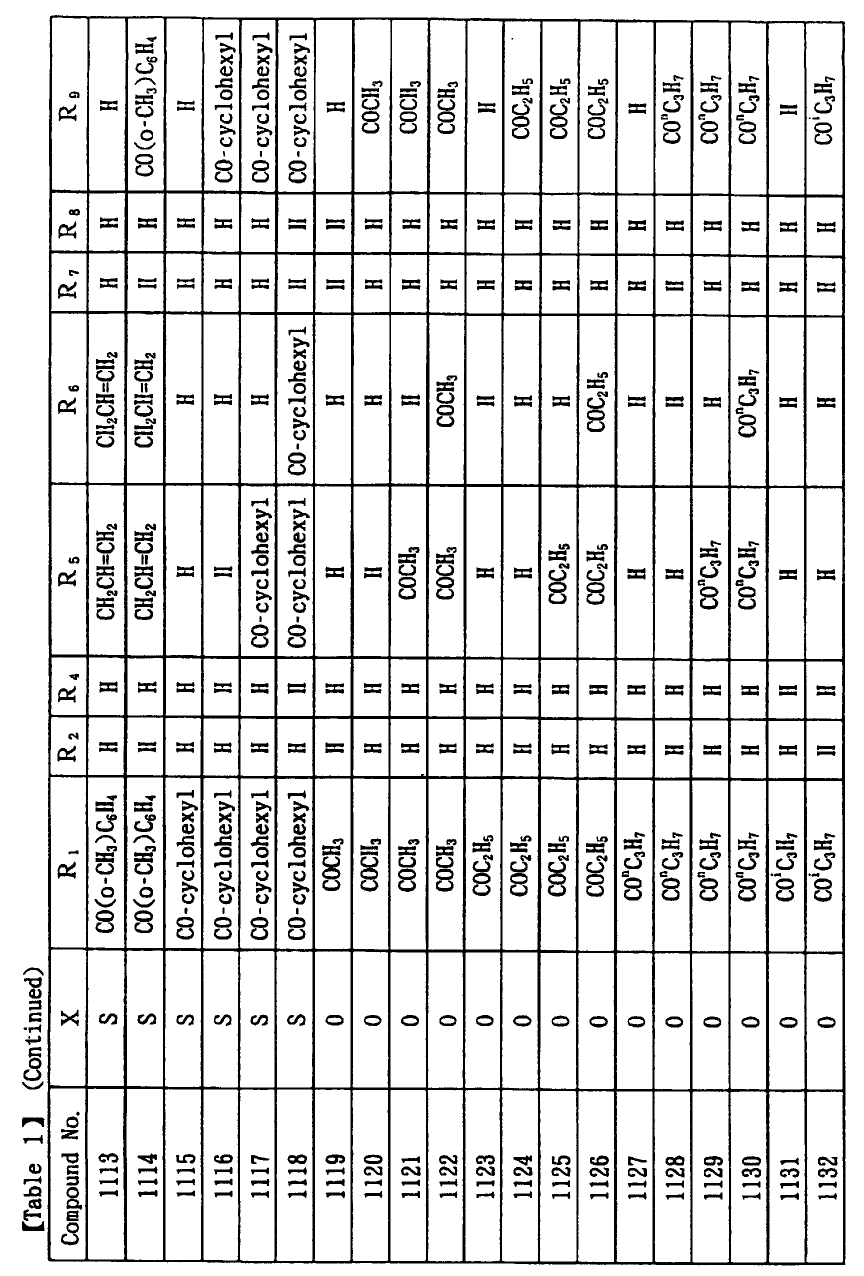

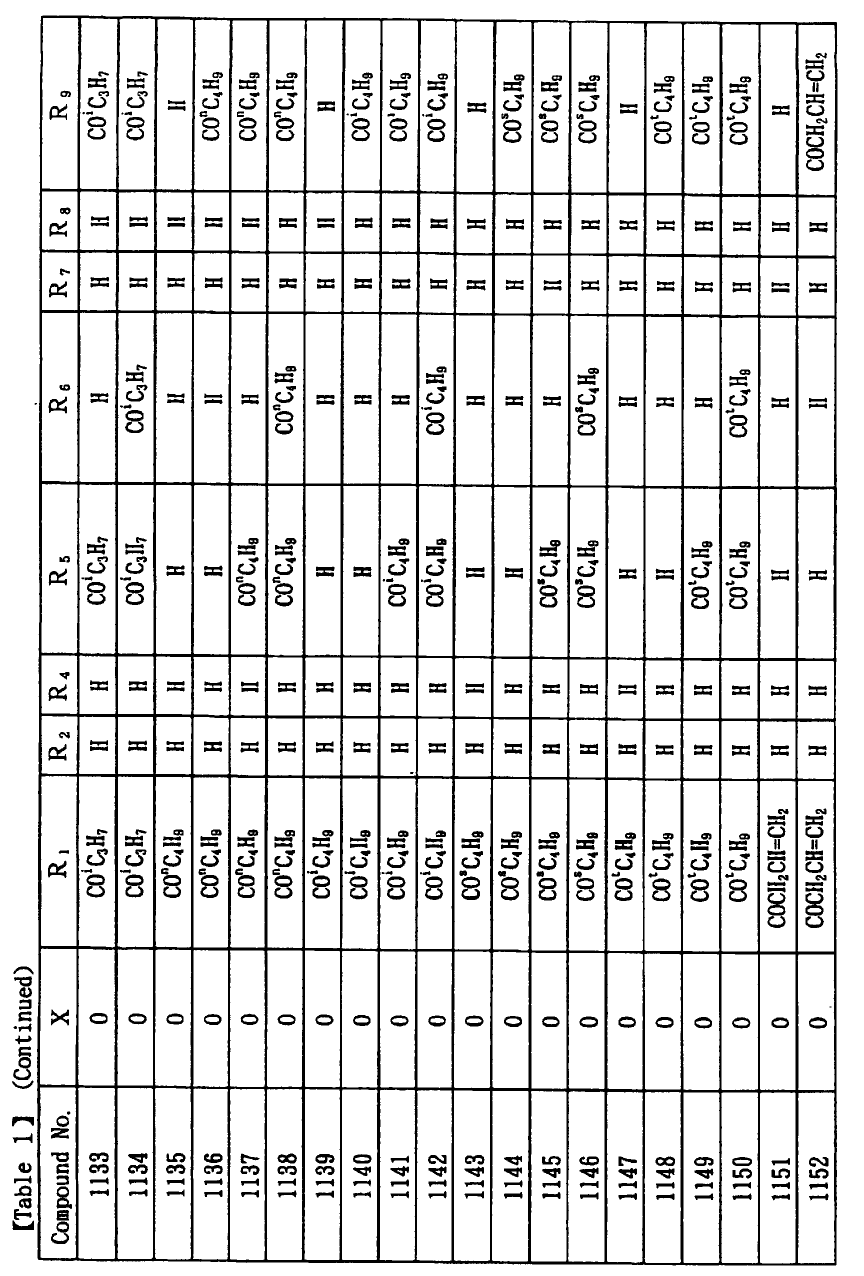

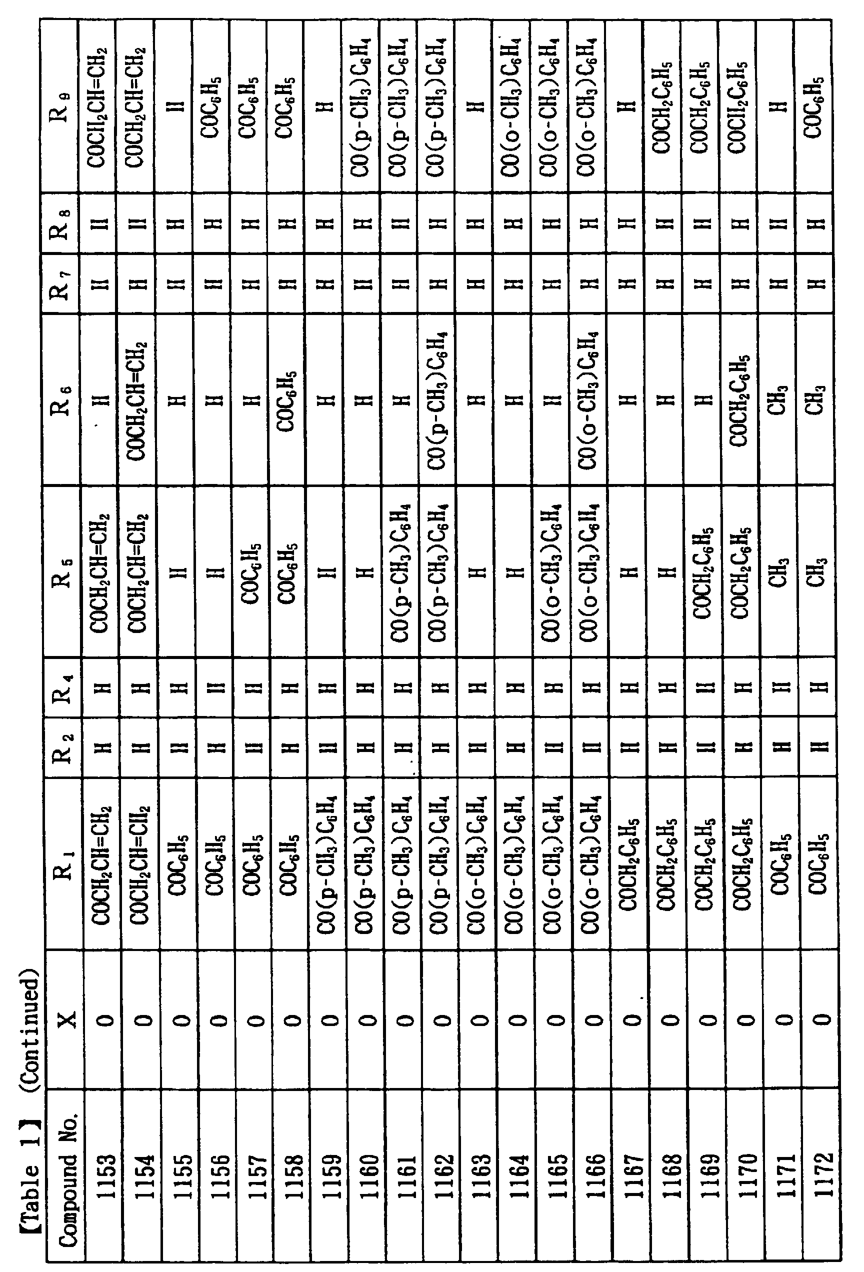

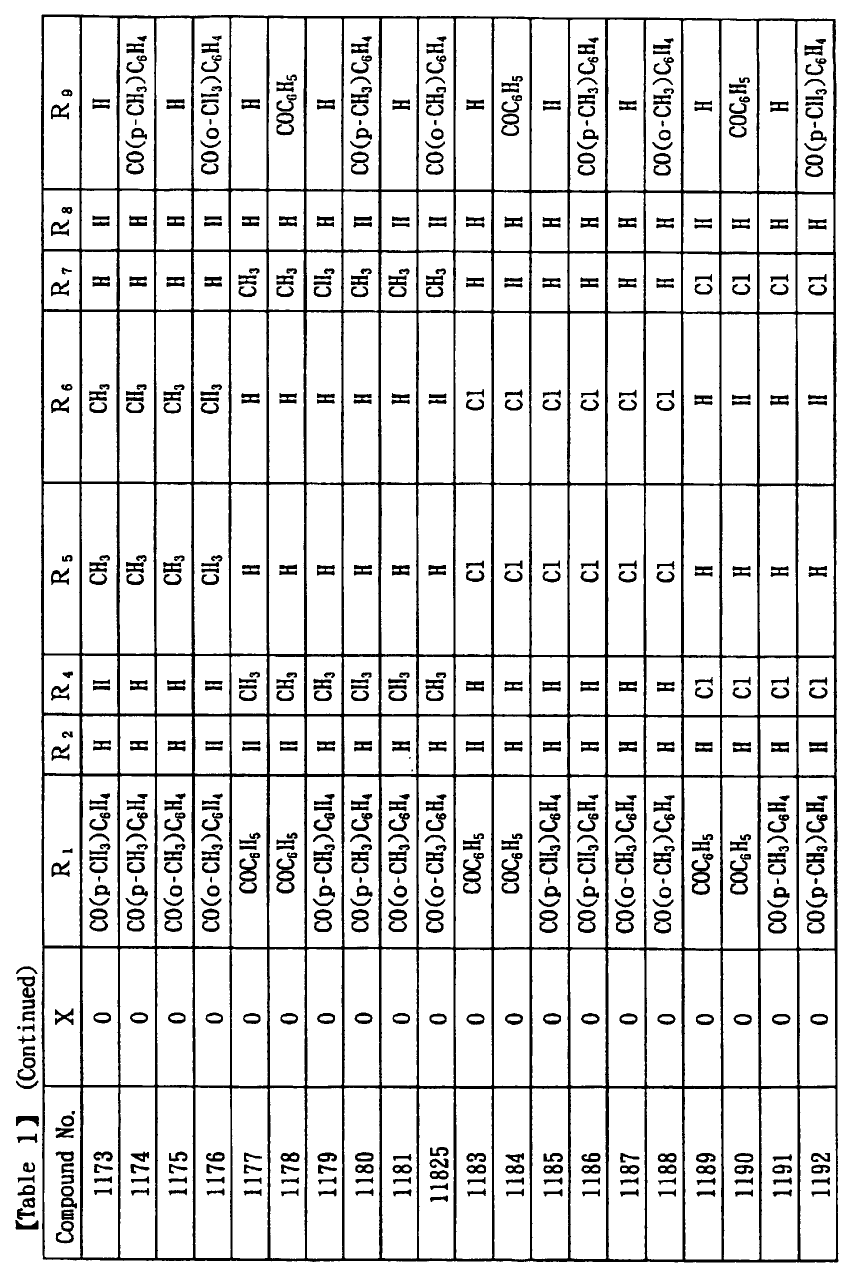

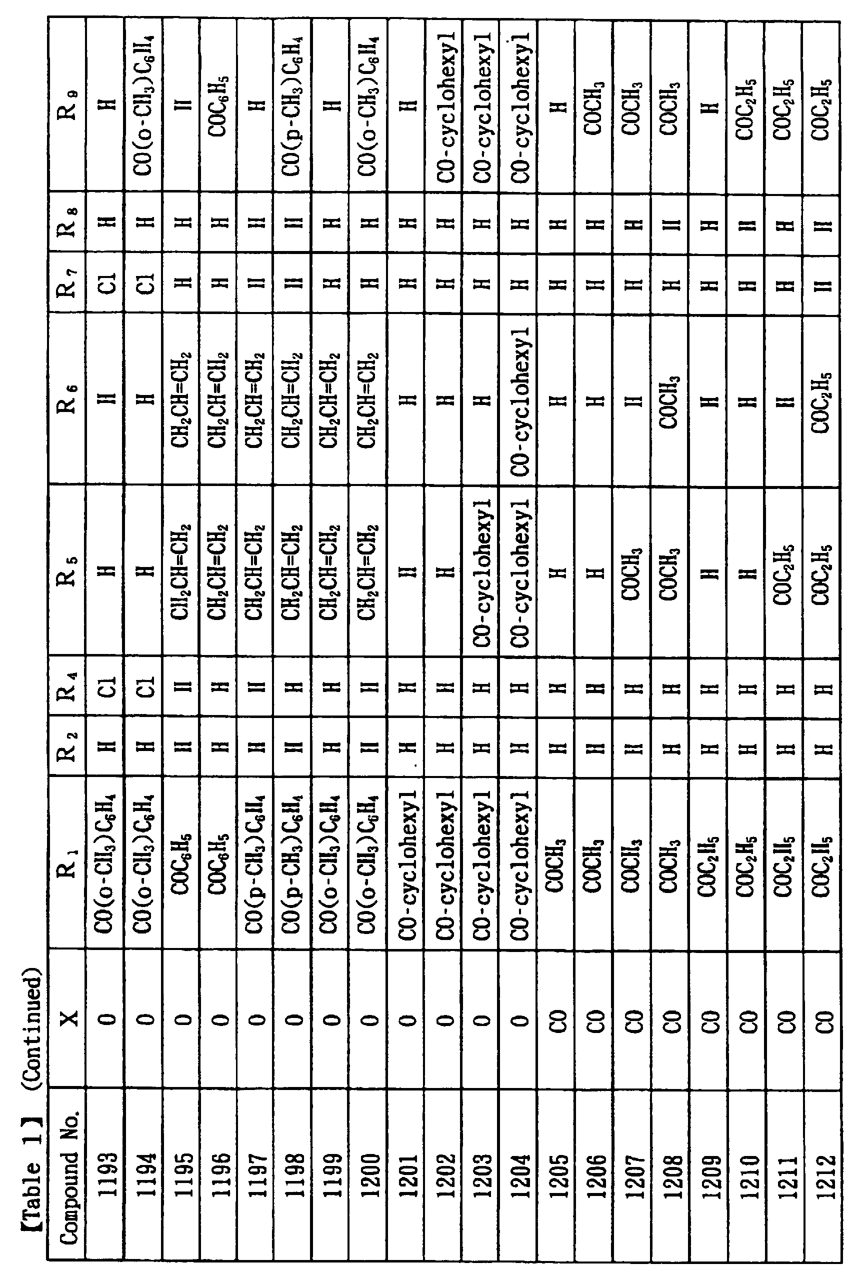

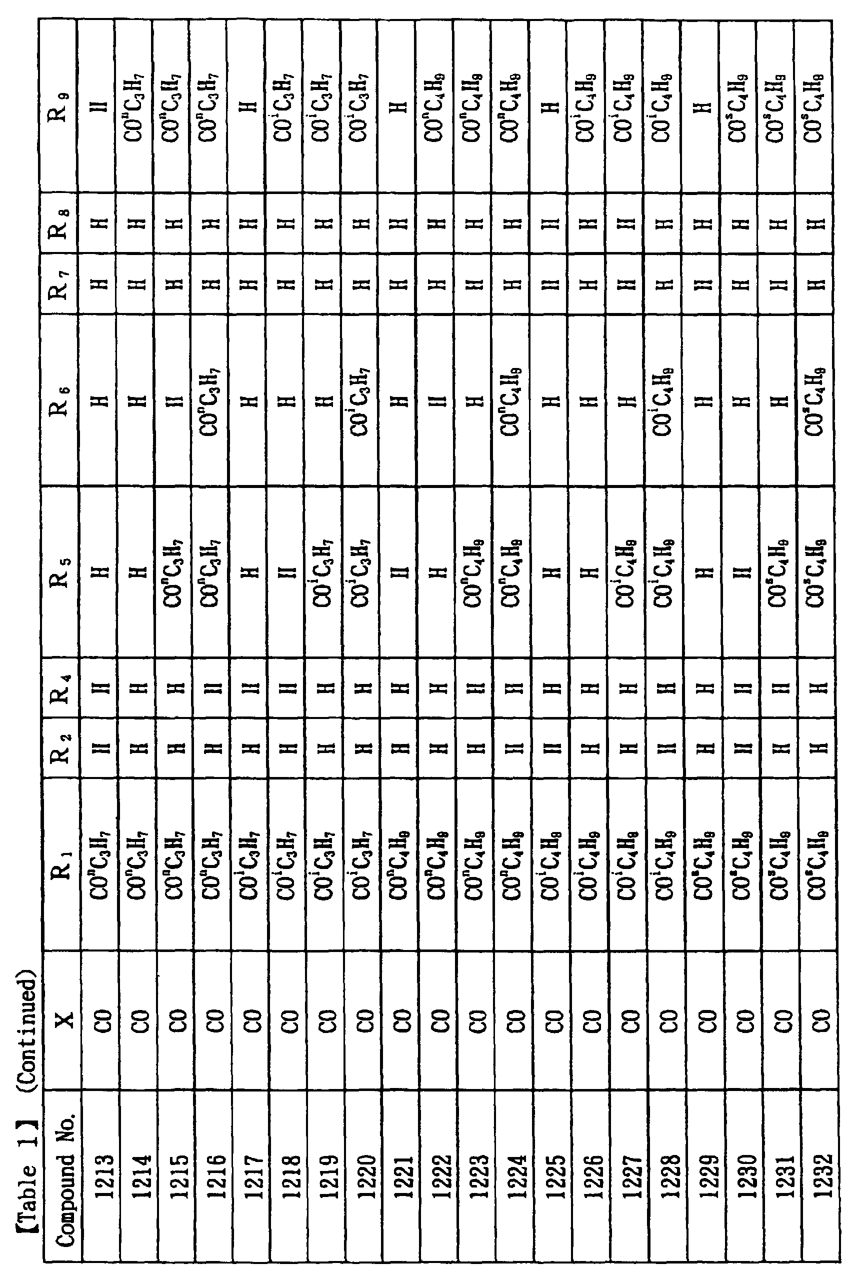

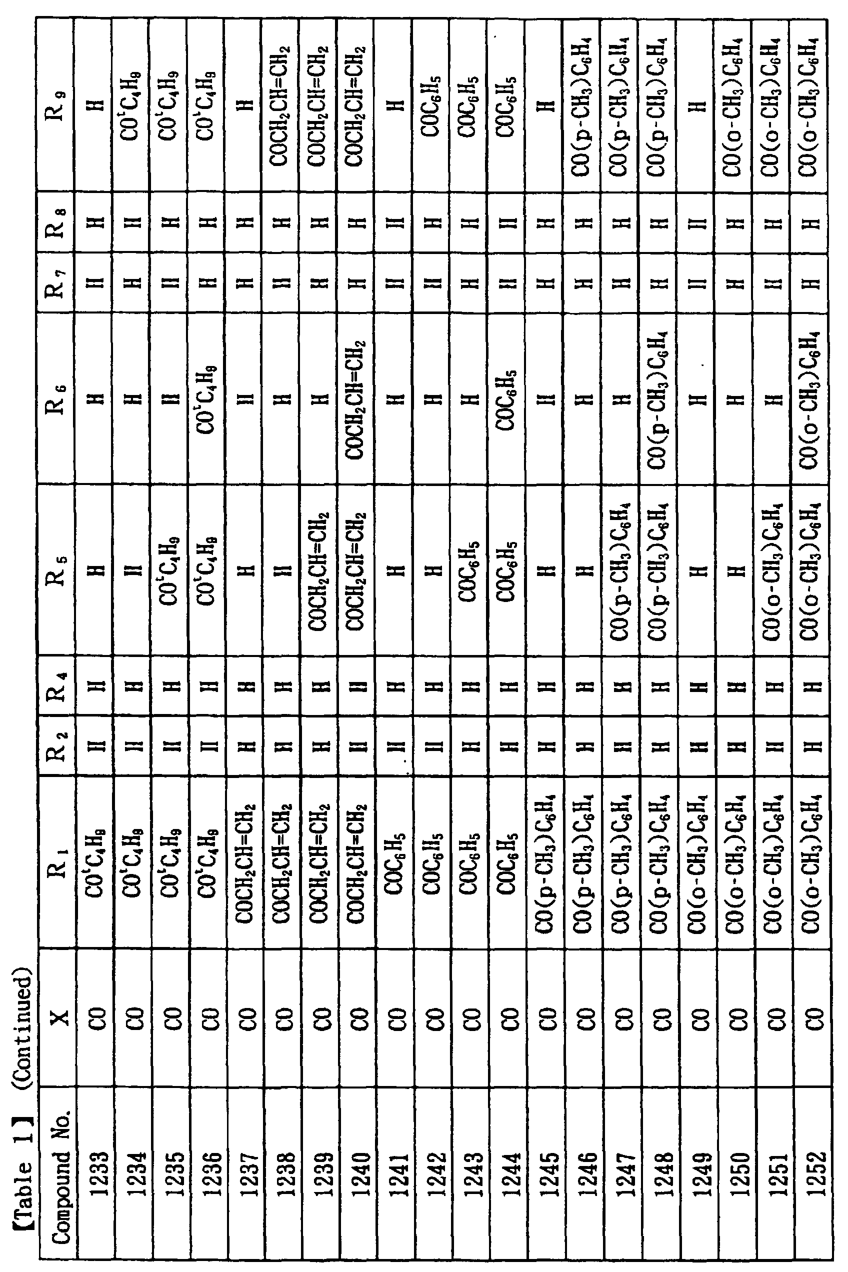

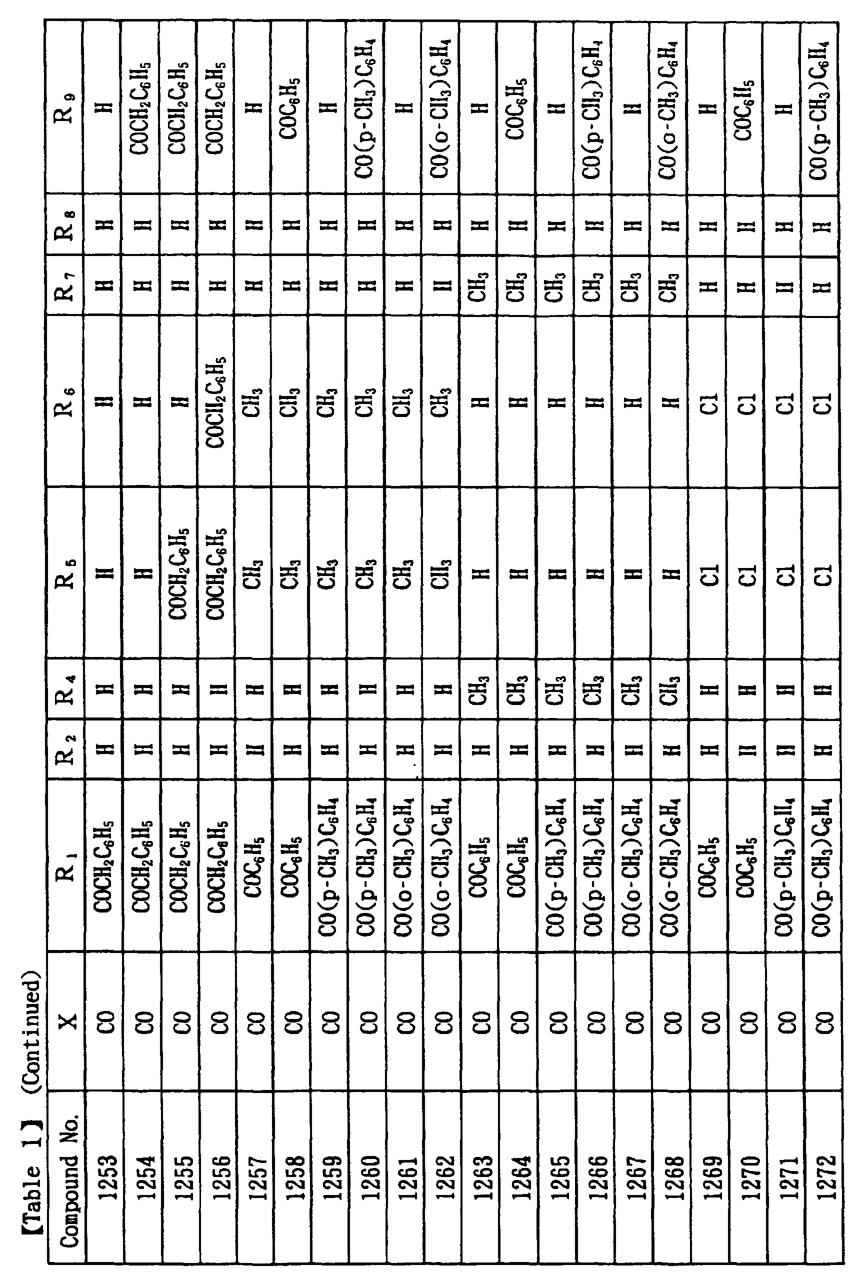

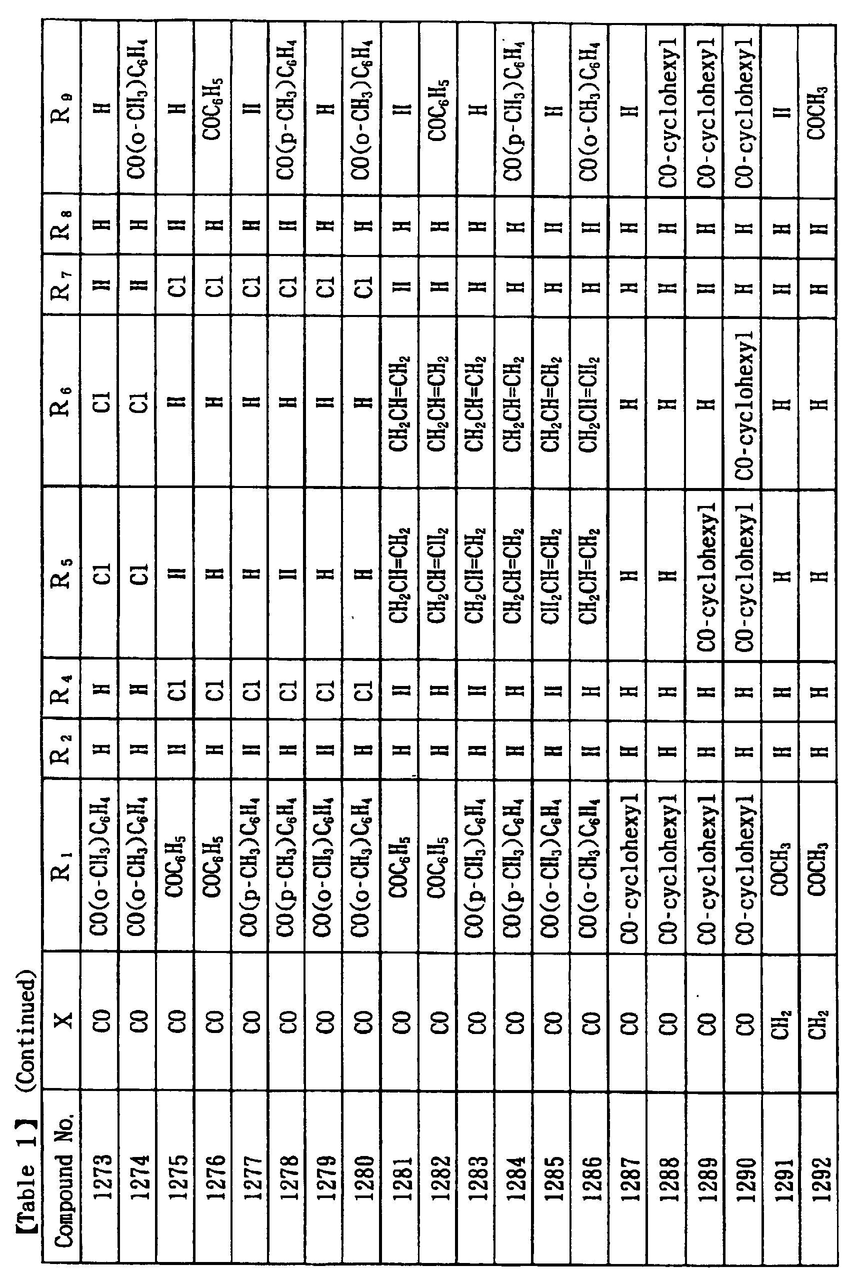

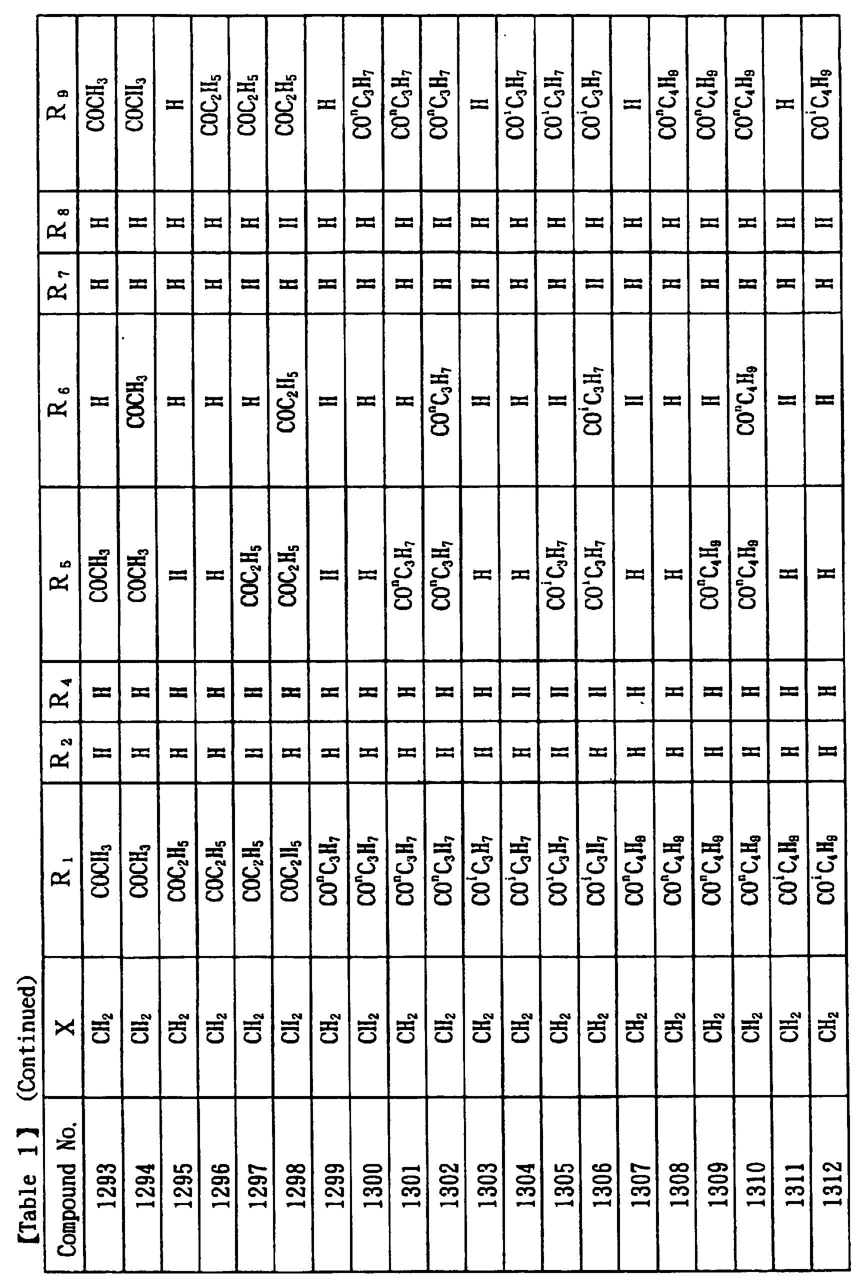

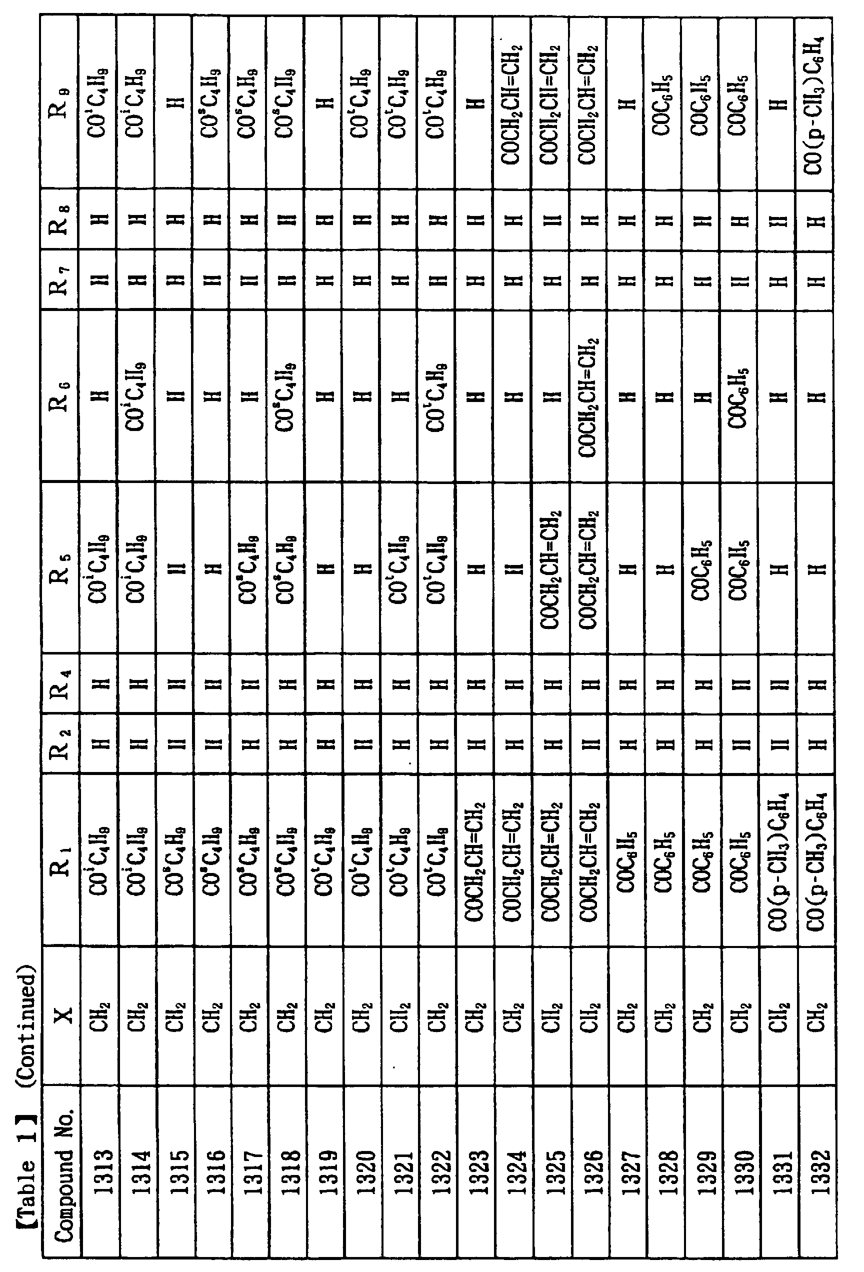

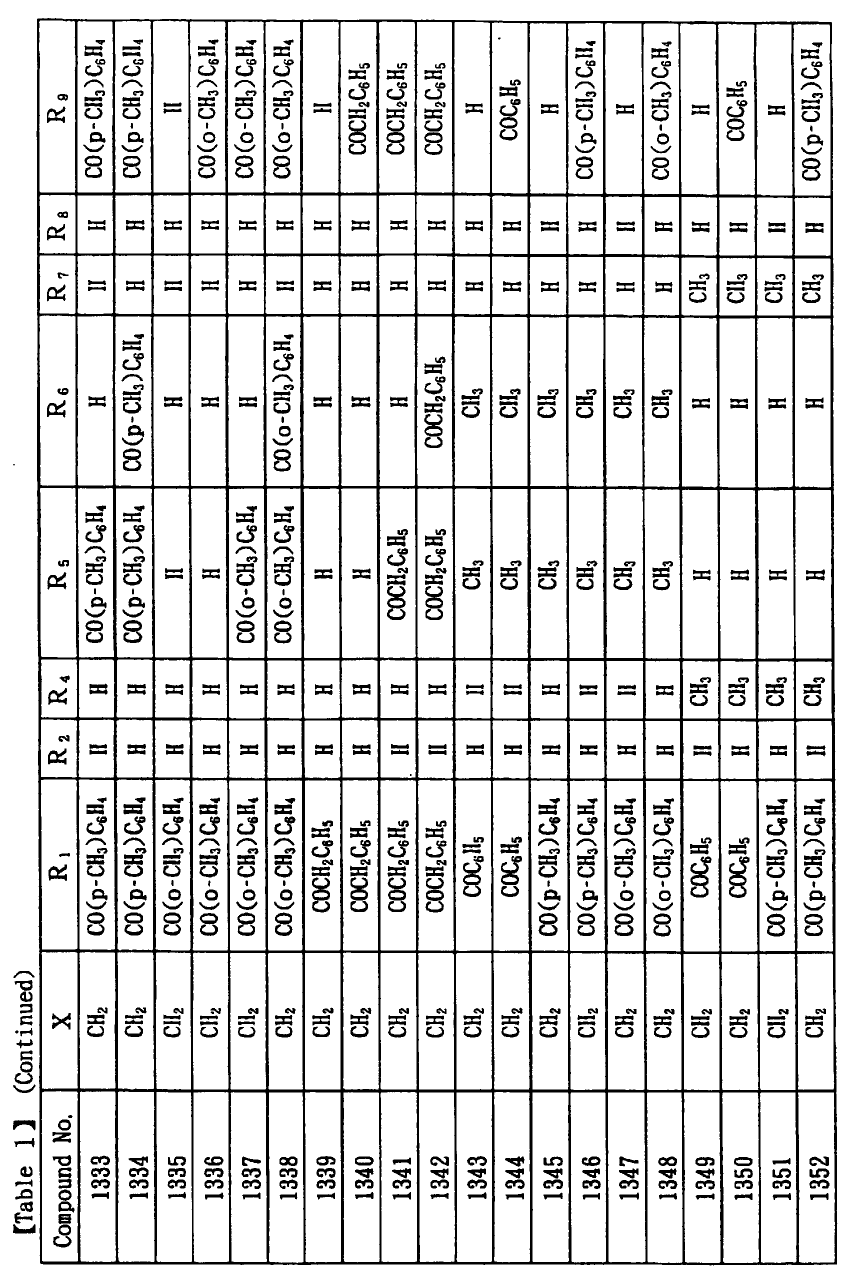

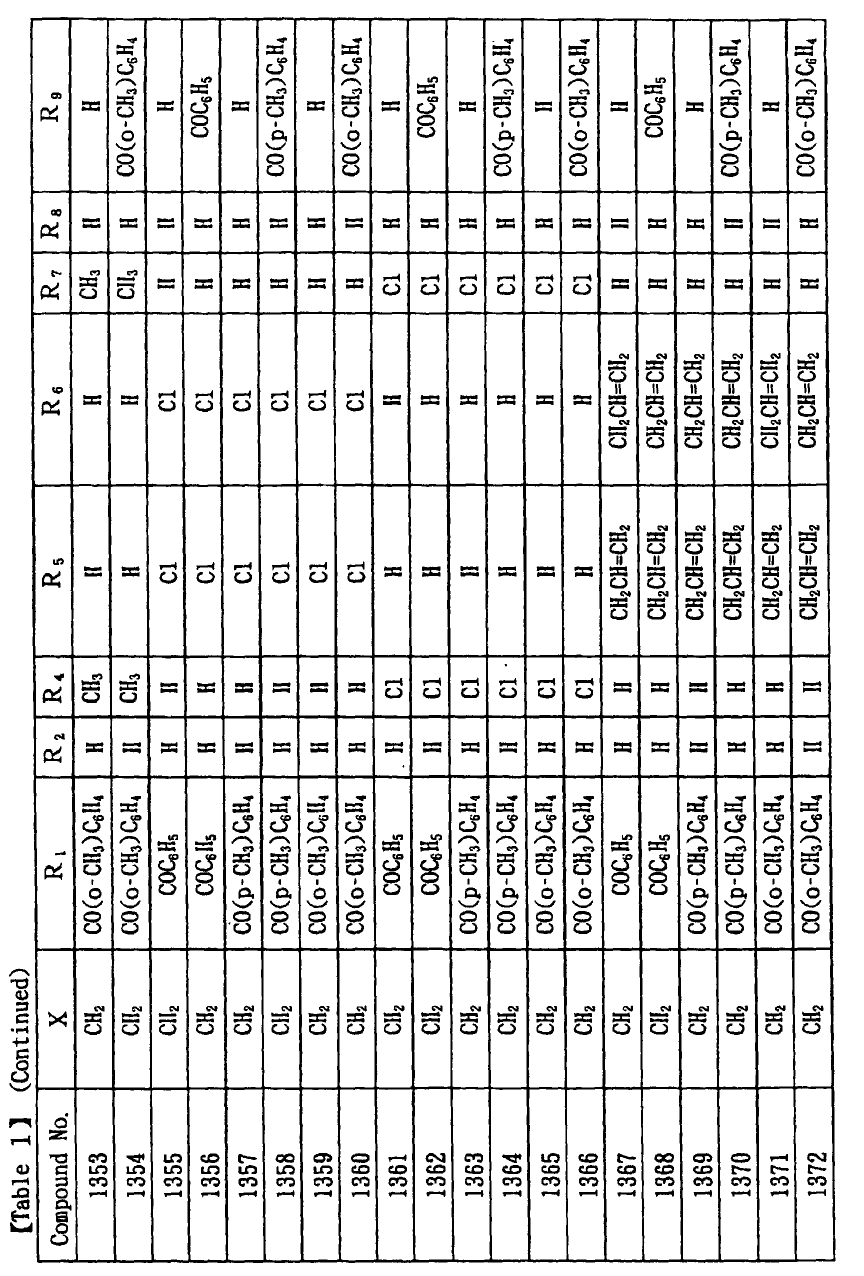

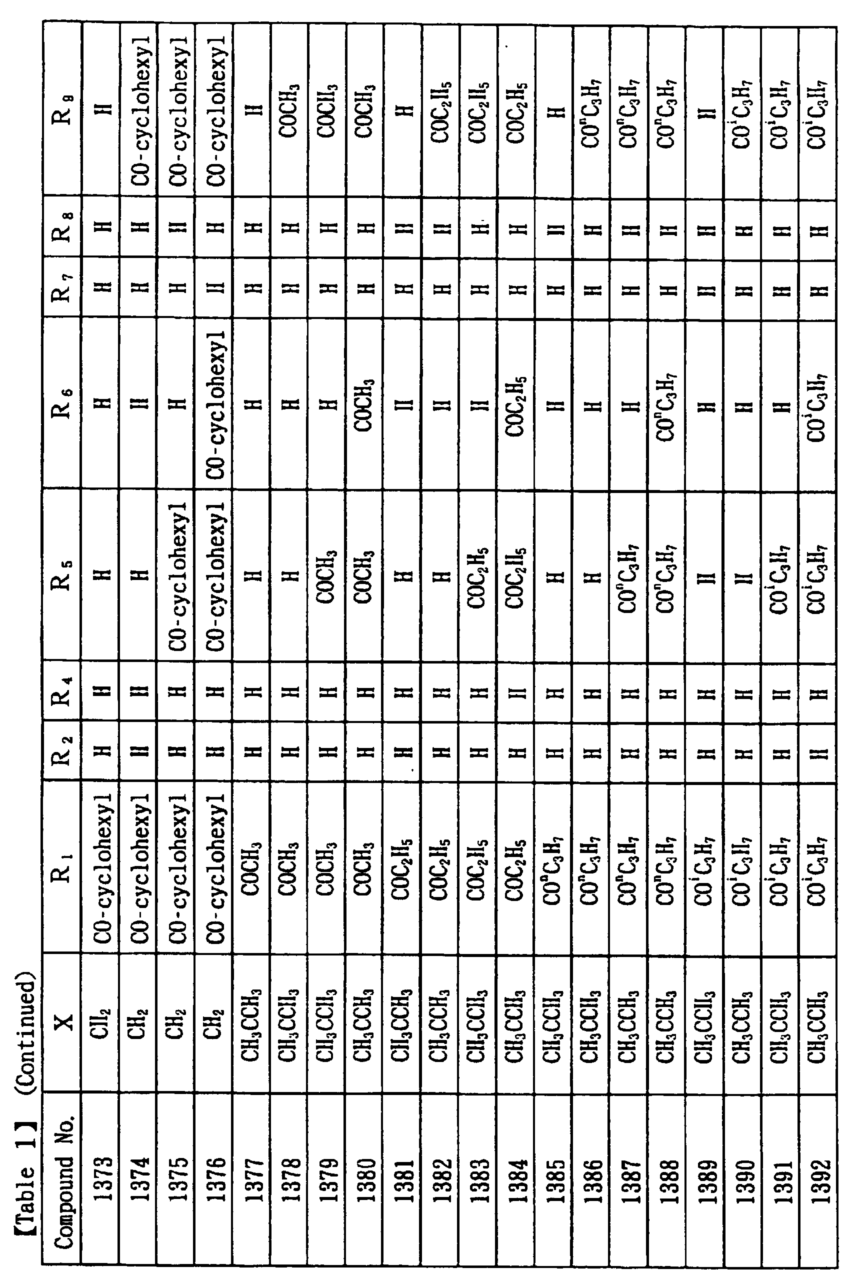

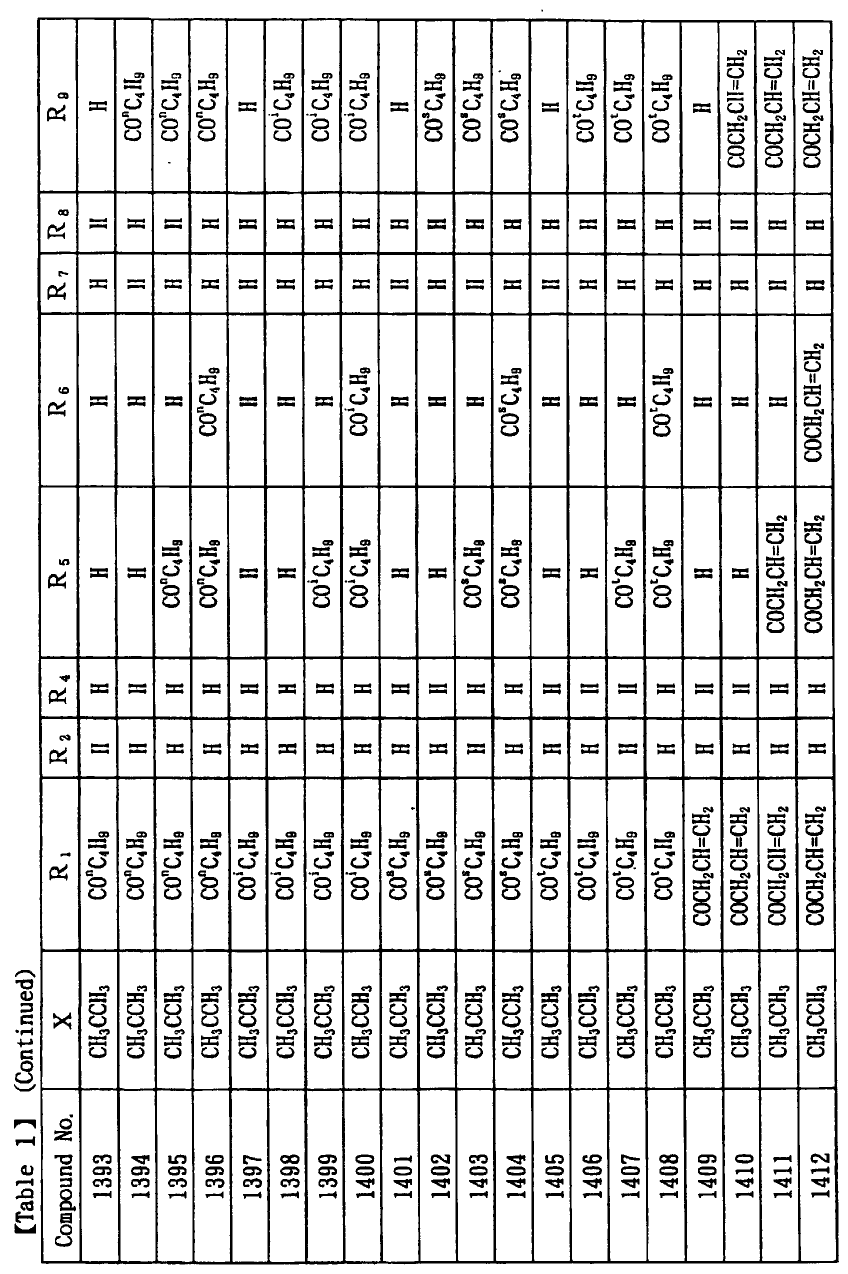

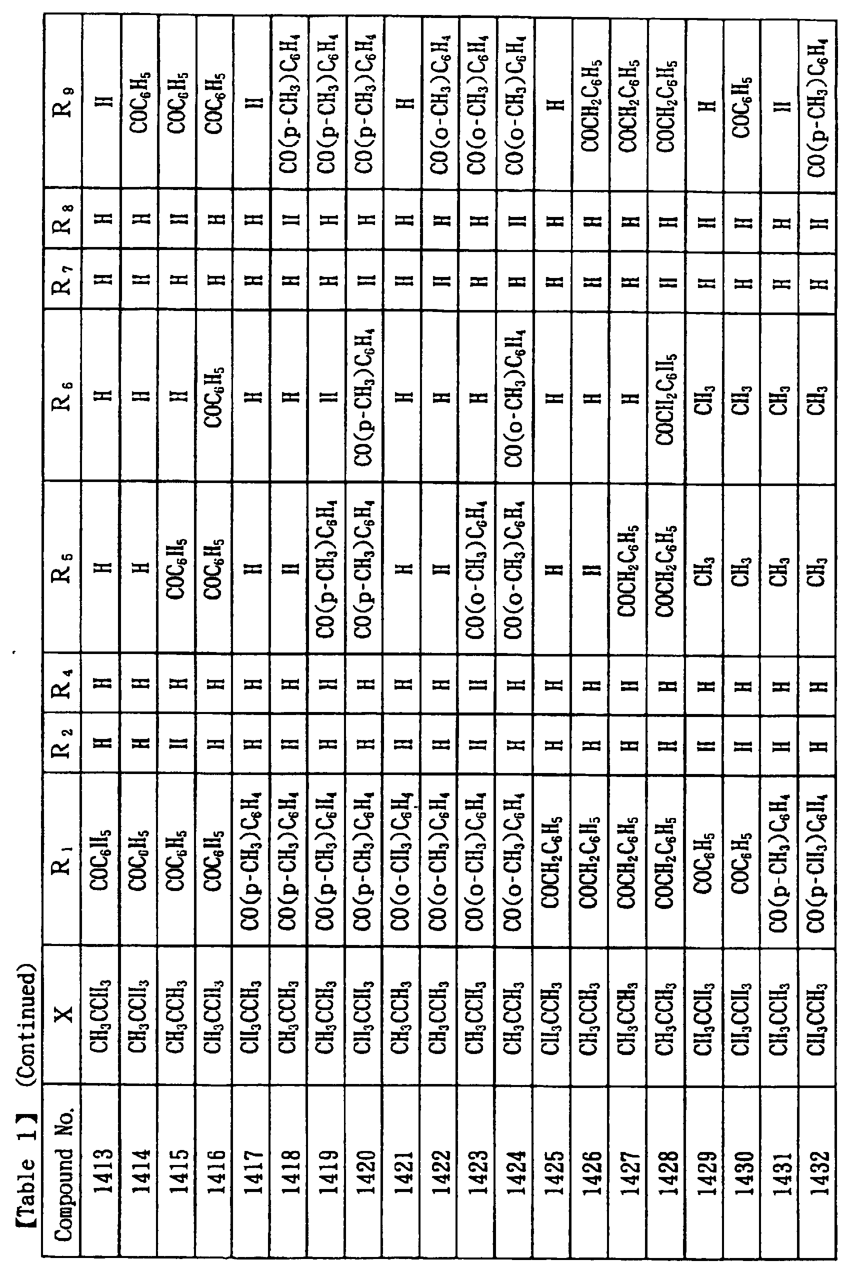

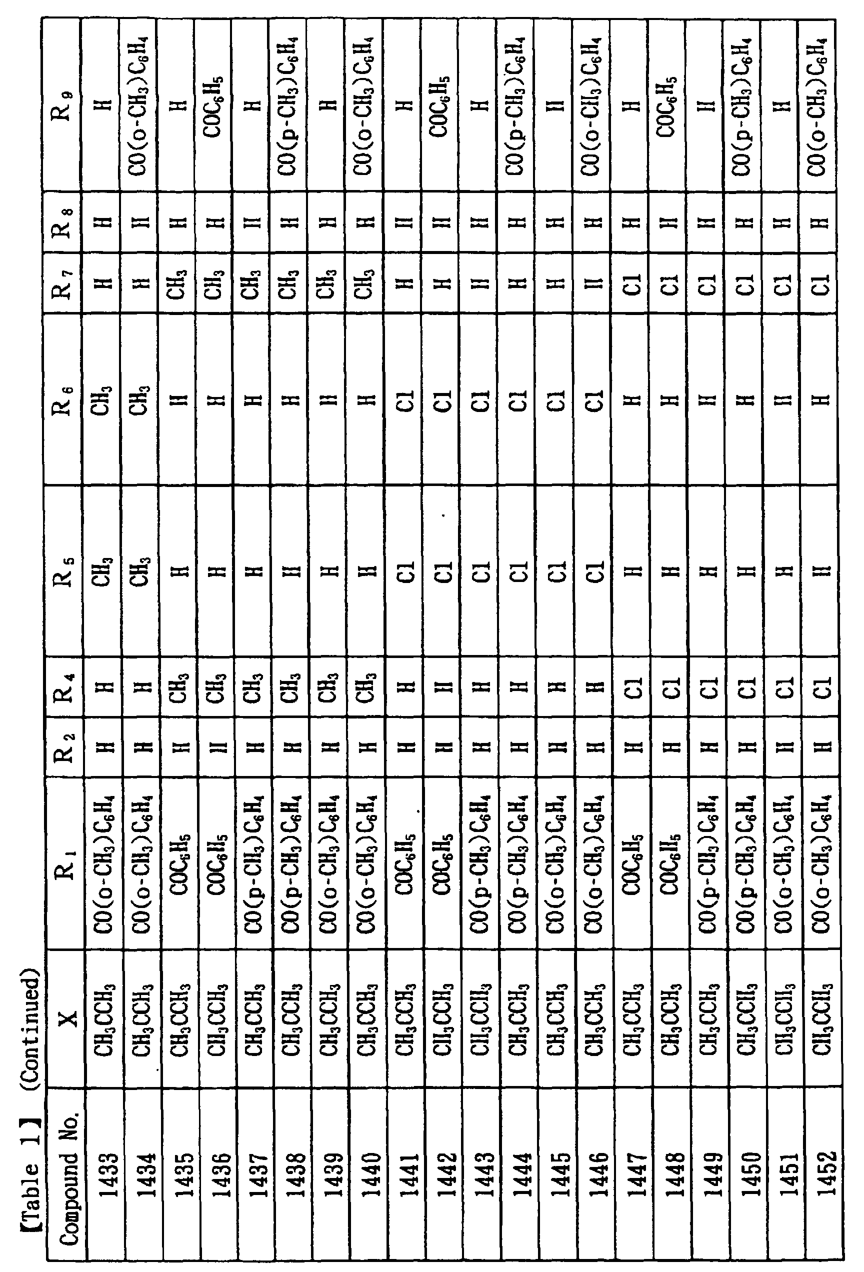

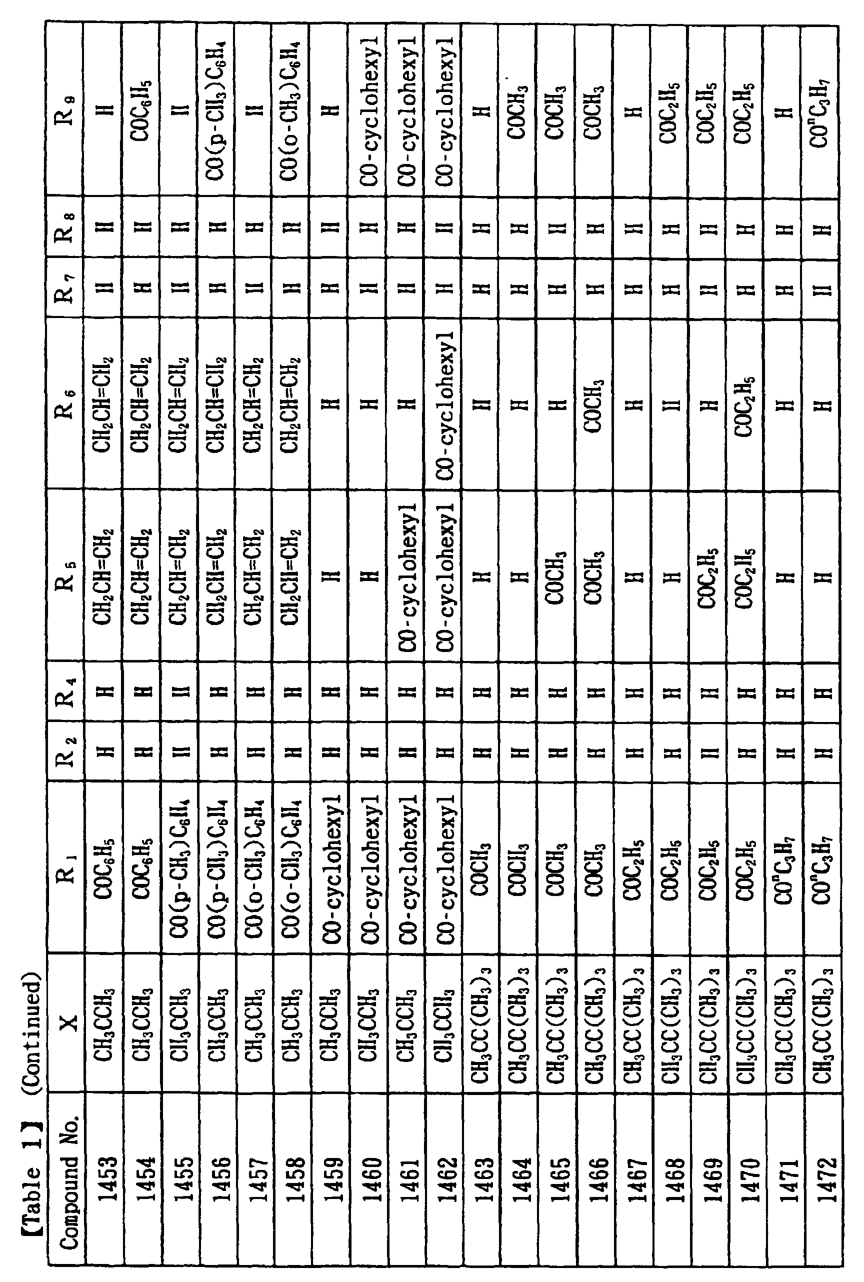

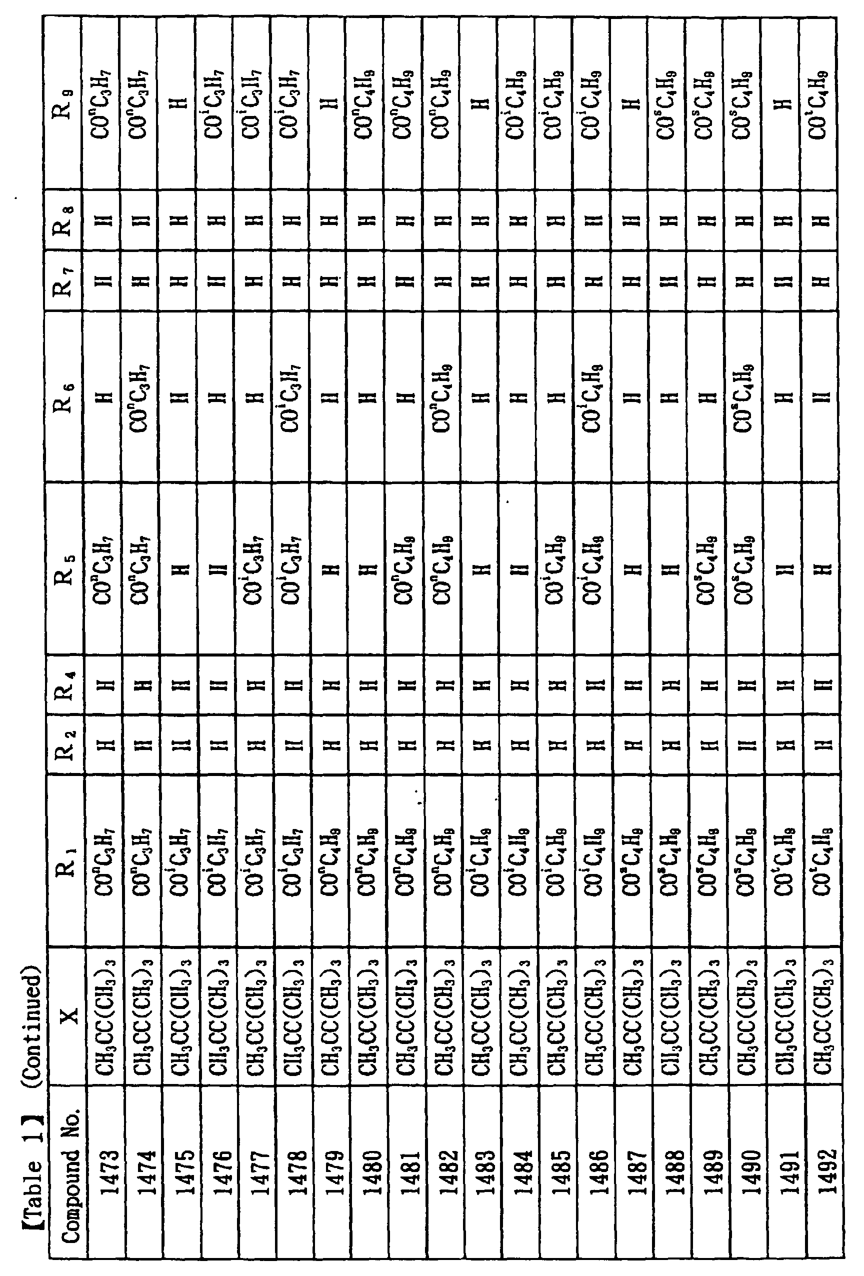

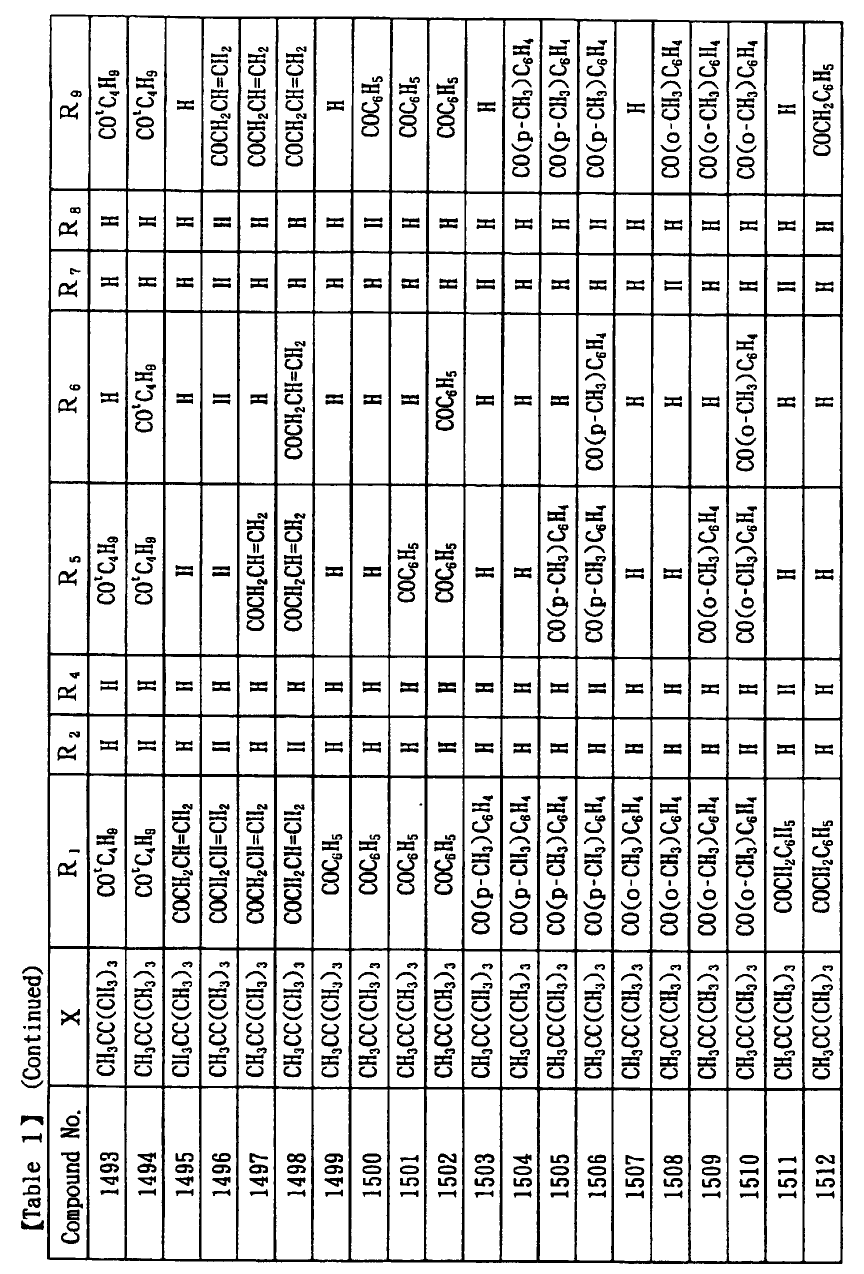

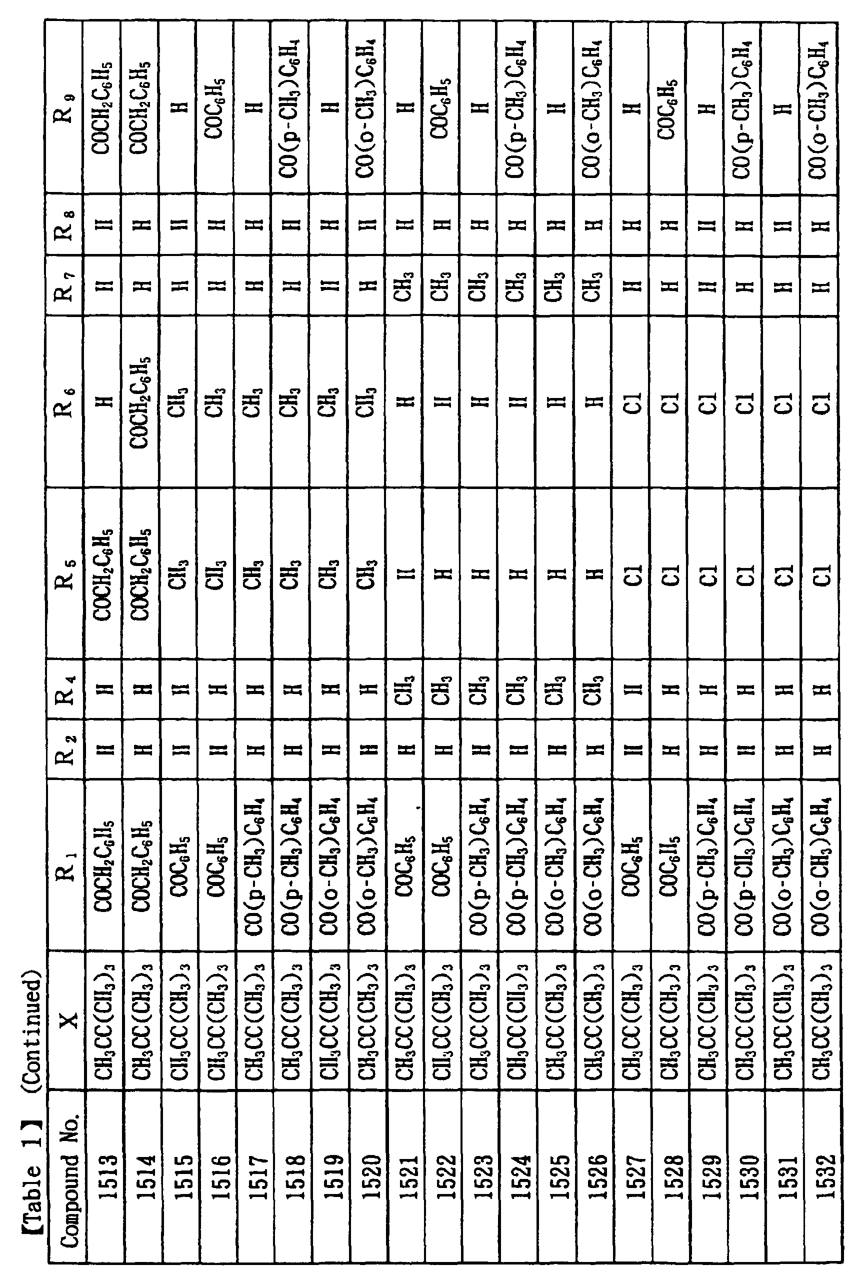

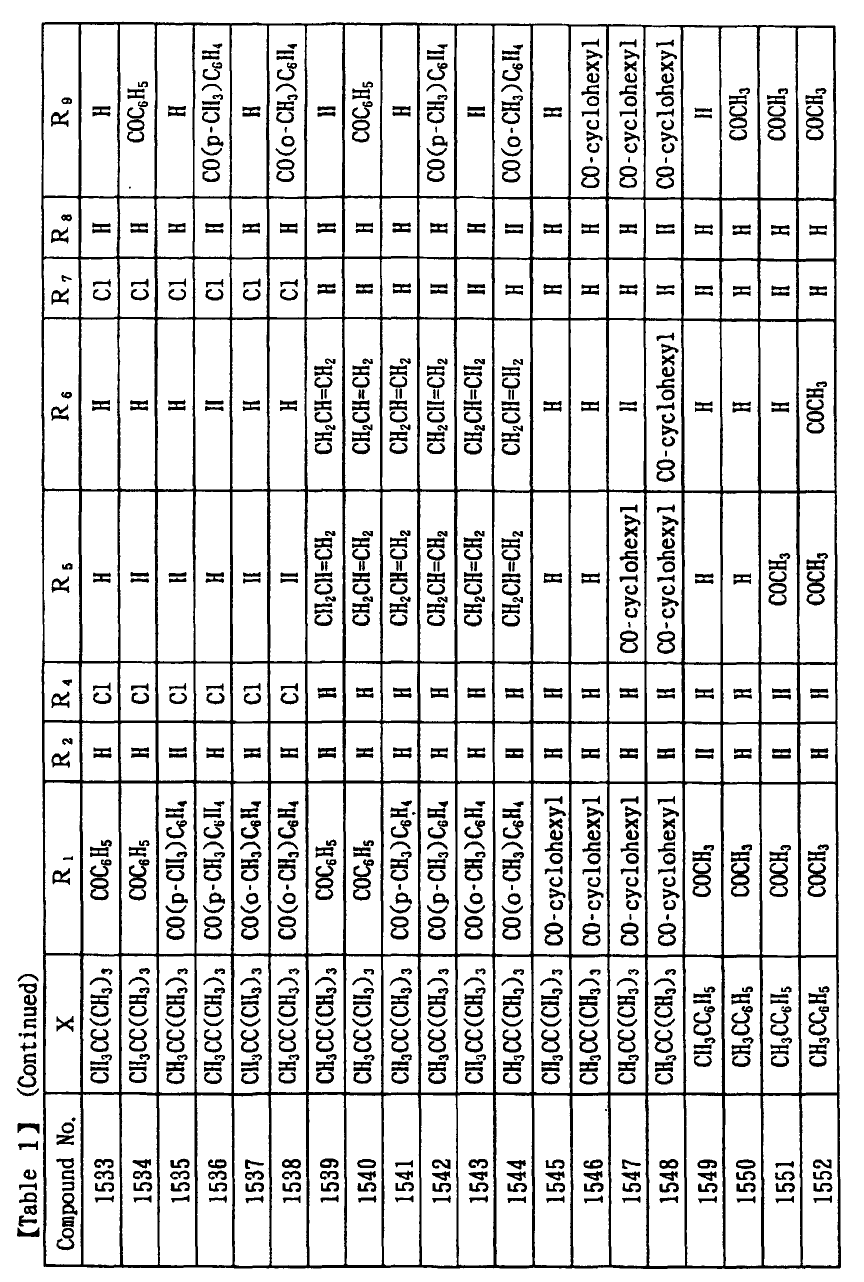

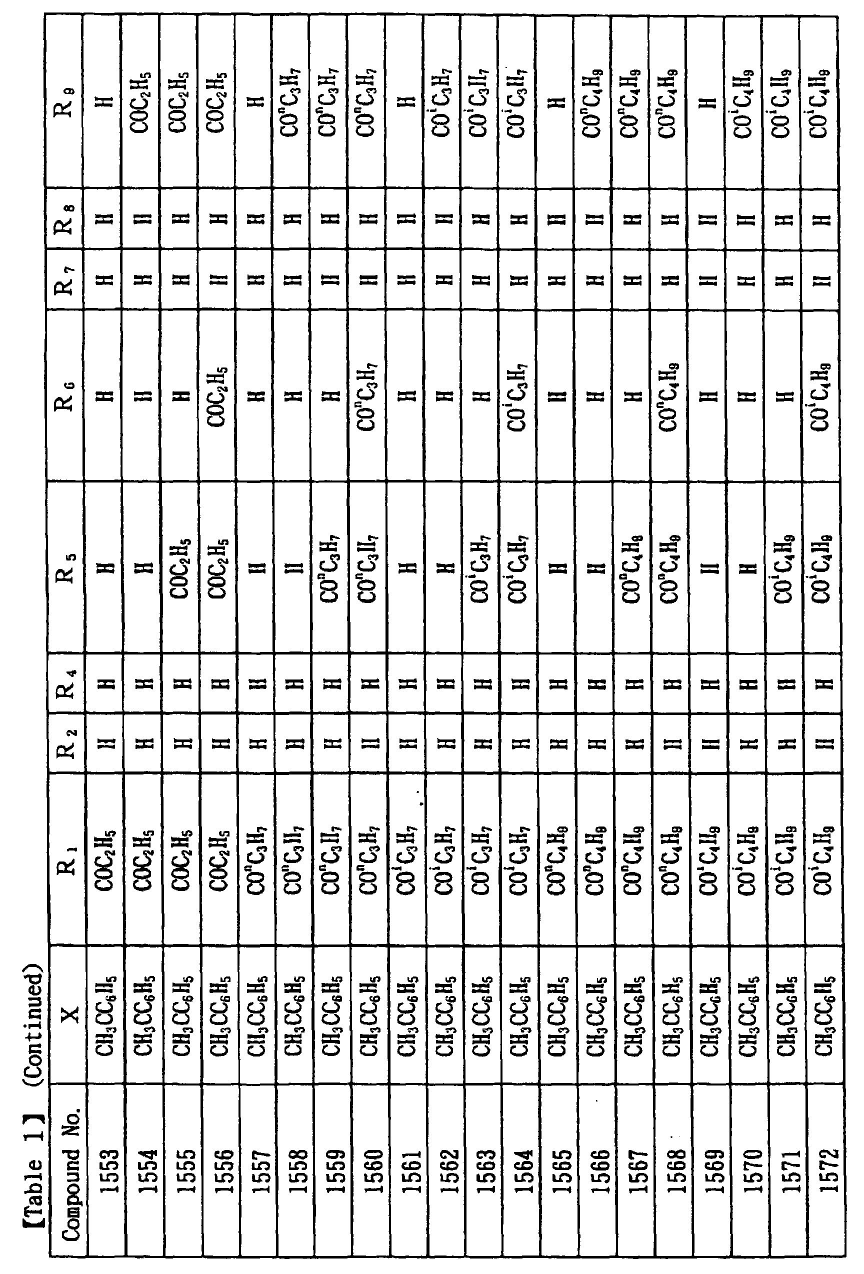

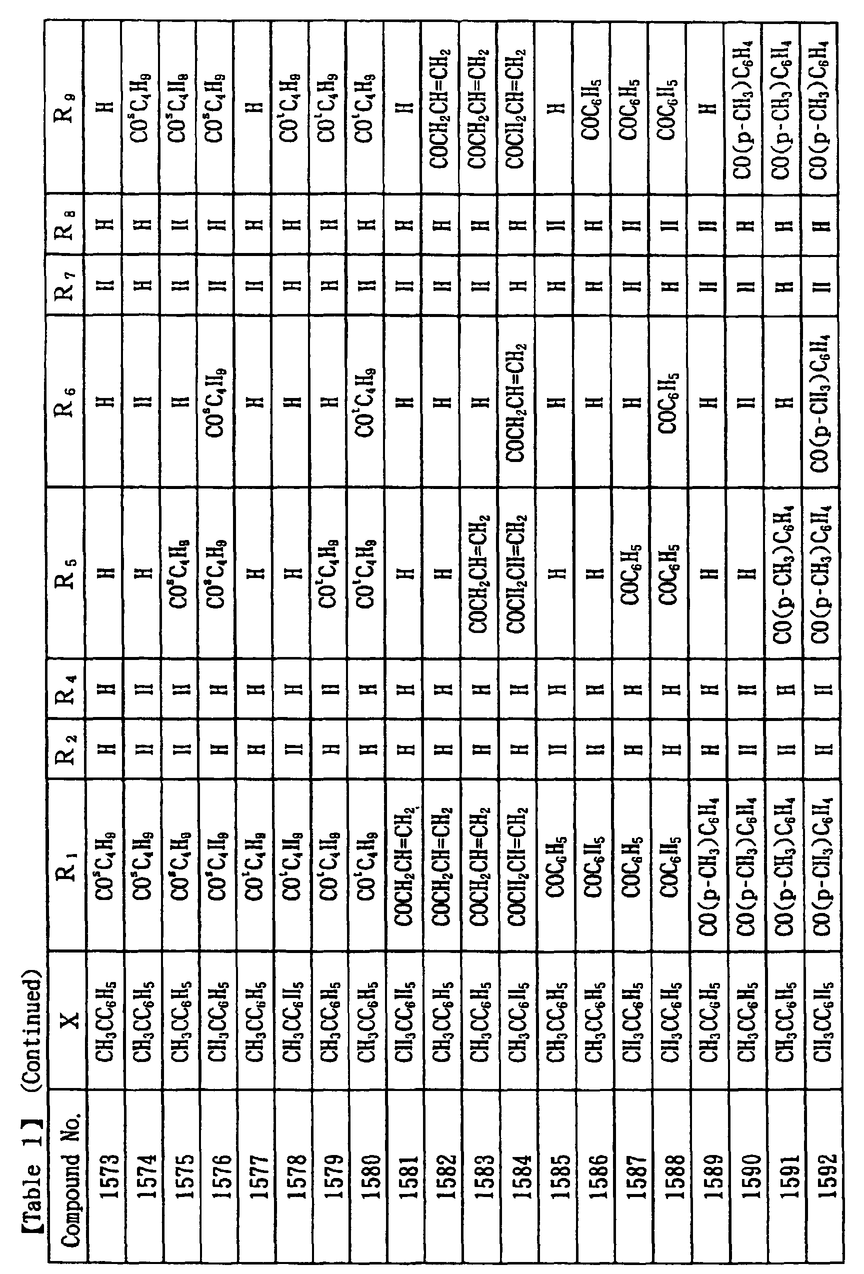

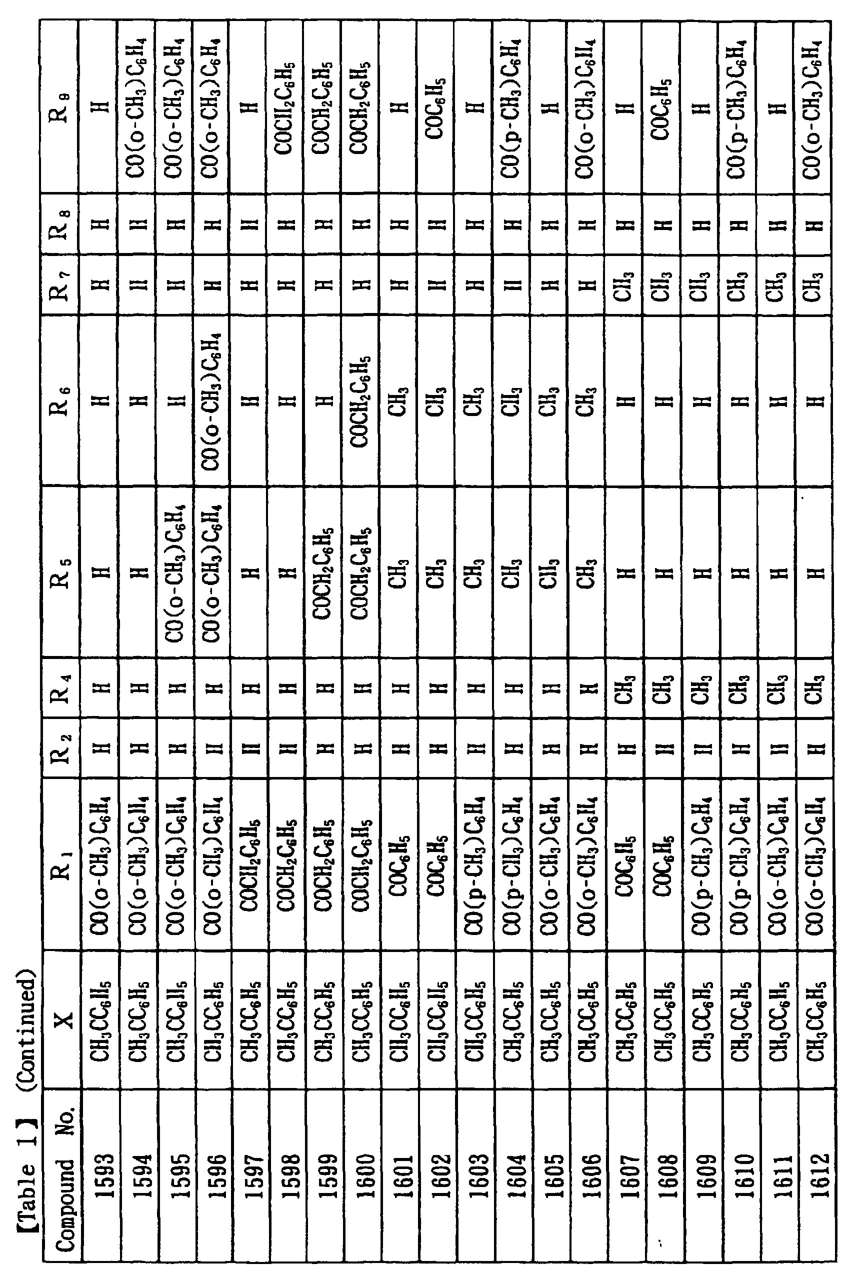

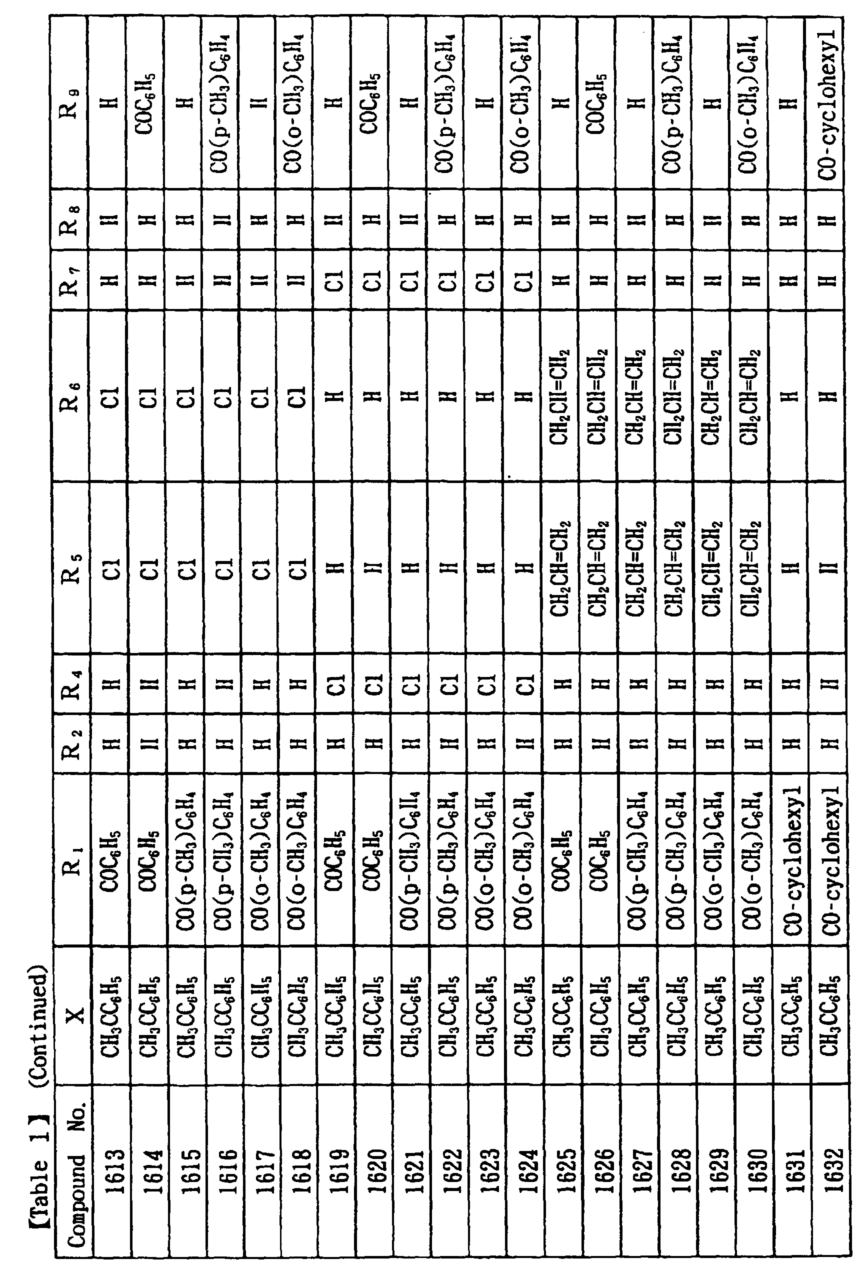

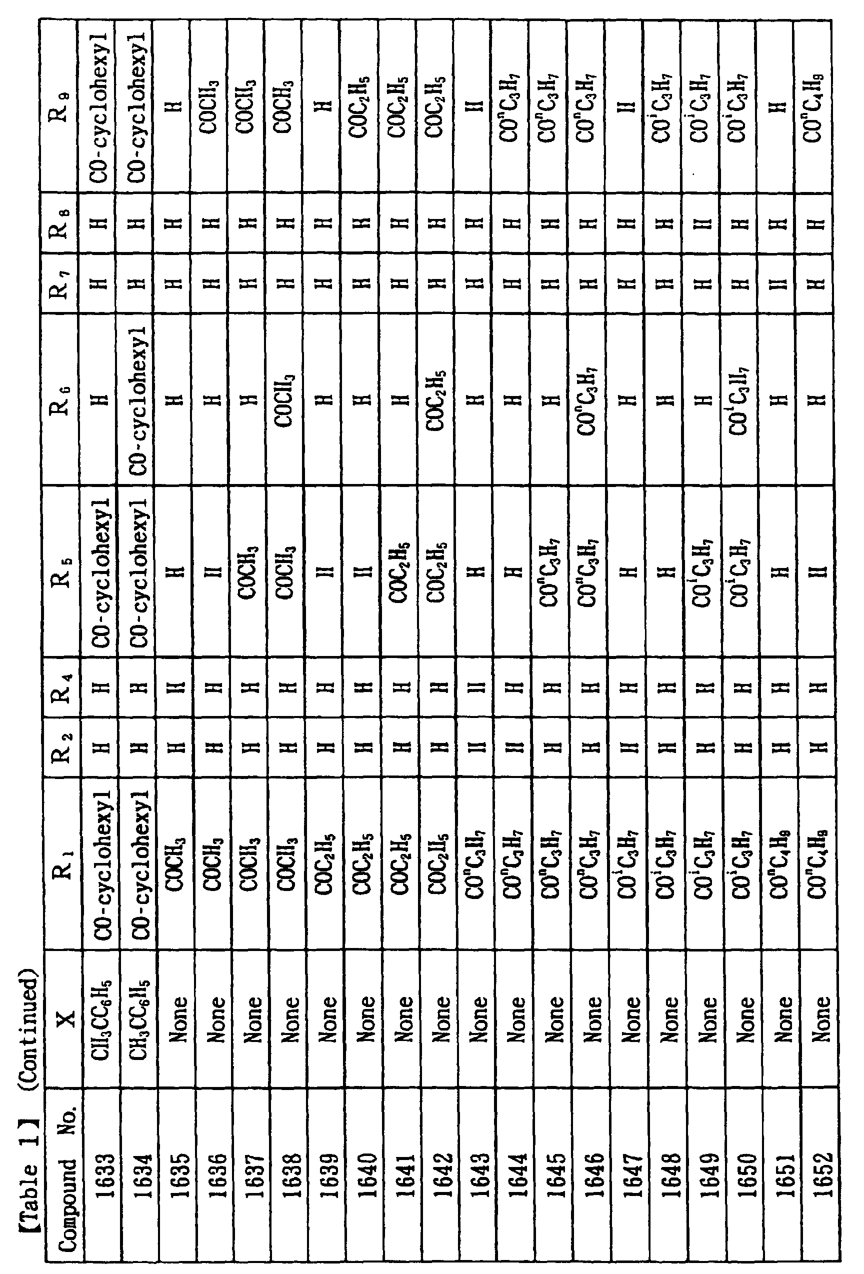

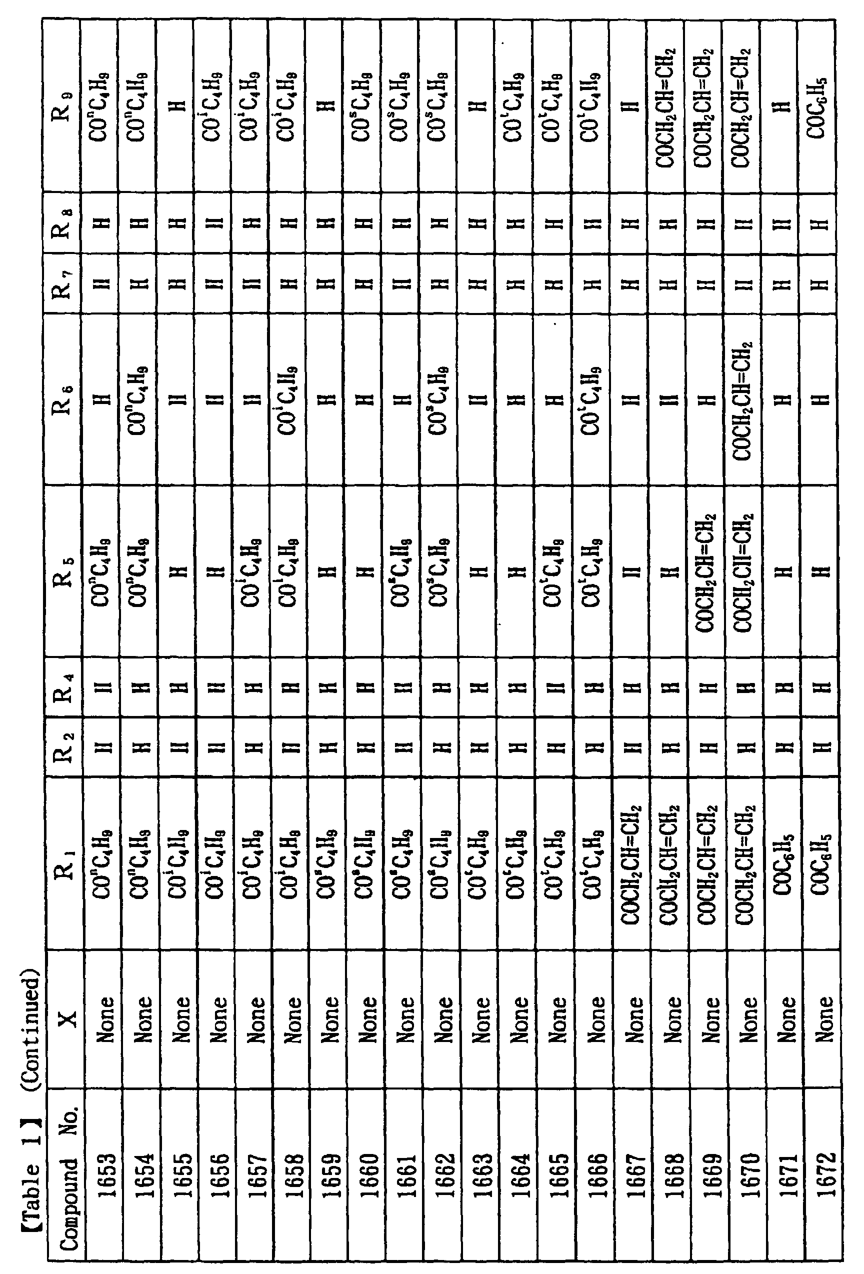

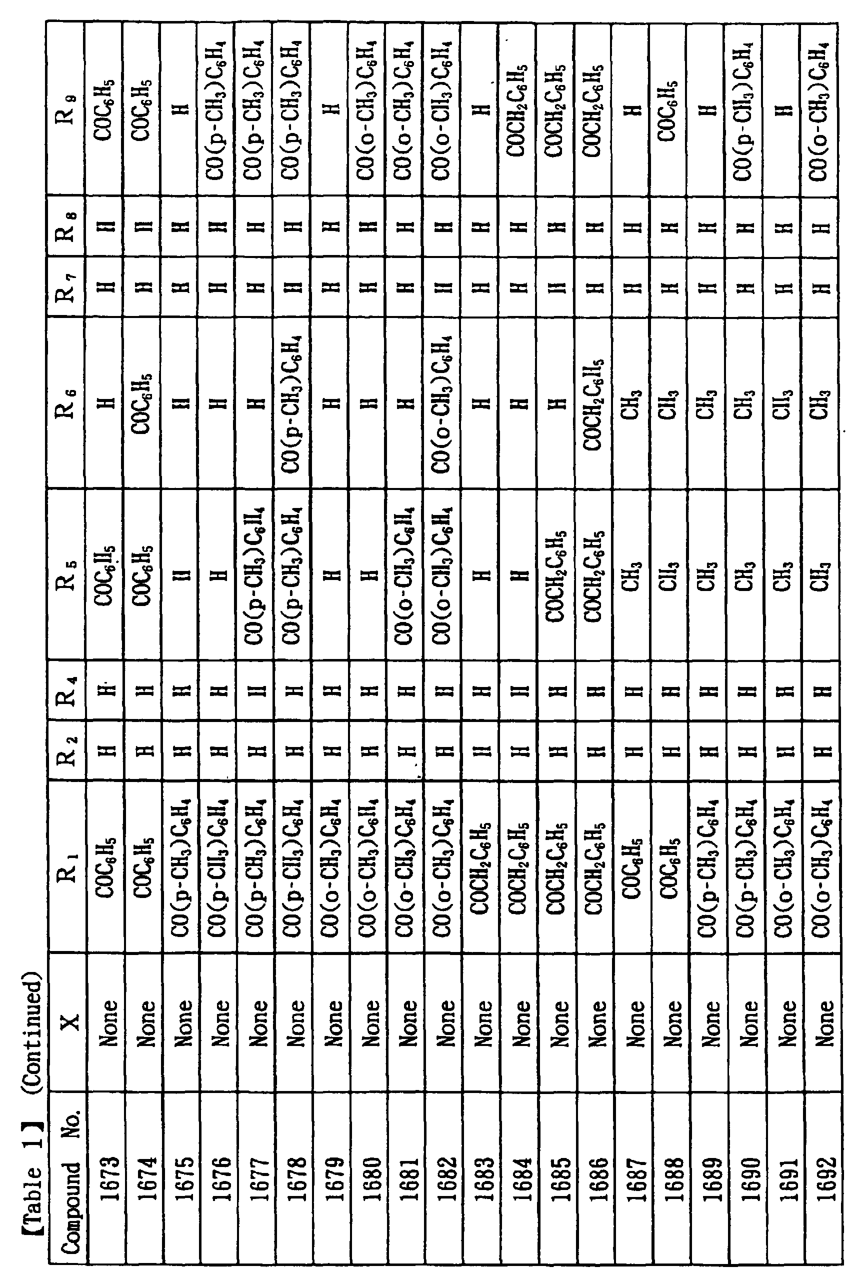

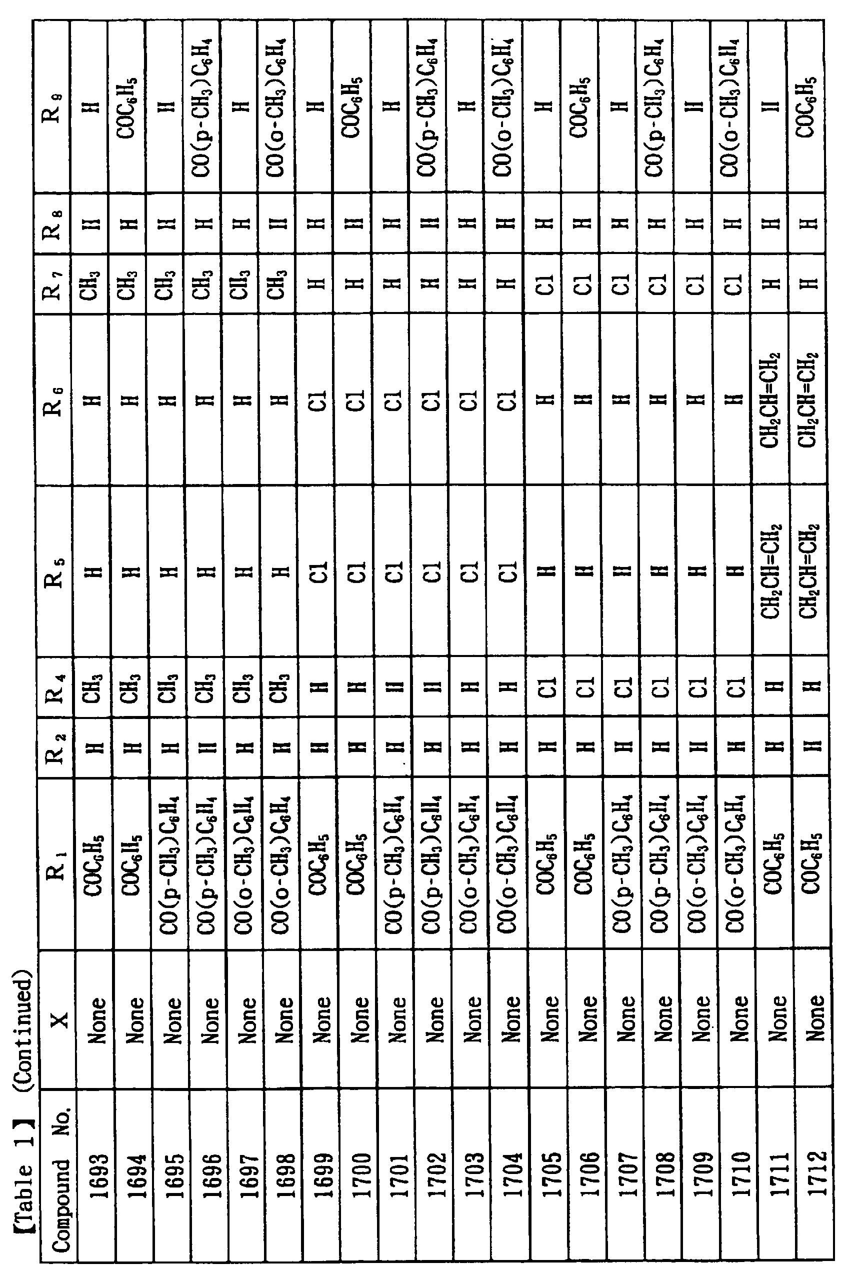

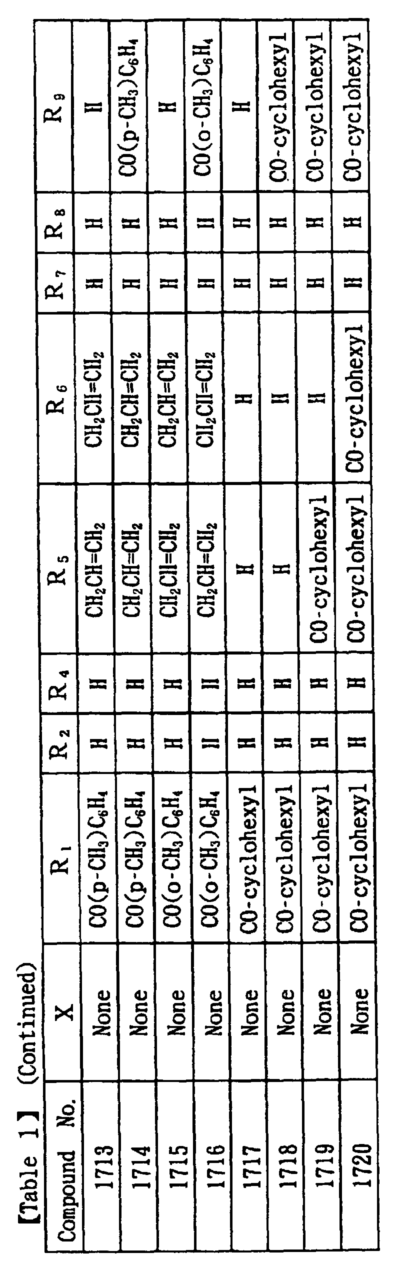

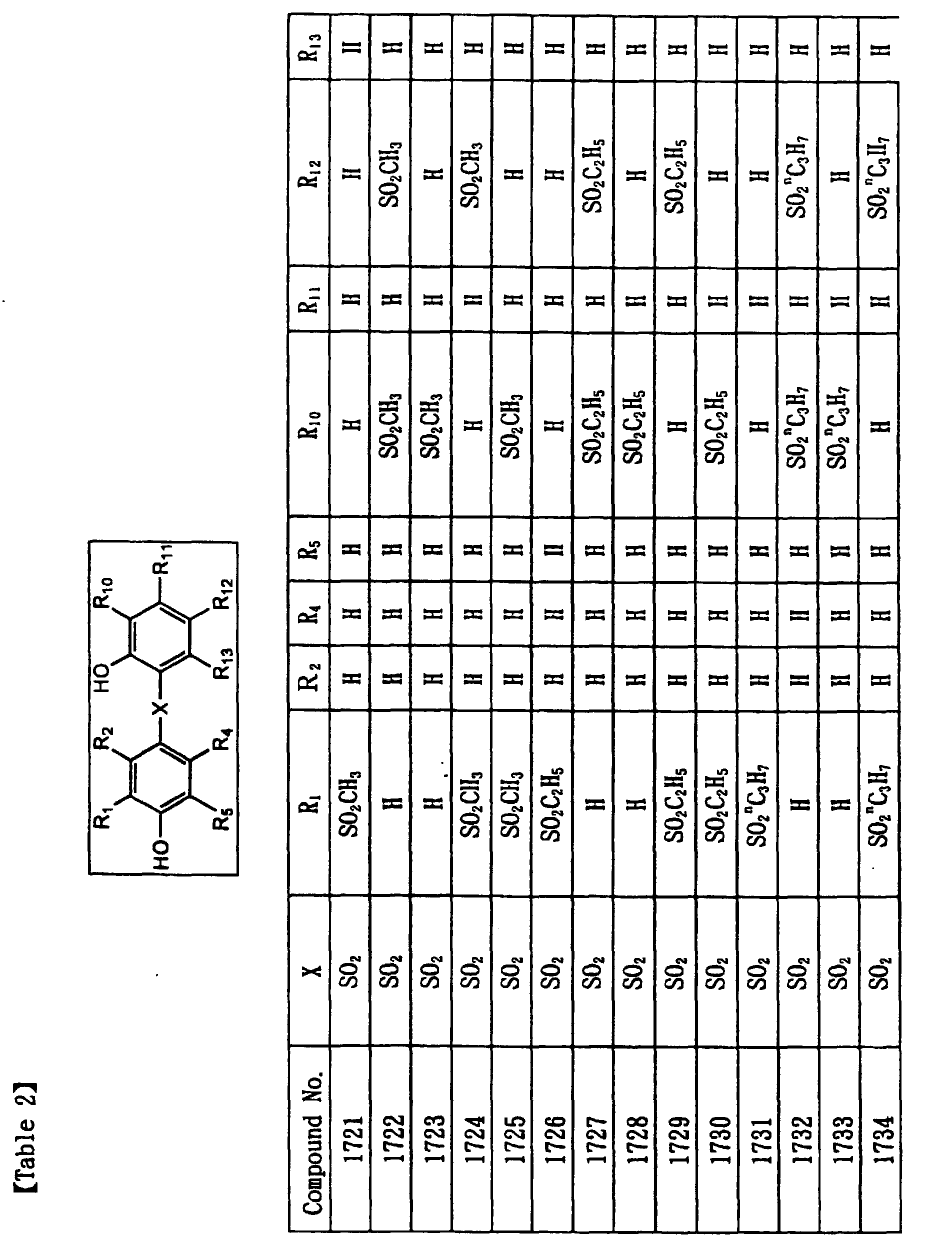

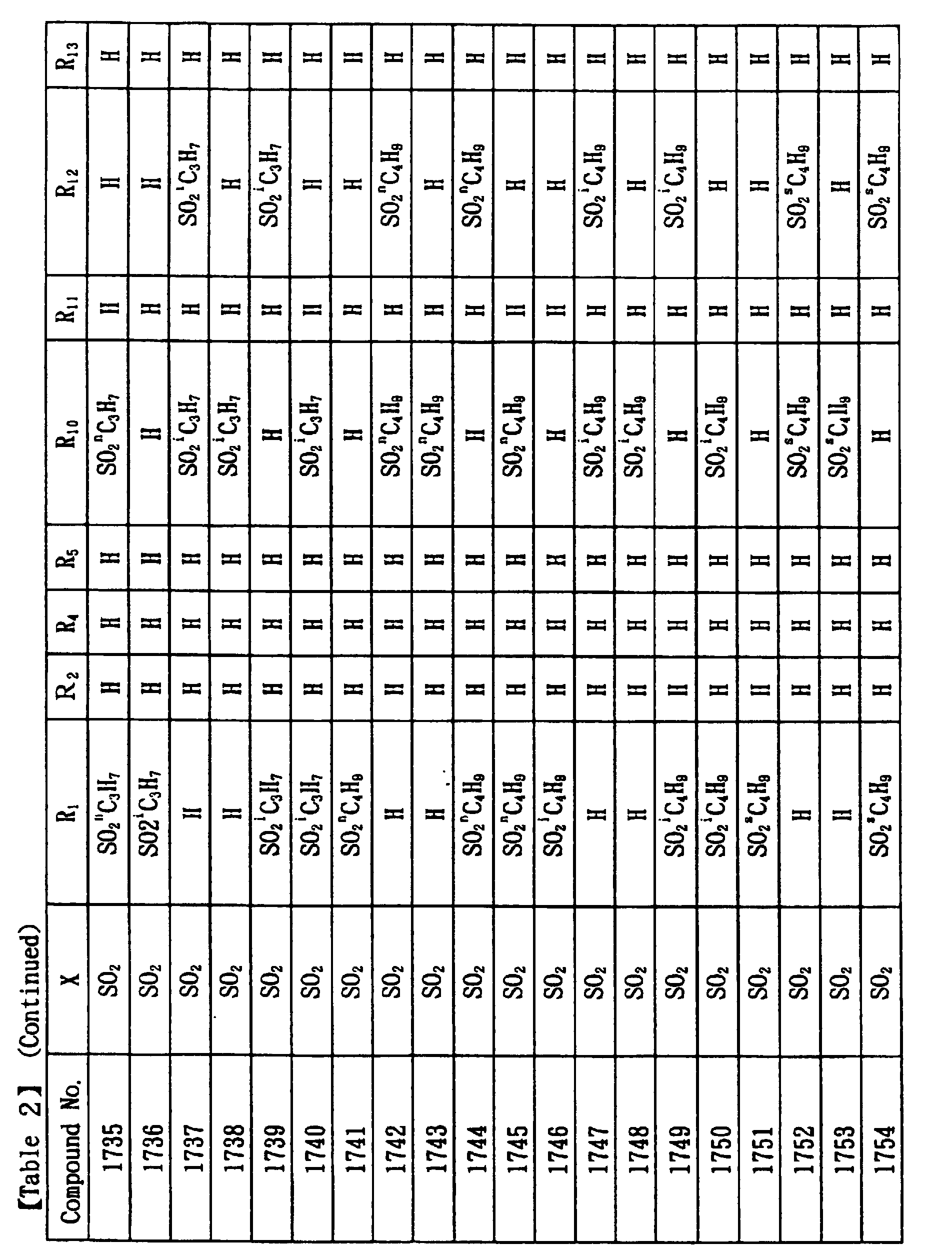

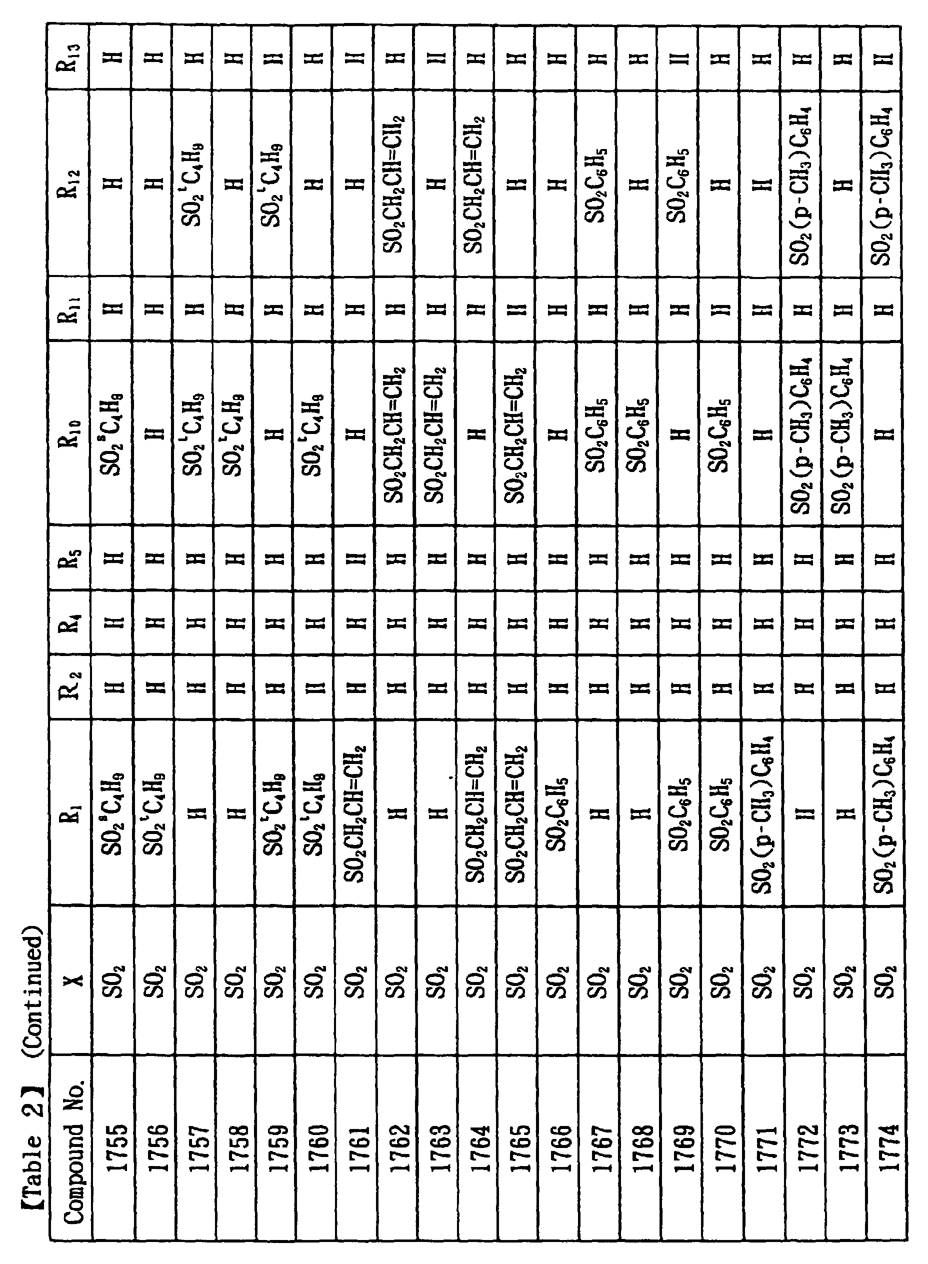

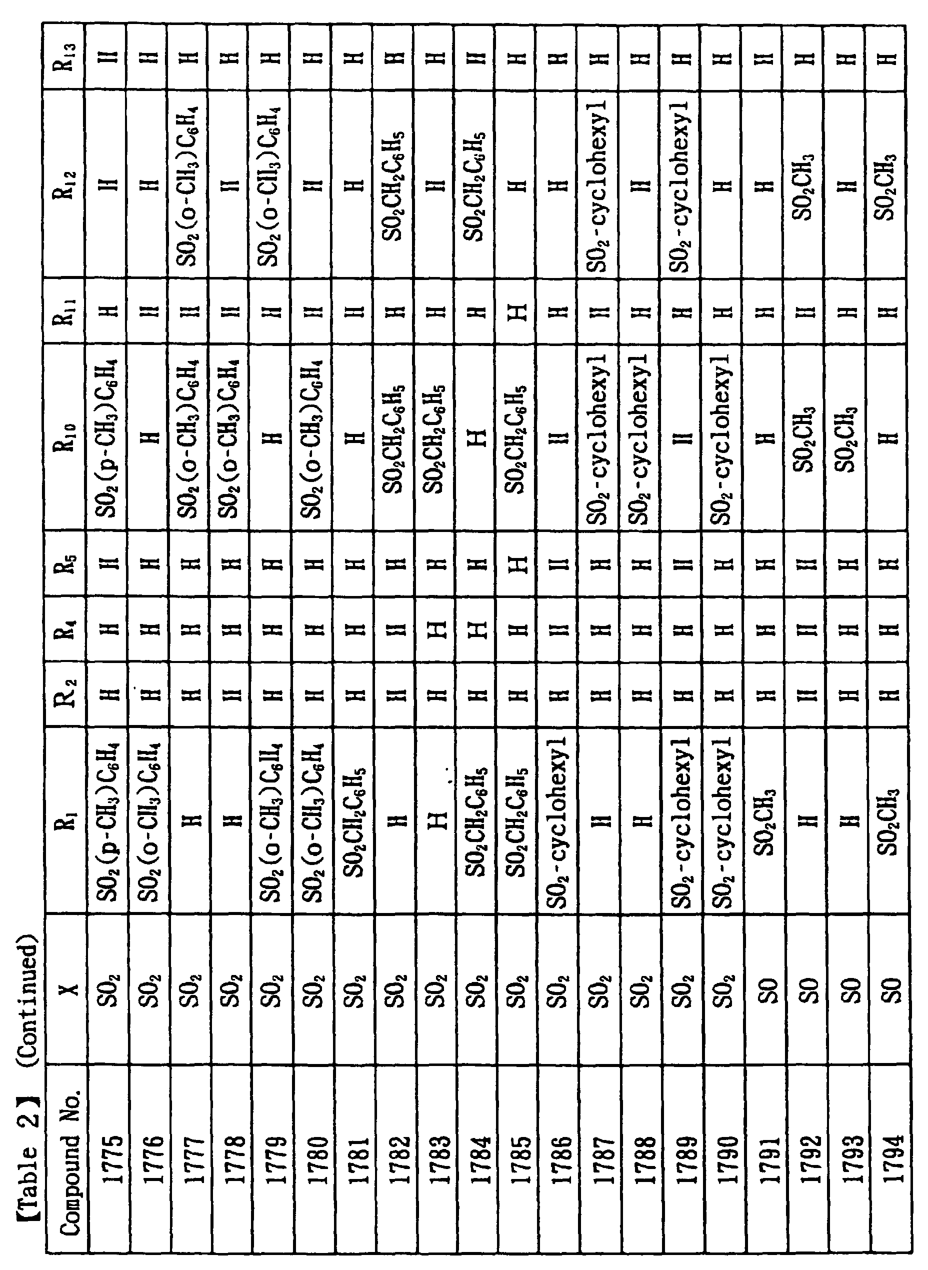

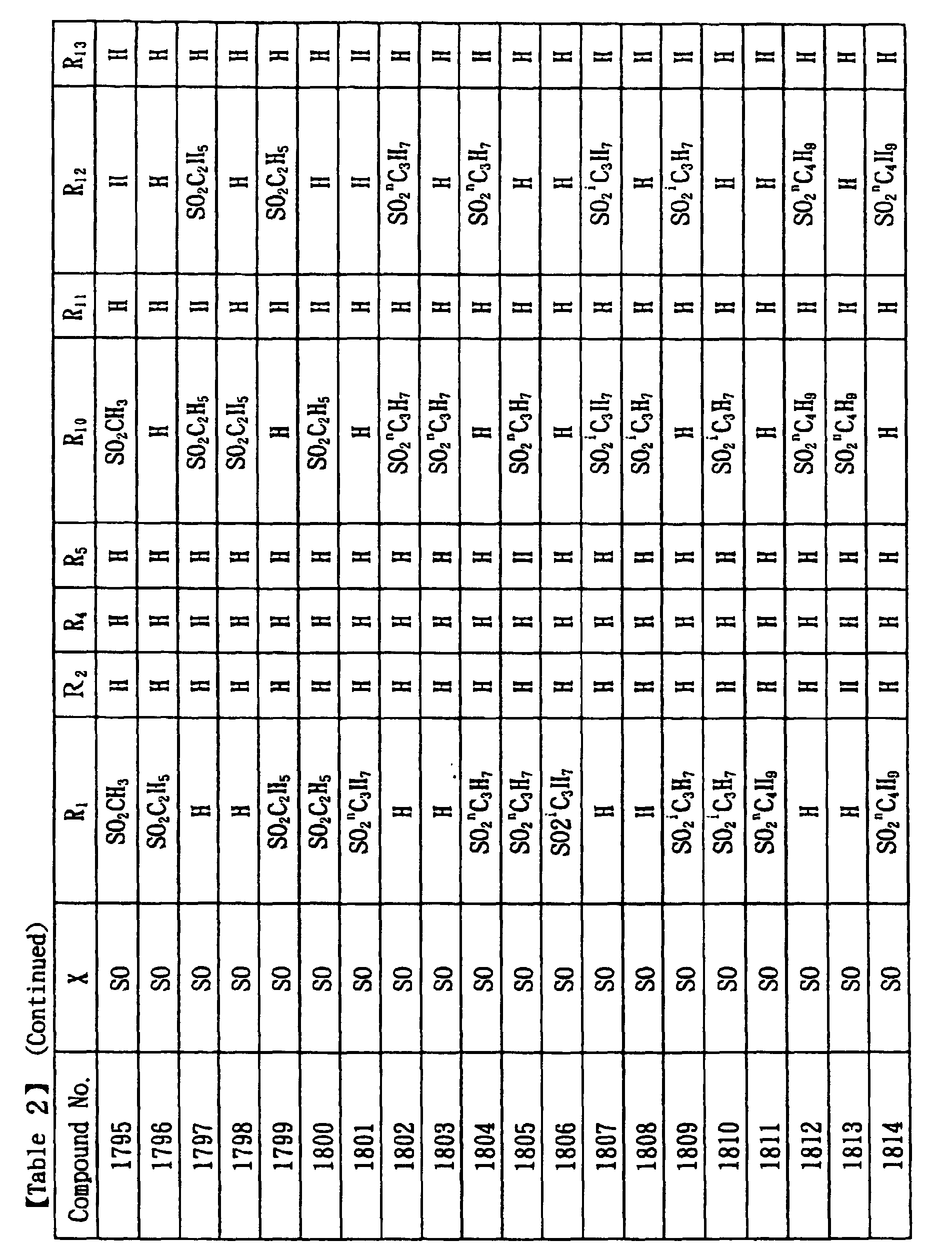

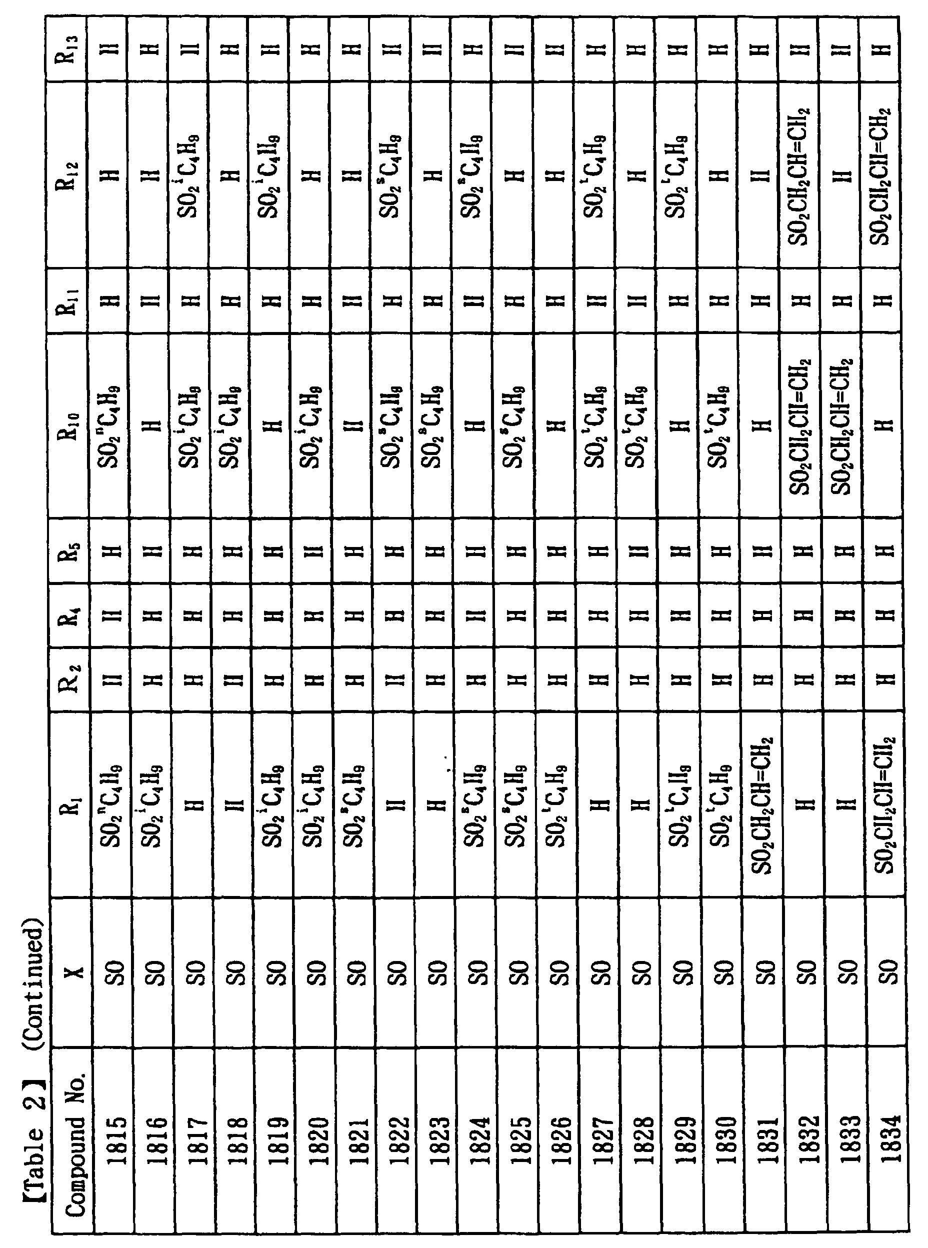

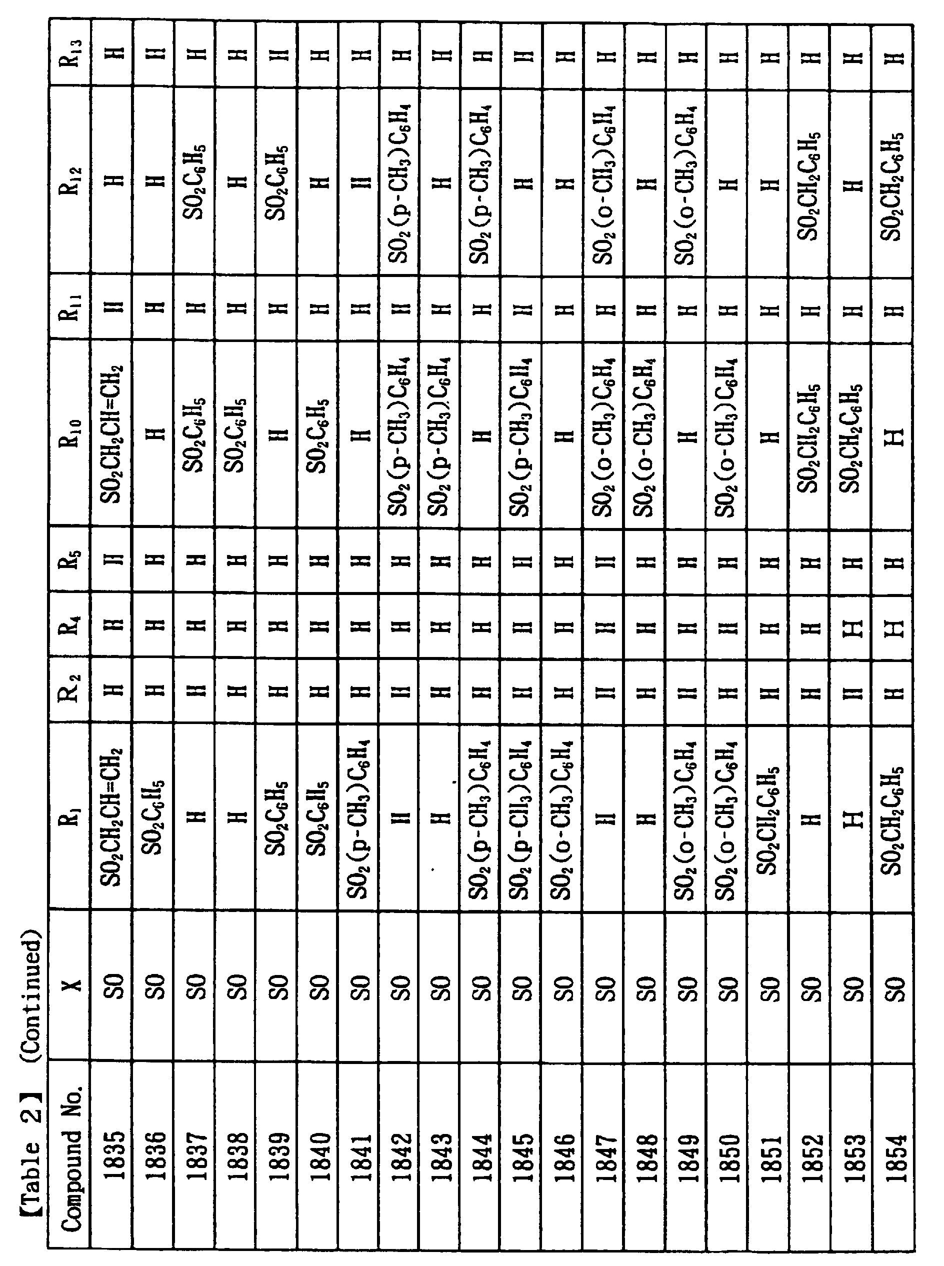

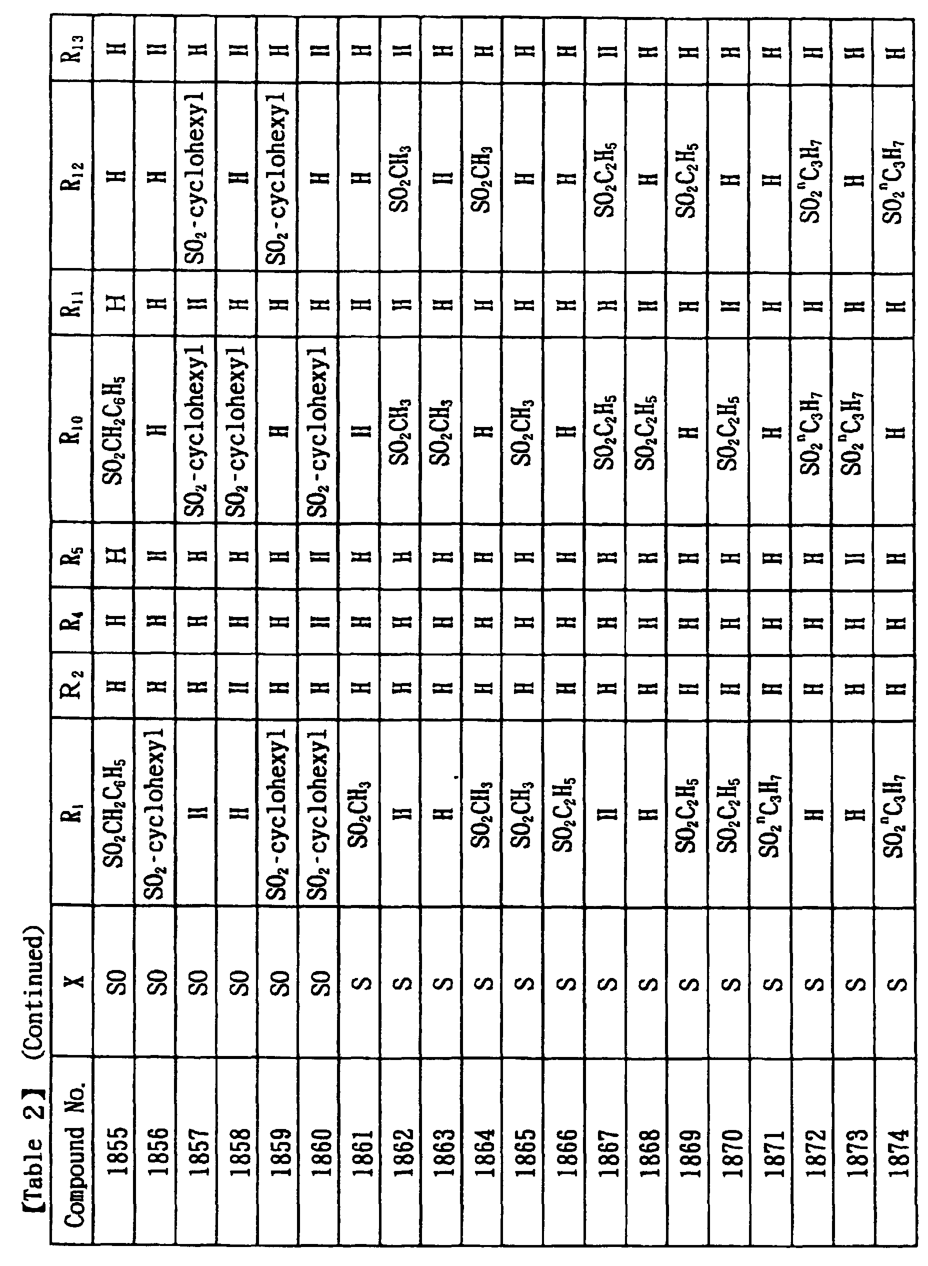

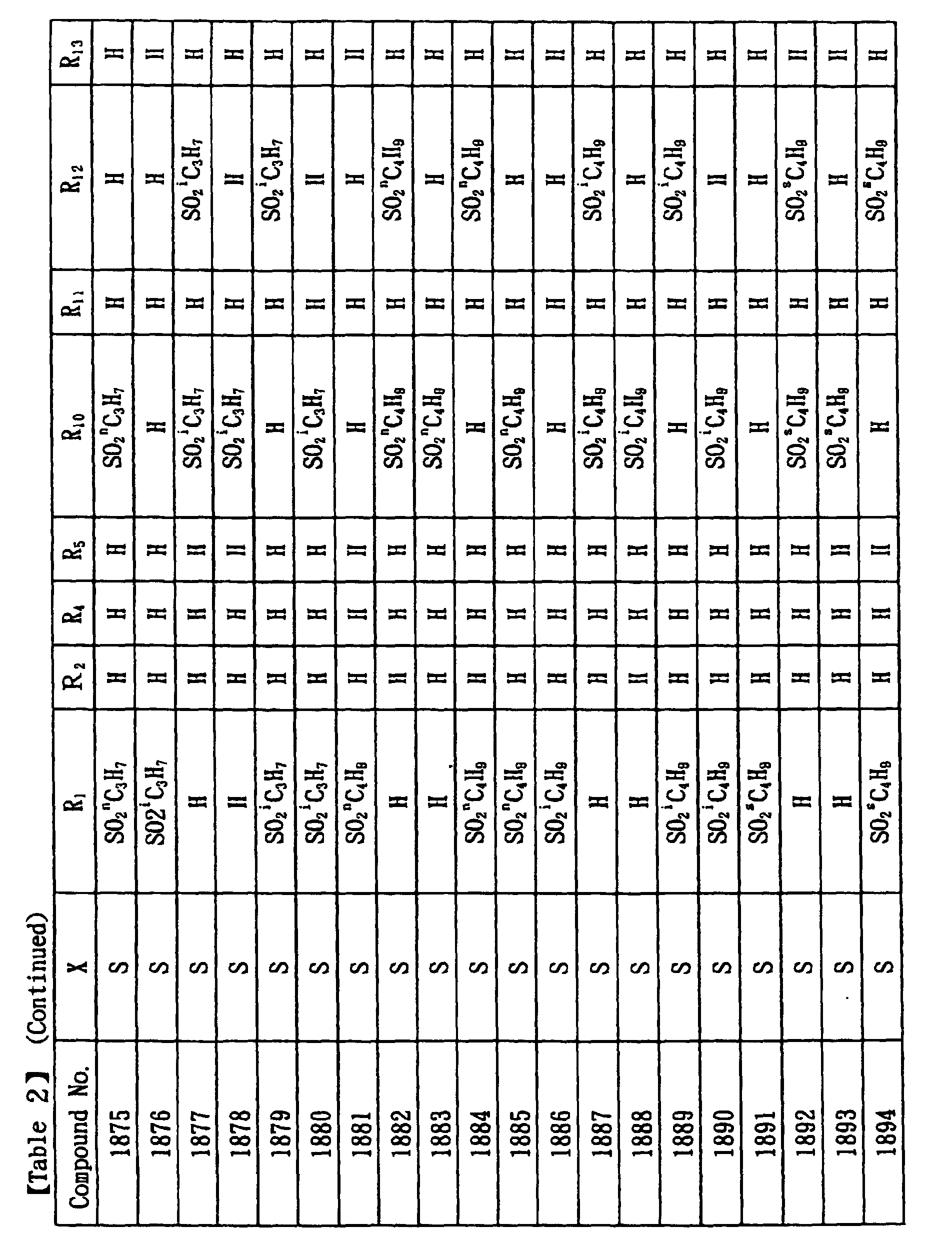

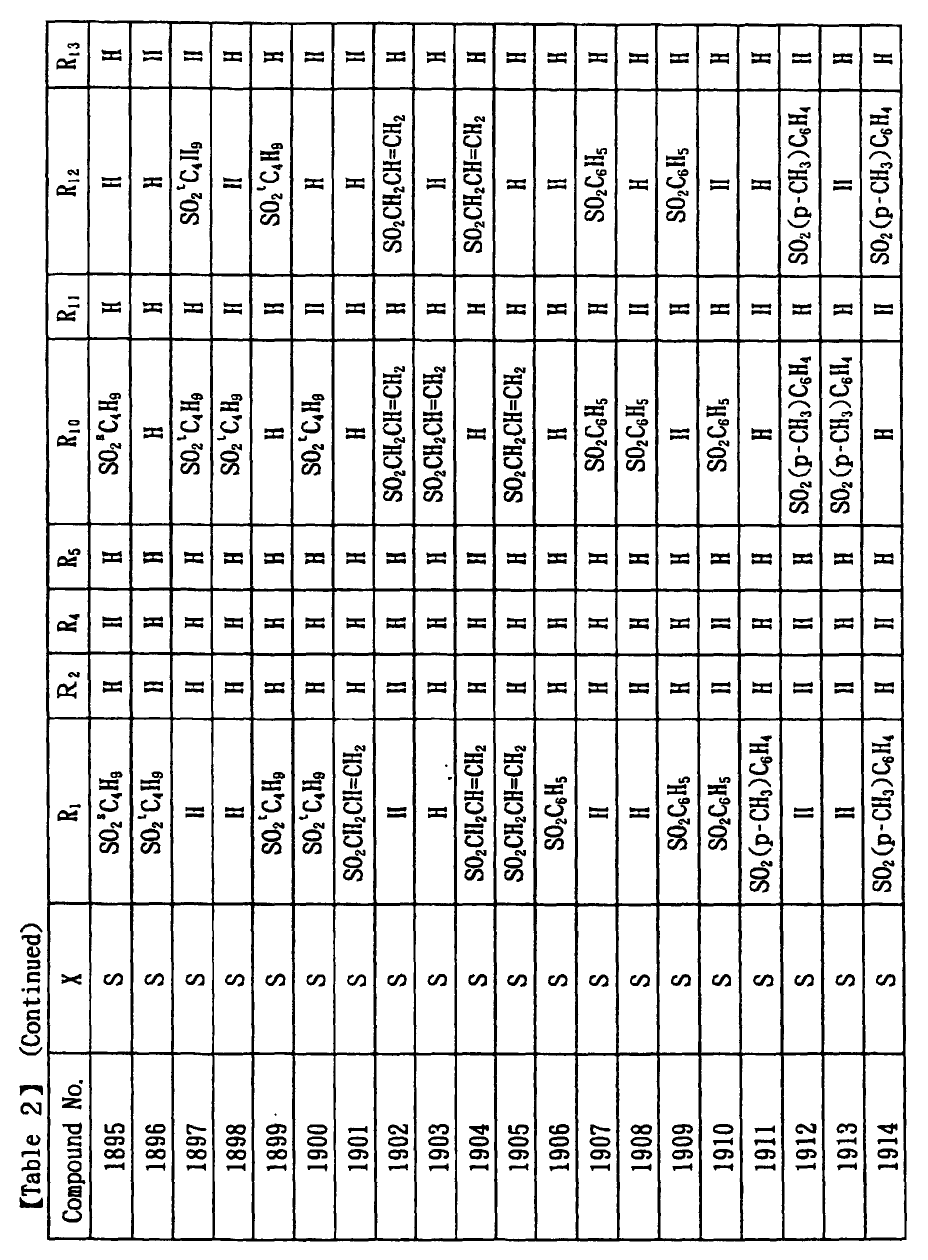

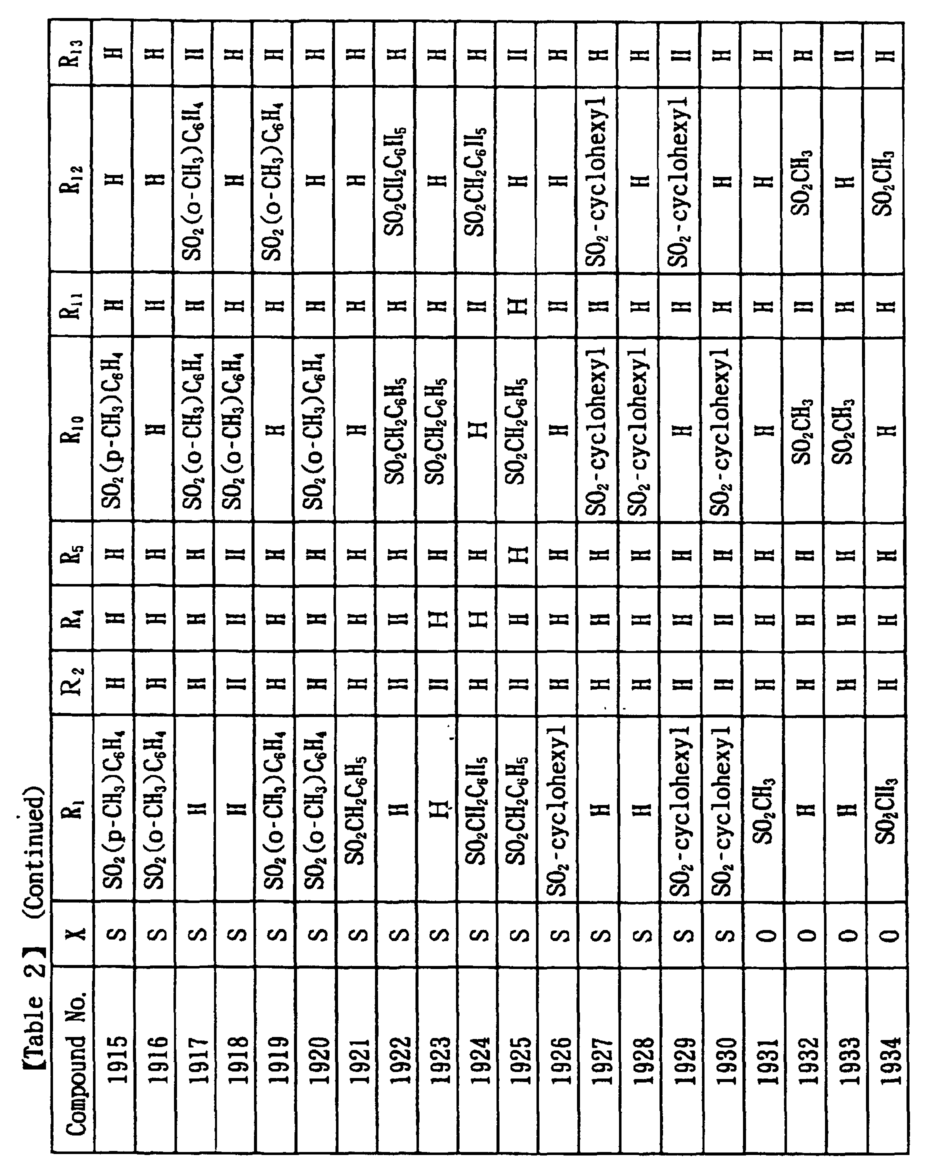

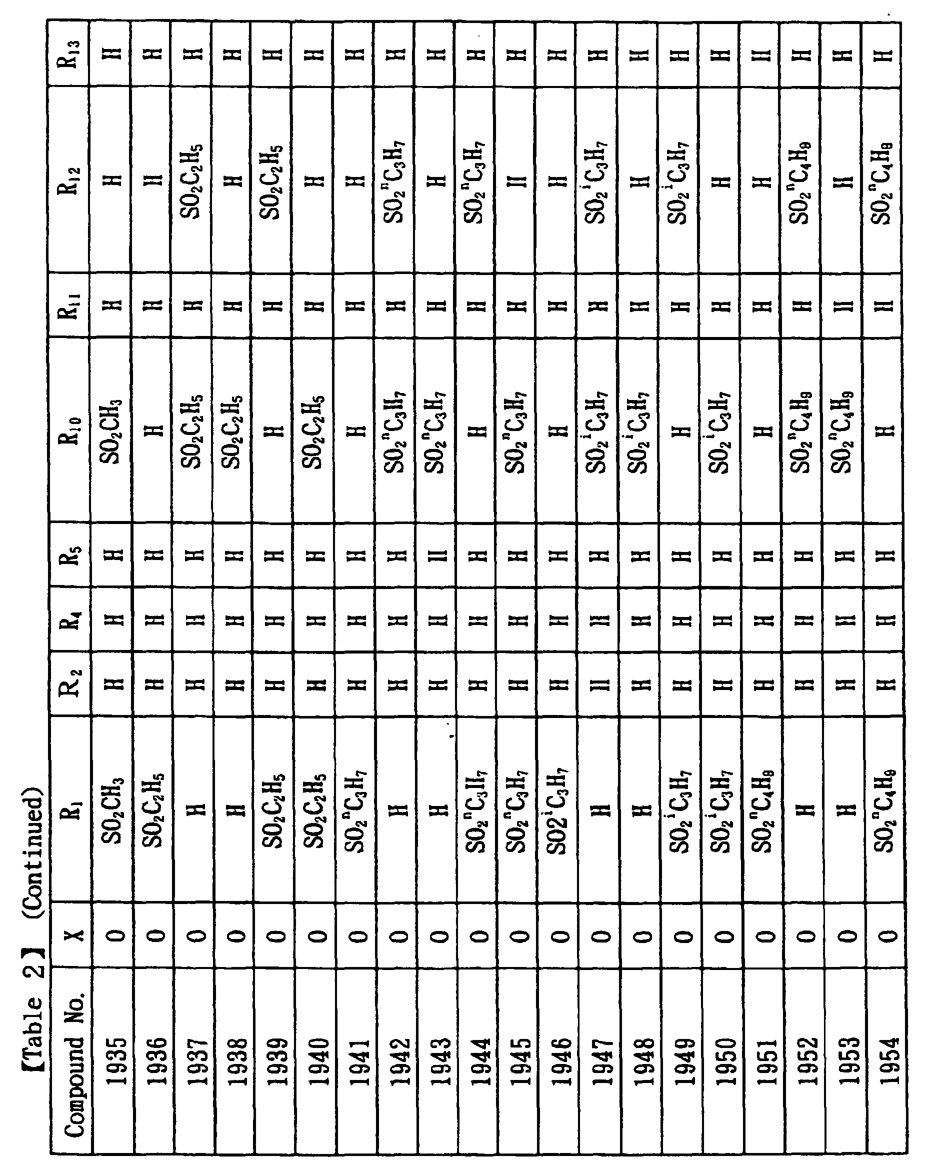

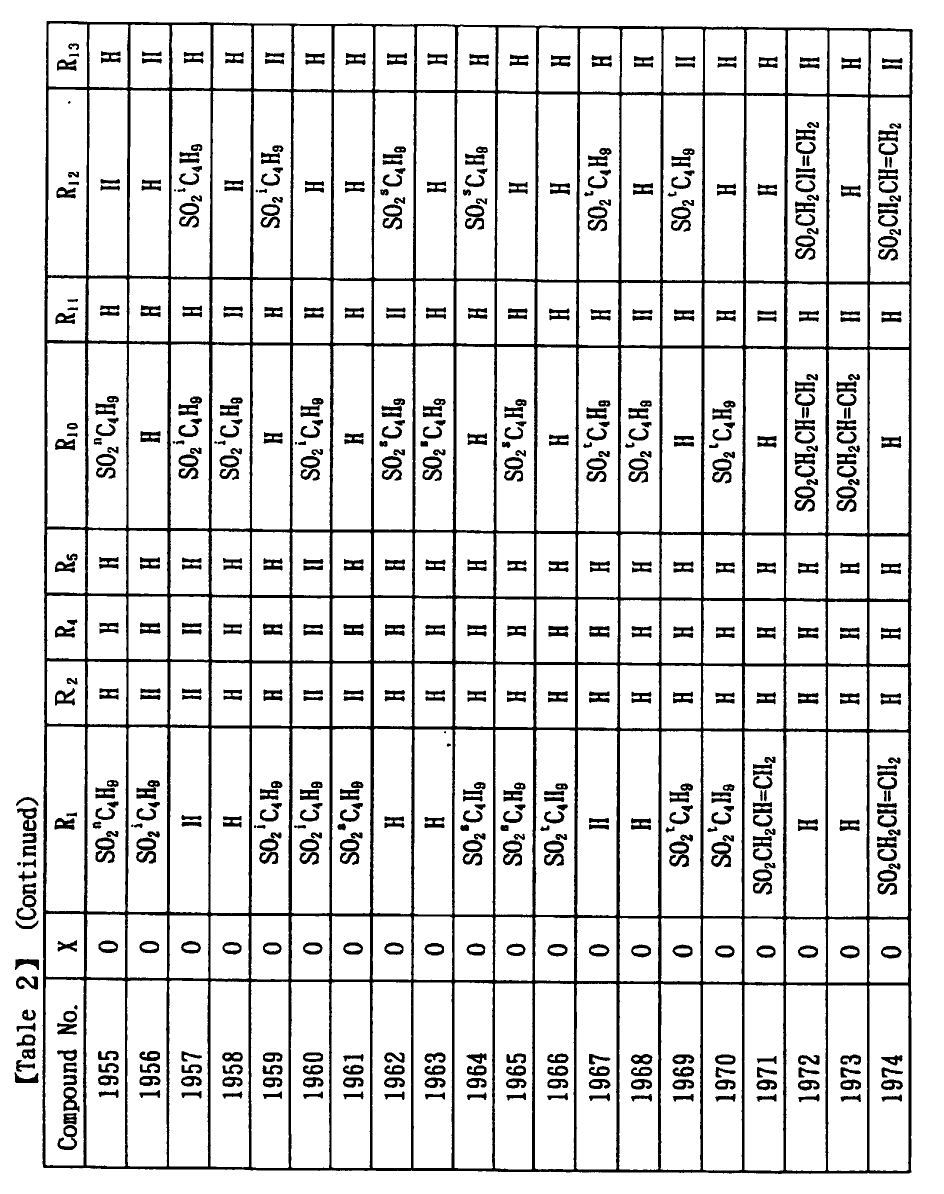

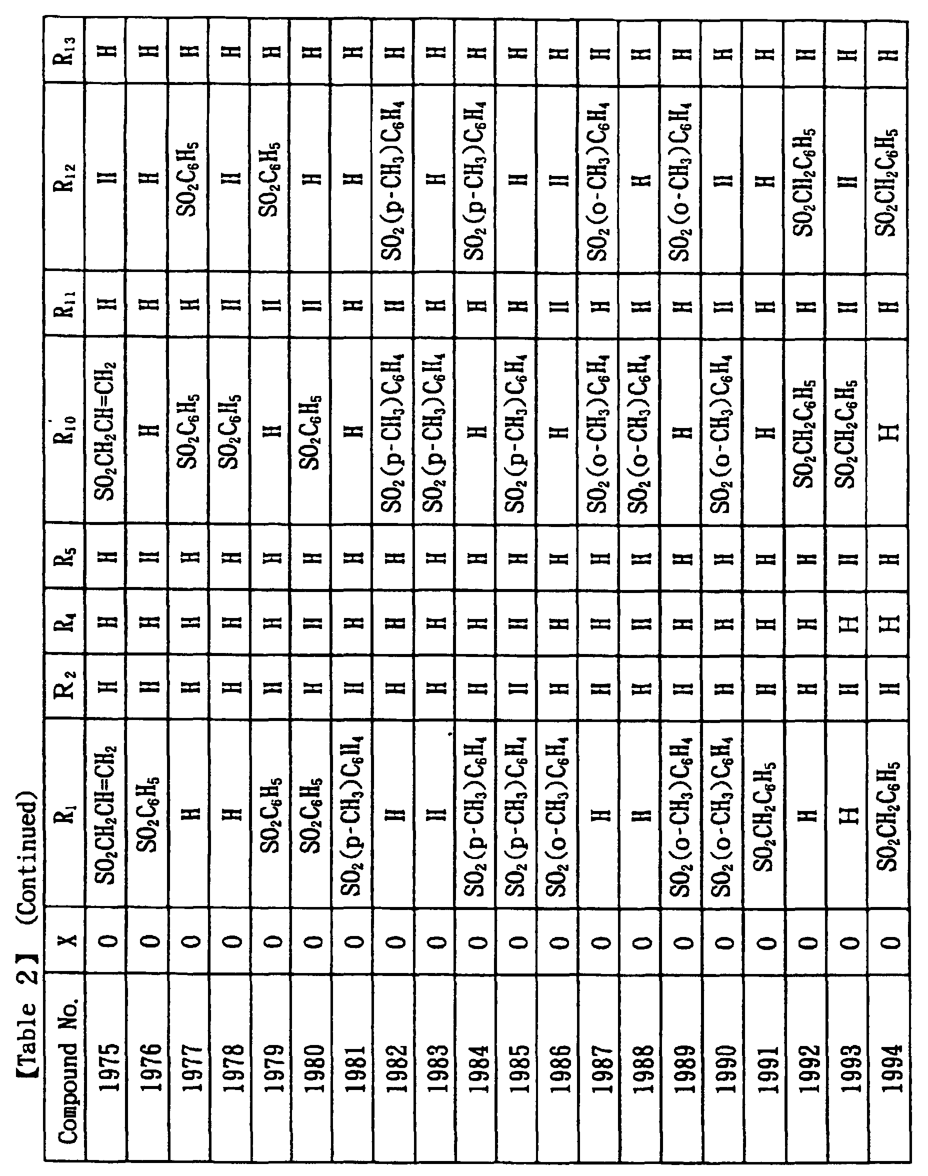

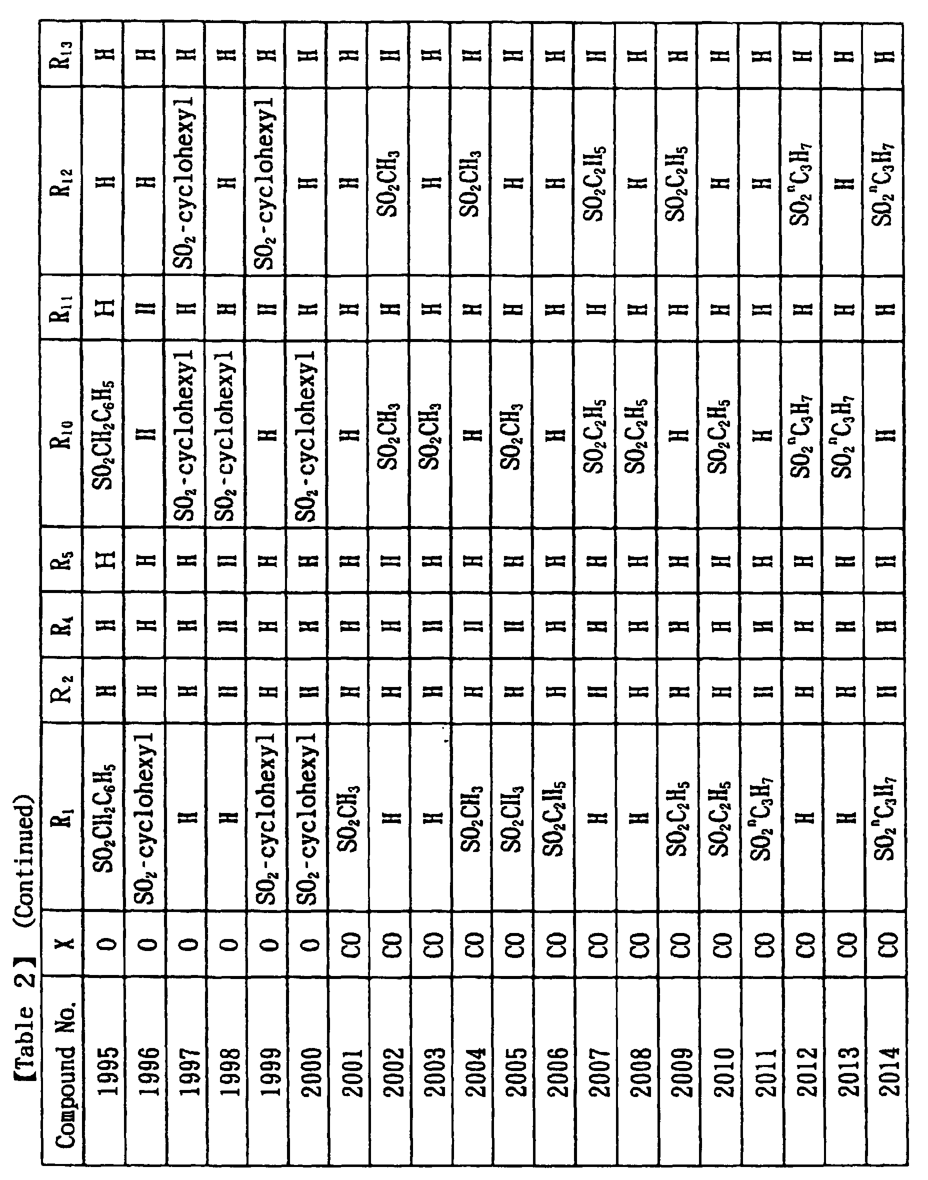

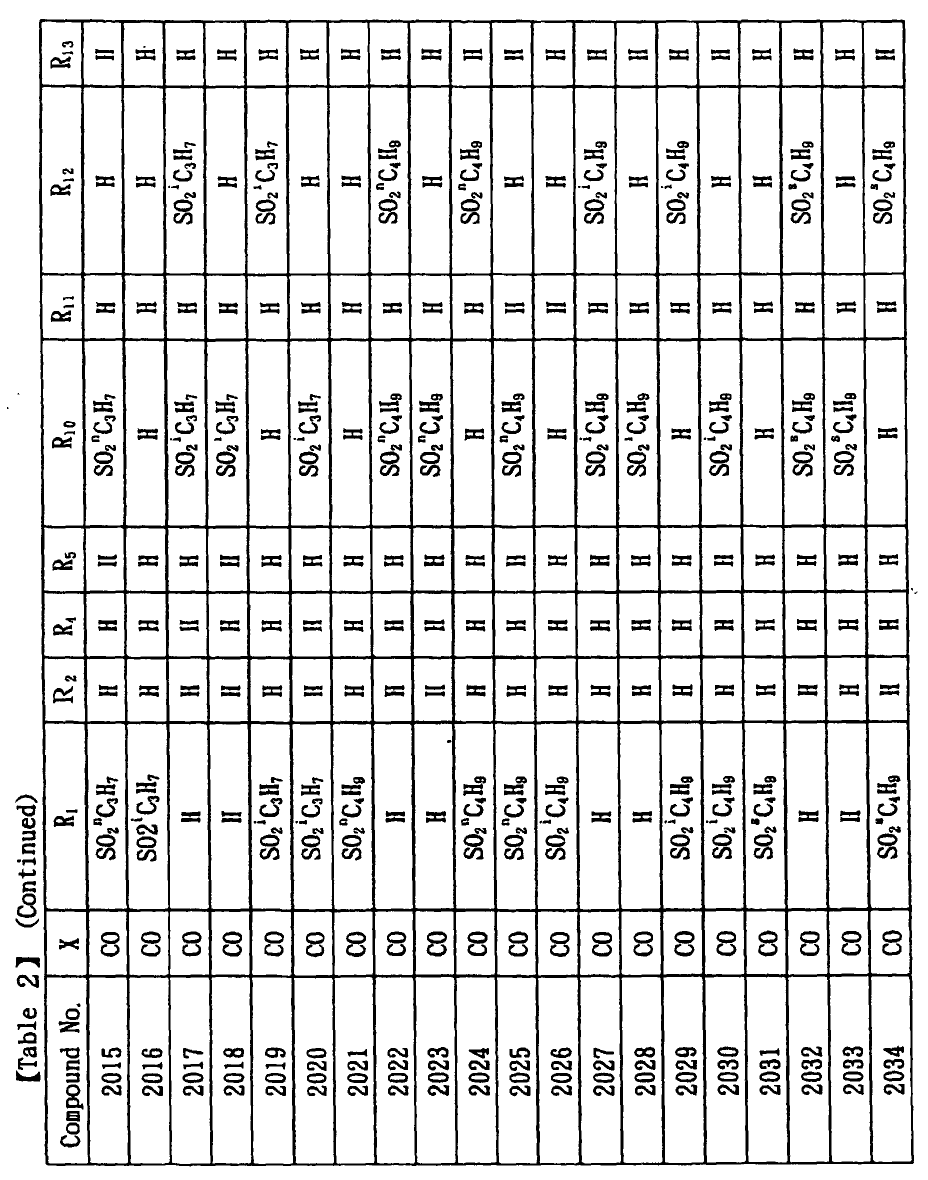

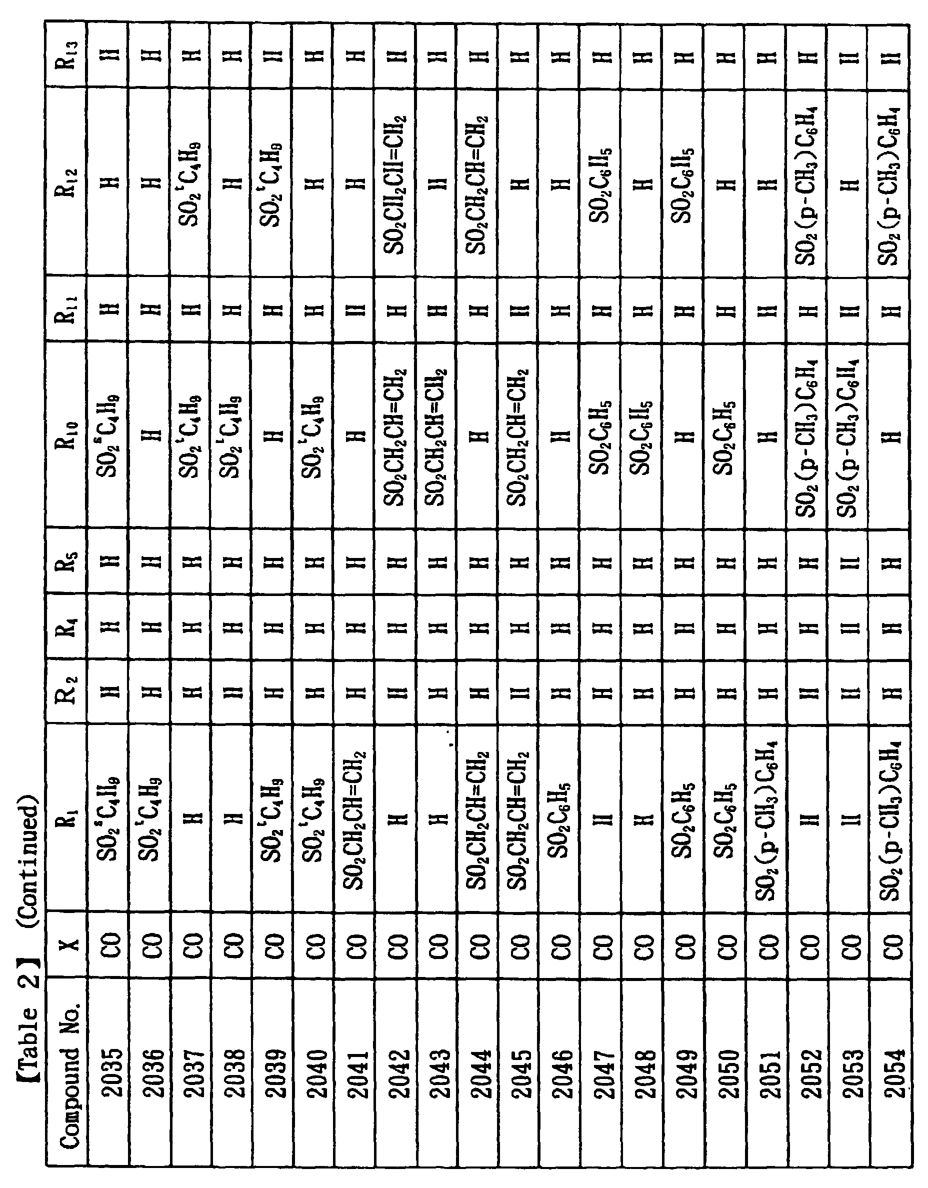

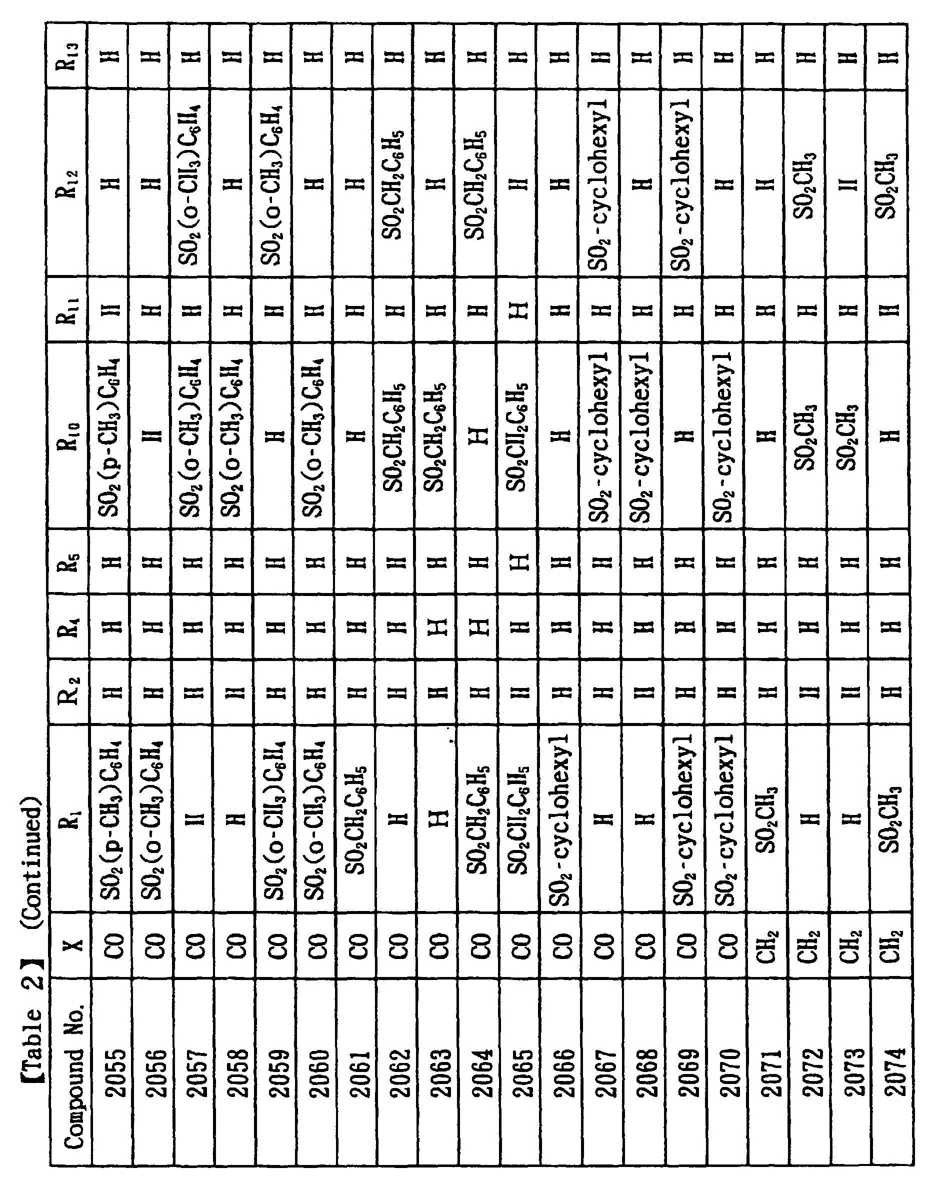

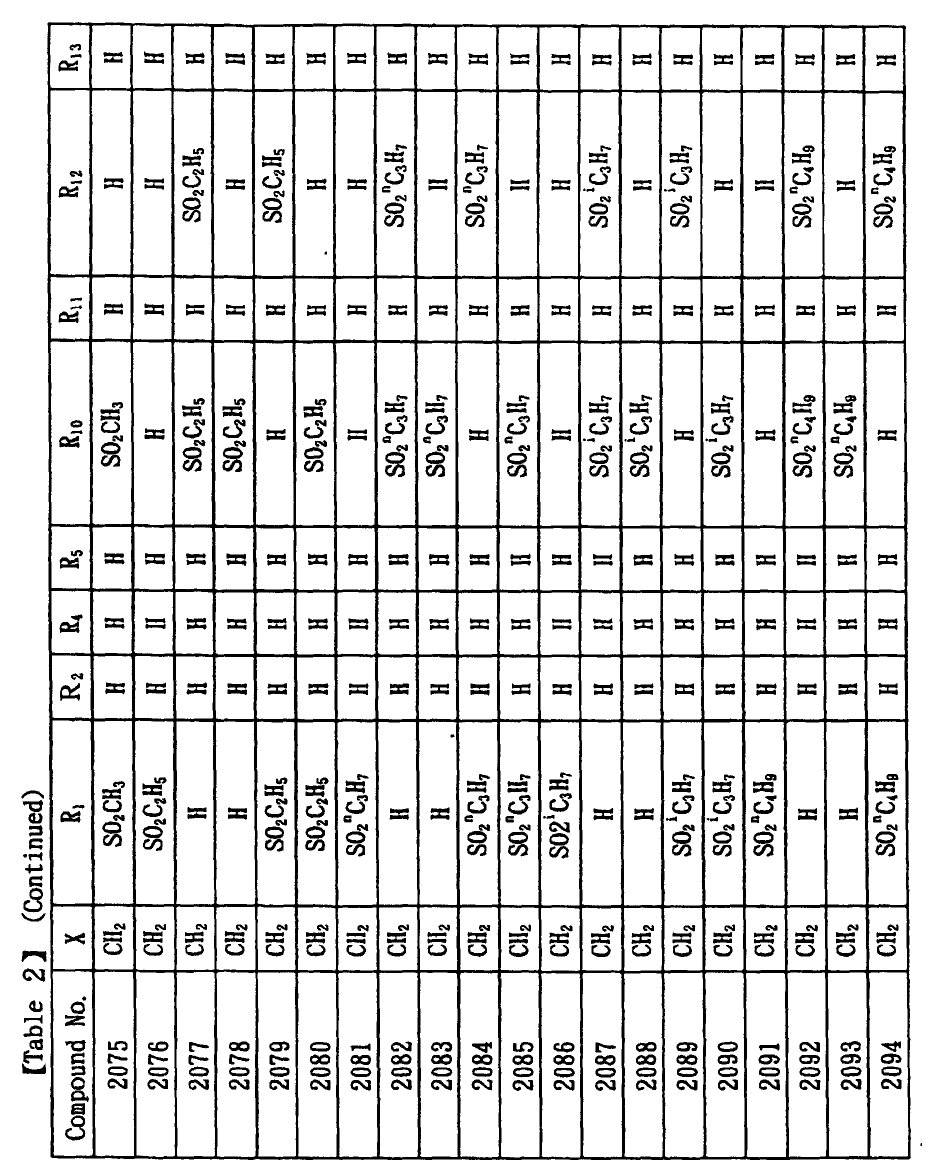

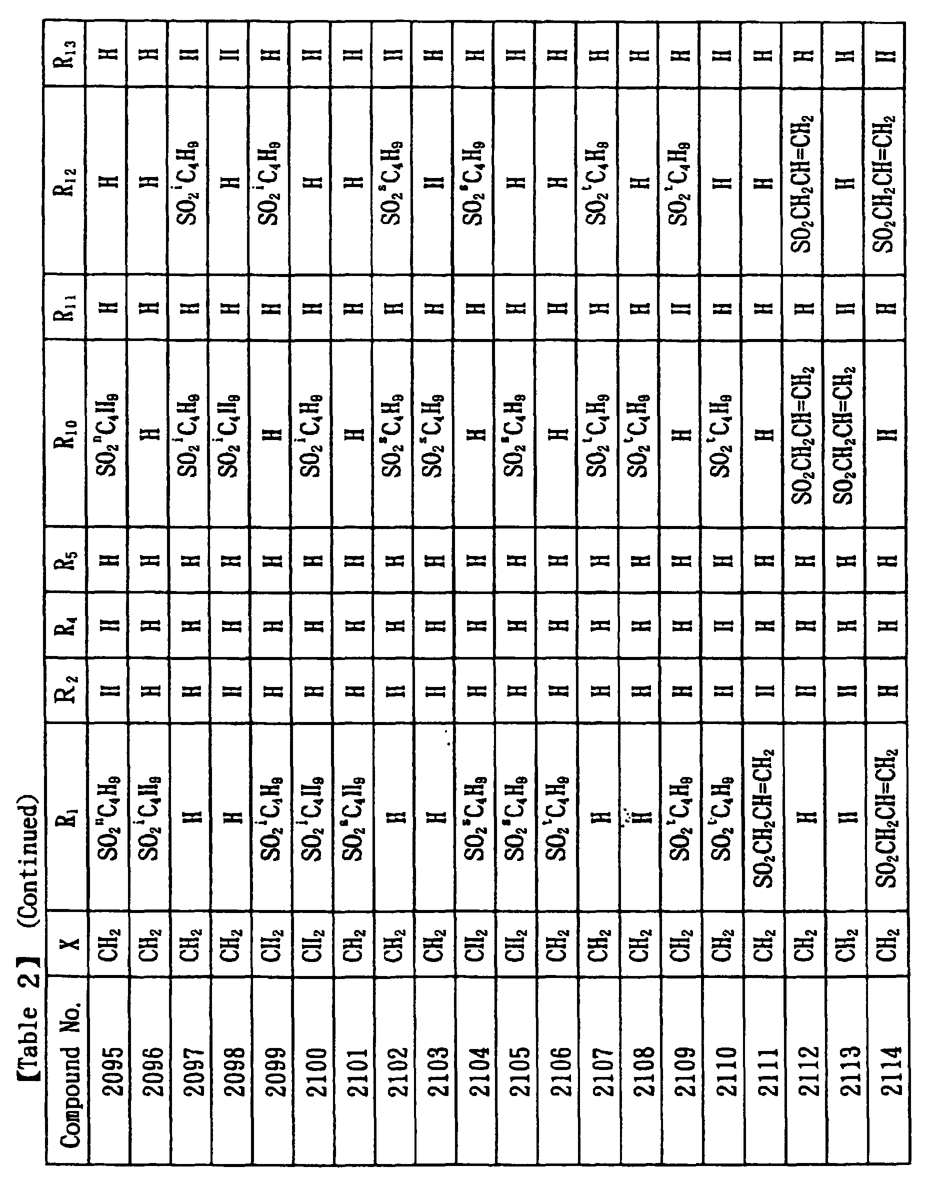

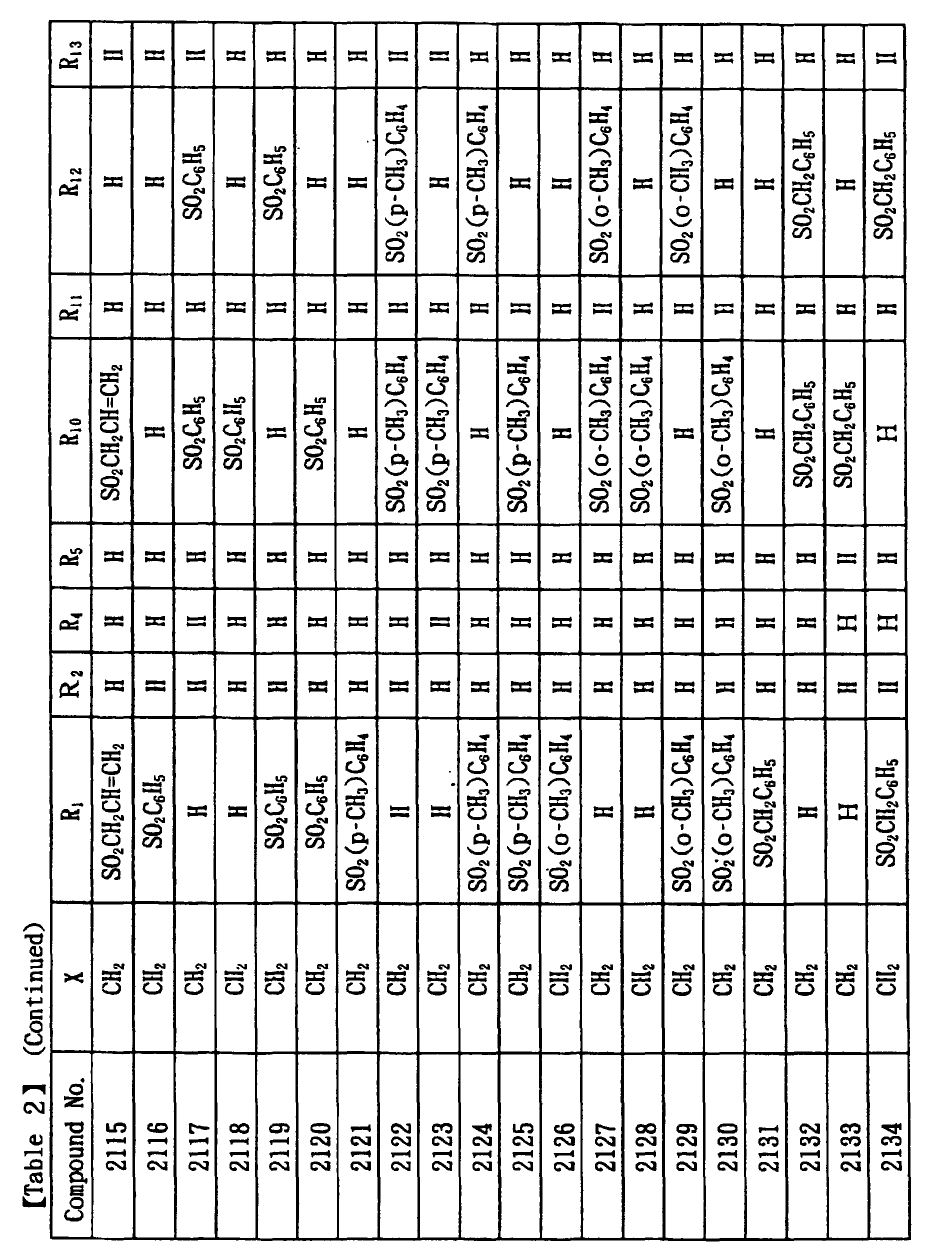

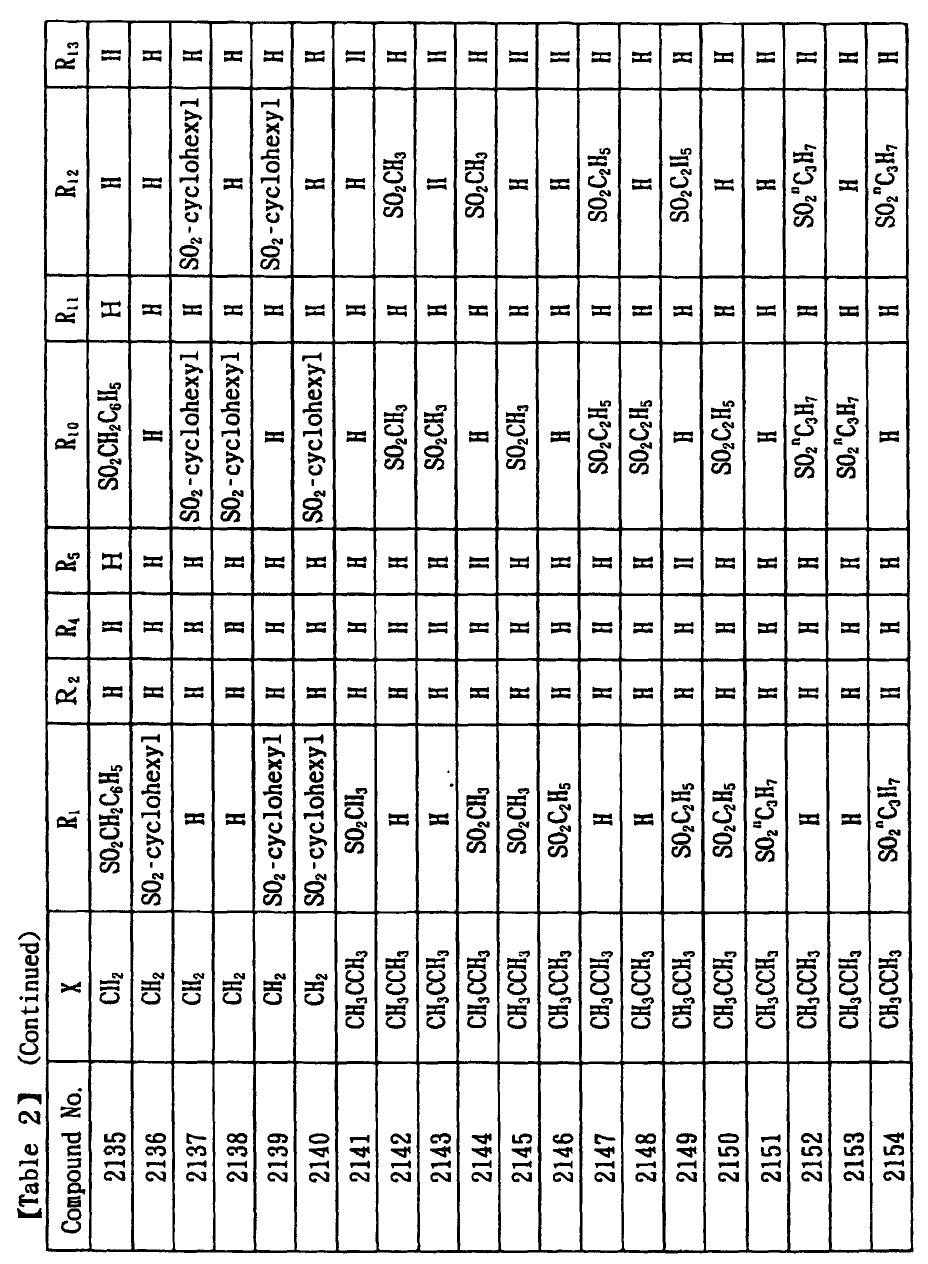

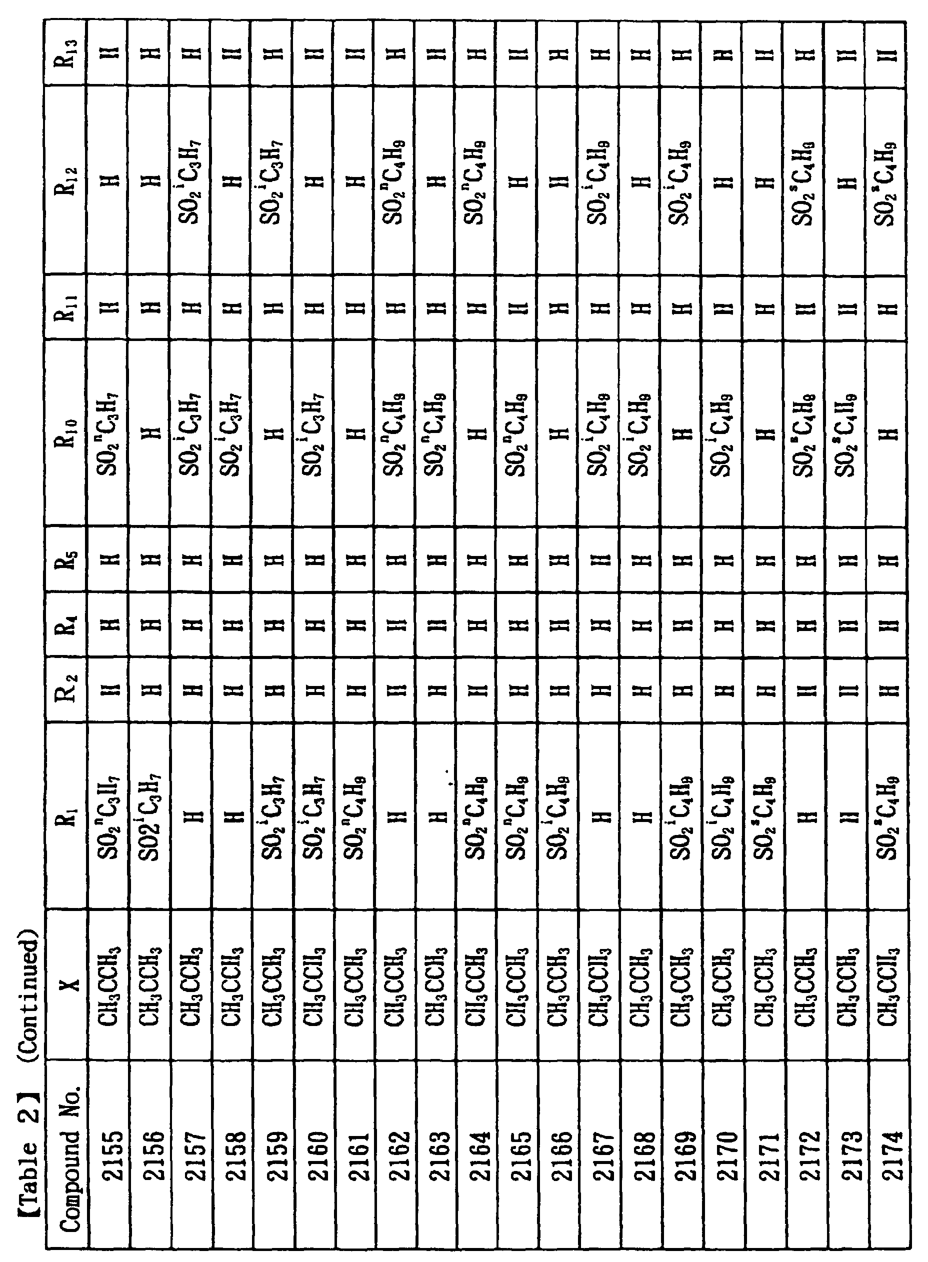

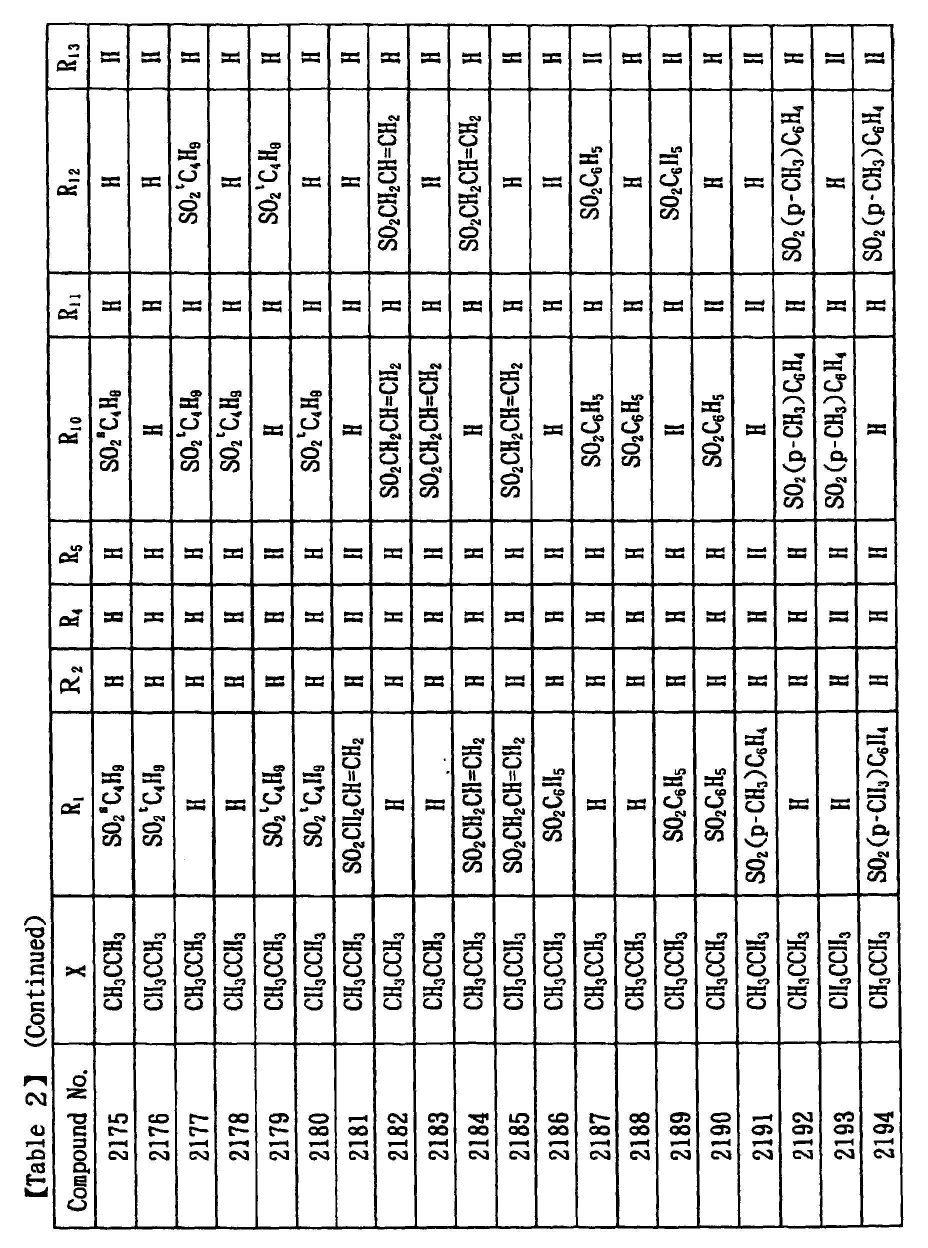

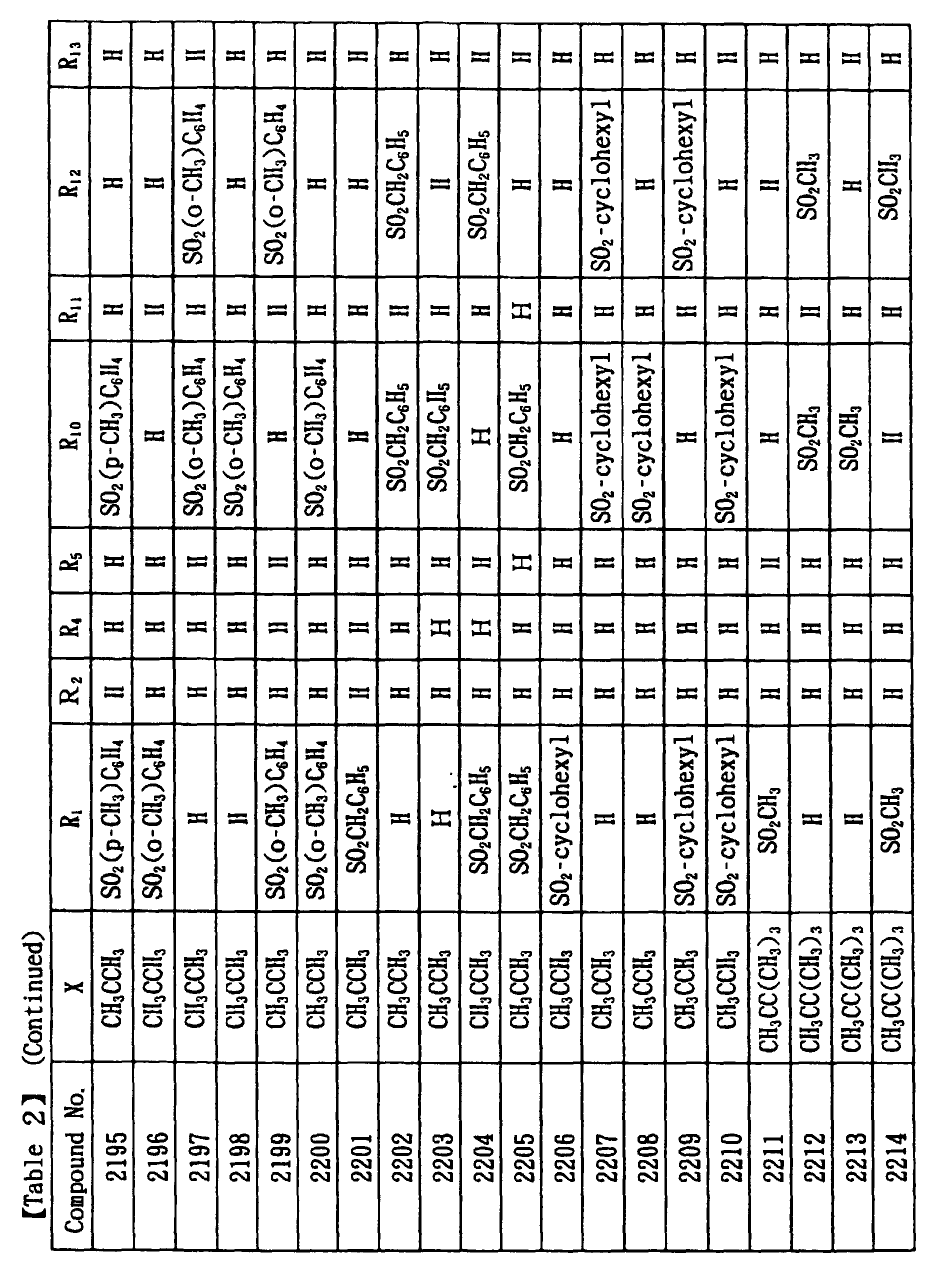

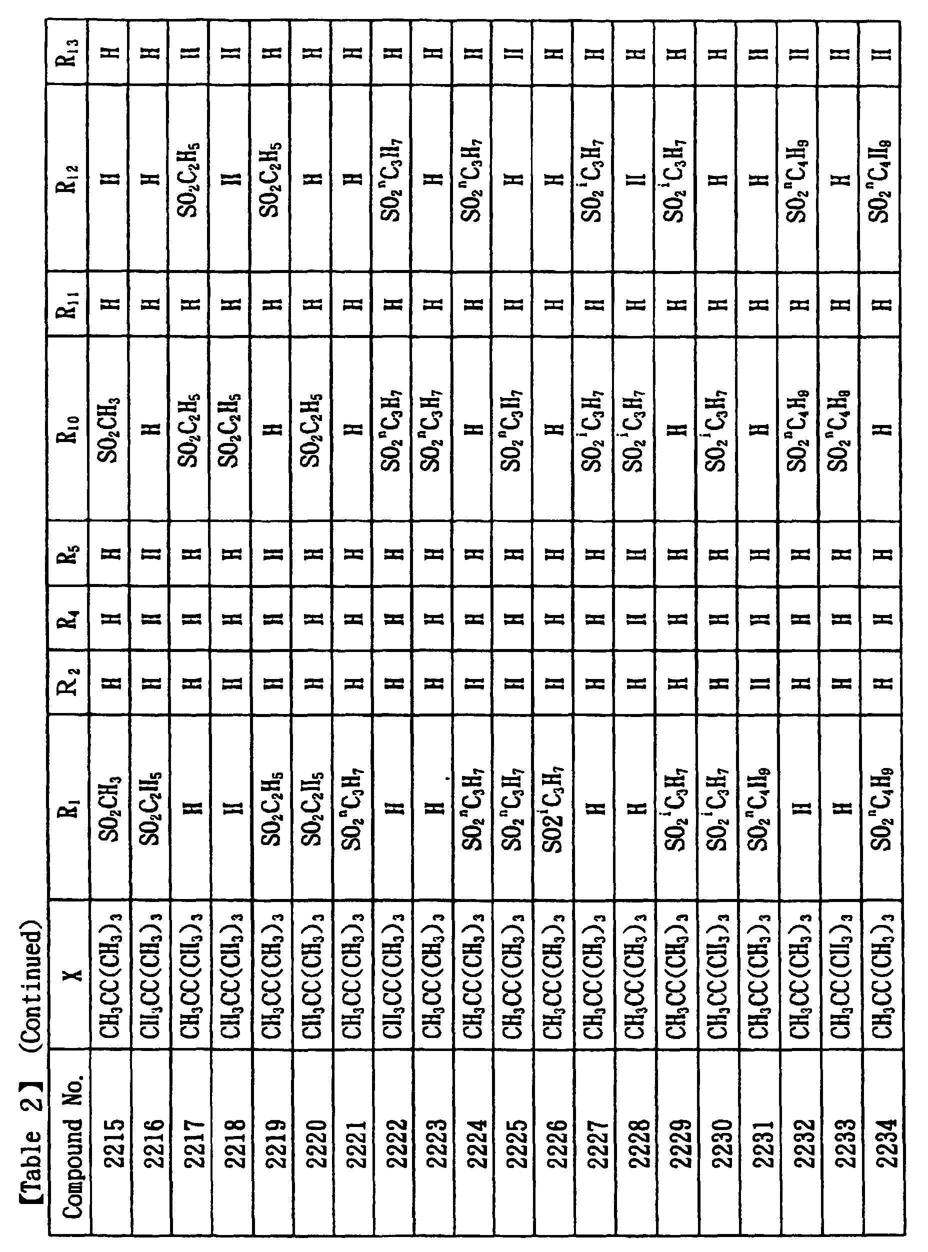

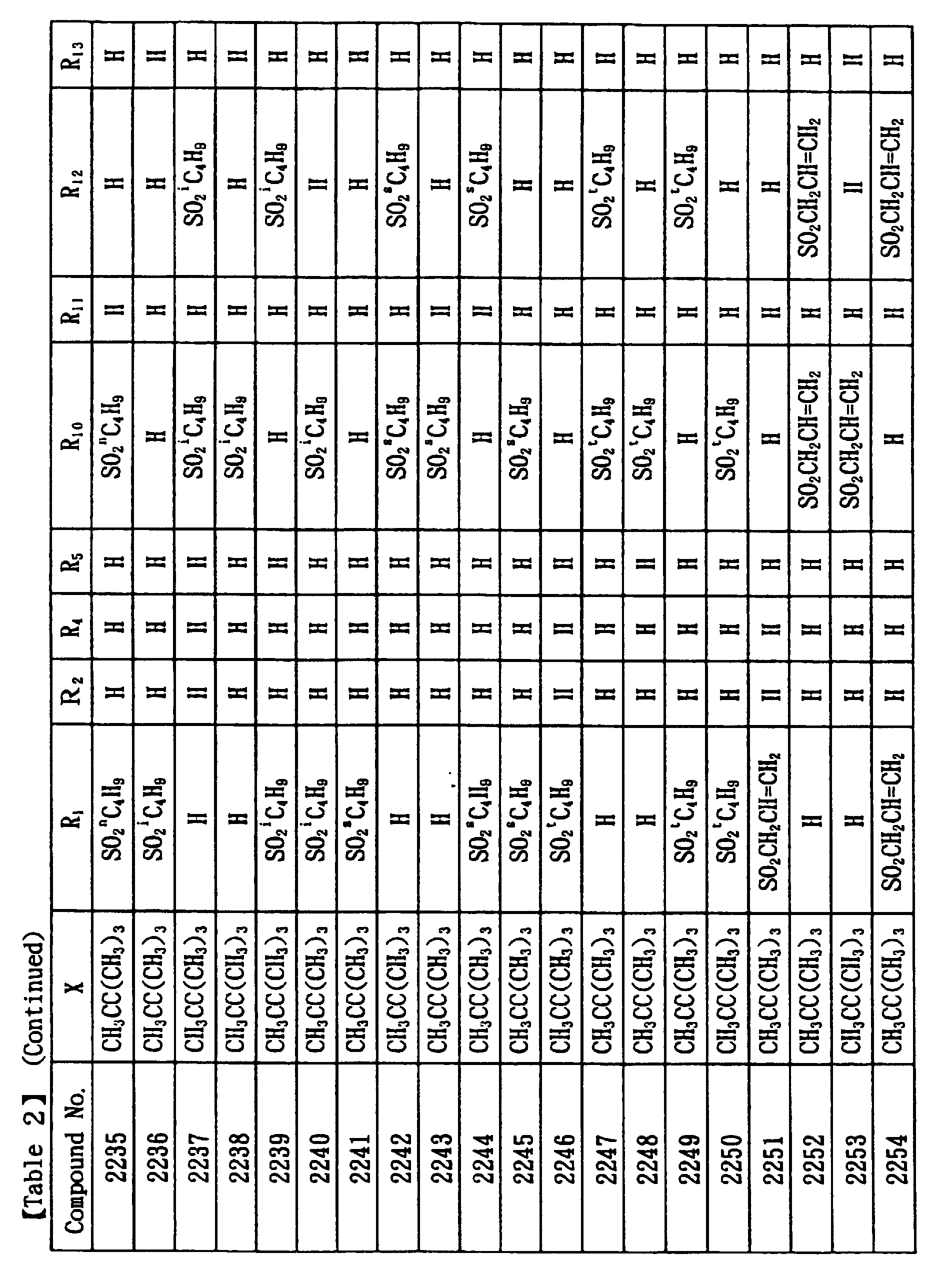

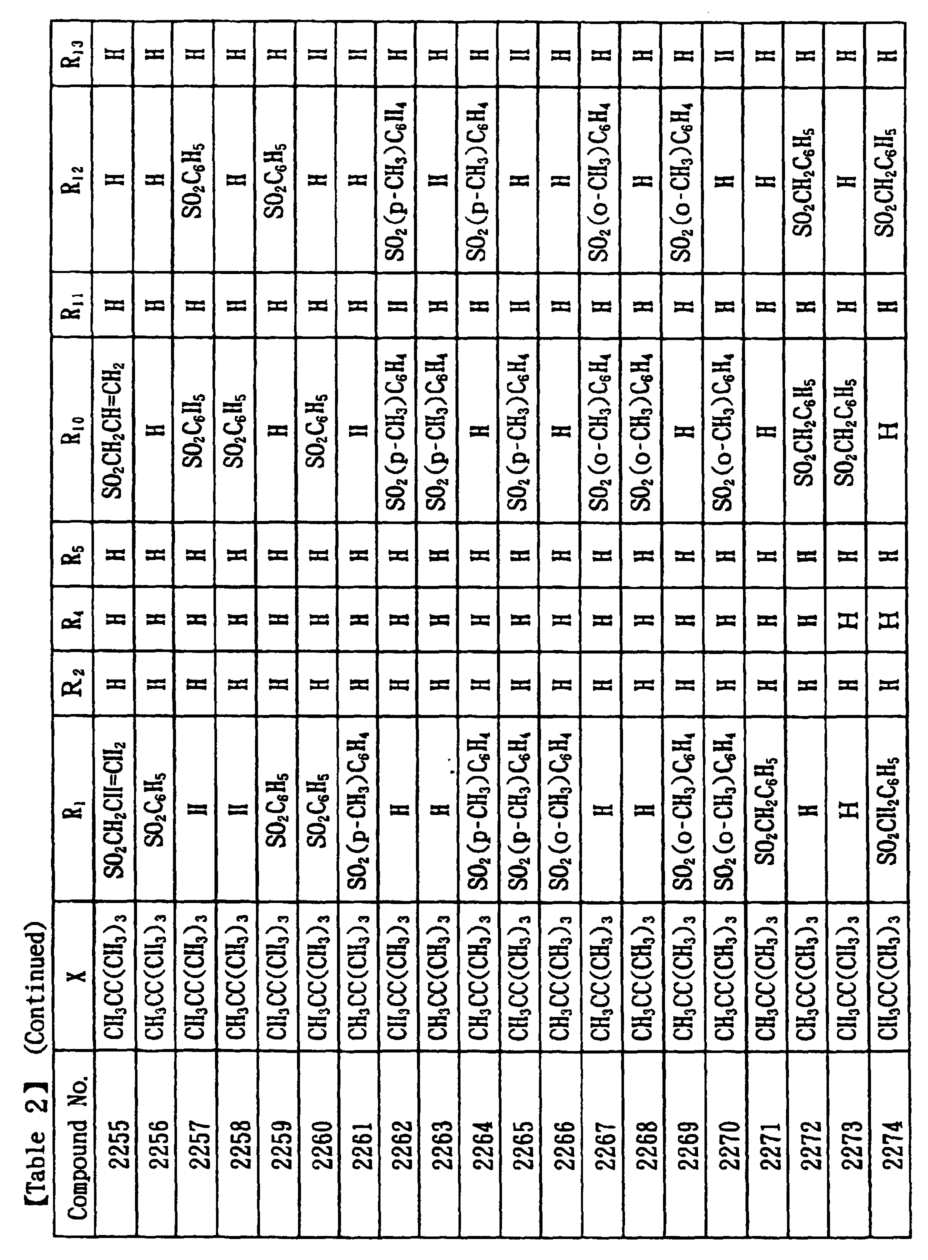

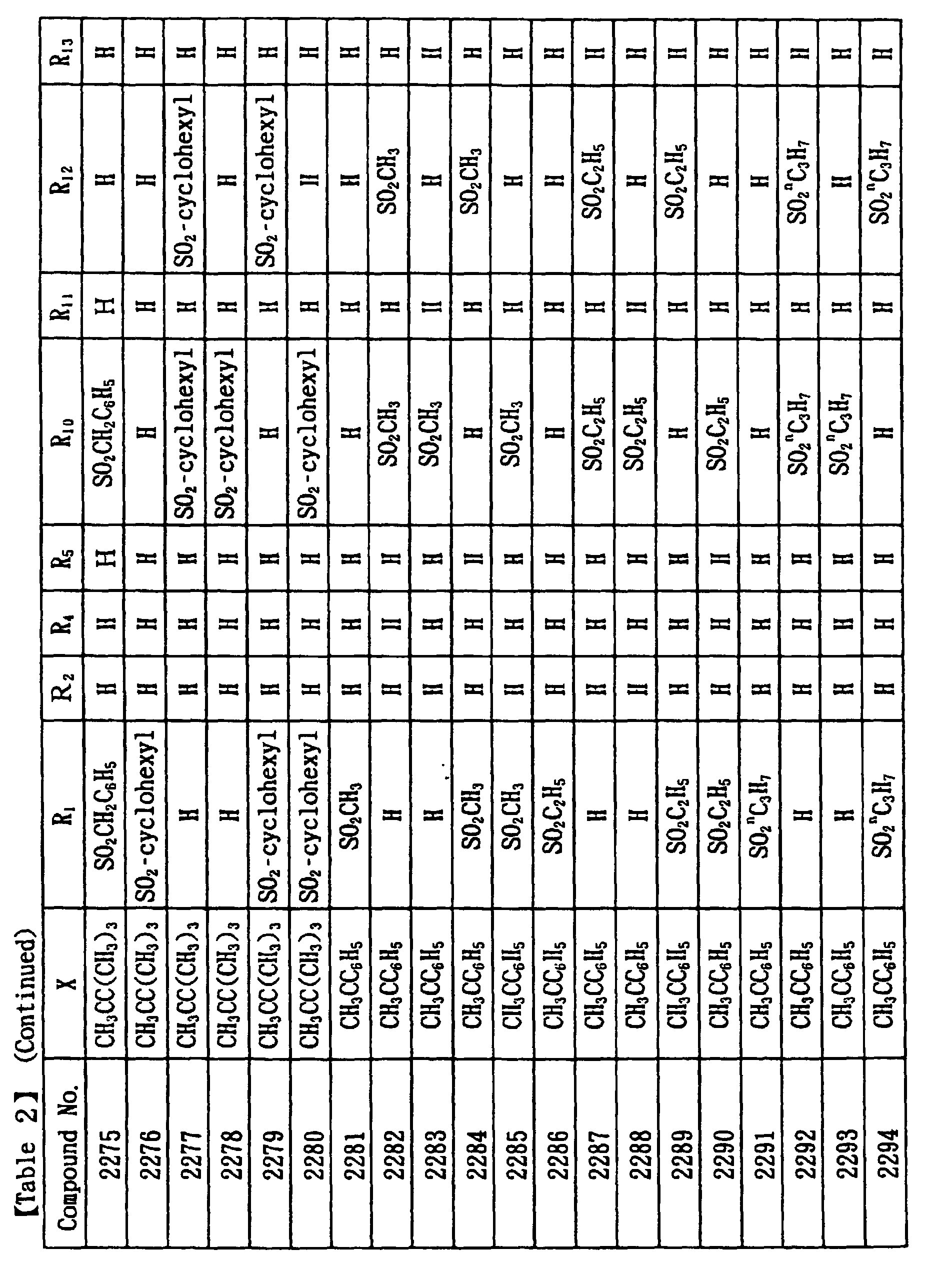

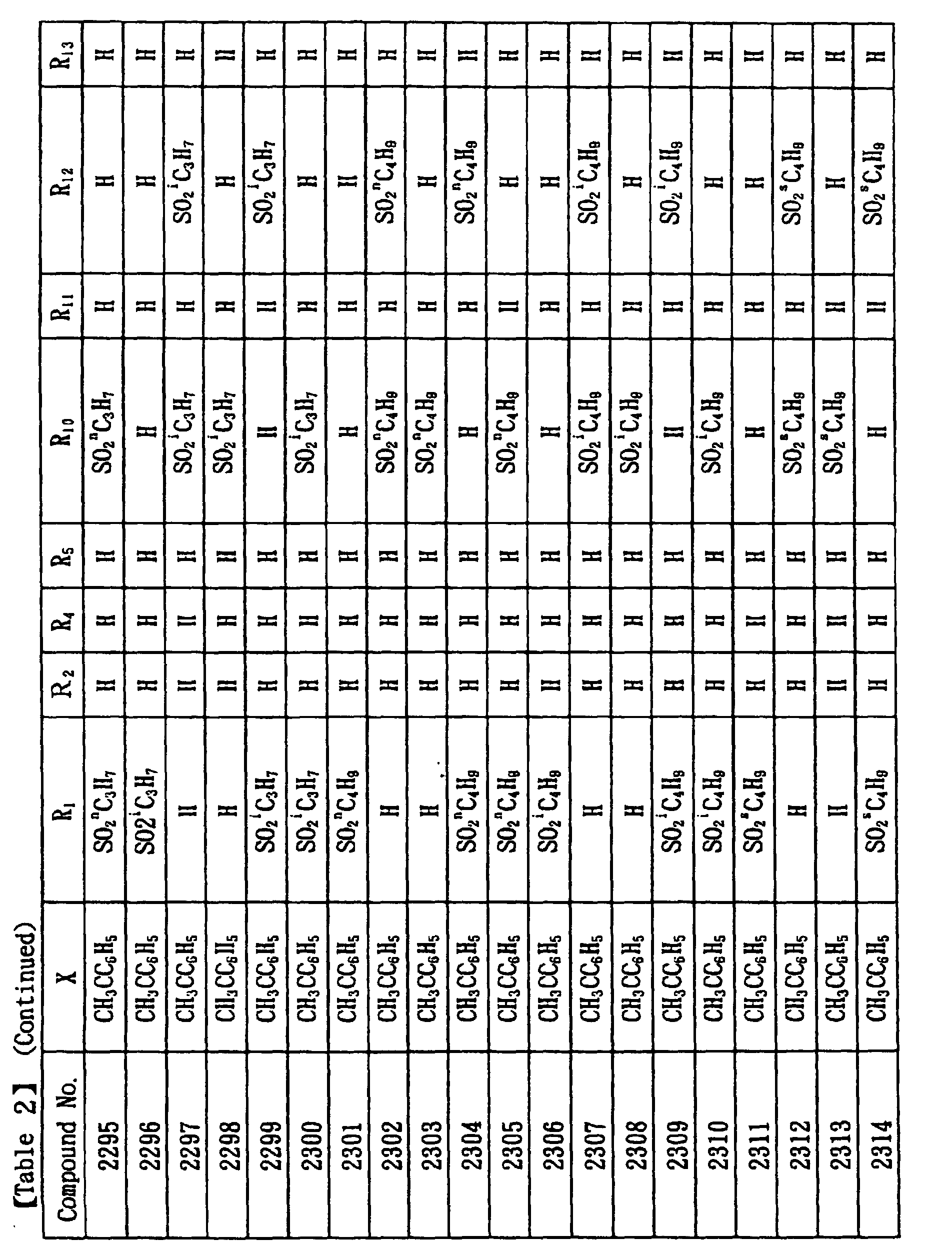

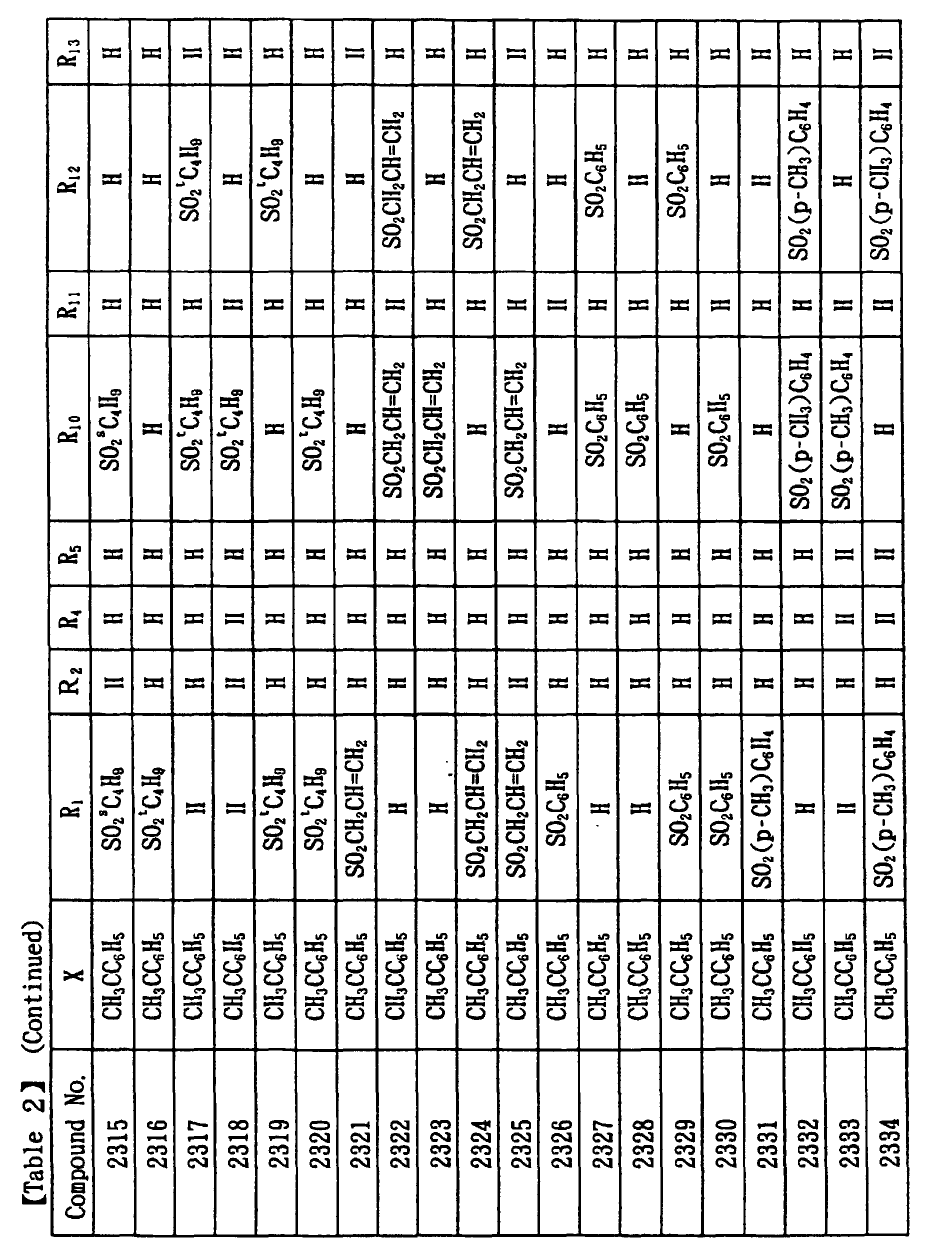

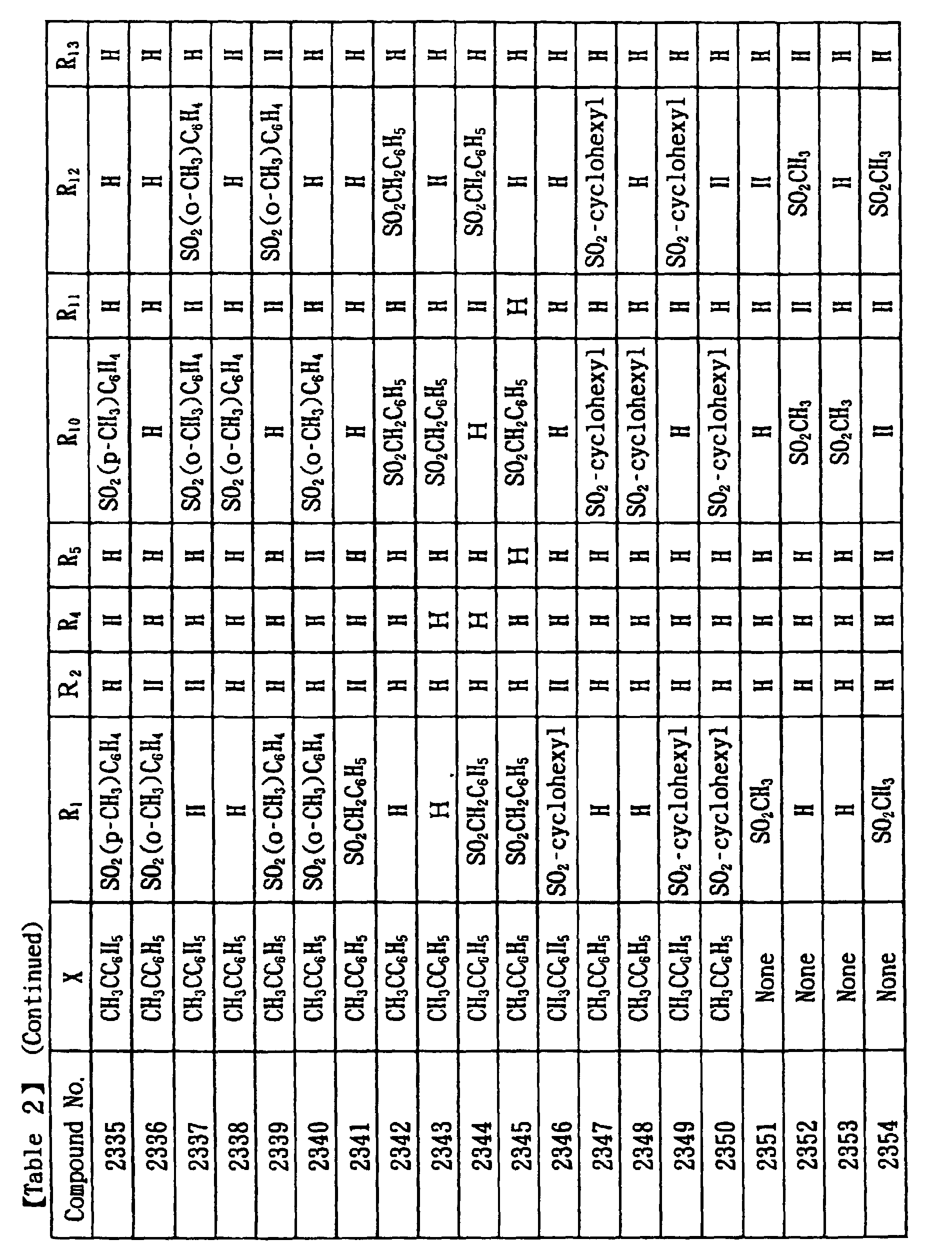

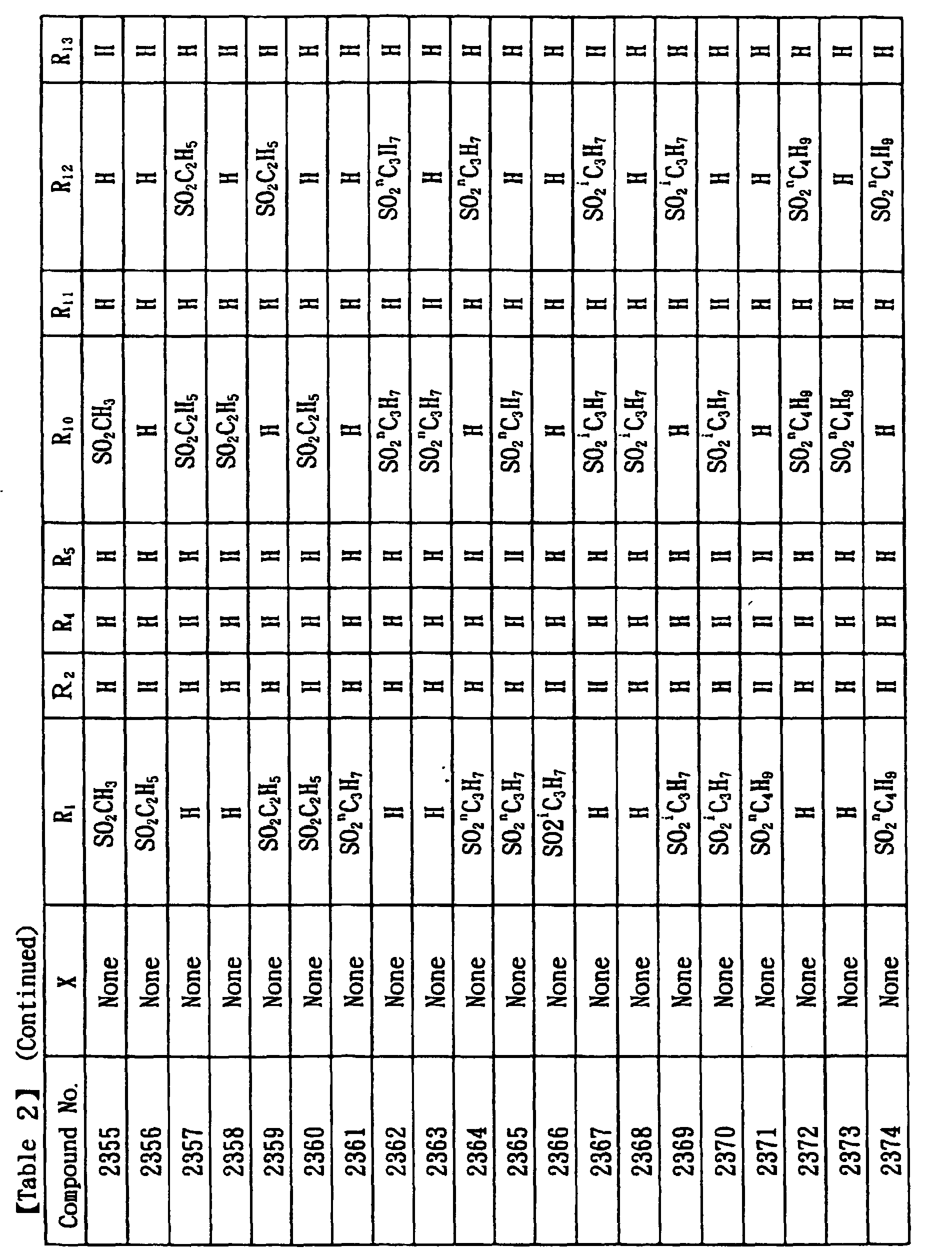

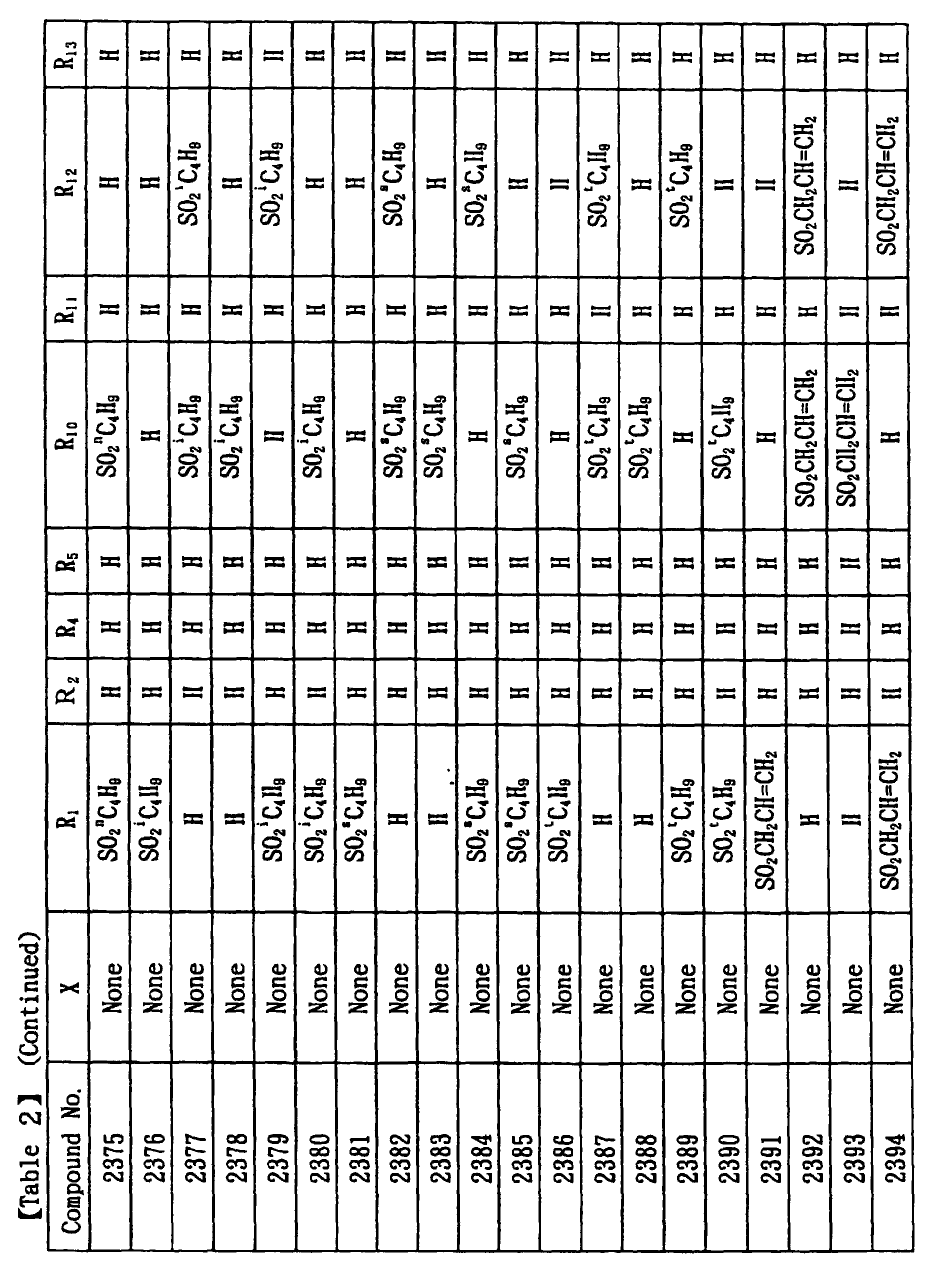

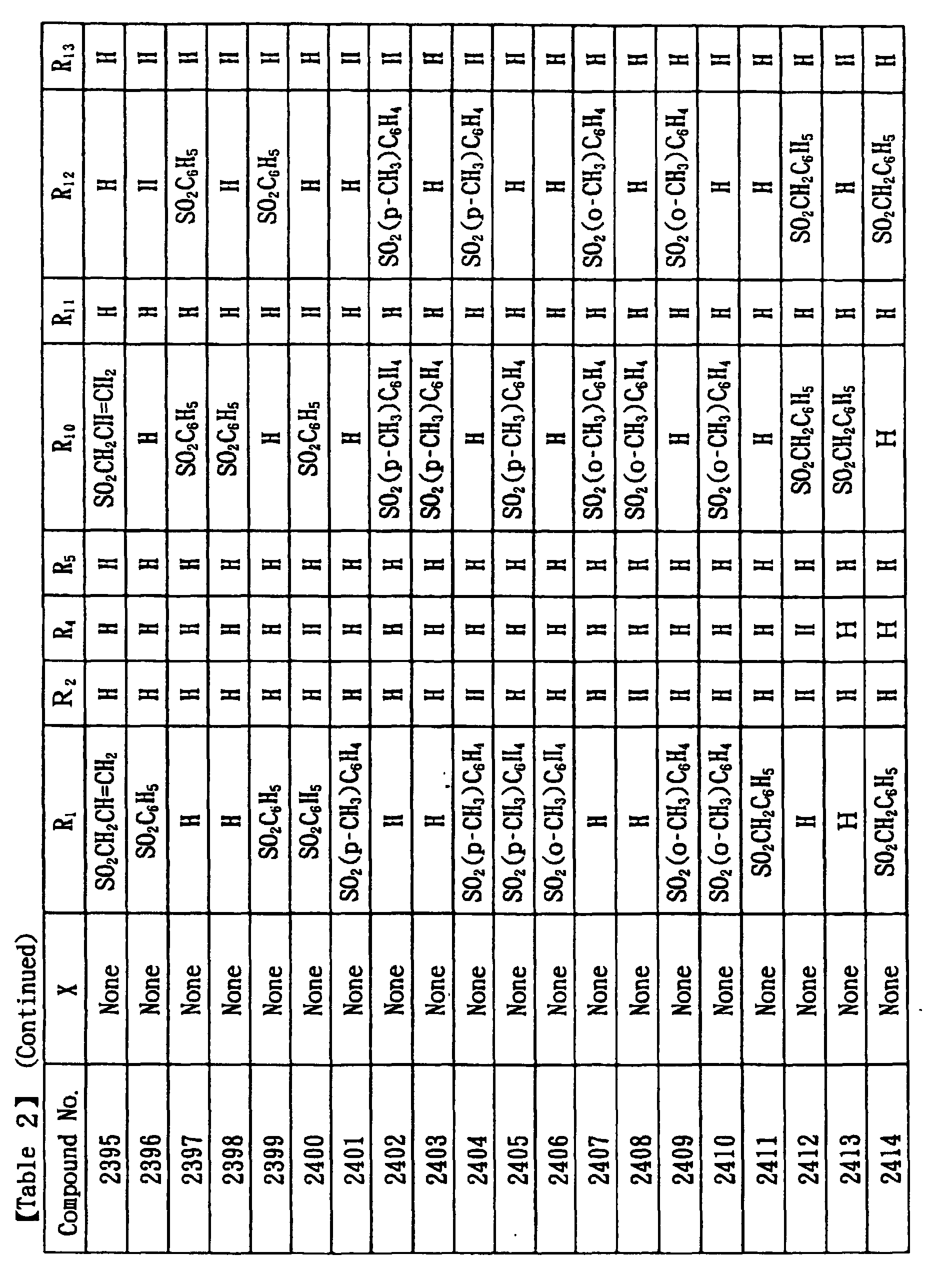

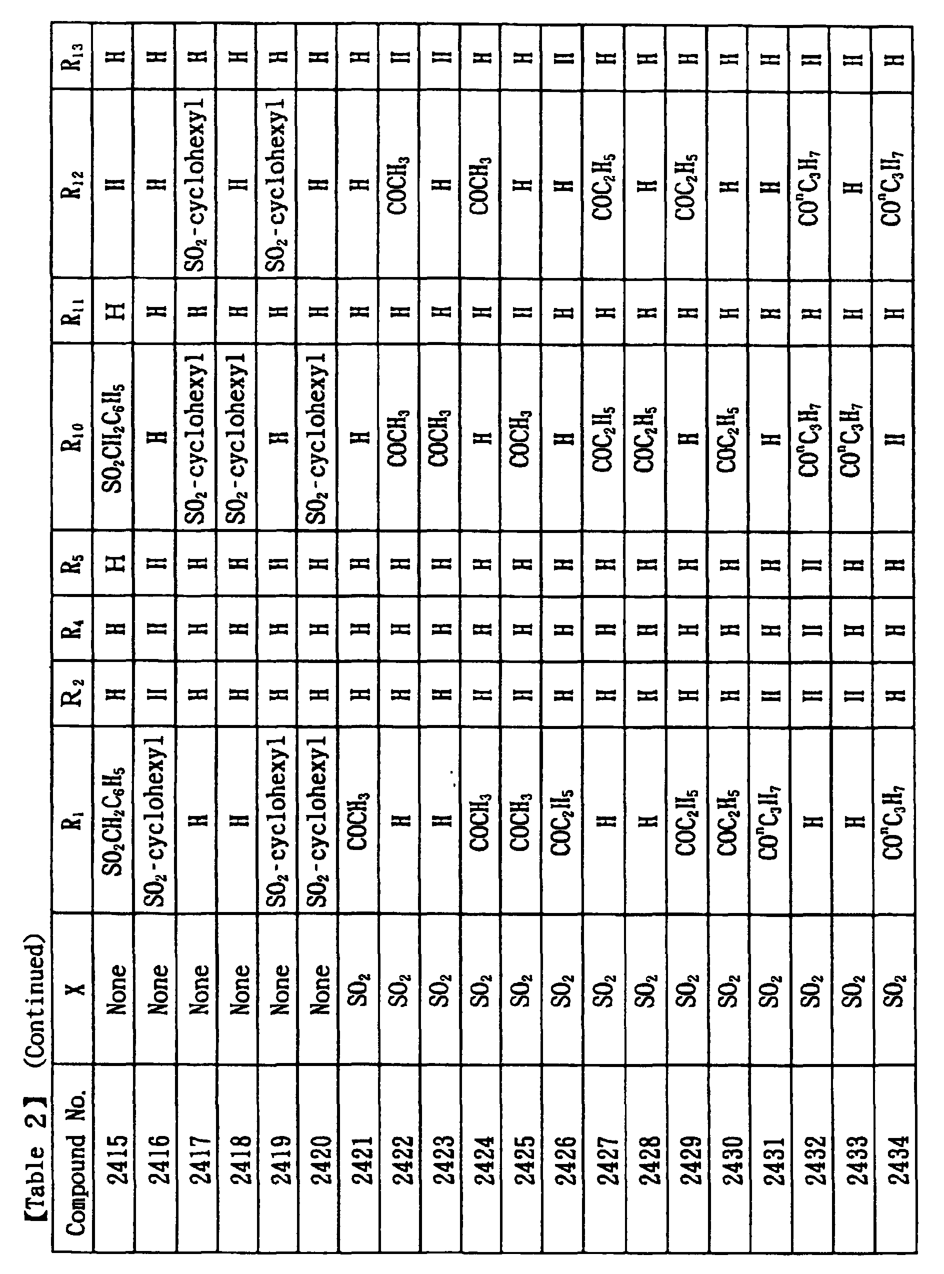

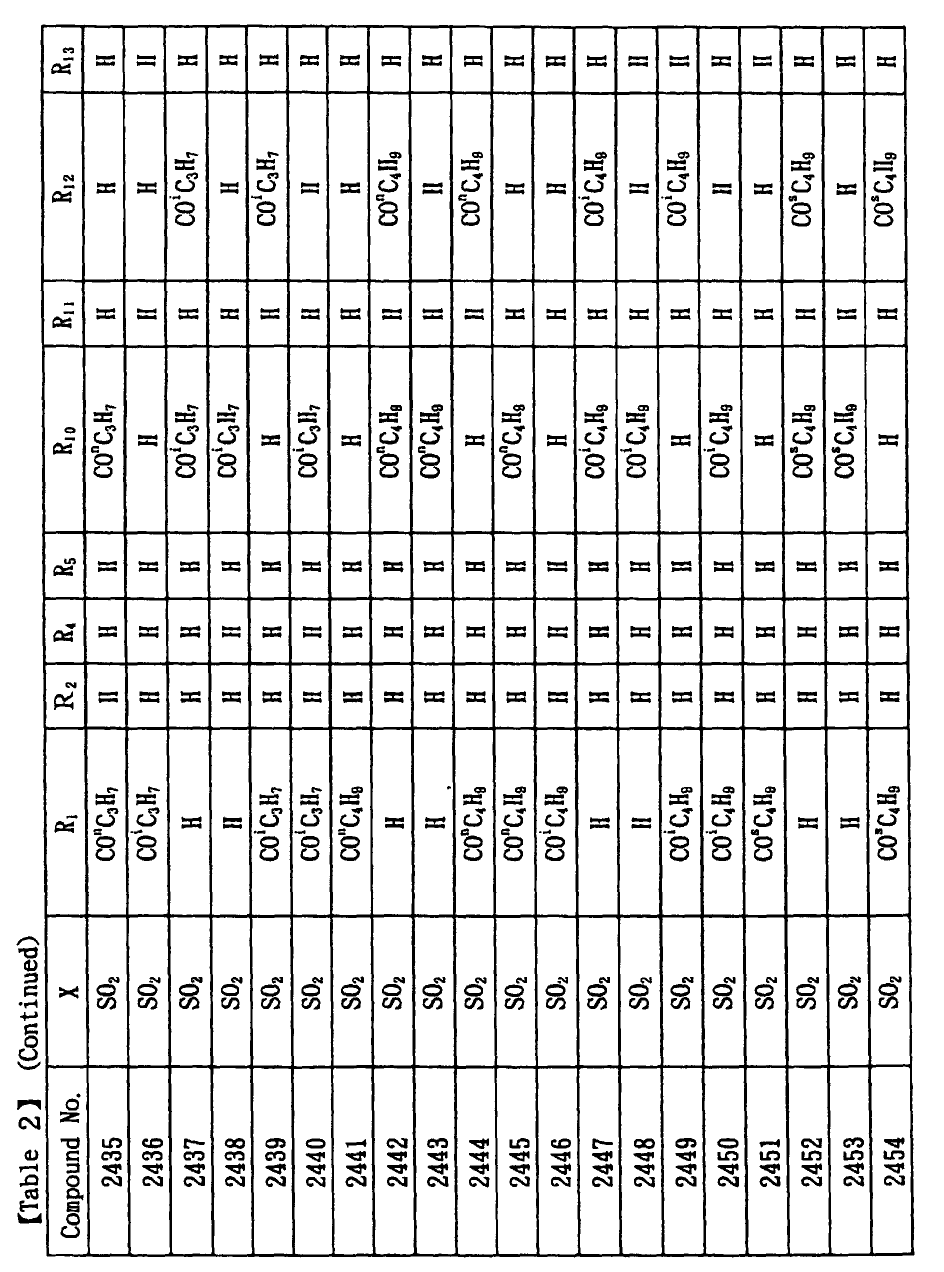

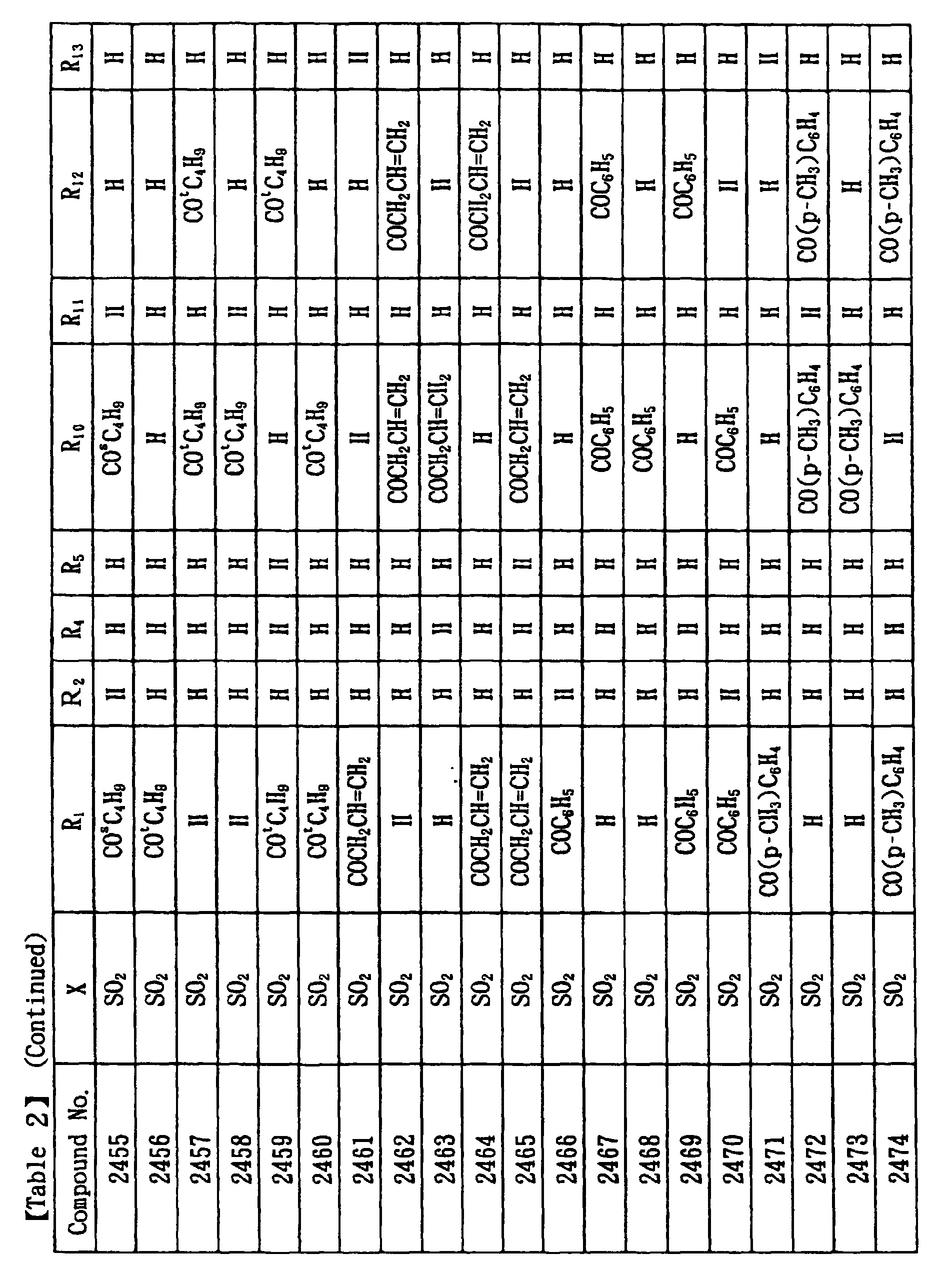

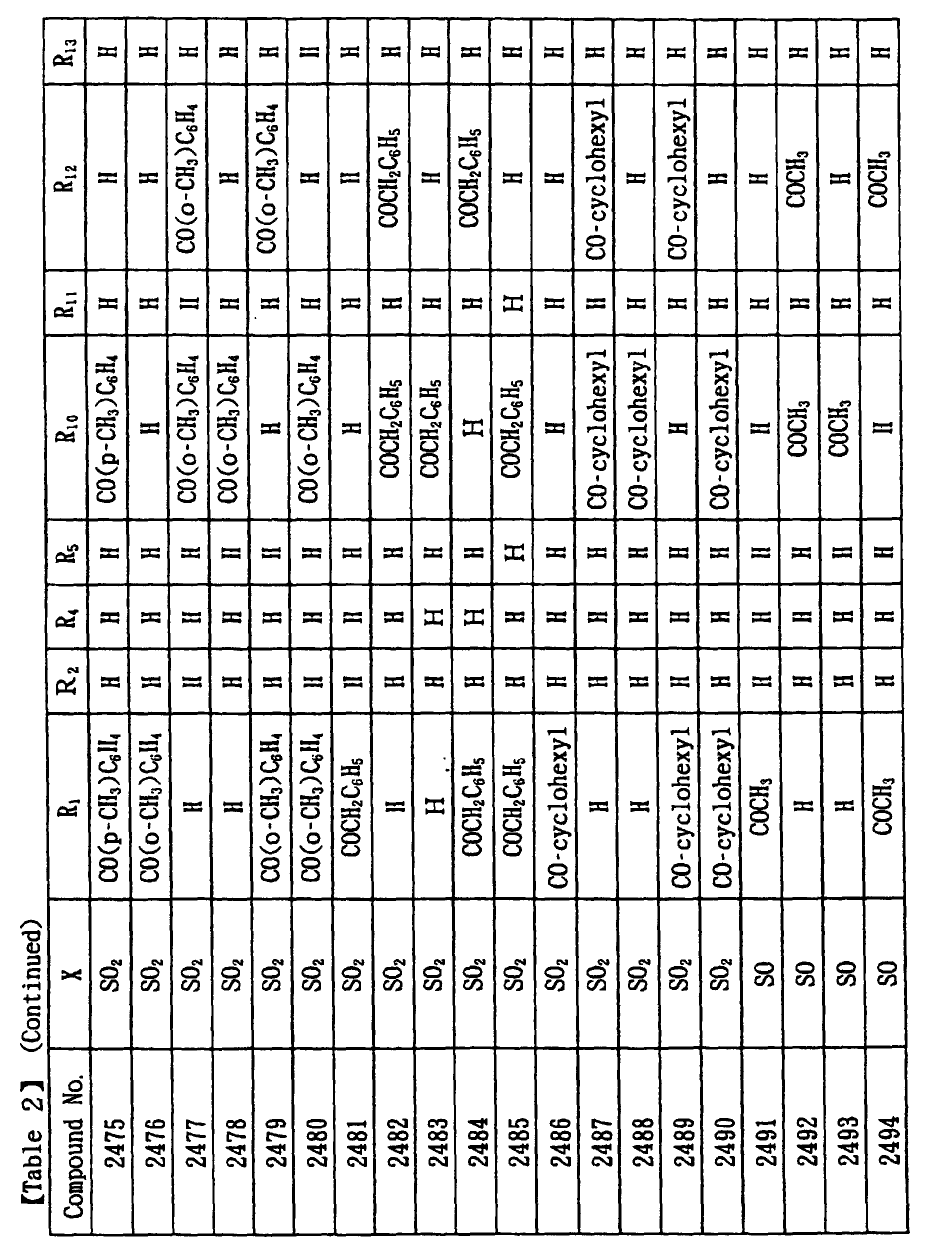

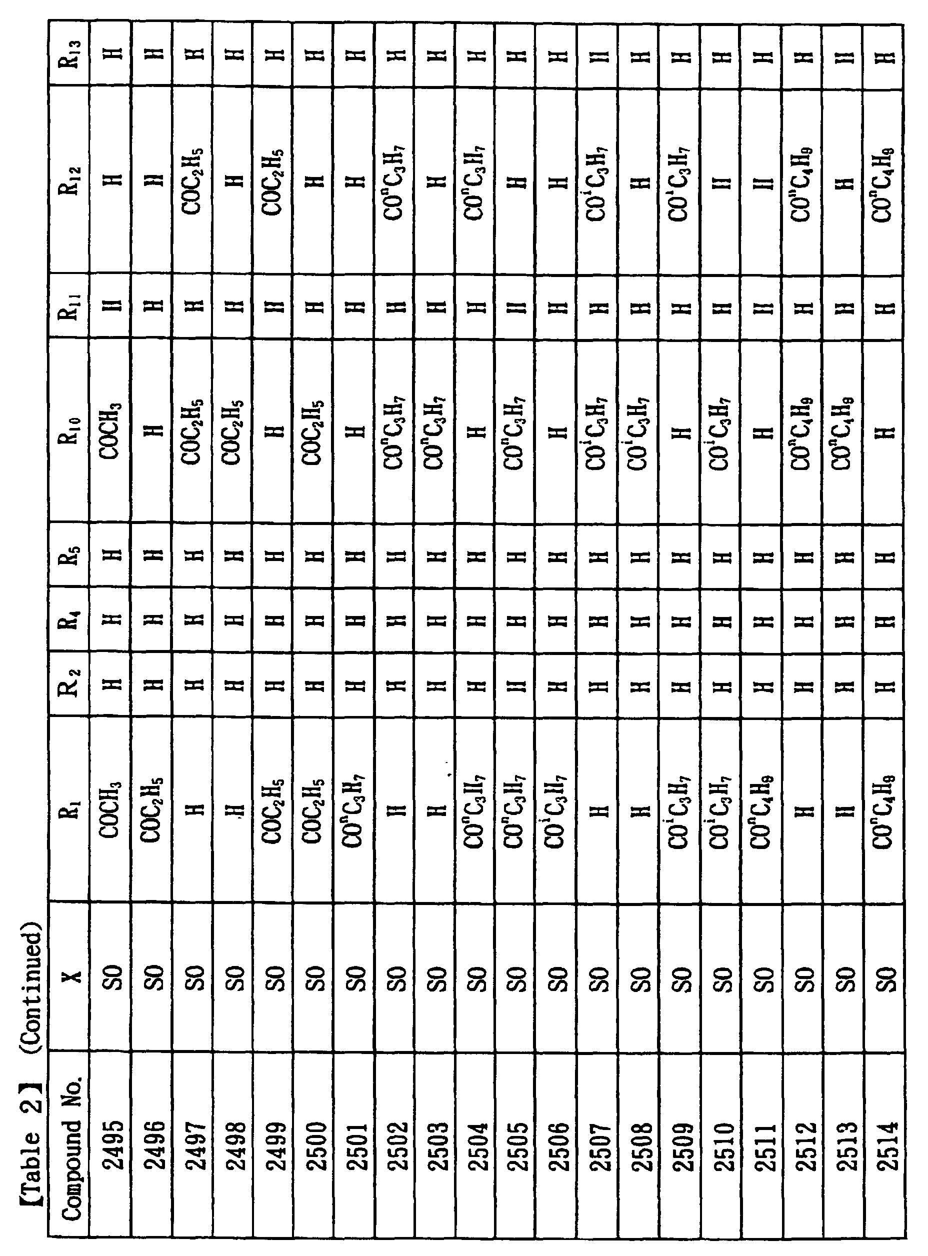

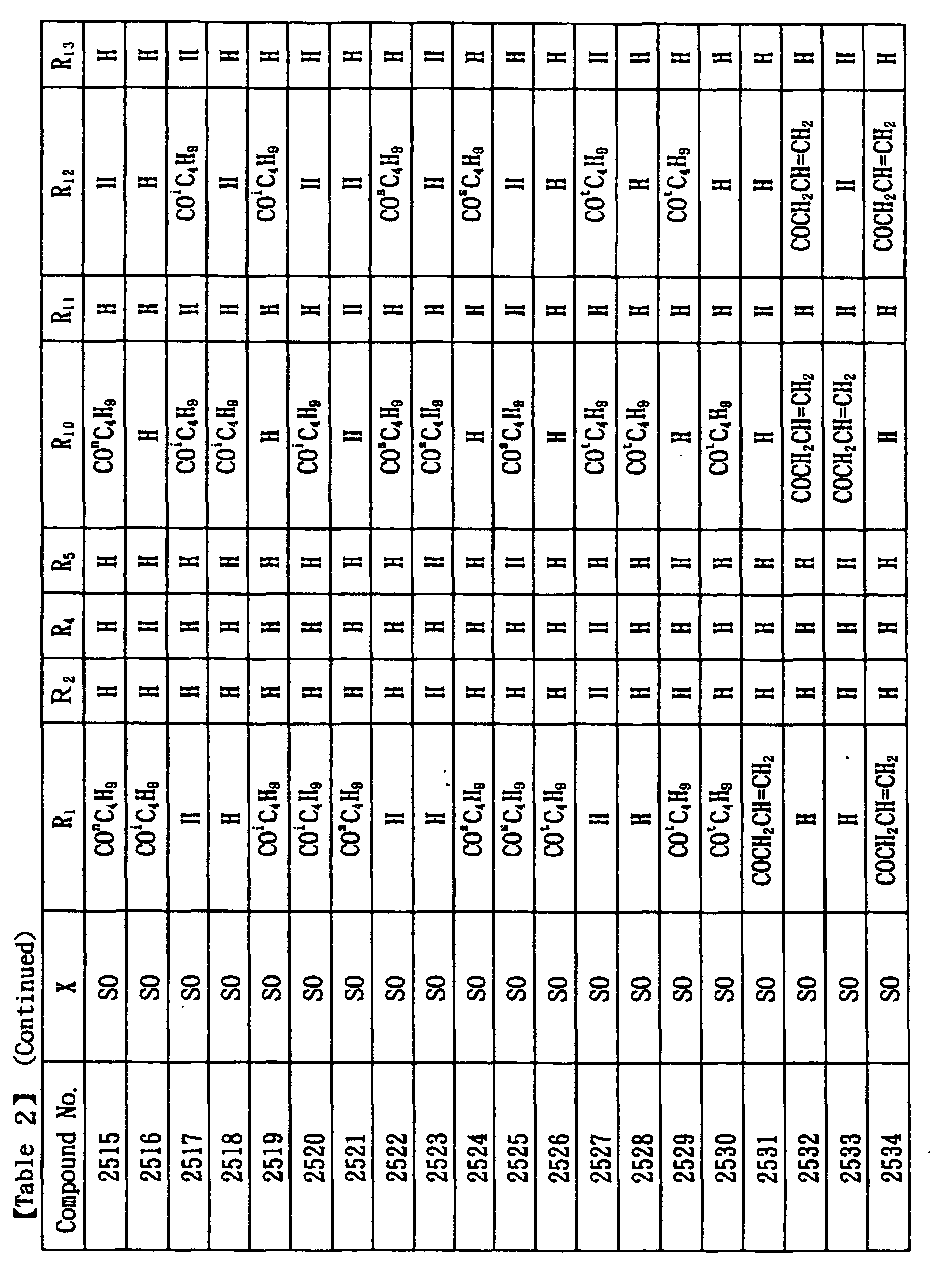

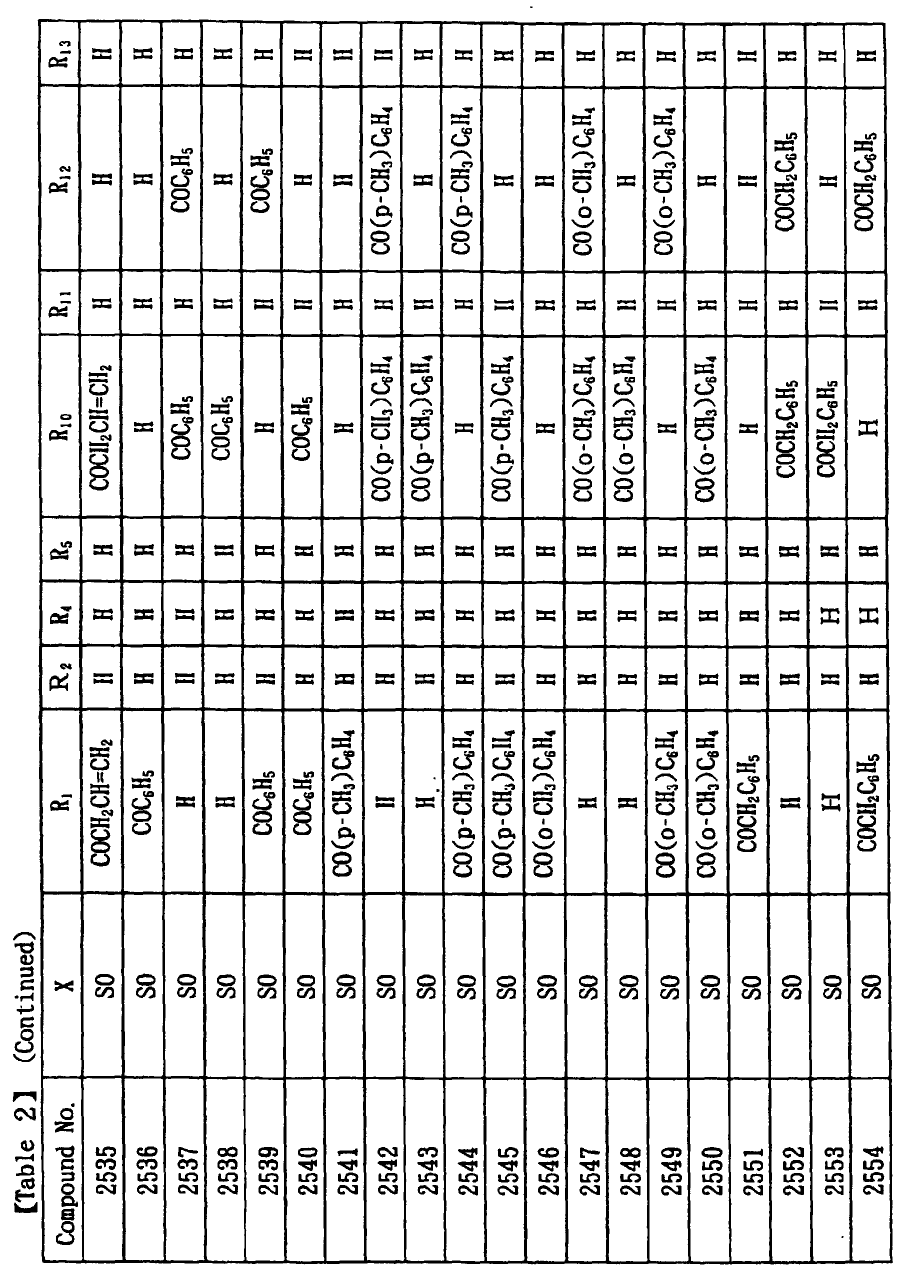

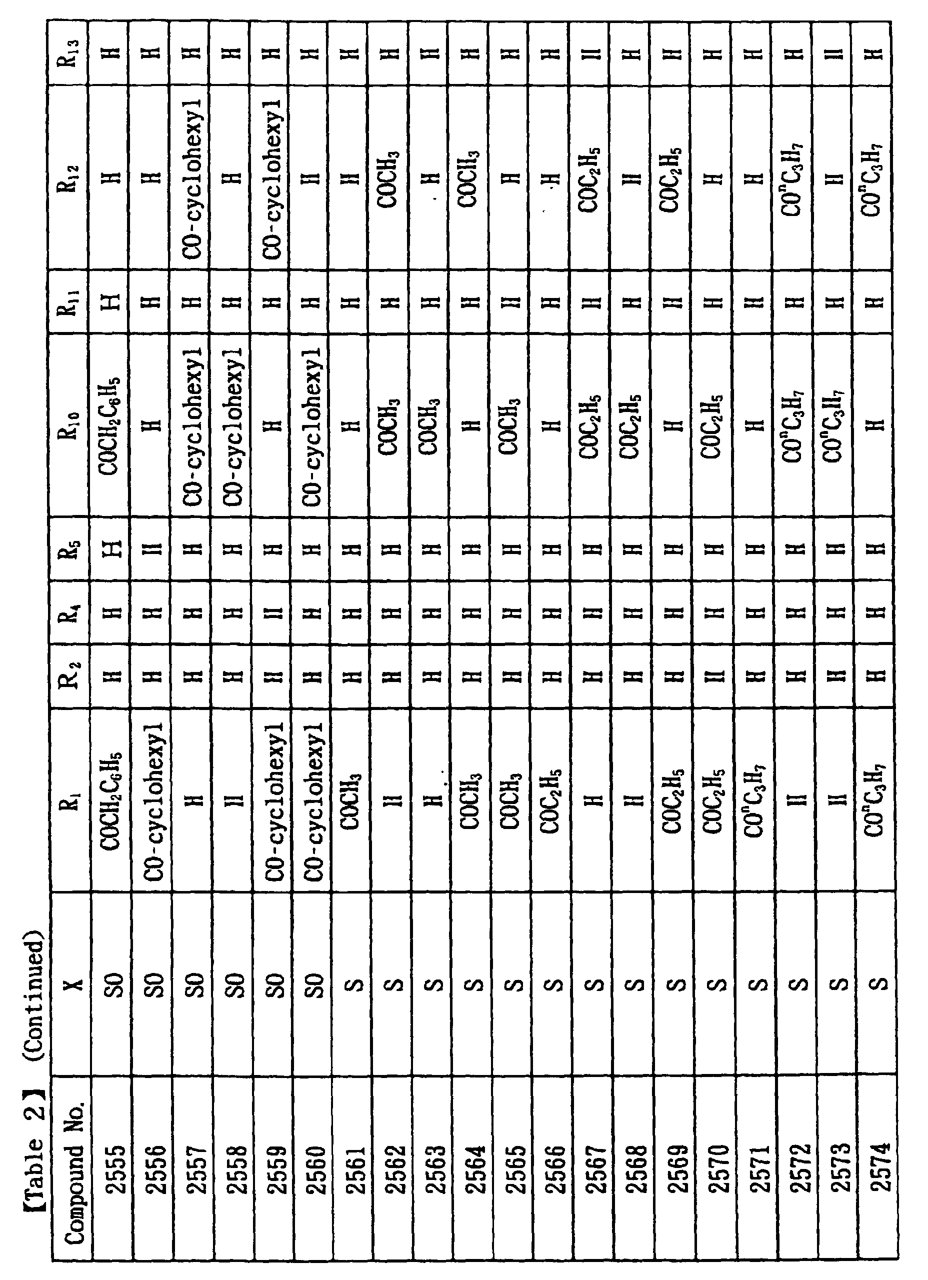

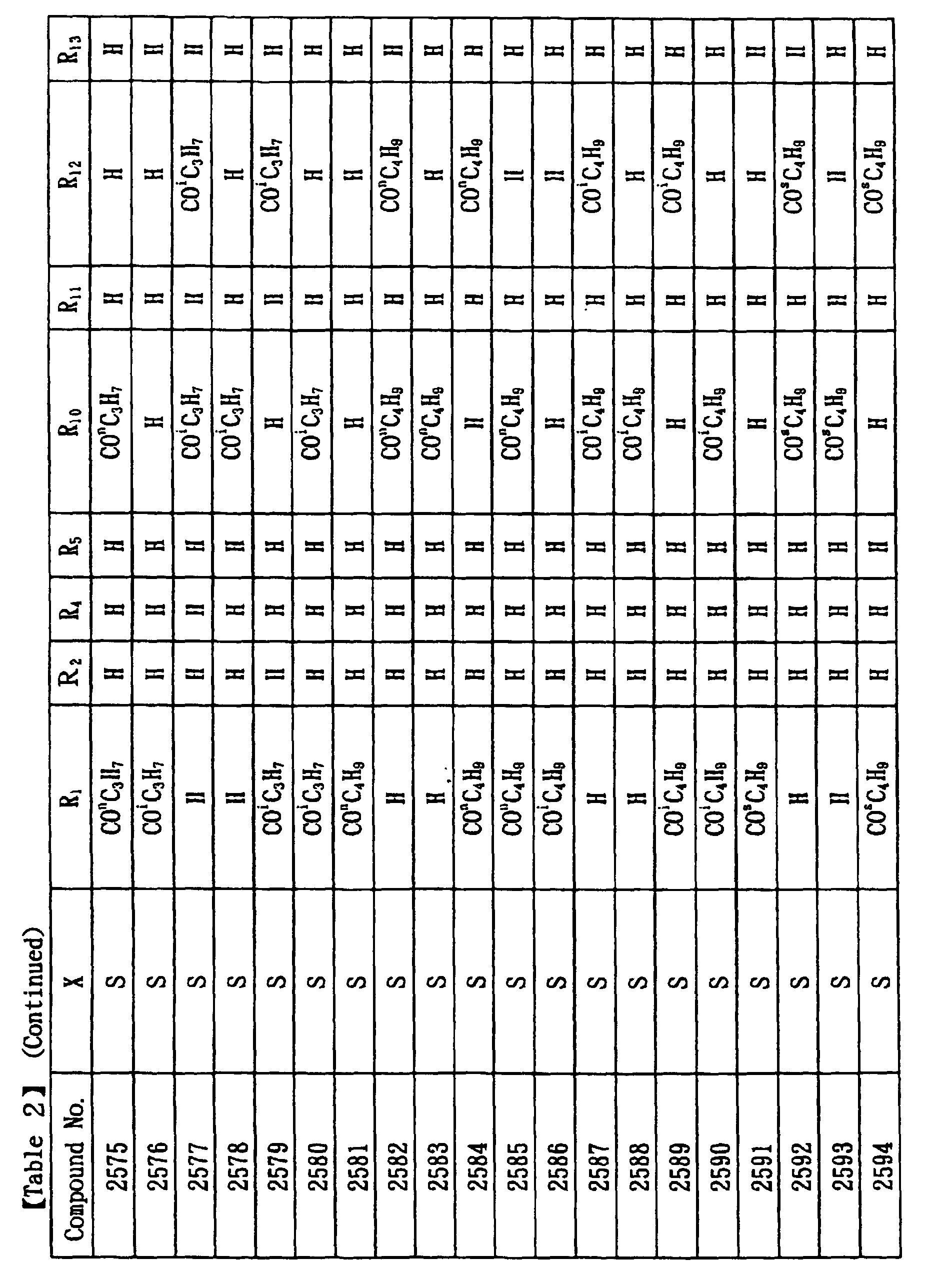

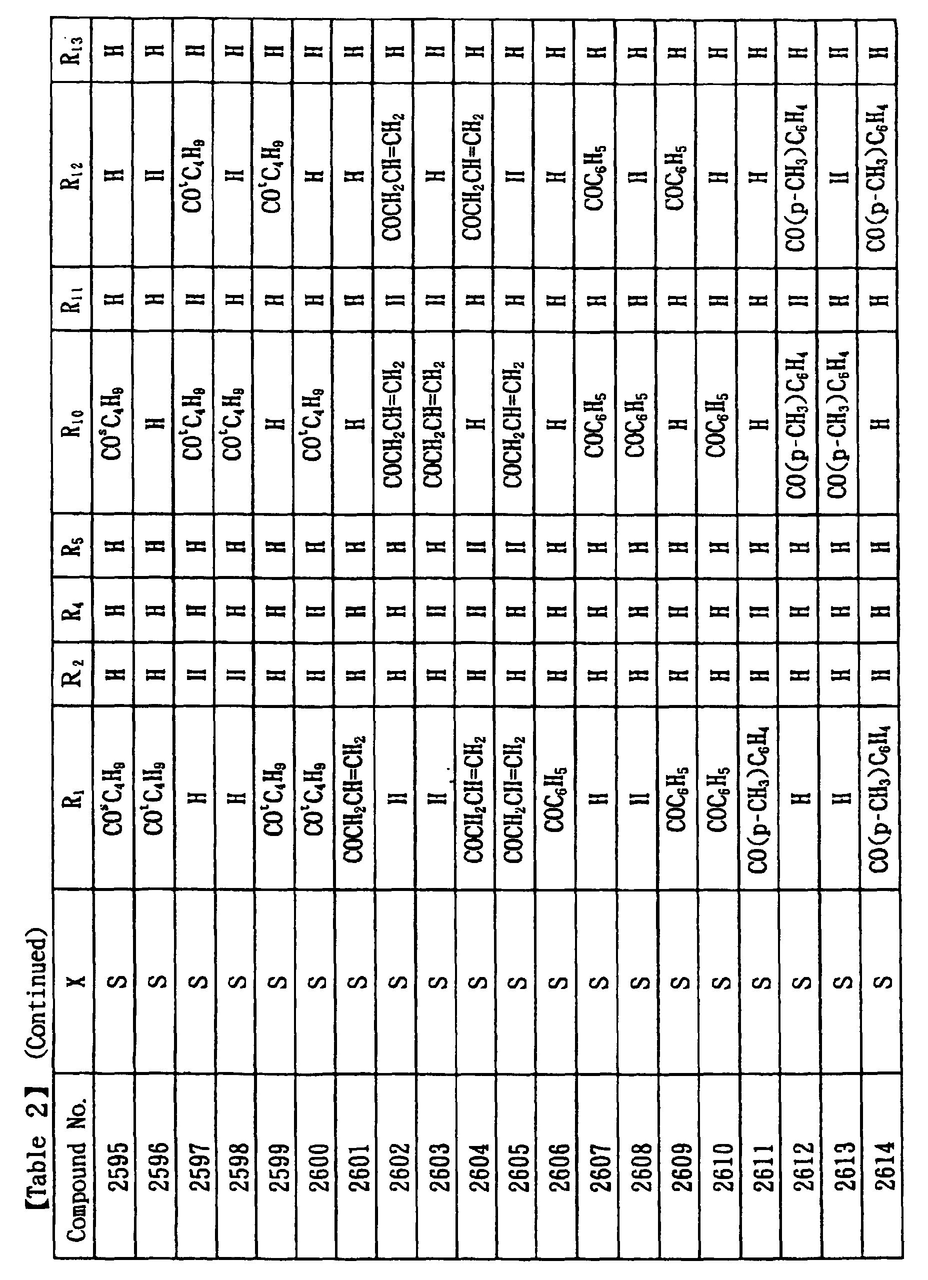

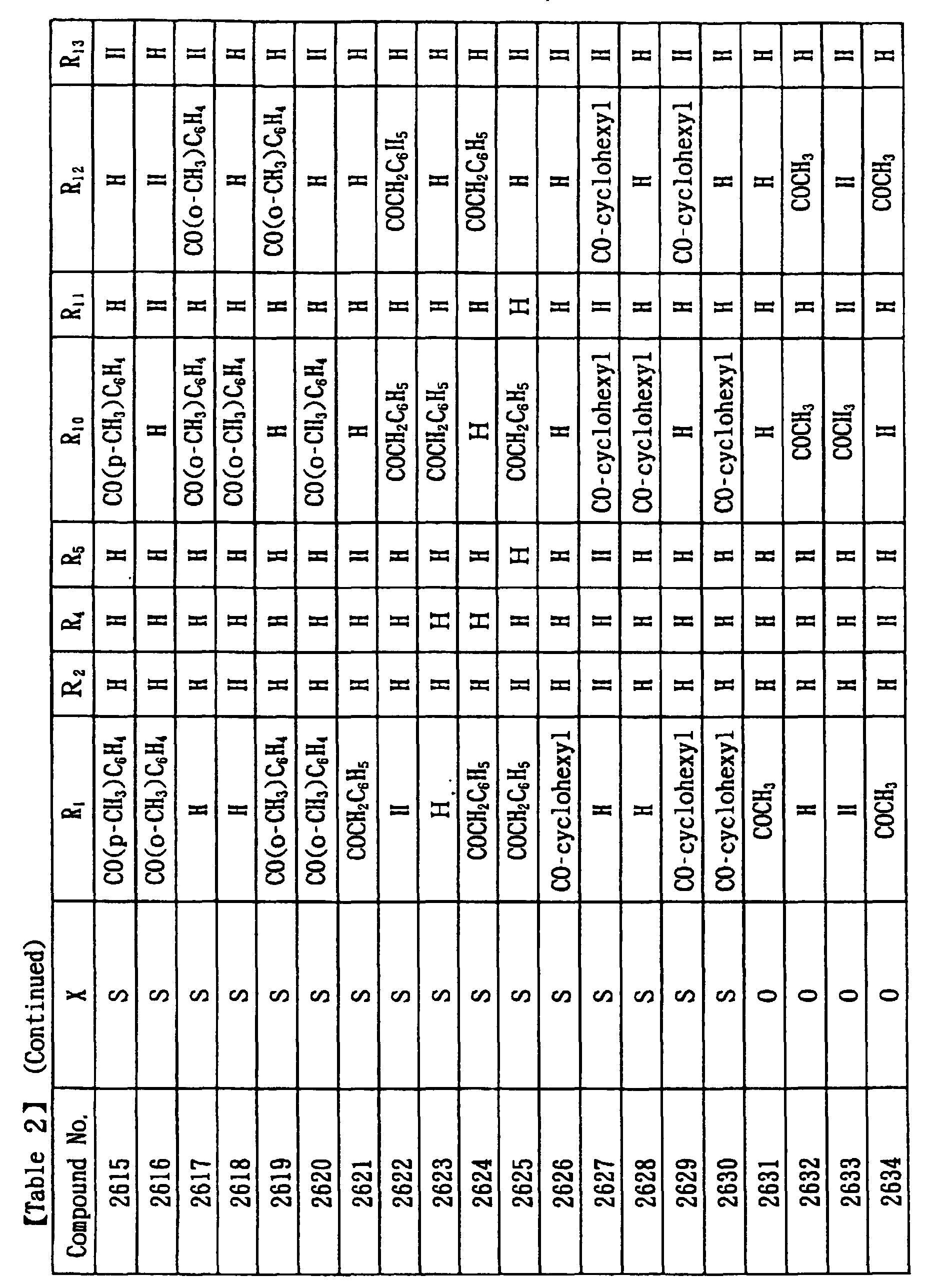

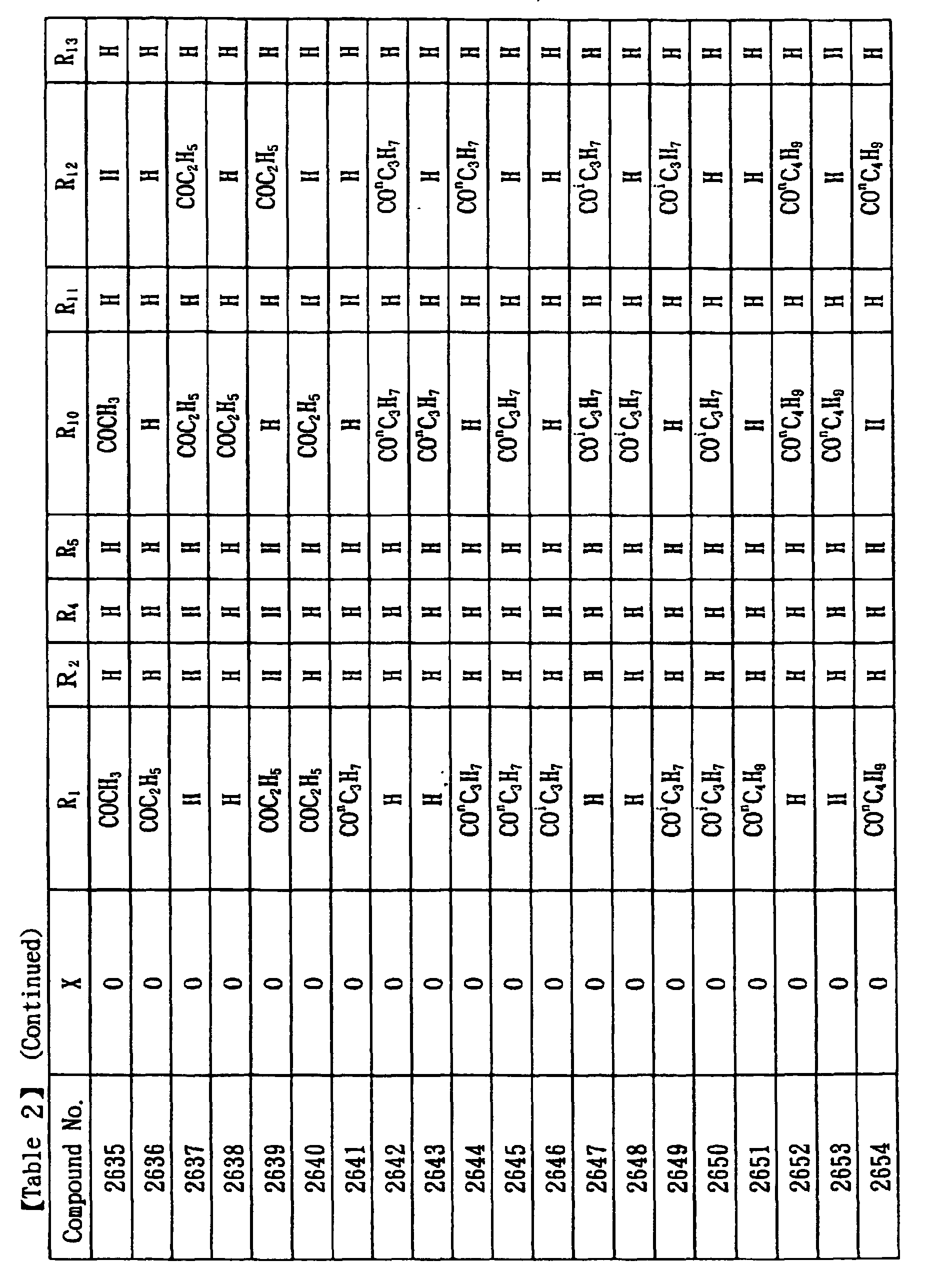

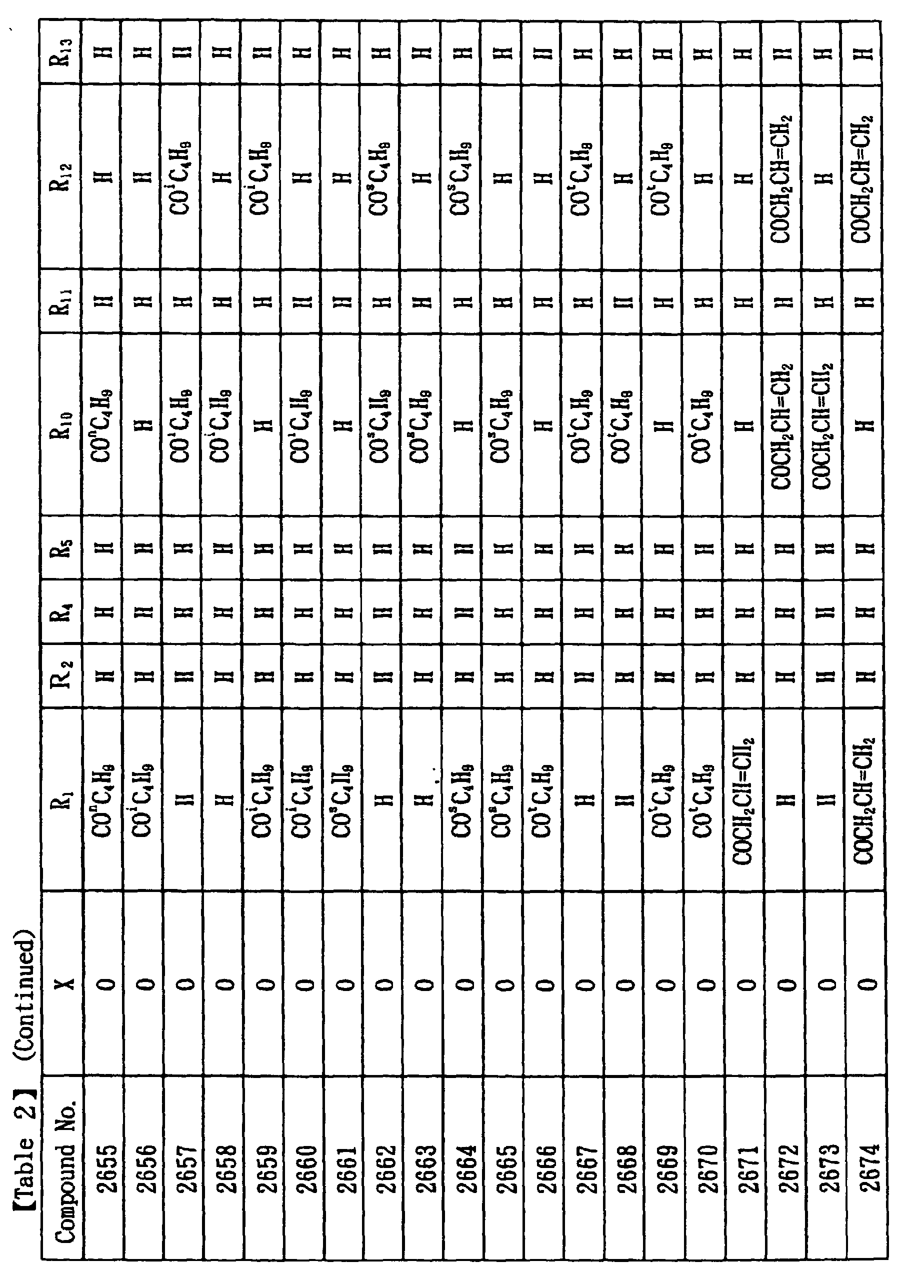

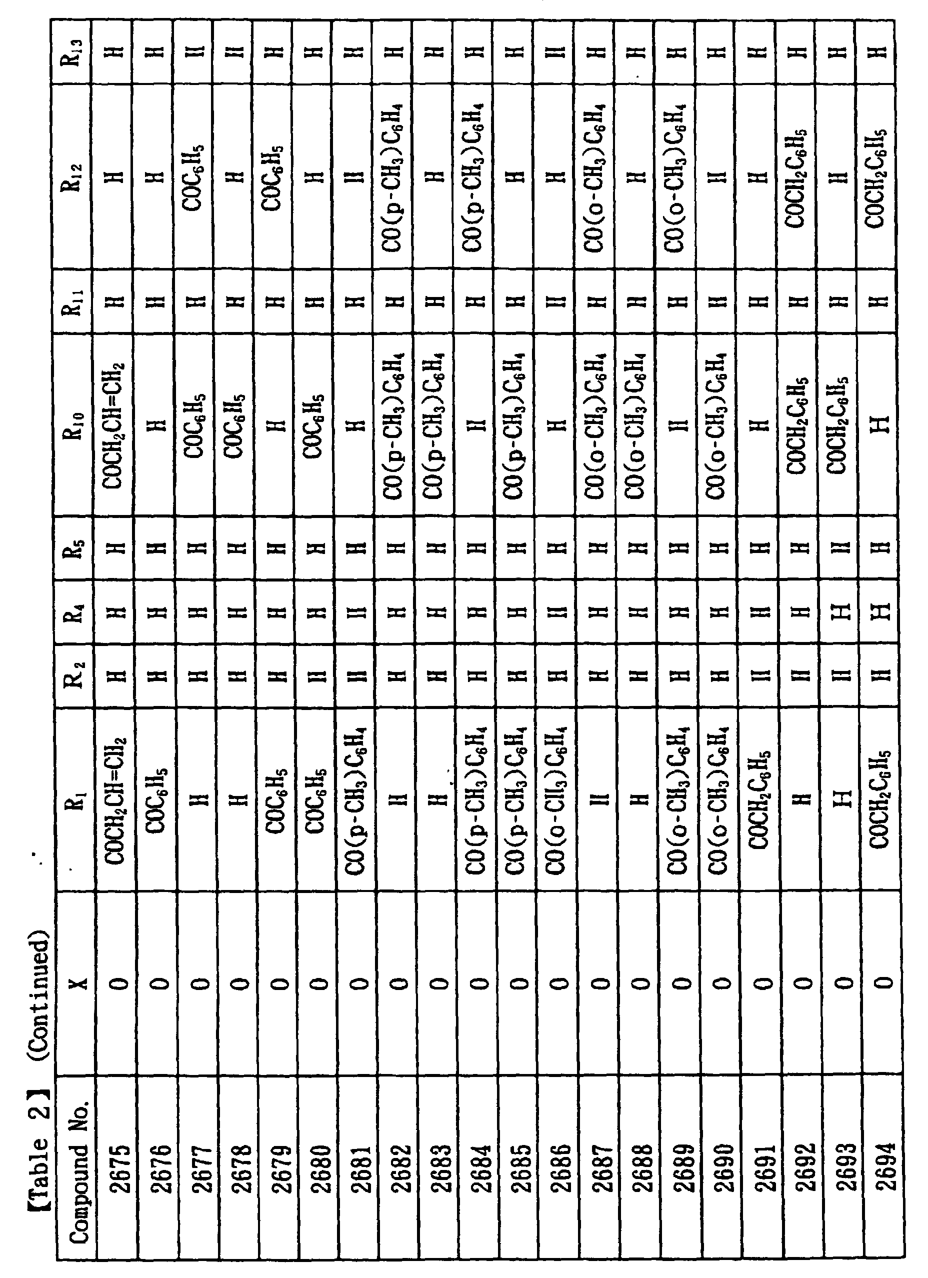

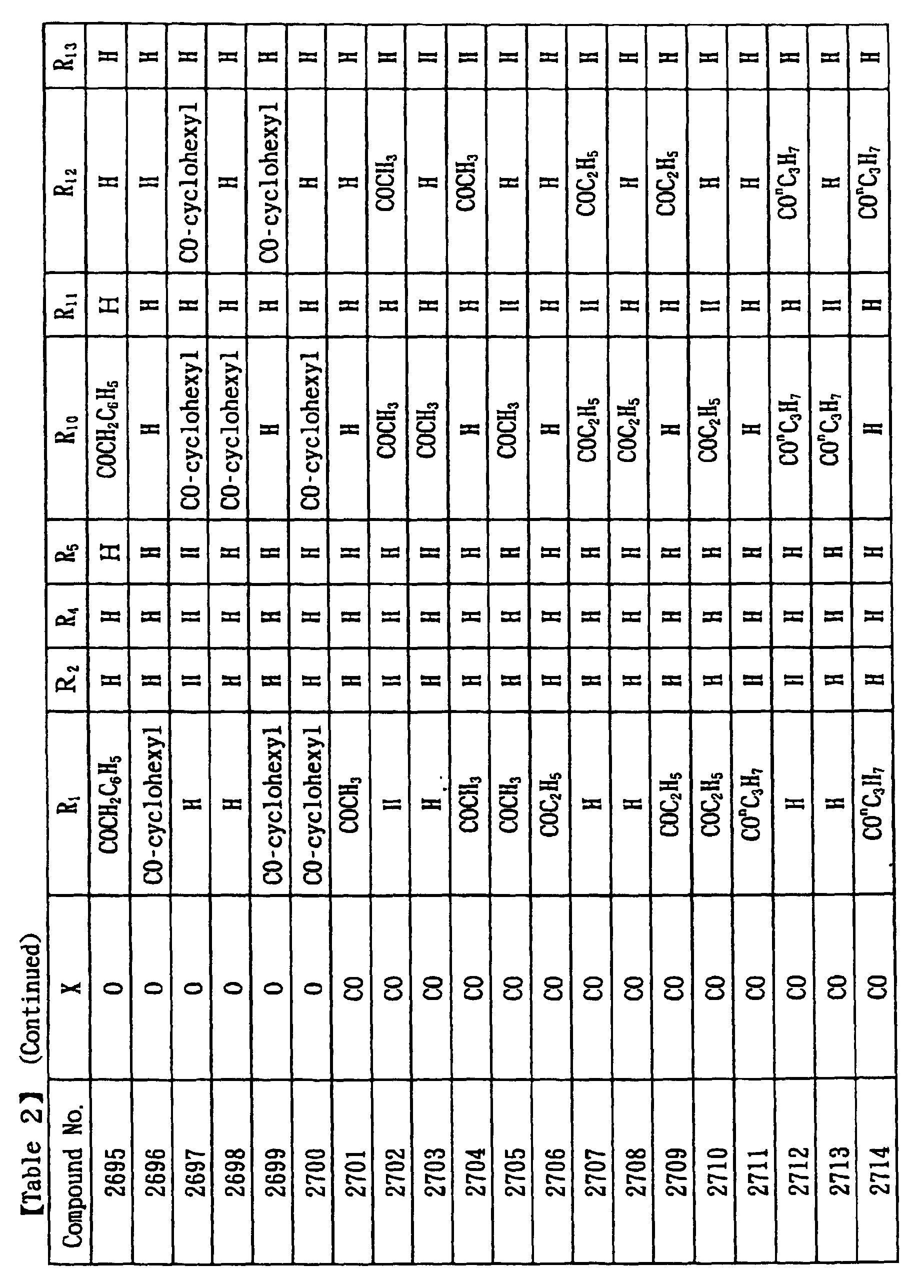

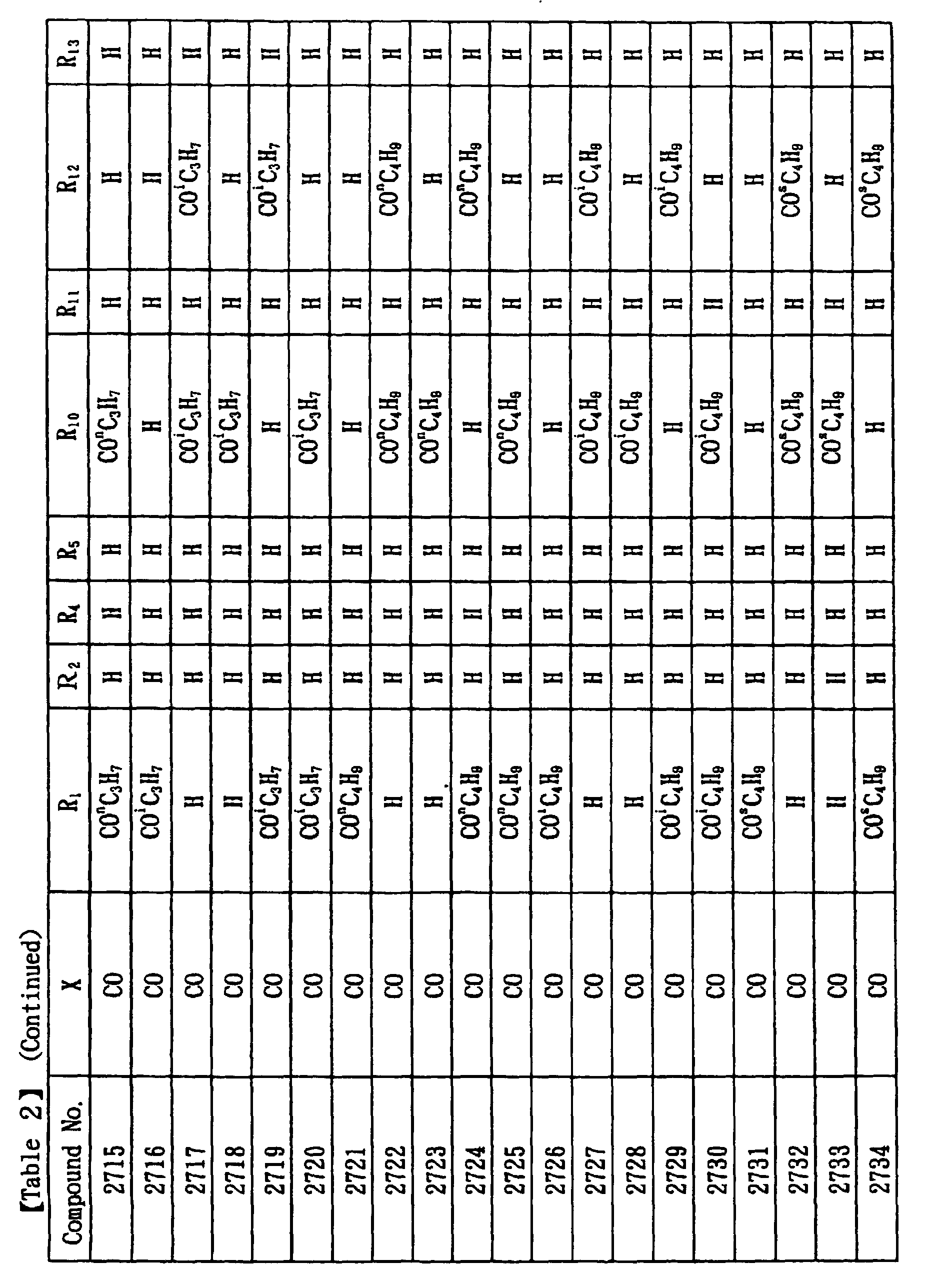

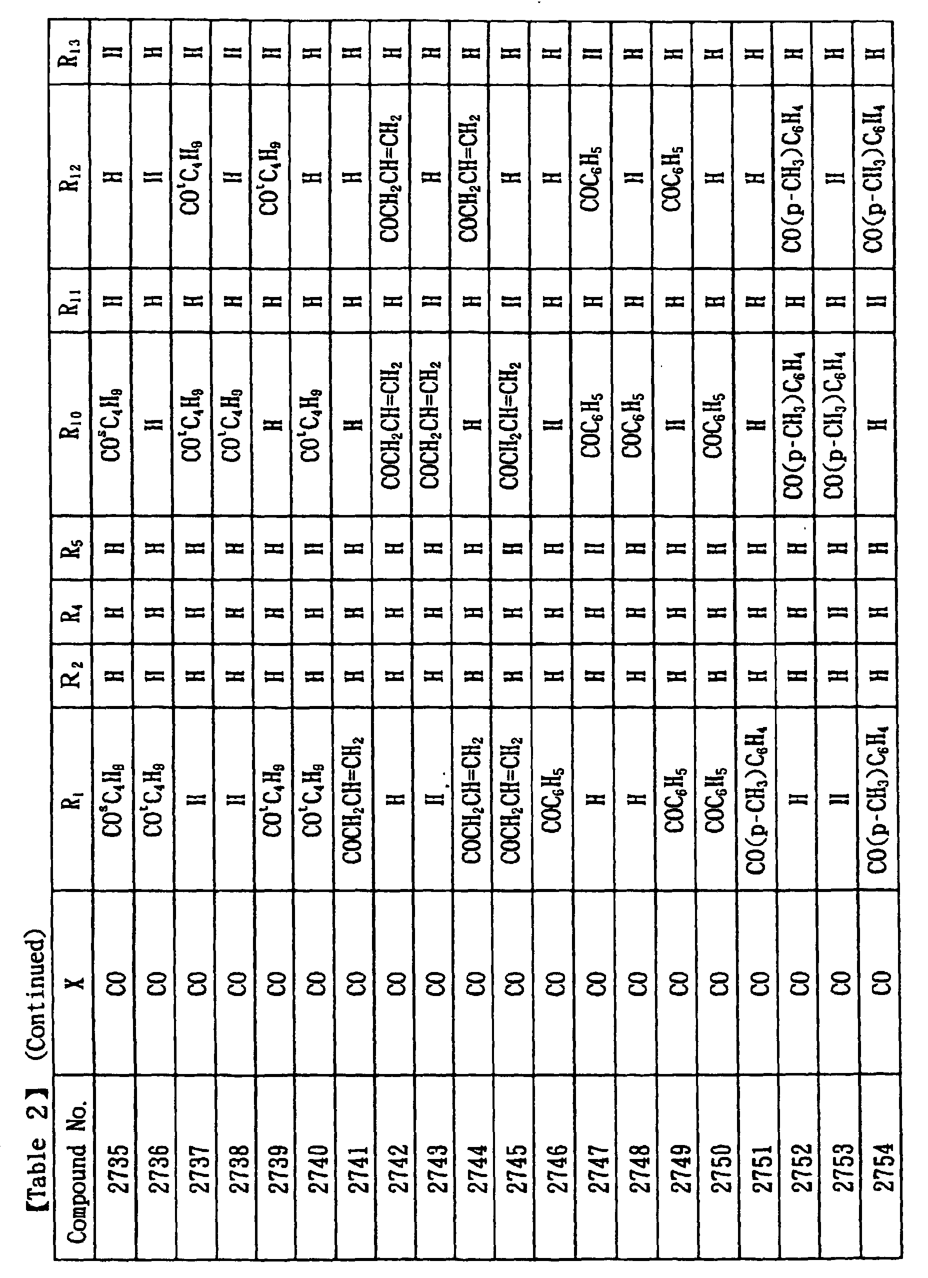

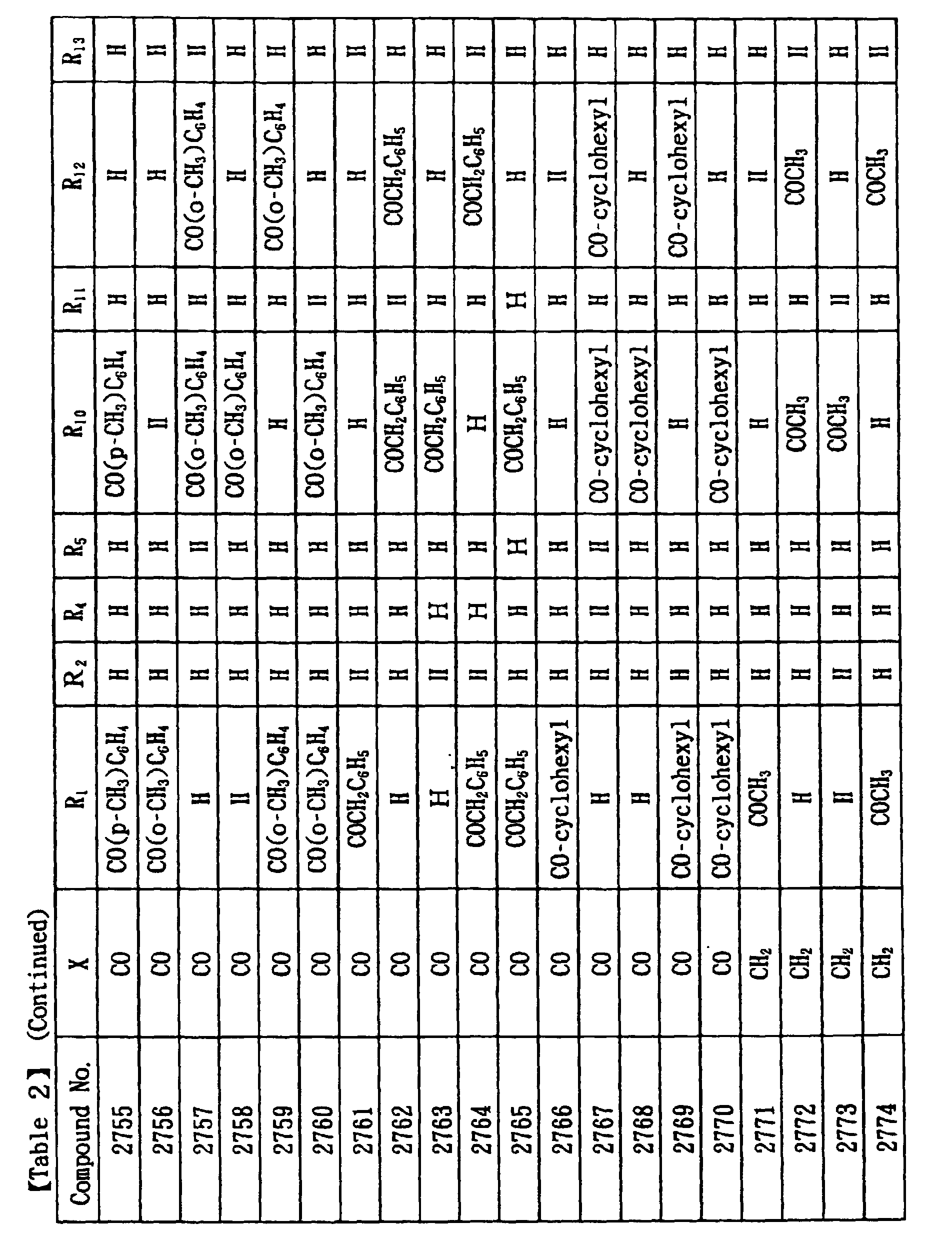

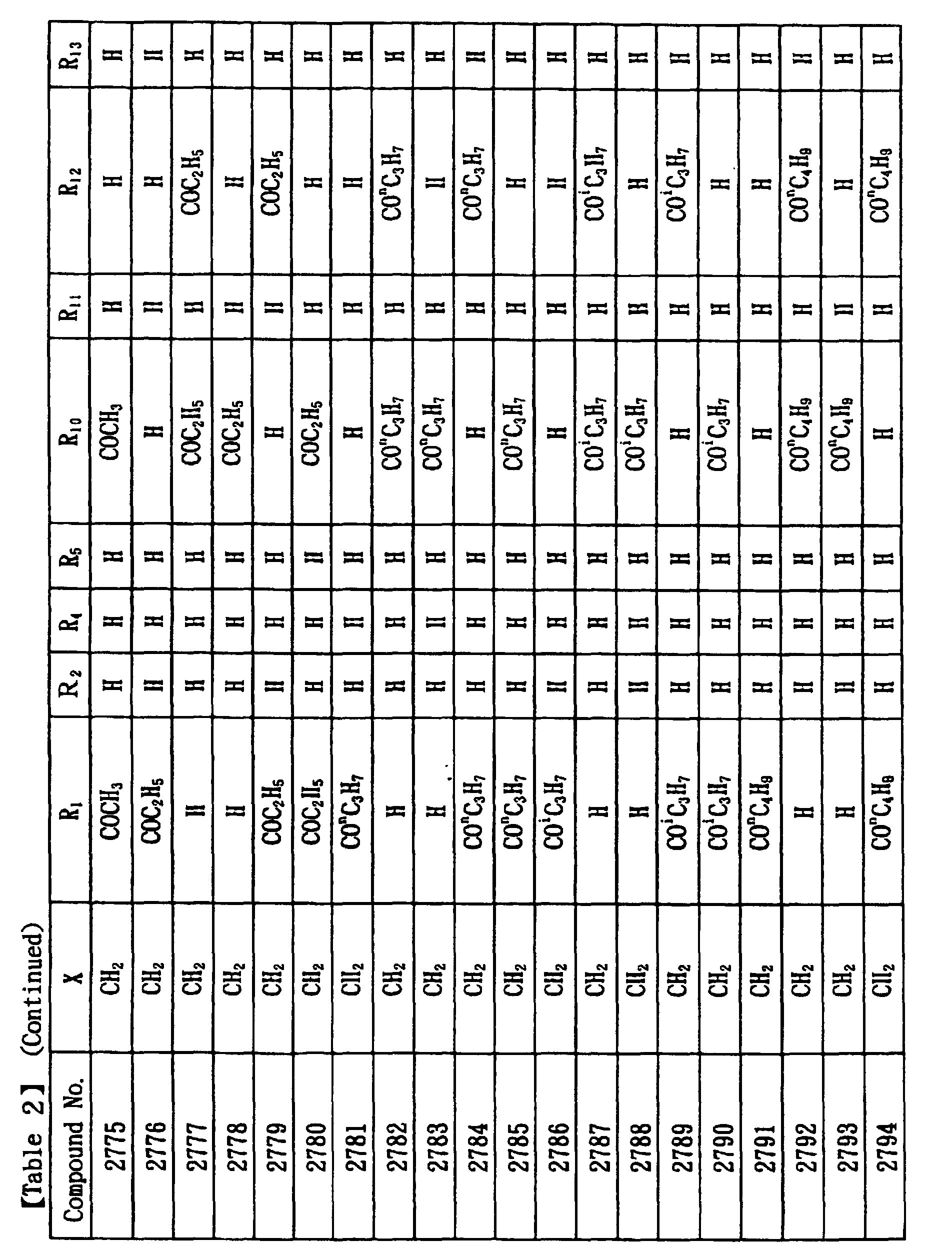

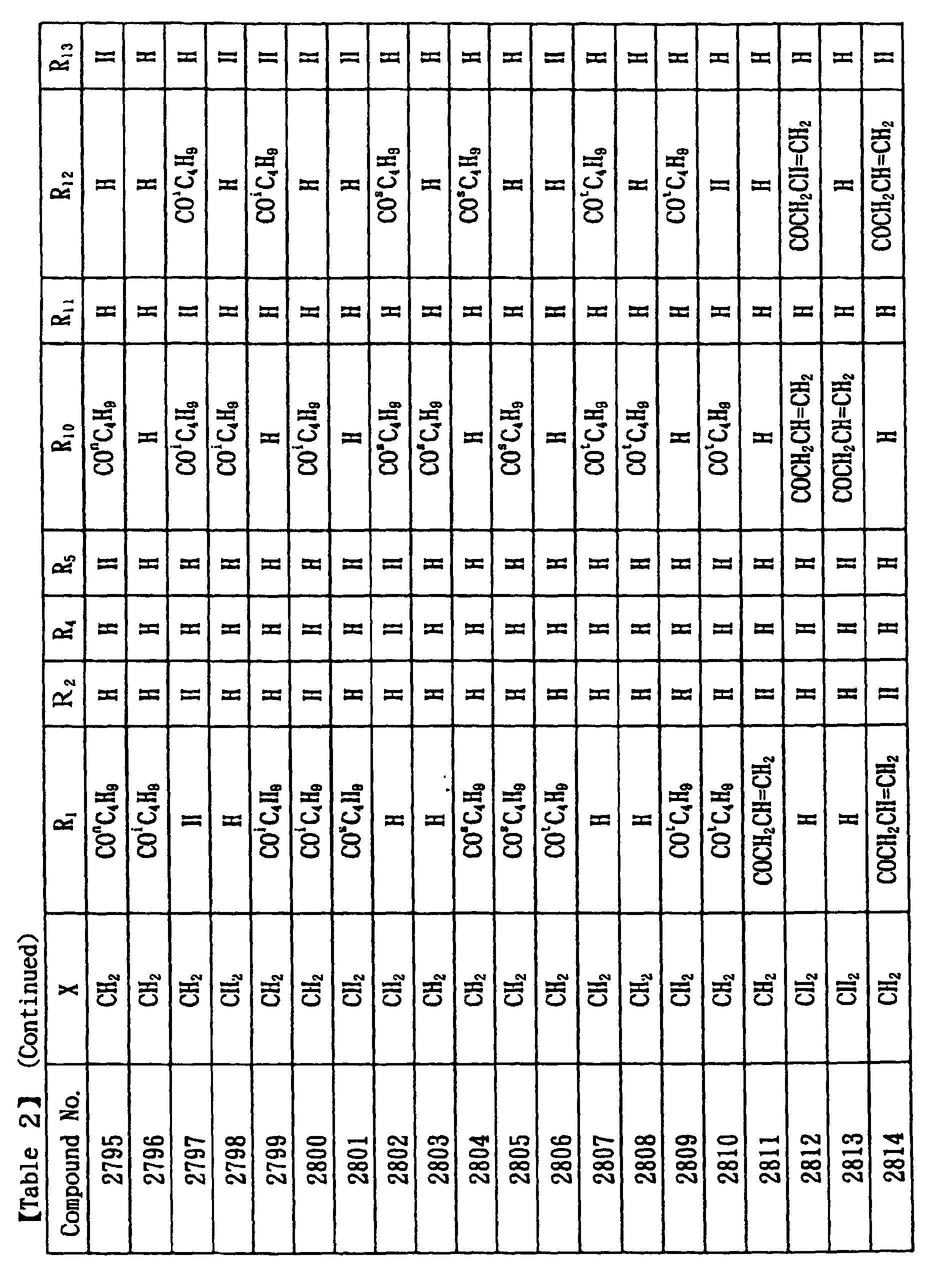

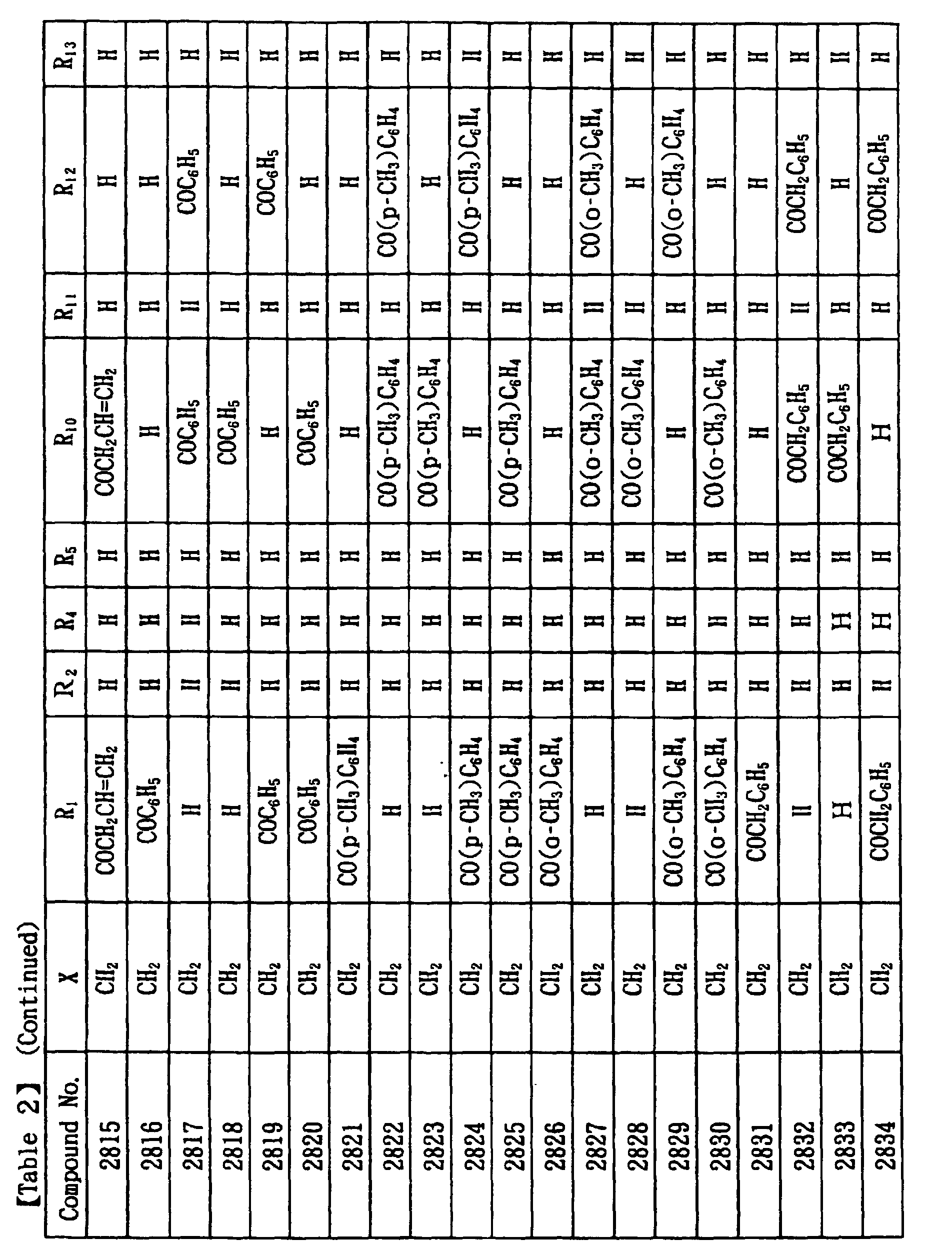

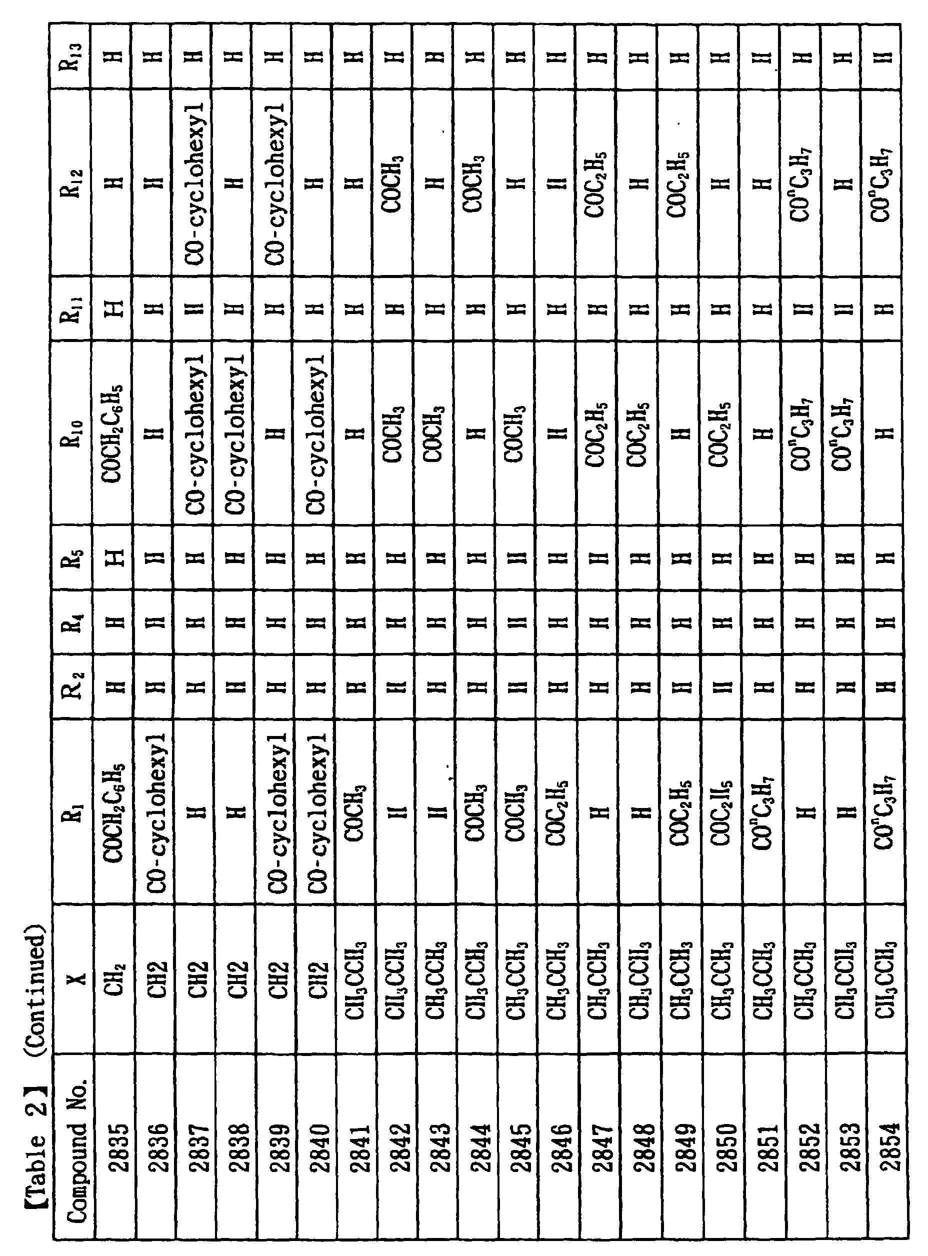

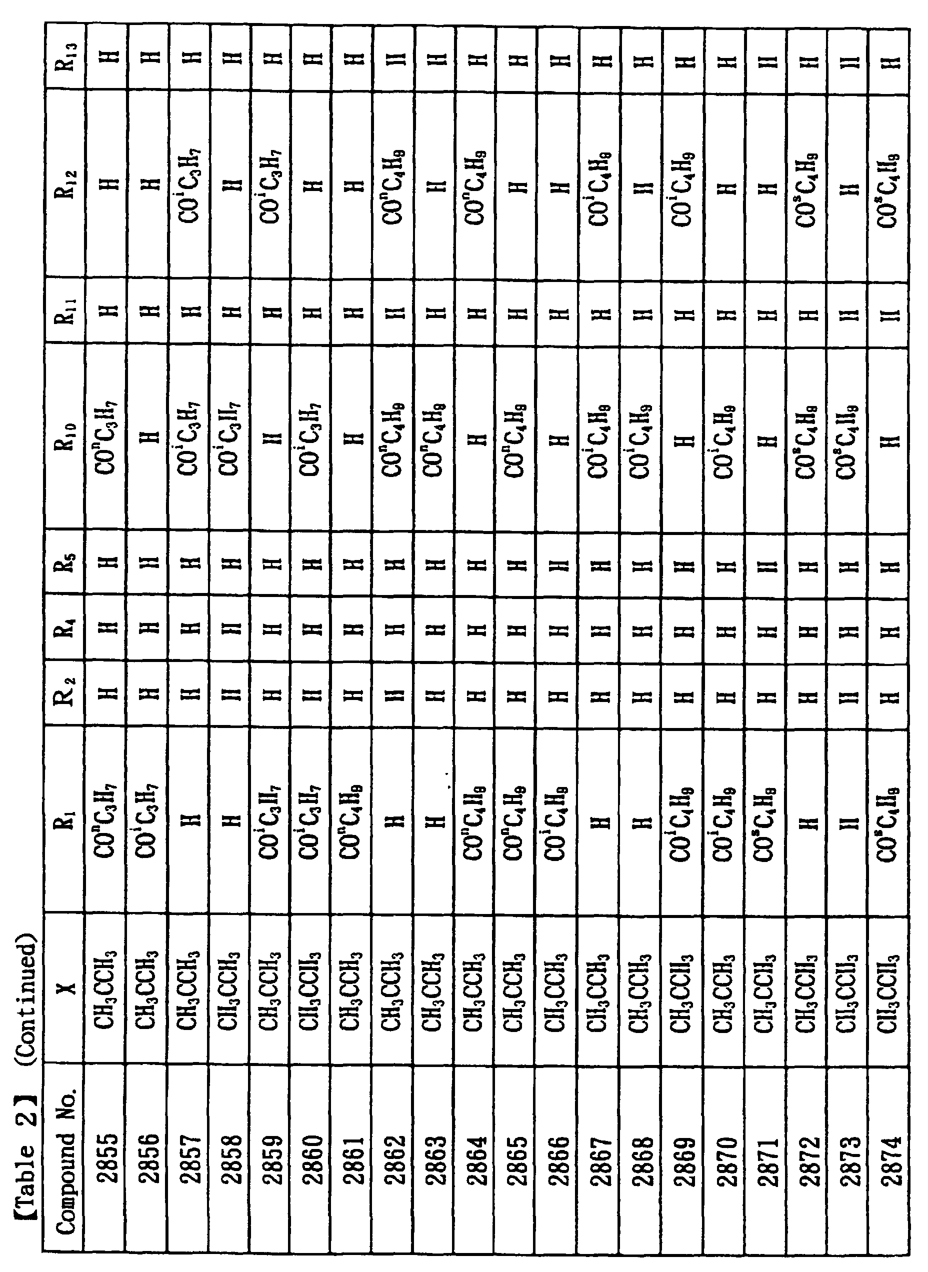

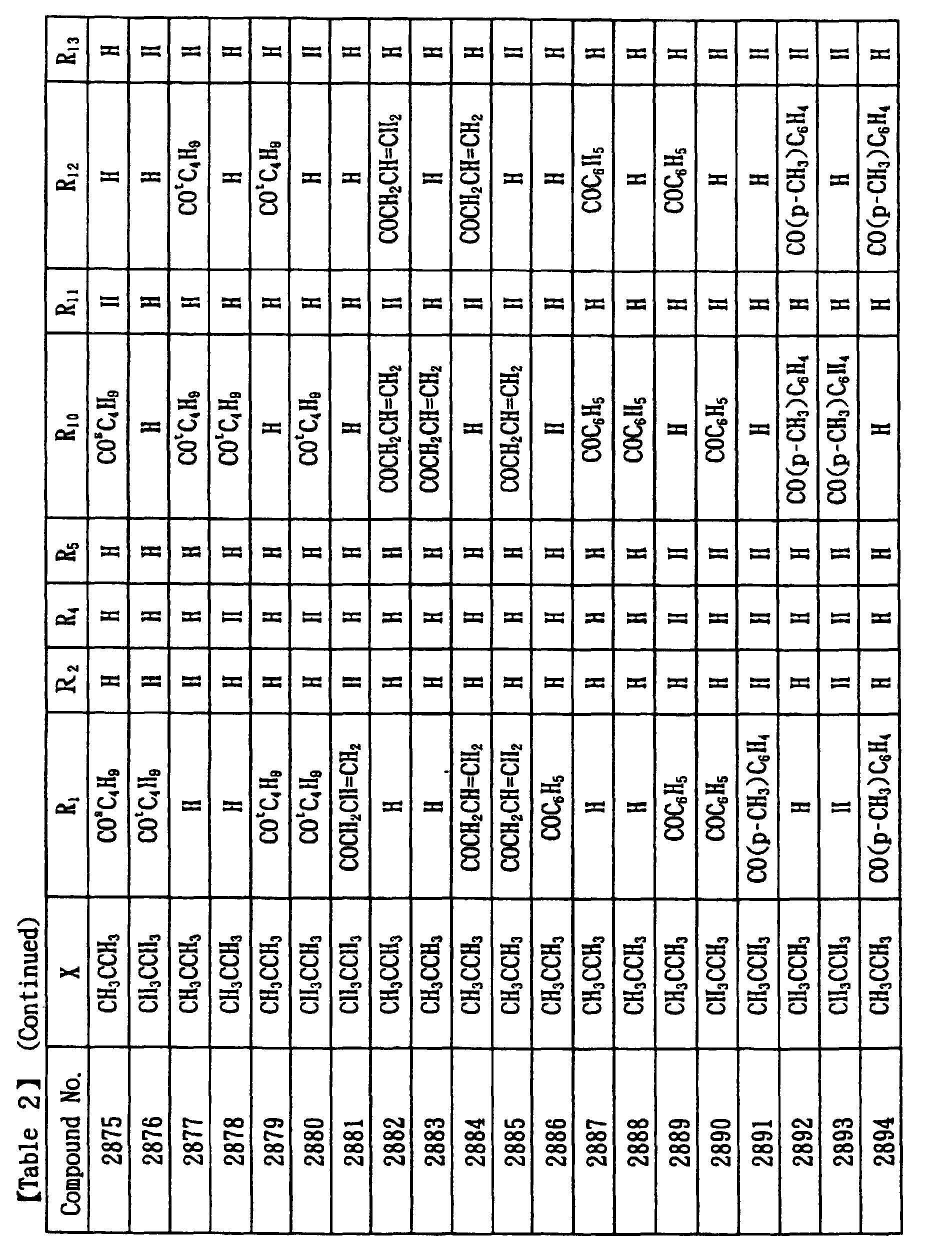

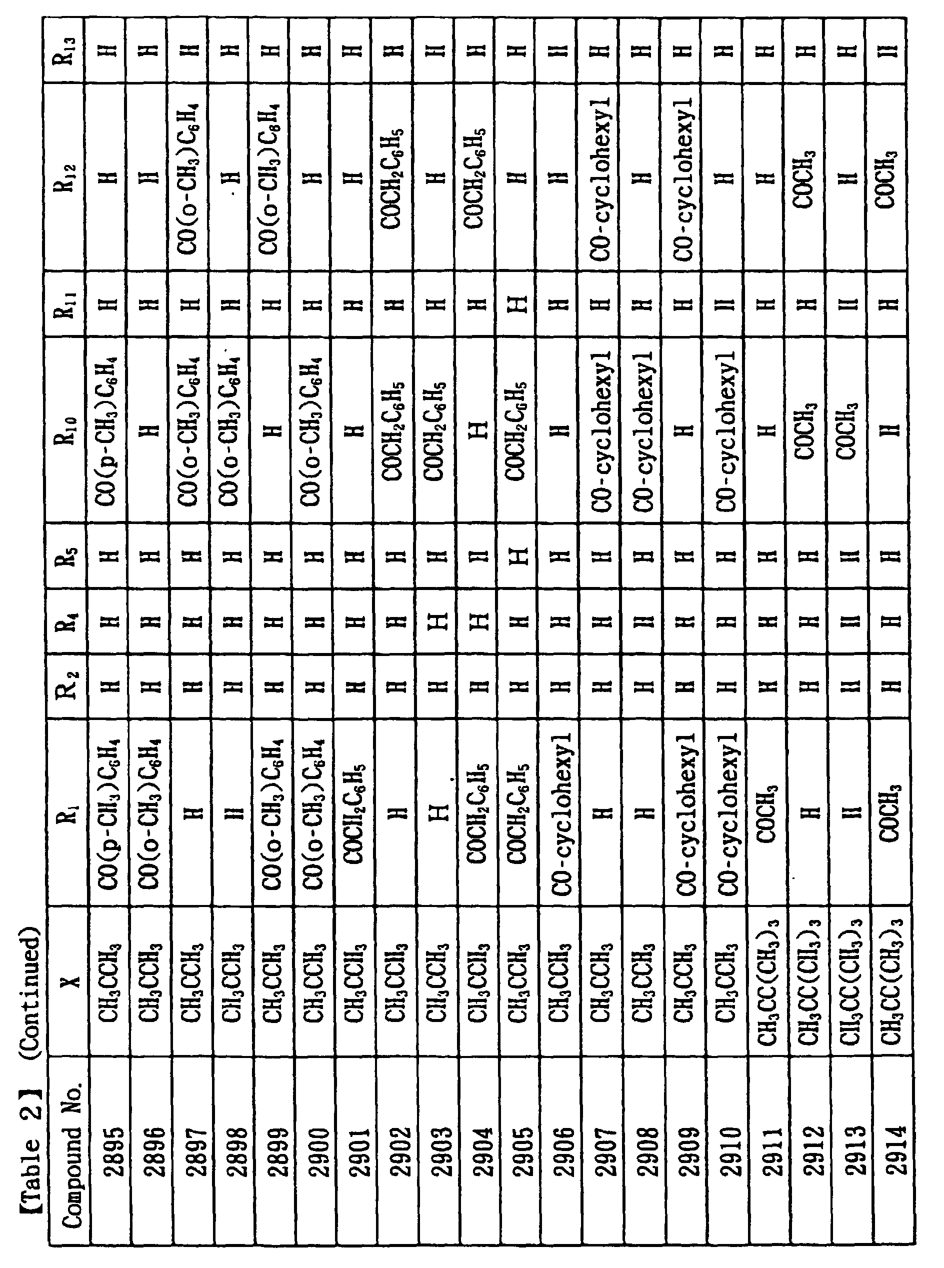

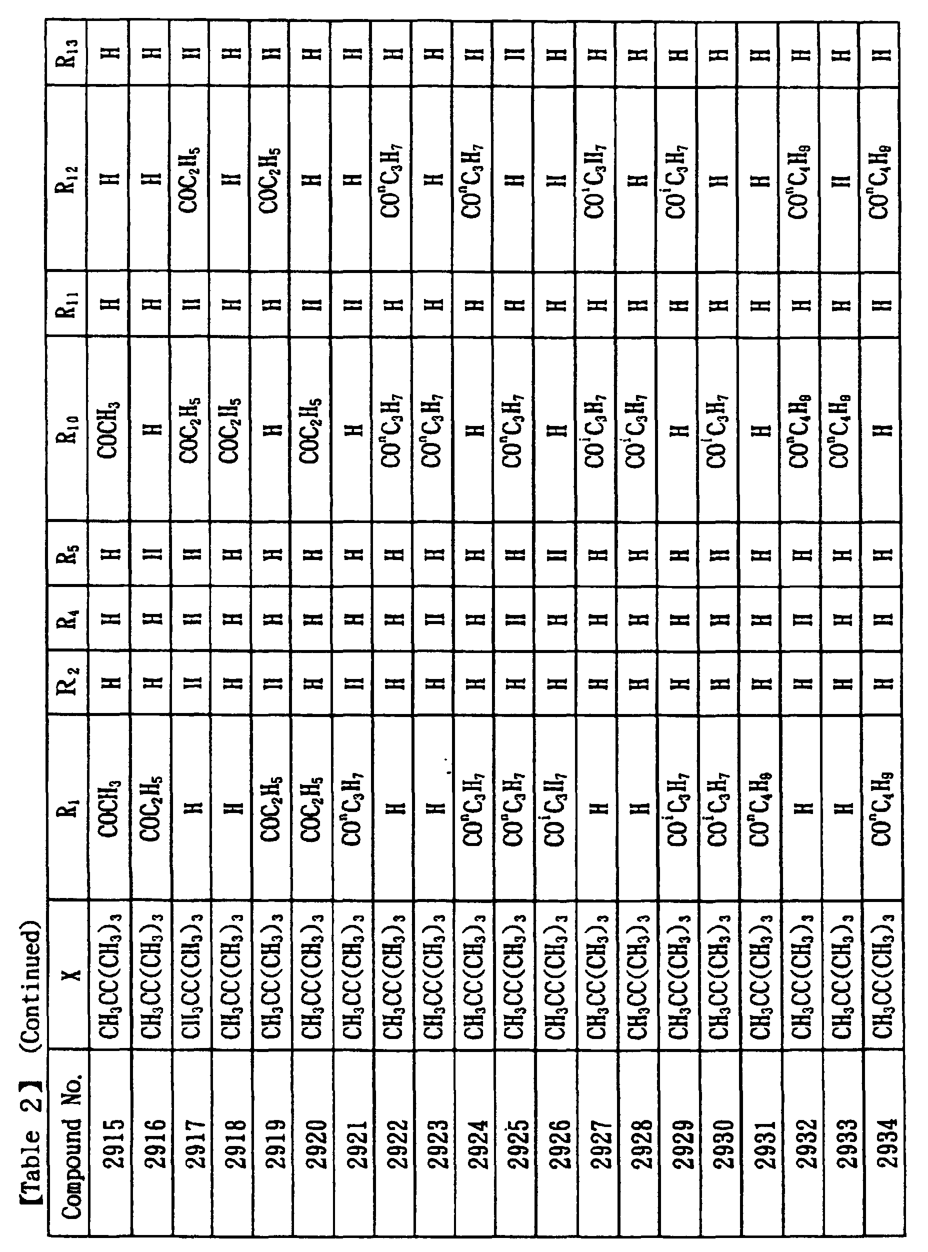

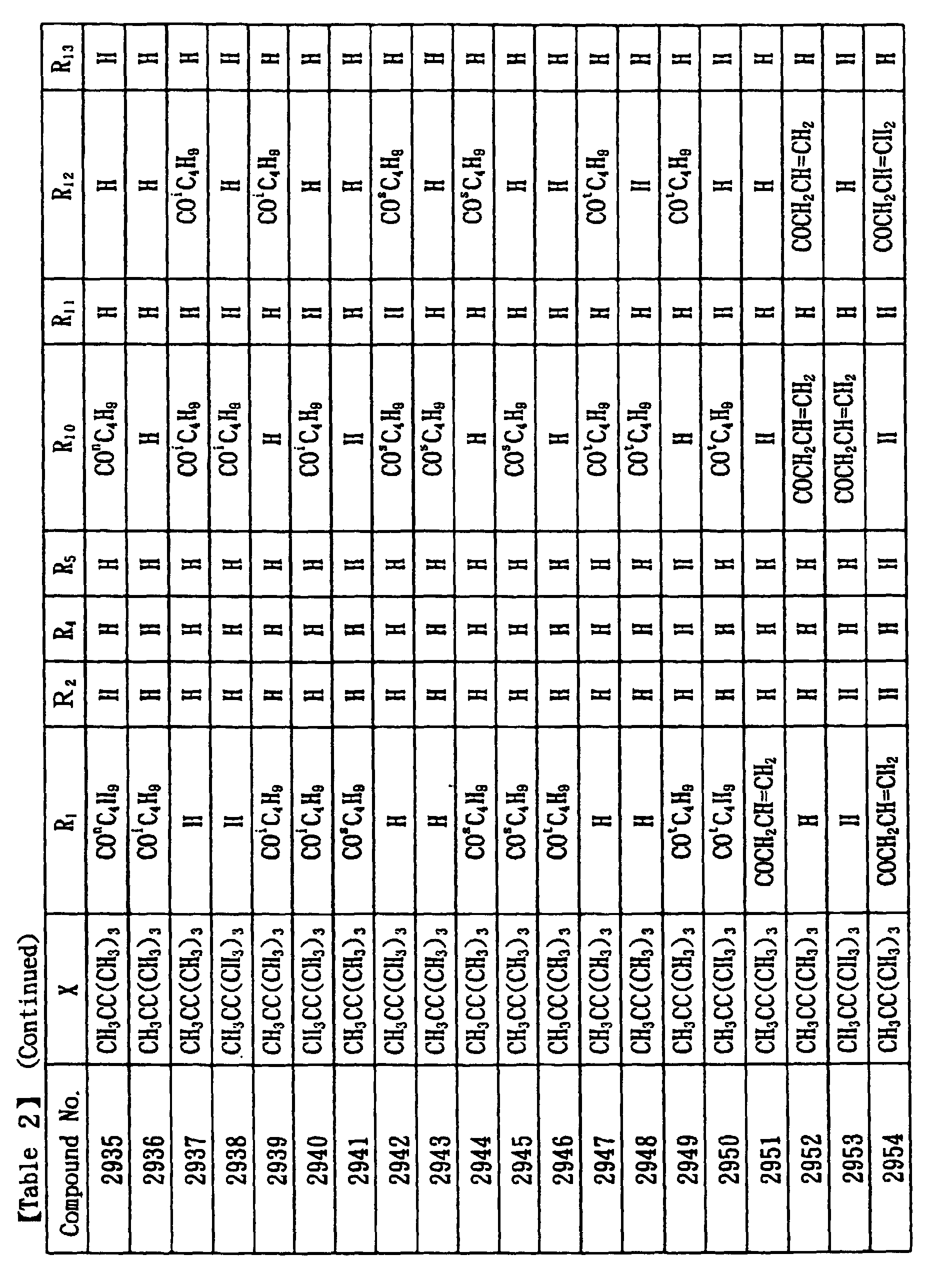

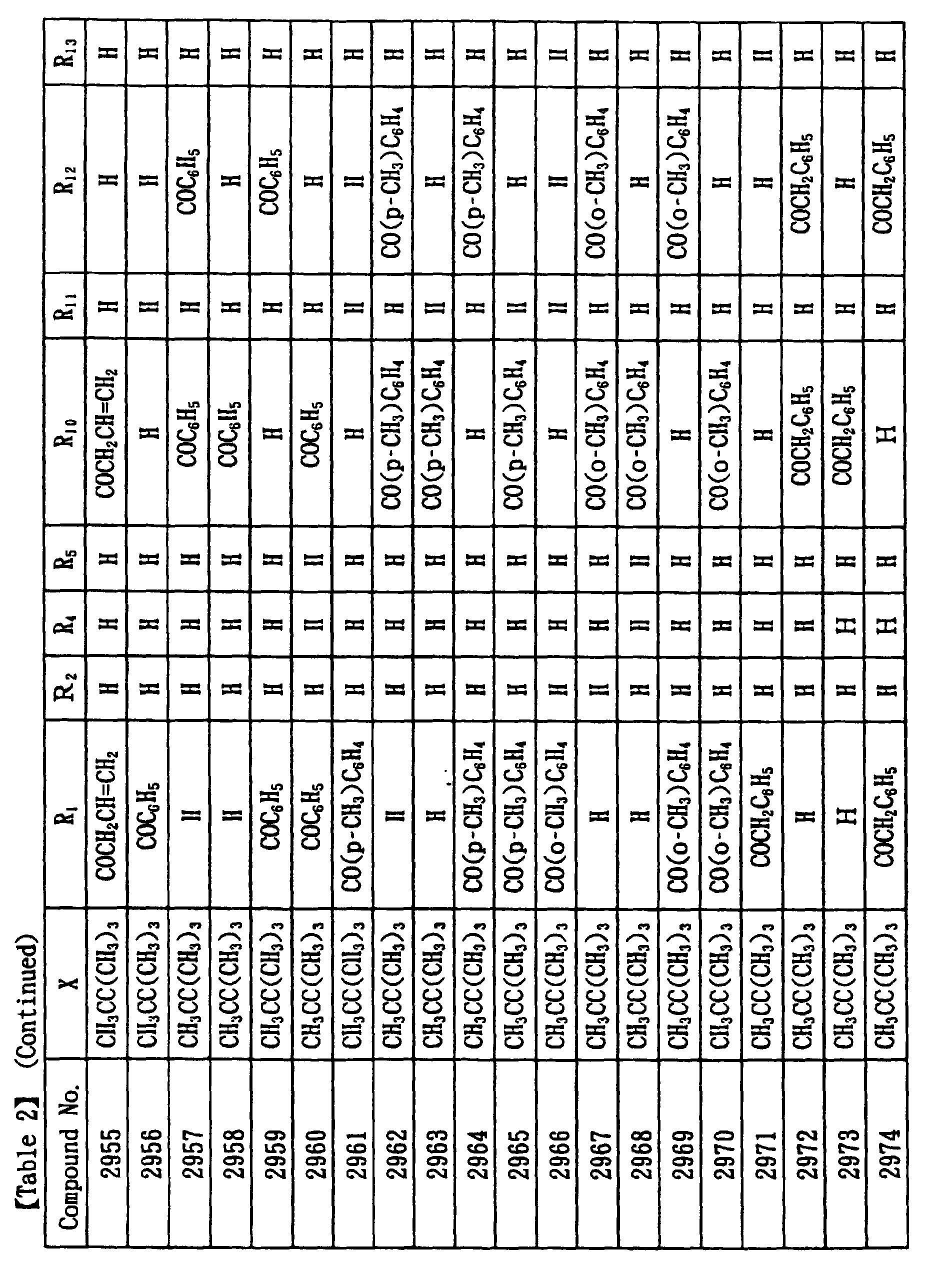

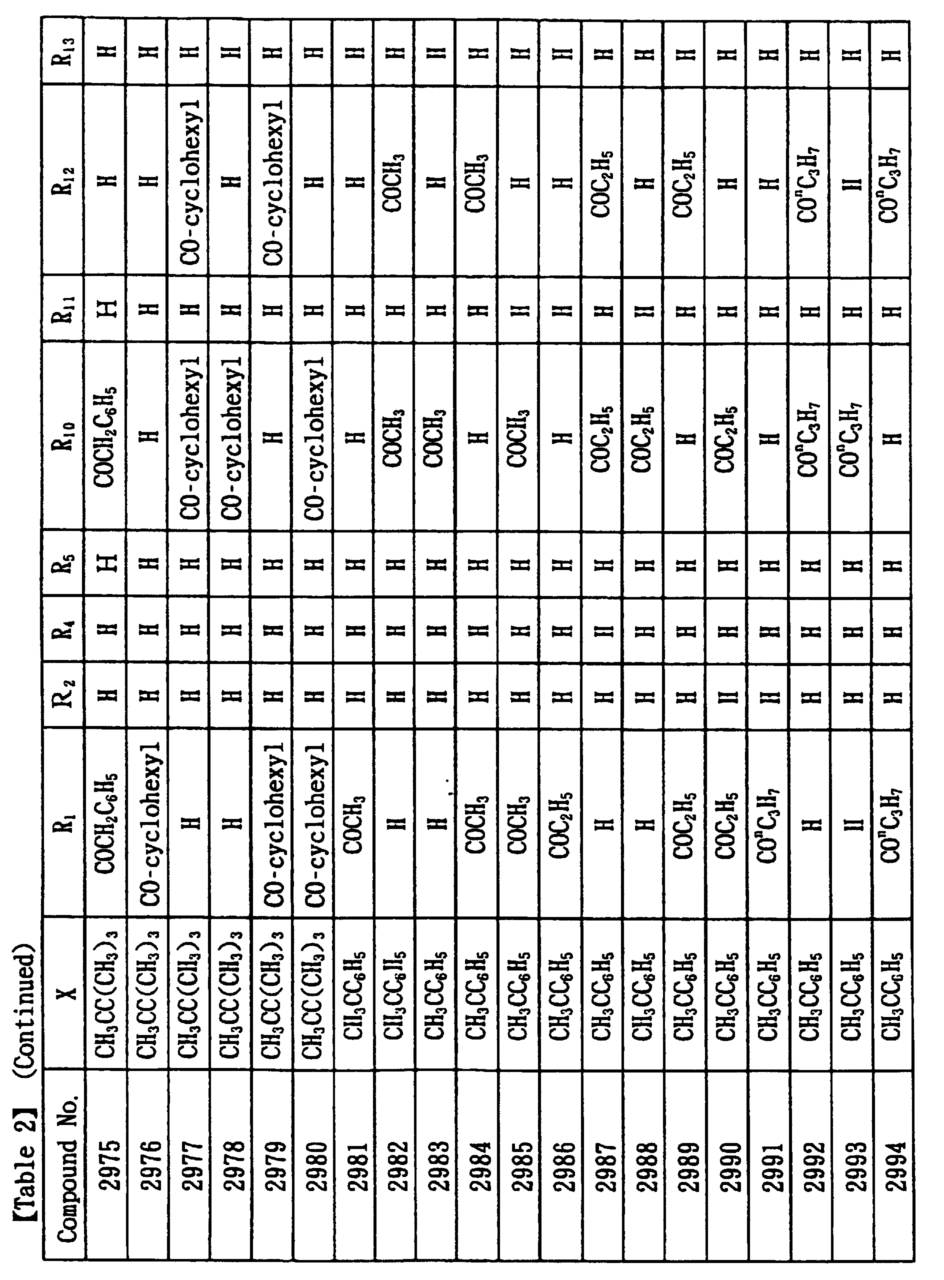

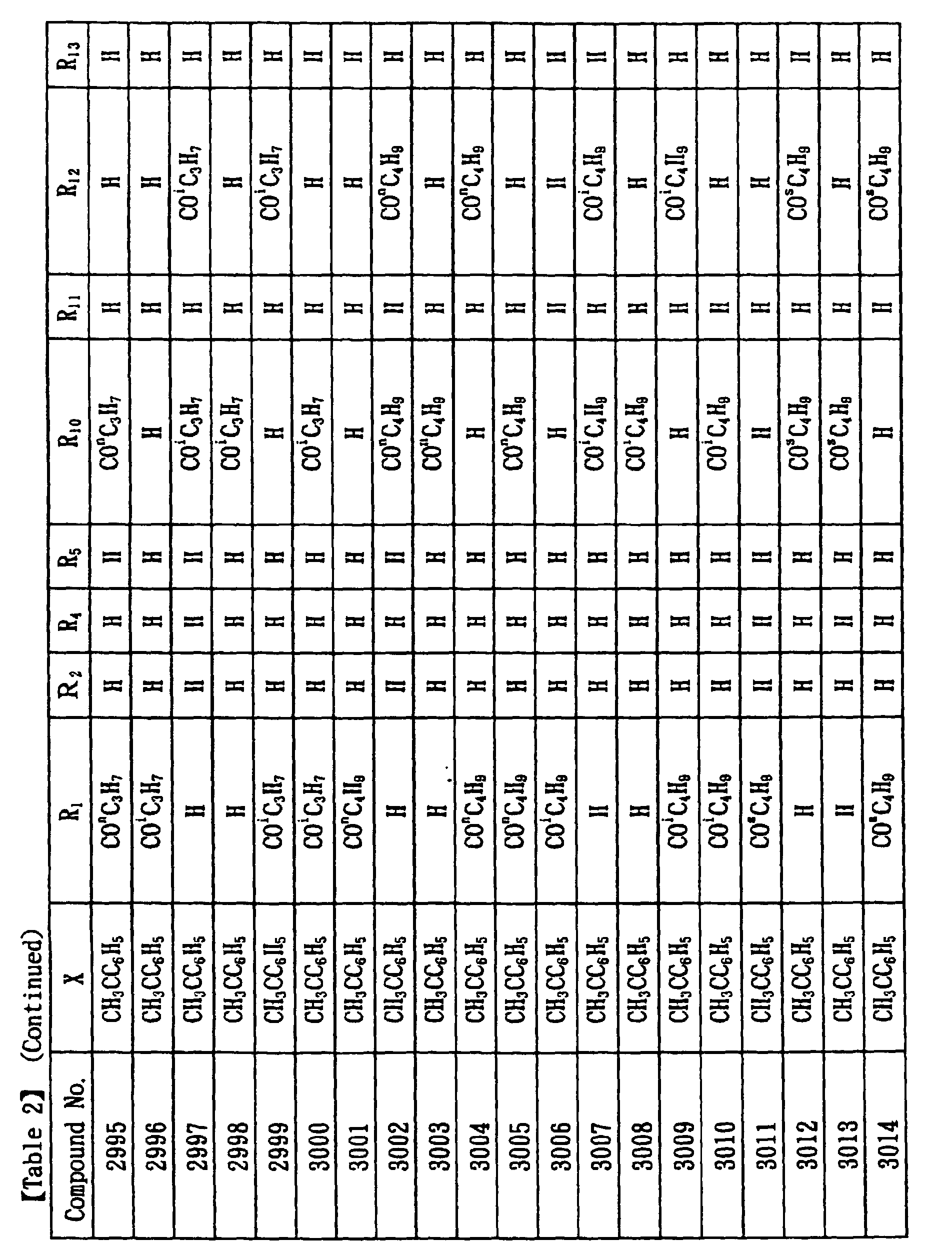

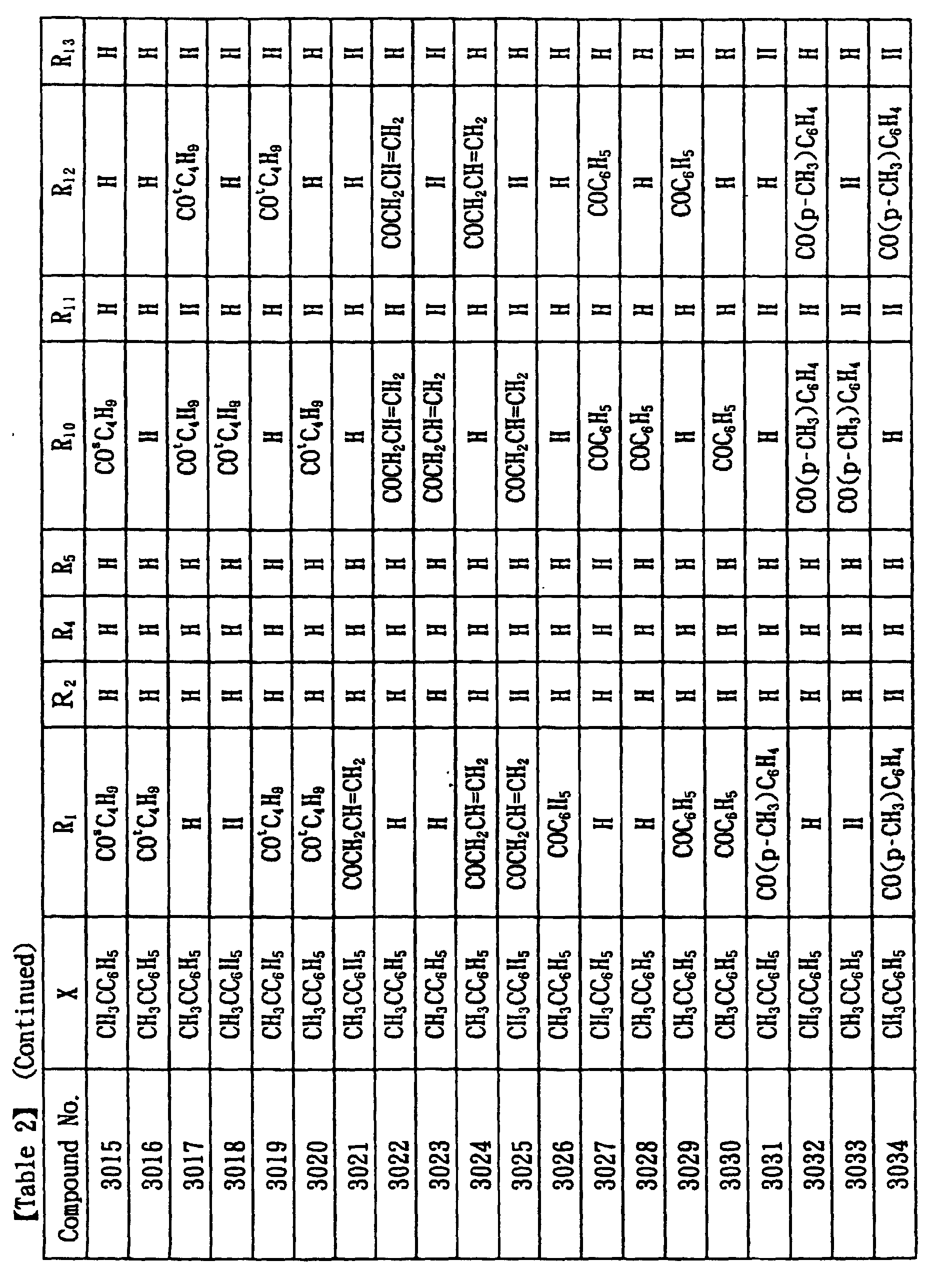

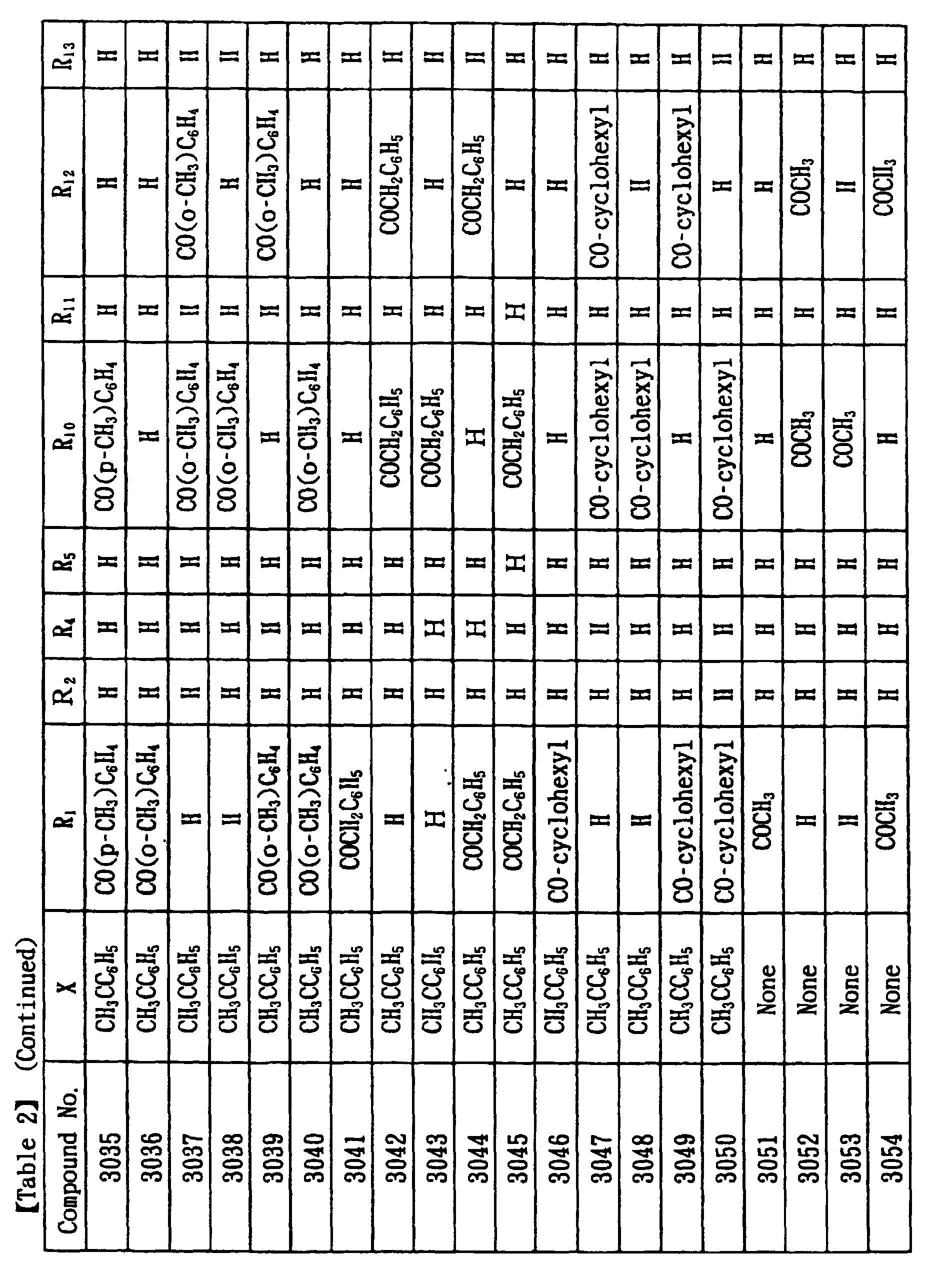

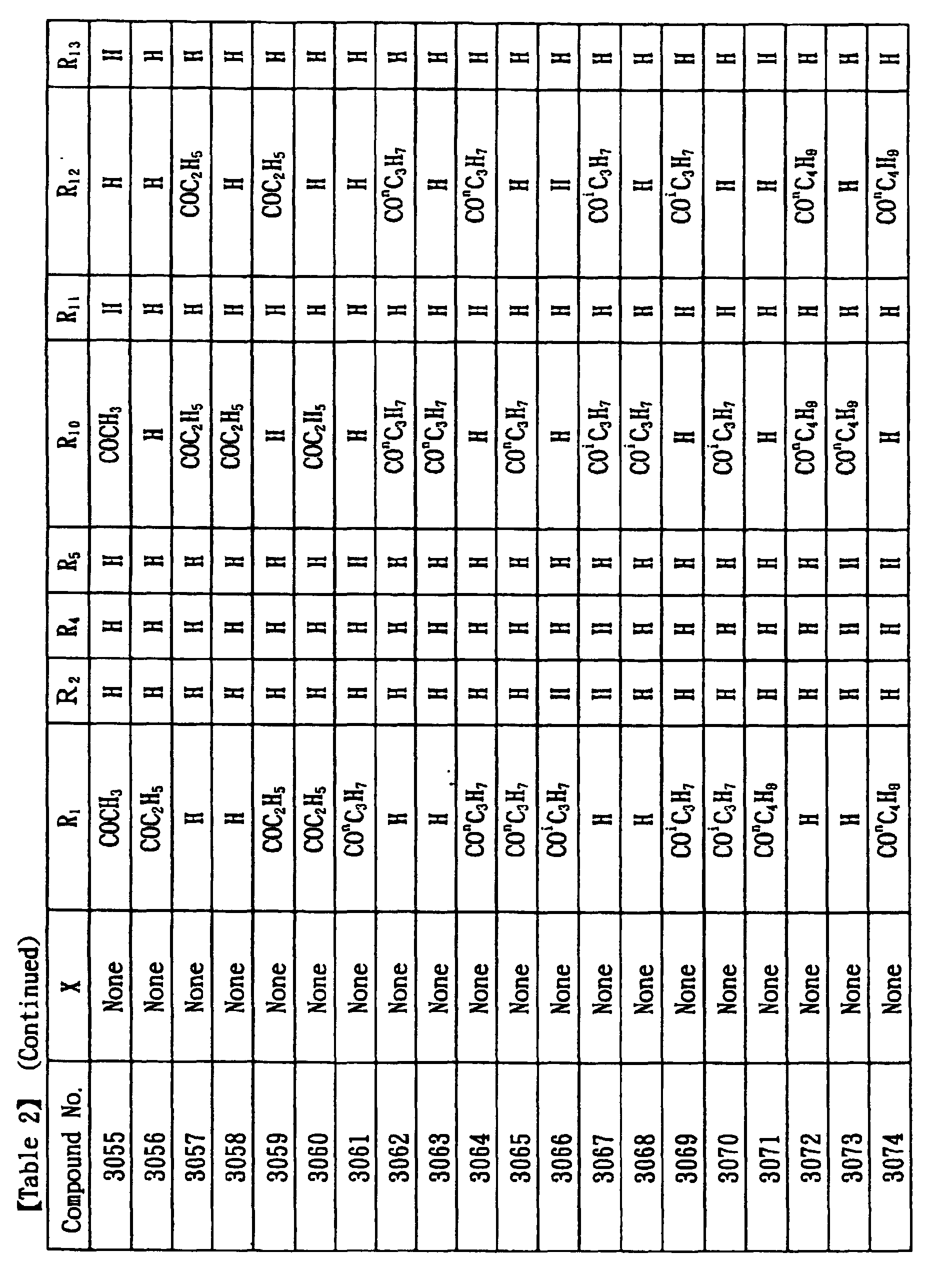

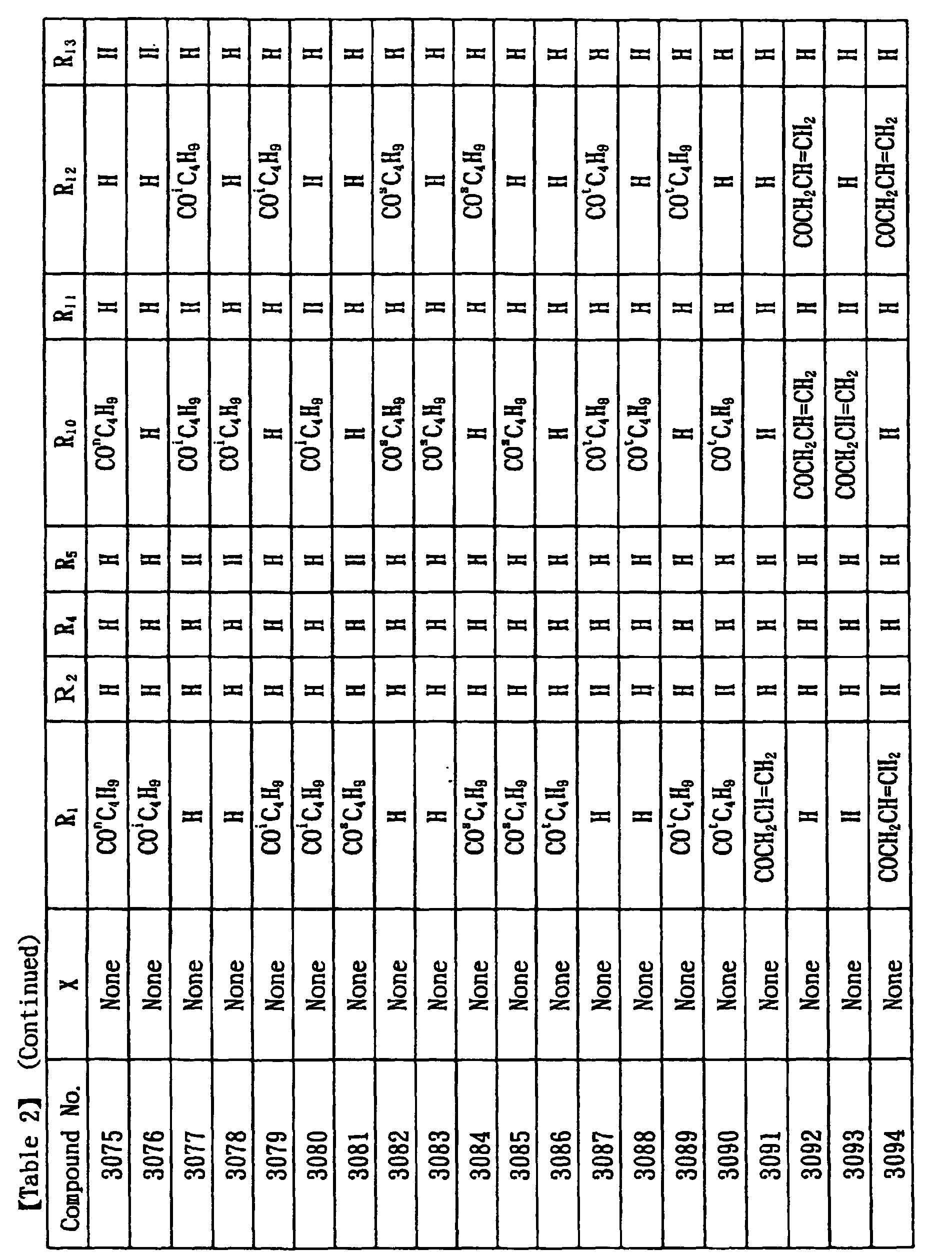

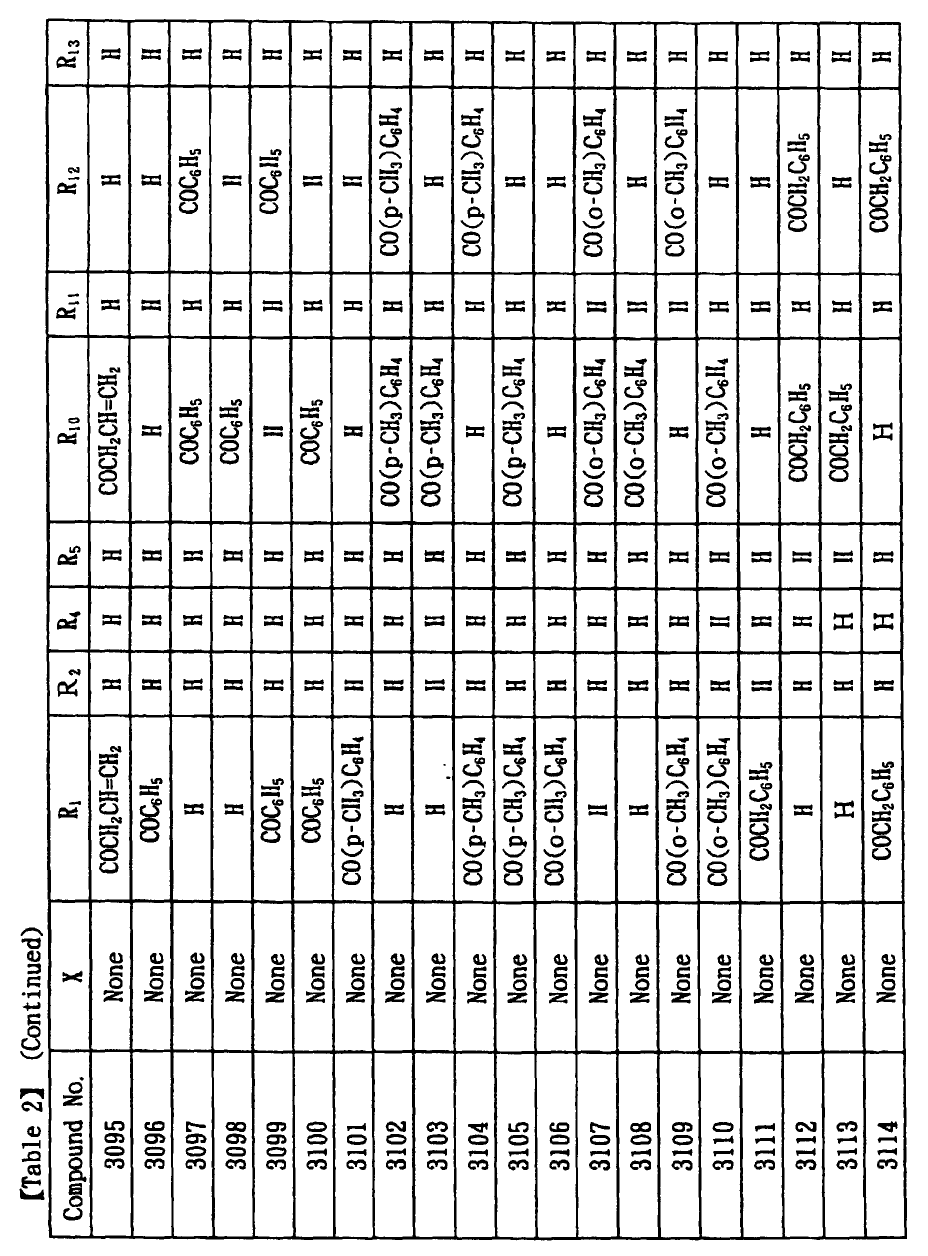

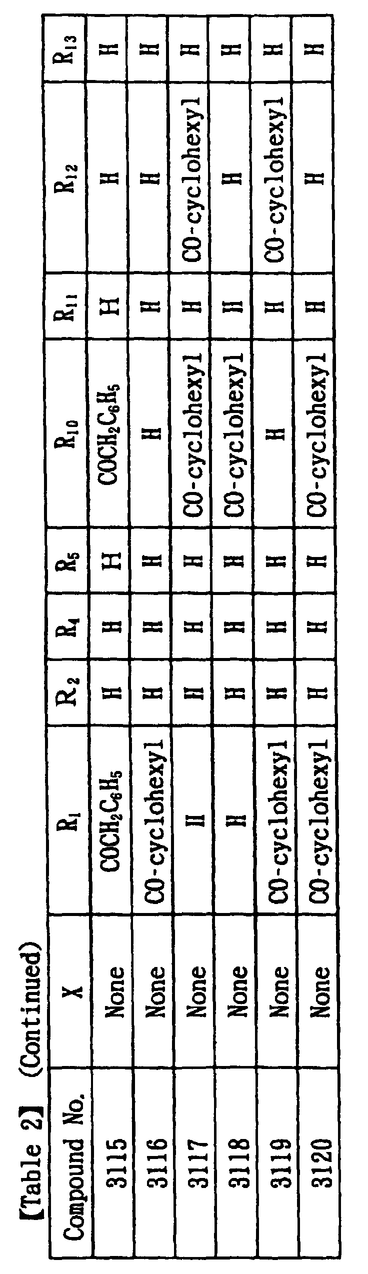

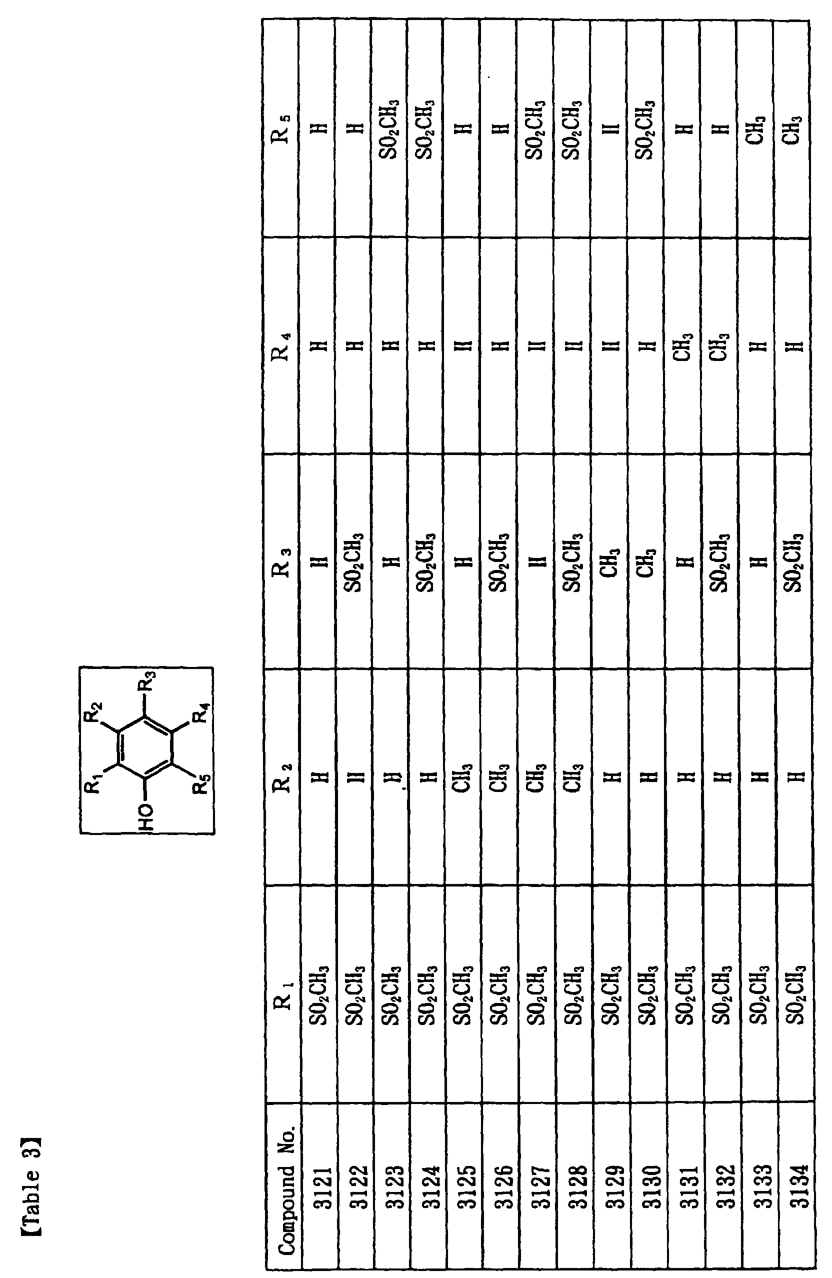

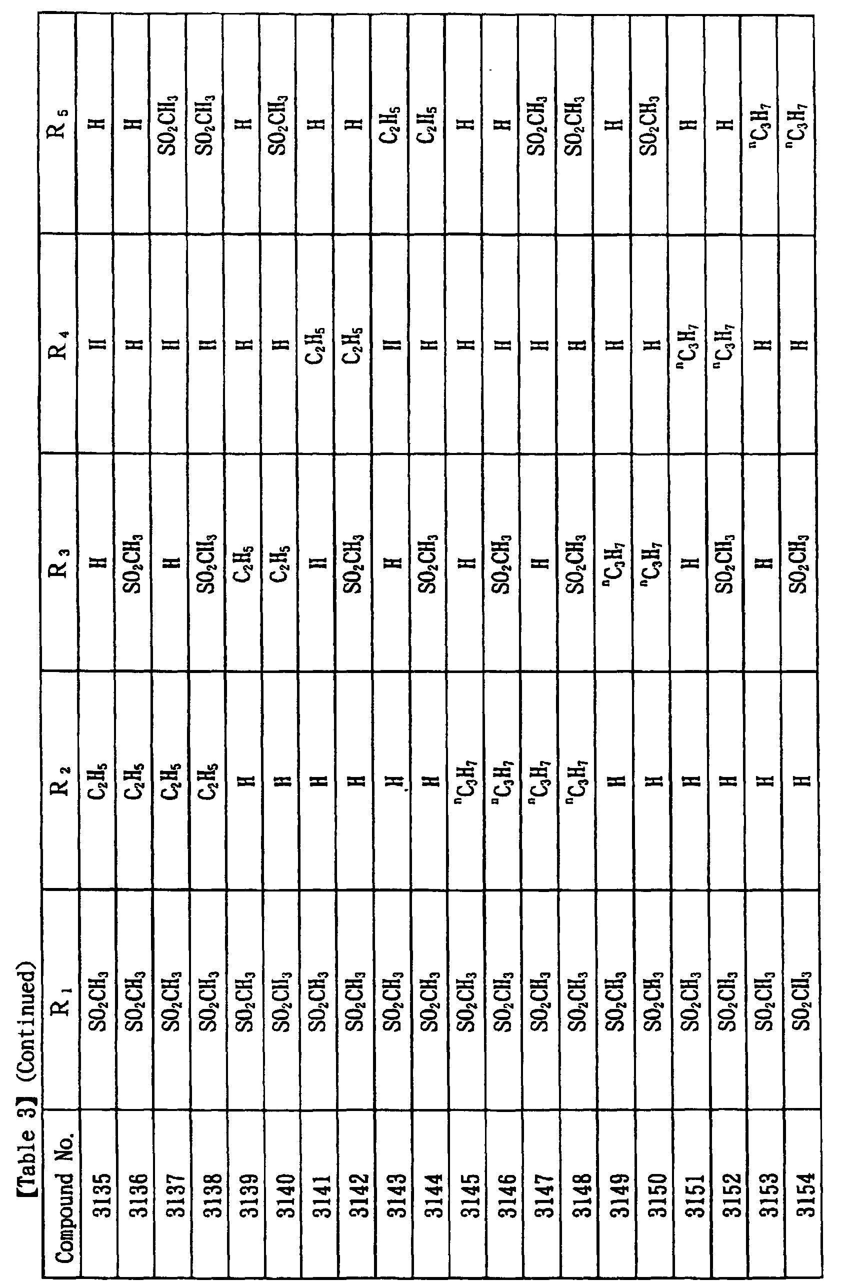

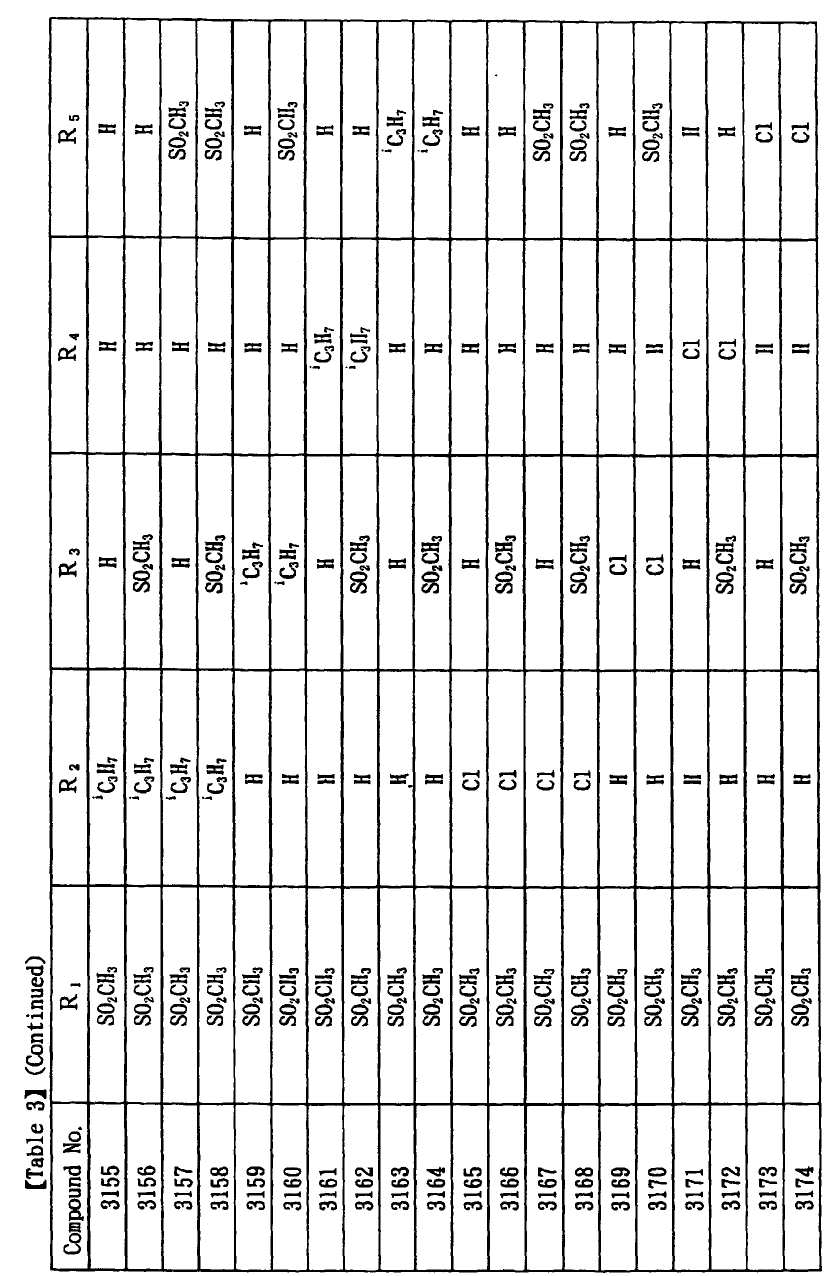

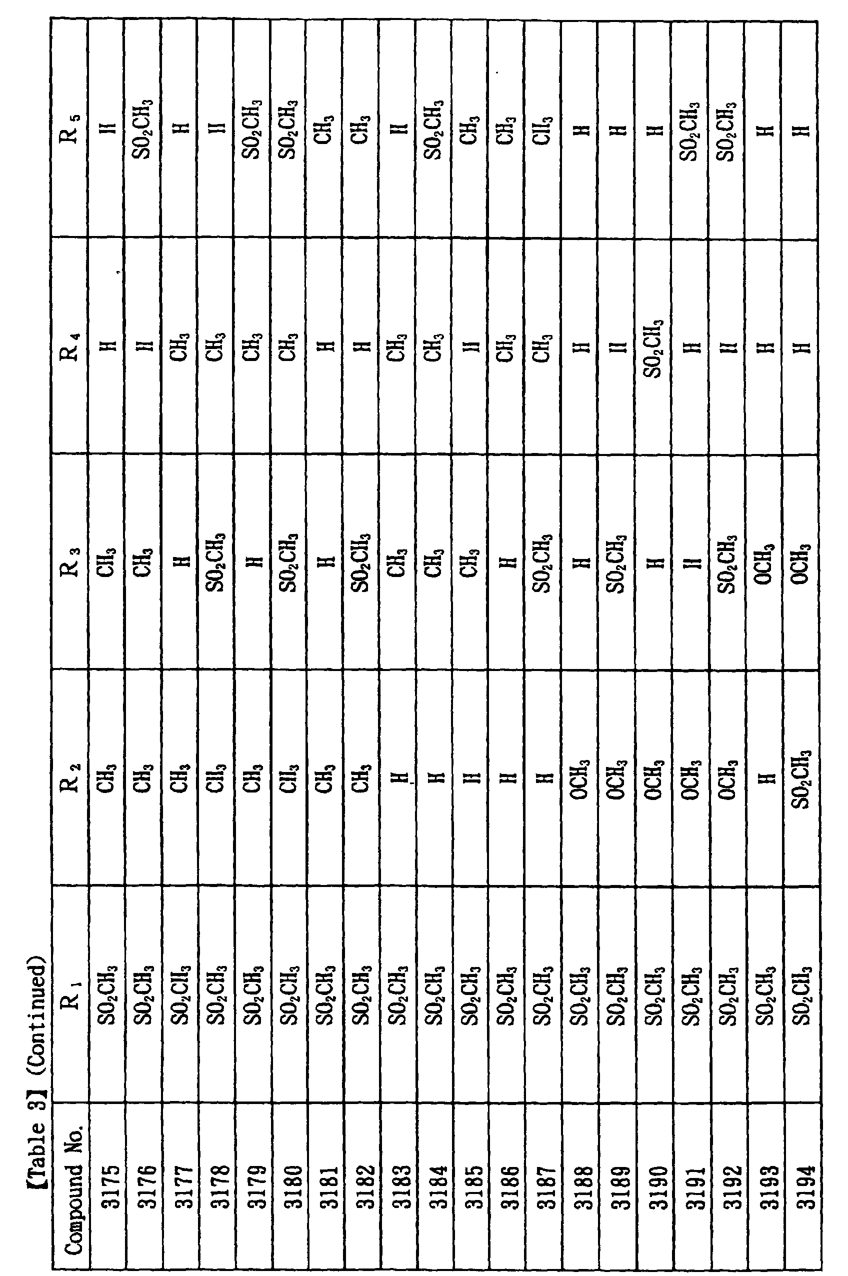

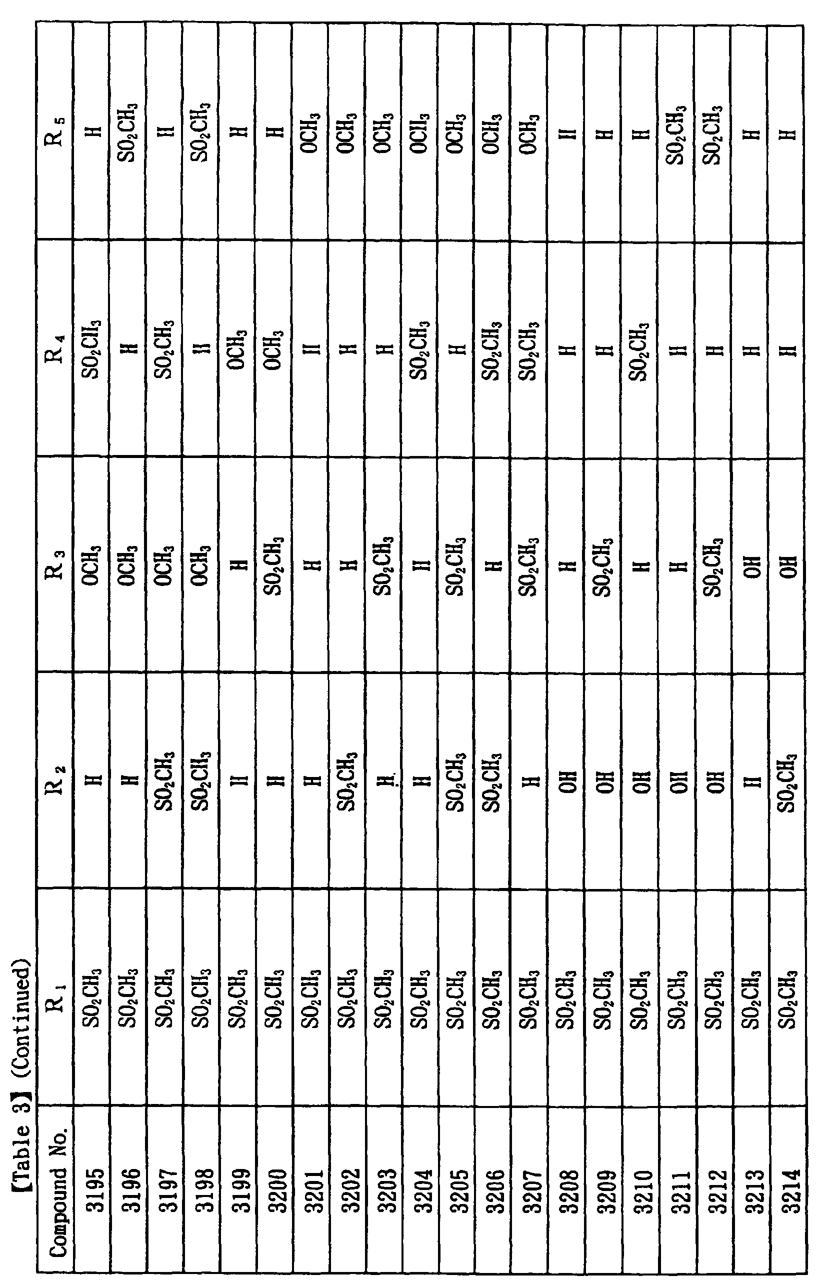

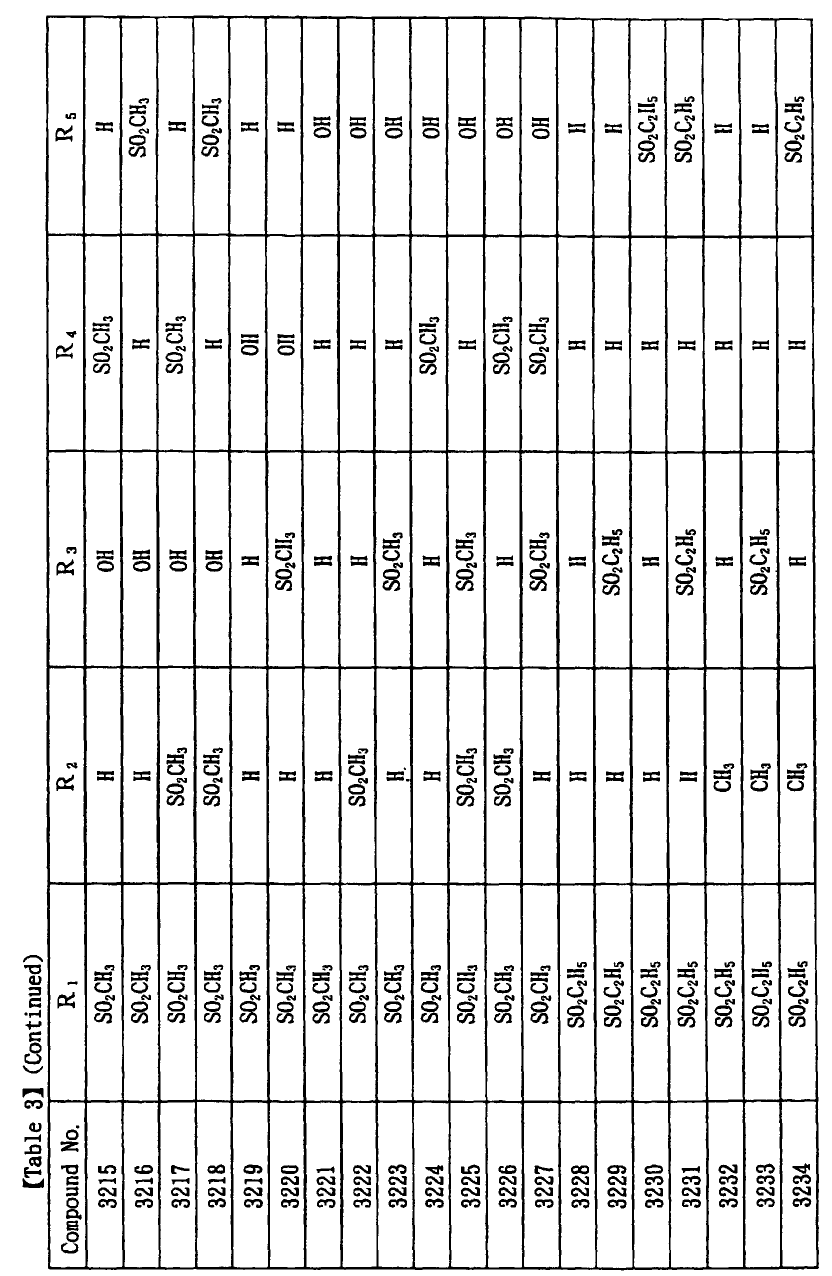

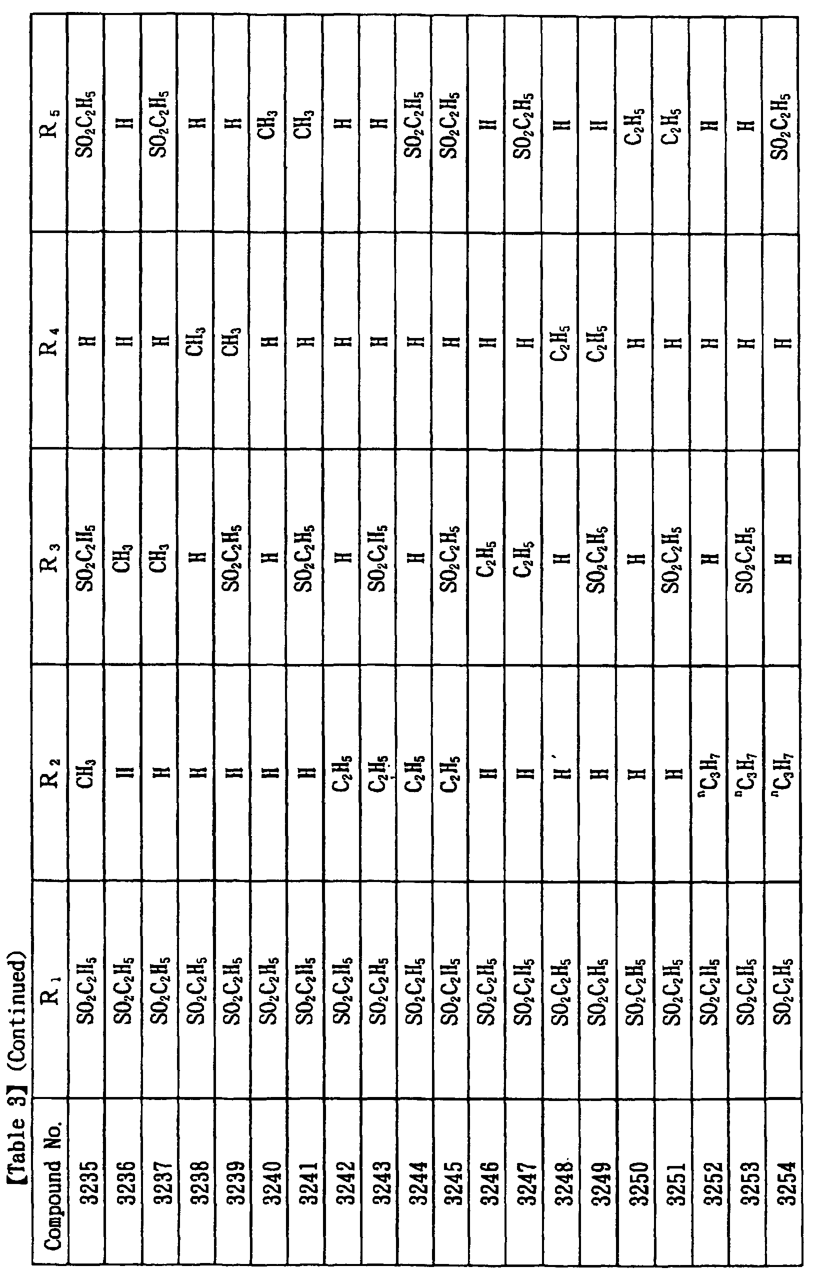

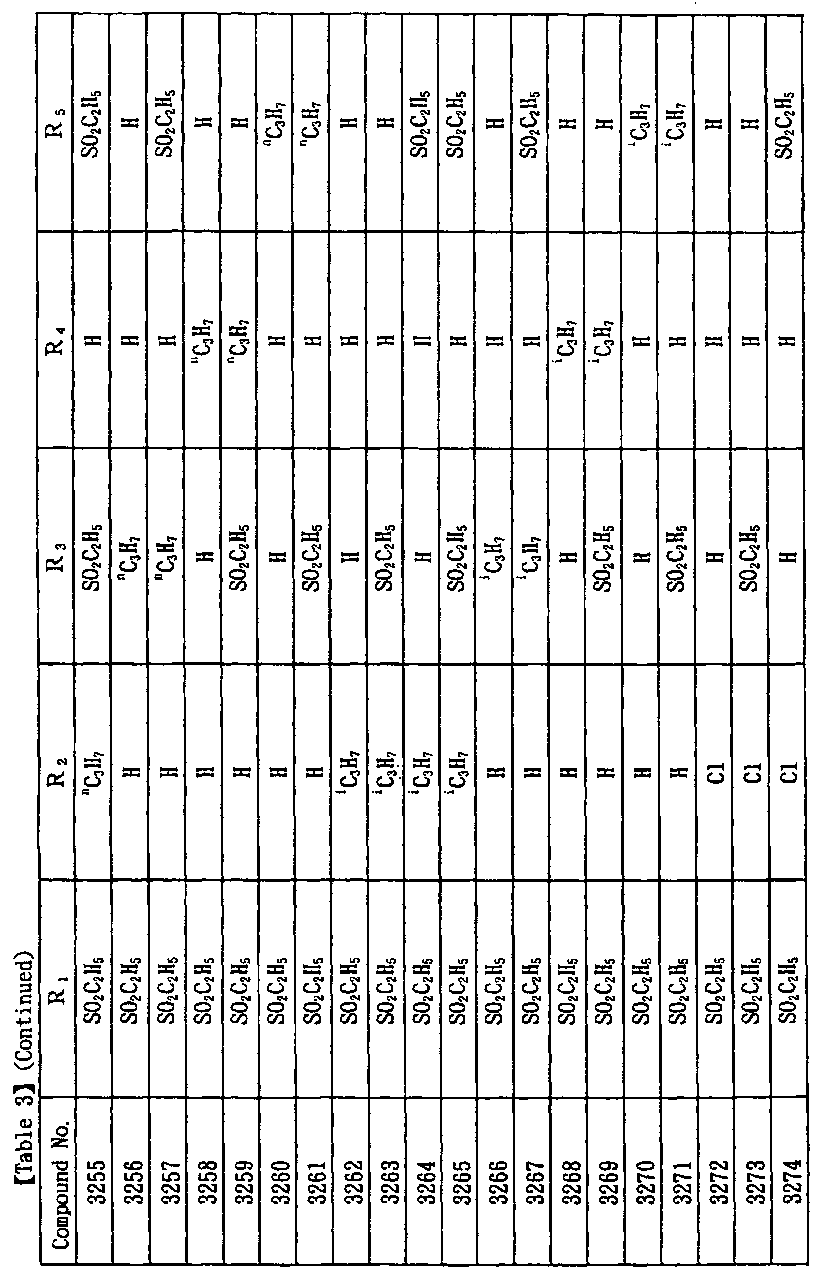

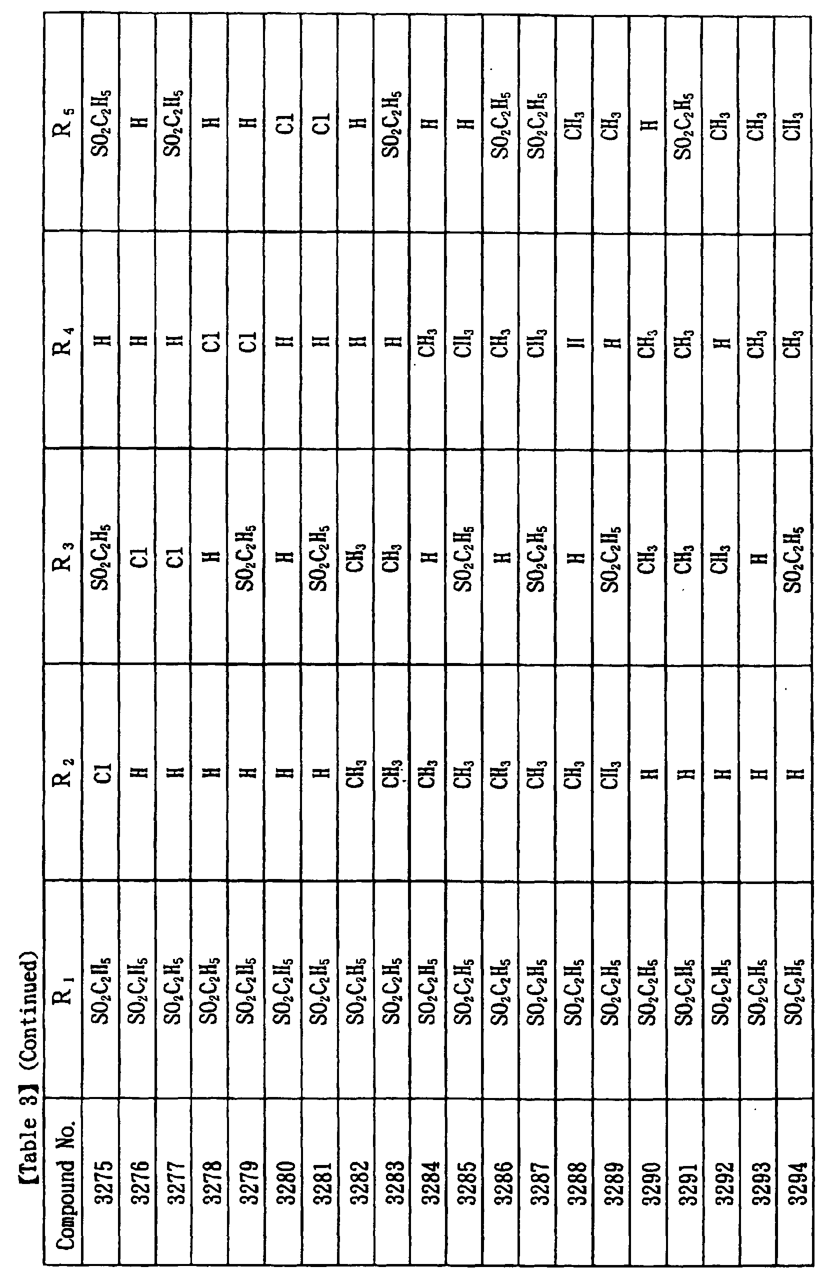

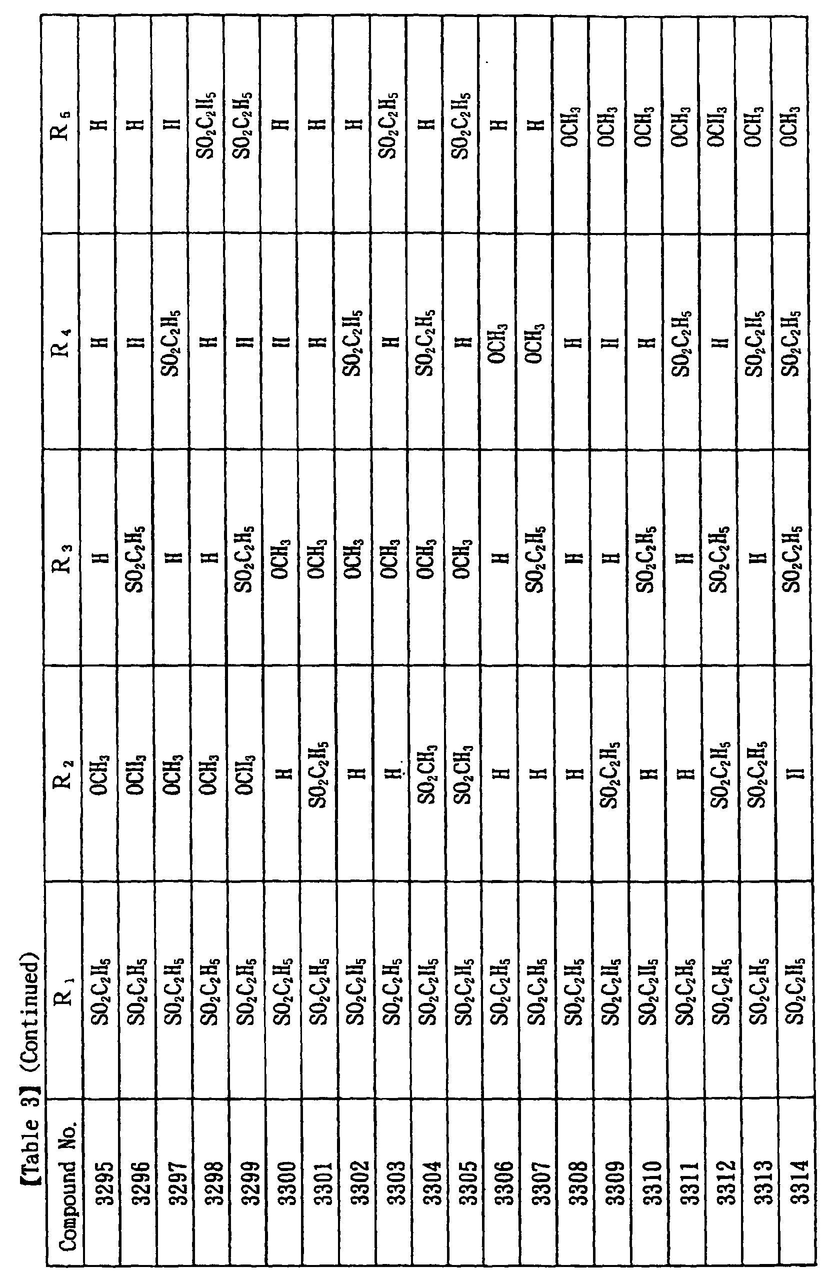

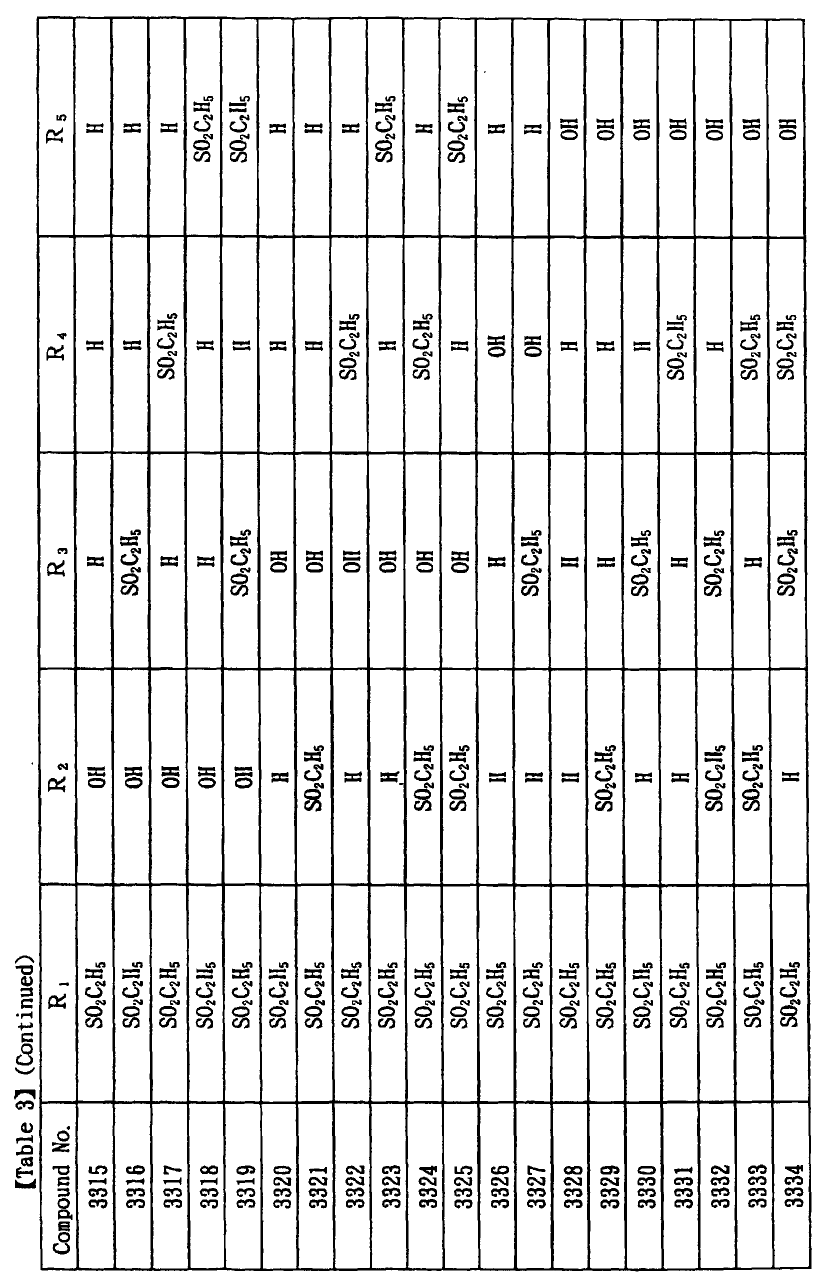

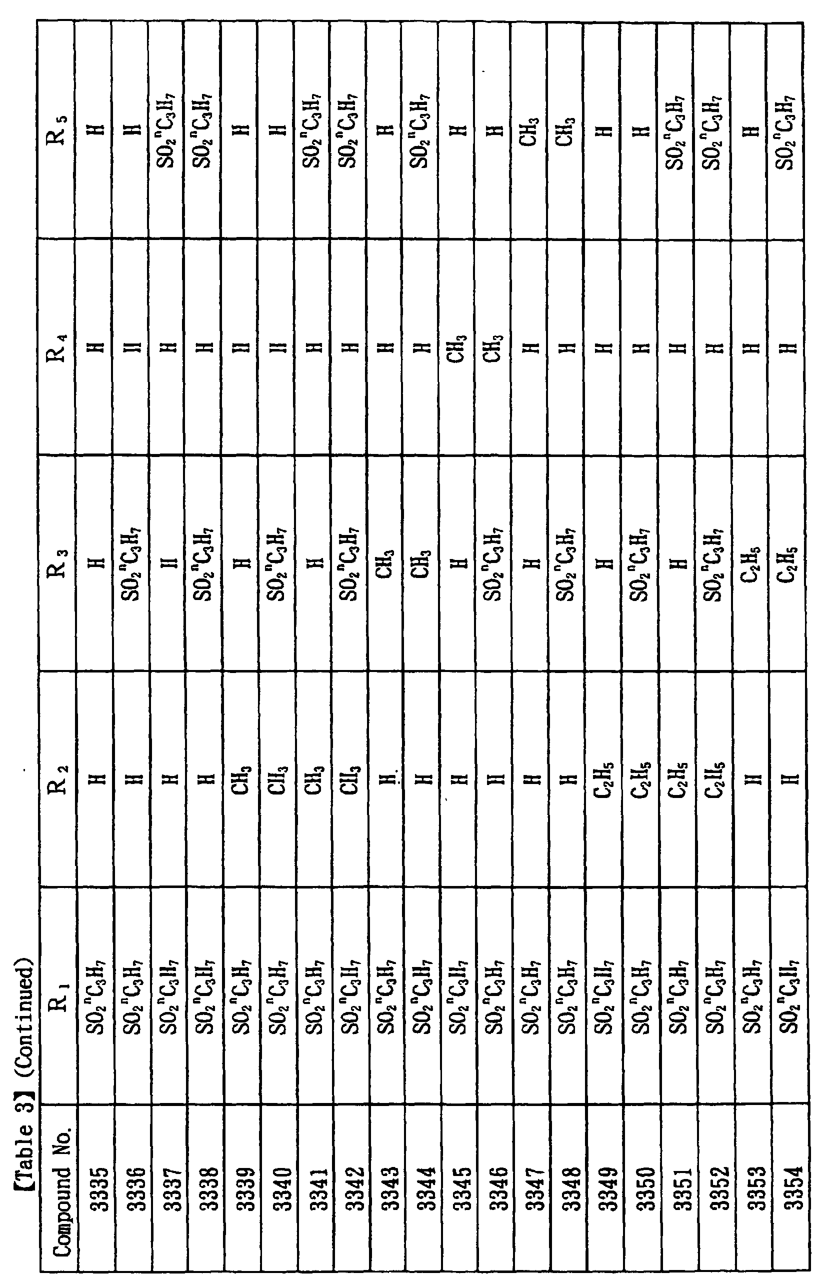

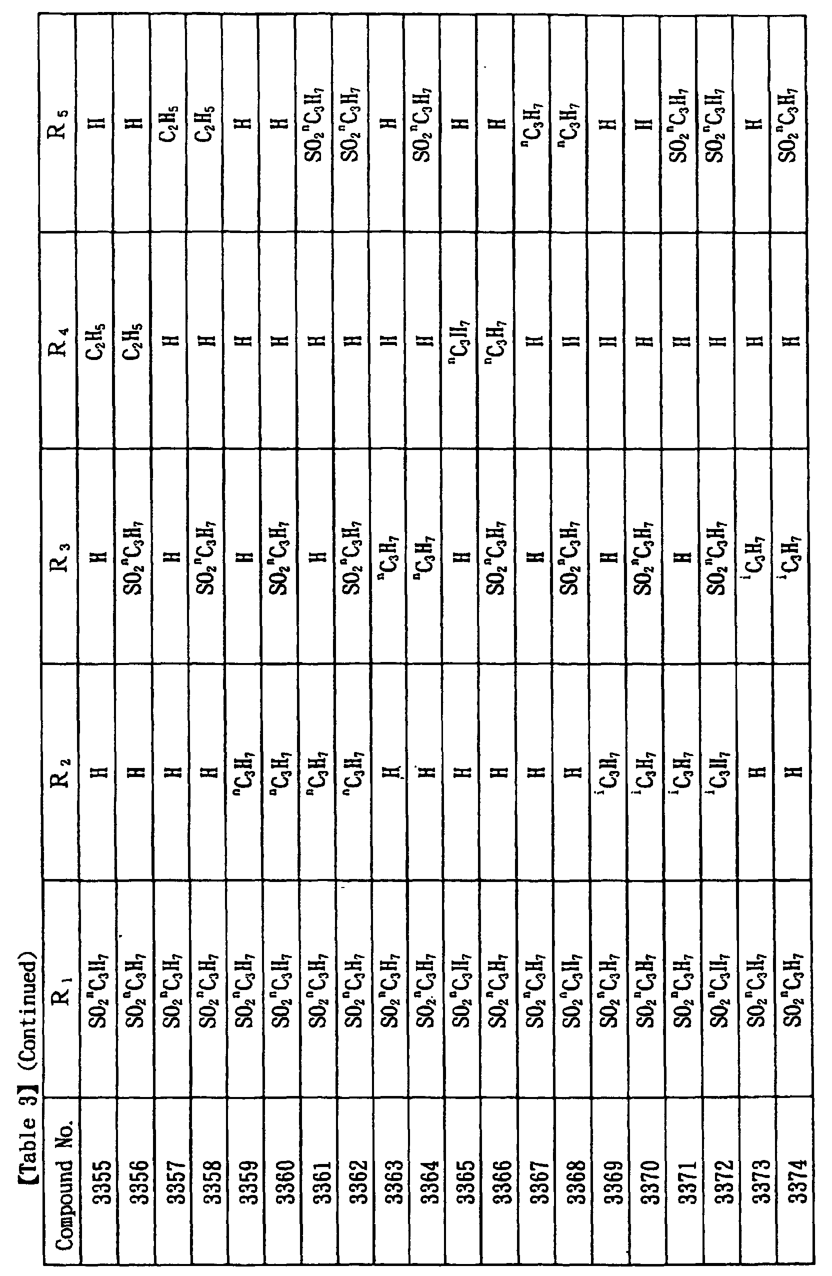

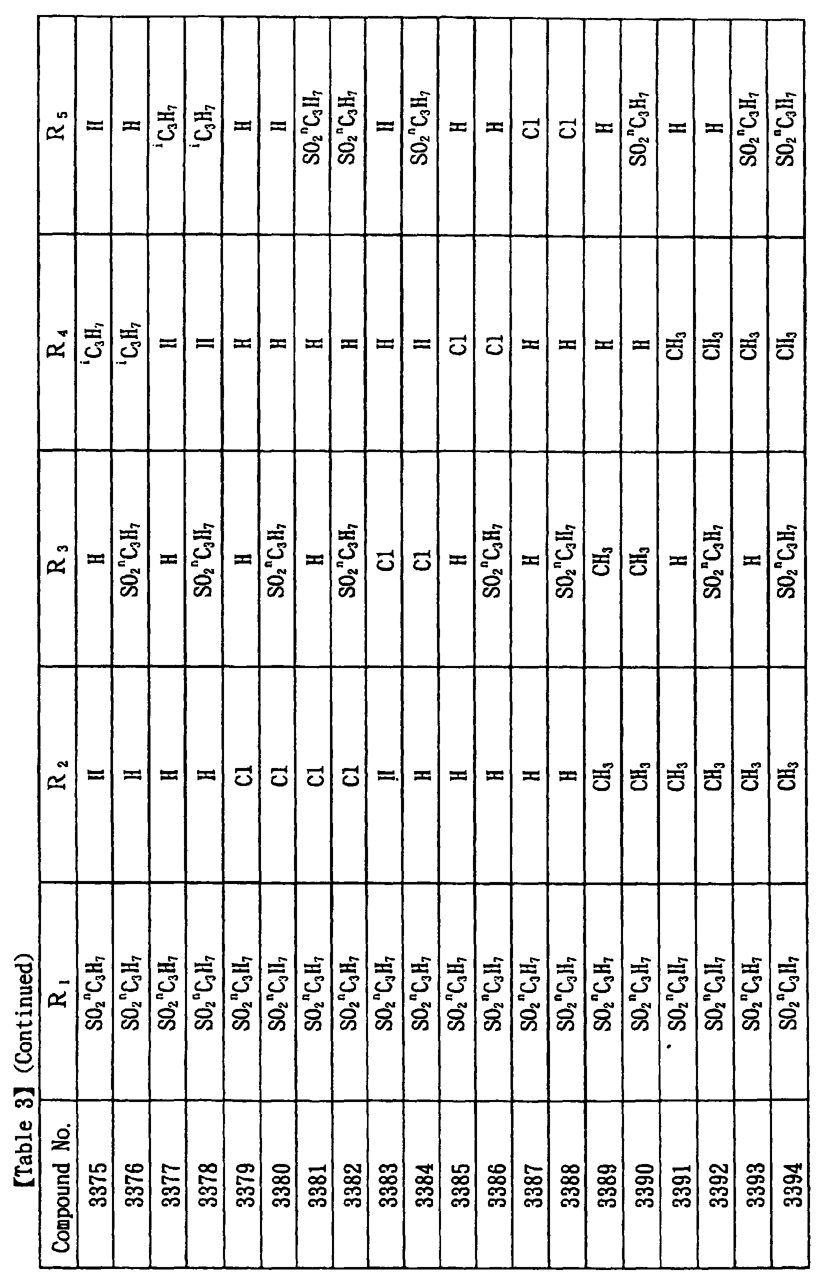

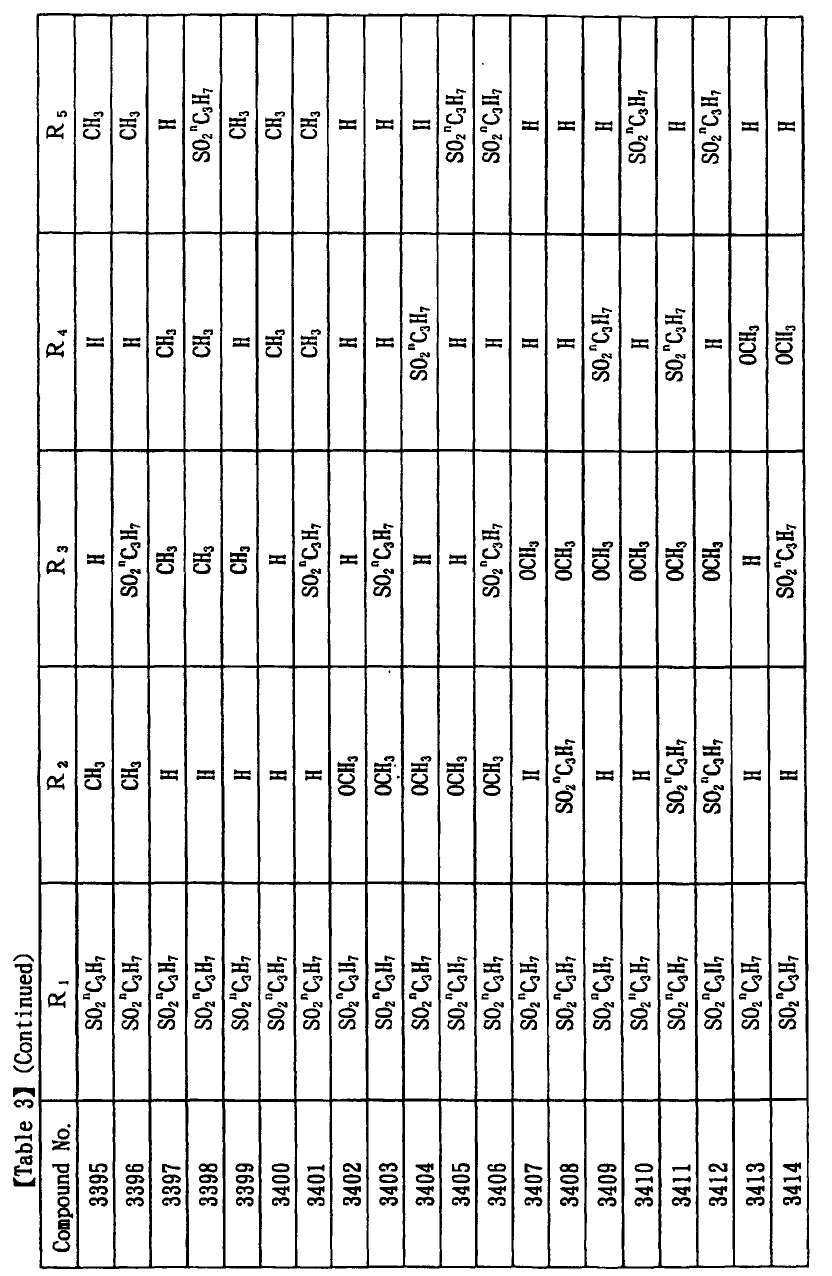

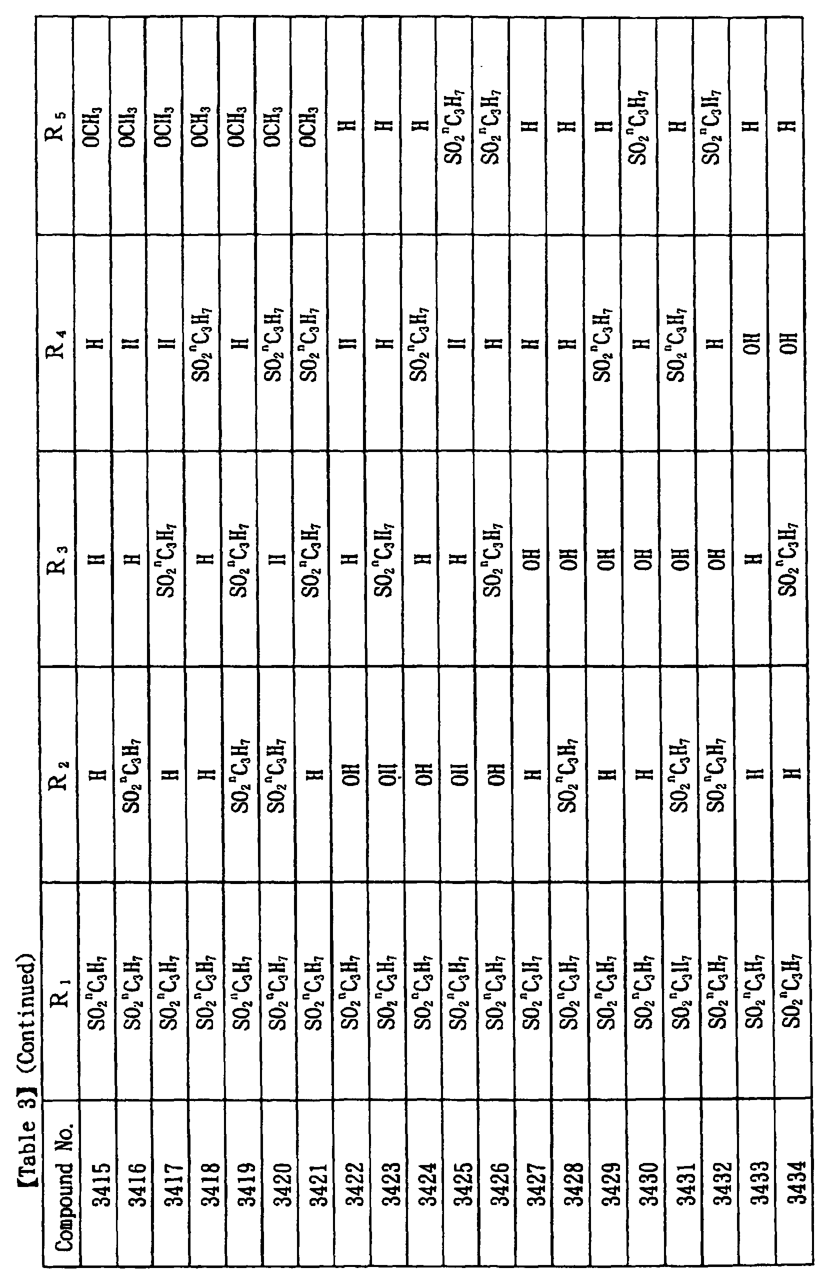

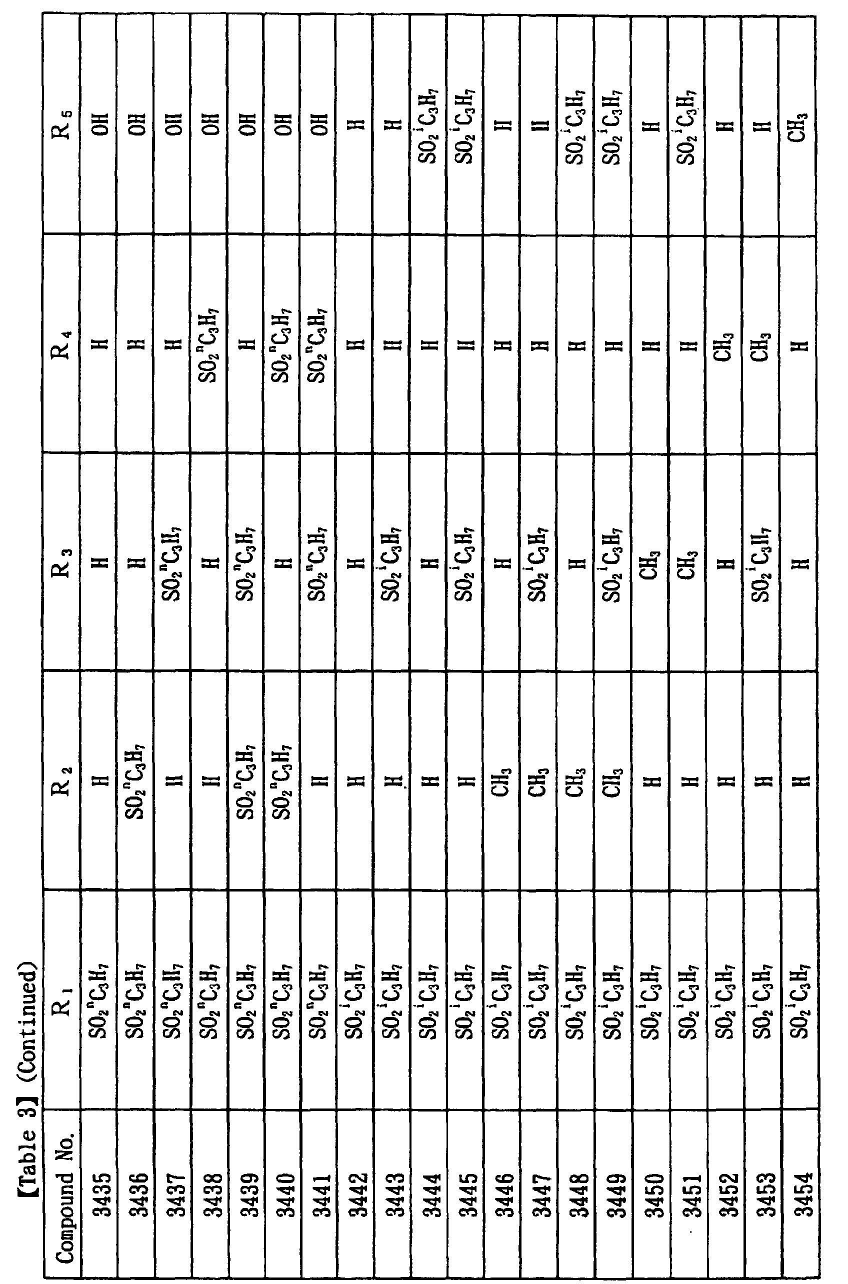

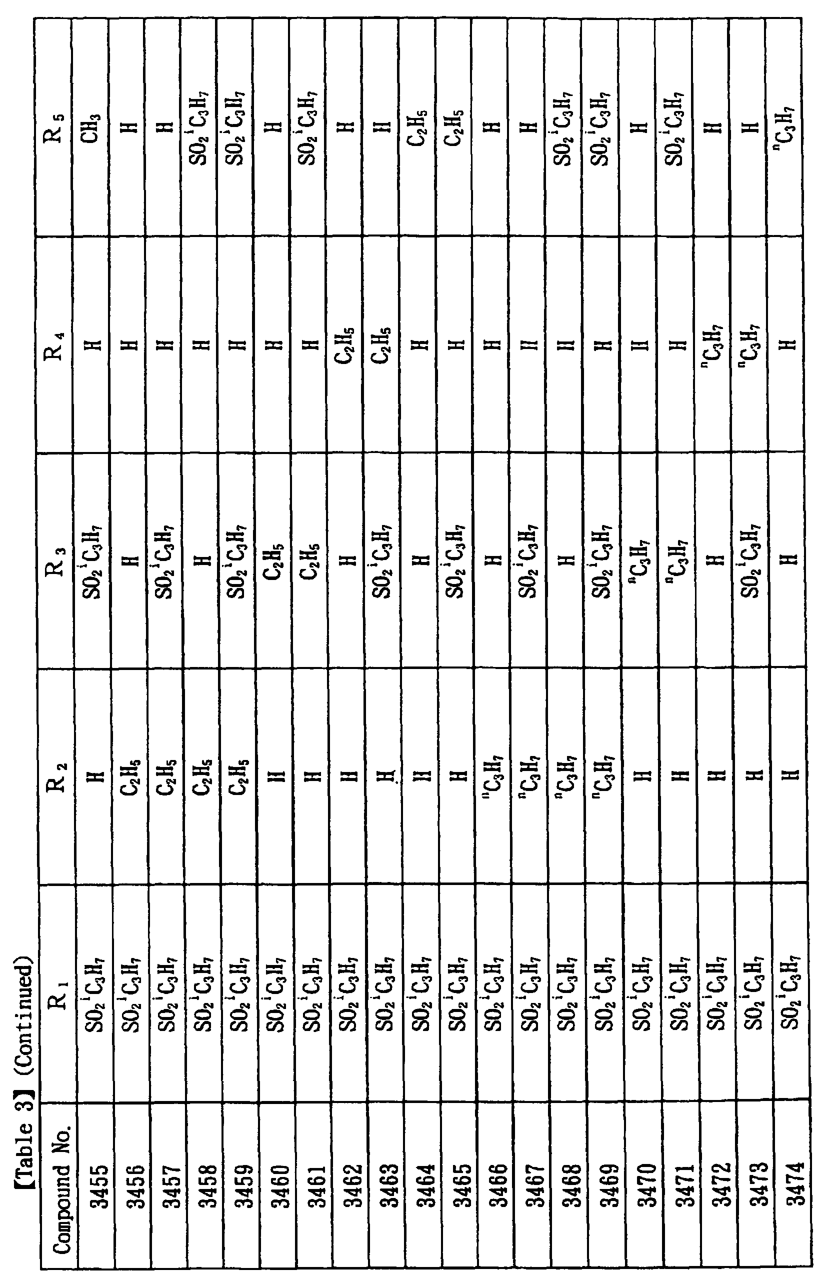

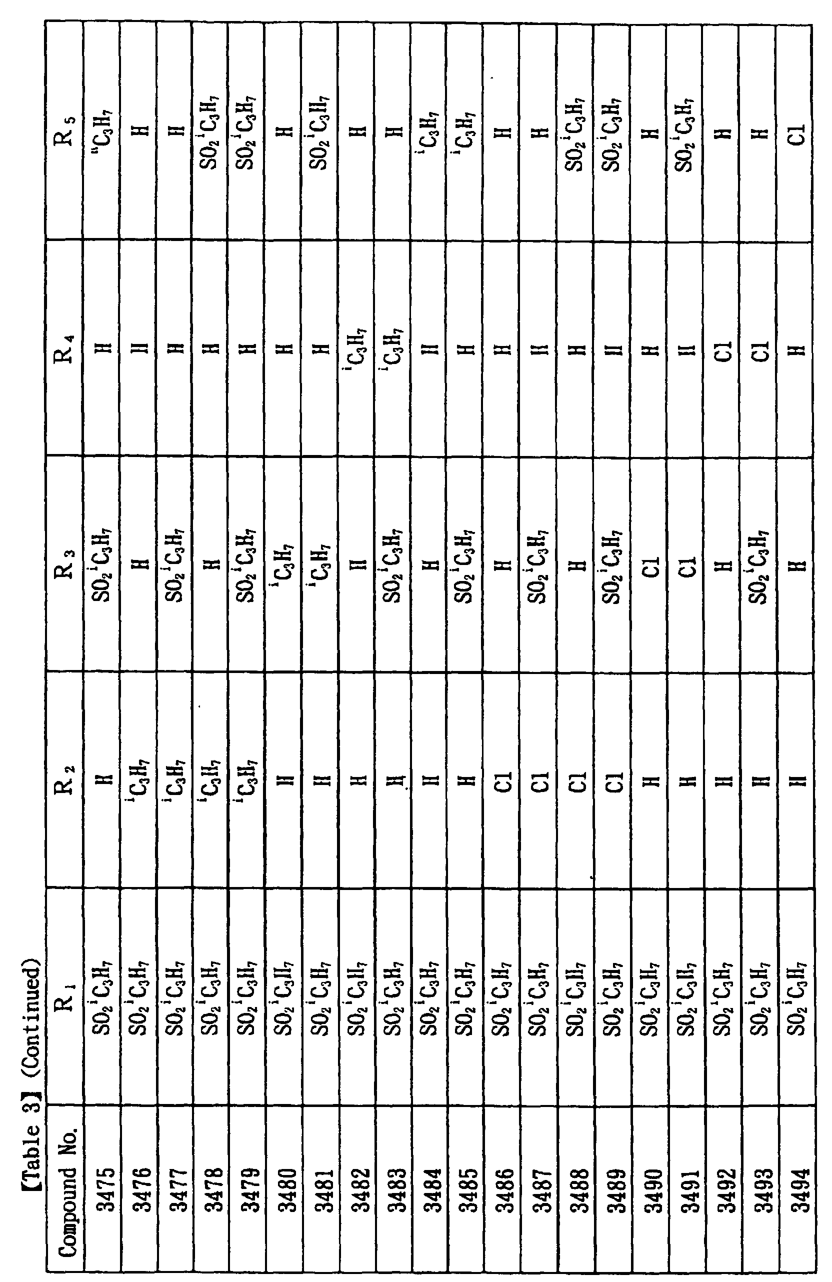

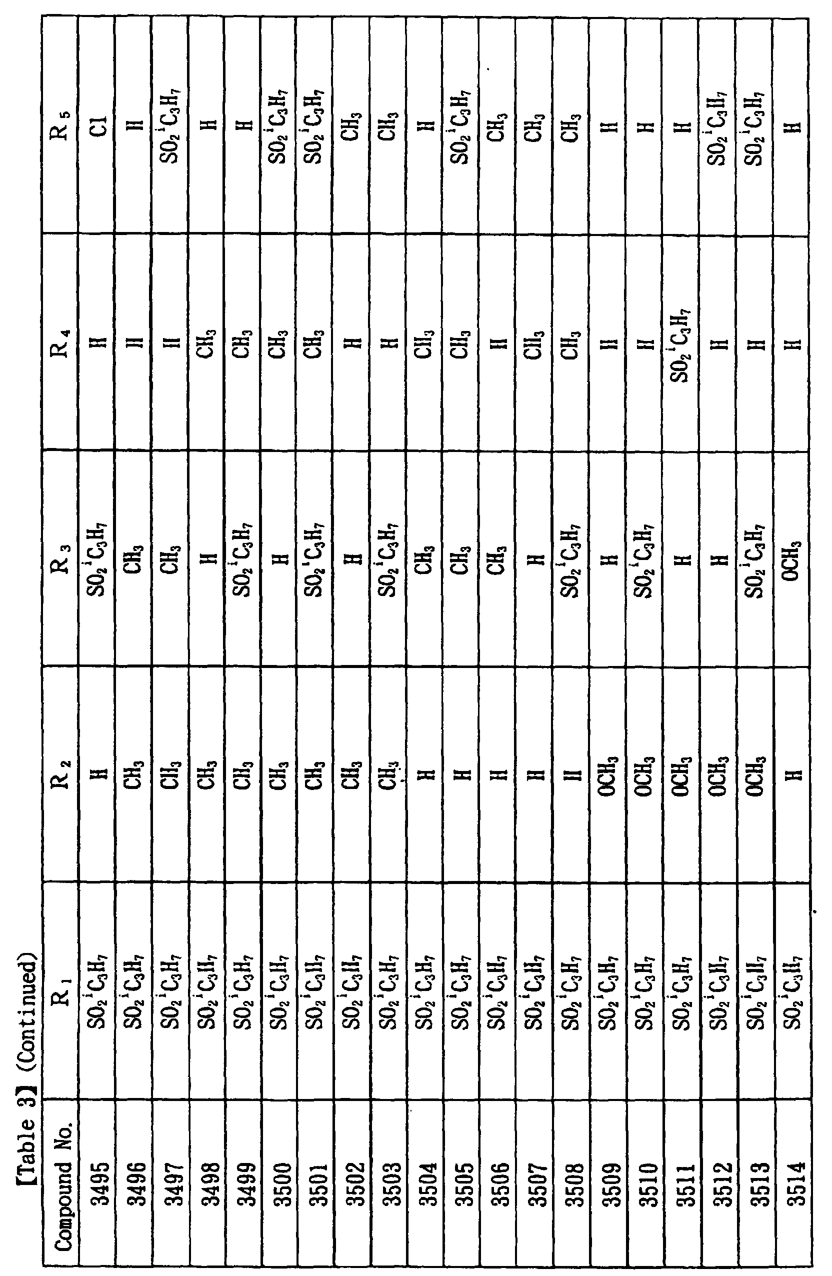

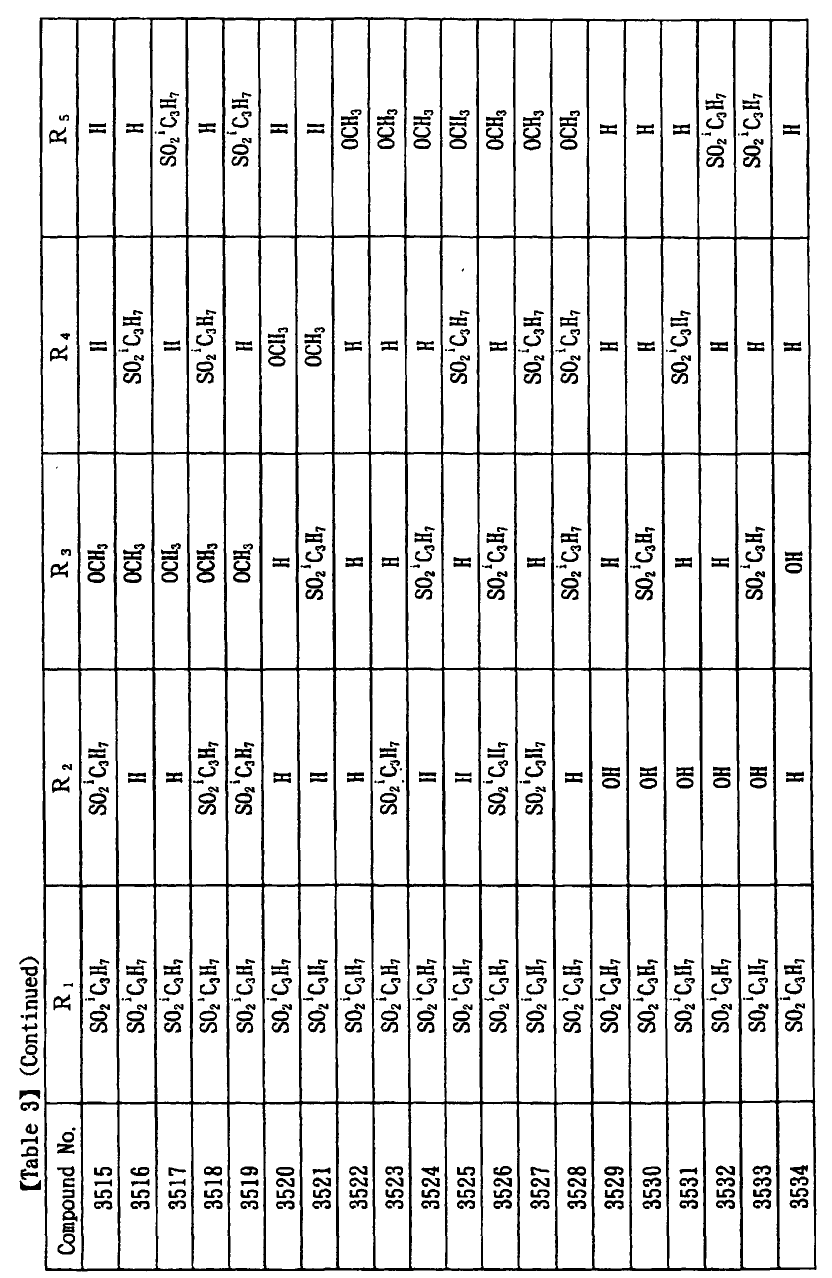

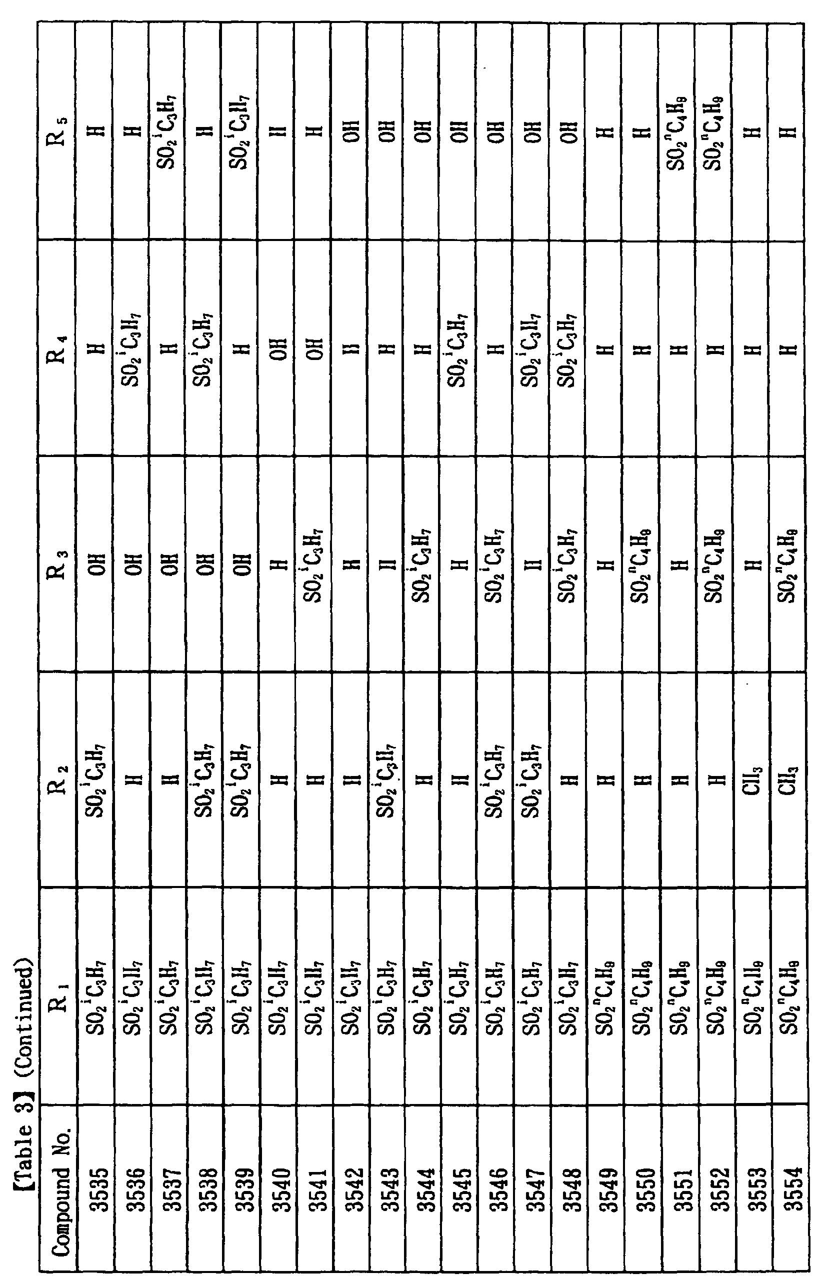

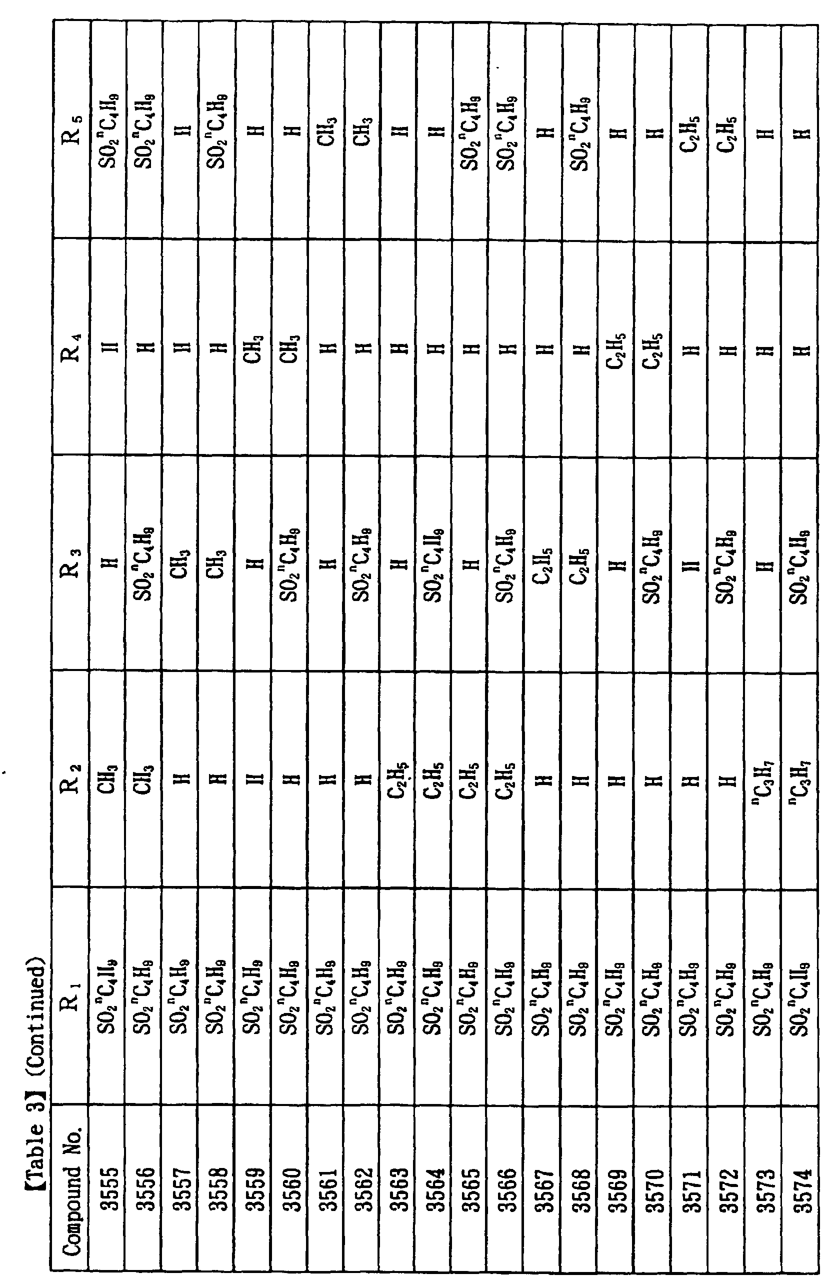

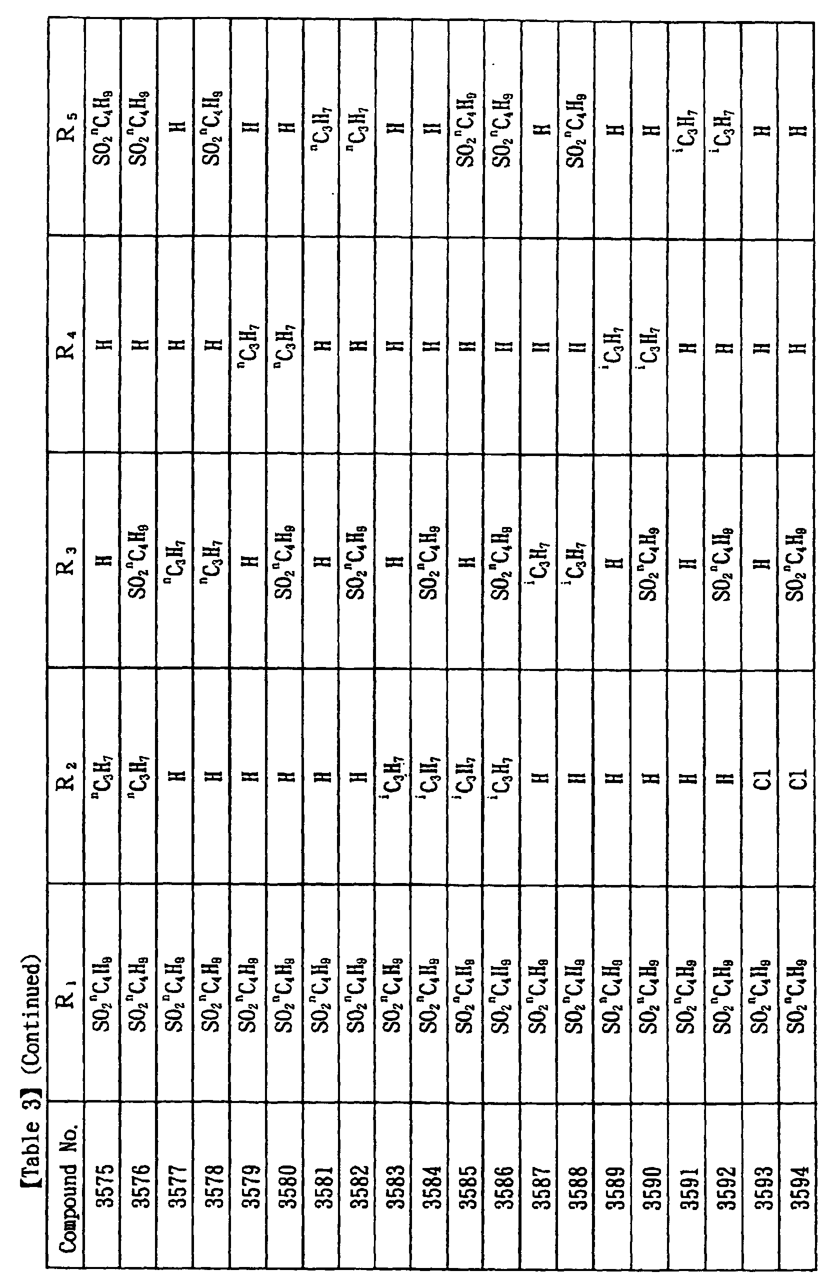

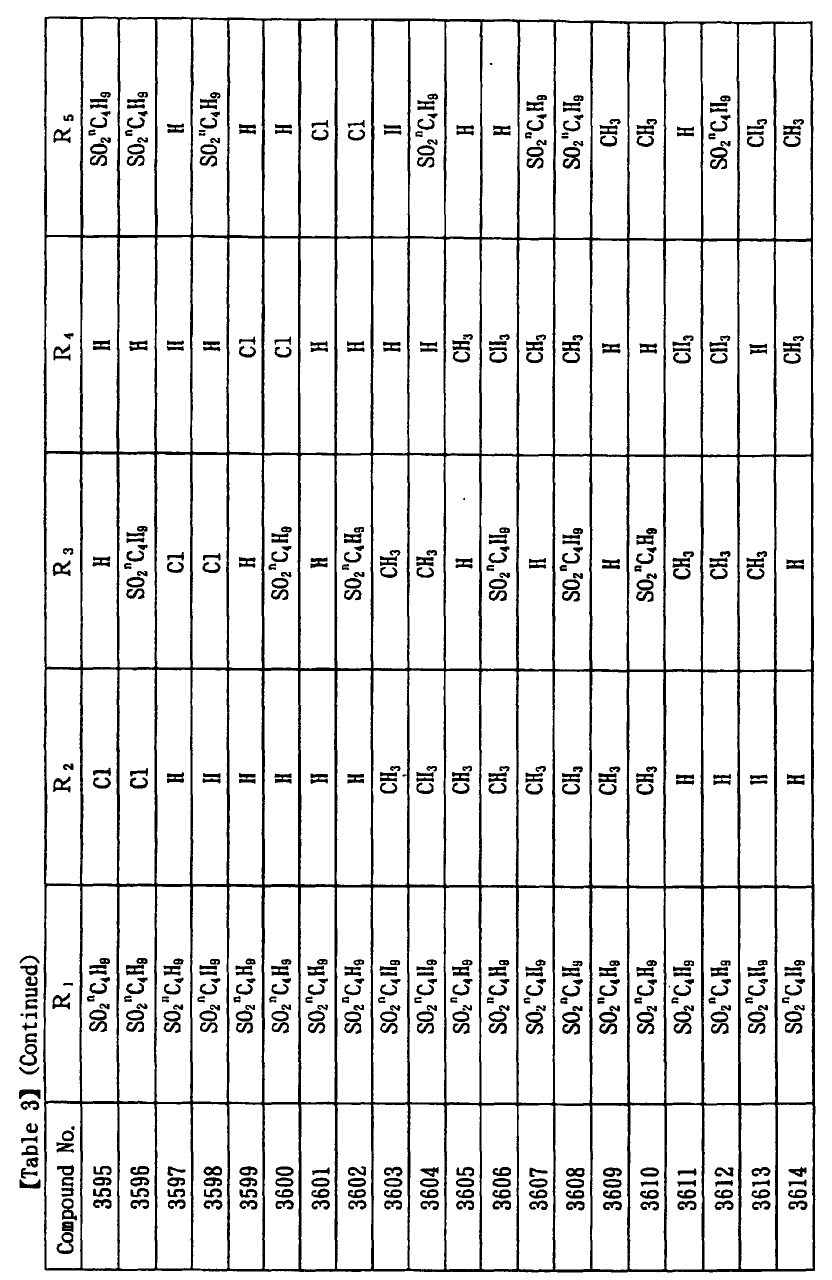

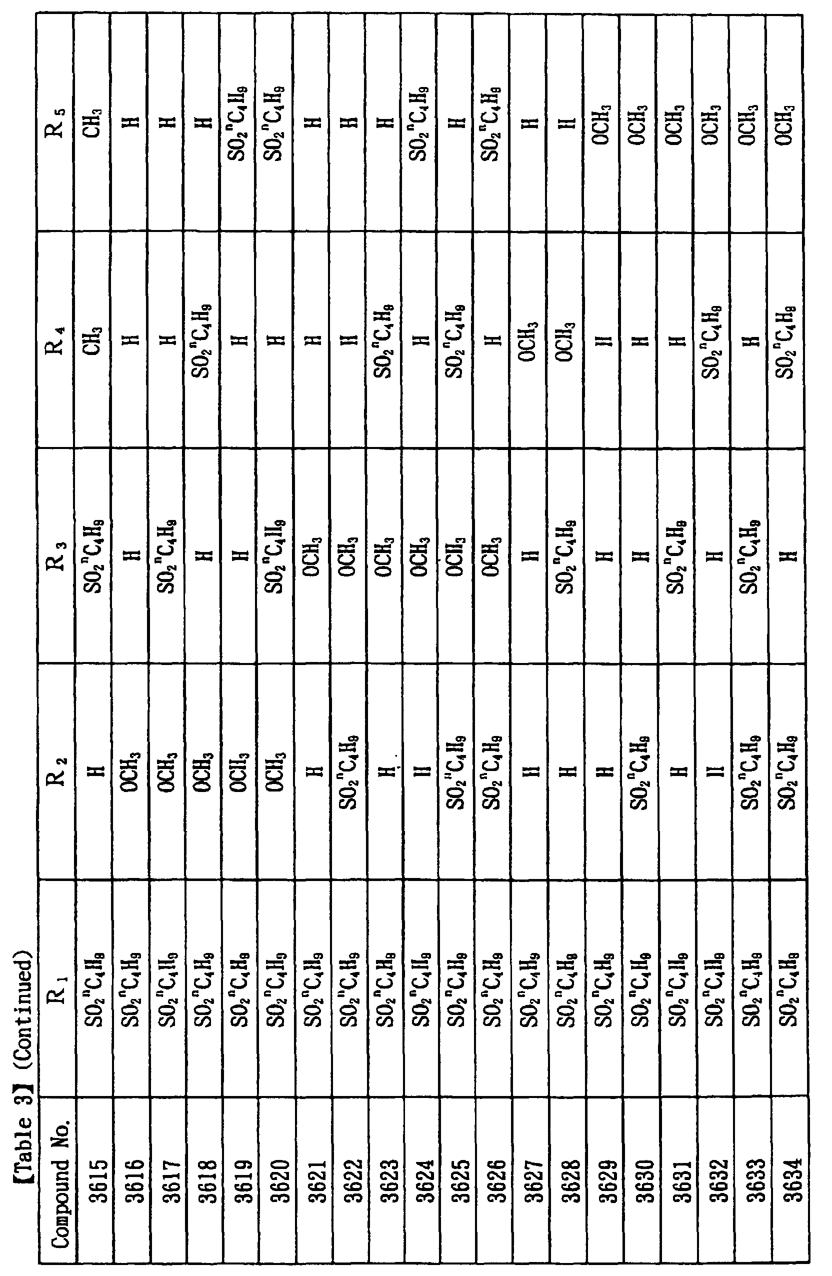

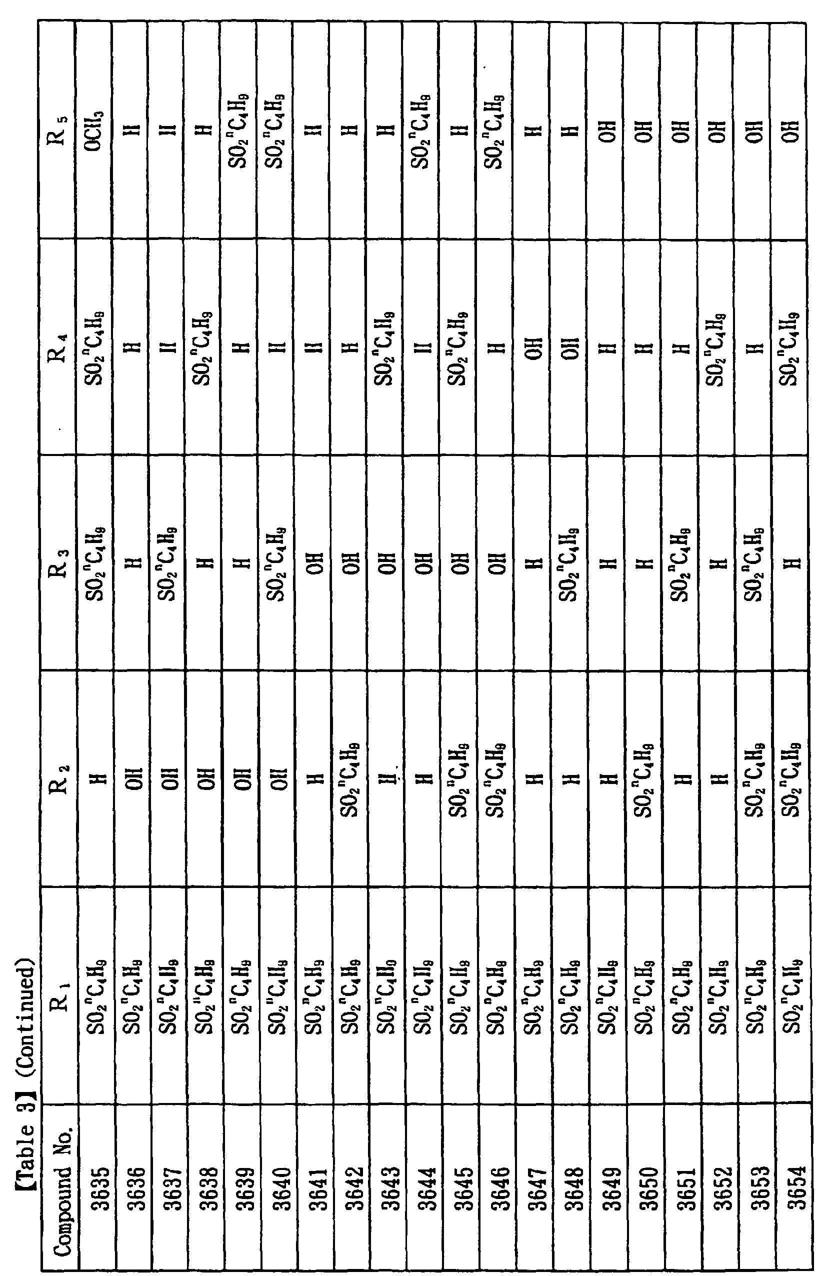

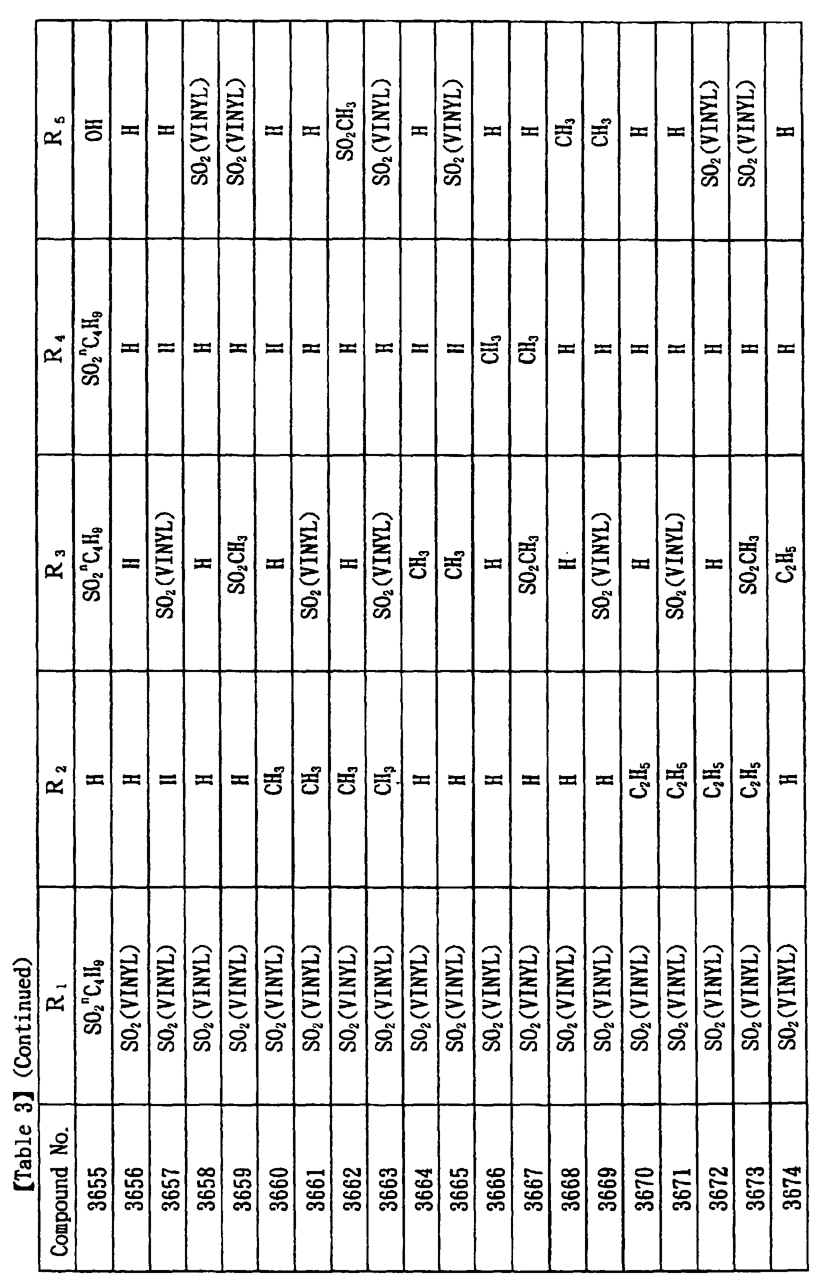

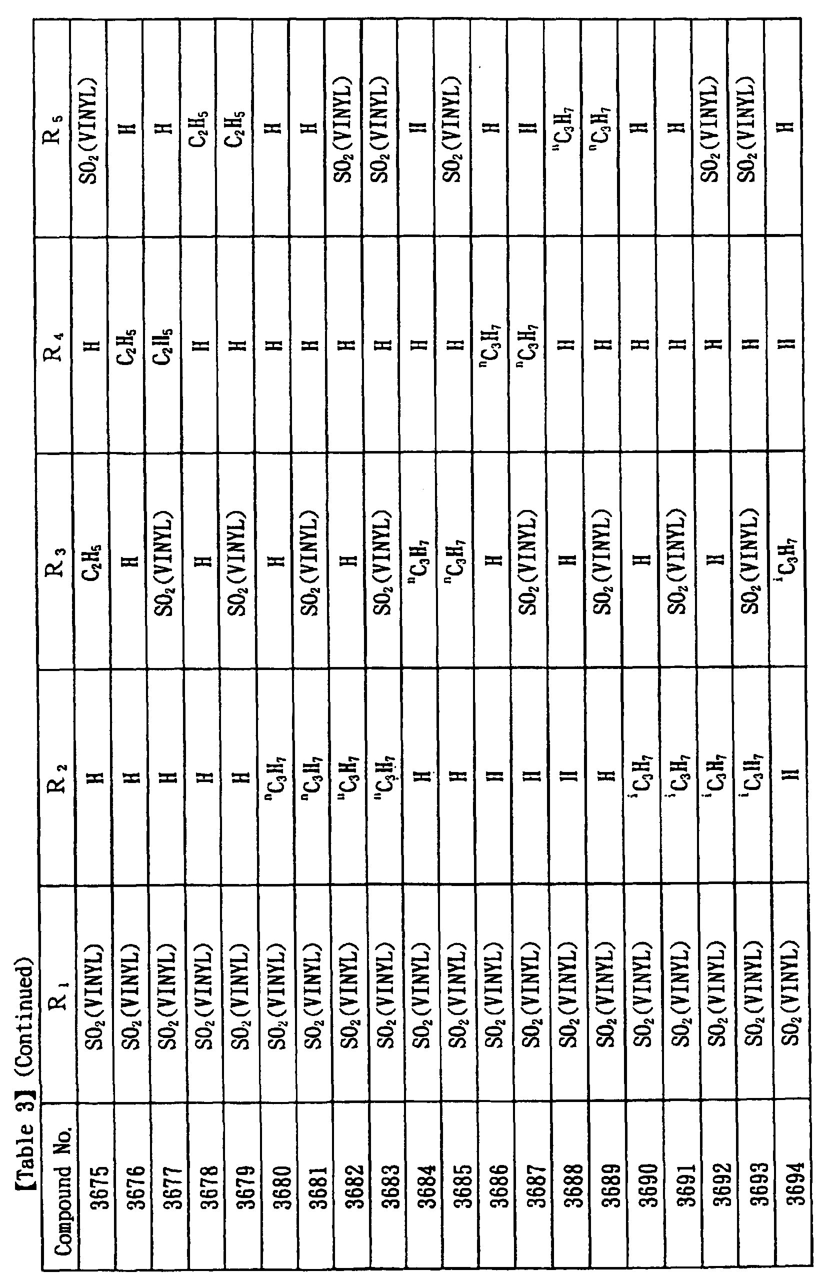

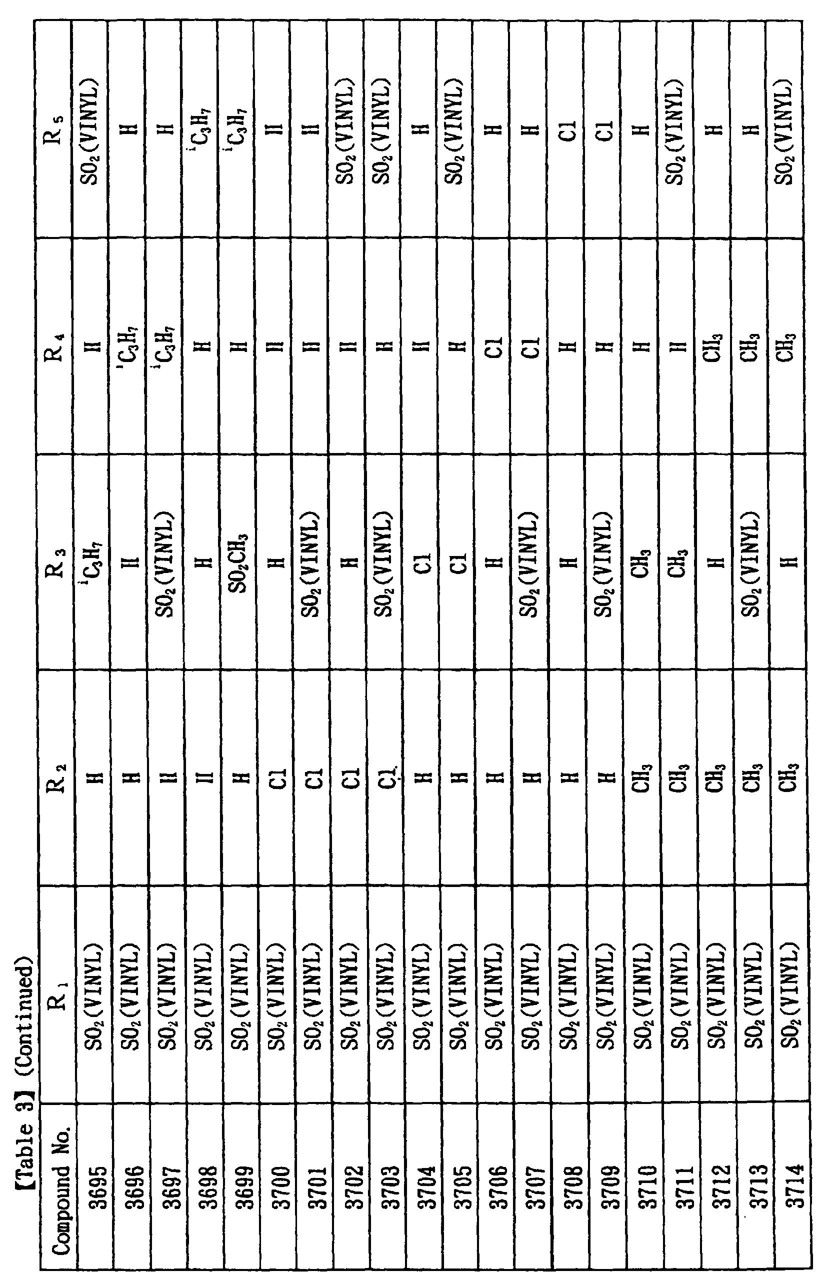

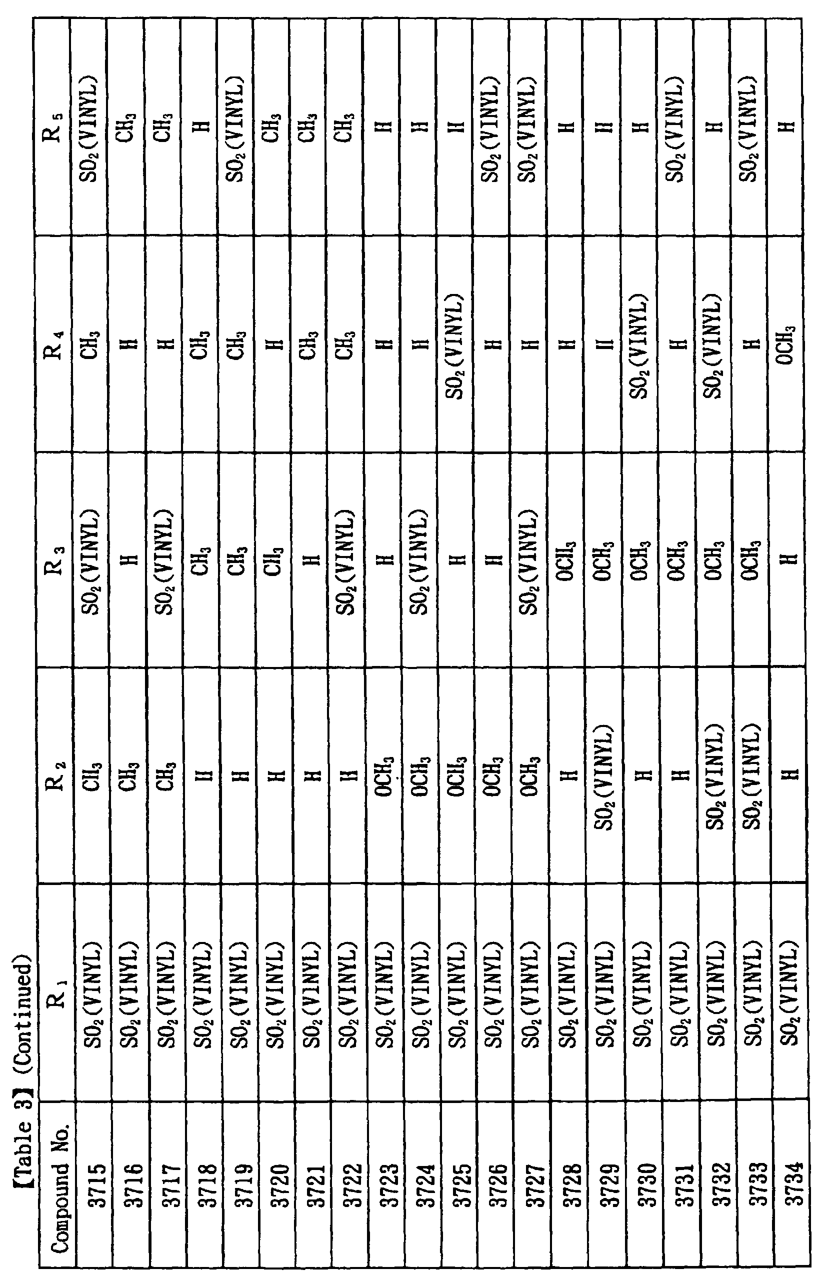

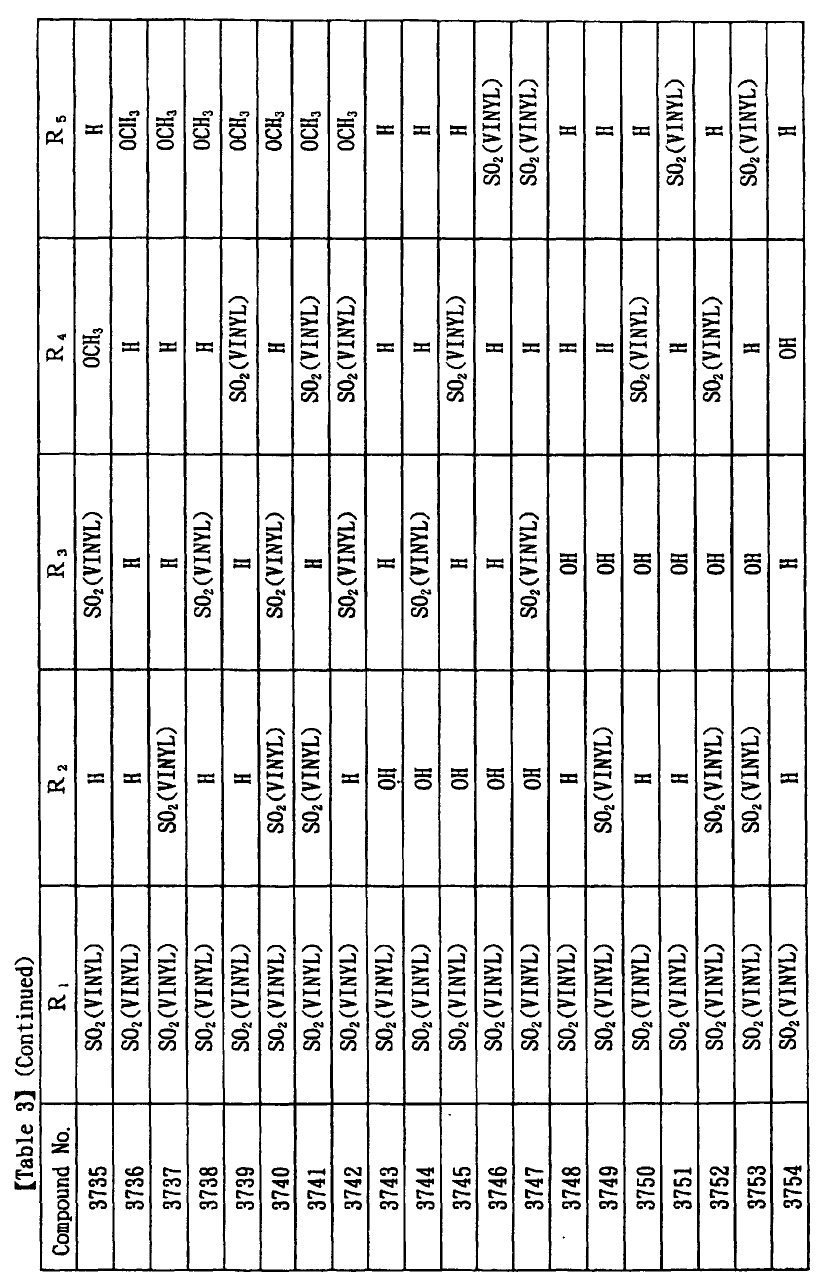

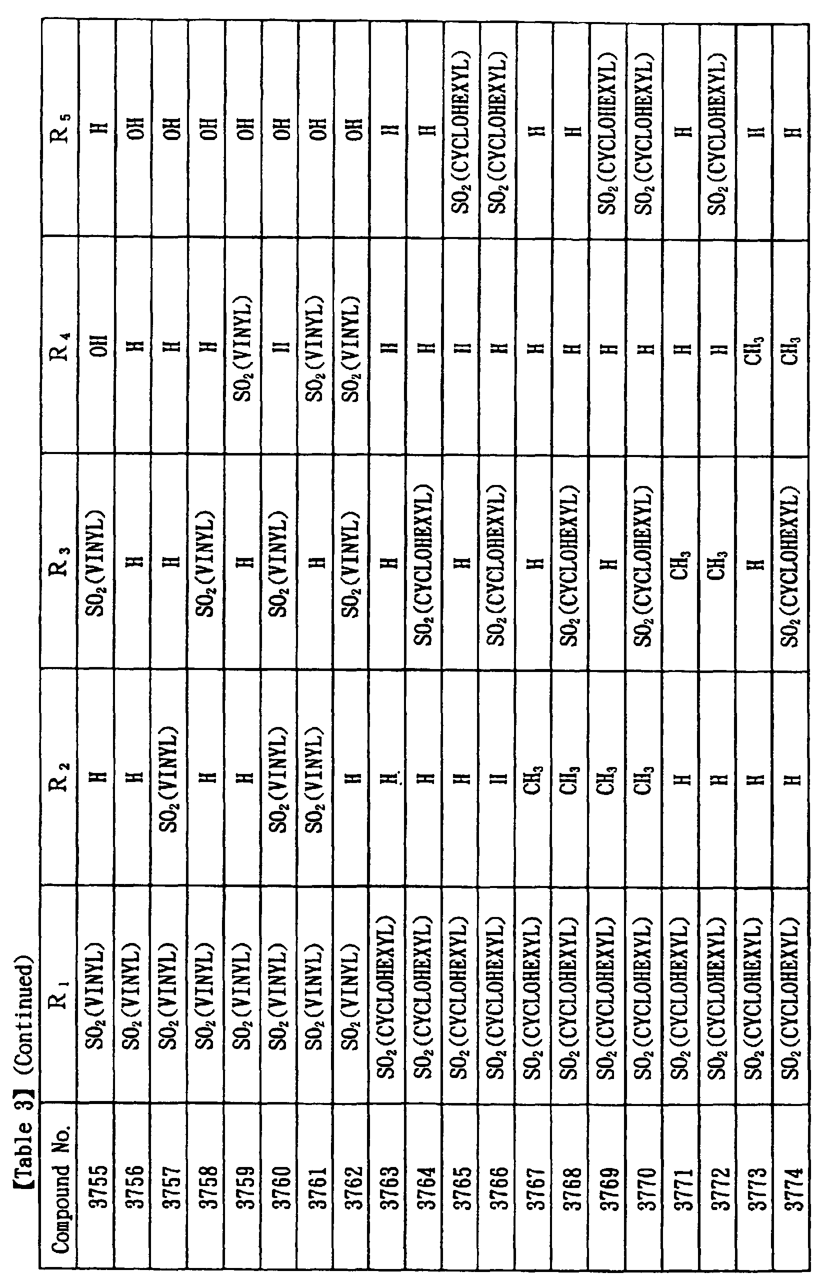

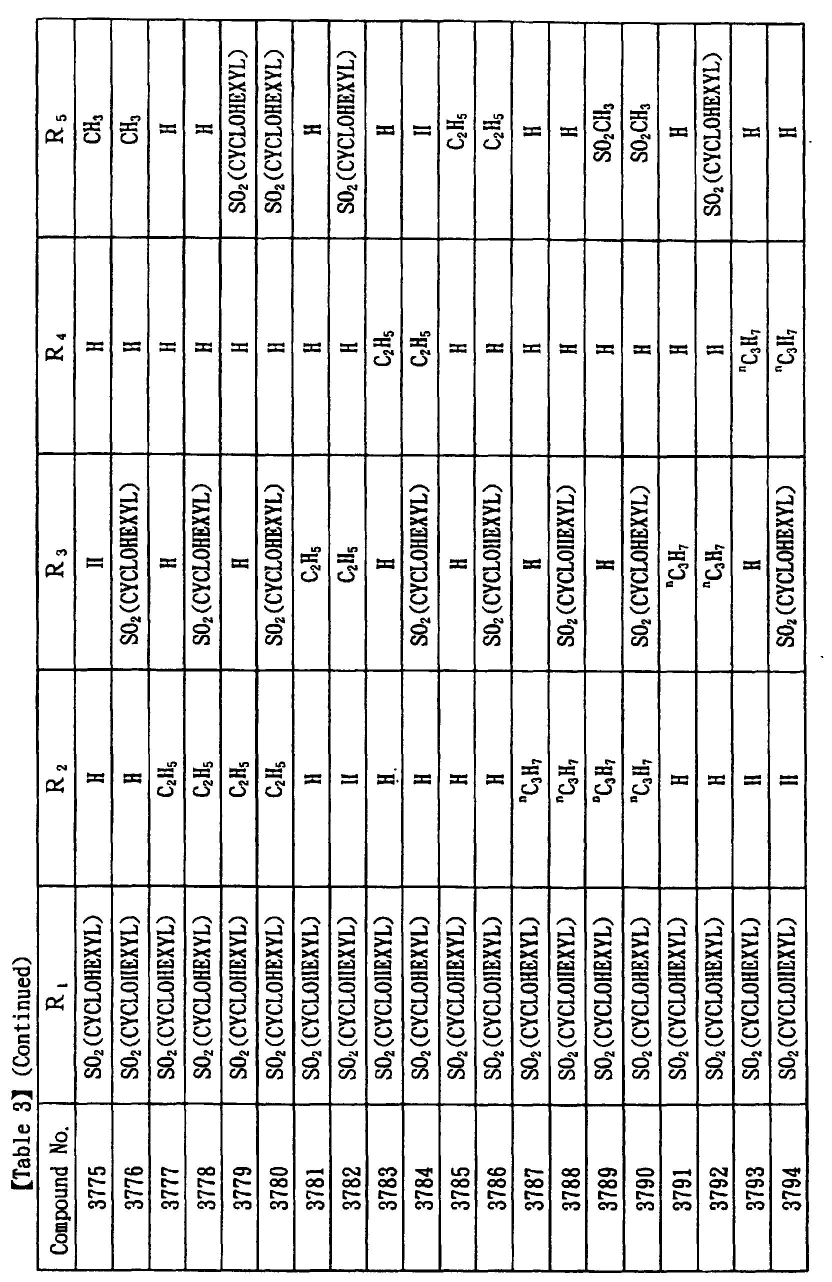

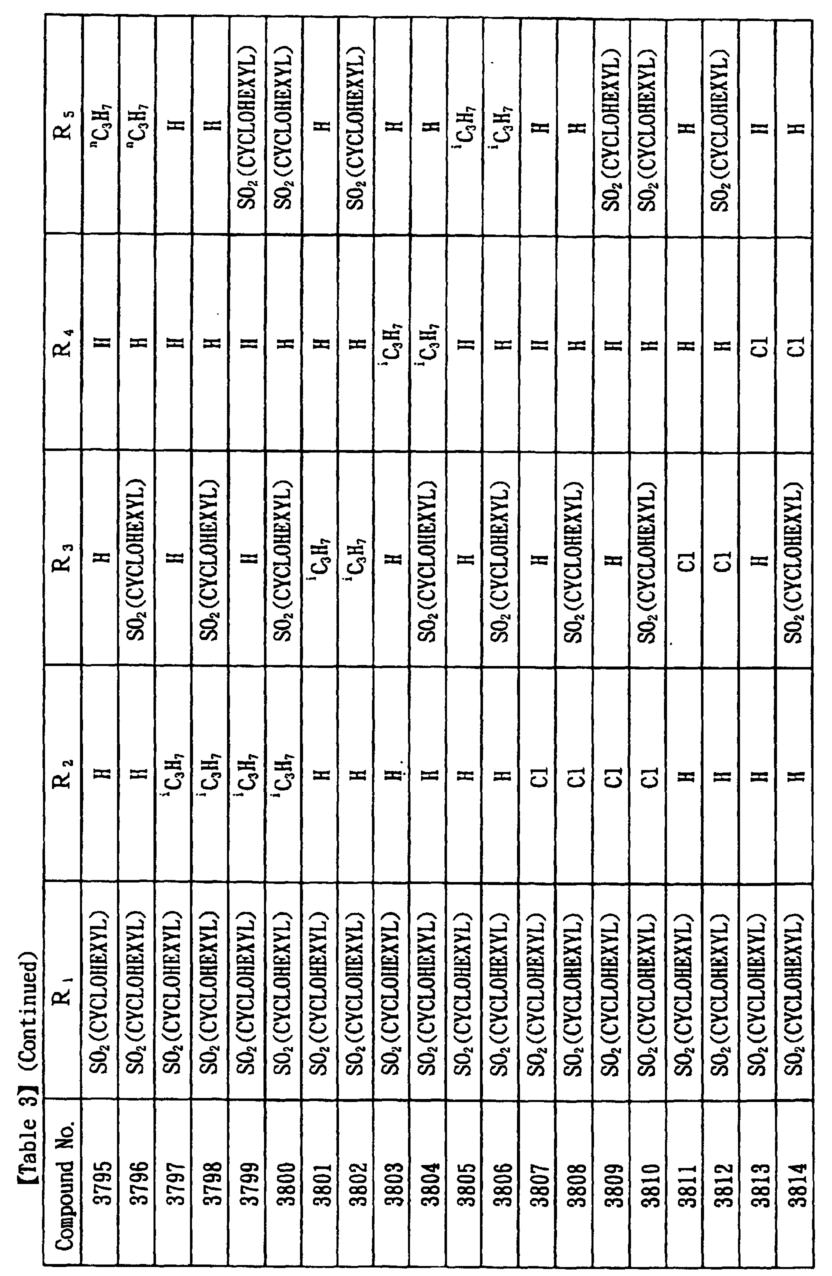

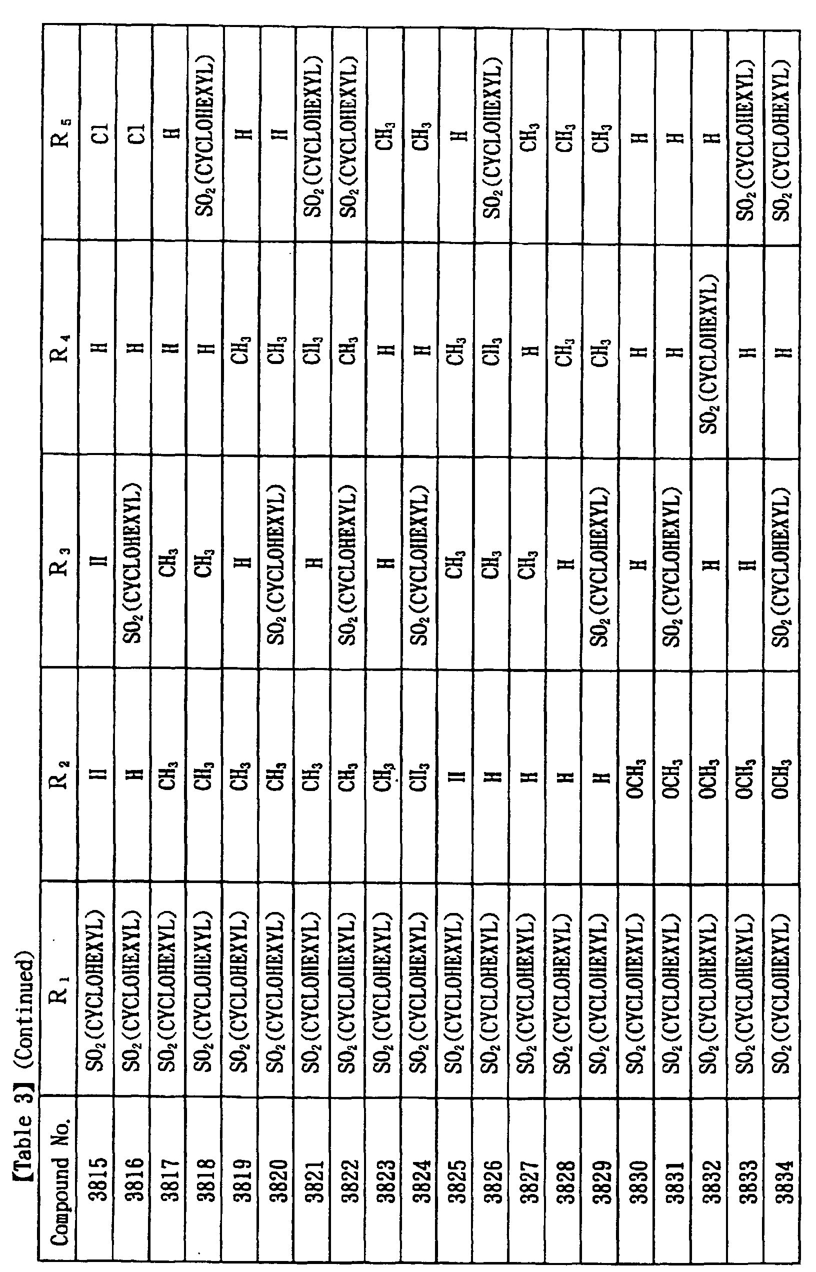

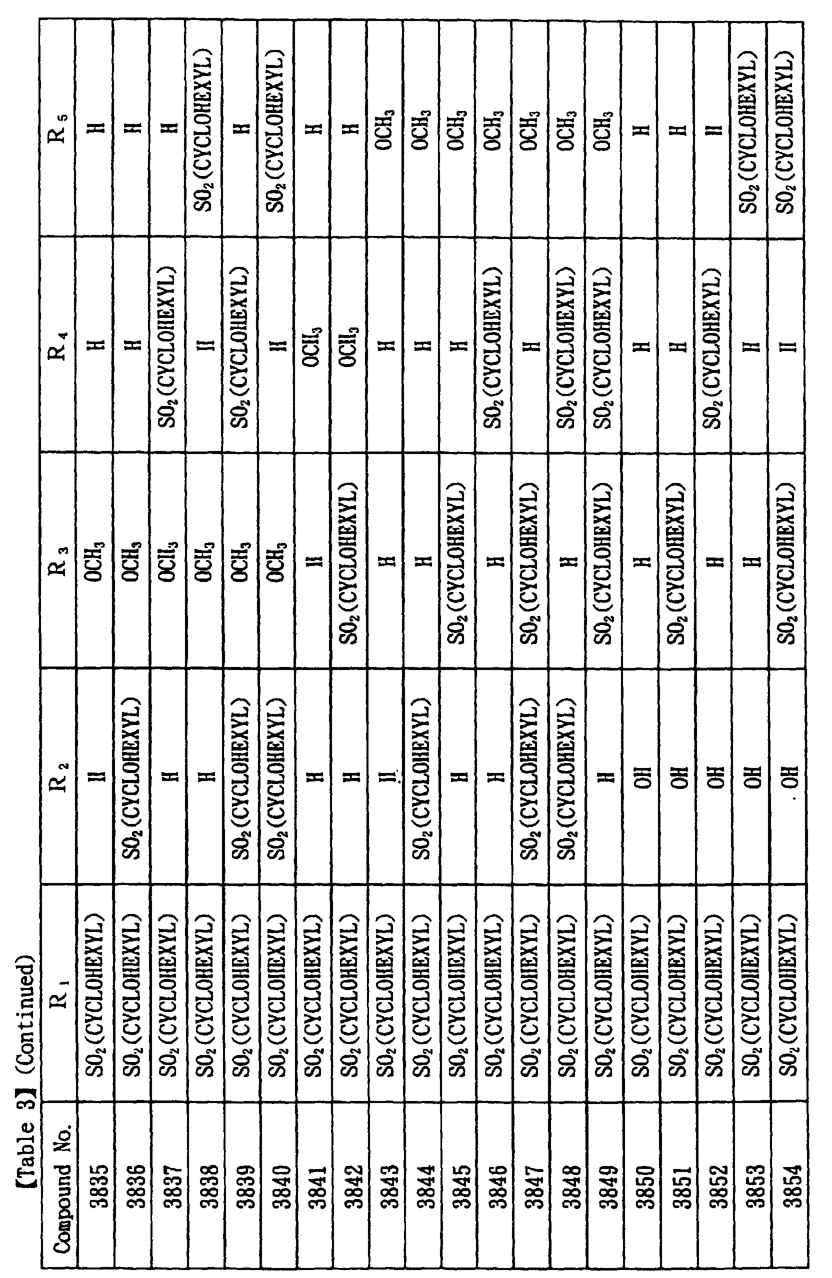

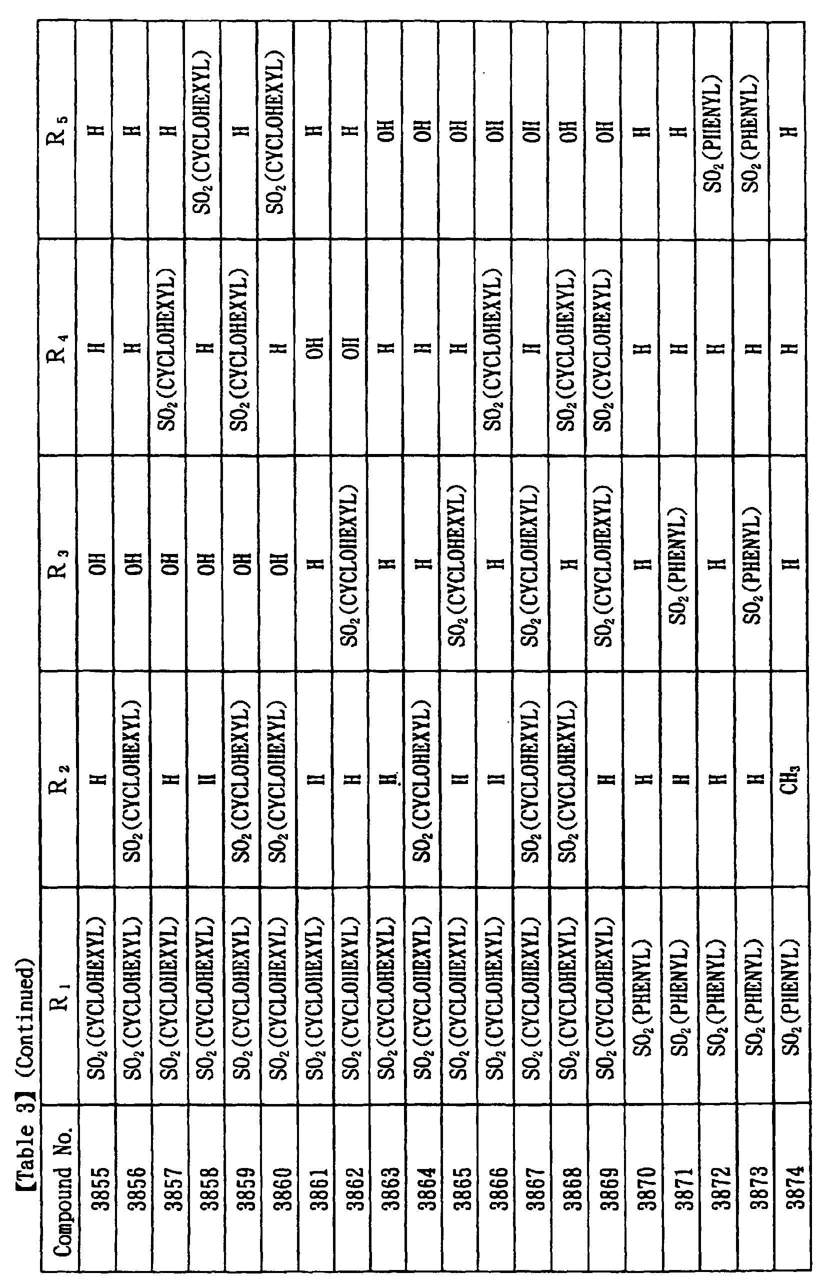

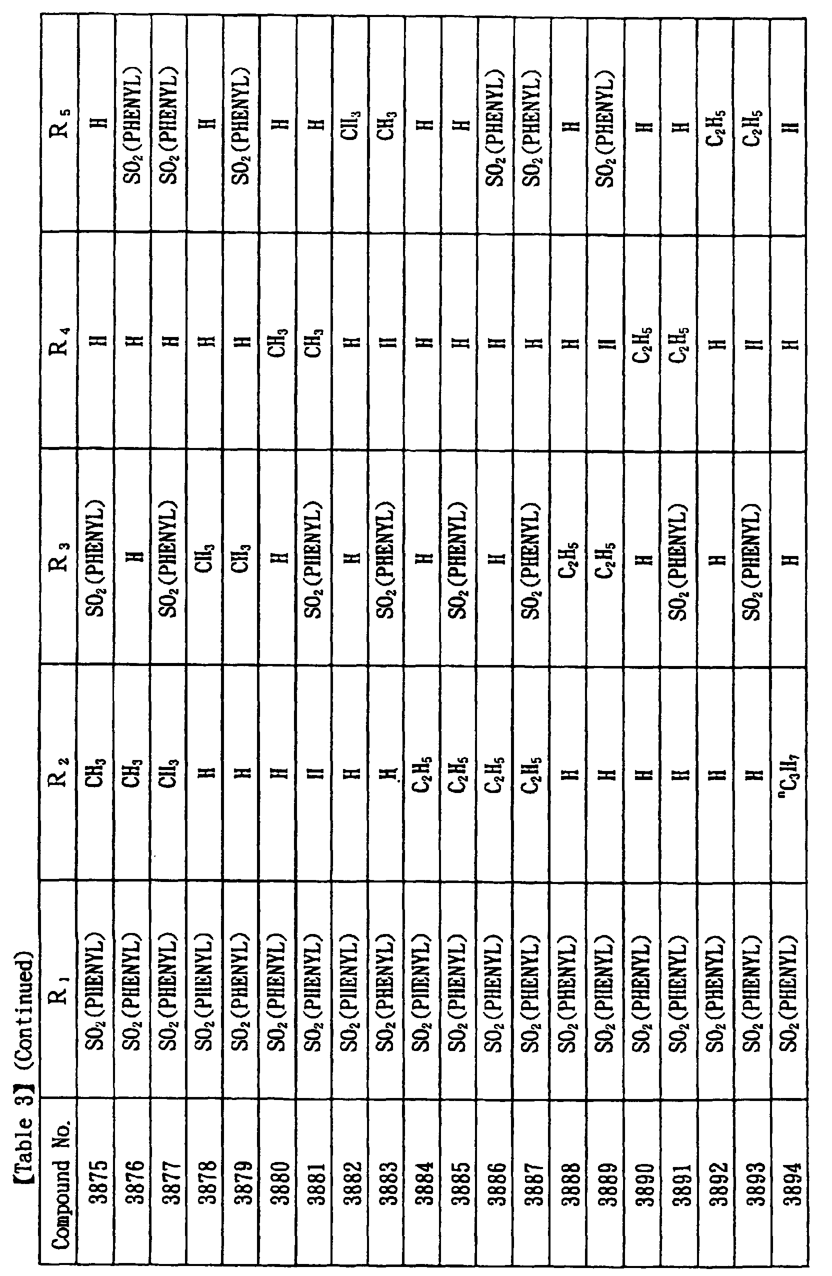

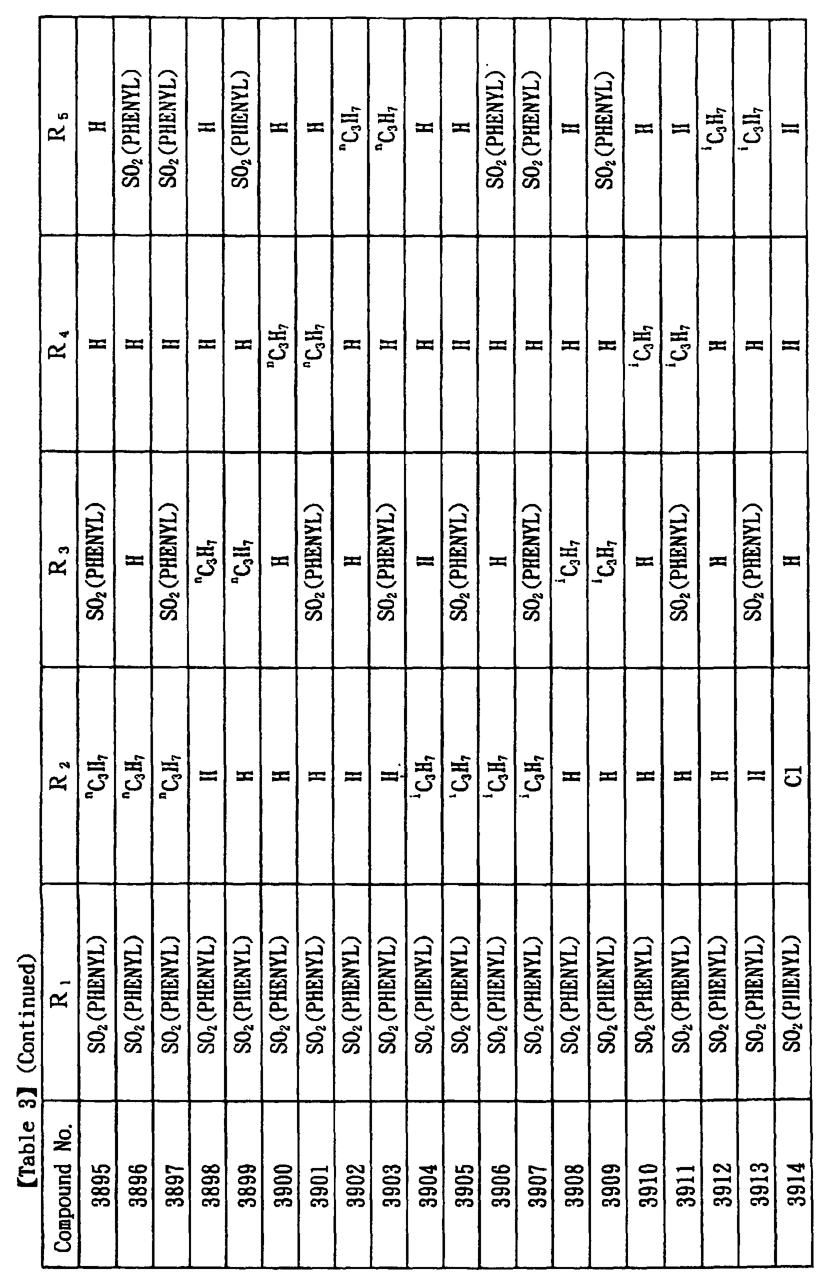

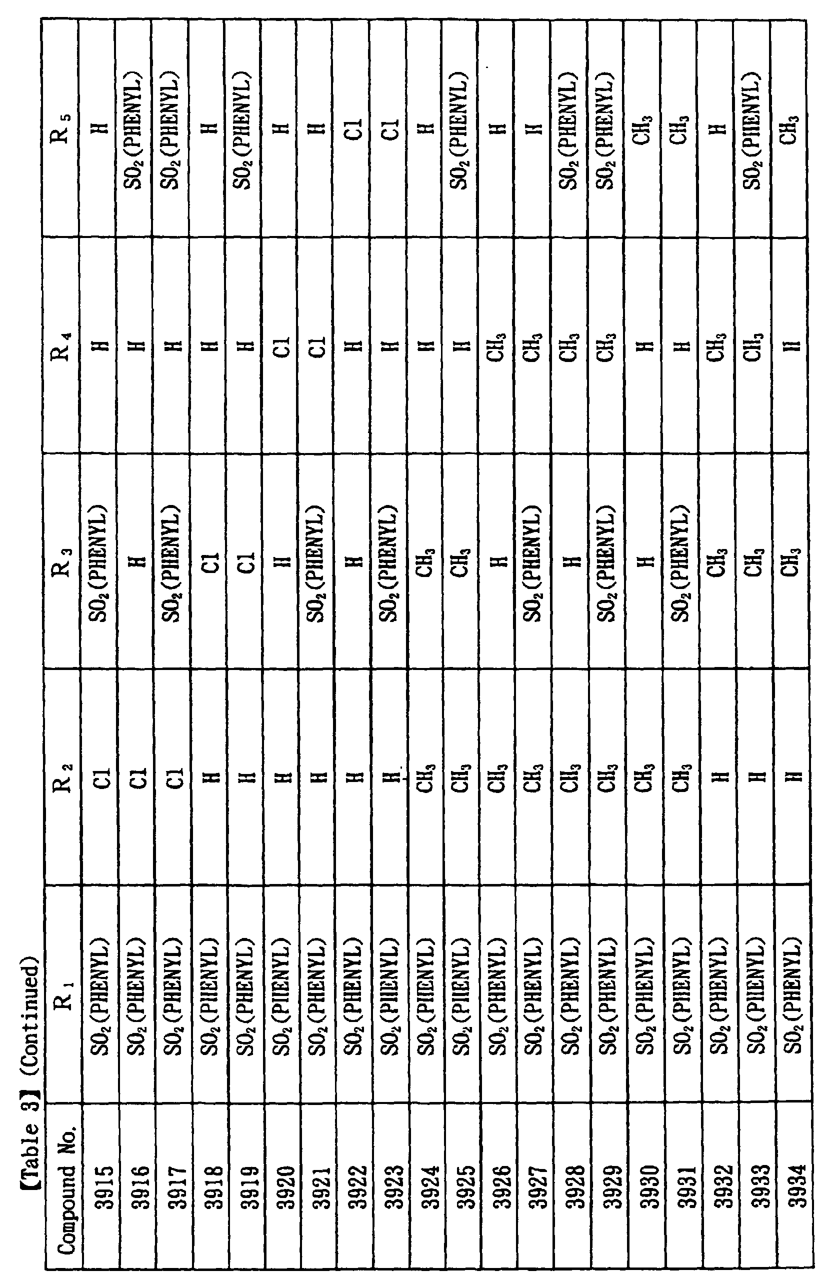

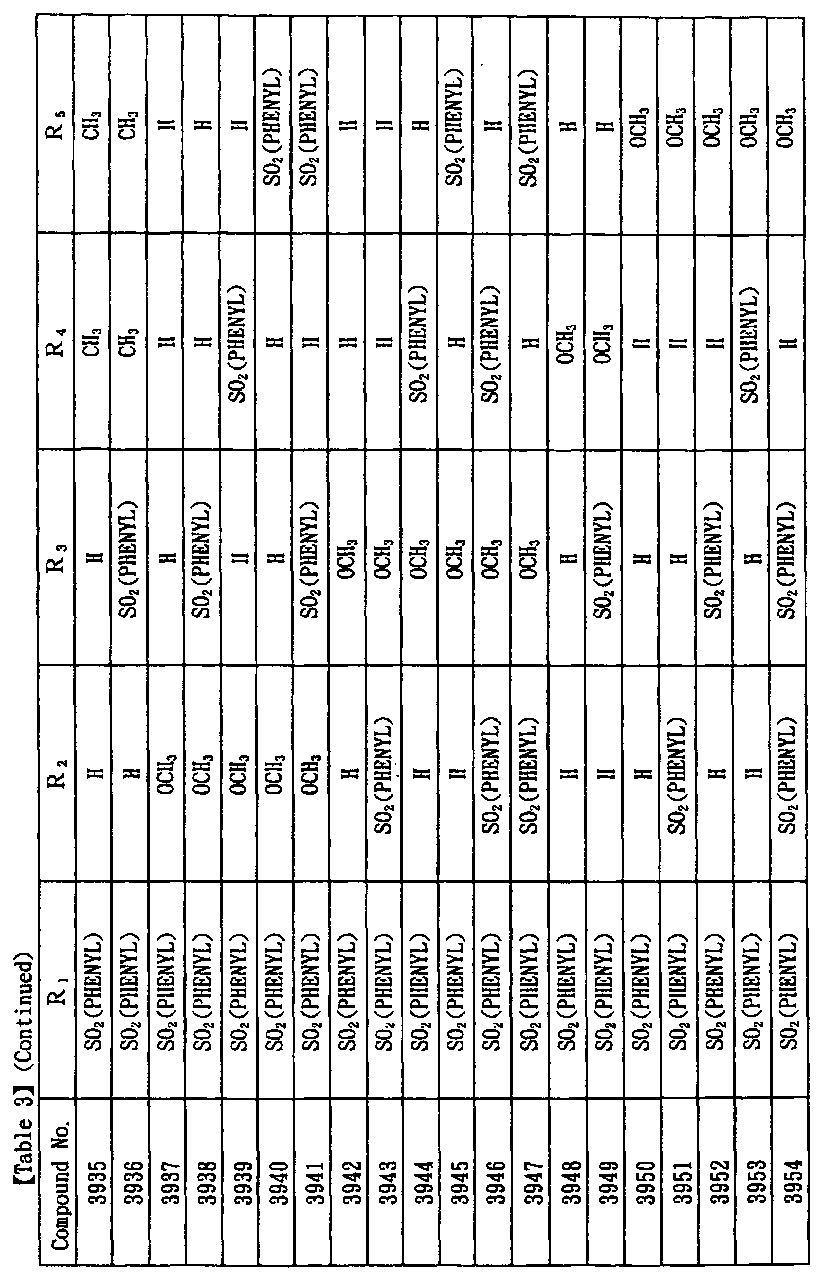

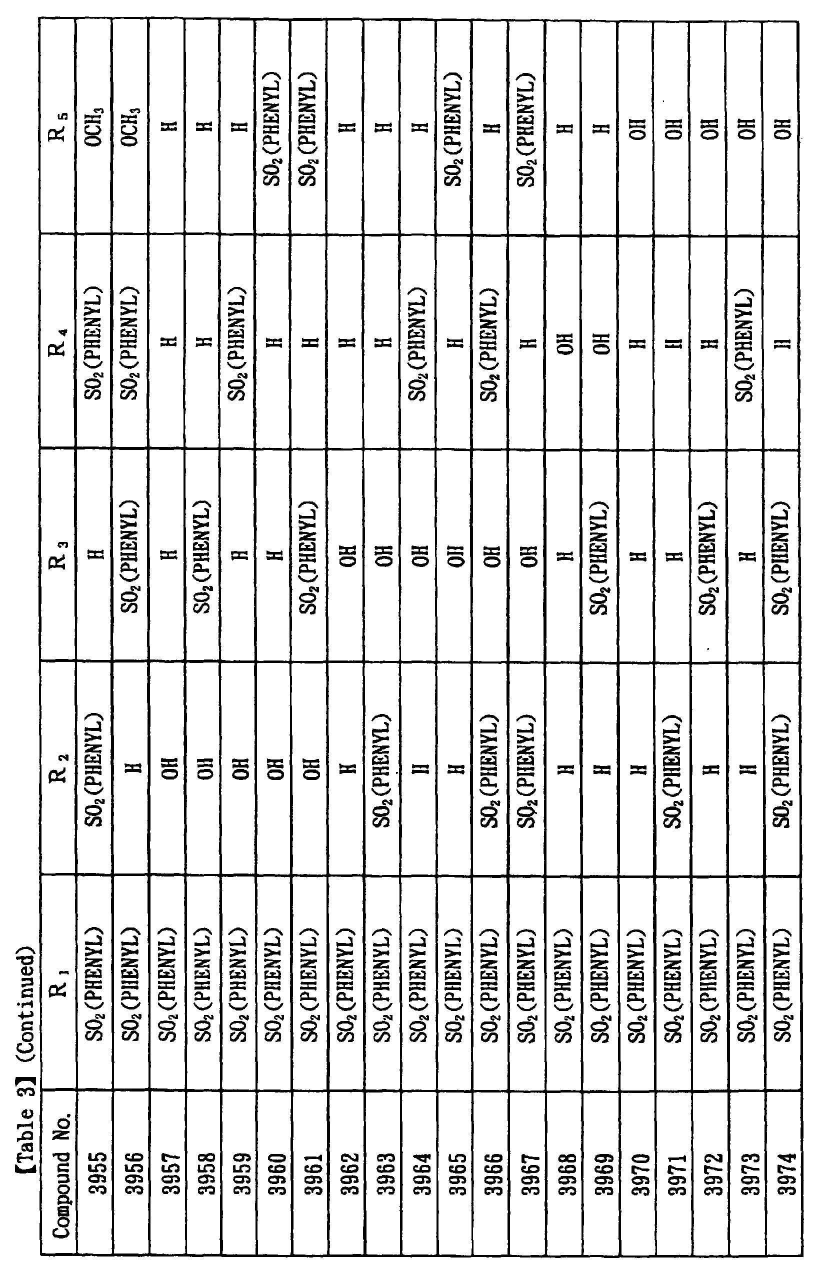

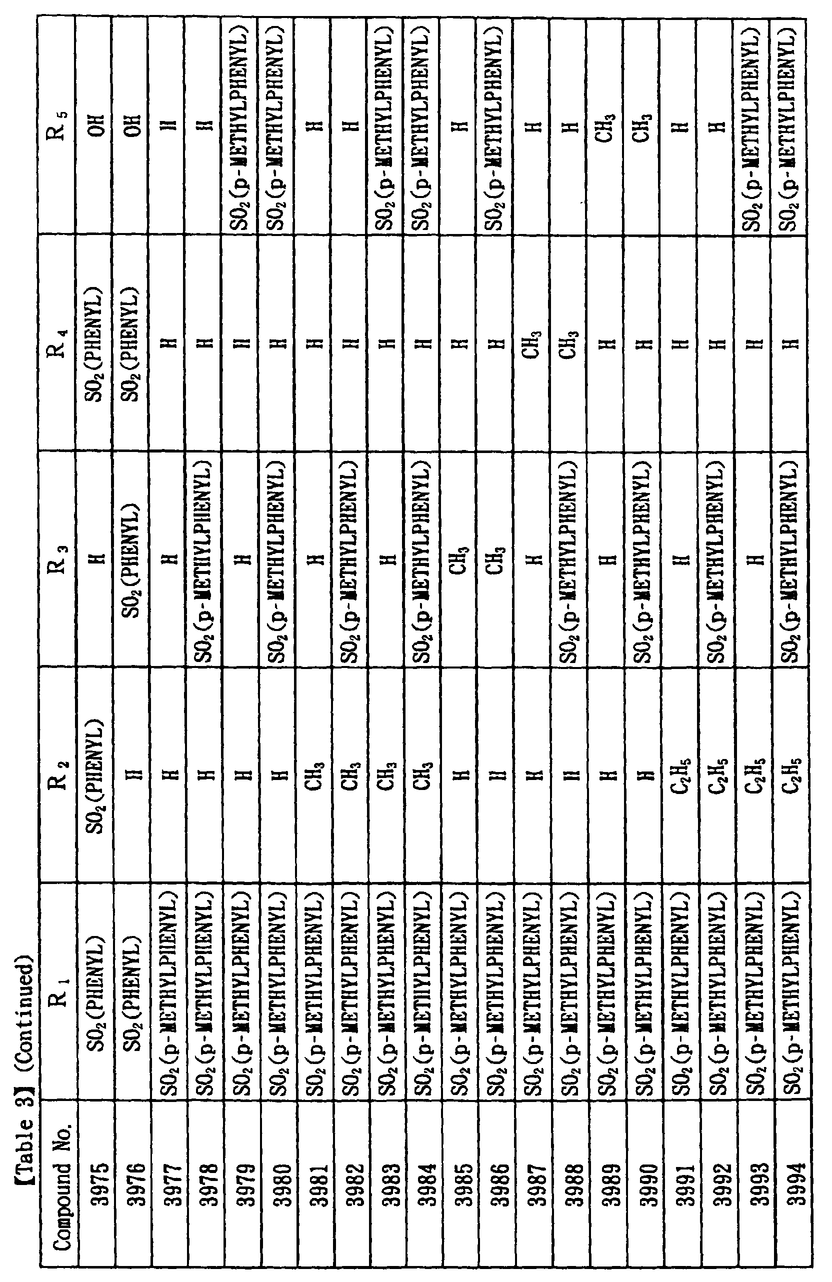

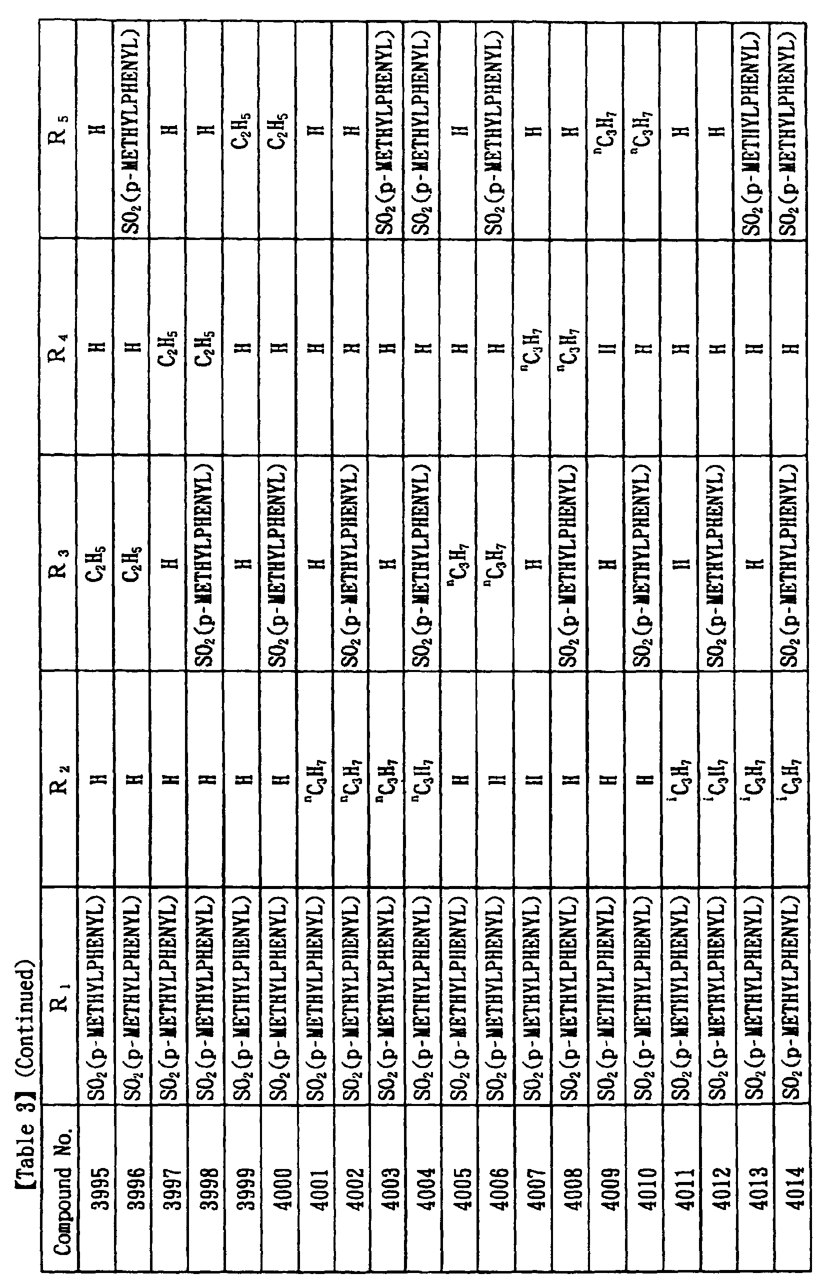

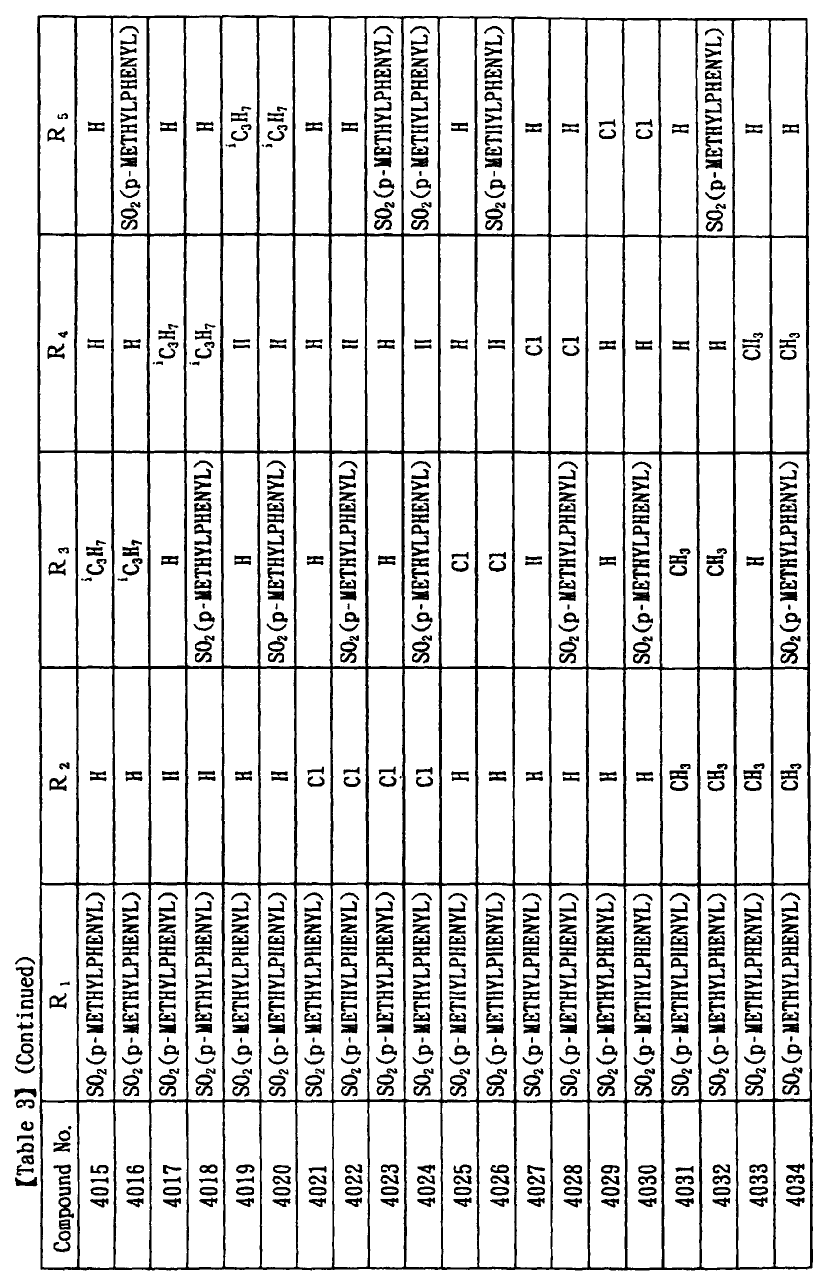

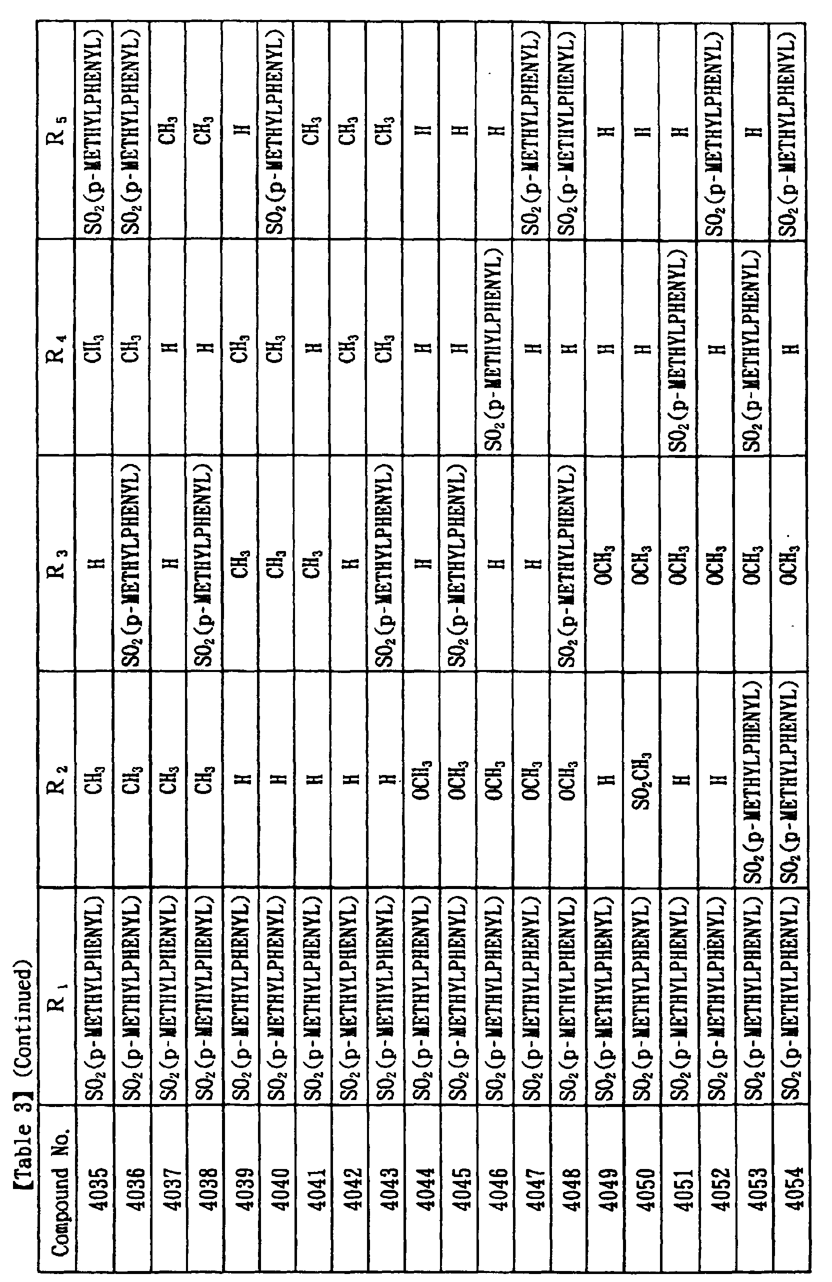

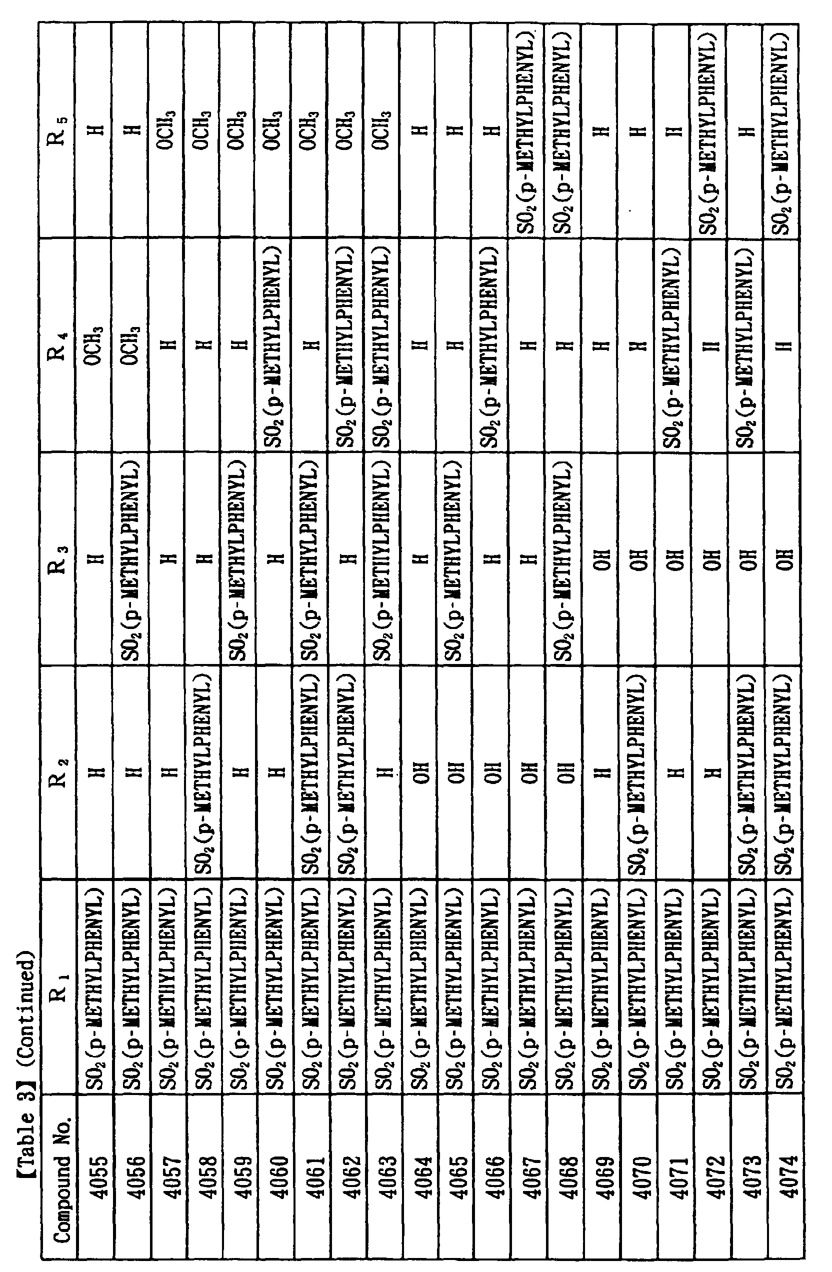

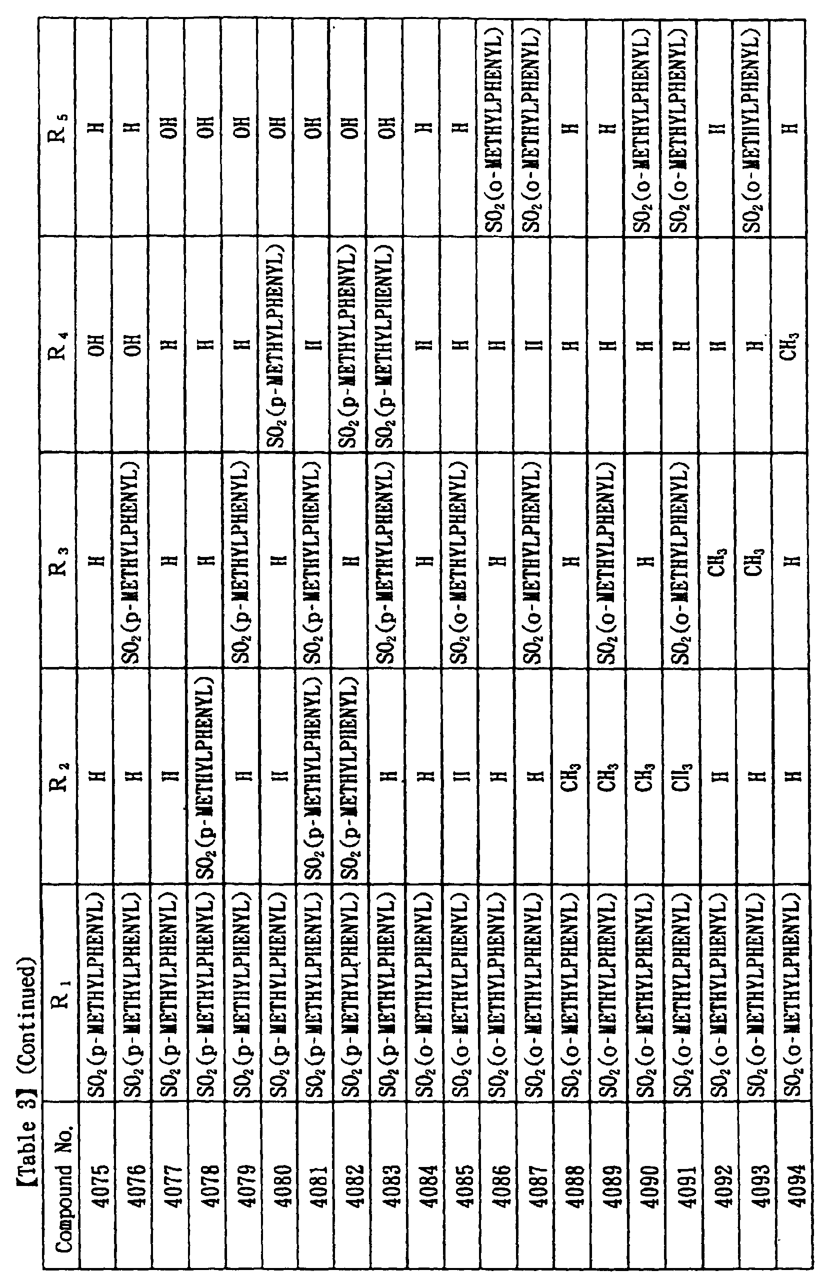

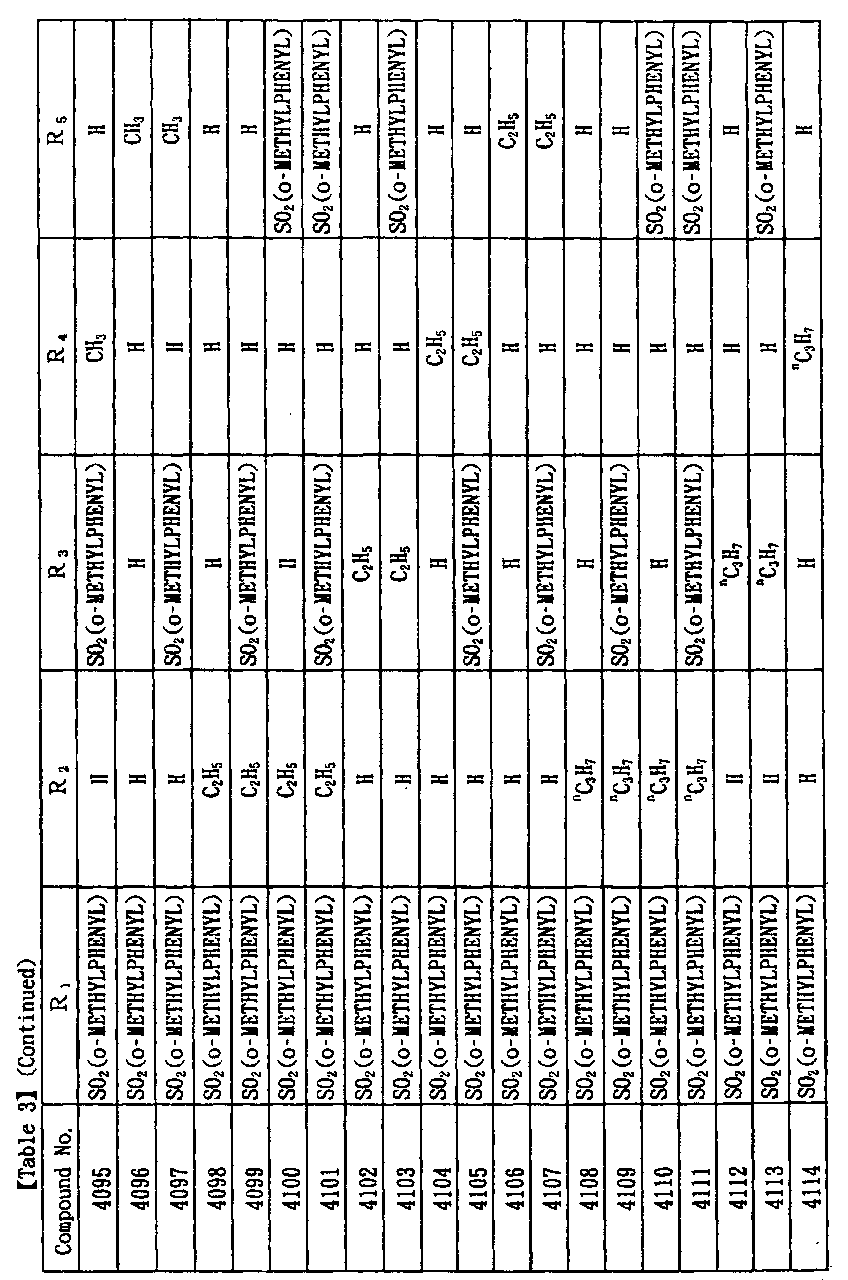

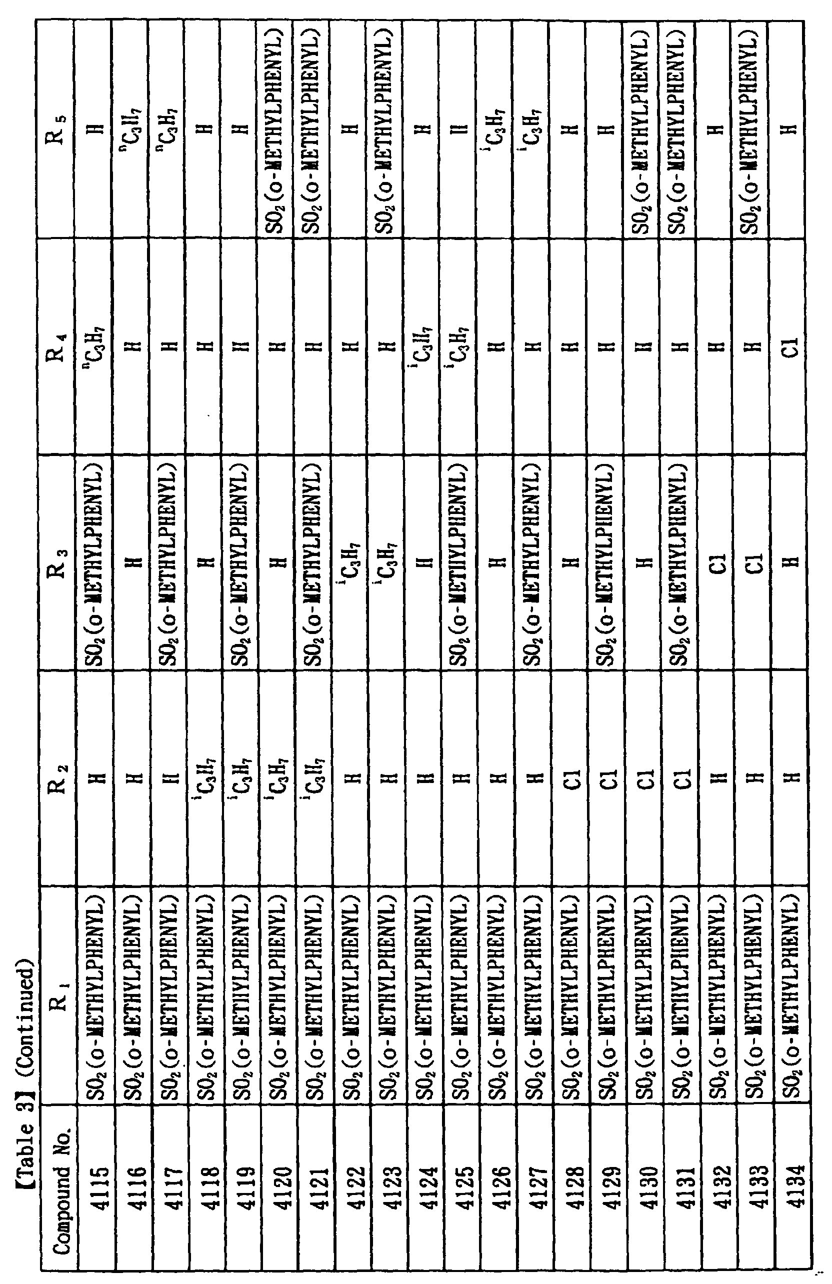

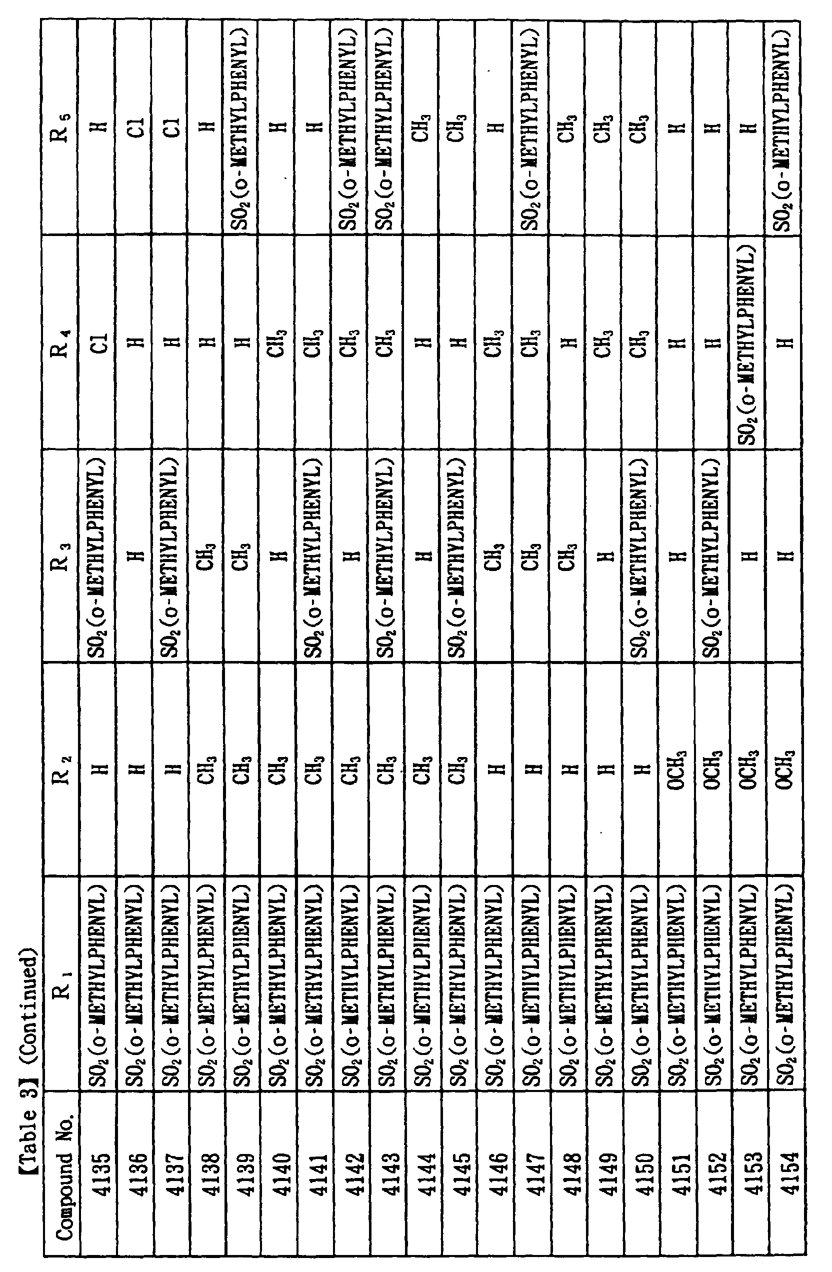

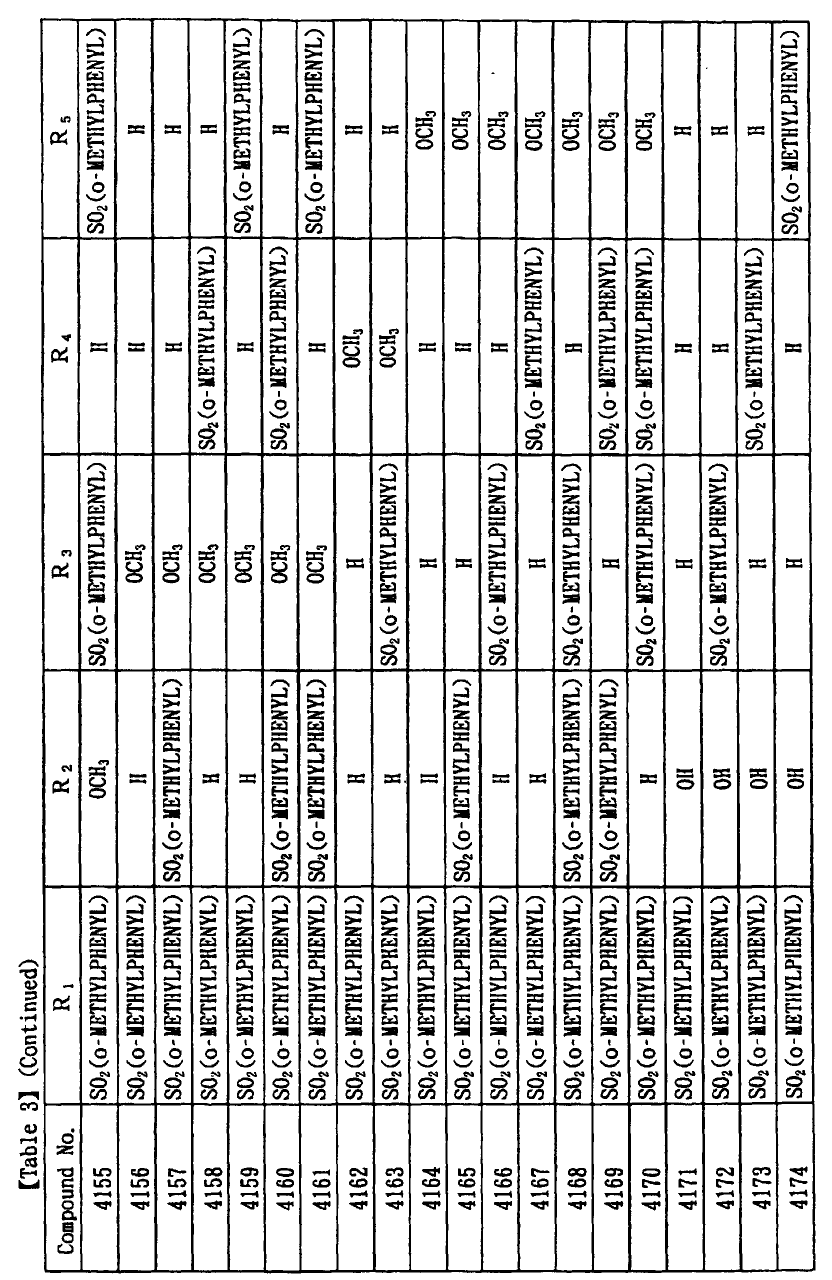

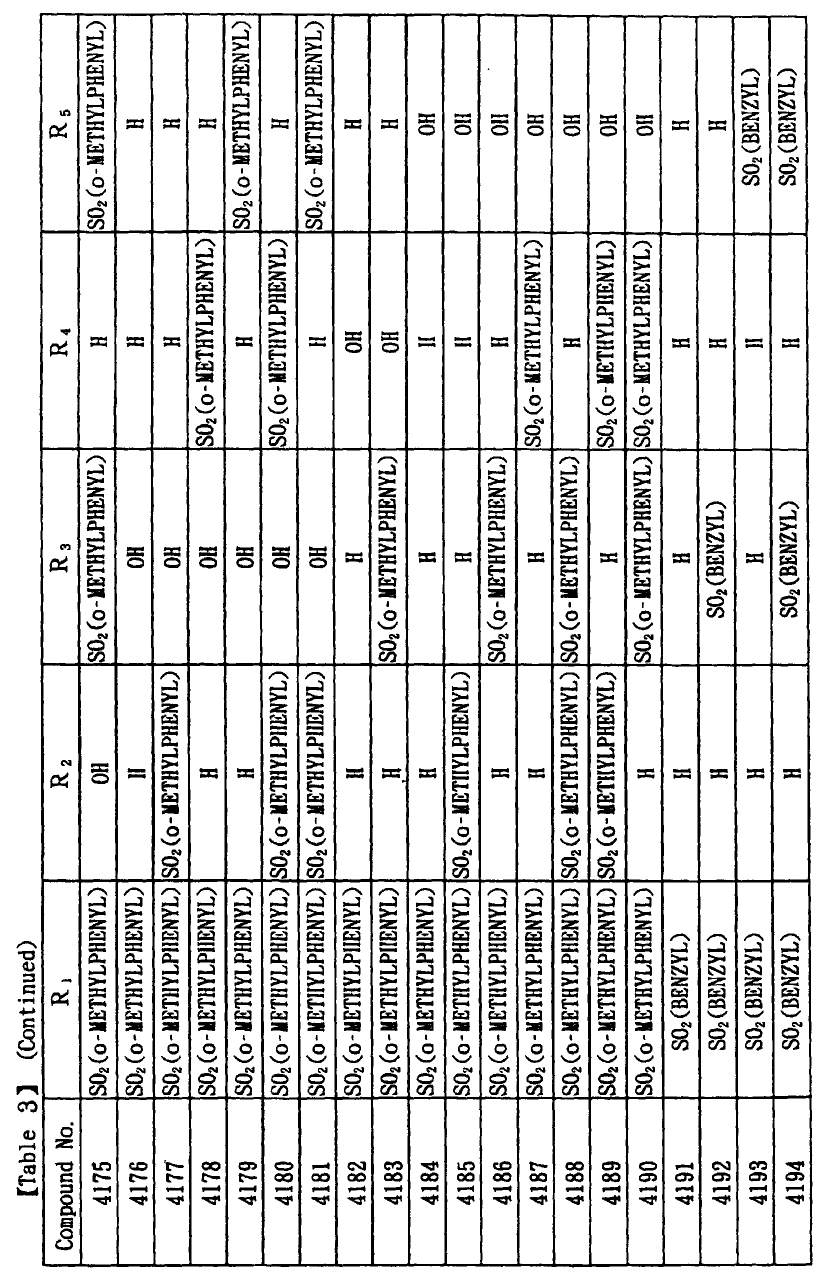

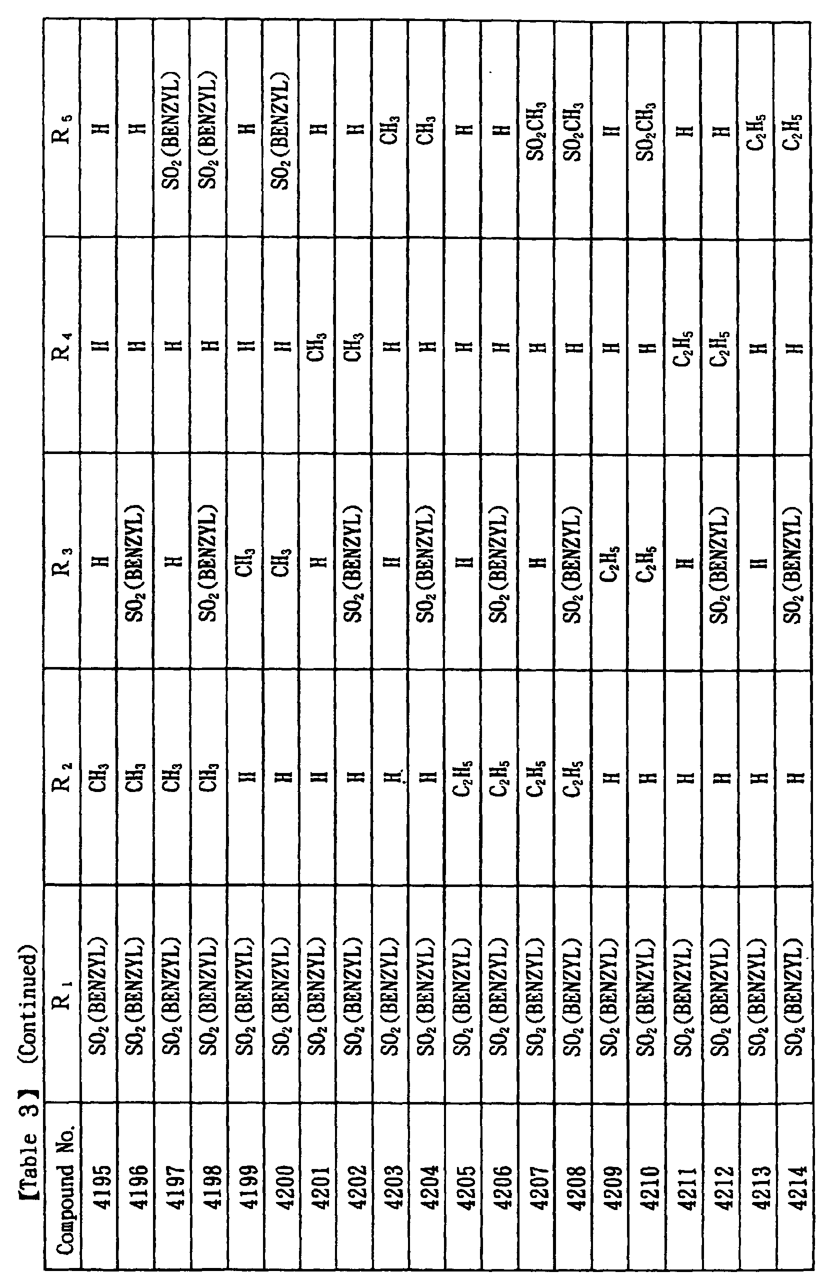

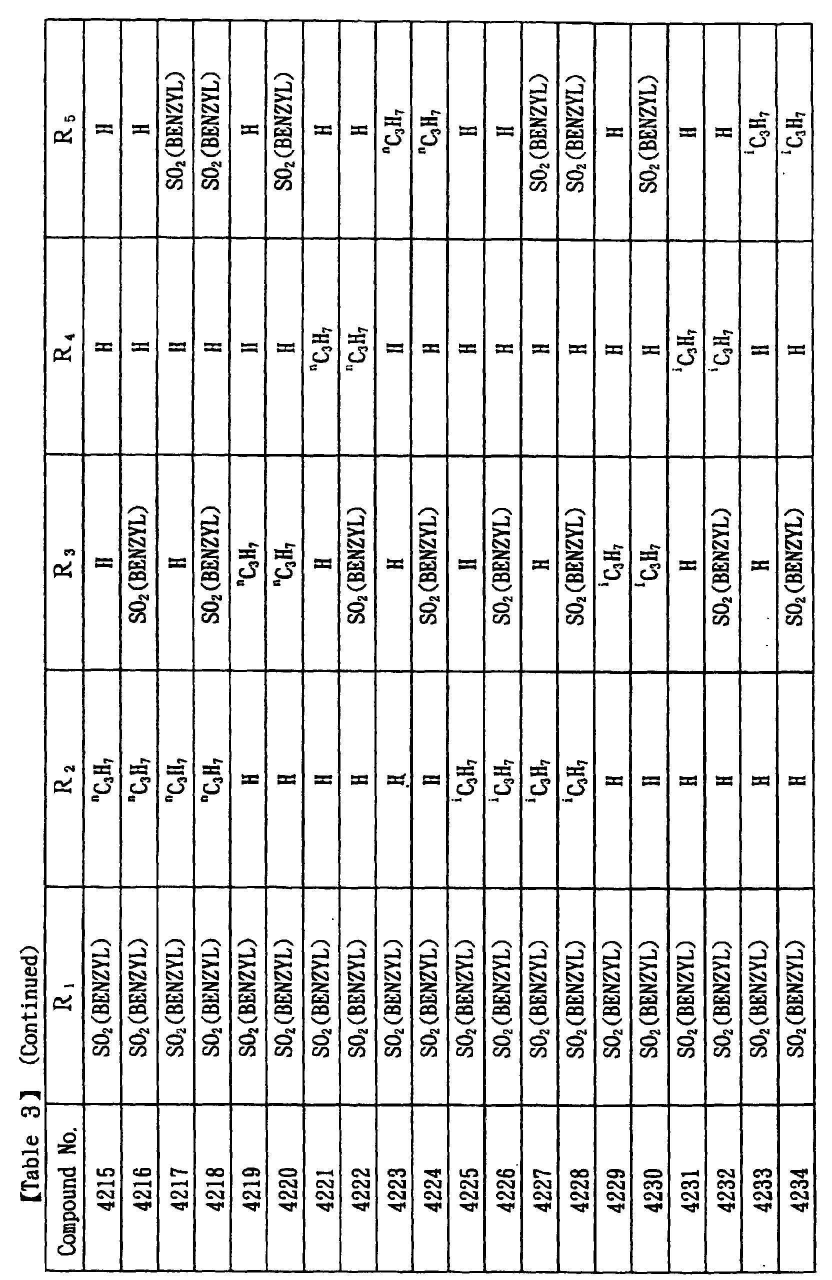

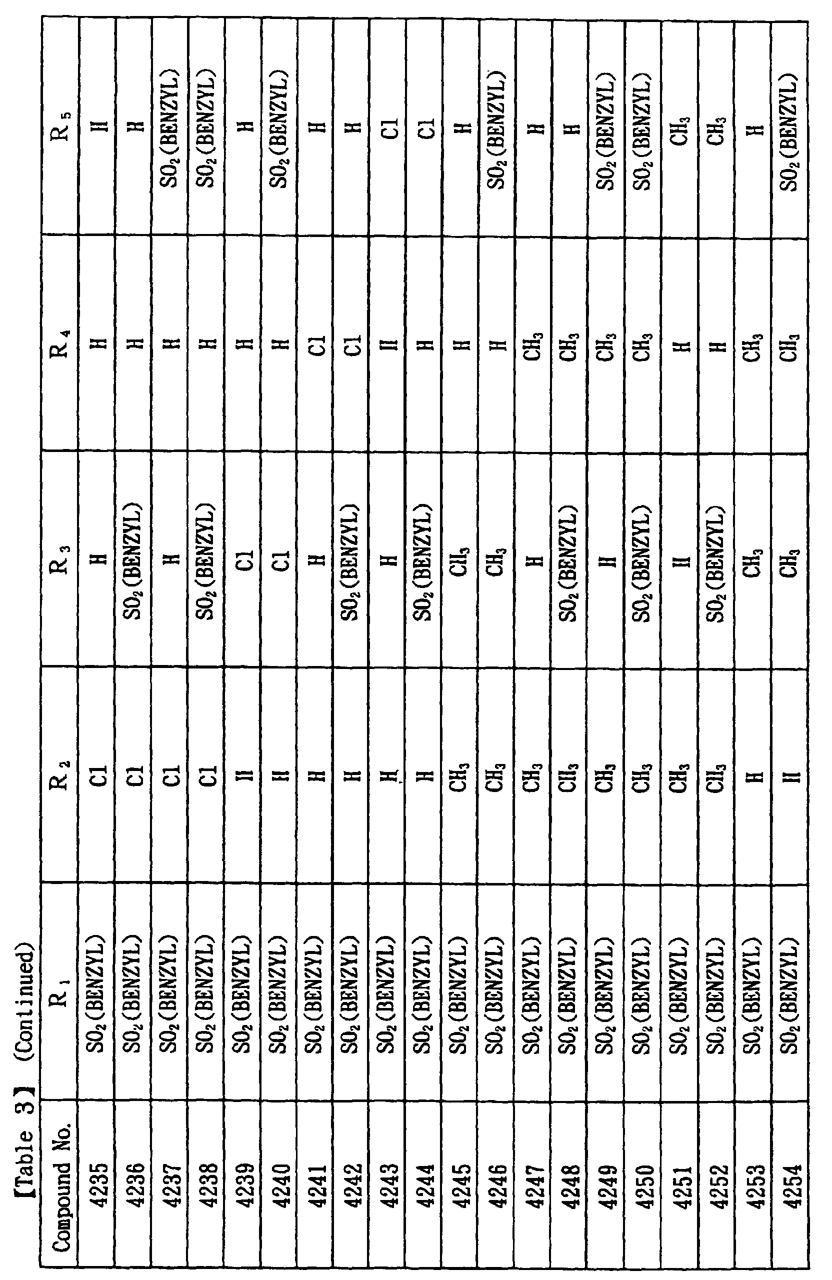

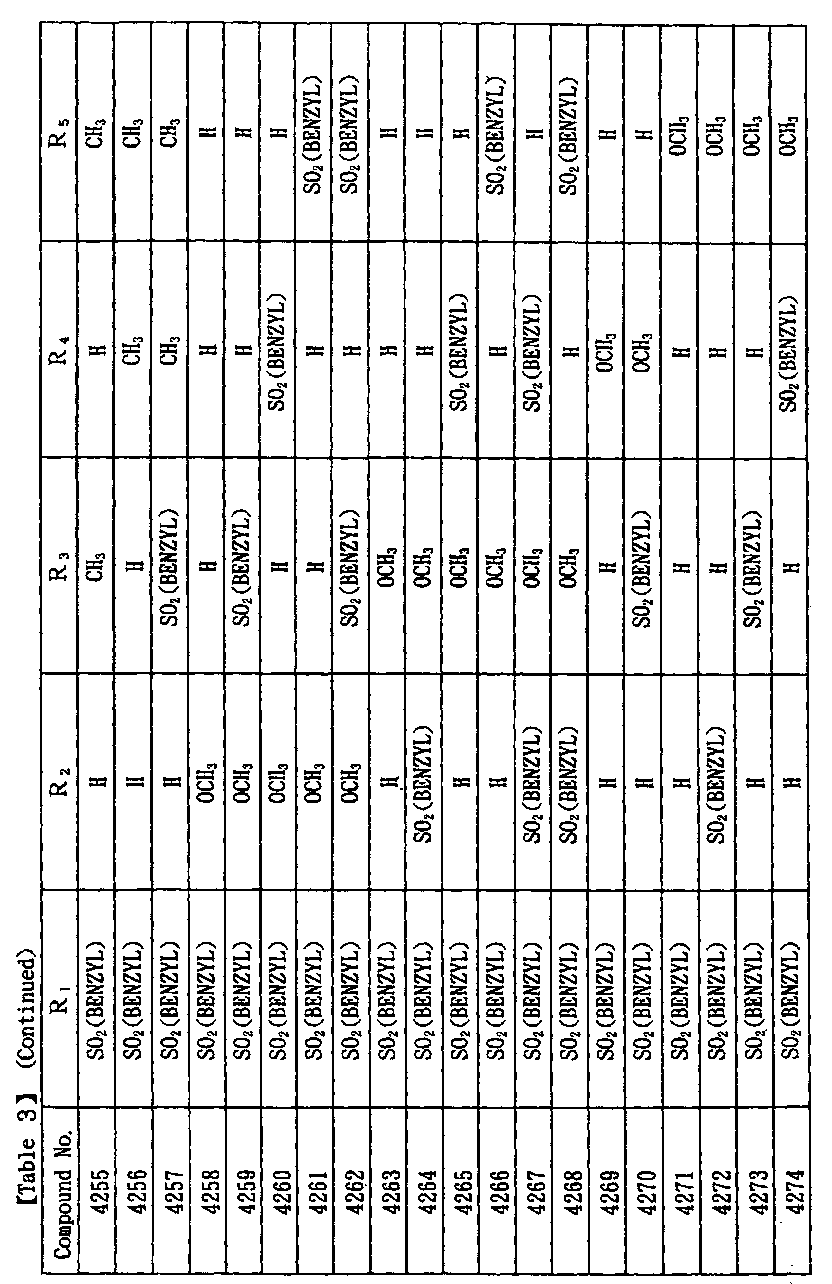

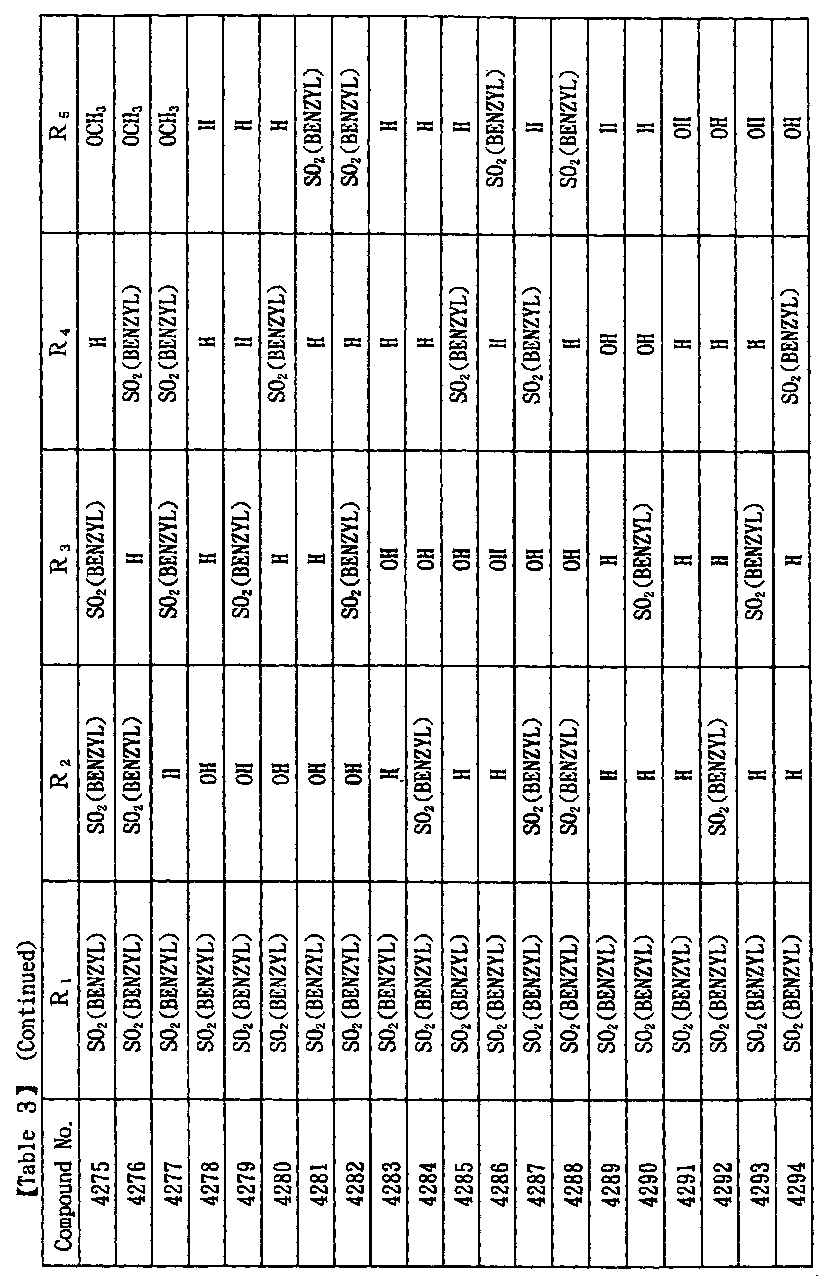

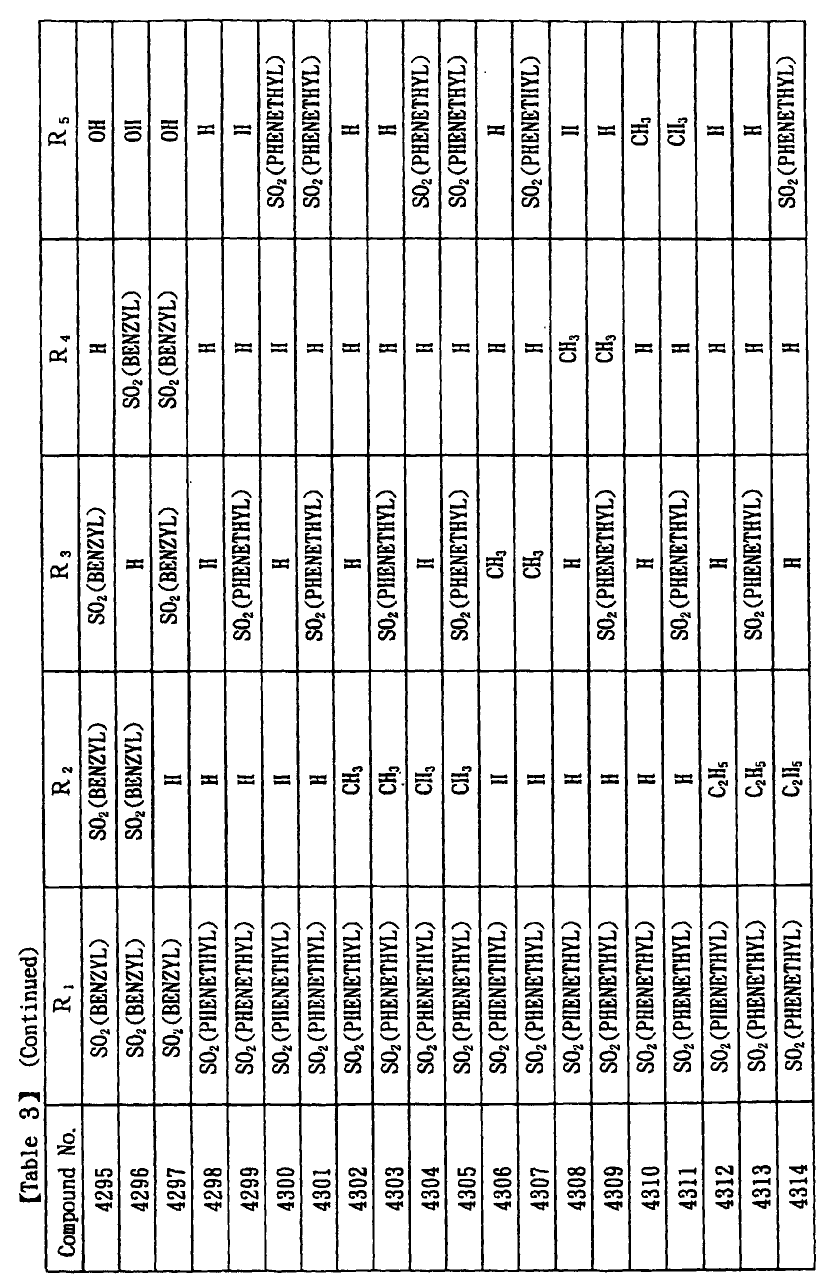

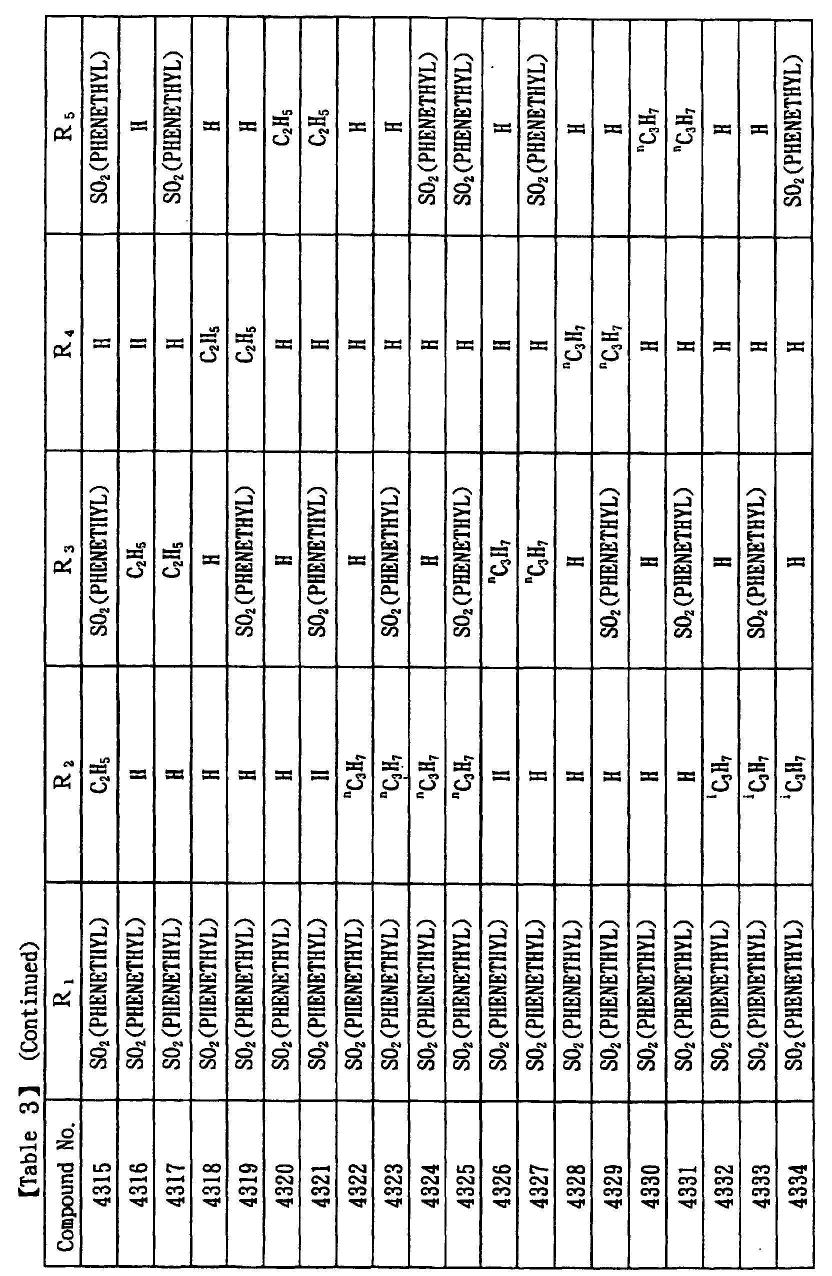

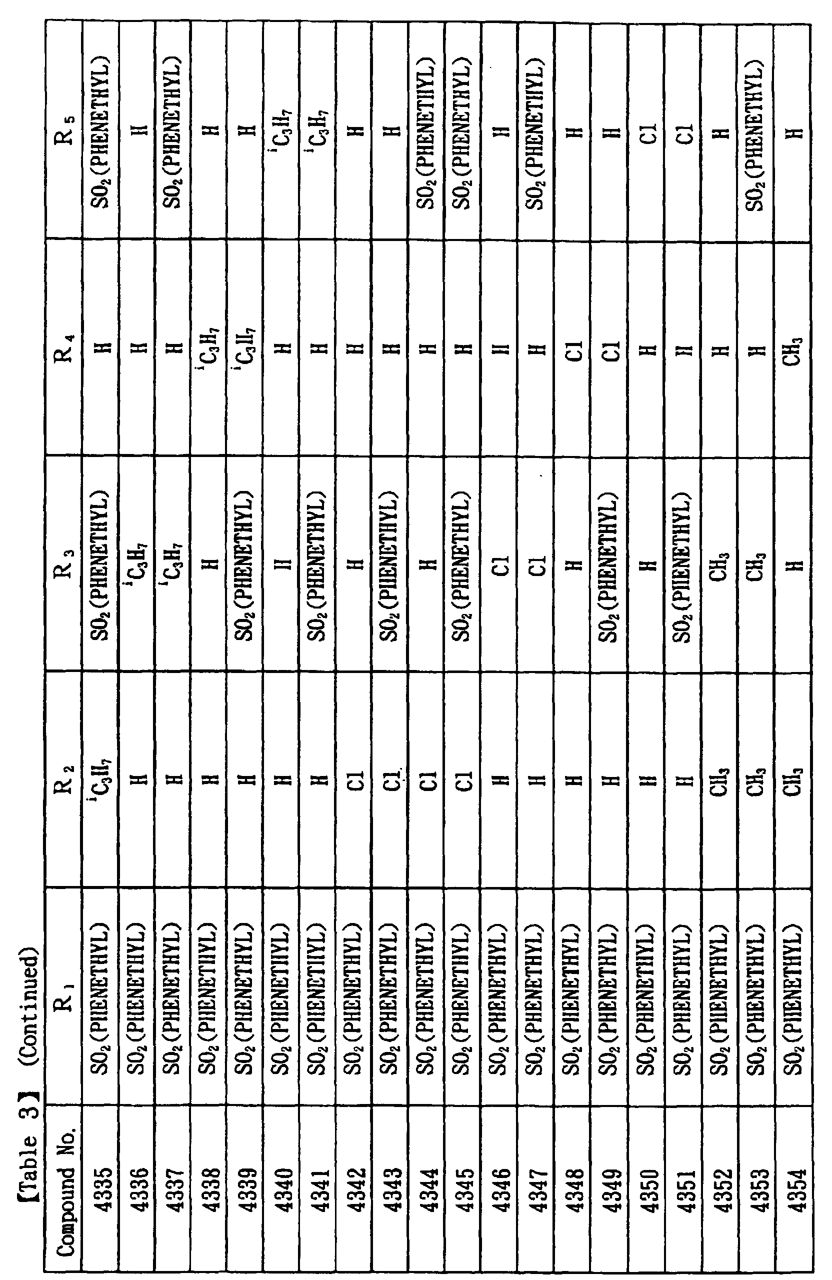

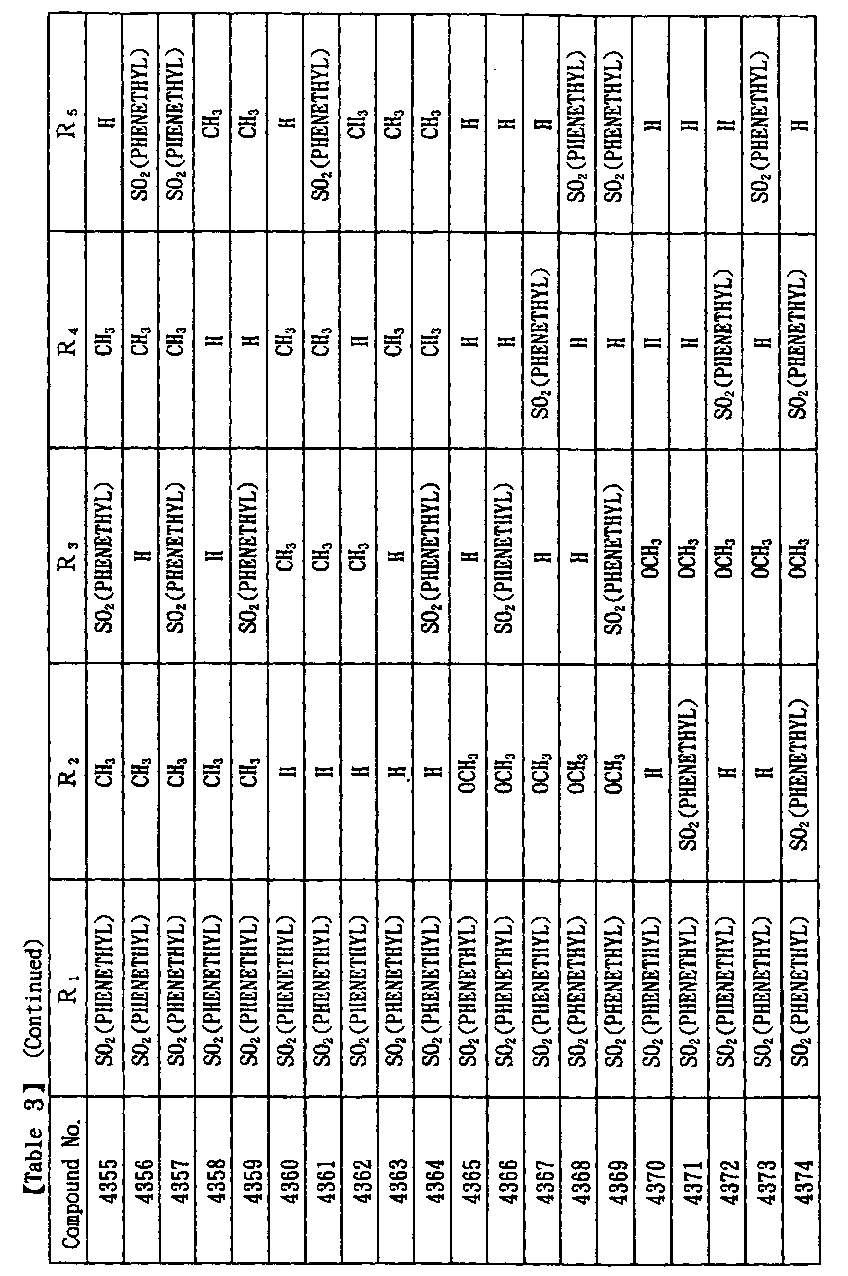

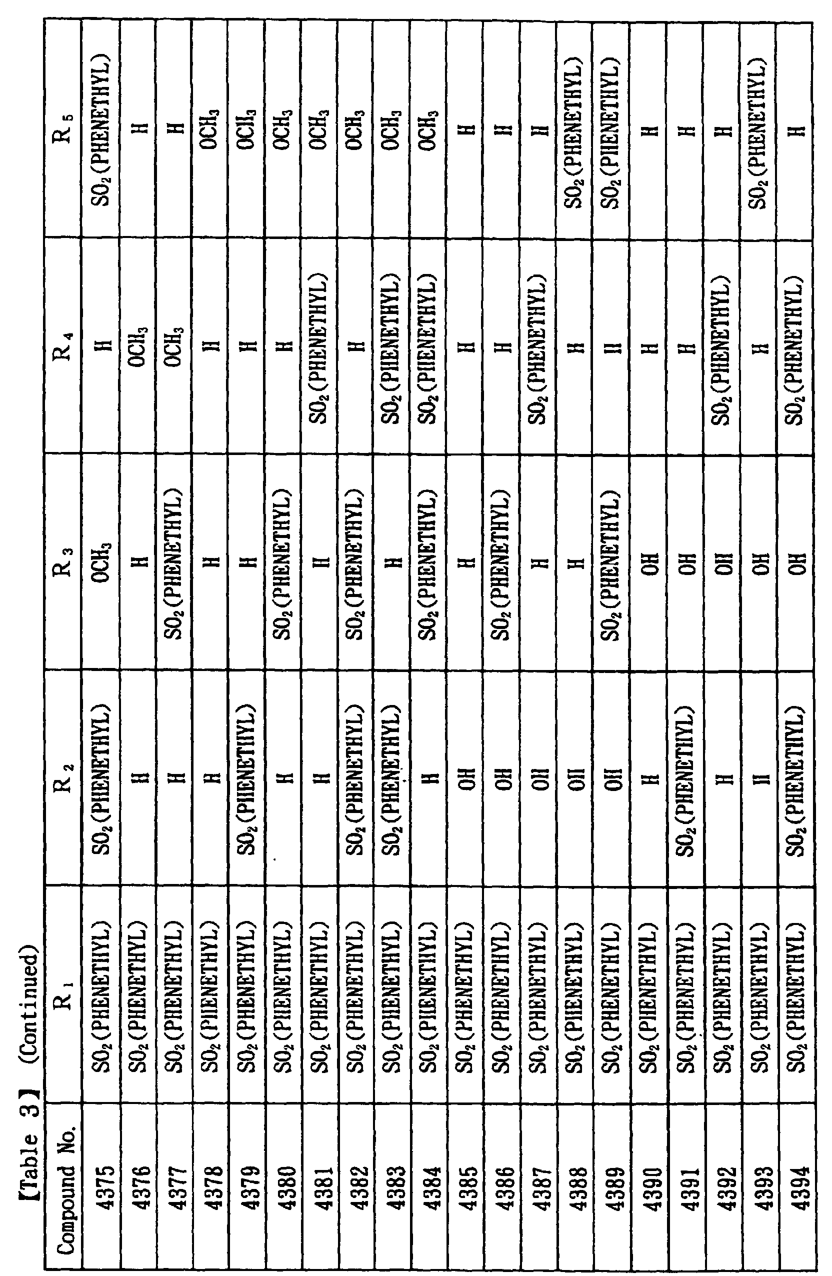

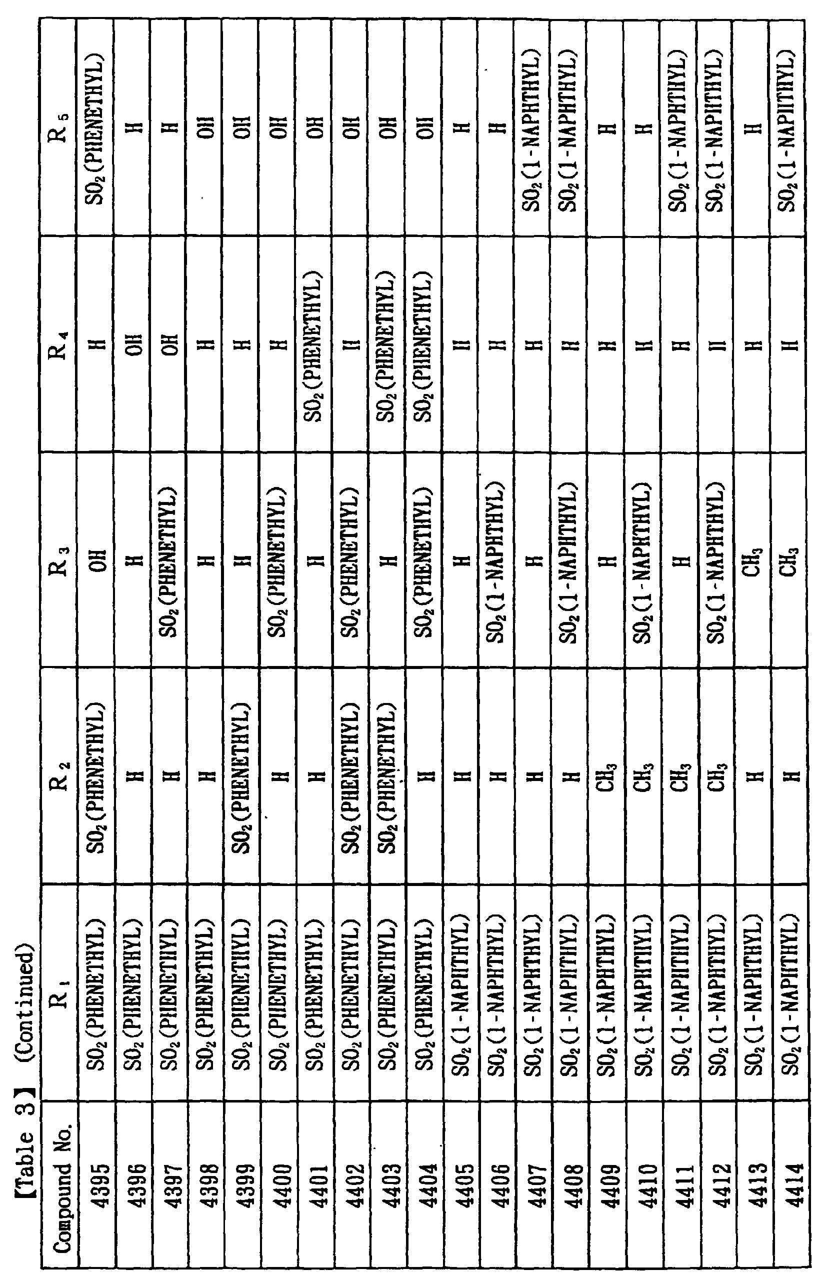

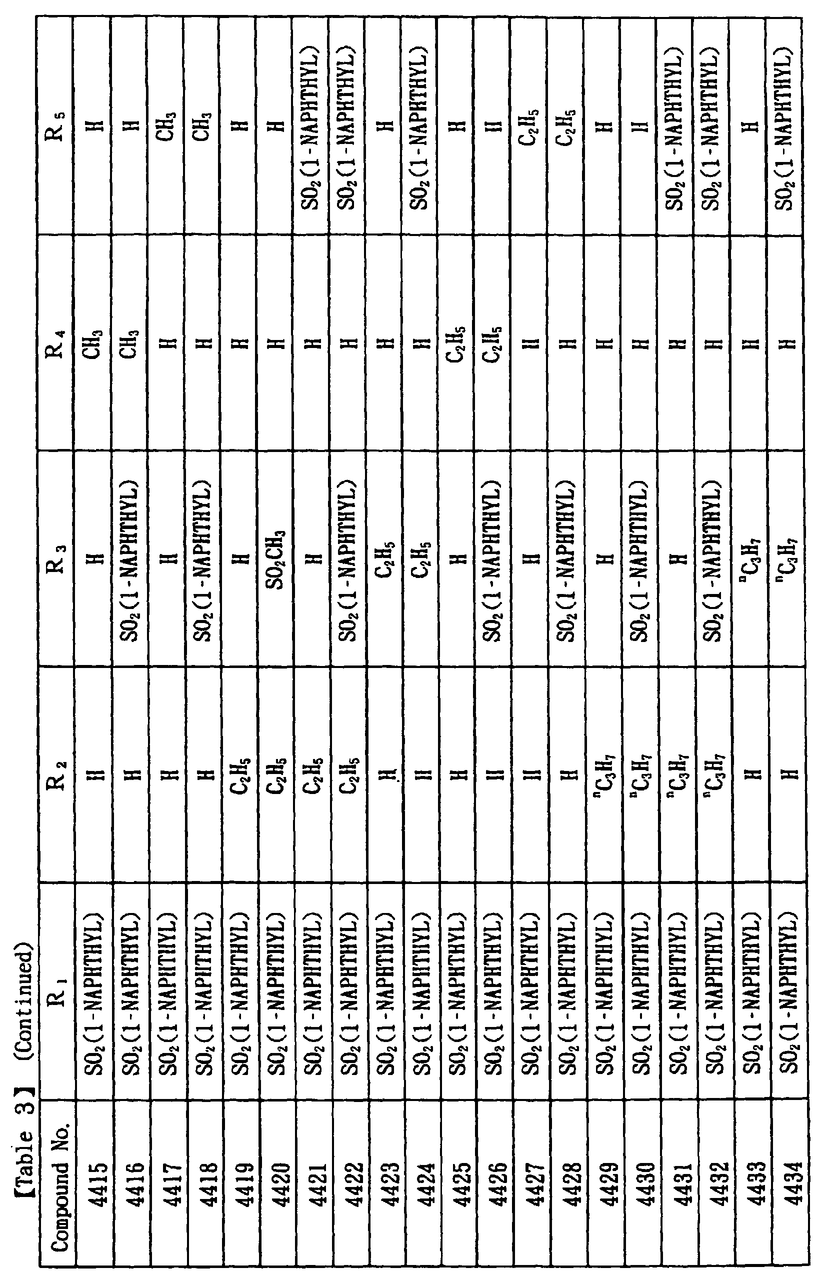

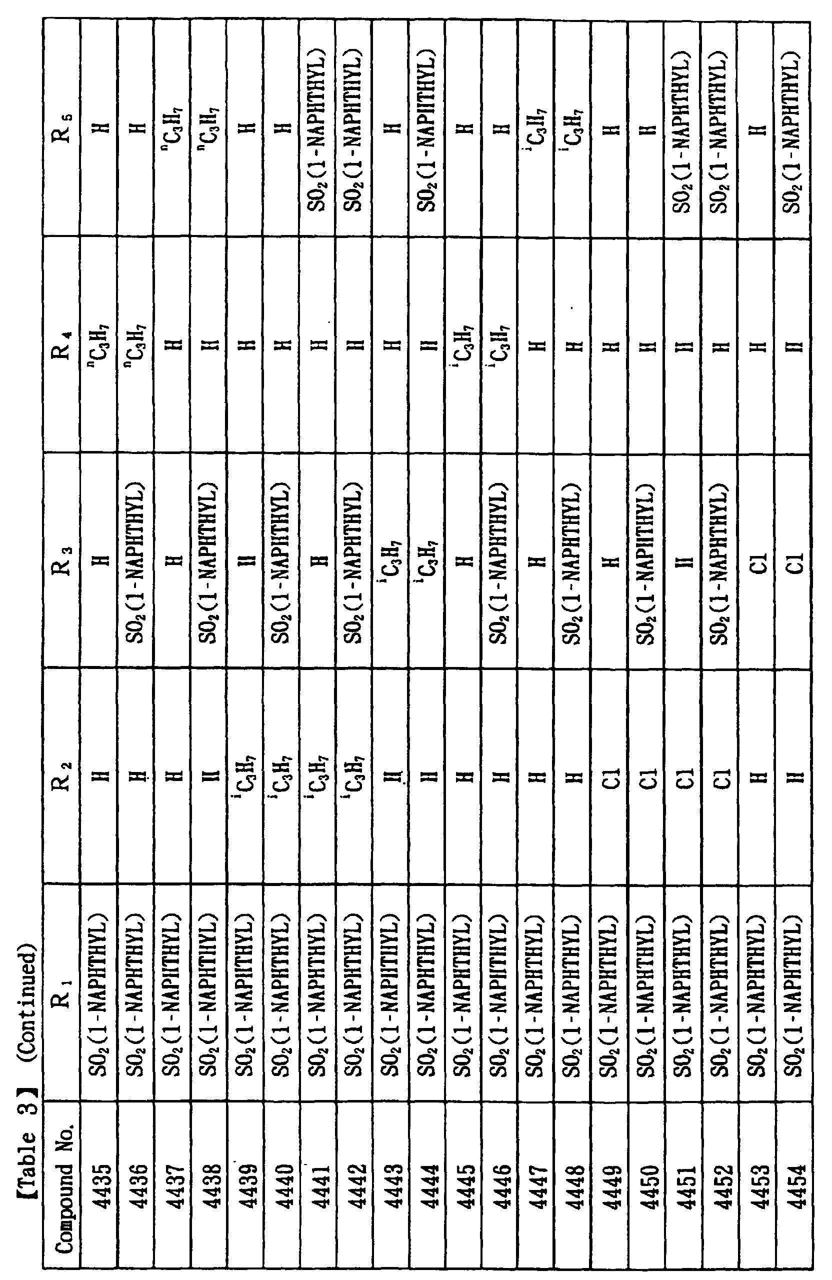

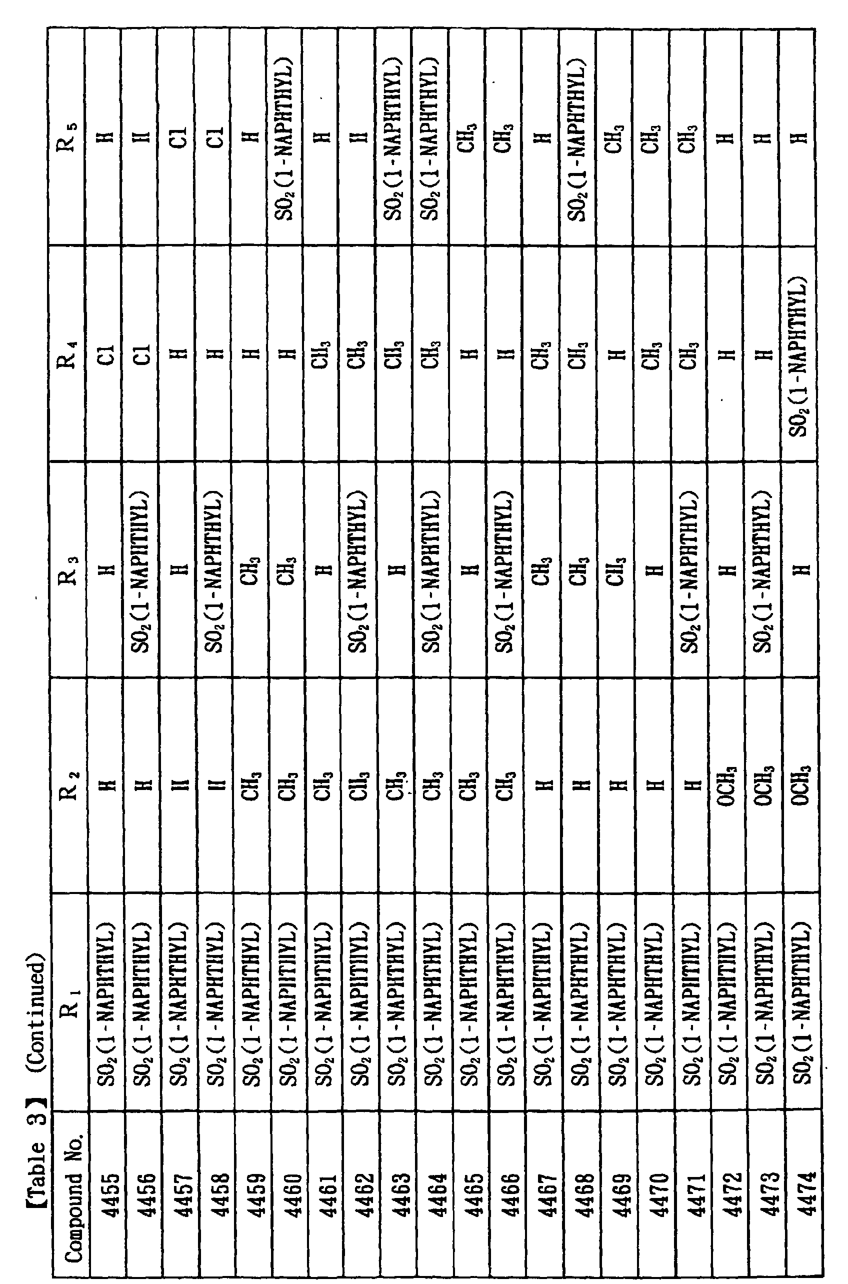

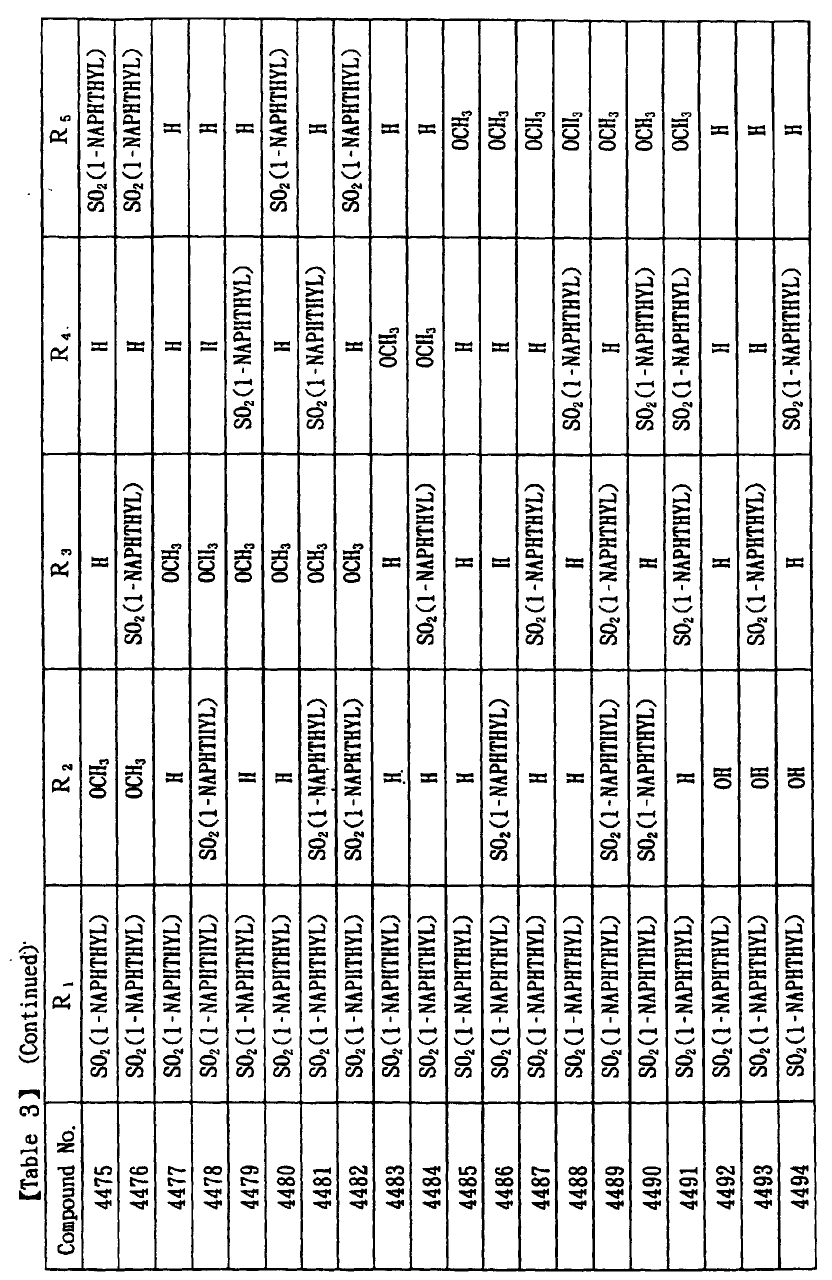

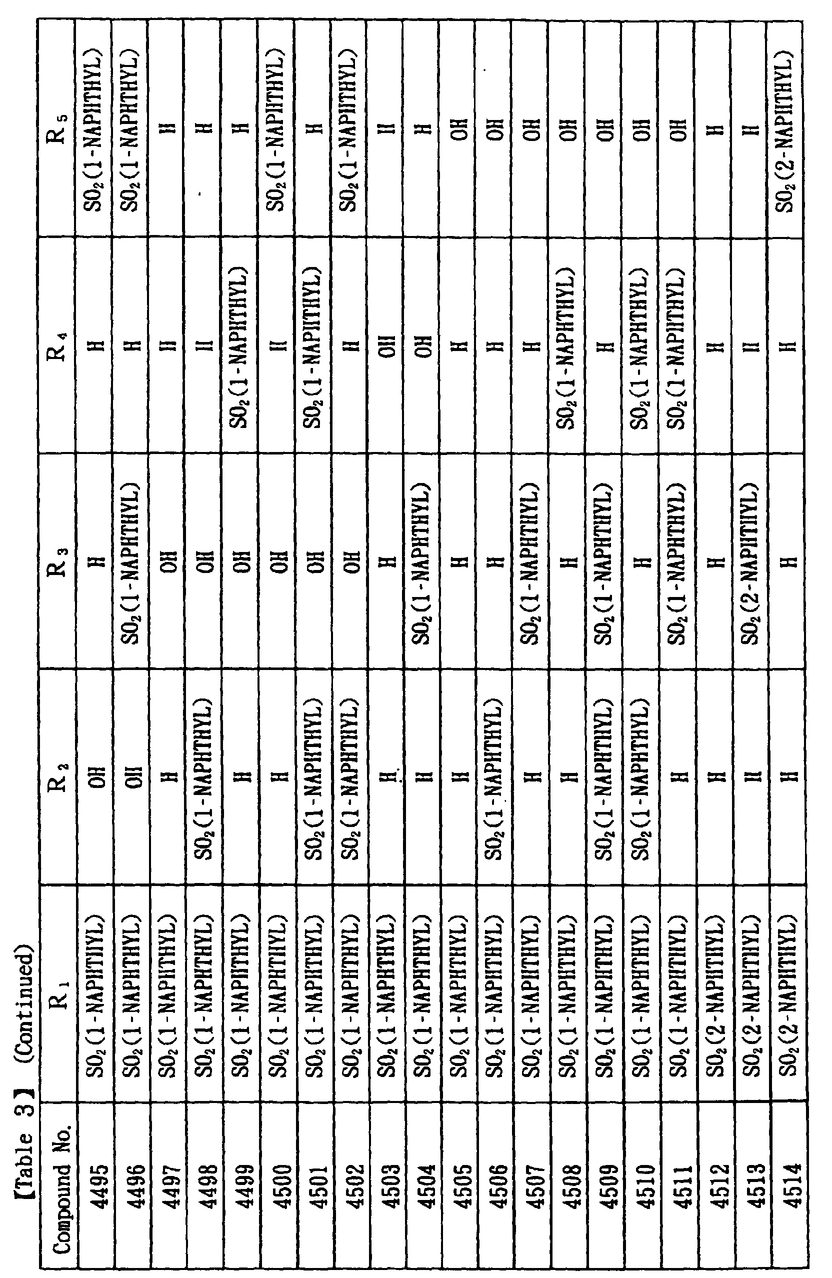

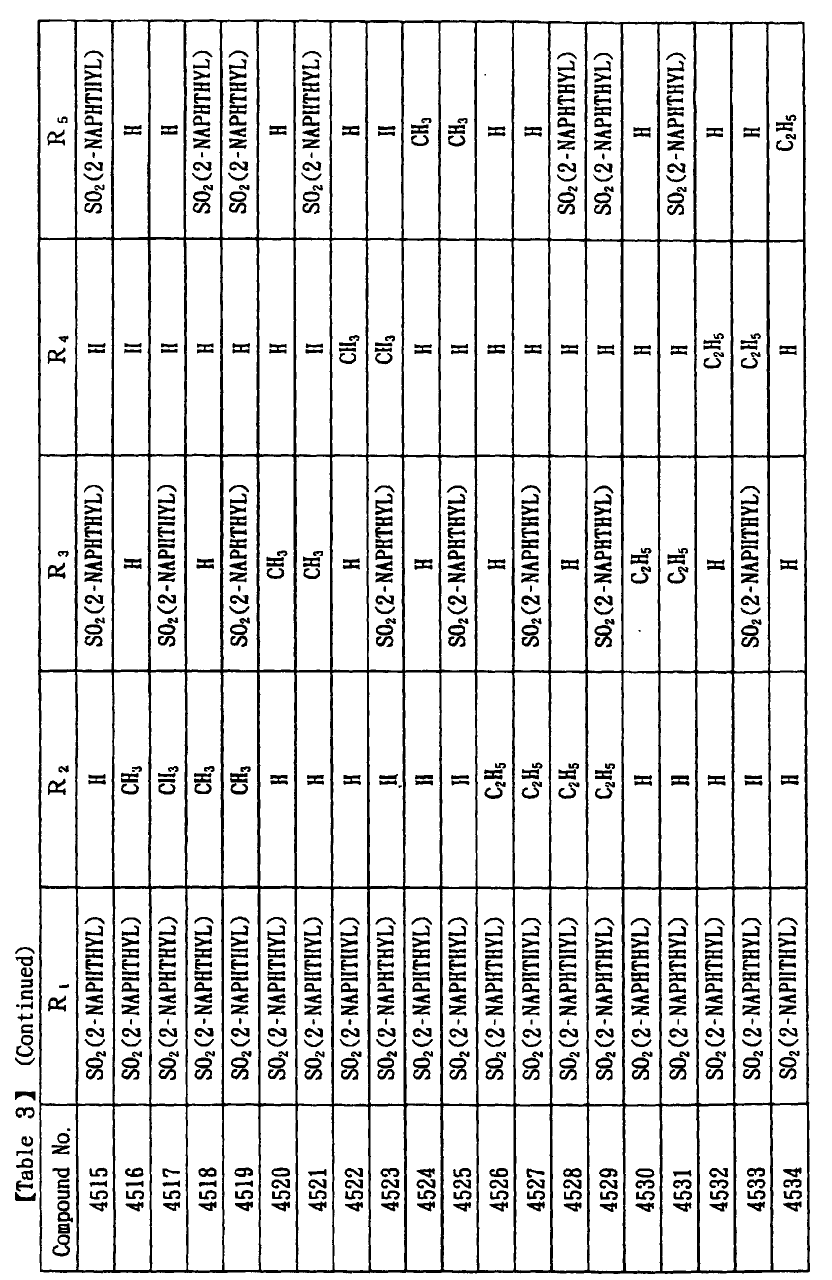

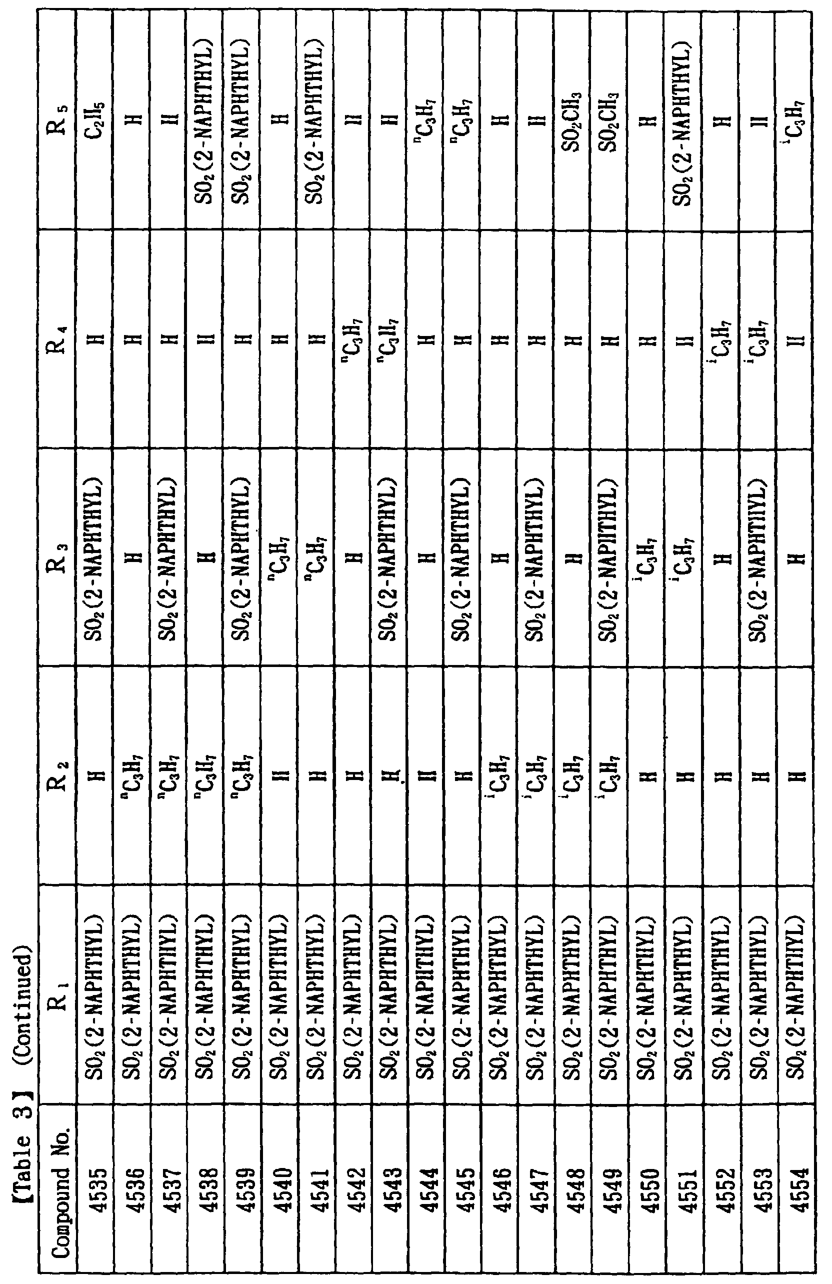

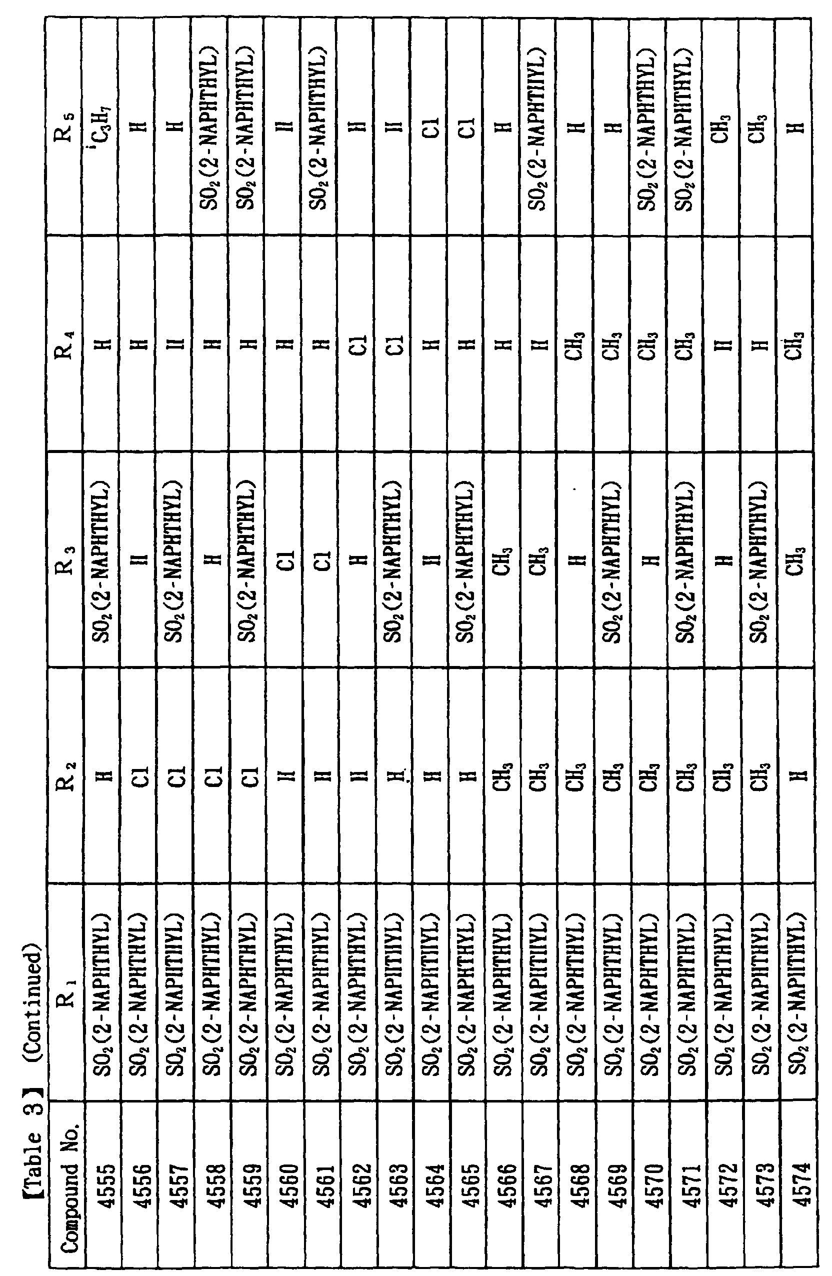

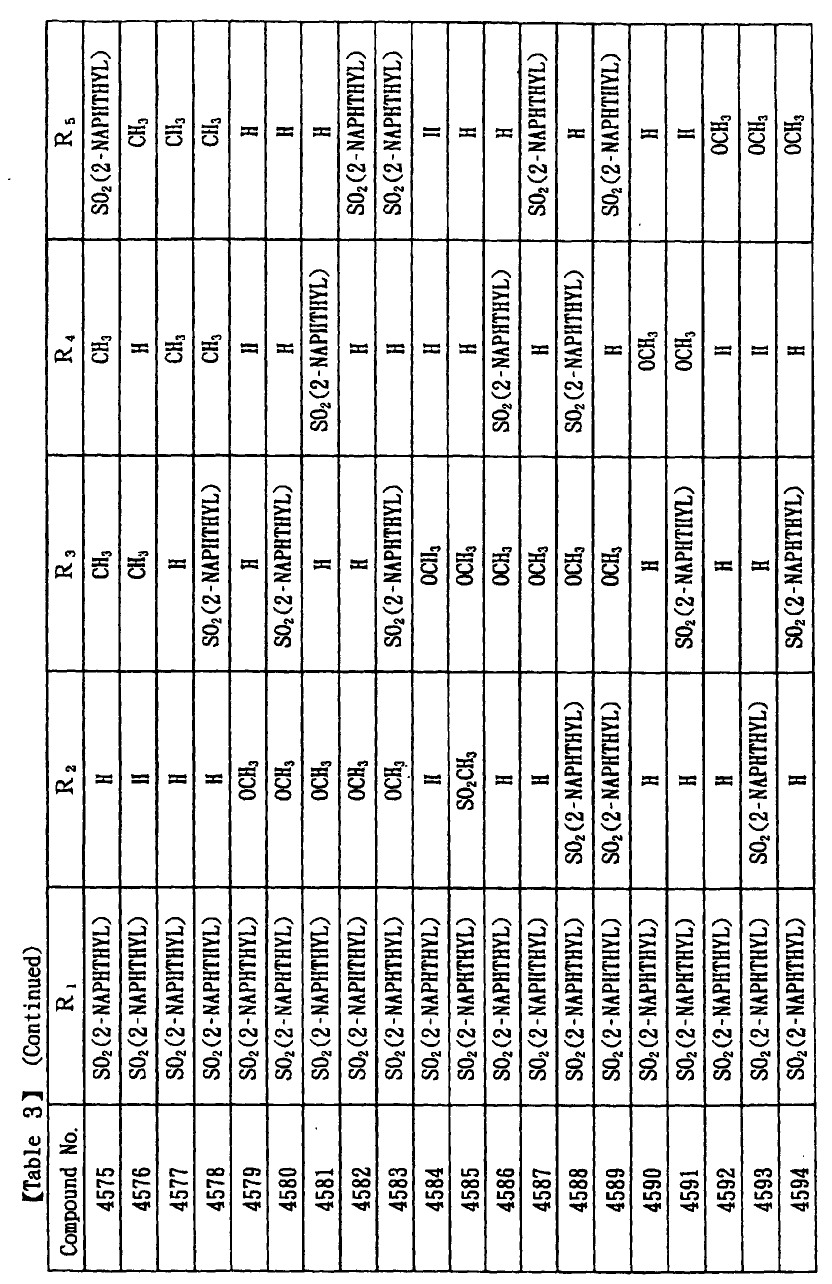

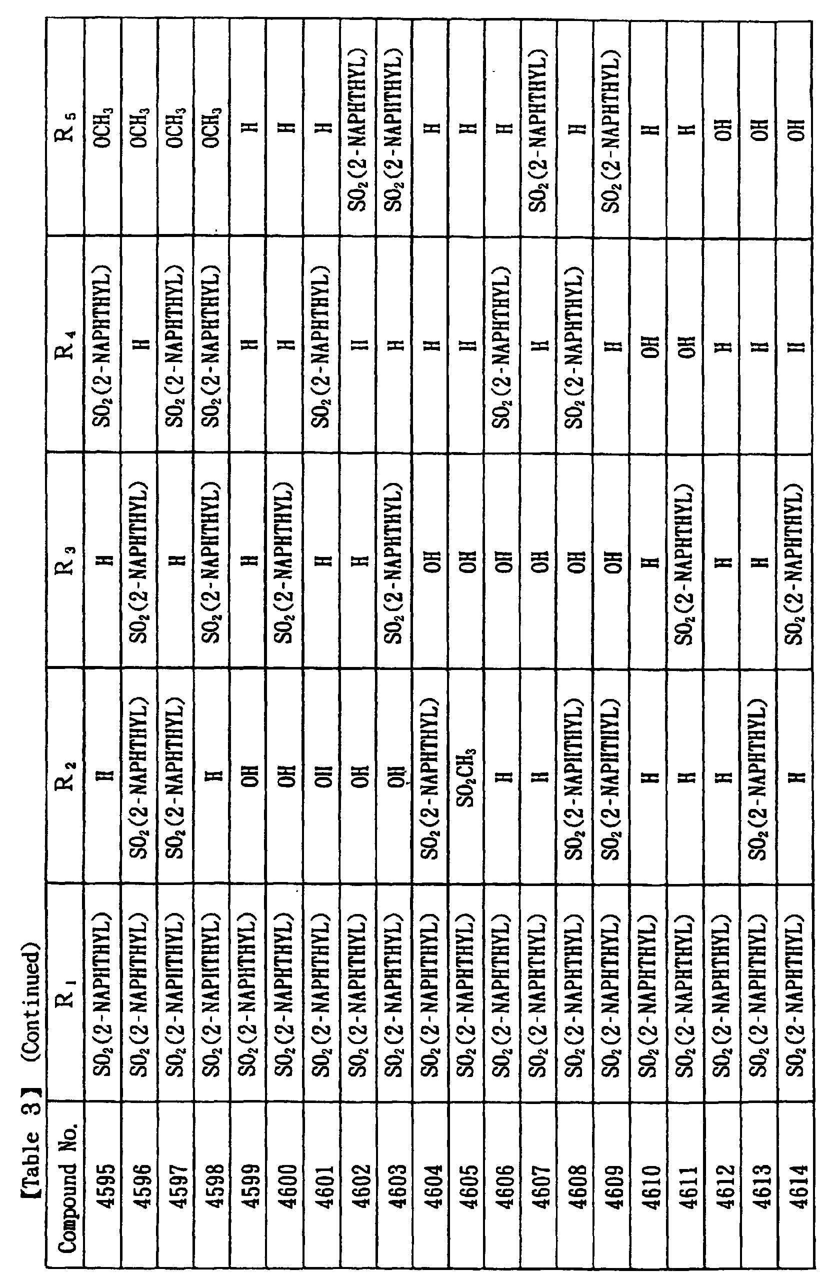

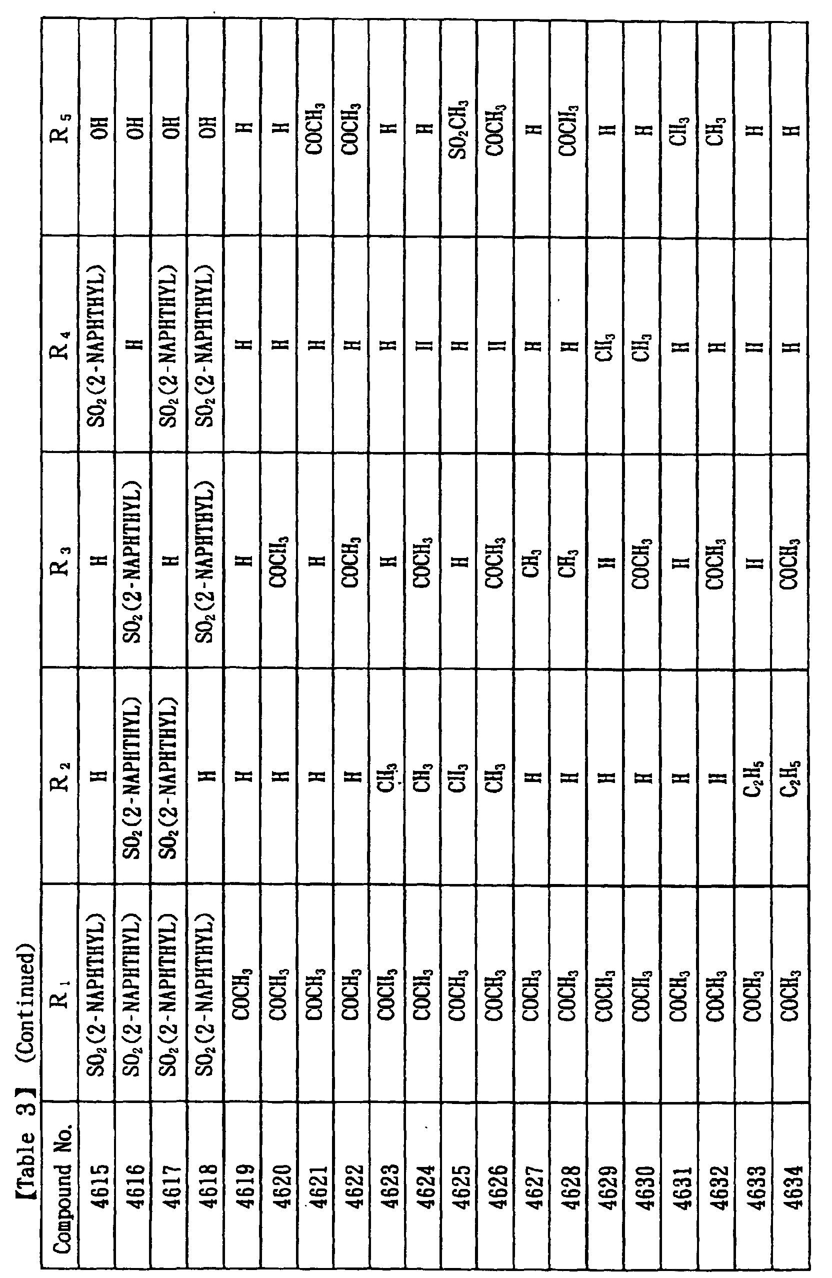

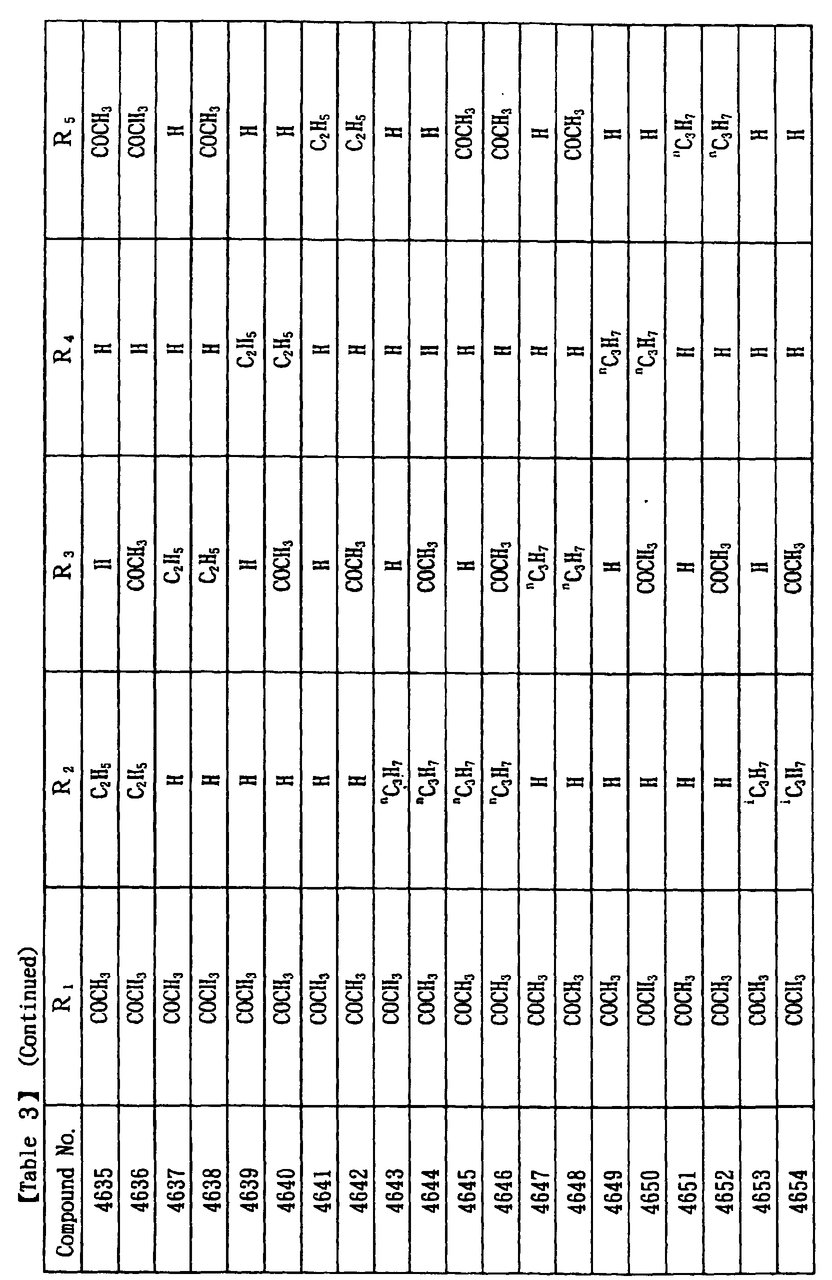

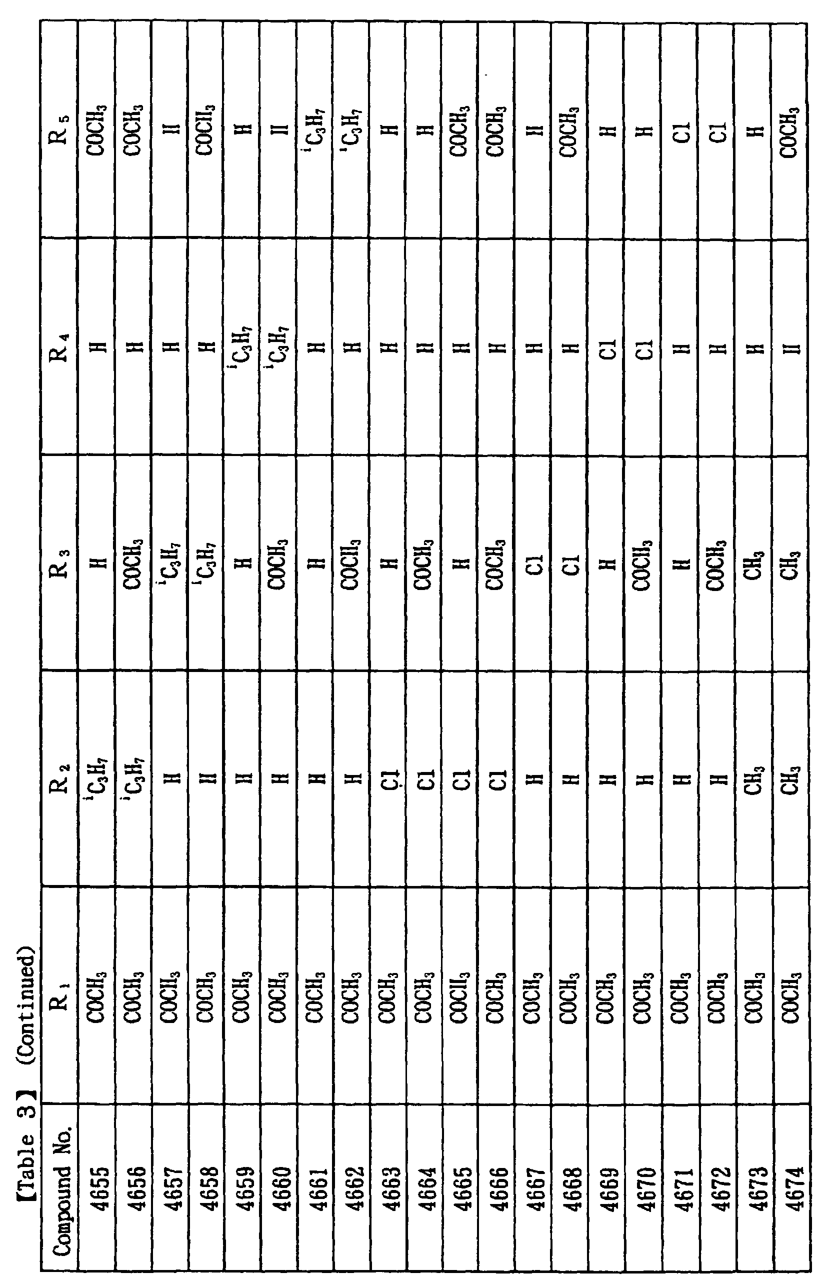

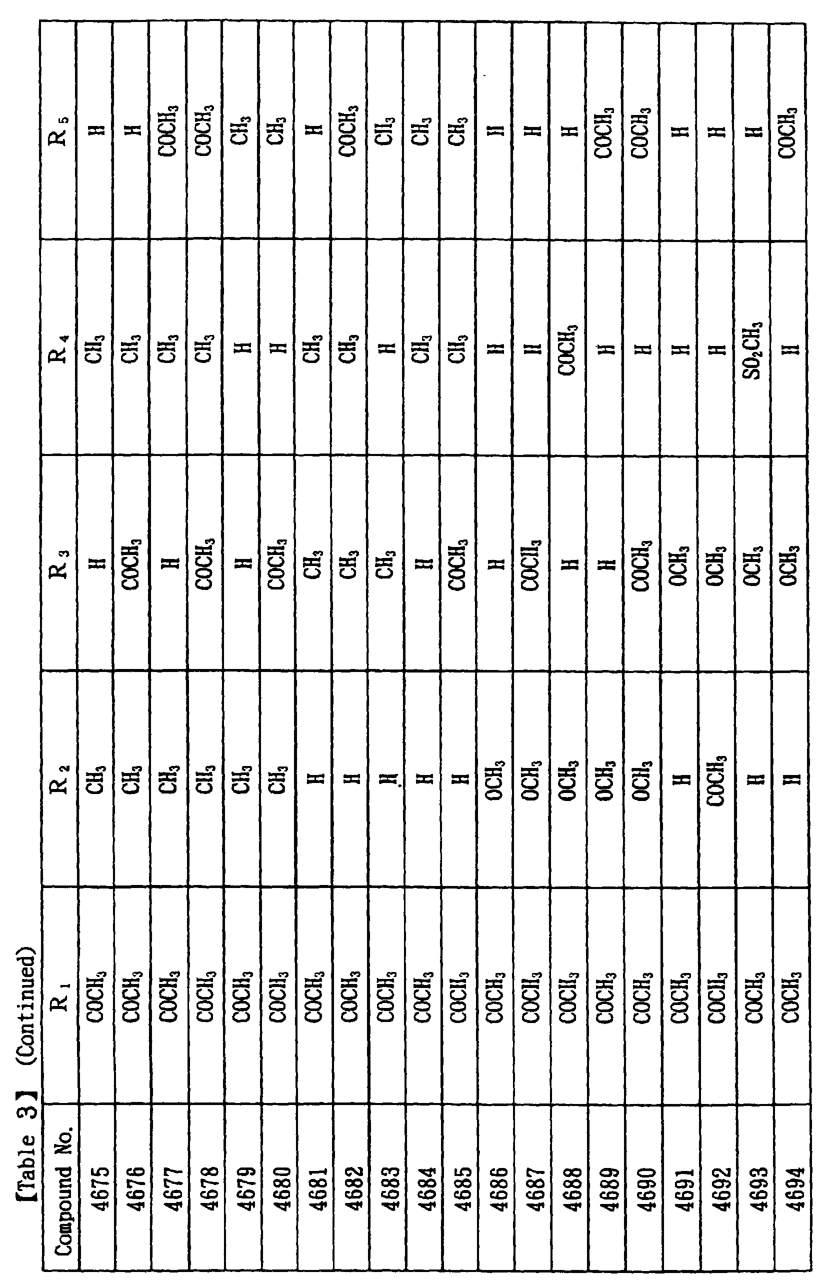

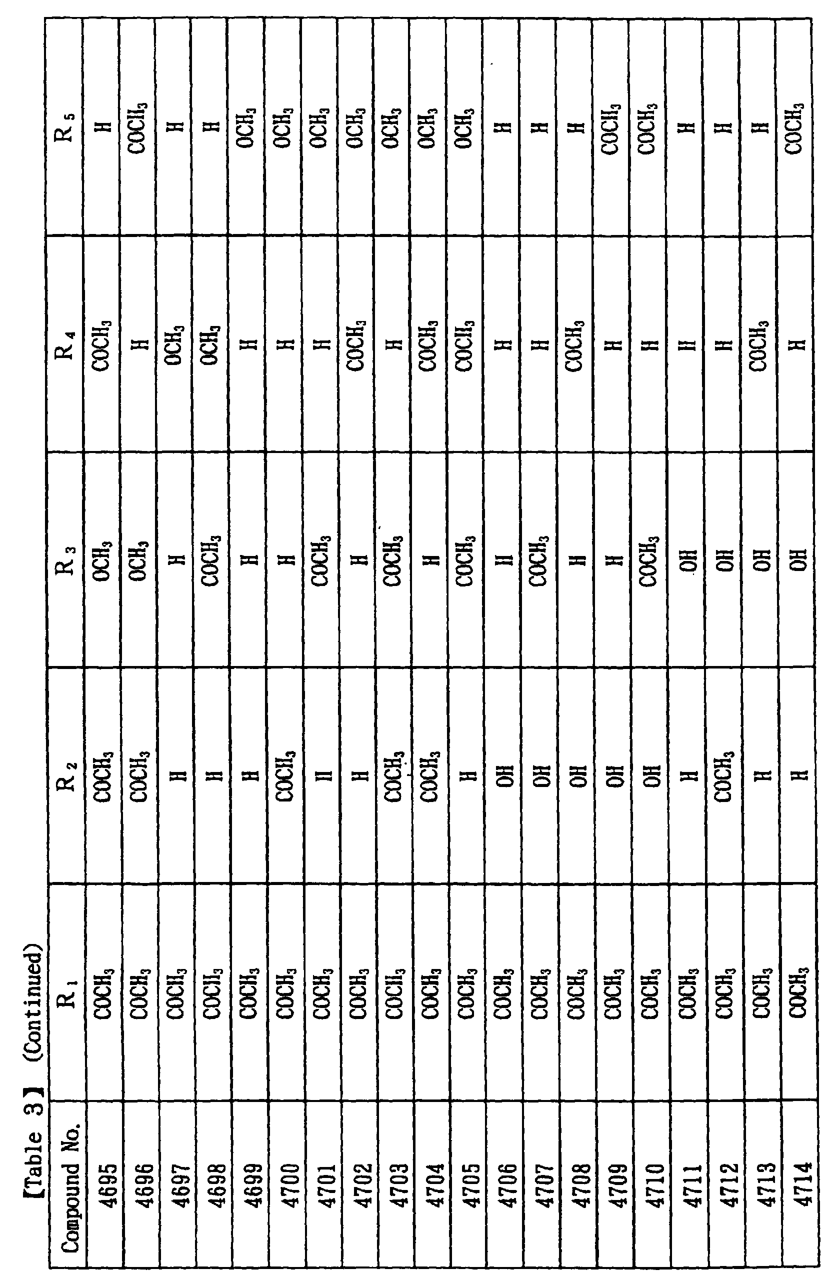

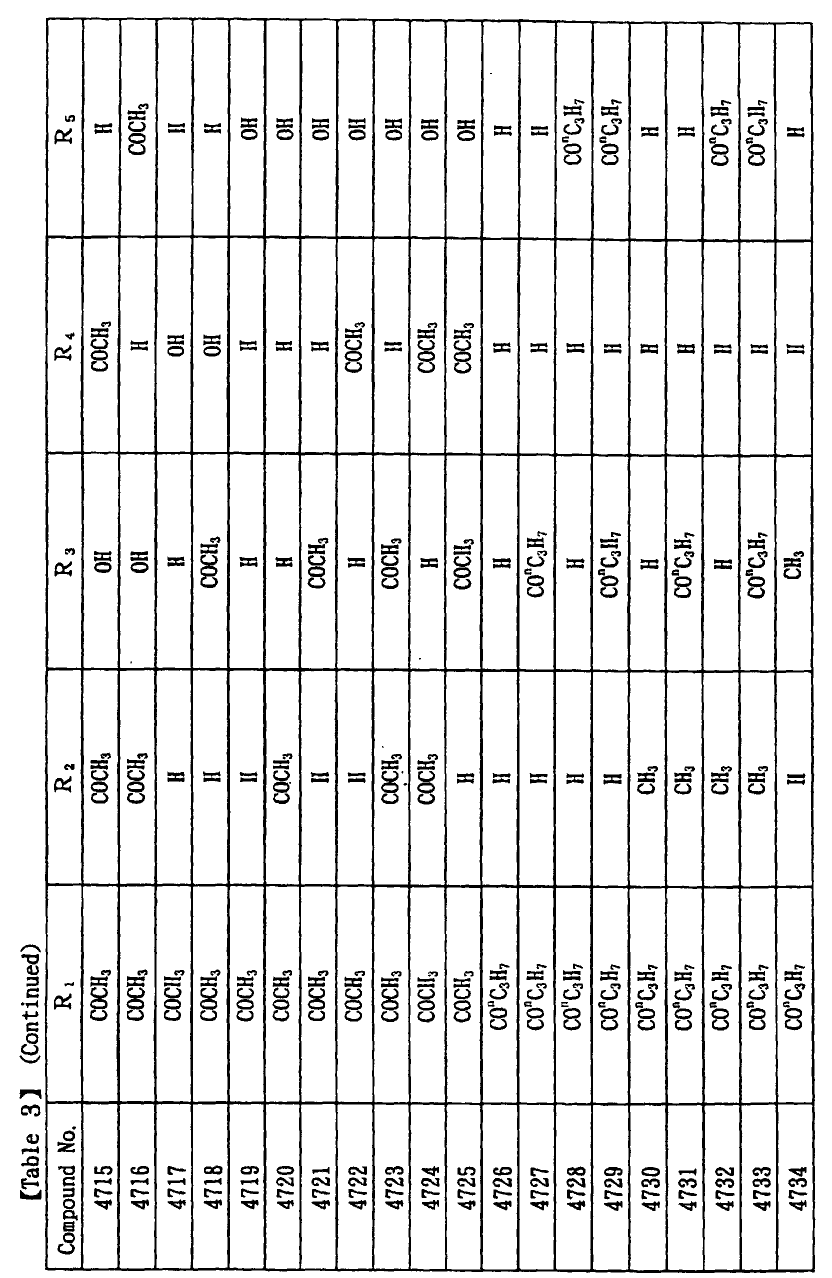

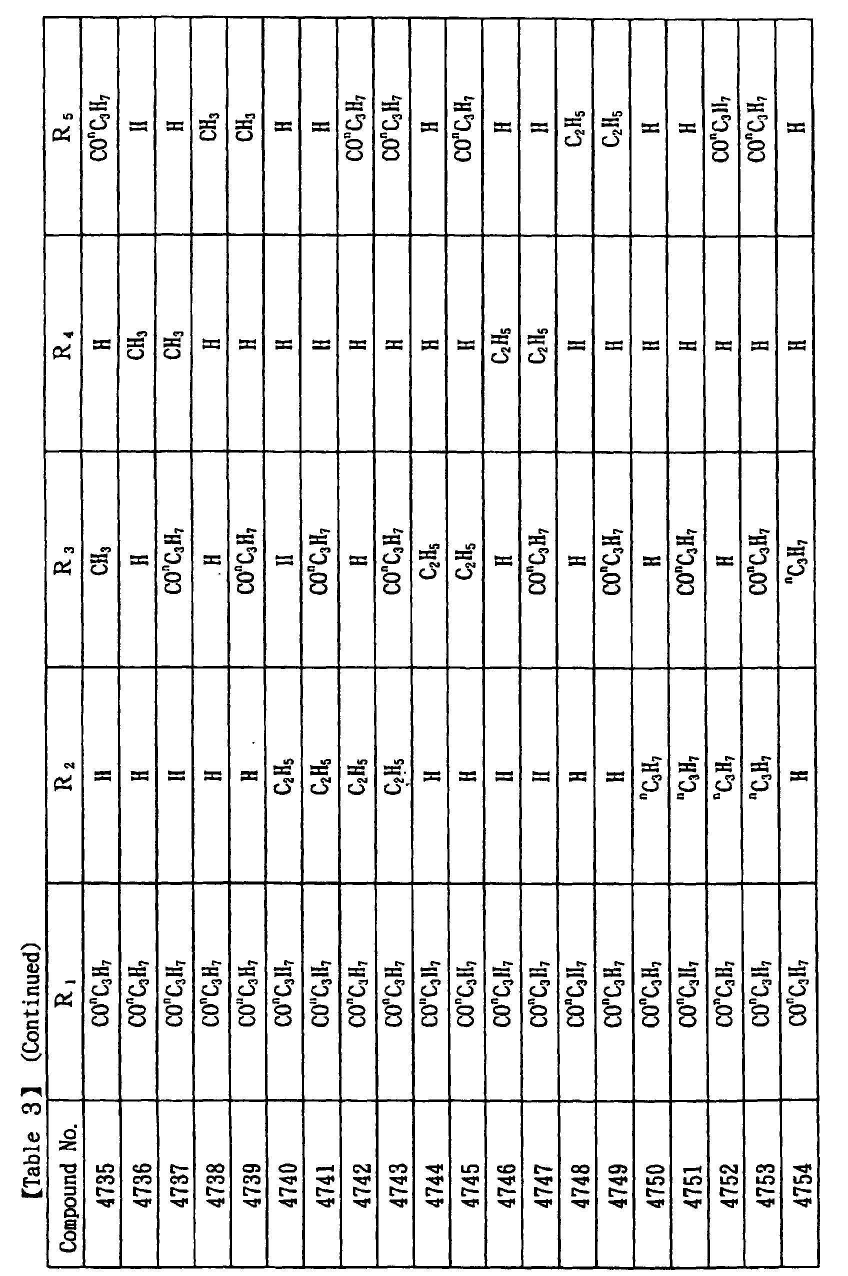

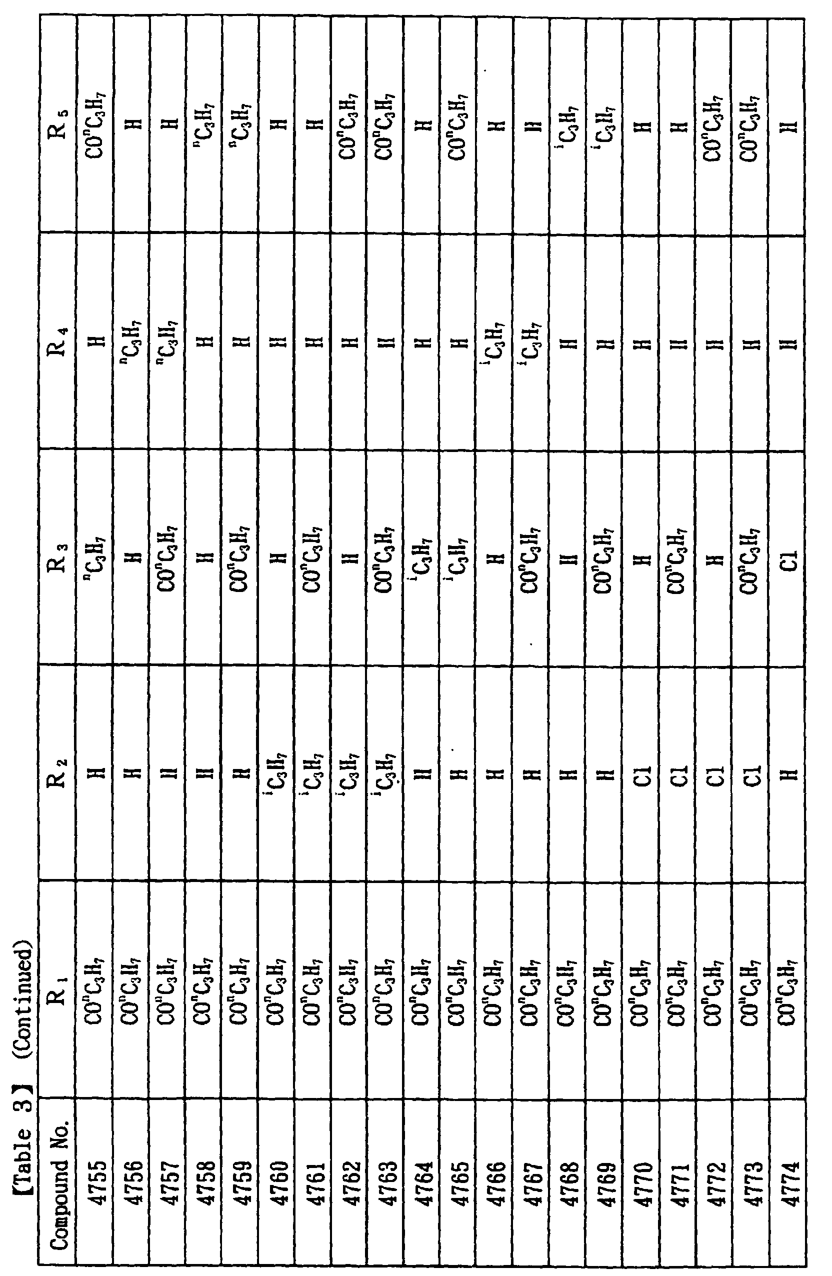

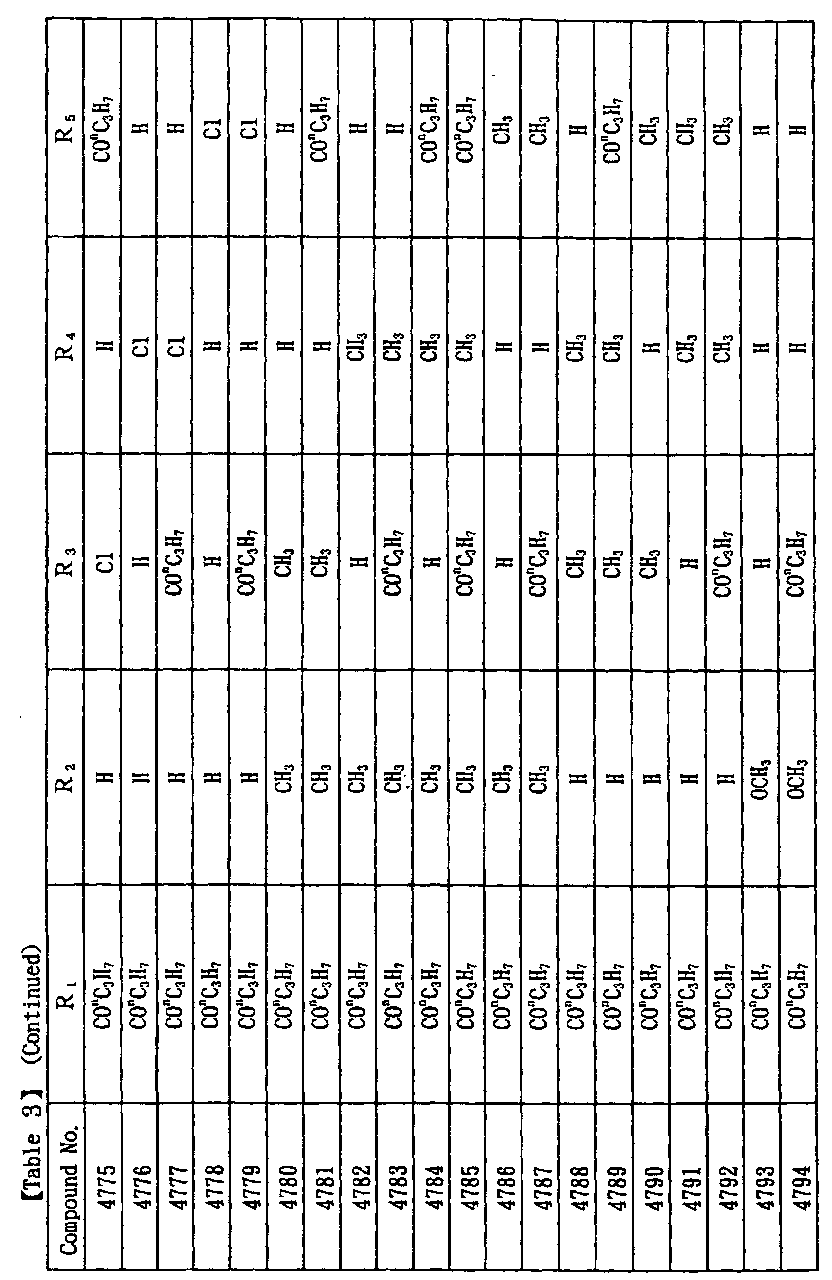

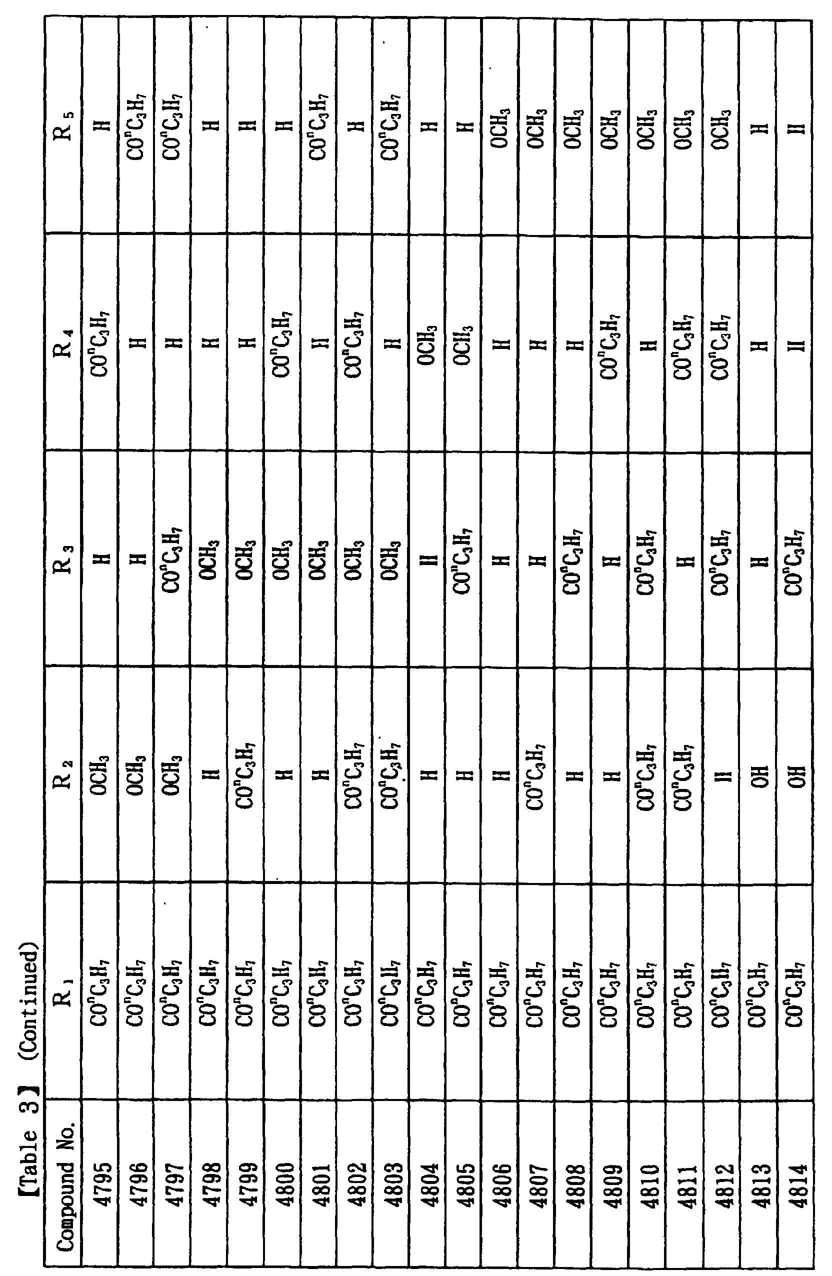

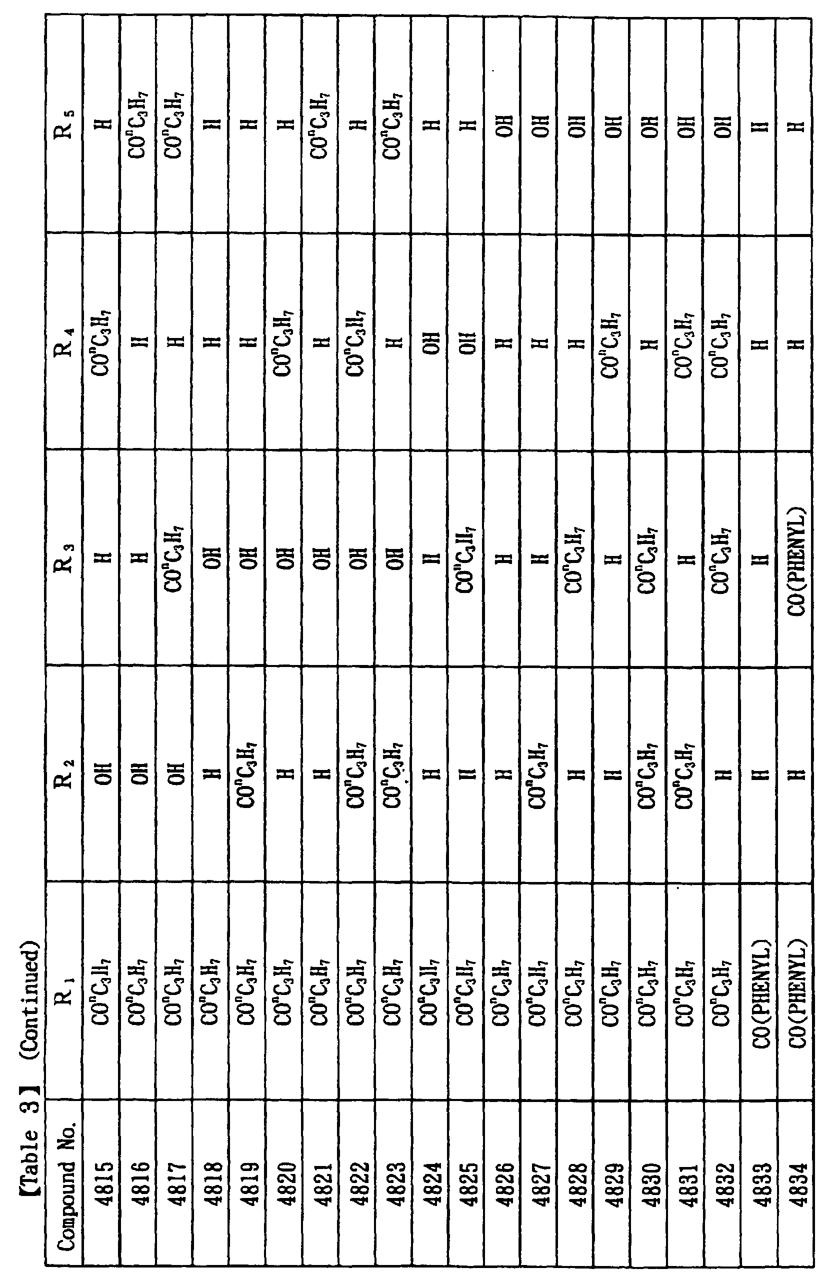

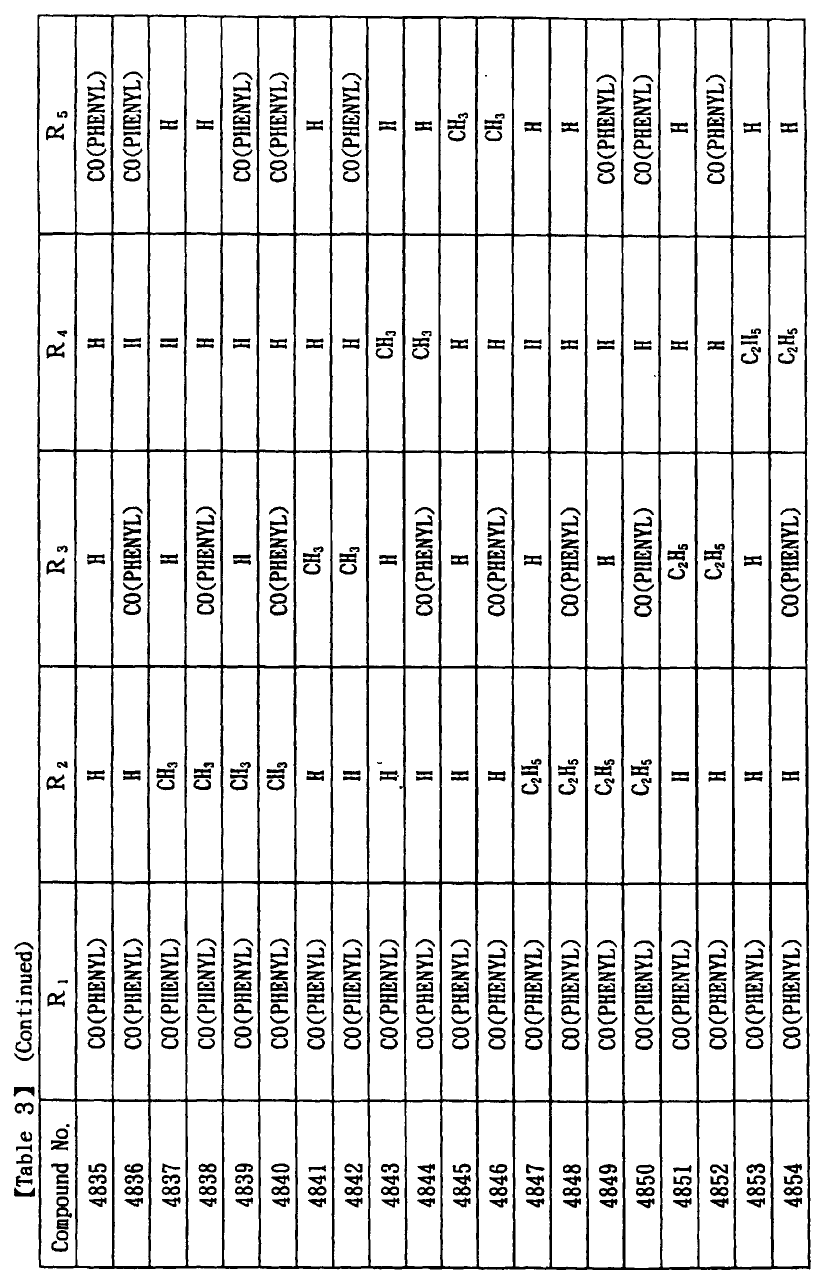

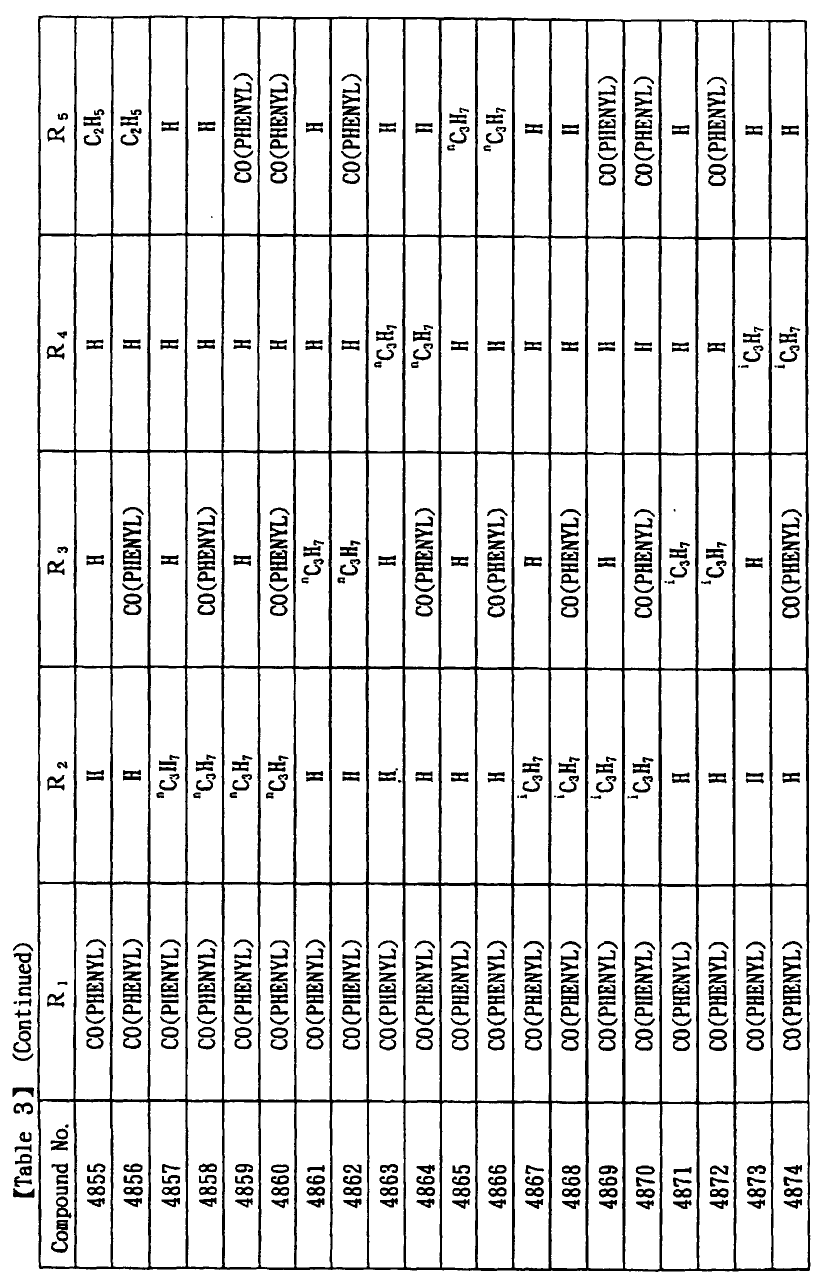

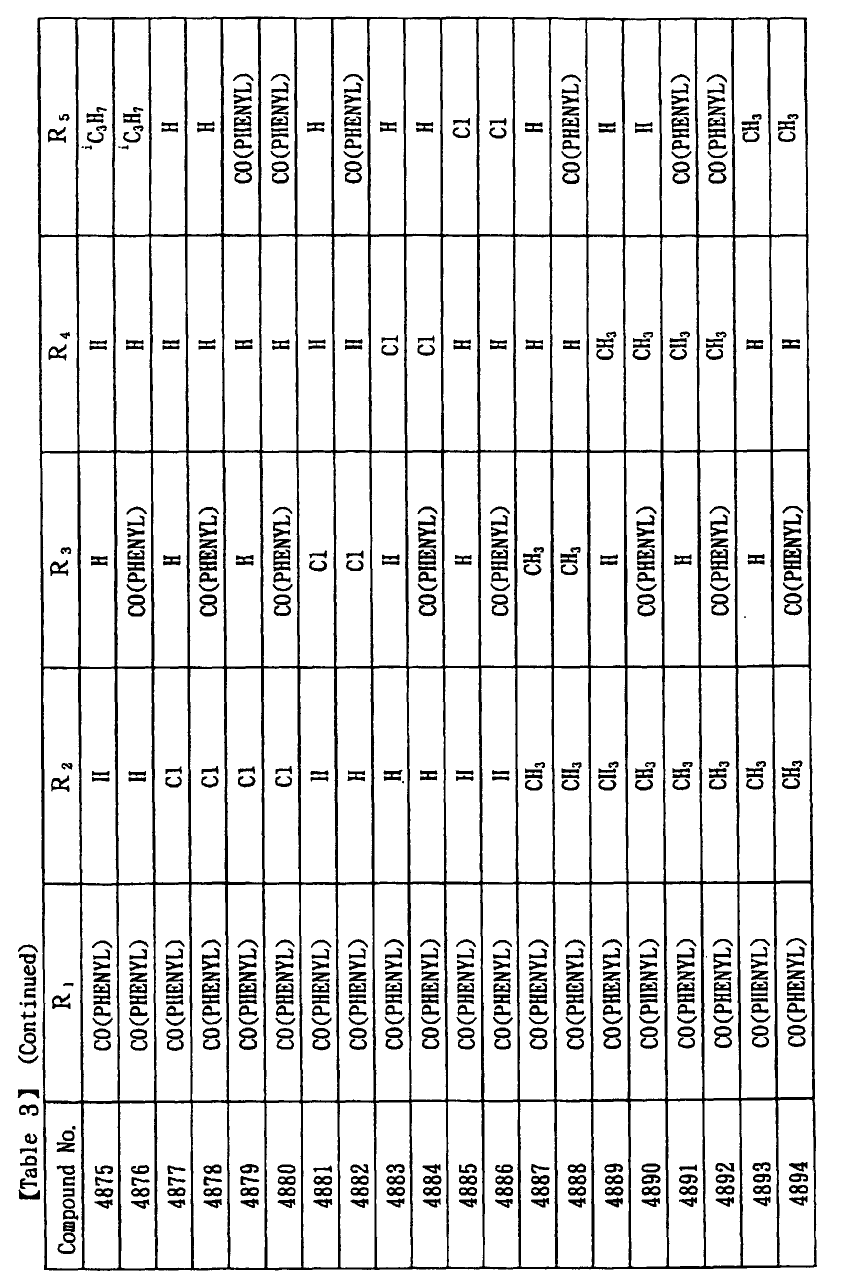

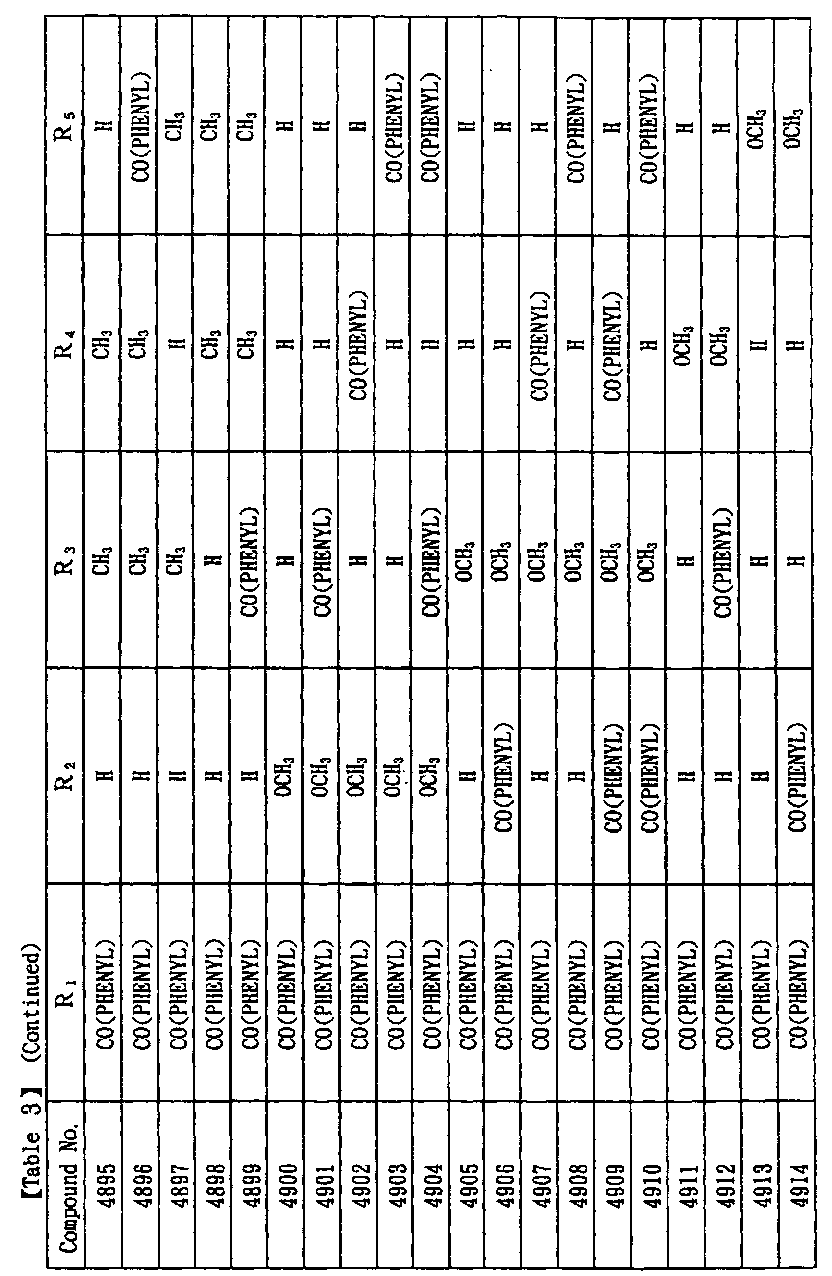

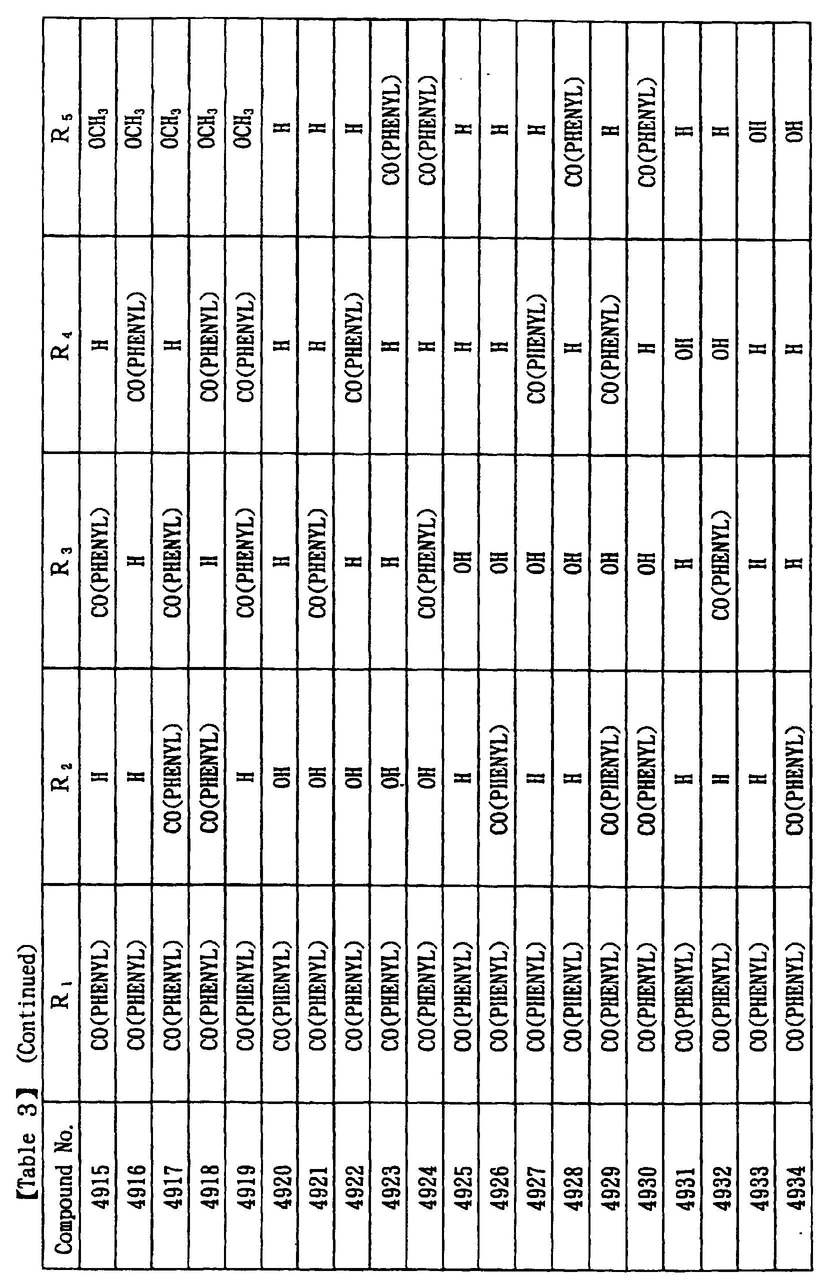

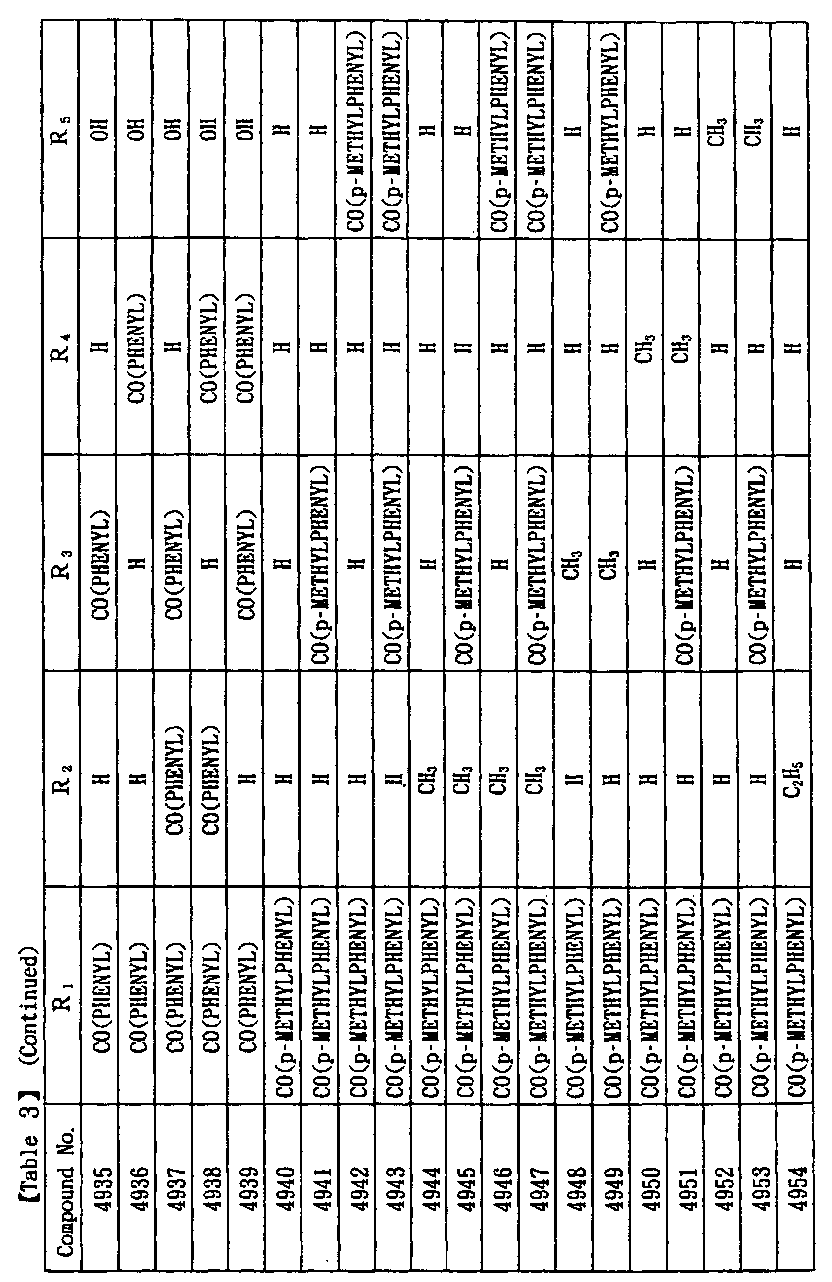

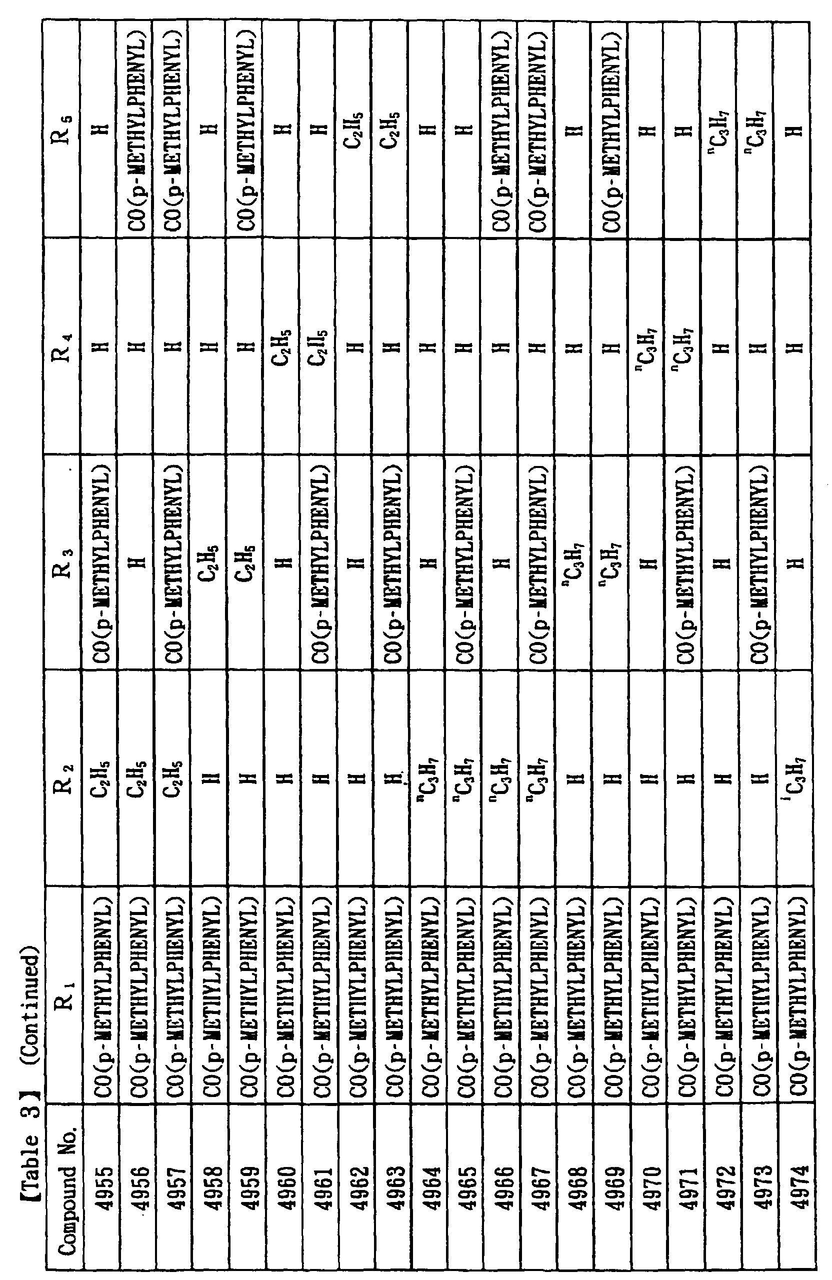

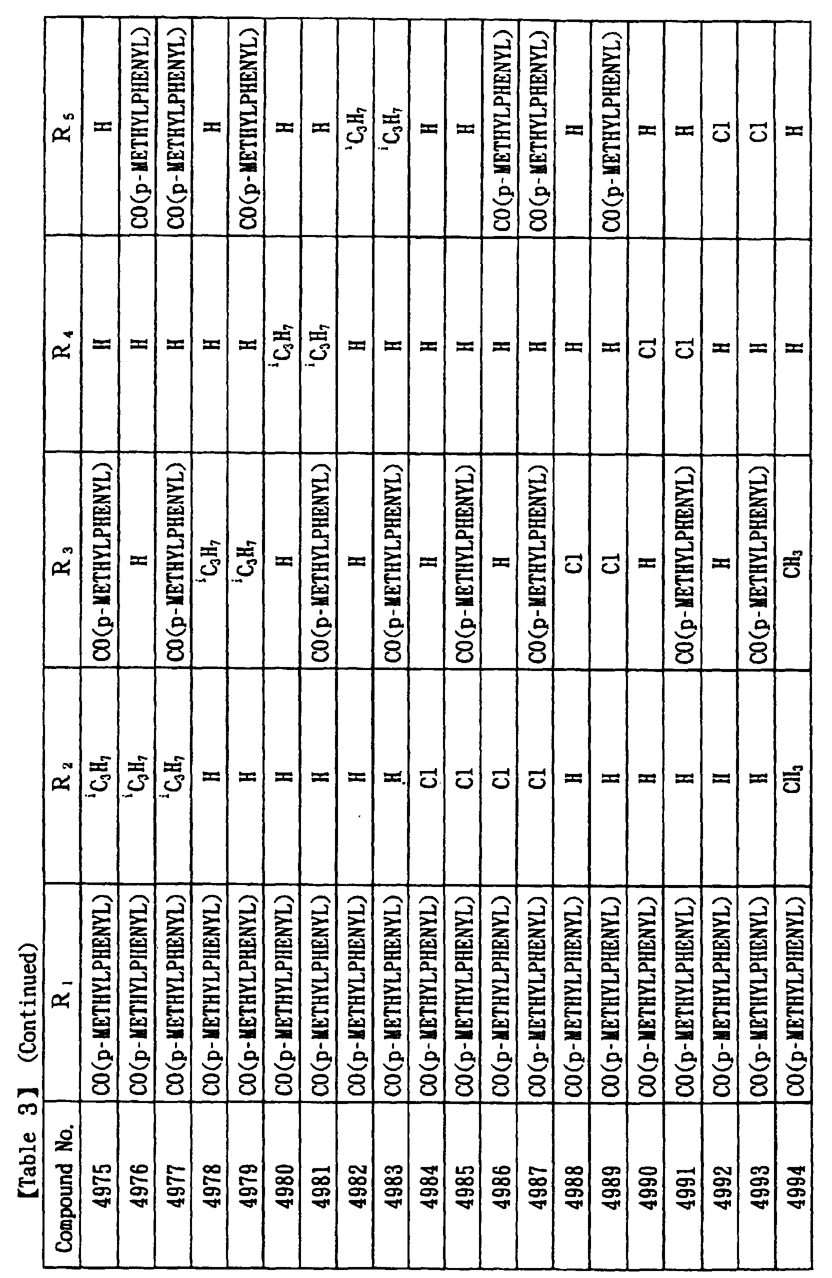

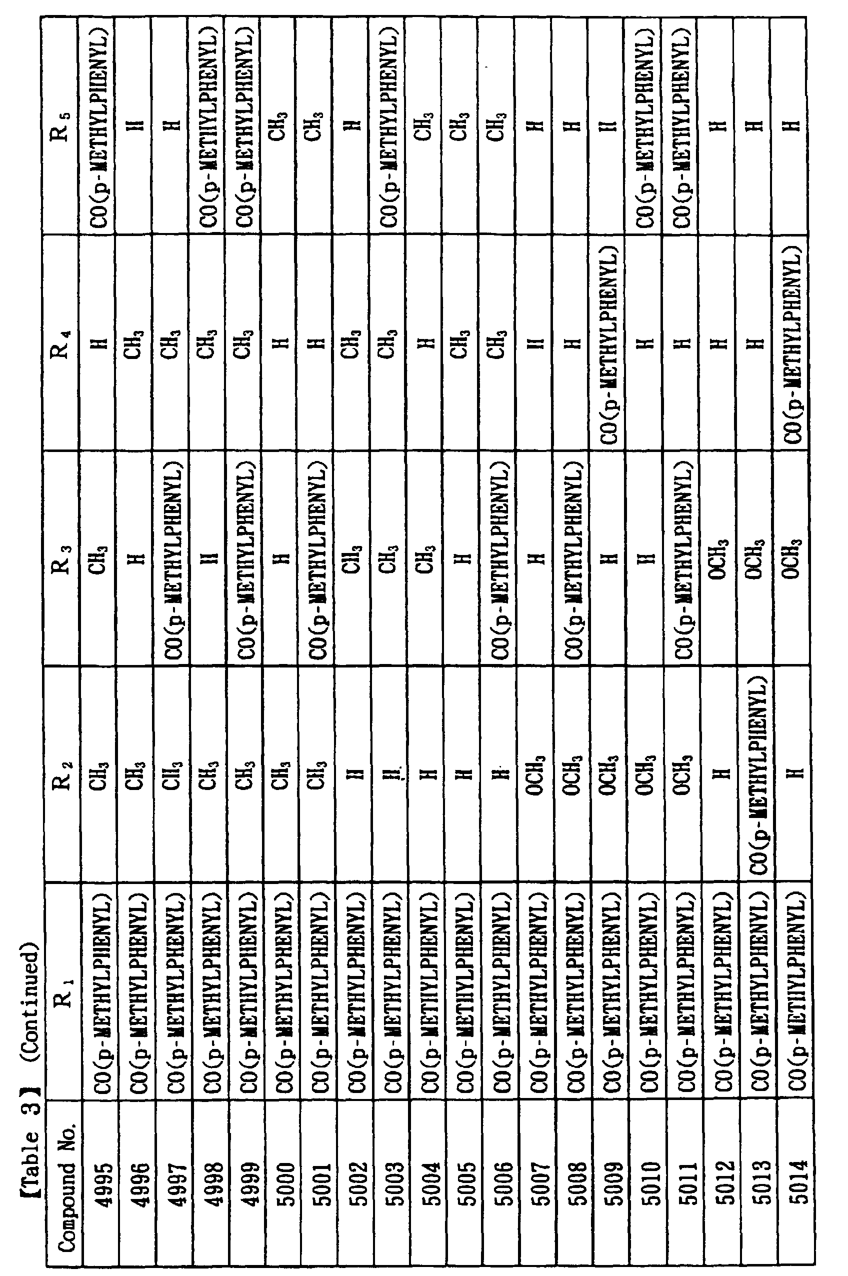

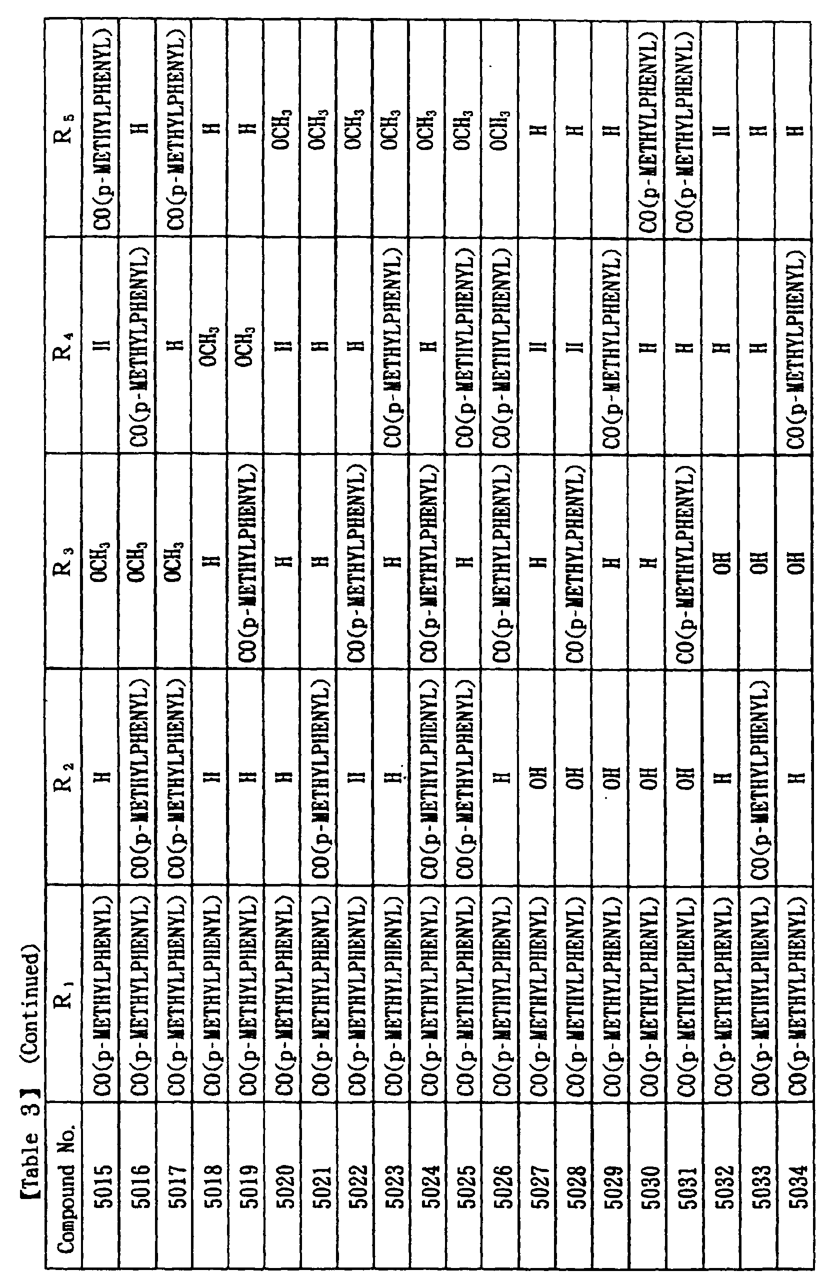

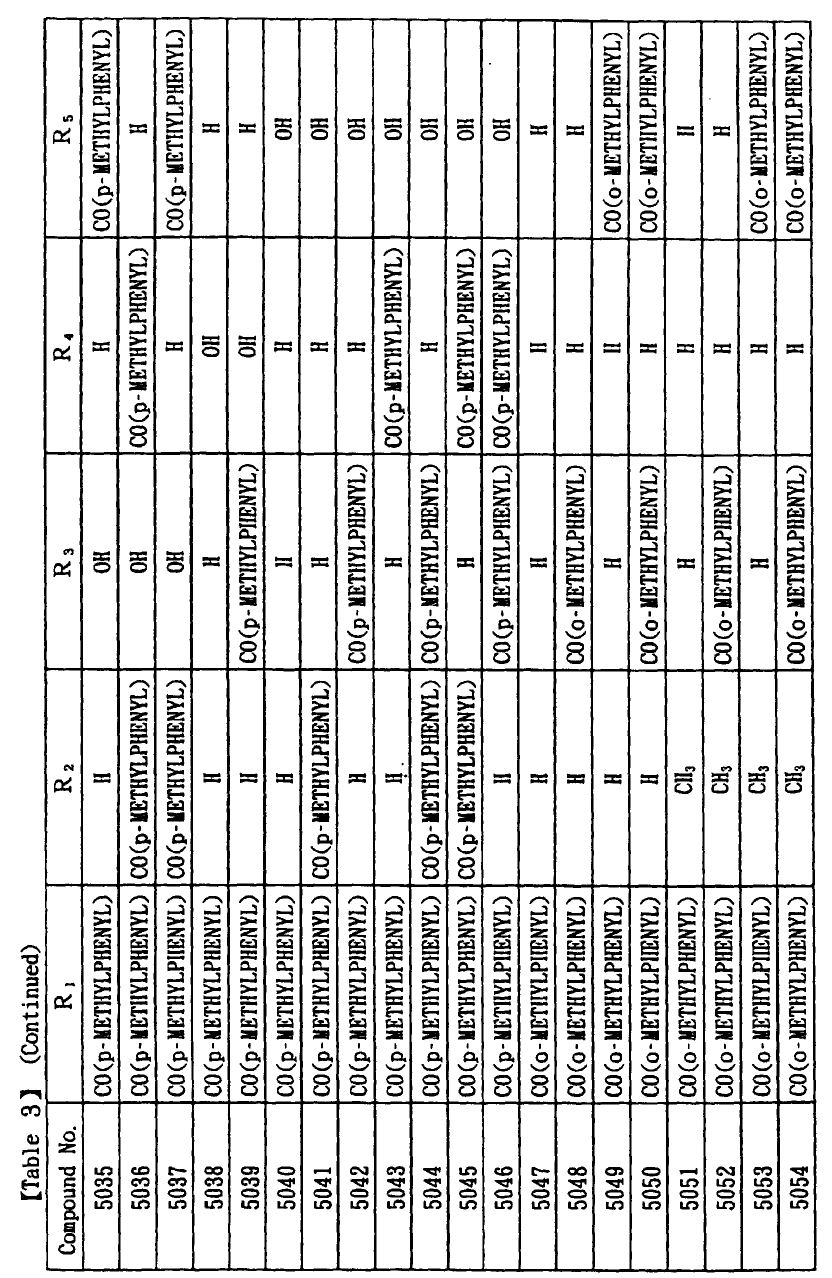

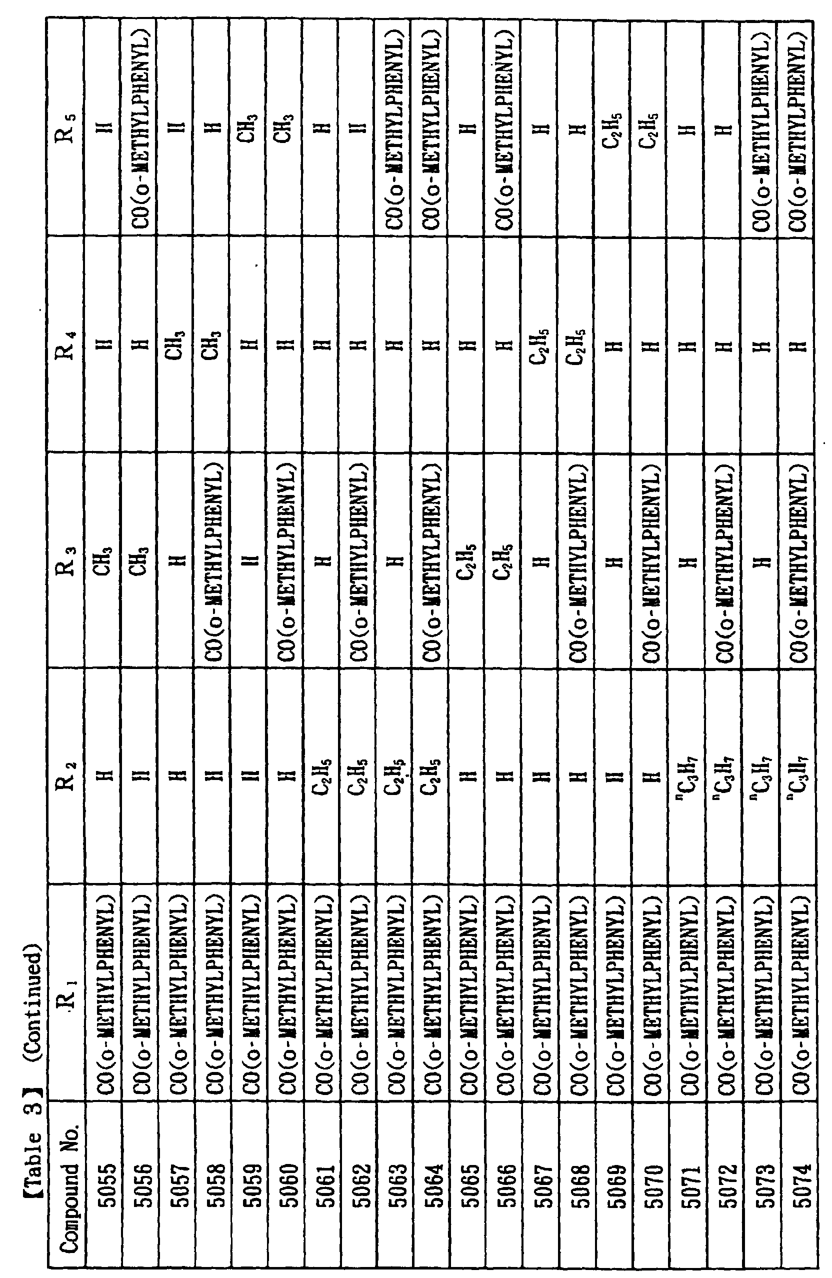

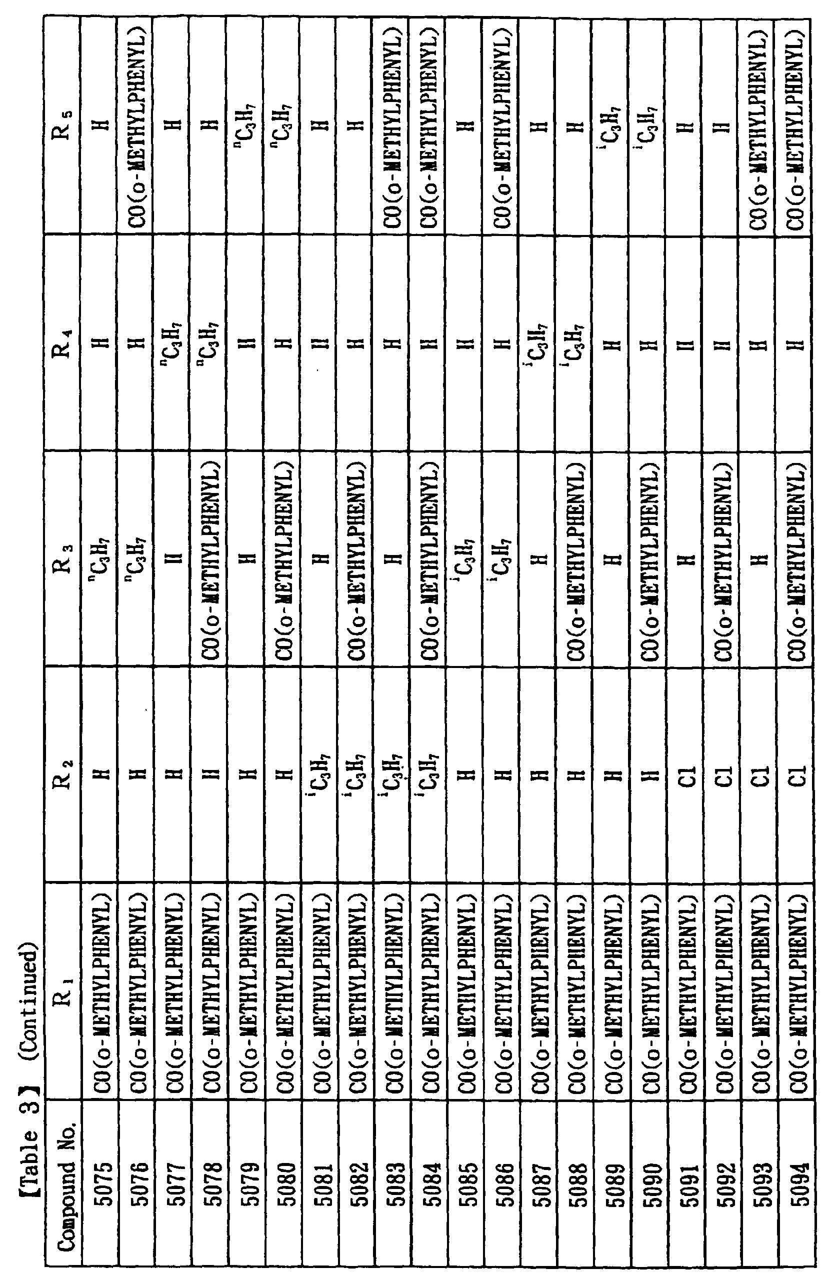

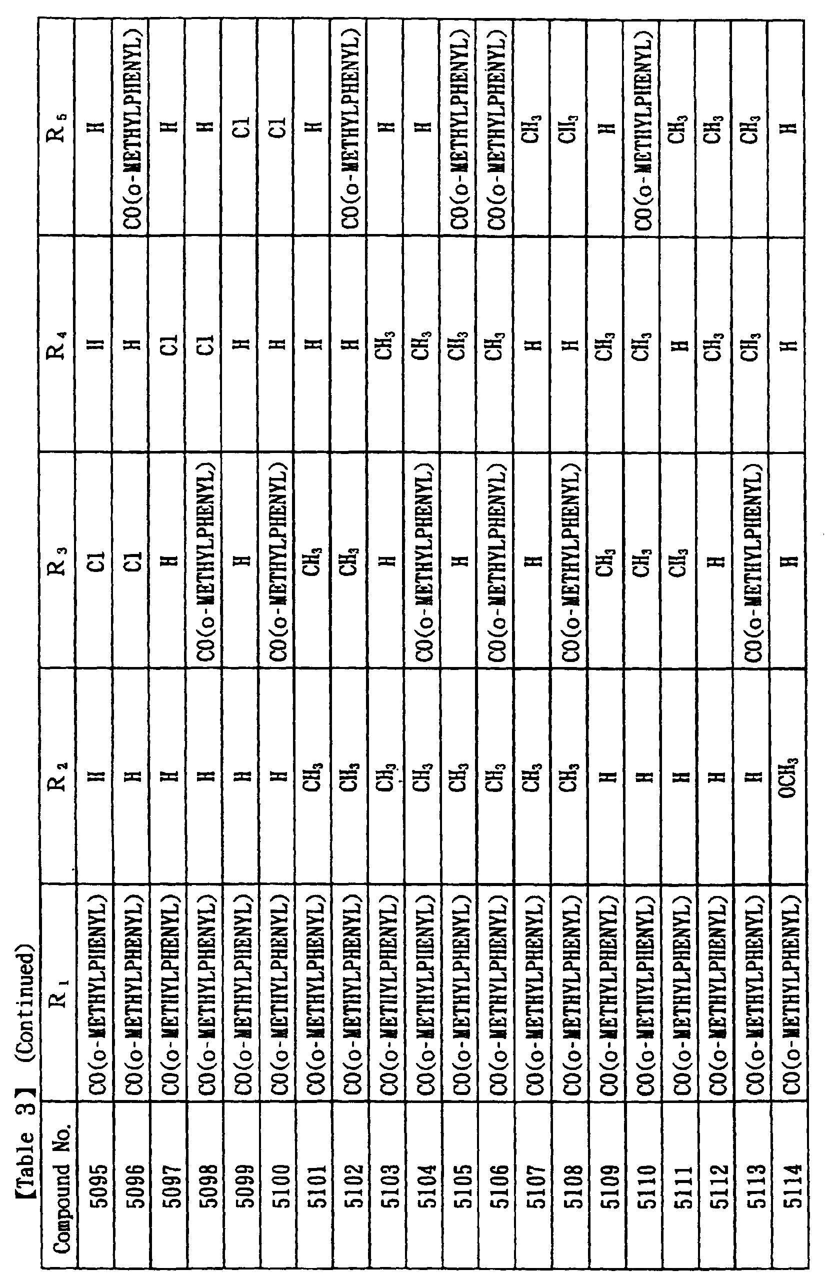

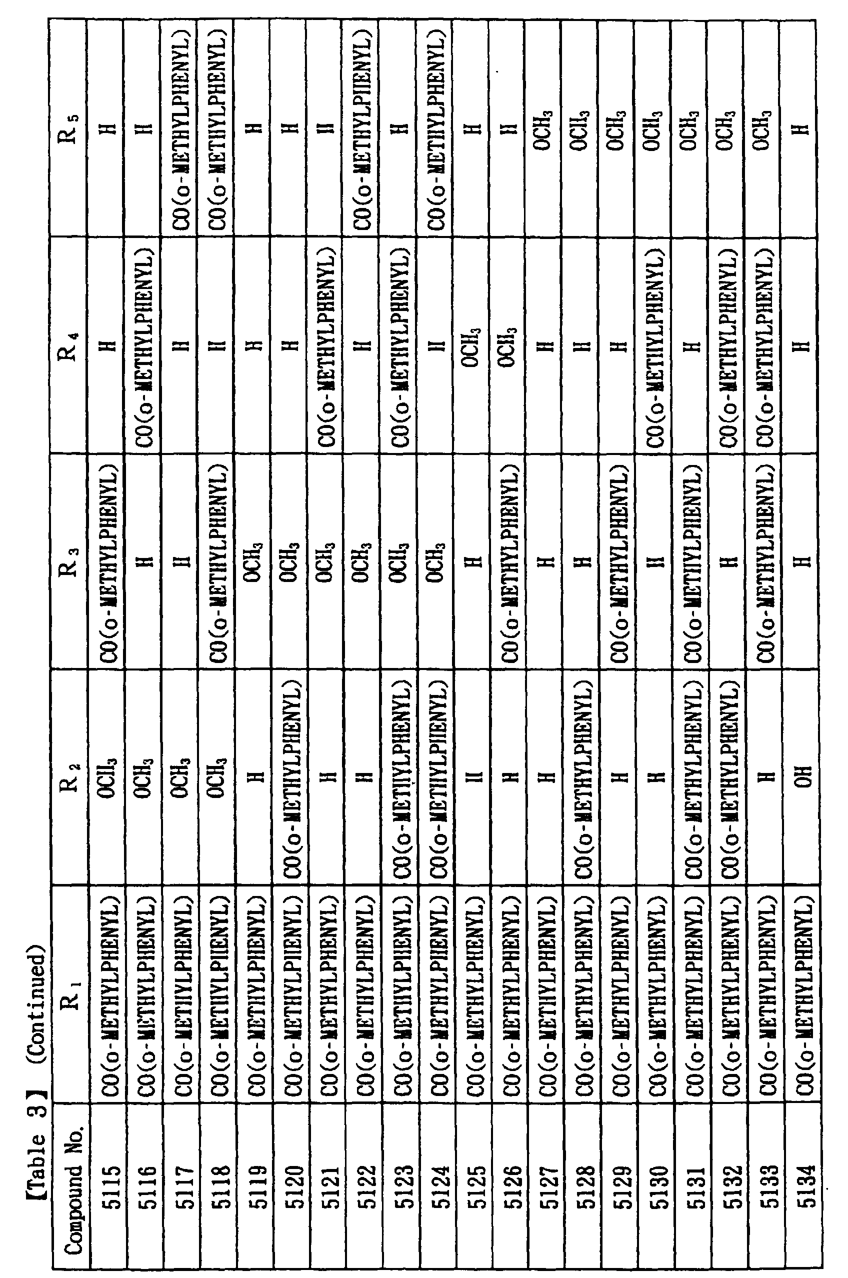

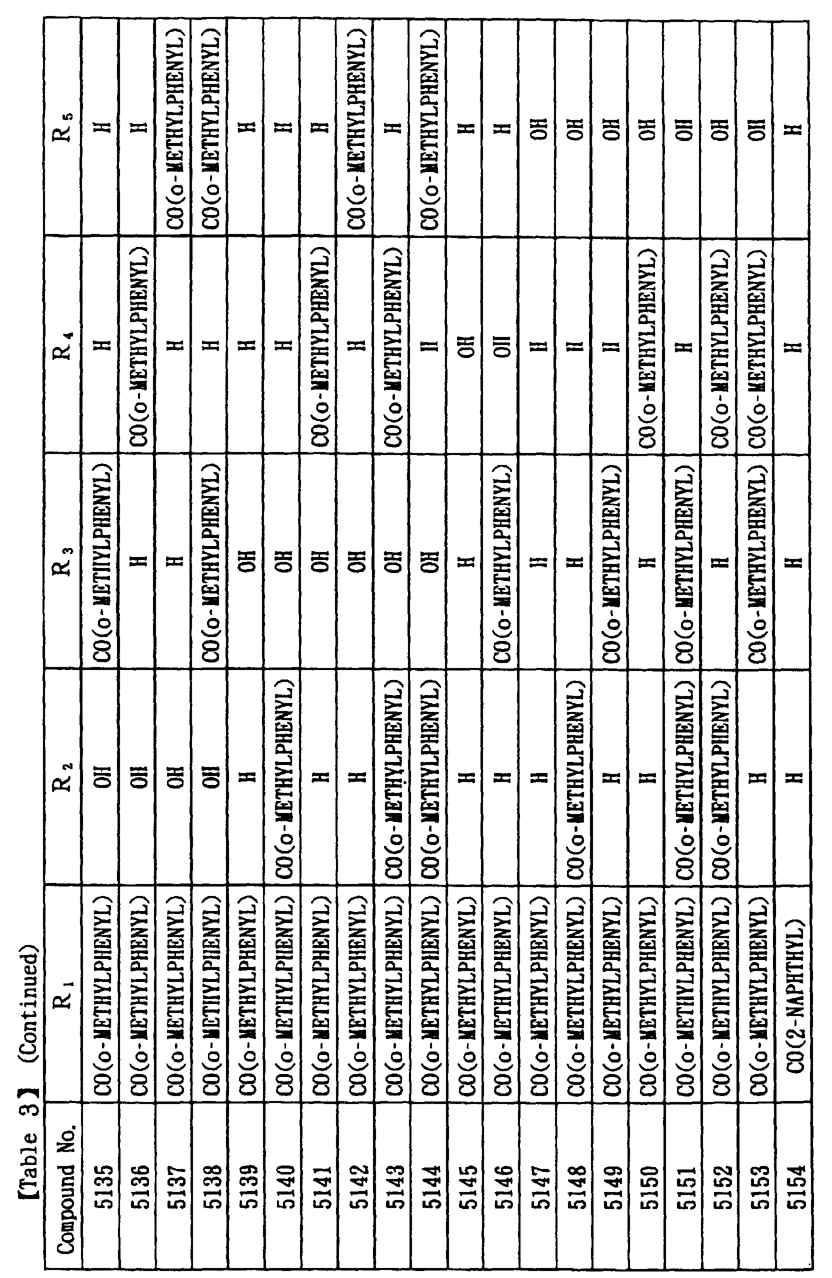

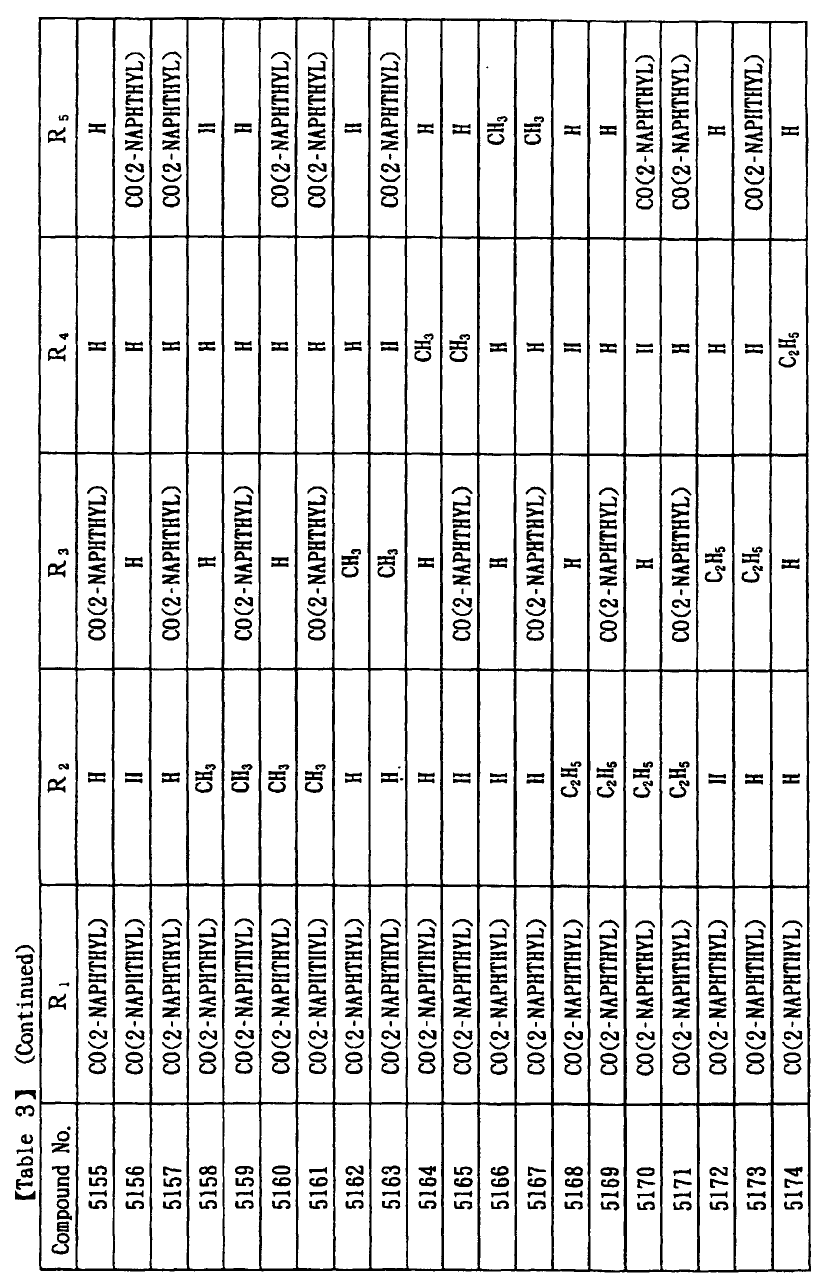

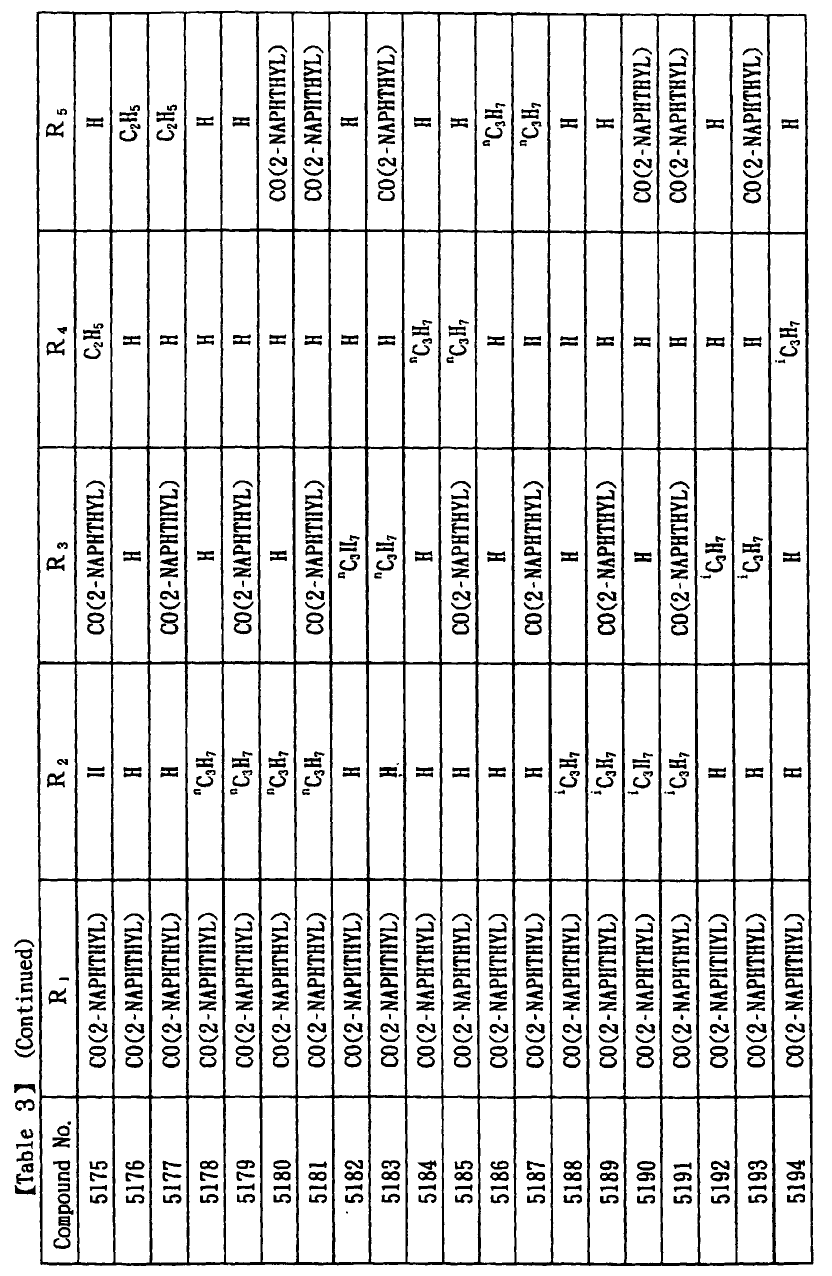

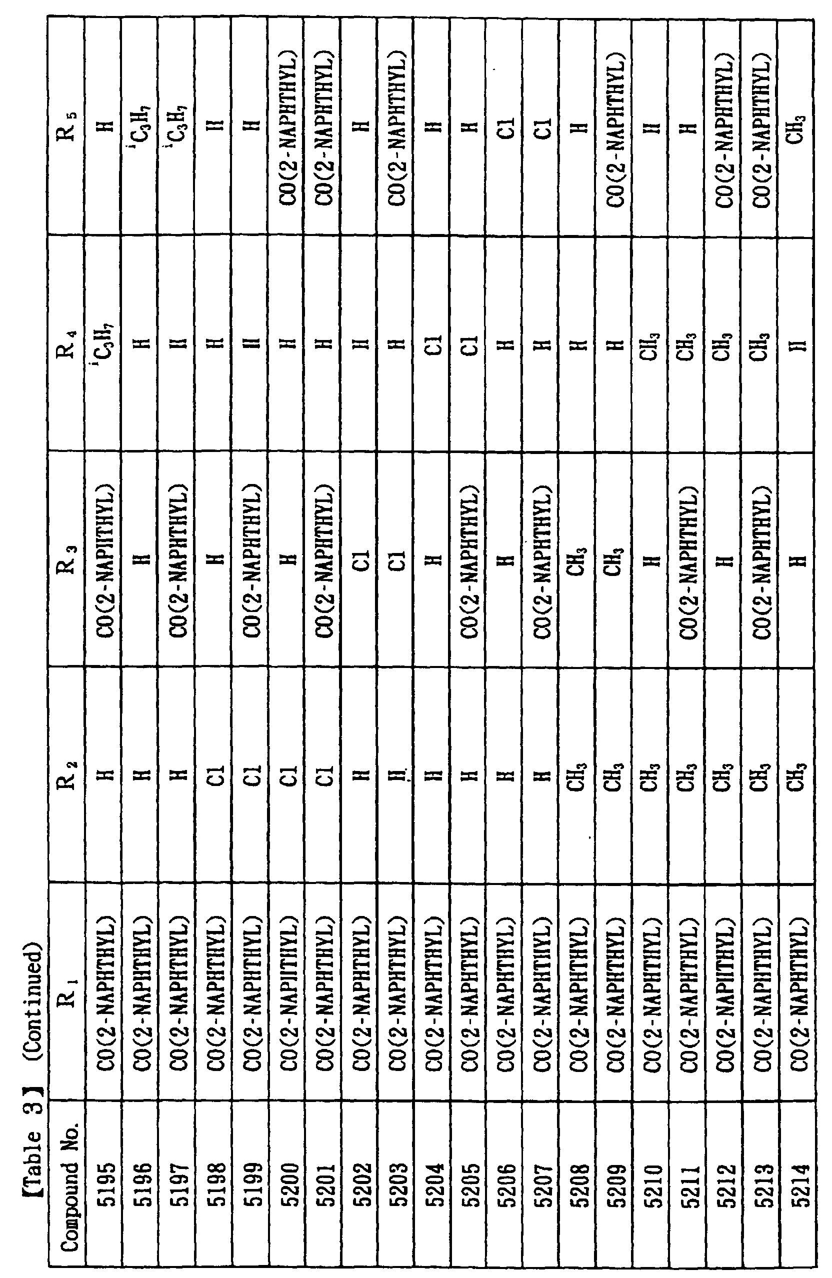

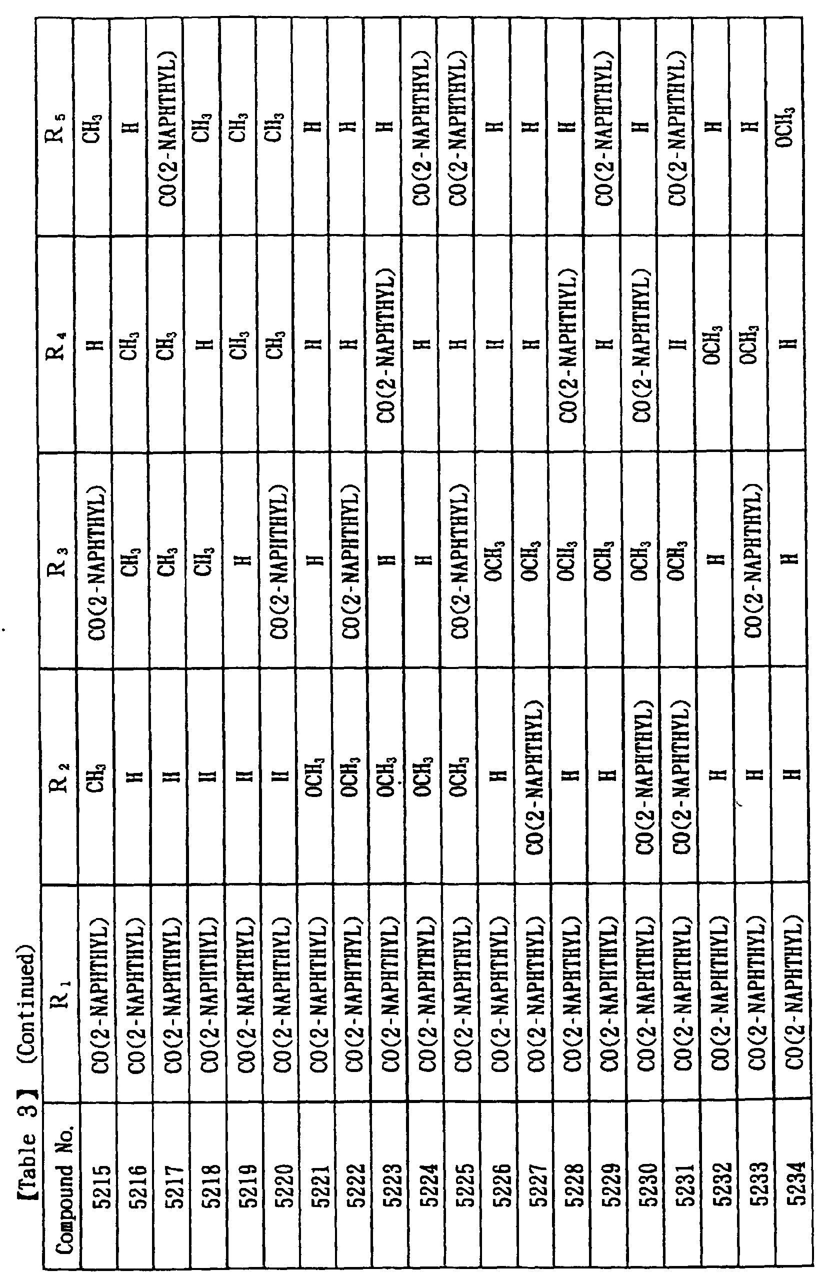

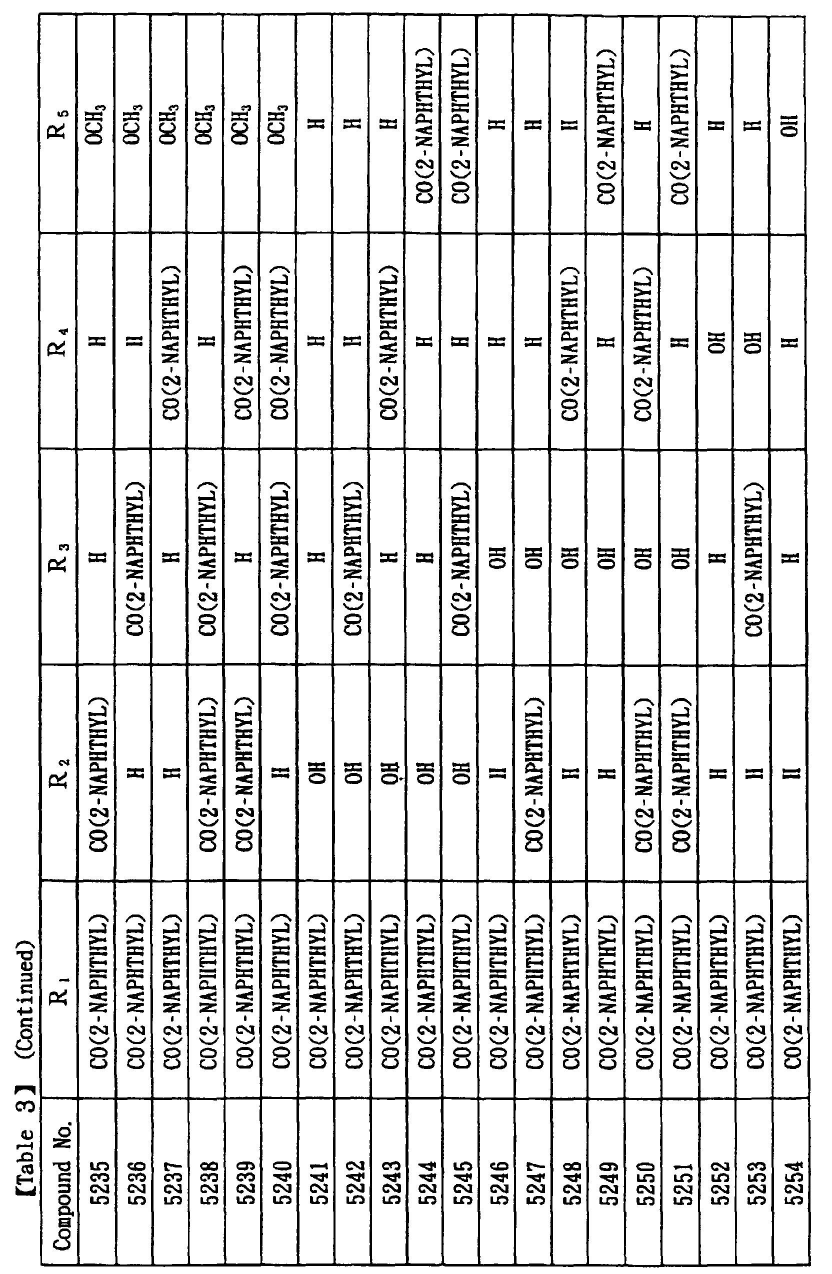

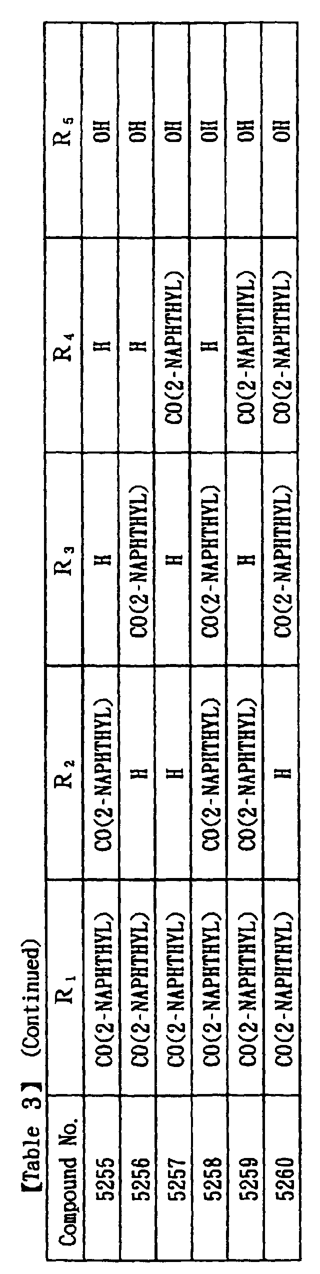

From the viewpoint of the ease of synthesis and performances in which a useful substance is selectively separated, chemically stabilized, rendered nonvolatile, gradually releasable, powdered or otherwise treated, of the phenol derivatives of Formula (I), particularly preferred compounds listed in Table 1 are 1 to 64, 77 to 86, 123 to 138, 209 to 230, 295 to 310, 381 to 396, 467 to 482, 553 to 568, 639 to 654, 695 to 740, 811 to 826, 861 to 924, 937 to 942, 983 to 998, 1069 to 1090, 1155 to 1170, 1241 to 1256, 1327 to 1342, 1413 to 1428, 1499 to 1514, 1585 to 1600, 1631 to 1634 and 1671 to 1686. More preferred compounds in Table 1 are 37 to 48, 77 to 82, 123 to 134, 209 to 220, 295 to 306, 381 to 392, 467 to 478, 553 to 564, 639 to 650, 695 to 736, 811 to 822, 897 to 908, 983 to 994, 1069 to 1080, 1155 to 1166, 1241 to 1252, 1327 to 1338, 1413 to 1424, 1499 to 1510, 1585 to 1596 and 1671 to 1682. Of them, particularly preferred are 37 to 39, 41 to 43, 45 to 47, 209 to 211, 213 to 215, 295 to 297, 299 to 301, 381 to 383, 385 to 387, 389 to 391, 467 to 469, 471 to 473, 553 to 555, 557 to 559, 695 to 697, 699 to 701, 811 to 813, 815 to 817, 897 to 899, 901 to 903, 905 to 907, 1069 to 1071, 1073 to 1075, 1155 to 1157, 1159 to 1161, 1241 to 1243, 1245 to 1247, 1327 to 1329, 1331 to 1333, 1413 to 1415, 1417 to 1419, 1585 to 1587, 1589 to 1591, 1671 to 1673 and 1675 to 1677.

Particularly preferred compounds listed in Table 2 are 1721 to 1790, 1836 to 1850, 1906 to 1920, 1976 to 1990, 2046 to 2060, 2116 to 2130, 2188 to 2200, 2256 to 2270, 2326 to 2345, 2396 to 2410, 2421 to 2490, 2536 to 2550, 2606 to 2620, 2676 to 2692, 2746 to 2760, 2816 to 2830, 2886 to 2900, 2956 to 2970, 3026 to 3040 and 3096 to 3110. More preferred compounds in Table 2 are 1766 to 1780, 1909, 1910, 1914, 1915, 1919, 1920, 1979, 1980, 1984, 1985, 2049, 2050, 2054, 2055, 2059, 2060, 2119, 2120, 2124, 2125, 2189, 2190, 2194, 2195, 2329, 2330, 2334, 2335, 2399, 2400, 2404, 2405, 2466 to 2480, 2609, 2610, 2614, 2615, 2679, 2680, 2684, 2685, 2749, 2750, 2754, 2755, 2819, 2820, 2824, 2825, 2889, 2890, 2894, 2895, 3029, 3030, 3034, 3035, 3099, 3100, 3104 and 3105.

Particularly preferred compounds listed in Table 3 are 3870 to 4297, 4404 to 4618 and 4833 to 5260. Of them, particularly preferred compounds in Table 3 are 3870 to 4190 and 4512 to 4618. More preferred are 3870 to 3883, 3977 to 3990, 4084 to 4097 and 4084 to 4097.

The phenol derivatives of Formula (I) can be produced by the Friedel-Crafts reaction of a compound such as dihydroxydiphenyl sulfone derivatives, dihydroxydiphenyl ether derivatives, dihydroxydiphenyl thioether derivatives, dihydroxydiphenyl ketone derivatives, 2,2-bis(hydroxyphenyl)propane derivatives or substituted phenols, with alkylsulfonyl chloride, alkenylsulfonyl chloride, phenylsulfonyl chloride, alkylcarbonyl chloride, alkenylcarbonyl chloride, phenylcarbonyl chloride or the like, in the presence of a Lewis acid, such as iron chloride, aluminum chloride and zinc chloride.

The phenol derivatives of this invention are usually crystalline solids but may be amorphous or oily. They may be polymorphic. Regardless of the forms, all of the phenol derivatives of Formula (I) are covered by the present invention.

In the present invention, substances that form molecular compounds with the phenol derivatives of Formula (I) are any substances and are not particularly restricted if they can form molecular compounds with the derivatives. Their examples include water; alcohols such as methanol, ethanol, isopropanol, n-butanol, n-octanol, 2-ethylhexanol, allyl alcohol, propargyl alcohol, 1,2-butanediol, 1,3-butanediol, 1,4-butanediol, cyclohexanediol, 2-bromo-2-nitropropane-1,3-diol, 2,2-dibromo-2-nitro ethanol and 4-chlorophenyl-3-iodopropargyl formal; aldehydes such as formaldehyde, acetaldehyde, n-butylaldehyde, propionaldehyde, benzaldehyde, phthalaldehyde, α-bromocynnamaldehyde and phenylacetaldehyde; ketones such as acetone, methyl ethyl ketone, diethyl ketone, dibutyl ketone, methyl isobutyl ketone, cyclohexanone, acetyl acetone and 2-bromo-4'-hydroxyacetophenone; nitriles such as acetonitrile, acrylonitrile, n-butylonitrile, malononitrile, phenylacetonitrile, benzonitrile, cyanopyridine, 2,2-dibromomethylglutaronitrile, 2,3,5,6-tetrachloroisophthalonitrile, 5-chloro-2,4,6-trifluoroisophthalonitrile and 1,2-dibromo-2,4-dicyanobutane; ethers such as diethyl ether, dibutyl ether, tetrahydrofuran, dioxane, tetrahydropyran, dioxolane and trioxane; esters such as methyl acetate, ethyl acetate, butyl acetate, n-heptyl acetate and bis-1,4-bromoacetoxy-2-butene; sulfone amides such as benzene sulfone amide; amides such as N-methyl formamide, N,N-dimethyl formamide, dicyane diamide, dibromonitrile propionamide, 2,2-dibromo-3-nitrilo propionamide and N,N-diethyl-m-toluamide; halogenated hydrocarbons such as dichloromethane, chloroform, dichloroethylene and tetrachloroethylene; lactams such as ε-caprolactam; lactones such as ε-caprolactone; oxyranes such as arylglycidyl ether; morphorines; phenols such as phenol, cresol, resorcinol and p-chloro-m-cresol; carboxylic acids and thiocarboxylic acids such as formic acid, acetic acid, propionic acid, oxalic acid, citric acid, adipic acid, tartaric acid, benzoic acid, phthalic acid and salicylic acid; sulfaminic acids; thiocarbamic acids; thiosemicarbazides; ureas and thioureas such as urea, phenylurea, diphenylurea, thiourea, phenylthiourea, diphenylthiourea and N,N-dimethyldichlorophenylurea; isothioureas; sulfonylureas; thiols such as thiophenol, allyl mercaptan, n-butyl mercaptan and benzyl mercaptan; sulfides such as benzyl sulfide and butyl methyl sulfide; disulfides such as dibutyl disulfide, dibenzyl disulfide and tetramethylthiuram disulfide; sulfoxides such as dimethyl sulfoxide, dibutyl sulfoxide and dibenzyl sulfoxide; sulfones such as dimethyl sulfone, phenyl sulfone, phenyl-(2-cyano-2-chlorovinyl) sulfone, hexabromodimethyl sulfone and diiodomethylparatolyl sulfone; thiocyanic acids and isothiocyanic acids such as methyl thiocyanate and methyl isothiocyanate; amino acids such as glycine, alanine, leucine, lysine, methionine and glutamine; amides and urethane compounds; acid anhydrides; aromatic hydrocarbons such as benzene, toluene and xylene; alkanes; alkenes; alkynes; isocyanates such as butyl isocyanate, cyclohexyl isocyanate and phenyl isocyanate; thiocyanates and isothiocyanates such as methylene bisthiocyanate and methylene bisisothiocyanate; nitro compounds such as tris(hydroxymethyl)nitromethane; non-cyclic aliphatic amines such as ammonia, methylamine, ethylamine, propylamine, butylamine, pentylamine, hexylamine, allylamine, hydroxylamine, ethanolamine, benzylamine, ethylenediamine, 1,2-propanediamine, 1,3-propanediamine, 1,4-butanediamine, 1,5-pentanediamine, 1,6-hexanediamine, diethylenetriamine, triethylenetetramine, tetraethylenepentamine, dipropylenediamine, N-N-dimethylethylenediamine, N,N'-dimethylethylenediamine, N,N-dimethyl-1,3-propanediamine, N-ethyl-1,3-propanediamine, trimethylhexamethylenediamine, alkyl-t-monoamine, menthanediamine, isophoronediamine, guanidine and N-(2-hydroxypropyl)amino methanol; cyclic aliphatic amines such as cyclohexylamine, cyclohexanediamine, bis(4-aminocyclohexyl)methane, pyrrolidines, azetidines, piperidines, piperadines such as piperadine, N-aminoethylpiperadine and N,N'-dimethylpiperadine, and pyrrolines; aromatic amines such as aniline, N-methylaniline, N,N-dimethylaniline, o-phenylenediamine, m-phenylenediamine, p-phenylenediamine, diaminodiphenylmethane, diaminodiphenyl sulfone and m-xylenediamine; modified polyamines such as epoxy compound-added polyamines, Micheul-added polyamines, Mannich-added polyamines, thiourea-added polyamines and ketone-blocked polyamines; imidazoles such as imidazole, 2-methylimidazole, 2-ethylimidazole, 2-isopropylimidazole, 2-n-propylimidazole, 2-ethyl-4-methylimidazole, 1-benzyl-2-methylimidazole, 2-undecyl-1H-imidazole, 2-heptadecyl-1H-imidazole, 2-phenyl-1H-imidazole, 4-methyl-2-phenyl-1H-imidazole and 1-benzyl-2-methylimidazole; heterocyclic compounds containing nitrogen such as pyrrole, pyridine, picoline, pyrazine, pyridazine, pyrimidine, pyrazole, triazole, benzotriazole, triazine, tetrazole, purine, indole, quinoline, isoquinoline, carbazole, imidazoline, pyrroline, oxazole, piperine, pyrimidine, piridazine, benzimidazole, indazole, quinazoline, quinoxaline, phthalimide, adenine, cytosine, guanine, uracil, 2-methoxycarbonylbenzimidazole, 2,3,5,6-tetrachloro-4-methanesulfonylpyridine, 2,2-dithio-bis-(pyridine-1-oxide), N-methylpyrrolidone, 2-benzimidazole, methyl carbamate, sodium 2-pyridinethiol-1-oxide, hexahydro-1,3,5-tris(2-hydroxyethyl)-s-triazine, hexahydro-1,3,5-triethyl-s-triazine, 2-methylthio-4-t-butylamino-6-cyclopropylamino-s-triazine, N-(fluorodichloromethylthio)phthalimide, 1-bromo-3-chloro-5,5-dimethylhydantoin, 2-methoxycarbonylbenzimidazole and 2,4,6-trichlorophenylmaleimide; heterocyclic compounds containing oxygen such as furan, furfuryl alcohol, tetrahydrofurfuryl alcohol, furfurylamine, pyrane, coumarin, benzofuran, xanthene and benzodioxane; heterocyclic compounds containing nitrogen and oxygen such as oxazole, isooxazole, benzoxazole, benzoisooxazole, 5-methyloxazolidine, 4-(2-nitrobutyl)morpholine and 4,4'-(2-ethyl-2-nitrotrimethylene)dimorpholine; heterocyclic compounds containing sulfur such as thiophene, 3,3,4,4-tetrahydrothiophene-1,1-dioxide, 4,5-dichloro-1,2-dithiolan-3-one, 5-chrolo-4-phenyl-1,2-dithiolan-3-one and 3,3,4,4-tetrachlorotetrahydrothiophene-1,1-dioxide; heterocyclic compounds containing nitrogen and sulfur such as thiazole, benzothiazole, 5-chloro-2-methyl-4-isothiazolin-3-one, 2-methyl-4-isothiazolin-3-one, 4,5-dichloro-3-n-octylisothiazolin-3-one, 2-octyl-4-isothiazolin-3-one, 1,2-benzisothiazolin-3-one, 2-thiocyanomethylbenzothiazole, 2-(4-thiazolyl)benzimidazole and 2-thiocyanomethylbenzothiazole; steroids such as cholesterol; alkaloids such as brucine, quinine and theophylline; natural essential oils such as cineol, hinokitiol, menthol, terpineol, borneol, nopol, citral, citronellol, citronellal, geraniol, menthone, eugenol, linalool and dimethyloctanol; synthetic perfumes such as fragrant olive, jasmine and lemon; vitamins and related compounds such as ascorbic acid, nicotinic acid and nicotinamide.

The molecular compounds of the present invention can be produced by mixing directly or mixing in a solvent a phenol derivative of Formula (I) and substances, such as those mentioned above, that form a molecular compound with the said derivative. In case a substance has a low boiling point or high vapor pressure, a target molecular compound can be produced by reacting a phenol derivative of the present invention with the vapor of the substance. In addition, a target molecular compound may be obtained by a way that first a molecular compound composed of a phenol derivative of the present invention and a certain substance is formed and then this molecular compound is reacted with another substance by such a method as mentioned above.

It can be confirmed by such techniques as thermal analyses (TG and DTA), infrared spectra (IR), X-ray diffraction patterns or solid NMR spectra that the substances obtained by these methods are certainly molecular compounds. The compositions of the molecular compounds can be confirmed by thermal analyses, ¹H-NMR spectra, high-performance liquid chromatography (HPLC), elemental analyses and the like.

The molecular compounds of the present invention may vary in the ratio of the constituents, depending on their production conditions. It is possible to produce multi-constituent molecular compounds composed of three or more constituents, by reacting two or more substances with a phenol derivative of this invention.

It is preferable that the molecular compounds of the present invention are crystalline from the viewpoint of functions such that a useful substance is selectively separated, chemically stabilized, rendered nonvolatile or powdered and for the purpose of the stable production of molecular compounds of a constant composition. Particularly crystalline clathrate compounds are more preferred.

The same substance may be polymorphic. Crystallinity is examined mainly by X-ray diffraction patterns. The existence of polymorphism can be confirmed by thermal analyses, X-ray diffraction patterns, solid NMR and the like. In this invention clathrate compounds are defined as substances of which there are cavities of appropriate size inside a three-dimensional structure formed by atomic or molecular bonds and other atoms or molecules are included at a constant composition ratio in the cavities by non-covalent bonding interactions.

There are no particular restrictions on ways of using the molecular compounds of the present invention. For example, a mixture of two or more molecular compounds, each of which is formed with different constituent compounds, can be used. Other substances can be used together with the molecular compounds of this invention as long as target functions are not damaged. A way of using the molecular compounds of this invention is to mix with an agent such as an excipient, to form granules or tablets. In addition, the compounds may be used to add to resins, coating materials, and their raw materials or raw material compositions. The molecular compounds of this invention can be used, as they are, as materials for organic syntheses or as specific sites for reactions.

For example, a clathrate compound composed of a phenol derivative of the aforementioned Formula (I) of the present invention as a host compound and, as guest compounds, substances including isothiazolone bactericides such as 5-chloro-2-methyl-4-isothiazolin-3-one and 2-methyl-4-isothiazolin-3-one; antibacterial, insecticidal and moss proofing agents such as hinokitiol and 1,8-cineol; perfumes such as rosemary; antifouling agents such as isothiazolone compounds; catalysts including curing agents for epoxy resins such as phthalic anhydride, tetrahydrophthalic anhydride and 2-ethyl-4-methylimidazole and curing accelerators for epoxy resins such as 1,8-diazabicyclo(4,5,0)undecene-7; and organic solvents such as toluene, xylene and pyridine, has additional new functions such that a useful substance is gradually releasable, reduced in skin stimulation, chemically stabilized, rendered nonvolatile, powdered and selectively separated, in addition to the original actions of the guest compounds. Compounds such as the above are very useful, with new characteristics, as bactericides, antibacterial agents, insecticides, moss proofing agents, perfumes, antifouling agents, catalysts such as curing agents for epoxy resins, and organic solvents.

### Brief Description of Figures:

Figure 1 shows a thermal analysis (TG/DTA) chart of the molecular compound composed of 3,3'-bis(phenylsulfonyl)-4,4'-dihydroxydiphenyl sulfone and 5-chloro-2-methyl-4-isothiazolin-3-one of the composition ratio of 1:2 (molar ratio), that was obtained in Example 1 of this invention.
Figure 2 shows a ¹H-NMR spectrum (for which a d-chloroform solvent was used) of the molecular compound composed of 3,3'-bis(phenylsulfonyl)-4,4'-dihydroxydiphenyl sulfone and 5-chloro-2-methyl-4-isothiazolin-3-one of the composition ratio of 1:2 (molar ratio), that was obtained in Example 1 of this invention.
Figure 3 shows a X-ray diffraction pattern of the molecular compound composed of 3,3'-bis(phenylsulfonyl)-4,4'-dihydroxydiphenyl sulfone and 5-chloro-2-methyl-4-isothiazolin-3-one of the composition ratio of 1:2 (molar ratio), that was obtained in Example 1 of this invention.
Figure 4 shows a ¹H-NMR spectrum (for which a dimethyl sulfoxide-d₆ solvent was used) of the molecular compound composed of 3,3'-bis(phenylsulfonyl)-4,4'-dihydroxydiphenyl sulfone and 2-ethyl-4-methylimidazole of the composition ratio of 1:2 (molar ratio), that was obtained in Example 4 of this invention.
Figure 5 shows a X-ray diffraction pattern of the molecular compound composed of 3,3'-bis(phenylsulfonyl)-4,4'-dihydroxydiphenyl sulfone and 2-ethyl-4-methylimidazole of the composition ratio of 1:2 (molar ratio), that was obtained in Example 4 of this invention.
Figure 6 shows the measuring results of DSC showing curing characteristics of epoxy resins when the clathrated catalyst composed of 3,3'-bis(phenylsulfonyl)-4,4'-dihydroxydiphenyl sulfone and 2-ethyl-4-methylimidazole of the composition ratio of 1:2 (molar ratio), that was obtained in Example 4 of this invention, was used.
Figure 7 shows a ¹H-NMR spectrum (for which a dimethyl sulfoxide-d₆ solvent was used) of the molecular compound composed of 3,3'-bis(phenylsulfonyl)-4,4'-dihydroxydiphenyl sulfone and pyridine of the composition ratio of 1:2 (molar ratio), that was obtained in Example 5 of this invention.
Figure 8 shows a thermal analysis (TG/DTA) chart of the molecular compound composed of 3,3'-bis(phenylsulfonyl)-4,4'-dihydroxydiphenyl sulfone and pyridine of the composition ratio of 1:2 (molar ratio), that was obtained in Example 5 of this invention.
Figure 9 shows a ¹H-NMR spectrum (for which a dimethyl sulfoxide-d₆ solvent was used) of the molecular compound composed of 3,3'-bis(phenylsulfonyl)-4,4'-dihydroxydiphenyl sulfone, pyridine and 1,3-dimethyl-2-imidazolidinone of the composition ratio of 1:1:1 (molar ratio), that was obtained in Example 6 of this invention.
Figure 10 shows a thermal analysis (TG/DTA) chart of the molecular compound composed of 3,3'-bis(phenylsulfonyl)-4,4'-dihydroxydiphenyl sulfone, pyridine and 1,3-dimethyl-2-imidazolidinone of the composition ratio of 1:1:1 (molar ratio), that was obtained in Example 6 of this invention.
Figure 11 shows a ¹H-NMR spectrum (for which a dimethyl sulfoxide-d₆ solvent was used) of the molecular compound composed of 2,4-bis(phenylsulfonyl)phenol and 1,3-dimethyl-2-imidazolidinone of the composition ratio of 1:1 (molar ratio), that was obtained in Example 8 of this invention.
Figure 12 shows a ¹H-NMR spectrum (for which a dimethyl sulfoxide-d₆ solvent was used) of the molecular compound composed of 2,4-bis(phenylsulfonyl)phenol and pyridine of the composition ratio of 1:1 (molar ratio), that was obtained in Example 8 of this invention.
Figure 13 shows a ¹H-NMR spectrum (for which a dimethyl sulfoxide-d₆ solvent was used) of the molecular compound composed of 2,4-bis(phenylsulfonyl)phenol and N,N-dimethylformamide of the composition ratio of 1:1 (molar ratio), that was obtained in Example 8 of this invention.
Figure 14 shows a ¹H-NMR spectrum (for which a dimethyl sulfoxide-d₆ solvent was used) of the molecular compound composed of 2,4-bis(phenylsulfonyl)phenol and dimethyl sulfoxide of the composition ratio of 1:0.75 (molar ratio), that was obtained in Example 8 of this invention.
Figure 15 shows a thermal analysis (TG/DTA) chart of the molecular compound composed of 2,4-bis(phenylsulfonyl)phenol and 1,3-dimethyl-2-imidazolidinone of the composition ratio of 1:1 (molar ratio), that was obtained in Example 8 of this invention.
Figure 16 shows a thermal analysis (TG/DTA) chart of the molecular compound composed of 2,4-bis(phenylsulfonyl)phenol and pyridine of the composition ratio of 1:1 (molar ratio), that was obtained in Example 8 of this invention.
Figure 17 shows a thermal analysis (TG/DTA) chart of the molecular compound composed of 2,4-bis(phenylsulfonyl)phenol and N,N-dimethylformamide of the composition ratio of 1:1 (molar ratio), that was obtained in Example 8 of this invention.
Figure 18 shows a thermal analysis (TG/DTA) chart of the molecular compound composed of 2,4-bis(phenylsulfonyl)phenol and dimethyl sulfoxide of the composition ratio of 1:0.75 (molar ratio), that was obtained in Example 8 of this invention.
Figure 19 shows a ¹H-NMR spectrum (for which a dimethyl sulfoxide-d₆ solvent was used) of 2,4-bis(phenylsulfonyl)phenol.
Figure 20 shows a ¹H-NMR spectrum (for which a dimethyl sulfoxide-d₆ solvent was used) of the molecular compound composed of 2,4,6-tris(phenylsulfonyl)phenol and acetone of the composition ratio of 1:1 (molar ratio), that was obtained in Example 9 of this invention.
Figure 21 shows a ¹H-NMR spectrum (for which a dimethyl sulfoxide-d₆ solvent was used) of the molecular compound composed of 2,4,6-tris(phenylsulfonyl)phenol and ethyl acetate of the composition ratio of 1:1 (molar ratio), that was obtained in Example 9 of this invention.
Figure 22 shows a ¹H-NMR spectrum (for which a dimethyl sulfoxide-d₆ solvent was used) of the molecular compound composed of 2,4,6-tris(phenylsulfonyl)phenol and tetrahydrofuran of the composition ratio of 1:1 (molar ratio), that was obtained in Example 9 of this invention.
Figure 23 shows a ¹H-NMR spectrum (for which a dimethyl sulfoxide-d₆ solvent was used) of the molecular compound composed of 2,4,6-tris(phenylsulfonyl)phenol and 1,4-dioxane of the composition ratio of 1:1 (molar ratio), that was obtained in Example 9 of this invention.
Figure 24 shows a thermal analysis (TG/DTA) chart of the molecular compound composed of 2,4,6-tris(phenylsulfonyl)phenol and acetone of the composition ratio of 1:1 (molar ratio), that was obtained in Example 9 of this invention.
Figure 25 shows a thermal analysis (TG/DTA) chart of the molecular compound composed of 2,4,6-tris(phenylsulfonyl)phenol and ethyl acetate of the composition ratio of 1:1 (molar ratio), that was obtained in Example 9 of this invention.
Figure 26 shows a thermal analysis (TG/DTA) chart of the molecular compound composed of 2,4,6-tris(phenylsulfonyl)phenol and tetrahydrofuran of the composition ratio of 1:1 (molar ratio), that was obtained in Example 9 of this invention.
Figure 27 shows a thermal analysis (TG/DTA) chart of the molecular compound composed of 2,4,6-tris(phenylsulfonyl)phenol and 1,4-dioxane of the composition ratio of 1:1 (molar ratio), that was obtained in Example 9 of this invention.
Figure 28 shows a ¹H-NMR spectrum (for which a dimethyl sulfoxide-d₆ solvent was used) of 2,4,6-tris(phenylsulfonyl)phenol.

### Most Preferred Embodiment:

The present invention is described in more detail in reference to Examples and Comparative Examples. The scope of this invention is not, however, restricted by these examples.

### Example 1

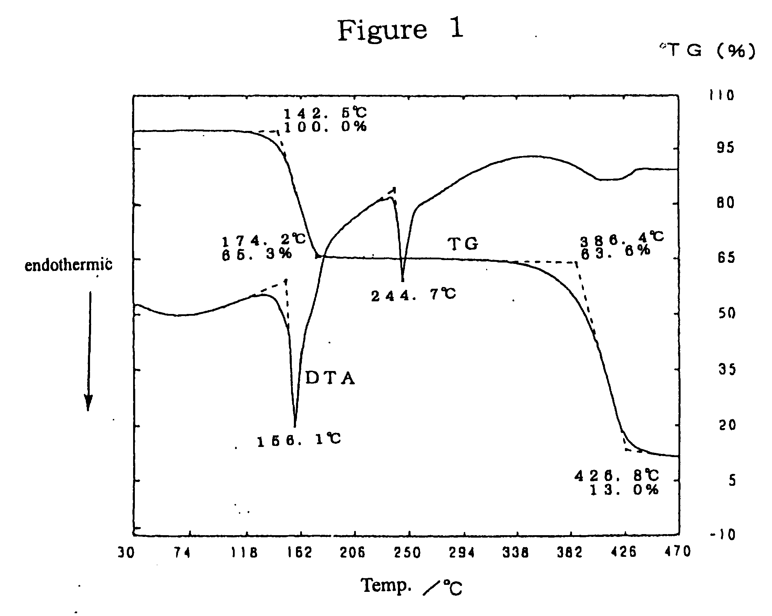

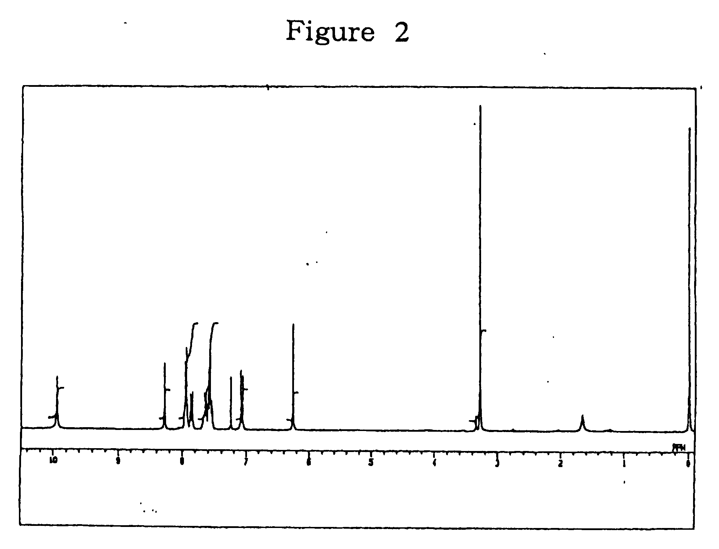

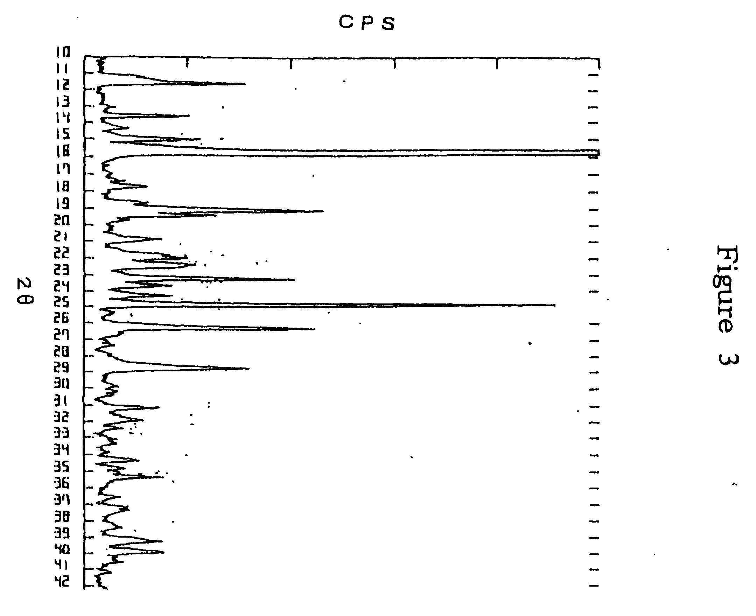

26 g (50 mmol) of 3,3'-bis(phenylsulfonyl)-4,4'dihydroxydiphenyl sulfone (Compound No. 38 in Table 1, melting point: 245°C) was dispersed and suspended in 500 ml of ethyl acetate. Into the mixture were added 220 ml of an industrial bactericide, Kathon WT (product of Rohm and Haas Co) [that contained 22 g (150 mmol) of 5-chloro-2-methyl-4-isothiazolin-3-one, 8.4 g of 2-methyl-4-isothiazolin-3-one and the remaining part of magnesium chloride, magnesium nitrate and water]. The resulting mixture was heated with stirring for 10 minutes and stood at room temperature for 24 hours. The ethyl-acetate layer was separated and concentrated by distilling ethyl acetate under reduced pressure. The deposited crystals were separated by filtration and dried under reduced pressure by a rotary vacuum pump at room temperature for 5 hours to give a molecular compound composed of 3,3'-bis(phenylsulfonyl)-4,4'-dihydroxydiphenyl sulfone and 5-chloro-2-methyl-4-isothiazolin-3-one of the composition ratio of 1:2 (molar ratio). It was confirmed by thermal analyses (TG/DTA), ¹H-NMR and X-ray diffraction patterns that the obtained was the molecular compound of the said composition. X-ray diffraction patterns showed apparently that the molecular compound was crystalline. This molecular compound released 5-chloro-2-methyl-4-isothiazolin-3-one in the range of approximately 140°C and 160°C. Figures 1, 2 and 3 show a thermal analysis (TG/DTA) chart, a ¹H-NMR spectrum (for which a d-chloroform solvent was used) and an X-ray diffraction pattern of this molecular compound, respectively.

As described above, the molecular compound of the present invention powdered and thermally stabilized 5-chloro-2-methyl-4-isothiazolin-3-one, which is the active ingredient of Kathon WT and a liquid, stimulative and highly decomposing bactericide.

### Example 2

26 g (50 mmol) of 3,3'-bis(phenylsulfonyl)-4,4'-dihydroxydiphenyl sulfone, 220 ml of Kathon WT and 900 ml of methanol were mixed and heated to dissolve with stirring. Methanol was gradually evaporated under reduced pressure at room temperature to concentrate the resulting solution. The deposited crystals were separated by filtration and dried under reduced pressure by a rotary vacuum pump at room temperature for 5 hours to give a molecular compound composed of 3,3'-bis(phenylsulfonyl)-4,4'-dihydroxydiphenyl sulfone and 5-chloro-2-methyl-4-isothiazolin-3-one of the composition ratio of 1:2 (molar ratio). It was confirmed by thermal analyses (TG/DTA), ¹H-NMR and X-ray diffraction patterns that the obtained was the molecular compound of the said composition. X-ray diffraction patterns showed apparently that the molecular compound was crystalline.

The same procedure as the above was repeated, except that methanol was rapidly evaporated under reduced pressure at room temperature to concentrate the solution to deposit crystals. The obtained was a molecular compound composed of 3,3'-bis(phenylsulfonyl)-4,4'-dihydroxydiphenyl sulfone and 5-chloro-2-methyl-4-isothiazolin-3-one of the composition ratio of 1:1 (molar ratio). It was confirmed by thermal analyses (TG/DTA), ¹H-NMR and X-ray diffraction patterns that the obtained was the molecular compound of the said composition. X-ray diffraction patterns showed apparently that the molecular compound was crystalline.

As described above, the molecular compound of the present invention powdered and thermally stabilized 5-chloro-2-methyl-4-isothiazolin-3-one, which is the active ingredient of Kathon WT and a liquid, stimulative and highly decomposing bactericide.

### Example 3

Into 220 ml of an industrial bactericide, Kathon WT (product of Robin and Haas Co.), was added 26 g of 3,3'-bis(phenylsulfonyl)-4,4'-dihydroxydiphenyl sulfone. The mixture was stirred for 10 minutes under the suspension condition at room temperature, and stood at room temperature for 24 hours. The solid matter was separated by filtration and dried under reduced pressure by a rotary vacuum pump at room temperature for 5 hours to give a molecular compound composed of 3,3'-bis(phenylsulfonyl)-4,4'-dihydroxydiphenyl sulfone and 5-chloro-2-methyl-4-isothiazolin-3-one of the composition ratio of 1:1 (molar ratio). It was confirmed by thermal analyses (TG/DTA), ¹H-NMR and X-ray diffraction patterns that the obtained was the molecular compound of the said composition. X-ray diffraction patterns showed apparently that the molecular compound was crystalline. This molecular compound released 5-chloro-2-methyl-4-isothiazolin-3-one in the range of approximately 120°C and 205°C.

As described above, the molecular compound of the present invention powdered and thermally stabilized 5-chloro-2-methyl-4-isothiazolin-3-one, which is the active ingredient of Kathon WT and a liquid, stimulative and highly decomposing bactericide.

### Comparative Example 1

Examples 1 to 3 were repeated except that the same mole number of 4,4'-dihydroxydiphenyl sulfone, bis(4-hydroxyphenyl) ether, bis(4-hydroxyphenyl) thioether, bis(4-hydroxyphenyl)methane, 2,2-bis(4-hydroxyphenyl)propane, bis(4-hydroxyphenyl) ketone or 2,4'-dihydroxydiphenyl sulfone was used instead of 3,3'-bis(phenylsulfonyl)-4,4'-dihydroxydiphenyl sulfone. In all the cases no molecular compound of 5-chloro-2-methyl-4-isothiazolin-3-one was produced.

### Example 4

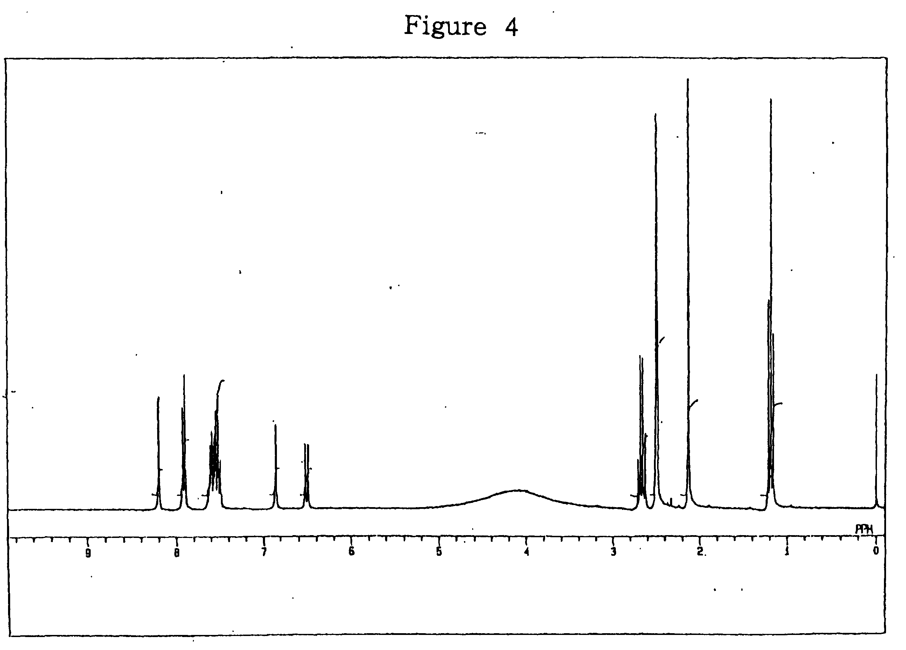

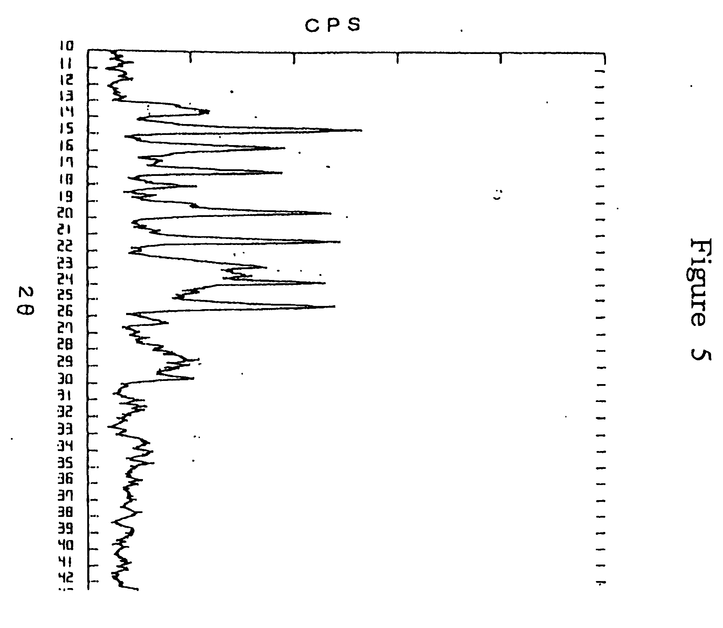

Into 400 ml of ethyl acetate were added 26 g (50 mmol) of 3,3'-bis(phenylsulfonyl)-4,4'-dihydroxydiphenyl sulfone and 17 g (150 mmol) of 2-ethyl-4-methylimidazole. The mixture was heated to dissolve and stood for 24 hours at room temperature. The deposited crystals were separated by filtration and dried under reduced pressure by a rotary vacuum pump at room temperature for 5 hours to give a molecular compound composed of 3,3'-bis(phenylsulfonyl)-4,4'-dihydroxydiphenyl sulfone and 2-ethyl-4-methylimidazole of the composition ratio of 1:2 (molar ratio). It was confirmed by thermal analyses (TG/DTA), ¹H-NMR and X-ray diffraction patterns that the obtained was the molecular compound of the said composition. X-ray diffraction patterns showed that the molecular compound was crystalline. The melting point of 2-ethyl-4-methylimidazole is 47°C in comparison to 199°C of the obtained molecular compound. The compound released 2-ethyl-4-methylimidazole at about 195°C. Figures 4 and 5 show a ¹H-NMR spectrum (for which a dimethyl sulfoxide-d₆ solvent was used) and an X-ray diffraction pattern of the molecular compound, respectively.

As described above, the molecular compound of the present invention made it possible to crystallize 2-ethyl-4-methylimidazole, which has a low melting point, and to control its melting and volatility.

A molecular compound composed of the aforementioned 3,3'-bis(phenylsulfonyl)-4,4'-dihydroxydiphenyl sulfone and 2-ethyl-4-methylimidazole, which acts as a curing agent and curing accelerator for epoxy resins, of the composition ratio of 1:2 (molar ratio) was used as a clathrated catalyst in order to study curing characteristics of epoxy resins.

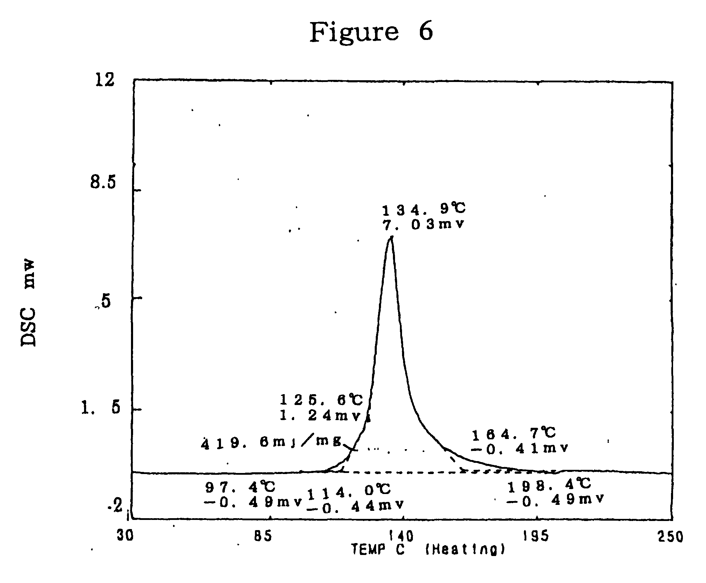

UVR-6410, a general-purpose monomer produced by Union Carbide Co, was used as an epoxy monomer. The clathrated catalyst was added so that the net weight of the curing agent (2-ethyl-4-methylimidazole) was 0.4 g to 10 g of the monomer. The mixture was well stirred in a 50-ml Teflon beaker for 5 minutes. Part of the resulting mixture was used as a sample for DSC (Differential Scanning Calorimeter) measurements. Figure 6 shows the results of the DSC measurements.

As seen from Figure 6, when the 2-ethyl-4-methylimidazole clathrated catalyst with the host compound of 3,3'-bis(phenylsulfonyl)-4,4'-dihydroxydiphenyl sulfone was used, curing started at 114°C and the curing reaction peaked at 135°C.

The above reaction was repeated except that 2-ethyl-4-methylimidazole was used as a curing agent instead of the clathrated catalyst. Curing started at 79°C and the curing reaction peaked at 114°C.

Based on the above results, it was confirmed that, with the use of the clathrated catalyst of 2-ethyl-4-methylimidazole, curing starting temperature was raised and a difference in temperature between the start of curing and the peak of the curing reaction was reduced so as to improve heat sensitivity.

### Example 5

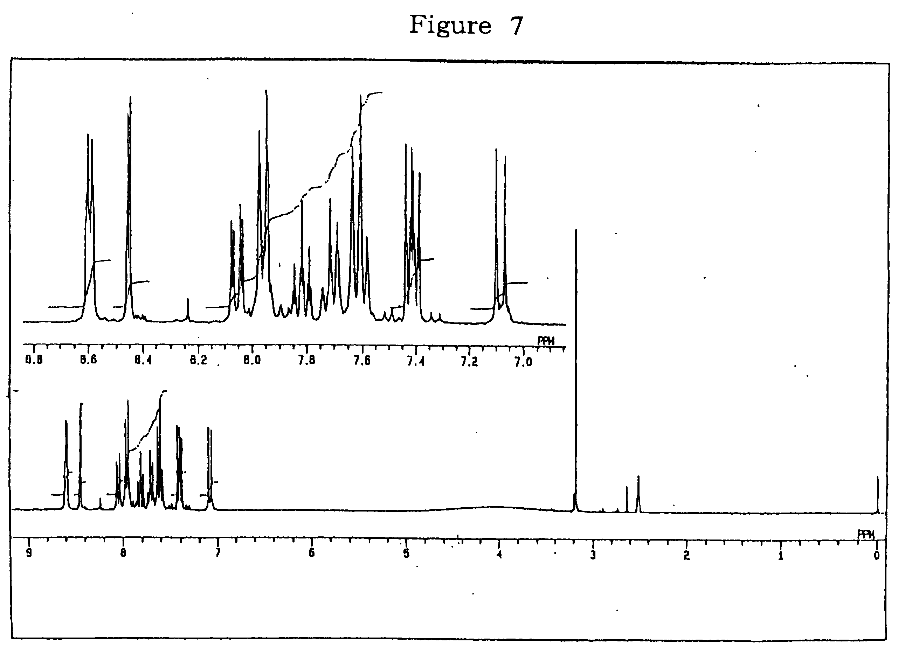

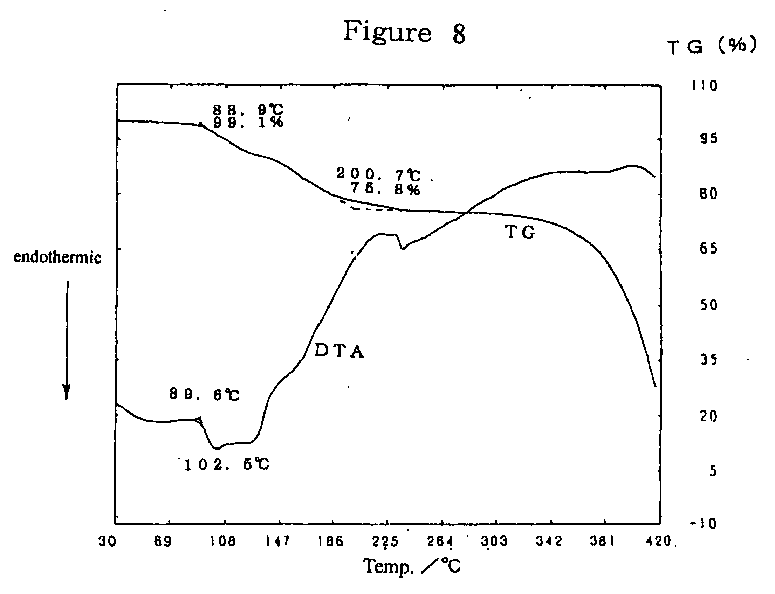

20 g (38 mmol) of 3,3'-bis(phenylsulfonyl)-4,4'dihydroxydiphenyl sulfone and 12 g (150 mmol) of pyridine were dissolved in 100 ml of methanol at room temperature. The resulting solution stood at 0°C for 24 hours. The deposited crystals were separated by filtration and dried under reduced pressure by a rotary vacuum pump at room temperature for 5 hours to give a molecular compound composed of 3,3'-bis(phenylsulfonyl)-4,4'-dihydroxydiphenyl sulfone and pyridine of the composition ratio of 1:2 (molar ratio). It was confirmed by thermal analyses (TG/DTA), ¹H-NMR and X-ray diffraction patterns that the obtained was the molecular compound of the said composition. X-ray diffraction patterns showed that the molecular compound was crystalline. This molecular compound released pyridine in the range of approximately 90°C and 200°C. Figures 7 and 8 show a ¹H-NMR spectrum (for which a dimethyl sulfoxide-d₆ solvent was used) and a thermal analysis (TG/DTA) chart of the molecular compound, respectively.

As described above, the molecular compound of the present invention made it possible to powder pyridine, which is a liquid at room temperature, and to control its volatility.

### Example 6

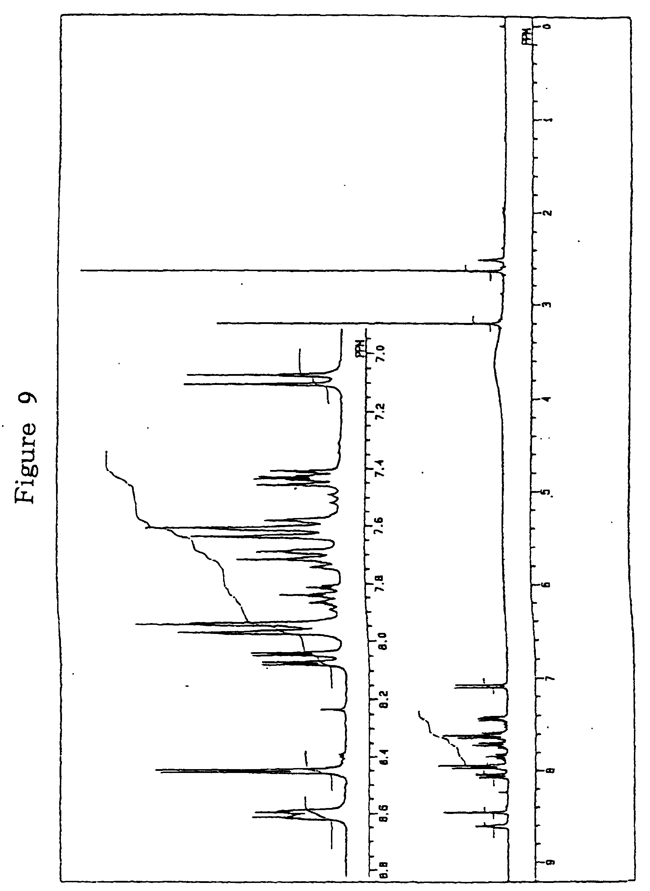

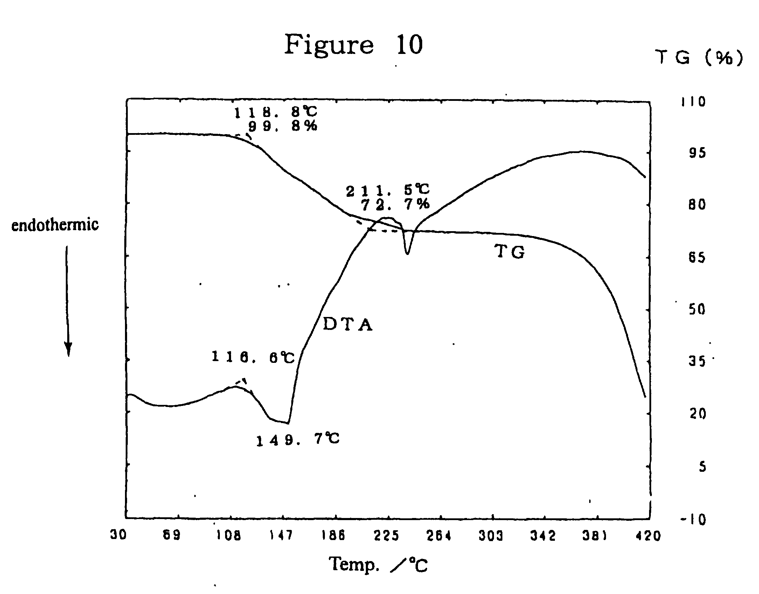

20 g (38 mmol) of 3,3'-bis(phenylsulfonyl)-4,4'dihydroxydiphenyl sulfone, 12 g (150 mmol) of pyridine and 4.6 g (40 mmol) of 1,3-dimethyl-2-imidazolidinone were added into 200 ml of ethyl acetate at room temperature and heated to dissolve. The resulting solution stood at 0°C for 24 hours. The deposited crystals were separated by filtration and dried under reduced pressure by a rotary vacuum pump at room temperature for 5 hours to give a molecular compound composed of 3,3'-bis(phenylsulfonyl)-4,4'-dihydroxydiphenyl sulfone, pyridine and 1,3-dimethyl-2-imidazolidinone of the composition ratio of 1:1:1 (molar ratio). It was confirmed by thermal analyses (TG/DTA), ¹H-NMR and X-ray diffraction patterns that the obtained was the molecular compound of the said composition. X-ray diffraction patterns showed that the molecular compound was crystalline. This molecular compound released pyridine and 1,3-dimethyl-2-imidazolidinone in the range of approximately 118°C and 212°C. Figures 9 and 10 show a ¹H-NMR spectrum (for which a dimethyl sulfoxide-d₆ solvent was used) and a thermal analysis (TG/DTA) chart of the molecular compound, respectively.

As described above, the molecular compound of the present invention made it possible to powder pyridine and 1,3-dimethyl-2-imidazolidinone, which are liquids at room temperature, and to control their volatility.

### Comparative Example 2

Examples 5 and 6 were repeated except that the same mole number of 4,4'-dihydroxydiphenyl sulfone, bis(4-hydroxyphenyl) ether, bis(4-hydroxyphenyl) thioether, bis(4-hydroxyphenyl)methane, 2,2-bis(4-hydroxyphenyl)propane, bis(4-hydroxyphenyl) ketone or 2,4'-dihydroxydiphenyl sulfone was used instead of 3,3'-bis(phenylsulfonyl)-4,4'-dihydroxydiphenyl sulfone. In all the cases no molecular compounds of pyridine and 1,3-dimethyl-2-imidazolidinone were produced.

### Example 7

15 g (28 mmol) of 3,3'-bis(phenylsulfonyl)-4,4'dihydroxydiphenyl sulfone was added into 100 ml of tetrahydrofuran and heated to dissolve. The resulting solution stood at room temperature for 72 hours. The deposited crystals were separated by filtration and dried under reduced pressure by a rotary vacuum pump at room temperature for 5 hours to give a molecular compound composed of 3,3'-bis(phenylsulfonyl)-4,4'-dihydroxydiphenyl sulfone and tetrahydrofuran of the composition ratio of 1:4 (molar ratio). The same procedure was repeated except that 1,4-dioxane and N,N-dimethylformamide were used, instead of tetrahydrofuran, to give molecular compounds composed of 3,3'-bis(phenylsulfonyl)-4,4'dihydroxydiphenyl sulfone and 1,4-dioxane of the composition ratio of 1:1 (molar ratio), and of 3,3'-bis(phenylsulfonyl)-4,4'dihydroxydiphenyl sulfone and N,N-dimethylformamide of the composition ratio of 1:1.5 (molar ratio), respectively. It was confirmed by thermal analyses (TG/DTA), ¹H-NMR and X-ray diffraction patterns that the obtained were the molecular compounds of the said compositions. X-ray diffraction patterns showed that the molecular compounds were crystalline.

As described above, the molecular compounds of the present invention made it possible to powder tetrahydrofuran, 1,4-dioxane and N,N-dimethylformamide, which are liquids at room temperature.

### Comparative Example 3

Example 7 was repeated except that the same mole number of 4,4'-dihydroxydiphenyl sulfone, bis(4-hydroxyphenyl) ether, bis(4-hydroxyphenyl) thioether, bis(4-hydroxyphenyl)methane, 2,2-bis(4-hydroxyphenyl)propane, bis(4-hydroxyphenyl) ketone or 2,4'-dihydroxydiphenyl sulfone was used instead of 3,3'-bis(phenylsulfonyl)-4,4'-dihydroxydiphenyl sulfone. In all the cases no molecular compounds of tetrahydrofuran, 1,4-dioxane and N,N-dimethylformamide were produced.

### Example 8

20 g of 2,4-bis(phenylsulfonyl)phenol was added into 100 ml of a mixed solvent of 1,3-dimethyl-2-imidazolidinone and methanol of 1:1 (volume ratio) and heated to dissolve. The resulting solution stood at 5°C for 24 hours. The deposited crystals were separated by filtration and dried under reduced pressure by a rotary vacuum pump at room temperature for 5 hours to give a molecular compound composed of 2,4-bis(phenylsulfonyl)phenol and 1,3-dimethyl-2-imidazolidinone of the composition ratio of 1:1 (molar ratio). The same procedure was repeated except that pyridine was used, instead of 1,3-dimethyl-2-imidazolidinone and methanol, to give a molecular compound composed of 2,4-bis(phenylsulfonyl)phenol and pyridine of the composition ratio of 1:1 (molar ratio). 20 g of 2,4-bis(phenylsulfonyl)phenol was added into 50 ml of N,N-dimethylformamide and heated to dissolve. N,N-dimethylformamide was removed by a rotary evaporator. The solid residue was dried under reduced pressure by a rotary vacuum pump at 80°C for 5 hours to give a molecular compound composed of 2,4-bis(phenylsulfonyl)phenol and N,N-dimethylformamide of the composition ratio of 1:1 (molar ratio). The same procedure was repeated using dimethyl sulfoxide, instead of N,N-dimethylformamide, to give a molecular compound composed of 2,4-bis(phenylsulfonyl)phenol and dimethyl sulfoxide of the composition ratio of 1:0.75 (molar ratio). It was confirmed by thermal analyses (TG/DTA), ¹H-NMR and X-ray diffraction patterns that the obtained were the molecular compounds of the said compositions. X-ray diffraction patterns showed apparently that each of the molecular compounds was crystalline. Each of the molecular compounds released 1,3-dimethyl-2-imidazolidinone in the range of approximately 130°C and 230°C, pyridine in the range of approximately 90°C and 210°C, N,N-dimethylformamide in the range of approximately 95°C and 185°C, and dimethyl sulfoxide in the range of approximately 95°C and 220°C.

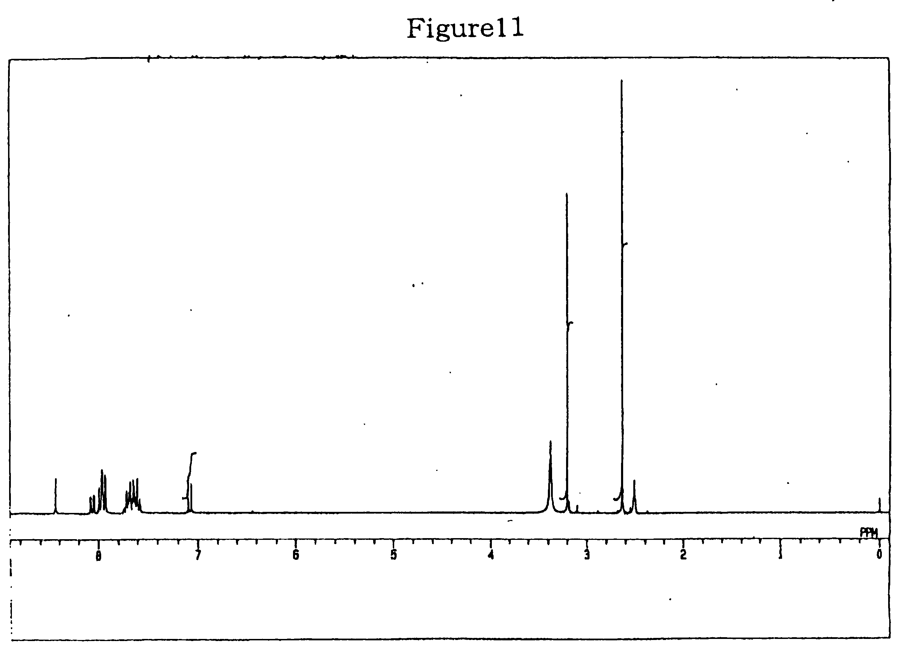

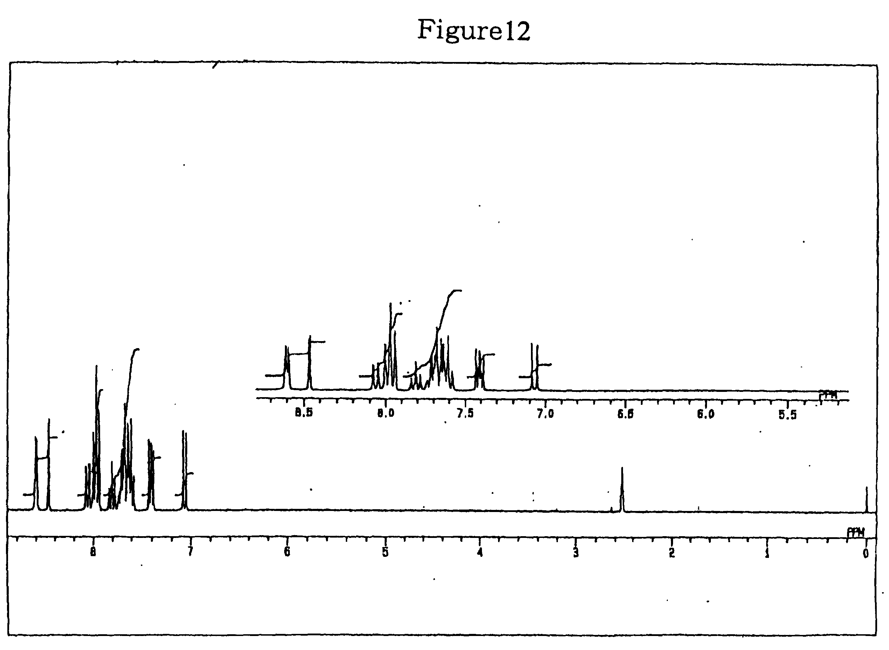

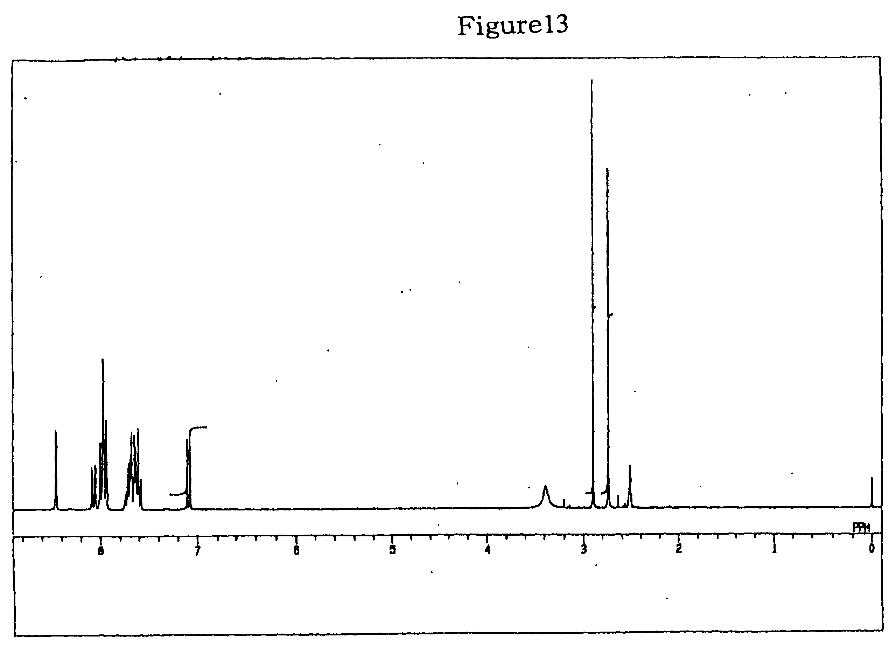

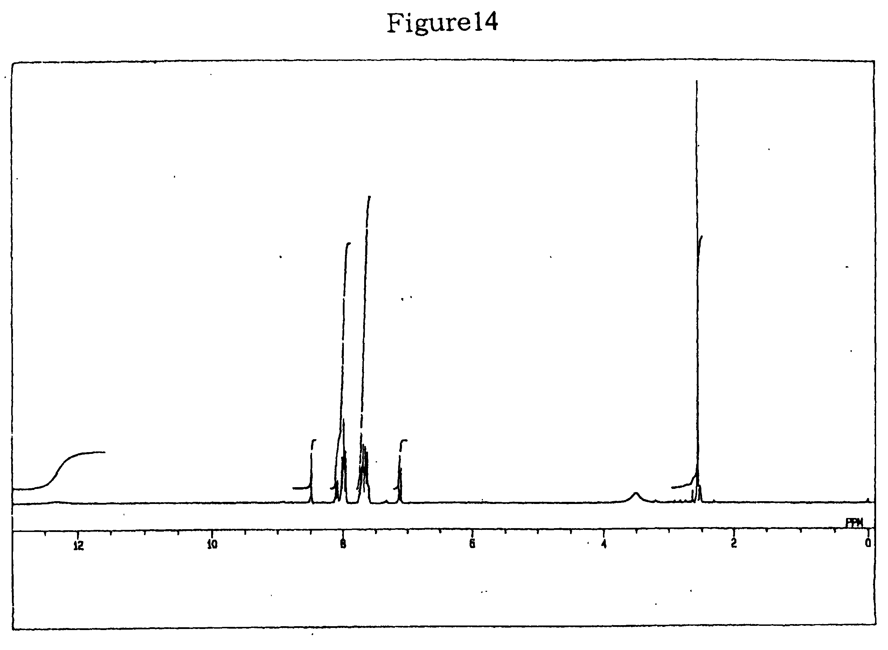

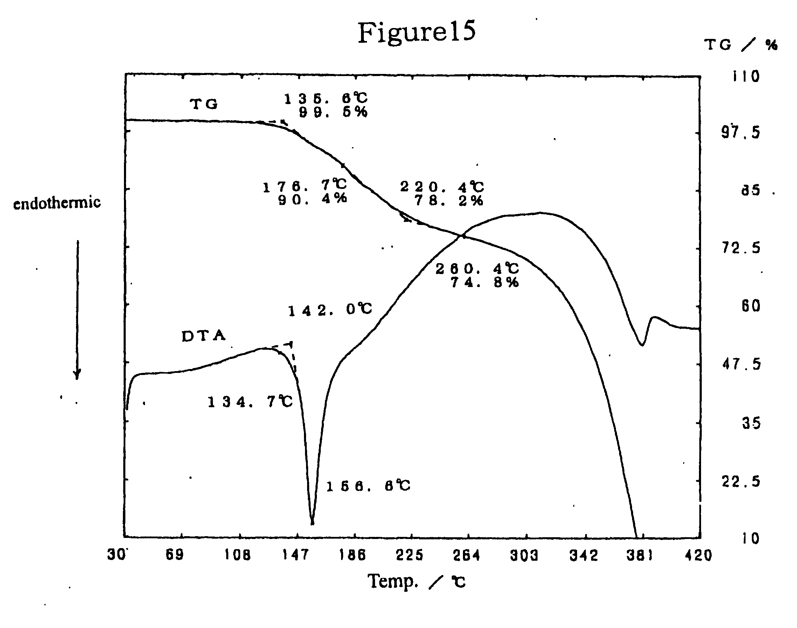

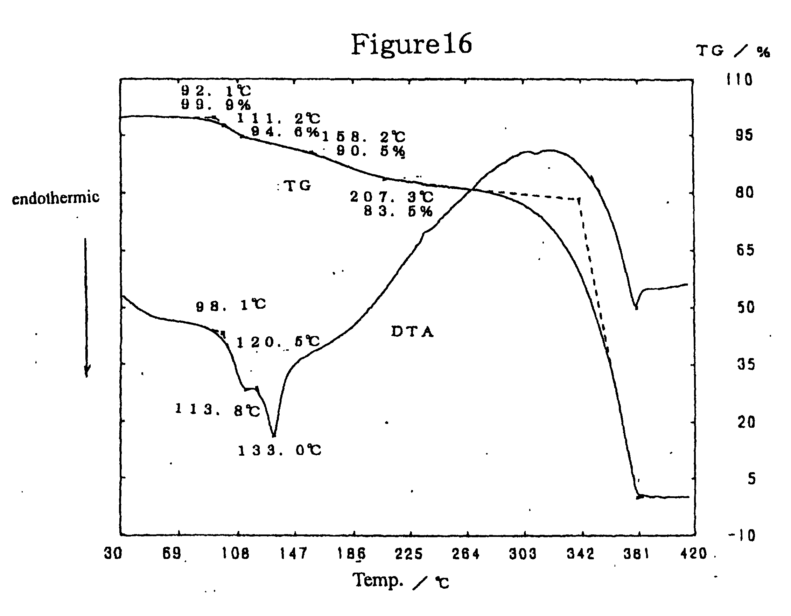

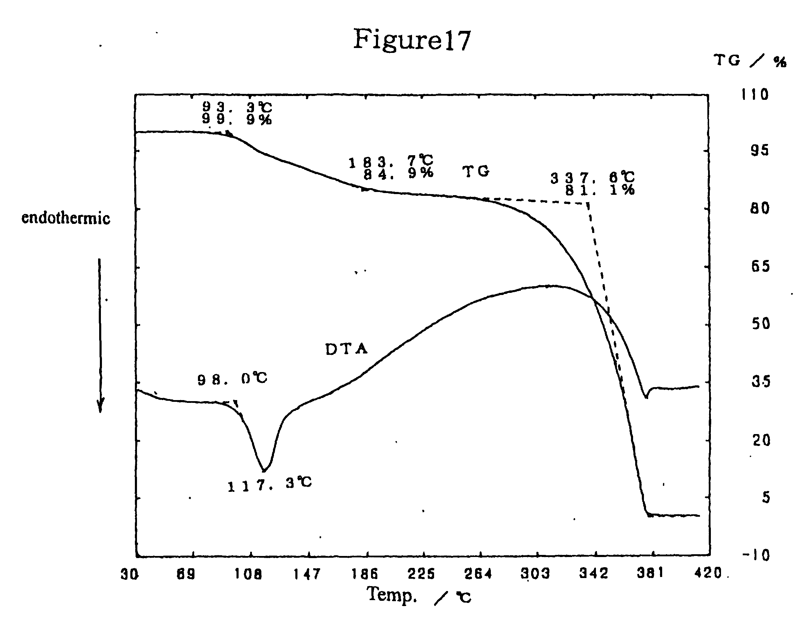

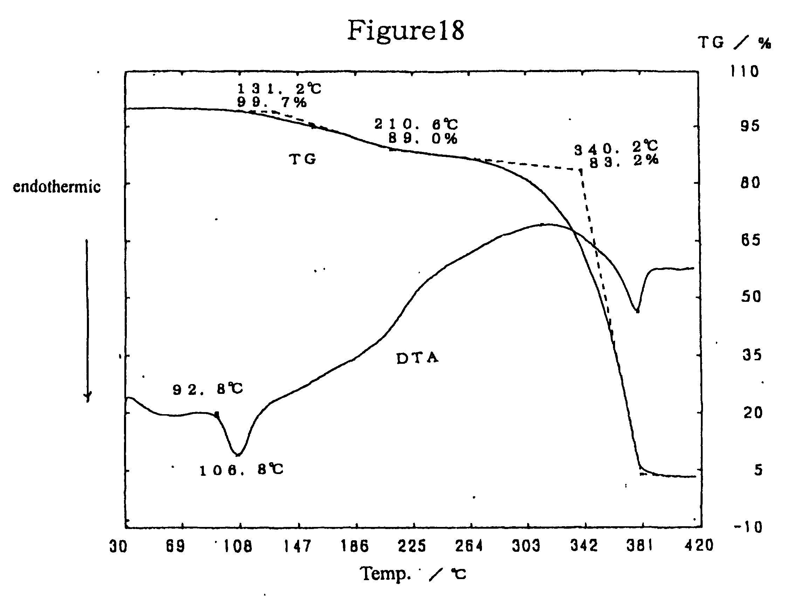

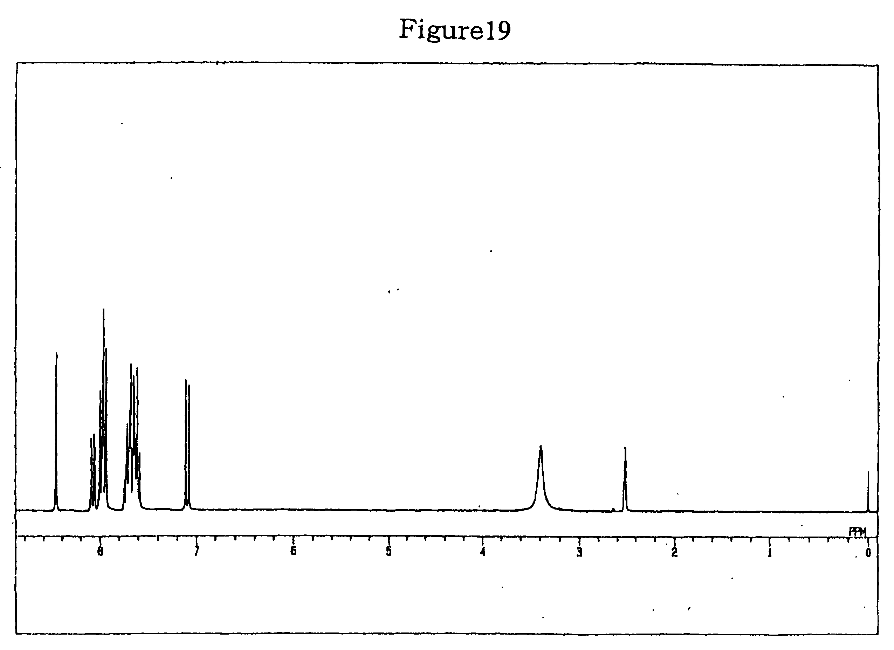

Figures 11, 12, 13 and 14 show ¹H-NMR spectra (for which a dimethyl sulfoxide-d₆ solvent was used) of the molecular compounds composed of 2,4-bis(phenylsulfonyl)phenol with 1,3-dimethyl-2-imidazolidinone, pyridine, N,N-dimethylformamide and dimethyl sulfoxide, respectively. Their thermal analysis (TG/DTA) charts are shown in Figures 15, 16, 17 and 18, respectively. For comparison, a ¹H-NMR spectrum (for which a dimethyl sulfoxide-d₆ solvent was used) of 2,4-bis(phenylsulfonyl)phenol is shown in Figure 19.

As described above, the molecular compounds of the present invention made it possible to powder 1,3-dimethyl-2-imidazolidinone, pyridine, N,N-dimethylformamide and dimethyl sulfoxide, which are liquids at room temperature, and to control their volatility.

### Example 9

20 g (38 mmol) of 2,4,6-tris(phenylsulfonyl)phenol was suspended in 100 ml of acetone. The mixture was heated at reflux temperature for 10 minutes and stood at 5°C for 24 hours. The deposited crystals were separated by filtration and dried under reduced pressure by a rotary vacuum pump at room temperature for 5 hours to give a molecular compound composed of 2,4,6-tris(phenylsulfonyl)phenol and acetone of the composition ratio of 1:1 (molar ratio). The same procedure was repeated except that ethyl acetate, tetrahydrofuran or 1,4-dioxane was used, instead of acetone, to give a molecular compound composed of 2,4,6-tris(phenylsulfonyl)phenol and ethyl acetate, tetrahydrofuran or 1,4-dioxane, of the composition ratio of 1:1 (molar ratio). It was confirmed by thermal analyses (TG/DTA), ¹H-NMR and X-ray diffraction patterns that the obtained were the molecular compounds of the said compositions. X-ray diffraction patterns showed apparently that each of the molecular compounds was crystalline. The molecular compounds released acetone in the range of approximately 90°C and 132°C, ethyl acetate in the range of approximately 70°C and 81°C, tetrahydrofuran in the range of approximately 85°C and 188°C and dimethyl sulfoxide in the range of approximately 92°C and 136°C.

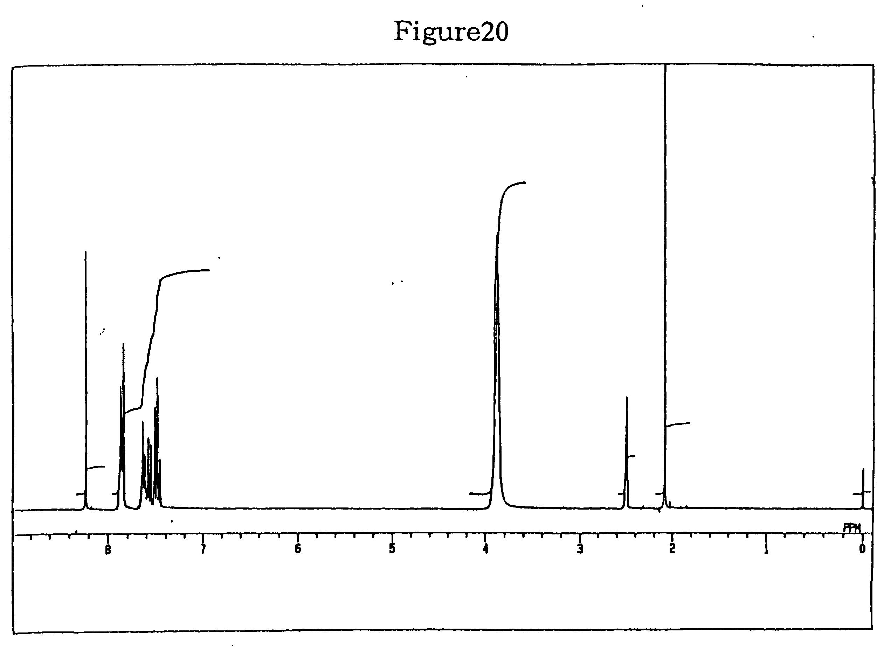

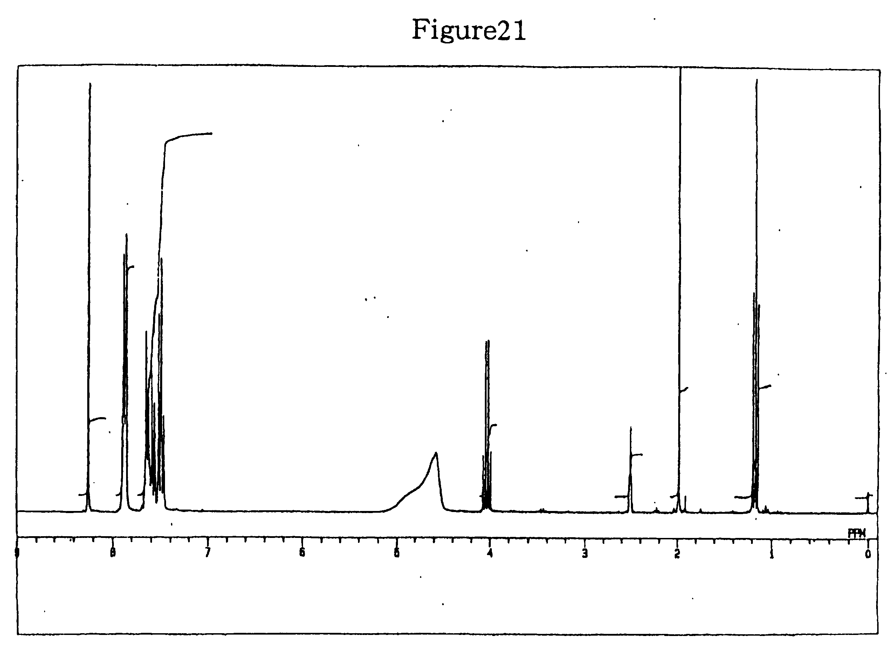

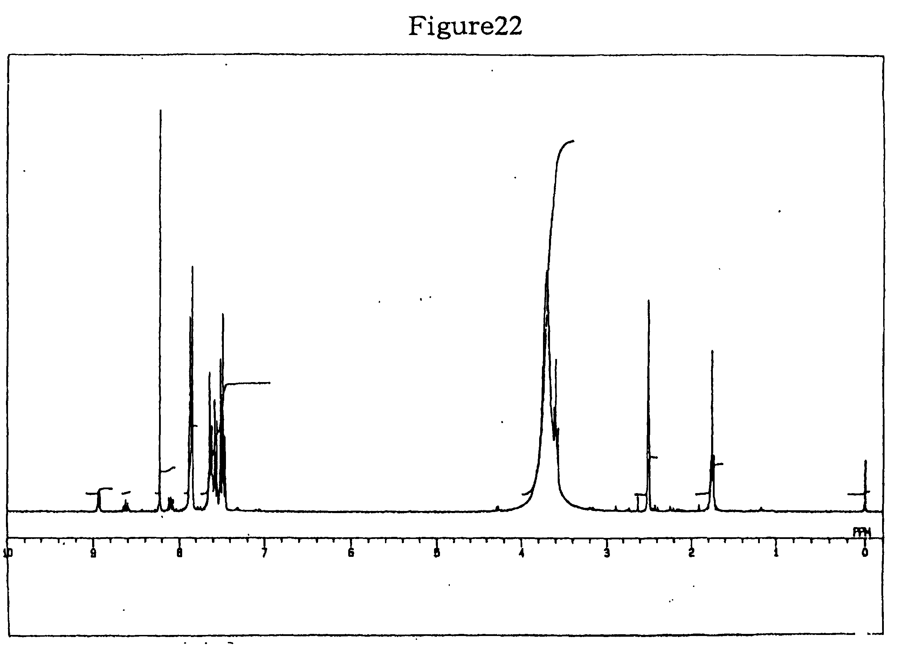

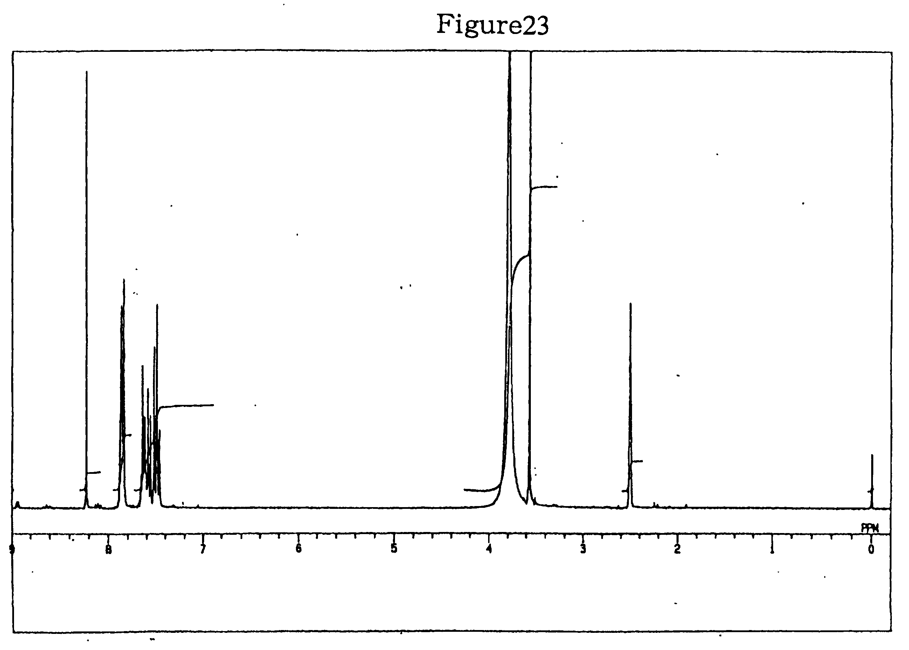

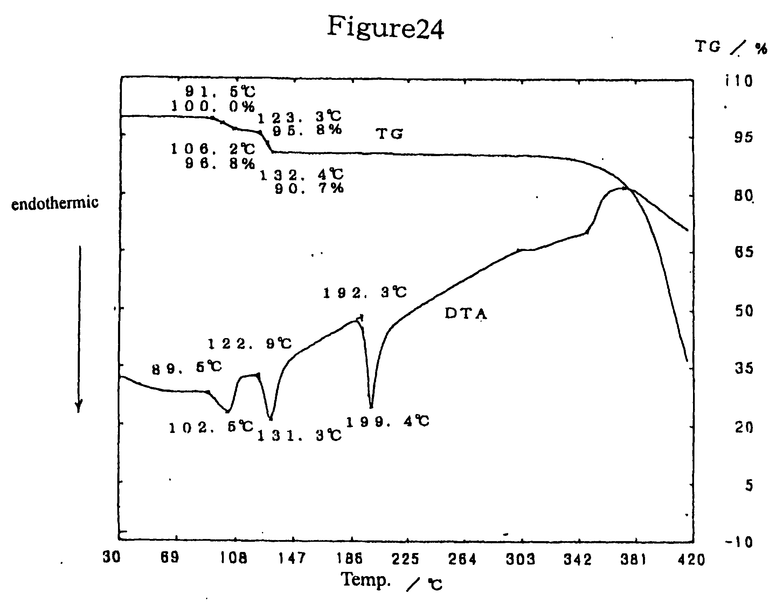

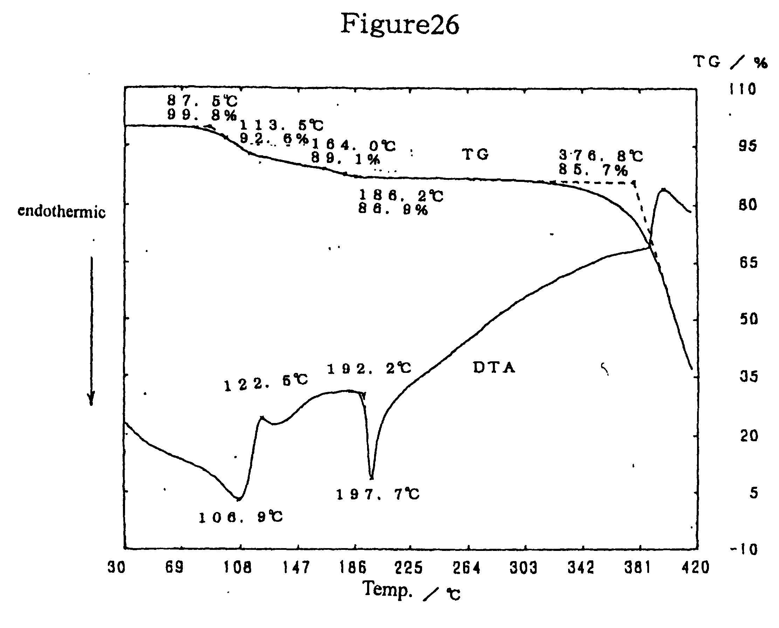

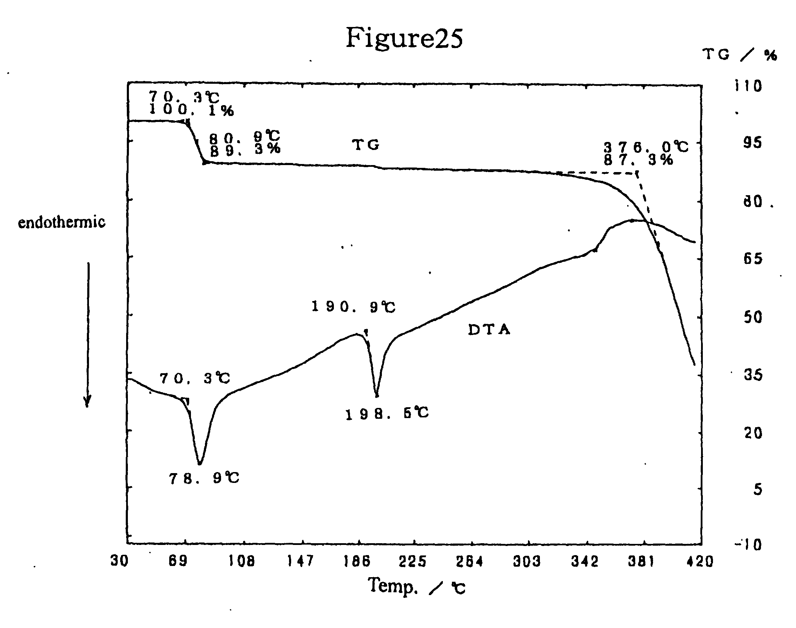

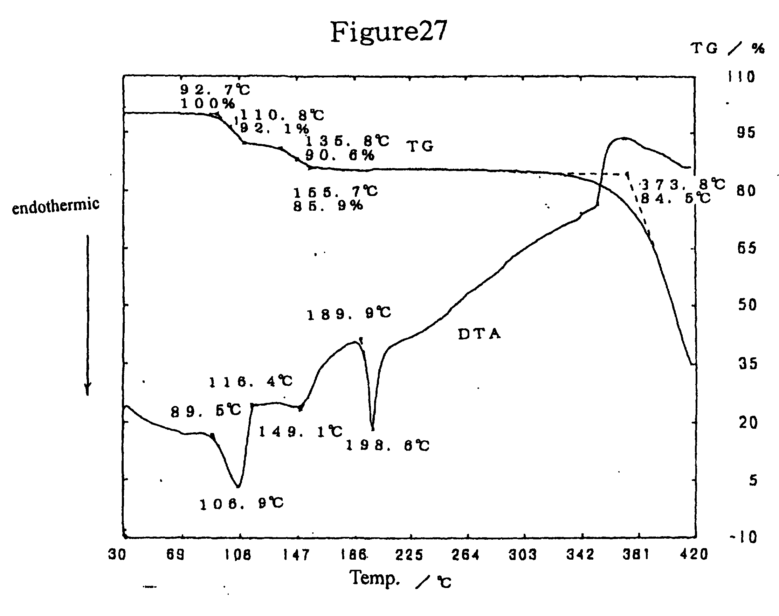

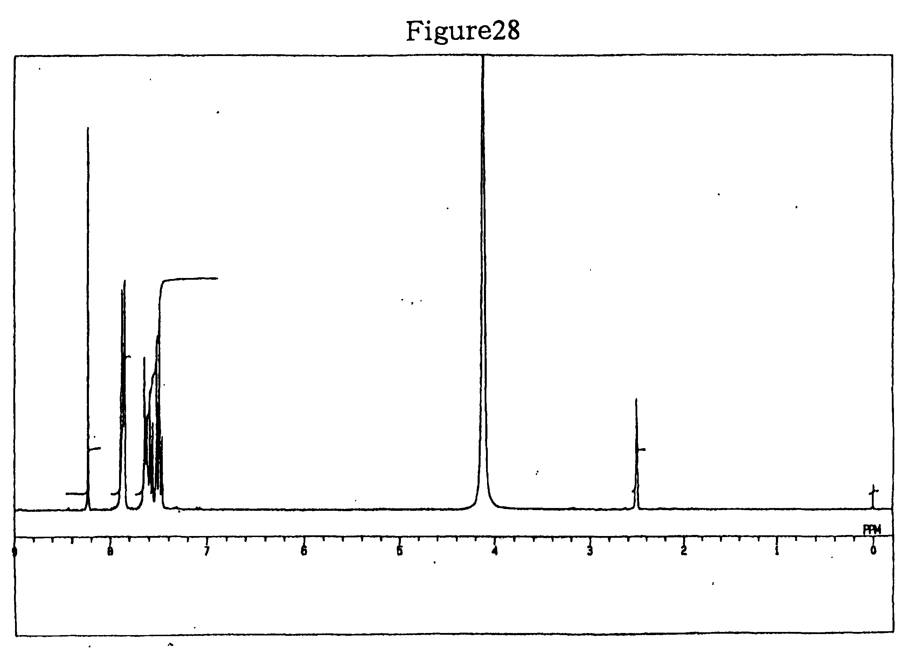

Figures 20, 21, 22 and 23 show ¹H-NMR spectra (for which a dimethyl sulfoxide-d₆ solvent was used) of the molecular compounds composed of 2,4,6-tris(phenylsulfonyl)phenol with acetone, ethyl acetate, tetrahydrofuran and 1,4-dioxane, respectively. Their thermal analysis (TG/DTA) charts are shown in Figures 24, 25, 26 and 27, respectively. For comparison, a ¹H-NMR spectrum (for which a dimethyl sulfoxide-d₆ solvent was used) of 2,4,6-tris(phenylsulfonyl)phenol is shown in Figure 28.

As described above, the molecular compounds of the present invention made it possible to powder acetone, ethyl acetate, tetrahydrofuran and 1,4-dioxane, which are liquids at room temperature, and to control their volatility.

### Applicability in Industry:

Novel molecular compounds of the present invention can be prepared by simple operations. Besides they chemically stabilize, make nonvolatile, slowly release and powder a variety of substances. They can be also used for the selective separation or recovery of specific substances. Furthermore, the molecular compounds of the present invention can be used together with various substances and in a variety of forms. Therefore, the present invention is applicable in very wide areas and has great significance in industry.

## Claims

1. A molecular compound containing, as a constituent, a phenol derivative represented by Formula (I) 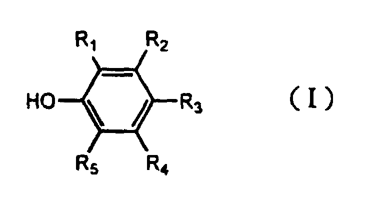 [wherein R₁ and R₅ are, same or different, groups selected from hydrogen, halogen, alkyl having 1 to 4 carbons, alkenyl having 2 to 4 carbons, alkoxy having 1 to 4 carbons, hydroxyl or 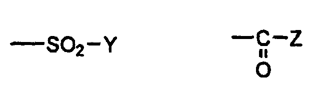 (wherein Y and Z are alkyl having 1 to 8 carbons, alkenyl having 2 to 8 carbons, alkoxy having 1 to 6 carbons, hydroxyl, optionally substituted amino, optionally substituted cycloalkyl, optionally substituted phenyl or optionally substituted aralkyl);
R₂ and R₄ are, same or different, hydrogen, halogen, alkyl having 1 to 4 carbons, alkenyl having 2 to 4 carbons, alkoxy having 1 to 4 carbons or hydroxyl, but they are groups selected from hydrogen, halogen, alkyl having 1 to 4 carbons, alkenyl having 2 to 4 carbons, alkoxy having 1 to 4 carbons, hydroxyl or 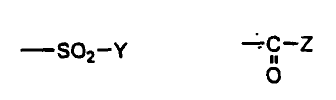 (wherein Y and Z are as defined above), in case R₁, R₃ or R₅ is alkoxy having 1 to 4 carbons or hydroxyl;
R₃ is hydrogen, halogen, alkyl having 1 to 4 carbons, alkenyl having 2 to 4 carbons, alkoxy having 1 to 4 carbons, hydroxyl, Formula (II) or Formula (III) 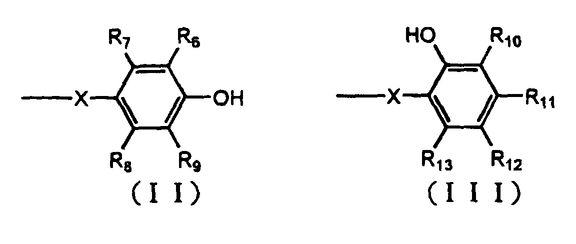 {wherein X is 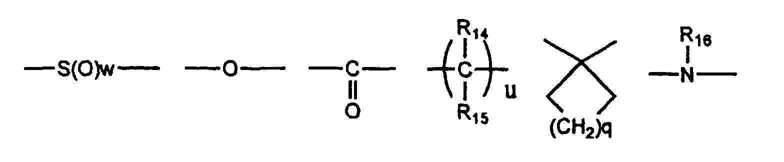 (wherein w is 0, 1 or 2; u is 0 or 1; q is 0 to 4; R₁₄ and R₁₅ are, same or different, hydrogen, halogen, alkyl having 1 to 4 carbons, alkenyl having 2 to 4 carbons, alkoxy having 1 to 4 carbons, hydroxyl, optionally substituted phenyl or optionally substituted aralkyl; R₁₆ is hydrogen, alkyl having 1 to 4 carbons, alkenyl having 2 to 4 carbons, alkoxy having 1 to 4 carbons, hydroxyl, optionally substituted phenyl or optionally substituted aralkyl);
R₆, R₉ and R₁₀ are, same or different, groups selected from hydrogen, halogen, alkyl having 1 to 4 carbons, alkenyl having 2 to 4 carbons, alkoxy having 1 to 4 carbons, hydroxyl, or 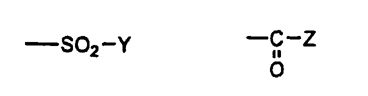 (wherein Y and Z are as defined above);
R₇, R₈, R₁₁, and R₁₃ are, same or different, hydrogen, halogen, alkyl having 1 to 4 carbons, alkenyl having 2 to 4 carbons, alkoxy having 1 to 4 carbons or hydroxyl, but R₁₁ is a group selected from hydrogen, halogen, alkyl having 1 to 4 carbons, alkenyl having 2 to 4 carbons, alkoxy having 1 to 4 carbons, hydroxyl or 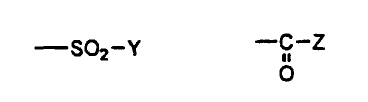 (wherein Y and Z are as defined above) in case R₁₂ is alkoxy having 1 to 4 carbons or hydroxyl;
R₁₂ is a group selected from hydrogen, halogen, alkyl having 1 to 4 carbons, alkenyl having 2 to 4 carbons, alkoxy having 1 to 4 carbons, hydroxyl or 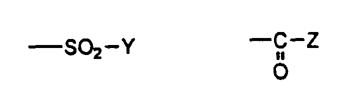 (wherein Y and Z are as defined above)}, or 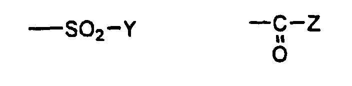 (wherein Y and Z are as defined above), or
when R₃ is of Formula (II), one of R₁, R₅, R₆ and R₉ is a group represented by 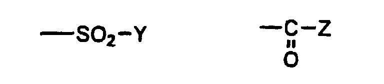 (wherein Y and Z are as defined above)
when R₃ is of Formula (III), at least one of R₁, R₅ and R₁₀ is a group represented by 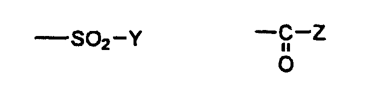 (wherein Y and Z are as defined above), and
when R₃ is a group other than Formula (II) or (III), either R₁ or R₅ is a group represented by 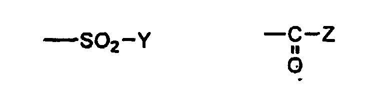 (wherein Y and Z are as defined above)].

2. A molecular compound containing, as a constituent, a phenol derivative represented by Formula (IV) 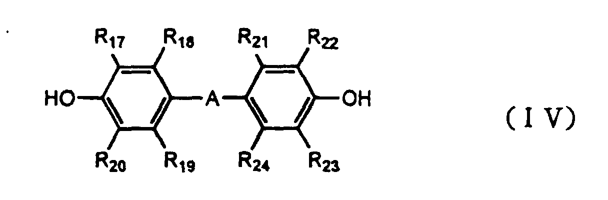 [wherein A is a group selected from 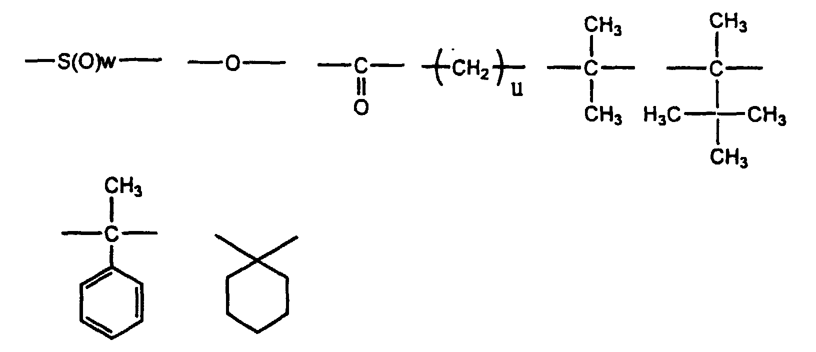 (wherein w is 0, 1 or 2 and u is 0 or 1); R₁₈, R₁₉, R₂₁ and R₂₄ are , same or different, hydrogen, halogen, alkyl having 1 to 4 carbons or alkenyl having 2 to 4 carbons; R₁₇ is 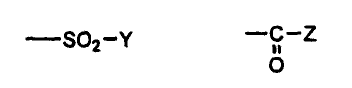 (wherein Y and Z are alkyl having 1 to 6 carbons, alkenyl having 2 to 6 carbons, cyclohexyl which may have alkyl having 1 to 4 carbons or alkenyl having 2 to 4 carbons or alkoxy having 1 to 4 carbons or hydroxyl or halogen, cyclopentyl which may have alkyl having 1 to 4 carbons or alkenyl having 2 to 4 carbons or alkoxy having 1 to 4 carbons or hydroxyl or halogen, phenyl which may have alkyl having 1 to 4 carbons or alkenyl having 2 to 4 carbons or alkoxy having 1 to 4 carbons or halogen, benzyl which may have alkyl having 1 to 4 carbons or alkenyl having 2 to 4 carbons or alkoxy having 1 to 4 carbons or hydroxyl or halogen, phenethyl which may have alkyl having 1 to 4 carbons or alkenyl having 2 to 4 carbons or alkoxy having 1 to 4 carbons or hydroxyl or halogen, α-methylbenzyl which may have alkyl having 1 to 4 carbons or alkenyl having 2 to 4 carbons or alkoxy having 1 to 4 carbons or hydroxyl or halogen, or naphthyl which may have alkyl having 1 to 4 carbons or alkenyl having 2 to 4 carbons or alkoxy having 1 to 4 carbons or hydroxyl or halogen), and R₂₀, R₂₂ and R₂₃ are , same or different, hydrogen, halogen, alkyl having 1 to 4 carbons, alkenyl having 2 to 4 carbons, alkoxy having 1 to 4 carbons or the same groups as those for R₁₇].

3. A molecular compound containing, as a constituent, a phenol derivative represented by Formula (V) 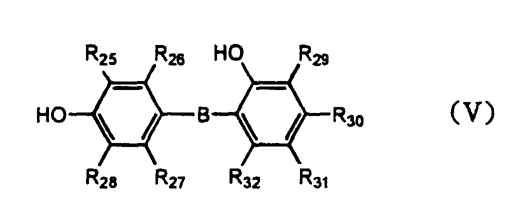 [wherein B is a group selected from 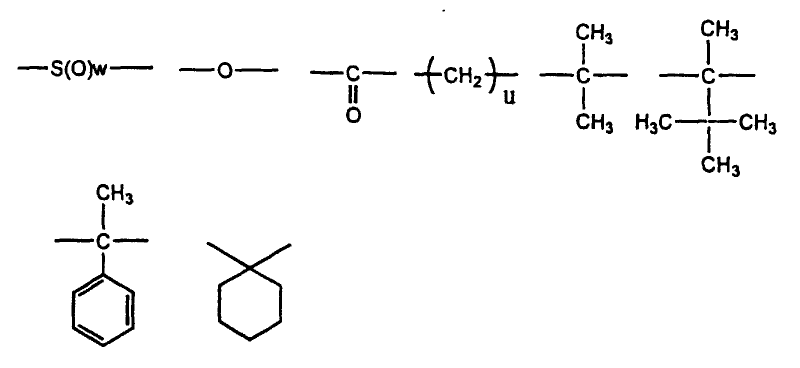 (wherein w is 0, 1 or 2 and u is 0 or 1); R₂₆, R₂₇, R₃₀ and R₃₂ are , same or different, hydrogen, halogen, alkyl having 1 to 4 carbons or alkenyl having 2 to 4 carbons; R₂₅, R₂₈, R₂₉ and R₃₁ are , same or different, hydrogen, halogen, alkyl having 1 to 4 carbons, alkenyl having 2 to 4 carbons, alkoxy having 1 to 4 carbons or 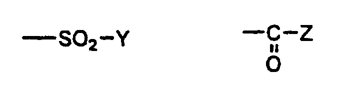 (wherein Y and Z are alkyl having 1 to 6 carbons, alkenyl having 2 to 6 carbons, cyclohexyl which may have alkyl having 1 to 4 carbons or alkenyl having 2 to 4 carbons or alkoxy having 1 to 4 carbons or hydroxyl or halogen, cyclopentyl which may have alkyl having 1 to 4 carbons or alkenyl having 2 to 4 carbons or alkoxy having 1 to 4 carbons or hydroxyl or halogen, phenyl which may have alkyl having 1 to 4 carbons or alkenyl having 2 to 4 carbons or alkoxy having 1 to 4 carbons or halogen, benzyl which may have alkyl having 1 to 4 carbons or alkenyl having 2 to 4 carbons or alkoxy having 1 to 4 carbons or hydroxyl or halogen, phenethyl which may have alkyl having 1 to 4 carbons or alkenyl having 2 to 4 carbons or alkoxy having 1 to 4 carbons or hydroxyl or halogen, α-methylbenzyl which may have alkyl having 1 to 4 carbons or alkenyl having 2 to 4 carbons or alkoxy having 1 to 4 carbons or hydroxyl or halogen, or naphthyl which may have alkyl having 1 to 4 carbons or alkenyl having 2 to 4 carbons or alkoxy having 1 to 4 carbons or hydroxyl or halogen), and at least one of R₂₅, R₂₈ and R₂₉ is 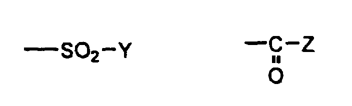 (wherein Y and Z are alkyl having 1 to 6 carbons, alkenyl having 2 to 6 carbons, cyclohexyl which may have alkyl having 1 to 4 carbons or alkenyl having 2 to 4 carbons or alkoxy having 1 to 4 carbons or hydroxyl or halogen, cyclopentyl which may have alkyl having 1 to 4 carbons or alkenyl having 2 to 4 carbons or alkoxy having 1 to 4 carbons or hydroxyl or halogen, phenyl which may have alkyl having 1 to 4 carbons or alkenyl having 2 to 4 carbons or alkoxy having 1 to 4 carbons or halogen, benzyl which may have alkyl having 1 to 4 carbons or alkenyl having 2 to 4 carbons or alkoxy having 1 to 4 carbons or hydroxyl or halogen, phenethyl which may have alkyl having 1 to 4 carbons or alkenyl having 2 to 4 carbons or alkoxy having 1 to 4 carbons or hydroxyl or halogen, α-methylbenzyl which may have alkyl having 1 to 4 carbons or alkenyl having 2 to 4 carbons or alkoxy having 1 to 4 carbons or hydroxyl or halogen, or naphthyl which may have alkyl having 1 to 4 carbons or alkenyl having 2 to 4 carbons or alkoxy having 1 to 4 carbons or hydroxyl or halogen].

4. A molecular compound containing, as a constituent, a phenol derivative represented by Formula (VI) 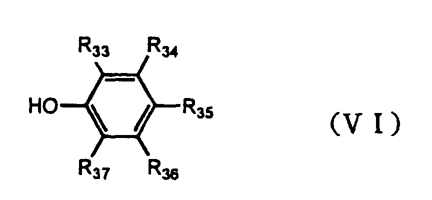 [wherein R₃₃ is 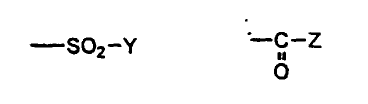 (wherein Y and Z are alkyl having 1 to 6 carbons, alkenyl having 2 to 6 carbons, cyclohexyl which may have alkyl having 1 to 4 carbons or alkenyl having 2 to 4 carbons or alkoxy having 1 to 4 carbons or hydroxyl or halogen, cyclopentyl which may have alkyl having 1 to 4 carbons or alkenyl having 2 to 4 carbons or alkoxy having 1 to 4 carbons or hydroxyl or halogen, phenyl which may have alkyl having 1 to 4 carbons or alkenyl having 2 to 4 carbons or alkoxy having 1 to 4 carbons or halogen, benzyl which may have alkyl having 1 to 4 carbons or alkenyl having 2 to 4 carbons or alkoxy having 1 to 4 carbons or hydroxyl or halogen, phenethyl which may have alkyl having 1 to 4 carbons or alkenyl having 2 to 4 carbons or alkoxy having 1 to 4 carbons or hydroxyl or halogen, α-methylbenzyl which may have alkyl having 1 to 4 carbons or alkenyl having 2 to 4 carbons or alkoxy having 1 to 4 carbons or hydroxyl or halogen, or naphthyl which may have alkyl having 1 to 4 carbons or alkenyl having 2 to 4 carbons or alkoxy having 1 to 4 carbons or hydroxyl or halogen), and R₃₄, R₃₅, R₃₆ and R₃₇ are , same or different, hydrogen, alkyl having 1 to 4 carbons, alkenyl having 2 to 4 carbons, alkoxy having 1 to 4 carbons, hydroxyl, halogen or the same groups as those for R₃₃].

5. A molecular compound according to Claims 1 to 4, in which the molecular compound is a clathrate compound.

6. A molecular compound according to Claims 1 to 5, in which the molecular compound contains, as constituents, a phenol derivative of Formula (I), (IV), (V) or (VI) and antibacterial agents, antifungal agents, insecticides, noxious insect repellants, perfumes, deodorants, antifouling agents, curing agents and accelerators for coating materials, resins and adhesives, natural essential oils, antioxidants, vulcanization accelerators or organic solvents, that react with the said phenol derivative to form a molecular compound.

7. A process for producing a molecular compound according to Claims 1 to 6, in which a phenol derivative of Formula (I), (IV), (V) or (VI) is reacted with constituent compounds that react with the said phenol compound to form a molecular compound.
